# EUROPÄISCHE PATENTSCHRIFT

(11) **EP 1 732 917 B1**
(45) Veröffentlichungstag und Bekanntmachung des Hinweises auf die Patenterteilung: **29.07.2009**
(21) Anmeldenummer: 05741783.4
(22) Anmeldetag: 23.03.2005
(51) Int. Cl.: C07D 401/04, C07D 401/14, A61K 31/551, A61P 25/06

(54) **AUSGEWAHLTE CGRP-ANTAGONISTEN, VERFAHREN ZU DEREN HERSTELLUNG SOWIE DEREN VERWENDUNG ALS ARZNEIMITTEL**
SELECTED CGRP ANTAGONISTS, METHODS FOR THE PRODUCTION THEREOF AND THEIR USE AS MEDICAMENTS
ANTAGONISTES CGRP SELECTIONNES, PROCEDES DE PRODUCTION ASSOCIES ET LEUR UTILISATION EN TANT QUE MEDICAMENTS

(30) Priorität: 29.03.2004 DE 102004015723
(43) Veröffentlichungstag der Anmeldung: 20.12.2006
(60) Teilanmeldung: 09160781.2
(73) Patentinhaber: Boehringer Ingelheim Pharma GmbH, 55216 Ingelheim am Rhein (DE); Boehringer Ingelheim Pharma GmbH & Co. KG, 55216 Ingelheim (DE)
(72) Erfinder: MUELLER, Stephan, Georg, 88447 Warthausen (DE); RUDOLF, Klaus, 88447 Warthausen (DE); LUSTENBERGER, Philipp, 4056 Basel (CH); STENKAMP, Dirk, 88400 Biberach (DE); DREYER, Alexander, 88484 Gutenzell-Hürbel (DE); ARNDT, Kirsten, 88400 Biberach (DE); DOODS, Henri, 88447 Warthausen (DE); SCHAENZLE, Gerhard, 88400 Biberach-Mettenberg (DE); SANTAGOSTINO, Marco, 88441 Mittelbiberach (DE); PALEARI, Fabio, I-20052 Monza (IT)
(74) Vertreter: Hammann, Heinz
(86) Internationale Anmeldenummer: PCT/EP2005/003094
(87) Internationale Veröffentlichungsnummer: WO 2005/092880

(56) Entgegenhaltungen:
- WO-A-20/04037810
- WO-A-20/04037811
- US-A1- 2003 069 231

## Beschreibung

Gegenstand der vorliegenden Erfindung sind die CGRP-Antagonisten der allgemeinen Formel 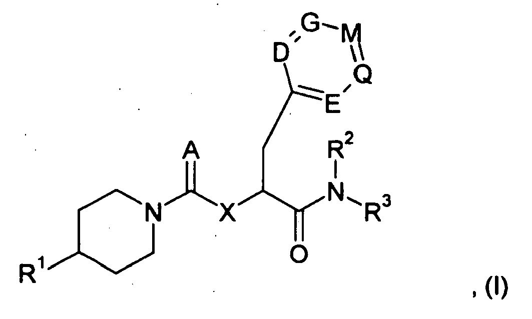 in denen A, D, E, G, M, Q, X, R¹, R² und R³ wie in Anspruch 1 definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

In US 2003/0069231 werden bereits strukturähnliche Verbindungen beschrieben, welche CGRP-antagonistische Eigenschaften besitzen.

In der obigen allgemeinen Formel (I) bedeuten in einer ersten Ausführungsform
A ein Sauerstoff- oder Schwefelatom,
X ein Sauerstoff- oder Schwefelatom,
(a) D, E unabhängig voneinander jeweils eine Methingruppe oder das Stickstoffatom und
   G eine durch die Gruppe R^{a} substituierte Methingruppe,
   M eine durch die Gruppe R^{b} substituierte Methingruppe,
   Q eine durch die Gruppe R^{c} substituierte Methingruppe,
   wobei eine oder zwei der Gruppen G, M und Q jeweils auch ein Stickstoffatom bedeuten können,
   oder
(b) D und E jeweils eine Methingruppe, wobei eine der Gruppen D und E auch ein Stickstoffatom bedeuten kann, und
   G, M und Q jeweils ein Stickstoffatom,
wobei R^{a}, R^{b} und R^{c} unabhängig voneinander jeweils ein Wasserstoff- oder Halogenatom, eine C₁₋₆-Alkyl-, C₂₋₆-Alkenyl-, C₂₋₆-Alkinyl-, Cyclo-C₃₋₇-alkyl-, Cyclo-C₃₋₇-alkenyl-, Cyano-, Hydroxy-, Hydroxy-C₁₋₆-alkyl-, Hydroxy-C₃₋₆-alkenyl-, Hydroxy-C₃₋₆-alkinyl-, C₁₋₆-Alkoxy-, C₁₋₆-Alkoxy-C₁₋₆-alkyl-, C₁₋₆-Alkoxy-C₃₋₆-alkenyl-, C₁₋₆-Alkoxy-C₃₋₆-alkinyl-, C₃₋₆-Alkenoxy-C₁₋₆-alkyl-, C₃₋₆-Alkenoxy-C₃₋₆-alkenyl-, C₃₋₆-Alkenoxy-C₃₋₆-alkinyl-, C₃₋₆-Alkinoxy-C₁₋₆-alkyl-, C₃₋₆-Alkinoxy-C₃₋₆-alkenyl-, C₃₋₆-Alkinoxy-C₃₋₆-alkinyl-, Thiohydroxy-, C₁₋₆-Alkylthio-, C₃₋₆-Alkenylthio-, C₃₋₆-Alkinylthio-, Amino-, C₁₋₆-Alkyl-amino-, C₃₋₆-Alkenyl-amino-, C₃₋₆-Alkinyl-amino-, Di-(C₁₋₆-alkyl)-amino-, Di-(C₃₋₆-alkenyl)-amino-, Di-(C₃₋₆-alkinyl)-amino-, Amino-C₁₋₆-alkyl-, C₁₋₃-Alkyl-amino-C₁₋₆-alkyl-, Di(C₁₋₃-Alkyl)-amino-C₁₋₆-alkyl-, Amino-C₃₋₆-alkenyl-, C₁₋₃-Alkyl-amino-C₃₋₆-alkenyl-, Di-(C₁₋₃-alkyl)-amino-C₃₋₆-alkenyl-, Amino-C₃₋₆-alkinyl-, C₁₋₃-Alkyl-amino-C₃₋₆-alkinyl-, Di-(C₁₋₃-alkyl)-amino-C₃₋₆-alkinyl-, Hydroxycarbonyl-, Phenylcarbonyl-, Pyridylcarbonyl-, C₁₋₆-Alkyl-carbonyl-, C₂₋₆-Alkenyl-carbonyl-, C₂₋₆-Alkinyl-carbonyl-, Formyl-, C₁₋₆-Alkoxy-carbonyl-, C₃₋₆-Alkenoxy-carbonyl-, C₃₋₆-Alkinoxy-carbonyl-, Aminocarbonyl-, C₁₋₆-Alkyl-aminocarbonyl-, C₃₋₆-Alkenyl-aminocarbonyl-, C₃₋₆-Alkinyl-aminocarbonyl-, Di-(C₁₋₆-alkyl)-aminocarbonyl-, Di-(C₃₋₆-alkenyl)-aminocarbonyl-, Di-(C₃₋₆-alkinyl)-aminocarbonyl-, Formylamino-, C₁₋₆-Alkyl-carbonylamino-, C₂₋₆-Alkenyl-carbonylamino-, C₂₋₆-Alkinyl-carbonylamino-, Formyl-C₁₋₆-alkyl-amino-, Formyl-C₃₋₆-alkenyl-amino-, Formyl-C₃₋₆-alkinyl-amino-, C₁₋₆-Alkyl-carbonyl-C₁₋₆-alkyl-amino-, C₂₋₆-Alkenyl-carbonyl-C₁₋₆-alkyl-amino-, C₂₋₆-Alkinyl-carbonyl-C₁₋₆-alkyl-amino-, C₁₋₆-Alkyl-carbonyl-C₃₋₆-alkenyl-amino-, C₂₋₆-Alkenyl-carbonyl-C₃₋₆-alkenyl-amino-, C₂₋₆-Alkinyl-carbonyl-C₃₋₆-alkenyl-amino-, C₁₋₆-Alkyl-carbonyl-C₃₋₆-alkinyl-amino-, C₂₋₆-Alkenyl-carbonyl-C₃₋₆-alkinyl-amino-, C₂₋₆-Alkinyl-carbonyl-C₃₋₆-alkinyl-amino-, C₁₋₆-Alkyl-sulfonyl-, C₂₋₆-Alkenyl-sulfonyl-, C₂₋₆-Alkinyl-sulfonyl-, C₁₋₆-Alkyl-sulfinyl-, C₂₋₆-Alkenyl-sulfinyl-, C₂₋₆-Alkinyl-sulfinyl-, C₁₋₆-Alkyl-sulfonylamino-, C₂₋₆-Alkenyl-sulfonylamino-, C₂₋₆-Alkinyl-sulfonylamino-, C₁₋₆-Alkyl-sulfonyl-C₁₋₆-alkylamino-, C₁₋₆-Alkyl-sulfonyl-C₃₋₆-alkenylamino-, C₁₋₆-Alkyl-sulfonyl-C₃₋₆-alkinylamino-, C₂₋₆-Alkenyl-sulfonyl-C₁₋₆-alkylamino-, C₂₋₆-Alkenyl-sulfonyl-C₃₋₆-alkenylamino-, C₂₋₆-Alkenyl-sulfonyl-C₃₋₆-alkinylamino-, C₂₋₆-Alkinyl-sulfonyl-C₁₋₆-alkylamino-, C₂₋₆-Alkinyl-sulfonyl-C₃₋₆-alkenylamino-, C₂₋₆-Alkinyl-sulfonyl-C₃₋₆-alkinylamino-, Aminosulfonyl-, C₁₋₆-Alkylaminosulfonyl-, Di-(C₁₋₆-alkyl)-aminosulfonyl-, C₃₋₆-Alkenylaminosulfonyl-, Di-(C₃₋₆-alkenyl)-aminosulfonyl-, C₃₋₆-Alkinylaminosulfonyl- oder Di-(C₃₋₆-alkinyl)-aminosulfonylgruppe bedeuten
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₆-Alkylgruppe darstellen,
(ii) R^{c} kein Wasserstoffatom bedeutet, wenn R^{a} sowie R^{b} jeweils eine C₁₋₆-Alkylgruppe darstellen,
(iii) R^{a} nicht die Bedeutungen eines Wasserstoff-, Fluor-, Chlor-, Brom- oder Iodatoms oder einer Difluor- oder Trifluormethylgruppe annimmt, wenn R^{c} eine C₁₋₆-Alkyl-, C₂₋₆-Alkenyl- oder C₂₋₆-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₆-Alkyl-, C₂₋₆-Alkenyl- oder C₂₋₆-Alkinylgruppe und R^{b} ein Chlor- oder Bromatom, eine Amino-, Methylamino- oder Hydroxygruppe darstellen, R¹ eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-c]chinolin-2-on-3-yl-, 1,3-Dihydro-naphth[1,2-d]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H-*thieno[3,2-*d*]pyrimidin-2-on-3-yl- oder 3,4-Dihydro-1*H*-thieno[3,4-d]pyrimidin-2-on-3-ylgruppe,
wobei die vorstehend unter R¹ erwähnten Heterocyclen im Kohlenstoffgerüst zusätzlich durch eine Methoxygruppe monosubstituiert sein können,
wobei alle in den vorstehend unter R¹ definierten Resten erwähnten oder enthaltenen aromatischen und heteroaromatischen Reste zusätzlich durch Halogenatome, durch Cyano- oder Hydroxygruppen mono-, di- oder trisubstituiert und die Substituenten gleich oder verschieden sein können, und
wobei die Doppel- und Dreifachbindungen der in den für R^{a}, R^{b}, R^{c} und R¹ vorstehend definierten Gruppen enthaltenen C₃₋₆-Alkenyl- oder C₃₋₆-Alkinylgruppen von in diesen Gruppen gegebenenfalls ebenfalls enthaltenen Heteroatomen isoliert sind,
R² das Wasserstoffatom,
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Diazinyl-, Hydroxy-, Amino-, C₁₋₆-Alkylamino-, Di-(C₁₋₆-alkyl)-amino-, C₃₋₆-Alkenylamino-, Di-(C₃₋₆-alkenyl)amino-, C₃₋₆-Alkinylamino-, Di-(C₃₋₆-alkinyl)amino-, Hydroxycarbonyl-, C₁₋₆-Alkoxycarbonyl-, Aminocarbonyl-, Aminocarbonylamino-, C₁₋₆-Alkylcarbonylamino-, C₂₋₆-Alkenyl-carbonylamino-, C₂₋₆-Alkinylcarbonylamino-, 4-Morpholinyl-, [Bis-(2-hydroxyethyl)]amino-, 4-(C₁₋₆-Alkyl)-1-piperazinyl- oder 4-(ω-Hydroxy-C₂₋₇-alkyl)-1-piperazinylgruppe substituiert sein kann,
eine Phenyl- oder Pyridinylgruppe,
wobei die in den für R² vorstehend definierten Gruppen genannten oder als Substituenten enthaltenen Phenyl-, Pyridinyl- und Diazinylgruppen zusätzlich im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkyl-amino-C₁₋₃-alkyl-, Di-(C₁₋₃-alkyl)-amino-C₁₋₃-alkyl-, C₁₋₃-Alkylcarbonylamino-, C₁₋₃-Alkylcarbonylamino-C₁₋₃-alkyl-, Aminocarbonyl-, C₁₋₃-Alkyl-aminocarbonyl-, Di-(C₁₋₃-alkyl)-aminocarbonyl-, Cyano-, Aminosulfonyl-, C₁₋₃-Alkyl-aminosulfonyl-, Di-(C₁₋₃-alkyl)-aminosulfonyl-, C₁₋₃-Alkyl-thio-, C₁₋₃-Alkyl-sulfinyl-, oder C₁₋₃-Alkyl-sulfonyl- mono-, di- oder trisubstituiert und die Substituenten gleich oder verschieden sein können,
R³ das Wasserstoffatom oder eine durch eine Phenyl- oder Pyridinylgruppe substituierte C₁₋₃-Alkylgruppe,
wobei die C₁₋₃-Alkylgruppe mit einer in R² enthaltenen Alkylgruppe oder einem in R² enthaltenen Phenyl- oder Pyridylring unter Einschluss des Stickstoffatoms, an welches R² und R³ gebunden sind, unter Ausbildung eines 4- 7-gliedrigen Ringes verbunden sein kann,
oder
R² und R³ zusammen mit dem eingeschlossenen Stickstoffatom einen Rest der allgemeinen Formel 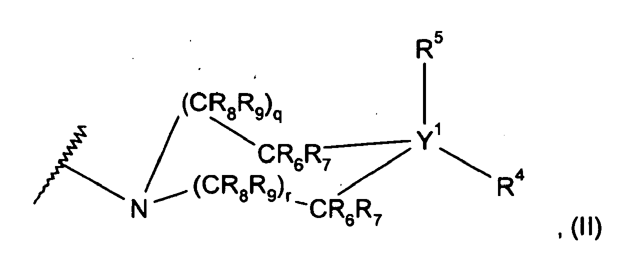 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl- oder Cyclo-C₃₋₇-alkenylgruppe, eine Amino-C₂₋₇-alkyl-, C₁₋₄-Alkyl-amino-C₂₋₇-alkyl-, Di-(C₁₋₄-alkyl-amino)-C₂₋₇-alkyl-, Aminoiminomethyl-, Aminocarbonylamino-, C₁₋₄-Alkyl-aminocarbonylamino-, Di-(C₁₋₄-alkyl)-aminocarbonylamino-, C₁₋₄-Alkyl-aminocarbonyl-C₁₋₄-alkyl-amino-, Di-(C₁₋₄-alkyl)-aminocarbonyl-C₁₋₄-alkyl-amino-, Phenylaminocarbonylamino-, Aminocarbonyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, C₃₋₆-Alkenoxycarbonyl-, C₃₋₆-Alkinoxycarbonyl-, C₁₋₆-Alkoxycarbonyl-C₁₋₃-alkyl-, C₁₋₆-Alkenoxycarbonyl-C₁₋₃-alkyl-, C₁₋₆-Alkinoxycarbohyl-C₁₋₃-alkyl- oder Hydroxycarbonyl-C₁₋₃-alkylgruppe,
eine Phenyl-, Pyridinyl-, Diazinyl-, 1-Naphthyl-, 2-Naphthyl-, Pyridinylcarbonyl- oder Phenylcarbonylgruppe, die jeweils im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkyl-amino-C₁₋₃-alkyl-, Di-(C₁₋₄-alkyl)-amino-C₁₋₃-alkyl-, C₁₋₄-Alkylcarbonylamino-, C₁₋₄-Alkylcarbonylamino-C₁₋₃-alkyl-, Aminocarbonyl-, C₁₋₄-Alkyl-aminocarbonyl-, Di-(C₁₋₄-alkyl)-aminocarbonyl-, Cyano-, Aminosulfonyl-, C₁₋₄-Alkyl-aminosulfonyl-, Di-(C₁₋₄-alkyl)-aminosulfonyl-, C₁₋₄-Alkyl-thio-, C₁₋₄-Alkyl-sulfinyl-, oder C₁₋₄-Alkylsulfonyl- mono-, di- oder trisubstituiert und die Substituenten gleich oder verschieden sein können,
ein Heterocyclus ausgewählt aus einer 4- bis 10-gliedrigen Azacycloalkylgruppe, einer 6- bis 10-gliedrige Oxaza-, Thiaza-, S,S-Dioxothiaza- und Diazacycloalkylgruppe sowie einer 6- bis 1 0-gliedrigen Azabicycloalkylgruppe,
eine 1-Alkyl-4-piperidinylcarbonyl- oder 4-Alkyl-1-piperazinylcarbonylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methingruppe durch ein Fluoratom sowie eine nicht direkt mit einem Stickstoff-, Sauerstoff- oder Schwefelatom verknüpfte Methylengruppe durch ein oder zwei Fluoratome substituiert sein kann,
die vorstehend genannten mono- und bicyclischen Heterocyclen sowie die 1-(C₁₋₆-Alkyl)-4-piperidinylcarbonyl- und 4-(C₁₋₆-Alkyl)-1-piperazinylcarbonylgruppe im Ring ein- bis vierfach durch Hydroxy-, C₁₋₆-Alkyl- oder Hydroxy-C₁₋₃-alkylgruppen, oder, gegebenenfalls zusätzlich, einfach durch eine Cyclo-C₃₋₇-alkyl-, Hydroxy-C₃₋₇-cycloalkyl-, Cyclo-C₃₋₇-alkenyl-, Cyclo-C₃₋₇-alkyl-C₁₋₃-alkyl-, Phenyl-C₁₋₃-alkyl-, Pyridyl-C₁₋₃-alkyl-, C₁₋₆-Alkylcarbonyl-, C₁₋₆-Alkylcarbonyl-C₁₋₃-alkyl-, Hydroxy-, C₁₋₆-Alkoxy-, Amino-, C₁₋₄-Alkylamino-, Di-(C₁₋₄-alkyl)amino-, Phenylcarbonyl-Pyridinylcarbonyl-, C₁₋₆-Alkoxycarbonyl-, Hydroxycarbonyl-carbonyl-, C₁₋₆-Alkoxycarbonyl-carbonyl-, Hydroxycarbonyl-C₁₋₃-alkyl-, C₁₋₆-Alkoxycarbonyl-C₁₋₃-alkyl, Hydroxycarbonyl-C₁₋₃-alkylcarbonyl-, C₁₋₆-Alkoxycarbonyl-C₁₋₃-alkylcarbonyl-, Aminocarbonyl-, C₁₋₄-Alkylaminocarbonyl-, Di-(C₁₋₄-alkyl)aminocarbonyl-, Aminosulfonyl-, C₁₋₄-Alkylaminosulfonyl-, Di-(C₁₋₄-alkyl)aminosulfonyl-, C₁₋₃-Alkylsulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)aminocarbonyl-C₁₋₃-alkyl-, Hydroxyaminocarbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppe, durch eine im Ring gegebenenfalls C₁₋₃-alkylsubstituierte Cyclo-C₃₋₇-alkylcarbonyl-, Azacyclo-C₄₋₇-alkylcarbonyl-, Diazacydo-C₅₋₇-alkylcarbonyl- oder Oxazacydo-C₅₋₇-alkylcarbonylgruppe substituiert sein können, wobei die Substituenten gleich oder verschieden und an ein Ring-Kohlenstoff- oder Ring-Stickstoffatom gebunden sein können,
wobei die in den für R⁴ vorstehend definierten Resten enthaltenen Phenyl- und Pyridinyl-Reste ihrerseits durch Halogenatome, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkylamino-C₁₋₃-alkyl-, Di-(C₁₋₄-alkyl)-amino-C₁₋₃-alkyl-, C₁₋₄-Alkylcarbonylamino-, C₁₋₄-Alkylcarbonylamino-C₁₋₃-alkyl-, Aminocarbonyl-, C₁₋₃-Alkyl-aminocarbonyl-, Di-C₁₋₄-alkyl-aminocarbonyl-, Cyano-, Aminosulfonyl-, C₁₋₄-Alkyl-aminosulfonyl-, Di-(C₁₋₄-alkyl)-aminosulfonyl-, C₁₋₃-Alkylthio-, C₁₋₃-Alkyl-sulfinyl-, oder C₁₋₃-Alkyl-sulfonyl- mono-, di- oder trisubstituiert sein können, wobei die Substituenten gleich oder verschieden sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom oder eine Hydroxygruppe,
einen C₁₋₄-Alkylrest, wobei ein unverzweigter Alkylrest in ω-Stellung durch eine Phenyl-, Pyridinyl-, Diazinyl-, Amino-, C₁₋₄-Alkylamino-, Di-(C₁₋₄-alkyl)-amino-, 4 C₁₋₄-Alkyl-1-piperazinyl- oder 4-Morpholinylgruppe substituiert sein kann,
eine C₁₋₆-Alkoxycarbonyl-, Cyano- oder Aminocarbonylgruppe oder auch, wenn Y¹ ein Stickstoffatom darstellt, ein freies Elektronenpaar,
oder, wenn Y¹ das Kohlenstoffatom darstellt, auch das Fluoratom, oder
R⁴ zusammen mit R⁵ und Y¹ einen 4- bis 7-gliedrigen cycloaliphatischen Ring, in dem eine Methylengruppe durch eine Gruppe -NH-, -N(C₁₋₄-Alkyl)-, -N(C₃₋₄-Alkenyl)-, -N(C₃₋₄-Alkinyl)-, -N(Cyclo-C₃₋₇-alkyl)-, -N(C₃₋₇-Cycloalkyl-C₁₋₃-alkyl)-, -N(Hydroxycarbonyl-C₁₋₃-alkyl)- oder -N(C₁₋₆-Alkoxycarbonyl-C₁₋₃-alkyl)- ersetzt sein kann,
wobei ein an ein Stickstoffatom in einer der für R⁴ vorstehend definierten Gruppen gebundenes Wasserstoffatom durch einen Schutzrest ersetzt sein kann,
R⁶ und R⁷, die gleich oder verschieden sein können, jeweils ein Wasserstoffatom, eine C₁₋₄-Alkylgruppe oder auch, wenn Y¹ ein Kohlenstoffatom darstellt, das Fluoratom, eine Amino-, C₁₋₄-Alkylamino- oder Di-(C₁₋₄-alkyl)-aminogruppe, wobei die beiden C₁₋₄-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können und
unter den in den vor- und nachstehenden Definitionen genannten Schutzresten die aus der Peptidchemie geläufigen Schutzgruppen zur verstehen sind, insbesondere
eine im Phenylkern gegebenenfalls durch ein Halogenatom, durch eine Nitro- oder Phenylgruppe, durch eine oder zwei Methoxygruppen substituierte Phenylalkoxycarbonylgruppe mit 1 bis 3 Kohlenstoffatomen im Alkoxyteil,
beispielsweise die Benzyloxycarbonyl-, 2-Nitrobenzyloxycarbonyl-, 4-Nitro-benzyloxycarbonyl-, 4-Methoxy-benzyloxycarbonyl-, 2-Chlor-benzyloxycarbonyl-, 3-Chlor-benzyloxycarbonyl-, 4-Chlor-benzyloxycarbonyl-, 4-Biphenylyl-α,α-dimethyl-benzyloxycarbonyl- oder 3,5-Dimethoxy-α,α-dimethyl-benzyloxycarbonylgruppe,
eine Alkoxycarbonylgruppe mit insgesamt 1 bis 5 Kohlenstoffatomen im Alkylteil,
beispielsweise die Methoxycarbonyl-, Ethoxycarbonyl-, *n*-Propoxycarbonyl-, Isopropoxycarbonyl-, *n*-Butoxycarbonyl-, 1-Methylpropoxycarbonyl-, 2-Methylpropoxy-carbonyl- oder *tert*.Butyloxycarbonylgruppe,
die Allyloxycarbonyl-, 2,2,2-Trichlor-(1,1-dimethylethoxy)carbonyl- oder 9-Fluorenylmethoxycarbonylgruppe oder
die Formyl-, Acetyl- oder Trifluoracetylgruppe.

In den vor- und nachstehenden Definitionen ist unter einer in ω-Stellung substituierten Gruppe eine terminal substituierte Gruppe,
unter einem Halogenatom ein Fluor-, Chlor-, Brom- oder lodatom und
unter einer von einem Heteroatom isolierten Doppel- oder Dreifachbindung eine Doppel- oder Dreifachbindung, die über mindestens ein gesättigtes Kohlenstoffatom mit einem Heteroatom verknüpft ist, zu verstehen.

Eine zweite Ausführungsform der vorliegenden Erfindung besteht in den Verbindungen der obigen allgemeinen Formel (I), in denen
A, X, D, E, G, M, Q und R¹ wie vorstehend unter der ersten Ausführungsform erwähnt definiert sind und
R² das Wasserstoffatom,
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Diazinyl-, Hydroxy-, Amino-, C₁₋₆-Alkylamino-, Di-(C₁₋₆-alkyl)-amino-, C₃₋₆-Alkenylamino-, Di-(C₃₋₆-alkenyl)amino-, C₃₋₆-Alkinylamino-, Di-(C₃₋₆-alkinyl)amino-, Hydroxycarbonyl-, C₁₋₆-Alkoxycarbonyl-, Aminocarbonyl-, Aminocarbonylamino-, C₁₋₆-Alkylcarbonylamino-, C₂₋₆-Alkenyl-carbonylamino-, C₂₋₆-Alkinylcarbonylamino-, 4-Morpholinyl-, [Bis-(2-hydroxyethyl)]amino-, 4-(C₁₋₆-Alkyl)-1-piperazinyl- oder 4-(ω-Hydroxy-C₂₋₇-alkyl)-1-piperazinylgruppe substituiert sein kann,
eine Phenyl- oder,Pyridinylgruppe,
wobei die in den für R² vorstehend definierten Gruppen genannten oder als Substituenten enthaltenen Phenyl-, Pyridinyl- und Diazinylgruppen zusätzlich im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkyl-amino-C₁₋₃-alkyl-, Di-(C₁₋₃-alkyl)-amino-C₁₋₃-alkyl-, C₁₋₃-Alkylcarbonylamino-, C₁₋₃-Alkylcarbonylamino-C₁₋₃-alkyl-, Aminocarbonyl-, C₁₋₃-Alkyl-aminocarbonyl-, Di-(C₁₋₃-alkyl)-aminocarbonyl-, Cyano-, Aminosulfonyl-, C₁₋₃-Alkyl-aminosulfonyl-, Di-(C₁₋₃-alkyl)-aminosulfonyl-, C₁₋₃-Alkyl-thio-, C₁₋₃-Alkyl-sulfinyl-, oder C₁₋₃-Alkyl-sulfonyl- mono-, di- oder trisubstituiert und die Substituenten gleich oder verschieden sein können,
R³ das Wasserstoffatom oder eine durch eine Phenyl- oder Pyridinylgruppe substituierte C₁₋₃-Alkylgruppe,
wobei die C₁₋₃-Alkylgruppe mit einer in R² enthaltenen Alkylgruppe oder einem in R² enthaltenen Phenyl- oder Pyridylring unter Einschluss des Stickstoffatoms, an welches R² und R³ gebunden sind, unter Ausbildung eines 4- 7-gliedrigen Ringes verbunden sein kann,
oder
R² und R³ zusammen mit dem eingeschlossenen Stickstoffatom einen Rest der allgemeinen Formel 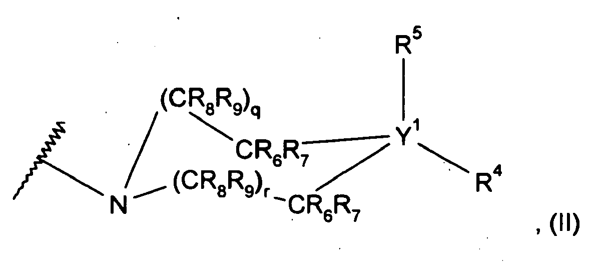 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, eine Cyclo-C₃₋₇-alkyl-, Cyclo-C₃₋₇-alkenyl-, Amino-C₂₋₇-alkyl-, C₁₋₄-Alkyl-amino-C₂₋₇-alkyl-, Di-(C₁₋₄-alkyl-amino)-C₂₋₇-alkyl-, Aminoiminomethyl-, Aminocarbonylamino-, C₁₋₄-Alkyl-aminocarbonylamino-, Di-(C₁₋₄-alkyl)-aminocarbonylamino-, C₁₋₄-Alkyl-aminocarbonyl-C₁₋₄-alkyl-amino-, Di-(C₁₋₄-alkyl)-aminocarbonyl-C₁₋₄-alkyl-amino-, Phenylaminocarbonylamino-, Aminocarbonyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, C₃₋₆-Alkenoxycarbonyl-, C₃₋₆-Alkinoxycarbonyl-, C₁₋₆-Alkoxycarbonyl-C₁₋₃-alkyl-, C₁₋₆-Alkenoxycarbonyl-C₁₋₃-alkyl-, C₁₋₆-Alkinoxycarbonyl-C₁₋₃-alkyl- oder Hydroxycarbonyl-C₁₋₃-alkylgruppe,
eine Phenyl-, Pyridinyl-, Diazinyl-, 1-Naphthyl-, 2-Naphthyl-, Pyridinylcarbonyl- oder Phenylcarbonylgruppe, die jeweils im Kohlenstoffgerüst durch Halogen, durch C₁₋₃-Alkyl-, C₂₋₃-Alkenyl-; C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkyl-amino-C₁₋₃-alkyl-, Di-(C₁₋₄-alkyl)-amino-C₁₋₃-alkyl-, C₁₋₄-Alkylcarbonylamino-, C₁₋₄-Alkylcarbonylamino-C₁₋₃-alkyl-, Aminocarbonyl-, C₁₋₄-Alkyl-aminocarbonyl-, Di-(C₁₋₄-alkyl)-aminocarbonyl-, Cyano-, Aminosulfonyl-, C₁₋₄-Alkyl-aminosulfonyl-, Di-(C₁₋₄-alkyl)-aminosulfonyl-, C₁₋₄-Alkyl-thio-, C₁₋₄-Alkyl-sulfinyl-, oder C₁₋₄-Alkylsulfonyl- mono-, di- oder trisubstituiert und die Substituenten gleich oder verschieden sein können,
ein Heterocyclus ausgewählt aus einer 4- bis 10-gliedrigen Azacycloalkylgruppe, einer 6- bis 10-gliedrige Oxaza-, Thiaza- und Diazacycloalkylgruppe sowie einer 6- bis 10-gliedrigen Azabicycloalkylgruppe,
eine 1-Alkyl-4-piperidinylcarbonyl-oder 4-Alkyl-1-piperazinylcarbonylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methingruppe durch ein Fluoratom sowie eine nicht direkt mit einem Stickstoff-, Sauerstoff- oder Schwefelatom verknüpfte Methylengruppe durch ein oder zwei Fluoratome substituiert sein kann,
die vorstehend genannten mono- und bicyclischen Heterocyclen sowie die 1-(C₁₋₆-Alkyl)-4-piperidinylcarbonyl- und 4-(C₁₋₆-Alkyl)-1-piperazinylcarbonylgruppe im Ring ein- bis vierfach durch C₁₋₆-Alkylgruppen, oder, gegebenenfalls zusätzlich, einfach durch eine Cyclo-C₃₋₇-alkyl-, Cyclo-C₃₋₇-alkenyl-, Cyclo-C₃₋₇-alkyl-C₁₋₃-alkyl-, Phenyl-C₁₋₃-alkyl-, Pyridyl-C₁₋₃-alkyl-, C₁₋₆-Alkylcarbonyl-, Hydroxy-, C₁₋₆-Alkoxy-, Amino-, C₁₋₄-Alkylamino-, Di-(C₁₋₄-alkyl)amino-, Phenylcarbonyl-, Pyridinylcarbonyl-, Hydroxycarbonyl-, Hydroxycarbonyl-C₁₋₃-alkyl-, C₁₋₆-Alkoxyca rbonyl-, C₁₋₆-Alkoxycarbonyl-C₁₋₃-alkyl, Aminocarbonyl-, C₁₋₄-Alkylaminocarbonyl-, Di-(C₁₋₄-alkyl)aminocarbonyl-, C₁₋₃-Alkylsulfonylgruppe, durch eine im Ring gegebenenfalls C₁₋₃-alkylsubstituierte Cyclo-C₃₋₇-alkylcarbonyl-, Azacyclo-C₄₋₇-alkylcarbonyl-, Diazacyclo-C₅₋₇-alkylcarbonyl- oder Oxazacyclo-C₅₋₇-alkylcarbonylgruppe substituiert sein können, wobei die Substituenten gleich oder verschieden und an ein Ring-Kohlenstoff- oder Ring-Stickstoffatom gebunden sein können,
wobei die in den für R⁴ vorstehend definierten Resten enthaltenen Phenyl- und Pyridinyl-Reste ihrerseits durch Halogenatome, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkylamino-C₁₋₃-alkyl-, Di-(C₁₋₄-alkyl)-amino-C₁₋₃-alkyl-, C₁₋₄-Alkylcarbonylamino-, C₁₋₄-Alkylcarbonylamino-C₁₋₃-alkyl-, Aminocarbonyl-, C₁₋₃-Alkyl-aminocarbonyl-, Di-C₁₋₄-alkyl-aminocarbonyl-, Cyano-, Aminosulfonyl-, C₁₋₄-Alkyl-aminosulfonyl-, Di-(C₁₋₄-alkyl)-aminosulfonyl-, C₁₋₃-Alkylthio-, C₁₋₃-Alkyl-sulfinyl-, oder C₁₋₃-Alkyl-sulfonyl- mono-, di- oder trisubstituiert sein können, wobei die Substituenten gleich oder verschieden sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom,
einen C₁₋₄-Alkylrest, wobei ein unverzweigter Alkylrest in ω-Stellung durch eine Phenyl-, Pyridinyl-, Diazinyl-, Amino-, C₁₋₄-Alkylamino-, Di-(C₁₋₄-alkyl)-amino-, 4 C₁₋₄-Alkyl-1-piperazinyl- oder 4-Morpholinylgruppe substituiert sein kann,
eine C₁₋₆-Alkoxycarbonyl-, Cyano- oder Aminocarbonylgruppe oder auch, wenn Y¹ ein Stickstoffatom darstellt, ein freies Elektronenpaar,
oder, wenn Y¹ das Kohlenstoffatom darstellt, auch das Fluoratom, oder
R⁴ zusammen mit R⁵ und Y¹ einen 4- bis 7-gliedrigen cycloaliphatischen Ring, in dem eine Methylengruppe durch eine Gruppe -NH-, -N(C₁₋₄-Alkyl)-, -N(C₃₋₄-Alkenyl)-, -N(C₃₋₄-Alkinyl)-, -N(Cyclo-C₃₋₇-alkyl)- oder -N(C₃₋₇-Cycloalkyl-C₁₋₃-alkyl)- ersetzt sein kann,
wobei ein an ein Stickstoffatom in einer der für R⁴ vorstehend definierten Gruppen gebundenes Wasserstoffatom durch einen Schutzrest ersetzt sein kann,
R⁶ und R⁷, die gleich oder verschieden sein können, jeweils ein Wasserstoffatom, eine C₁₋₄-Alkylgruppe oder auch, wenn Y¹ ein Kohlenstoffatom darstellt, das Fluoratom, eine C₁₋₄-Alkylamino- oder Di-(C₁₋₄-alkyl)-aminogruppe, wobei die beiden C₁₋₄-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine dritte Ausführungsform der vorliegenden Erfindung besteht in den Verbindungen der obigen allgemeinen Formel (I), in denen
A, X, R¹, R² und R³ wie vorstehend unter der ersten oder zweiten Ausführungsform erwähnt definiert sind und
(a) D, E unabhängig voneinander jeweils eine Methingruppe oder das Stickstoffatom und
   G eine durch die Gruppe R^{a} substituierte Methingruppe,
   M eine durch die Gruppe R^{b} substituierte Methingruppe,
   Q eine durch die Gruppe R^{c} substituierte Methingruppe,
   wobei eine oder zwei der Gruppen G, M und Q jeweils auch ein Stickstoffatom bedeuten kann,
   oder
(b) D und E jeweils eine Methingruppe, wobei eine der Gruppen D und E auch ein Stickstoffatom bedeuten kann, und
   G, M und Q jeweils ein Stickstoffatom bedeuten,
wobei R^{a}, R^{b} und R^{c} unabhängig voneinander jeweils ein Wasserstoff- oder - Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₄-alkyl-, Hydroxy-C₃₋₄-alkenyl-, Hydroxy-C₃₋₄₋alkinyl-, C₁₋₄-Alkoxy-, C₁₋₄-Alkoxy-C₁₋₄-alkyl-, C₁₋₄-Alkoxy-C₃₋₄-alkenyl-, C₁₋₄-Alkoxy-C₃₋₄-alkinyl-, Thiohydroxy-, C₁₋₄-Alkylthio-, Amino-, C₁₋₄-Alkyl-amino-, C₃₋₄-Alkenyl-amino-, C₃₋₄-Alkinyl-amino-, Di-(C₁₋₄-alkyl)-amino-, Di-(C₃₋₄-alkenyl)-amino-, Di-(C₃₋₄-alkinyl)-amino-, Amino-C₁₋₄-alkyl-, C₁₋₃-Alkyl-amino-C₁₋₄-alkyl-, Di-(C₁₋₃-alkyl)-amino-C₁₋₄-alkyl-, Amino-C₃₋₄-alkenyl-, C₁₋₃-Alkyl-amino-C₃₋₄-alkenyl-, Di-(C₁₋₃-alkyl)-amino-C₃₋₄-alkenyl-, Amino-C₃₋₄-alkinyl-, C₁₋₃-Alkyl-amino-C₃₋₄-alkinyl-, Di-(C₁₋₃-alkyl)-amino-C₃₋₄-alkinyl-, Hydroxycarbonyl-, Phenylcarbonyl-, Pyridylcarbonyl-, C₁₋₄-Alkyl-carbonyl-, Formyl-, C₁₋₄-Alkoxy-carbonyl-, C₃₋₄-Alkenoxy-carbonyl-, C₃₋₄-Alkinoxy-carbonyl-, Aminocarbonyl-, C₁₋₄-Alkyl-aminocarbonyl-, C₃₋₄-Alkenyl-aminocarbonyl-, C₃₋₄-Alkinyl-aminocarbonyl-, Di-(C₁₋₄-alkyl)-aminocarbonyl-, Di-(C₃₋₄-alkenyl)-aminocarbonyl-, Di-C₃₋₄-(alkinyl)-aminocarbonyl-, Formylamino-, C₁₋₄-Alkyl-carbonylamino-, Formyl-C₁₋₄-alkyl-amino-, Formyl-C₃₋₄-alkenyl-amino-, Formyl-C₃₋₄-alkinyl-amino-, C₁₋₄-Alkyl-carbonyl-C₁₋₄-alkyl-amino-, C₁₋₄-Alkyl-carbonyl-C₃₋₄-alkenyl-amino-, C₁₋₄-Alkyl-carbonyl-C₃₋₄-alkinyl-amino-, C₁₋₄-Alkyl-sulfonyl-, C₂₋₄-Alkenyl-sulfonyl-, C₂₋₄-Alkinyl-sulfonyl-, C₁₋₄-Alkyl-sulfinyl-, C₂₋₄-Alkenyl-sulfinyl-, C₂₋₄-Alkinyl-sulfinyl-, C₁₋₄-Alkyl-sulfonylamino-, C₁₋₄-Alkyl-sulfonyl-C₁₋₄-alkylamino-, C₁₋₄-Alkyl-sulfonyl-C₃₋₄-alkenylamino-, C₁₋₄-Alkyl-sulfonyl-C₃₋₄-alkinylamino-, Aminosulfonyl-, C₁₋₄-Alkylaminosulfonyl-, Di-(C₁₋₄-alkyl)-aminosulfonyl-, C₃₋₄-Alkenylamino-sulfonyl-, Di-(C₃₋₄-alkenyl)-aminosulfonyl-, C₃₋₄-Alkinylaminosulfonyl- oder Di-(C₃₋₄-alkinyl)-aminosulfonylgruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₄-Alkylgruppe darstellen,
(ii) R^{c} kein Wasserstoffatom bedeutet, wenn R^{a} sowie R^{b} jeweils eine C₁₋₄-Alkylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
wobei, soweit nichts anderes erwähnt wurde, alle in den vorstehend für R^{a}, R^{b} und R^{c} definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können,
die Doppel- und Dreifachbindungen der in den für R^{a}, R^{b} und R^{c} vorstehend definierten Gruppen enthaltenen C₃₋₄-Alkenyl- oder C₃₋₄-Alkinylgruppen von in diesen Gruppen gegebenenfalls ebenfalls enthaltenen Heteroatomen isoliert sind,
und alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine vierte Ausführungsform der vorliegenden Erfindung besteht in den Verbindungen der obigen allgemeinen Formel (I), in denen
A, X, R¹, R² und R³ wie vorstehend unter der ersten oder zweiten Ausführungsform erwähnt definiert sind und
(a) D, E unabhängig voneinander jeweils eine Methingruppe oder das Stickstoffatom und
   G eine durch die Gruppe R^{a} substituierte Methingruppe,
   M eine durch die Gruppe R^{b} substituierte Methingruppe,
   Q eine durch die Gruppe R^{c} substituierte Methingruppe,
   wobei eine oder zwei der Gruppen G, M und Q jeweils auch ein Stickstoffatom bedeuten kann,
   oder
(b) D und E jeweils eine Methingruppe, wobei eine der Gruppen D und E auch ein Stickstoffatom bedeuten kann, und
   G, M und Q jeweils ein Stickstoffatom bedeuten,
wobei R^{a}, R^{b} und R^{c} unabhängig voneinander jeweils ein Wasserstoff- oder - Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₂-alkyl-, Hydroxy-C₃-alkenyl-, Hydroxy-C₃-alkinyl-, C₁₋₄-Alkoxy-, C₁₋₄-Alkoxy-C₁₋₂-alkyl-, Amino-, C₁₋₄-Alkyl-amino-, C₃₋₄-Alkenyl-amino-, C₃₋₄-Alkinyl-amino-, Di-(C₁₋₄-alkyl)-amino-, Di-(C₃₋₄-alkenyl)-amino-, Di-(C₃₋₄-alkinyl)-amino-, Amino-C₁₋₂-alkyl-, C₁₋₃-Alkyl-amino-C₁₋₂-alkyl-, Di-(C₁₋₃-alkyl)-amino-C₁₋₂-alkyl-, Amino-C₃-alkenyl-, C₁₋₃-Alkyl-amino-C₃-alkenyl-, Di-(C₁₋₃-alkyl)-amino-C₃-alkenyl-, Amino-C₃-alkinyl-, C₁₋₃-Alkyl-amino-C₃-alkinyl-, Di-(C₁₋₃-alkyl)-amino-C₃-alkinyl-, Hydroxycarbonyl-, C₁₋₄-Alkyl-carbonyl-, Formyl-, C₁₋₄-Alkoxy-carbonyl-, Aminocarbonyl-, C₁₋₄-Alkyl-aminocarbonyl-, Di-(C₁₋₄-alkyl)-aminocarbonyl-, Formylamino-, C₁₋₄-Alkylcarbonylamino-, Formyl-C₁₋₄-alkyl-amino-, C₁₋₄-Alkyl-carbonyl-C₁₋₄-alkyl-amino-, C₁₋₄-Alkyl-sulfonyl-, C₁₋₄-Alkyl-sulfinyl-, C₁₋₄-Alkyl-sulfonylamino-, C₁₋₄-Alkyl-sulfonyl-C₁₋₄-alkylamino-, Aminosulfonyl-, C₁₋₄-Alkylaminosulfonyl- oder Di-(C₁₋₄-alkyl)-aminosulfonylgruppe darstellen, mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₄-Alkylgruppe darstellen,
(ii) R^{c} kein Wasserstoffatom bedeutet, wenn R^{a} sowie R^{b} jeweils eine C₁₋₄-Alkylgruppe darstellen,
(iii) R^{a} nicht die Bedeutungen eines Wasserstoff-, Fluor-, Chlor-, Brom- oder Iodatoms oder einer Difluor- oder Trifluormethylgruppe annimmt, wenn R^{c} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
wobei, soweit nichts anderes erwähnt wurde, alle in den vorstehend für R^{a}, R^{b} und R^{c} definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können, und
die Doppel- und Dreifachbindungen der in den für R^{a}, R^{b} und R^{c} vorstehend definierten Gruppen enthaltenen C₃₋₄-Alkenyl- oder C₃₋₄-Alkinylgruppen von in diesen Gruppen gegebenenfalls ebenfalls enthaltenen Heteroatomen isoliert sind,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine fünfte Ausführungsform der vorliegenden Erfindung besteht in den Verbindungen der obigen allgemeinen Formel (I), in denen
A, X, R¹, R² und R³ wie vorstehend unter der ersten oder zweiten Ausführungsform erwähnt definiert sind und
(a) D, E unabhängig voneinander jeweils eine Methingruppe oder das Stickstoffatom und
   G eine durch die Gruppe R^{a} substituierte Methingruppe,
   M eine durch die Gruppe R^{b} substituierte Methingruppe,
   Q eine durch die Gruppe R^{c} substituierte Methingruppe,
   wobei eine oder zwei der Gruppen G, M und Q jeweils auch ein Stickstoffatom bedeuten kann,
   oder
(b) D und E jeweils eine Methingruppe, wobei eine der Gruppen D und E auch ein Stickstoffatom bedeuten kann, und
   G, M und Q jeweils ein Stickstoffatom bedeuten,
wobei R^{a}, R^{b} und R^{c} unabhängig voneinander jeweils ein Wasserstoff- oder - Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₂-alkyl-, C₁₋₄-Alkoxy-, Amino-, C₁₋₄-Alkylamino-, Di-(C₁₋₄-alkyl)-amino-, Amino-C₁₋₂-alkyl-, C₁₋₃-Alkyl-amino-C₁₋₂-alkyl-, Di-(C₁₋₃-alkyl)-amino-C₁₋₂-alkyl-, Hydroxycarbonyl-, C₁₋₄-Alkyl-carbonyl-, Formyl-, C₁₋₄-Alkoxy-carbonyl-, Aminocarbonyl-, C₁₋₄-Alkyl-aminocarbonyl-, Di-(C₁₋₄-alkyl)-aminocarbonyl-, Formylamino-, C₁₋₄-Alkyl-carbonylamino-, Formyl-C₁₋₄-alkyl-amino- oder C₁₋₄-Alkyl-carbonyl-C₁₋₄-alkyl-aminogruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₄-Alkylgruppe darstellen,
(ii) R^{c} kein Wasserstoffatom bedeutet, wenn R^{a} sowie R^{b} jeweils eine C₁₋₄-Alkylgruppe darstellen,
(iii) R^{a} nicht die Bedeutungen eines Wasserstoff-, Fluor-, Chlor-, Brom- oder Iodatoms oder einer Difluor-oder Trifluormethylgruppe annimmt, wenn R^{c} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
wobei, soweit nichts anderes erwähnt wurde, alle in den vorstehend für R^{a}, R^{b} und R^{c} definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine sechste Ausführungsform der vorliegenden Erfindung besteht in den Verbindungen der obigen allgemeinen Formel (I), in denen
A, X, R¹, R² und R³ wie vorstehend unter der ersten oder zweiten Ausführungsform erwähnt definiert sind und
(a) D, E unabhängig voneinander jeweils eine Methingruppe oder das Stickstoffatom und
   G eine durch die Gruppe R^{a} substituierte Methingruppe,
   M eine durch die Gruppe R^{b} substituierte Methingruppe,
   Q eine durch die Gruppe R^{c} substituierte Methingruppe,
   wobei eine oder zwei der Gruppen G, M und Q jeweils auch ein Stickstoffatom bedeuten kann,
   oder
(b) D und E jeweils eine Methingruppe, wobei eine der Gruppen D und E auch ein Stickstoffatom bedeuten kann, und
   G, M und Q jeweils ein Stickstoffatom bedeuten,
wobei R^{a}, R^{b} und R^{c} unabhängig voneinander jeweils ein Wasserstoff- oder-Halogenatom, eine Methyl-, Difluormethyl-, Trifluormethyl-, Ethyl-, Vinyl-, Ethinyl-, Cyano-, Hydroxy-, Methoxy-, Difluormethoxy-, Trifluormethoxy-, Amino-, Methylamino- oder Dimethylaminogruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine Methyl-_ oder Ethylgruppe darstellen,
(ii) R^{c} kein Wasserstoffatom bedeutet, wenn R^{a} sowie R^{b} jeweils eine Methyl-_ oder Ethylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine Methyl-, Ethyl-, Vinyl- oder Ethinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine Methyl-, Ethyl-, Vinyl- oder Ethinylgruppe und R^{b} ein Chlor- oder Bromatom, eine Amino-, Methylamino- oder Hydroxygruppe darstellen,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine siebte Ausführungsform der vorliegenden Erfindung besteht in den Verbindungen der obigen allgemeinen Formel (I), in denen
A, X, D, E, G, M, Q und R¹ wie vorstehend unter der ersten, zweiten, dritten, vierten, fünften oder sechsten Ausführungsform erwähnt definiert sind und
R² das Wasserstoffatom oder
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Hydroxy-, Amino-, C₁₋₆-Alkylamino-, Di-(C₁₋₆-alkyl)-amino-, Hydroxycarbonyl-, C₁₋₆-Alkoxycarbonyl-, Aminocarbonyl-, Aminocarbonylamino-, C₁₋₆-Alkylamino-, 4-Morpholinylgruppe substituiert sein kann,
wobei die in den für R² vorstehend definierten Gruppen genannten oder als Substituenten enthaltenen Phenyl- und Pyridinylreste zusätzlich im Kohlenstoffgerüst durch Halogen, durch C₁₋₃-Alkyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkylamino-C₁₋₃-alkyl-, Di-(C₁₋₃-alkyl)-amino-C₁₋₃-alkyl-, C₁₋₃-Alkylcarbonylamino-, C₁₋₃-Alkylcarbonyl-C₁₋₃-alkylamino-, Aminocarbonyl-, C₁₋₃-Alkylaminocarbonyl- oder Di-(C₁₋₃-alkyl)-aminocarbonylgruppen mono-, di- oder trisubstituiert und die Substituenten gleich oder verschieden sein können,
R³ das Wasserstoffatom oder eine C₁₋₃-Alkylgruppe darstellen oder
R² und R³ zusammen mit dem eingeschlossenen Stickstoffatom einen Rest der allgemeinen Formel 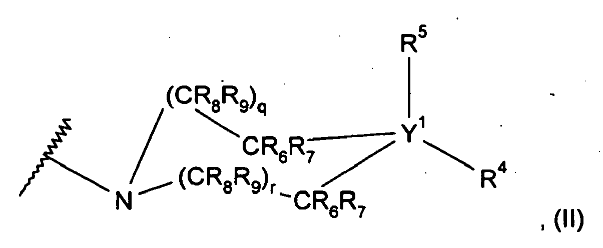 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl- oder Cyclo-C₃₋₇-alkenylgruppe, eine Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁-₄-alkyl)-aminocarbonyl-C₁₋₃-alkyl-, Aminocarbonylamino-C₁₋₃-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl-, Pyridinyl- oder Diazinylgruppe, die jeweils durch ein Halogen, durch eine C₁₋₃-Alkyl-, C₁₋₃-Alkoxy-, Hydroxy-, Amino-, C₁₋₄-Alkyl-amino-, Di-(C₁₋₄-alkyl)-amino- Amino-C₁₋₃-alkyl-, C₁₋₄-Alkyl-amino-C₁₋₃-alkyl-, Di-(C₁₋₄-alkyl)-amino-C₁₋₃-alkylgruppe substituiert sein können,
ein Heterocyclus ausgewählt aus einer 4- bis 7-gliedrigen Azacycloalkylgruppe, einer 6- bis 7-gliedrigen Oxaza-, S,S-Dioxothiaza- und Diazacycloalkylgruppe und einer 7- bis 9-gliedrigen Azabicycloalkylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methylengruppe durch ein oder zwei Fluoratome substituiert sein kann und
die vorstehend genannten mono- und bicyclischen Heterocyclen ein- oder zweifach durch Hydroxy-, C₁₋₃-Alkyl- oder Hydroxy-C₁₋₃-alkylgruppen oder einfach durch eine Benzyl-, Cyclo-C₃₋₆-alkyl-, Hydroxycyclo-C₃₋₆-alkyl-, Cyclo-C₃₋₆-alkyl-C₁₋₃-alkyl, C₁₋₄-Alkylcarbonyl-, C₁₋₄-Alkylcarbonyl-C₁₋₃-alkyl-, Hydroxy-, C₁₋₄-Alkoxy-, Amino-, C₁₋₄-Alkylamino-, Di-(C₁₋₄-alkyl)-amino-, C₁₋₃-Alkoxycarbonyl-, Hydroxycarbonyl-carbonyl-, C₁₋₃-Alkoxycarbonyl-carbonyl-, Hydroxycarbonyl-C₁₋₃-alkyl-, C₁₋₃-Alkoxycarbonyl-C₁₋₃-alkyl-, Hydroxycarbonyl-C₁₋₃-alkylcarbonyl-, C₁₋₃-Alkoxycarbonyl-C₁₋₃-alkylcarbonyl-, Aminosulfonyl-, C₁₋₄-Alkylaminosulfonyl-, Di-(C₁₋₄-alkyl)-aminosulfonyl-, C₁₋₃-Alkylsulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)-aminocarbonyl-C₁₋₃-alkyl-, Hydroxyaminocarbonl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppen substituiert sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom, einen C₁₋₃-Alkylrest oder, wenn Y¹ ein Stickstoffatom darstellt, auch ein freies Elektronenpaar,
R⁶ und R⁷, die gleich oder verschieden sein können, jeweils ein Wasserstoffatom, eine C₁₋₃-Alkylgruppe oder auch, wenn Y¹ ein Kohlenstoffatom darstellt, eine Amino-, C₁₋₃-Alkylamino- oder Di-(C₁₋₃-alkyl)-aminogruppe, wobei die beiden C₁₋₃-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
bedeuten,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, _ insbesondere deren physiologisch verträgliche Salze mit anorganischen, oder organischen Säuren oder Basen.

Eine achte Ausführungsform der vorliegenden Erfindung besteht in den Verbindungen der obigen allgemeinen Formel (I), in denen
A, X, D, E, G, M, Q und R¹ wie vorstehend unter der ersten, zweiten, dritten, vierten, fünften oder sechsten Ausführungsform erwähnt definiert sind und
R² das Wasserstoffatom oder
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Hydroxy-, Amino-, C₁₋₆-Alkylamino-, Di-(C₁₋₆-alkyl)-amino-, Hydroxycarbonyl-, C₁₋₆-Alkoxycarbonyl-, Aminocarbonyl-, Aminocarbonylamino-, C₁₋₆-Alkylamino-, 4-Morpholinylgruppe substituiert sein kann,
wobei die in den für R² vorstehend definierten Gruppen genannten oder als Substituenten enthaltenen Phenyl- und Pyridinylreste zusätzlich im Kohlenstoffgerüst durch Halogen, durch C₁₋₃-Alkyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkylamino-C₁₋₃-alkyl-, Di-(C₁₋₃-alkyl)-amino-C₁₋₃-alkyl-, C₁₋₃-Alkylcarbonylamino-, C₁₋₃-Alkylcarbonyl-C₁₋₃-alkylamino-, Aminocarbonyl-, C₁₋₃-Alkylaminocarbonyl- oder Di-(C₁₋₃-alkyl)-aminocarbonylgruppen mono-, di- oder trisubstituiert und die Substituenten gleich oder verschieden sein können,
R³ das Wasserstoffatom oder eine C₁₋₃-Alkylgruppe darstellen oder
R² und R³ zusammen mit dem eingeschlossenen Stickstoffatom einen Rest der allgemeinen Formel 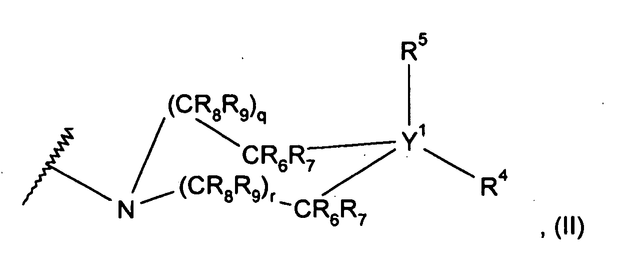 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, Cyclo-C₃₋₇-alkyl-, Cyclo-C₃₋₇-alkenyl-, Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₄-Alkyl-aminocarbonyl-, Di-(C₁₋₄-alkyl)-aminocarbonyl-, Aminocarbonyl-C₁₋₃-alkyl-, C₁-4-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl-, Pyridinyl- oder Diazinylgruppe, die jeweils durch ein Halogen, durch eine C₁₋₃-Alkyl-, C₁₋₃-Alkoxy-, Hydroxy-, Amino-, C₁₋₄-Alkyl-amino-, Di-(C₁₋₄-alkyl)-amino- Amino-C₁₋₃-alkyl-, C₁₋₄-Alkyl-amino-C₁₋₃-alkyl-, Di-(C₁₋₄-alkyl)-amino-C₁₋₃-alkylgruppe substituiert sein können,
ein Heterocyclus ausgewählt aus einer 4- bis 7-gliedrigen Azacycloalkylgruppe, einer 6- bis 7-gliedrigen Oxaza- und Diazacycloalkylgruppe und einer 7- bis 9-gliedrigen Azabicycloalkylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methylengruppe durch ein oder zwei Fluoratome substituiert sein kann und
die vorstehend genannten mono- und bicyclischen Heterocyclen ein- oder mehrfach, beispielsweise ein- bis dreifach, durch C₁₋₃-Alkylgruppen oder einfach durch eine Benzyl-, Cyclo-C₃₋₆-alkyl-, Cyclo-C₃₋₆-alkyl-C₁₋₃-alkyl, C₁₋₄-Alkylcarbonyl-, Hydroxy-, C₁₋₄-Alkoxy-, Amino-, C₁₋₄-Alkylamino-, Di-(C₁₋₄-alkyl)-amino-, Hydroxycarbonyl-, C₁₋₃-Alkoxycarbonyl-, Hydroxycarbonyl-carbonyl-, C₁₋₃-Alkoxycarbonyl-carbonyl-, Hydroxycarbonyl-C₁₋₃-alkyl-, C₁₋₃-Alkoxycarbonyl-C₁₋₃-alkyl- oder C₁₋₃-Alkylsulfonylgruppen substituiert sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom, einen C₁₋₃-Alkylrest oder, wenn Y¹ ein Stickstoffatom darstellt, auch ein freies Elektronenpaar,
R⁶ und R⁷, die gleich oder verschieden sein können, jeweils ein Wasserstoffatom, eine C₁₋₃-Alkylgruppe oder auch, wenn Y¹ ein Kohlenstoffatom darstellt, eine C₁₋₃-Alkylamino- oder Di-(C₁₋₃-alkyl)-aminogruppe, wobei die beiden C₁₋₃-Alkylgruppen unter Ausbildung eines Ringes miteinander verbunden sein können und
R⁸ und R⁹, die gleich oder verschieden sein können, jeweils ein Wasserstoffatom ' oder eine C₁₋₃-Alkylgruppe darstellen,
bedeuten,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine neunte Ausführungsform der vorliegenden Erfindung besteht in den Verbindungen der obigen allgemeinen Formel (I), in denen
A, X, D, E, G, M, Q und R¹ wie vorstehend unter der ersten, zweiten, dritten, vierten, fünften oder sechsten Ausführungsform erwähnt definiert sind und
R² das Wasserstoffatom oder
eine Phenylmethylgruppe oder eine C₂₋₇-Alkylgruppe, die in ω-Stellung durch eine Phenyl-, Amino-, C₁₋₆-Alkylamino-, Di-(C₁₋₆-alkyl)-aminogruppe substituiert sein kann,
wobei die vorstehend erwähnte Phenyl- und Phenylmethylgruppe zusätzlich an einem aromatischen Kohlenstoffatom durch Halogen, durch C₁₋₃-Alkyl-, C₁₋₃-Alkoxy-, Amino-C₁₋₃-alkyl-, C₁₋₃-Alkylamino-C₁₋₃-alkyl- oder Di-(C₁₋₃-alkyl)-amino-C₁₋₃-alkylgruppen mono- oder disubstituiert und die Substituenten gleich oder verschieden sein können,
R³ das Wasserstoffatom oder eine C₁₋₃-Alkylgruppe darstellen oder
R² und R³ zusammen mit dem eingeschlossenen Stickstoffatom einen Rest der allgemeinen Formel 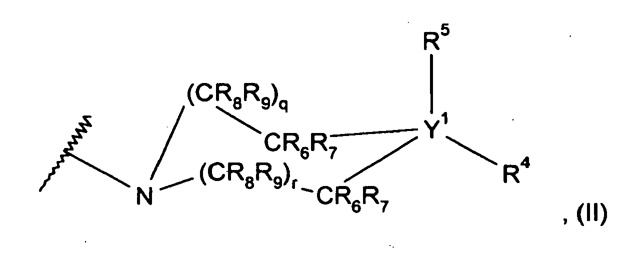 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁-₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte cyclo-C₃₋₇-alkyl- oder Cyclo-C₃₋₇-alkenylgruppe, eine Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl- oder Pyridylgruppe, die jeweils durch ein Halogen, durch eine C₁₋₃-Alkyl-, C₁₋₃-Alkoxy-, Amino-, C₁₋₄-Alkyl-amino-, Di-(C₁₋₄-alkyl)-aminogruppe substituiert sein kann,
ein Heterocyclus ausgewählt aus einer 6- bis 7-gliedrige Azacycloalkylgruppe, einer 6- bis 7-gliedrige S,S-Dioxothiaza- und Diazacycloalkylgruppe und einer 7-bis 9-gliedrige Azabicycloalkylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methylengruppe durch ein oder zwei Fluoratome substituiert sein kann und
die vorstehend genannten mono- und bicyclischen Heterocyclen ein- oder zweifach durch eine Hydroxy-, C₁₋₃-Alkyl- oder Hydroxy-C₁₋₃-alkylgruppe, durch eine Benzyl-, Cyclo-C₃₋₆-alkyl-, Hydroxy-C₃₋₆-cycloalkyl-, Cyclo-C₃₋₆-alkyl-C₁₋₃-alkyl, C₁₋₃-Alkylcarbonyl-C₁₋₃-alkyl-, Amino-, C₁₋₄-Alkylamino- oder Di-(C₁₋₄-alkyl)-amino-, Hydroxycarbonyl-carbonyl-, C₁₋₆-Alkoxycarbonyl-carbonyl-, Hydroxycarbonyl-C₁₋₃-alkylcarbonyl-, C₁₋₃-Alkoxycarbonyl-C₁₋₃-alkylcarbonyl-, Aminosulfonyl-, C₁₋₄-Alkylaminosulfonyl-, Di-(C₁₋₄-alkyl)-aminosulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)-aminocarbonyl-C₁₋₃-alkyl-, Hydroxyaminocärbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppen substituiert sein können,
oder auch, wenn Y¹ das Kohlenstoffatom darstellt die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom oder, wenn Y¹ ein Stickstoffatom darstellt, auch ein freies Elektronenpaar,
R⁶ und R⁷, die gleich oder verschieden sein können, jeweils ein Wasserstoffatom, eine C₁₋₃-Alkylgruppe oder auch, wenn Y¹ ein Kohlenstoffatom darstellt, eine C₁₋₃-Alkylamino- oder Di-(C₁₋₃-alkyl)-aminogruppe, wobei die beiden C₁₋₃-Alkylgruppen unter Ausbildung eines Ringes miteinander verbunden sein können und R⁸ und R⁹, die gleich oder verschieden sein können, jeweils ein Wasserstoffatom oder eine C₁₋₃-Alkylgruppe darstellen,
bedeuten, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine zehnte Ausführungsform der vorliegenden Erfindung besteht in den Verbindungen der obigen allgemeinen Formel (I), in denen
A, X, D, E, G, M, Q und R¹ wie vorstehend unter der ersten, zweiten, dritten, vierten, fünften oder sechsten Ausführungsform erwähnt definiert sind und
R² eine Phenylmethylgruppe oder eine C₂₋₇-Alkylgruppe, die in ω-Stellung durch eine Phenyl-, Amino-, C₁₋₆-Alkylamino-, Di-(C₁₋₆-alkyl)-aminogruppe substituiert sein kann,
wobei die vorstehend genannte Phenyl- und Phenylmethylgruppe an einem aromatischen Kohlenstoffatom durch eine Amino-C₁₋₃-alkyl-, C₁₋₃-Alkylamino-C₁₋₃-alkyl- oder Di-(C₁₋₃-alkyl)-amino-C₁₋₃-alkylgruppe substituiert sein kann,
R³ das Wasserstoffatom oder eine C₁₋₃-Alkylgruppe oder
R² und R³ zusammen mit dem eingeschlossenen Stickstoffatom einen Rest der allgemeinen Formel 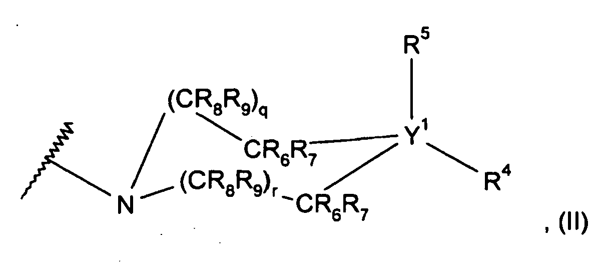 in der
R⁶ und R⁷ jeweils ein Wasserstoffatom oder eine Dimethylaminogruppe,
R⁸ und R⁹ jeweils das Wasserstoffatom und
(a) Y¹ das Kohlenstoffatom,
   q und r die Zahlen 0 oder 1,
   R⁴ das Wasserstoffatom,
   eine Phenyl-, Pyridinyl- oder Pyrimidinylgruppe, die jeweils durch ein Halogen, durch eine Amino-, Methylamino-, Dimethylamino-, Methyl- oder Methoxygruppe substituiert sein kann,
   eine Hydroxy-, 2-Diethylamino-ethyl-, Amino-, Methylamino-, Dimethylamino-, Diethylamino-, Pyrrolidin-1-yl-, 3-Hydroxy-pyrrolidin-1-yl-, 2-Hydroxycarbonyl-pyrrolidin-1-yl-, 2-Methoxycarbonyl-pyrrolidin-1-yl-, Piperidin-1-yl-, 4,4-Dimethylpiperidin-1-yl-, 4-Amino-4-methyl-piperidin-1-yl-, 2-Hydroxycarbonyl-piperidin-1-yl-, 2-Methoxycarbonyl-piperidin-1-yl-, 4-Hydroxymethyl-piperidin-1-yl-, 4-(1-Hydroxycyclopropyl)-piperidin-1-yl-, 4-Amino-piperidin-1-yl-, 4-Methylamino-piperidin-1-yl-, 4-Dimethylamino-piperidin-1-yl-, 4-Hydroxy-4-methyl-piperidin-1-yl-, 4-Hydroxy-4-ethyl-piperidin-1-yl-, 4-Hydroxy-4-trifluormethyl-piperidin-1-yl-, 4-Hydroxy-4-hydroxymethyl-piperidin-1-yl-, 3-Amino-piperidin-1-yl-, 3-Methylamino-piperidin-1-yl-, 3-Dimethylamino-piperidin-1-yl-, 3-Hydroxy-piperidin-1-yl-, 4-Hydroxypiperidin-1-yl-, 4-Hydroxycarbonylmethyl-piperidin-1-yl-, 4-Ethoxycarbonylmethyl-piperidin-1-yl-, Perhydro-azepin-1-yl-, Perhydro-1,4-diazepin-1-yl-, 4-Methyl-perhydro-1,4-diazepin-1-yl-, 1-Methyl-piperidin-4-yl-, Piperidin-4-yl-, 1-Ethylpiperidin-4-yl-, 1-(2-Hydroxyethyl)-piperidin-4-yl-, 1-Cyclopropyl-piperidin-4-yl-, 1-Cyclopropylmethyl-piperidin-4-yl-, 1-Methylsulfonyl-piperidin-4-yl-, 1-Ethylsulfonyl-piperidin-4-yl-, 1-Isopropylsulfonyl-piperidin-4-yl-, 1-Cyclopropylsulfonyl-piperidin-4-yl-, 4-Hydroxy-1-methylsulfonyl-piperidin-4-yl-, 1-Aminosulfonyl-piperidin-4-yl-, 1-(Methylaminosulfonyl)-piperidin-4-yl-, 1-(Dimethylaminosulfonyl)-piperidin-4-yl-, 1-Hydroxycarbonylmethyl-piperidin-4-yl-, 1-Ethoxycarbonylmethyl-piperidin-4-yl-, 1-(2-Hydroxycarbonylethyl)-piperidin-4-yl-, 1-(2-Ethoxycarbonylethyl)-piperidin-4-yl-, 1-(3-Hydroxycarbonyl-propionyl)-piperidin-4-yl-, 1-(3-Ethoxycarbonyl-propionyl)-piperidin-4-yl-, 1-(Hydroxycarbamoyl-methyl)-piperidin-4-yl-, 1-(Hydroxy-methylcarbamoyl-methyl)-piperidin-4-yl-, 1-(Methoxycarbamoyl-methyl)-piperidin-4-yl-, 1-Oxalyl-piperidin-4-yl-, 1-Ethoxyoxalyl-piperidin-4-yl-, Piperazin-1-yl-, 4-Methyl-piperazin-1 -yl-, 4-Cyclopropylmethyl-piperazin-1-yl-, 4-Ethyl-piperazin-1-yl-, 4-(2-Hydroxyethyl)-piperazin-1-yl-, 4-Cyclopropyl-piperazin-1-yl-, 4-Methylsulfonyl-piperazin-1-yl-, 4-Aminosulfonyl-piperazin-1-yl-, 4-(Methylaminosulfonyl)-piperazin-1-yl-, 4-(Dimethylaminosulfonyl)-piperazin-1-yl-, 4-Hydroxycarbonylmethyl-piperazin-1-yl-, 4-Ethoxycarbonylmethyl-piperazin-1-yl-, 4-(2-Hydroxycarbonylethyl)-piperazin-1-yl-, 4-(2-Ethoxycarbonylethyl)-piperazin-1-yl-, 4-(3-Hydroxycarbonyl-propionyl)-piperazin-1-yl-, 4-(3-Ethoxycarbonyl-propionyl)-piperazin-1-yl-, 4-(Hydroxycarbamoyl)-methyl-piperazin-1-yl-, 4-(Hydroxy-methyl-carbamoyl)-methyl-piperazin-1-yl-, 4-(Methoxycarbamoyl)-methyl-piperazin-1-yl-, 1,2-Dimethyl-piperazin-1-yl-, 3-Methyl-piperazin-1-yl-, 3,4,5-Trimethyl-piperazin-1-yl-, 3,5-Dimethyl-piperazin-1-yl-, 3,3,4-Trimethyl-piperazin-1-yl-, 3,3-Dimethyl-piperazin-1-yl-, 3,3,4,5,5-Pentamethyl-piperazin-1-yl-, 3,3,5,5-Tetramethyl-piperazin-1-yl-, 3,3,3-Trifluor-2-oxo-propyl)-piperazin-1-yl-, Morpholin-4-yl-, 1,1-Dioxo-1λ⁶-thiomorpholin-4-yl-, Tetrahydropyran-4-yl-, 4,4-Difluor-piperidin-1-yl-, 8-Methyl-8-aza-bicyclo[3.2.1]oct-3-yl-, 8-Aza-bicyclo[3.2.1]oct-3-yl-, Azetidin-1-yl-, 1-(Methoxycarbonylmethyl)-piperidin-4-yl-, 1-(Ethoxycarbonylmethyl)-piperidin-4-yl-, 4-(Ethoxycarbonylmethyl)-piperazin-1-yl-, 1-Hydroxycarbonylmethyl-piperidin-4-yl- oder 4-Hydroxycarbonylmethyl-piperazin-1-yl-gruppe, und
   R⁵ ein Wasserstoffatom, oder
(b) Y¹ ein Stickstoffatom,
   q und r die Zahlen 1 oder 2,
   R⁴ das Wasserstoffatom,
   eine Phenyl-, Pyridinyl- oder Pyrimidinylgruppe, die jeweils durch ein Halogen, durch eine Amino-, Methylamino-, Dimethylamino-, Methyl- oder Methoxygruppe substituiert sein kann,
   eine Methyl-, Ethyl-, Isopropyl-, Cyclopropyl-, Cyclopentyl-, Cyclohexyl-, Cycloheptyl-, 4-Hydroxycarbonylmethyl-cylohexyl-, 4-Ethoxycarbonylmethyl-cyclohexyl-, Cyclopropylmethyl-, 2-Diethylamino-propyl-, 1-Chinuclidin-3-yl-, Tetrahydropyran-4-yl-, 1-Piperidin-4-yl-, 1-Methyl-piperidin-4-yl-, 1-Ethyl-piperidin-4-yl-, 1-(2-Hydroxyethyl)-piperidin-4-yl-, 1-Methylsulfonyl-piperidin-4-yl-, 1-Aminosulfonyl-piperidin-4-yl-, 1-(Methylaminosulfonyl)-piperidin-4-yl-, 1-(Dimethylaminosulfonyl)-piperidin-4-yl-, 1-Hydroxycarbonylmethyl-piperidin-4-yl-, 1-Ethoxycarbonylmethyl-piperidin-4-yl-, 1-(2-Hydroxycarbonytethyl)-piperidin-4-yl-, 1-(2-Ethoxycarbonylethyl)-piperidin-4-yl-, 1-(3-Hydroxycarbonyl-propionyl)-piperidin-4-yl-, 1-(3-Ethoxycarbonyl-propionyl)-piperidin-4-yl-, 1-(Hydroxycarbamoyl-methyl)-piperidin-4-yl-, 1-(Hydroxy-methyl-carbamoyl-methyl)-piperidin-4-yl-, 1-(Methoxycarbamoyl-methyl)-piperidin-4-yl-, 1-Cyclopropyl-piperidin-4-yl-, 1-Cyclopropylmethyl-piperidin-4-yl-, 1-Hydroxycarbonylmethyl-piperidin-4-yl- oder 1-Ethoxycarbonylmethyl-piperidin-4-yl-gruppe und
   R⁵ ein freies Elektronenpaar darstellen,
bedeuten, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine elfte Ausführungsform der vorliegenden Erfindung besteht in den Verbindungen der obigen allgemeinen Formel (I), in denen
A, X, D, E, G, M, Q und R¹ wie vorstehend unter der ersten, zweiten, dritten, vierten, fünften oder sechsten Ausführungsform erwähnt definiert sind und
R² eine Phenylmethylgruppe, oder eine C₂₋₇-Alkylgruppe, die in ω-Stellung durch eine Phenyl-, Amino-, C₁₋₆-Alkylamino-, Di-(C₁₋₆-alkyl)-aminogruppe substituiert sein kann,
wobei die vorstehend genannte Phenyl- und Phenylmethylgruppe an einem aromatischen Kohlenstoffatom durch eine Amino-C₁₋₃-alkyl-, C₁₋₃-Alkylamino-C₁₋₃-alkyl- oder Di-(C₁₋₃-alkyl)-amino-C₁₋₃-alkylgruppe substituiert sein kann,
R³ das Wasserstoffatom oder eine C₁₋₃-Alkylgruppe oder
R² und R³ zusammen mit dem eingeschlossenen Stickstoffatom einen Rest der allgemeinen Formel 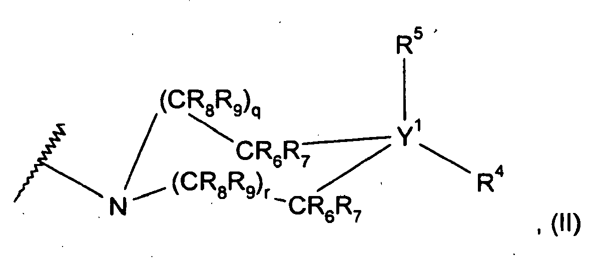 in der
R⁶ und R⁷ jeweils ein Wasserstoffatom oder eine Dimethylaminogruppe,
R⁸ und R⁹ jeweils das Wasserstoffatom und
(a) Y¹ das Kohlenstoffatom,
   q und r die Zahlen 0 oder 1,
   R⁴ das Wasserstoffatom,
   eine Phenyl-, Pyridinyl- oder Pyrimidinylgruppe, die jeweils durch ein Halogen, durch eine Amino-, Methylamino-, Dimethylamino-, Methyl- oder Methoxygruppe substituiert sein kann,
   eine Hydroxy-, 2-Diethylamino-ethyl-, Amino-, Methylamino-, Dimethylamino-, Diethylamino-, Pyrrolidin-1-yl-, Piperidin-1-yl-, 4-Amino-piperidin-1-yl-, 4-Methylamino-piperidin-1-yl-, 4-Dimethylamino-piperidin-1-yl-, 3-Amino-piperidin-1-yl-, 3-Methylamino-piperidin-1-yl-, 3-Dimethylamino-piperidin-1-yl-, Perhydro-azepin-1-yl-, Perhydro-1,4-diazepin-1-yl-, 4-Methyl-perhydro-1,4-diazepin-1-yl-, 1-Methyl-piperidin-4-yl-, Piperidin-4-yl-, 1-Ethylpiperidin-4-yl-, 1-Cyclopropyl-piperidin-4-yl-, 1-Cyclopropylmethyl-piperidin-4-yl-, Piperazin-1-yl-, 4-Methyl-piperazin-1-yl-, 4-Cyclopropylmethyl-piperazin-1-yl-, 4-Ethyl-piperazin-1-yl-, 4-Cyclopropyl-piperazin-1-yl-, 1,2-Dimethyl-piperazin-1-yl-, 3-Methyl-piperazin-1-yl-, 3,4,5-Trimethyl-piperazin-1-yl-, 3,5-Dimethyl-piperazin-1-yl-, 3,3,4-Trimethyl-piperazin-1-yl-, 3,3-Dimethyl-piperazin-1-yl-, 3,3,4,5,5-Pentamethyl-piperazin-1-yl-, 3,3,5,5-Tetramethyl-piperazin-1-yl-, Morpholin-4-yl-, 4,4-Difluor-piperidin-1-yl-, 8-Methyl-8-aza-bicyclo[3.2.1]oct-3-yl-, 8-Aza-bicyclo[3.2.1]oct-3-yl-, Azetidin-1-yl-, 1-(Methoxycarbonylmethyl)-piperidin-4-yl-, 1-(Ethoxycarbonylmethyl)-piperidin-4-yl-, 4-(Ethoxycarbonylmethyl)-piperazin-1-yl-, 1-Hydroxycarbonylmethyl-piperidin-4-yl- oder 4-Hydroxycarbonylmethyl-piperazin-1-ylgruppe, und
   R⁵ ein Wasserstoffatom, oder
(b) Y¹ ein Stickstoffatom,
   q und r die Zahlen 1 oder 2,
   R⁴ das Wasserstoffatom,
   eine Phenyl-, Pyridinyl- oder Pyrimidinylgruppe, die jeweils durch ein Halogen, durch eine Amino-, Methylamino-, Dimethylamino-, Methyl- oder Methoxygruppe substituiert sein kann,
   eine Methyl-, Ethyl-, Isopropyl-, Cyclopropyl-, Cyclopropylmethyl-, 2-Diethylamino-propyl-, 1-Chinuclidin-3-yl-, 1-Piperidin-4-yl-, 1-Methylpiperidin-4-yl-, 1-Ethyl-piperidin-4-yl-, 1-Cyclopropyl-piperidin-4-yl-, 1-Cyclopropylmethyl-piperidin-4-yl-, 1-Hydroxycarbonylmethyl-piperidin-4-yl- oder 1-Ethoxycarbonylmethyl-piperidin-4-ylgruppe und
   R⁵ ein freies Elektronenpaar darstellen,
bedeuten, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine zwölfte Ausführungsform der vorliegenden Erfindung besteht in den Verbindungen der obigen allgemeinen Formel (I), in denen
D, E, G, M, Q, R¹, R² und R³ wie vorstehend unter der ersten, zweiten, dritten, vierten, fünften, sechsten, siebten, achten, neunten oder zehnten Ausführungsform erwähnt definiert sind und
A und X jeweils ein Sauerstoffatom darstellen,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine dreizehnte Ausführungsform der vorliegenden Erfindung besteht in den Verbindungen der obigen allgemeinen Formel (I), in denen
A und X jeweils ein Sauerstoffatom darstellen,
R¹ eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-c]-chinolin-2-on-3-yl-, 1,3-Dihydro-naphth[1,2-*d*]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H*-thieno-[3,2-*d*]pyrimidin-2-on-3-yl- oder 3,4-Dihydro-1*H*-thieno[3,4-*d*]pyrimidin-2-on-3-ylgruppe bedeutet,
und R² und R³ wie vorstehend unter der ersten oder zweiten Ausführungsform erwähnt definiert sind,
wobei die vorstehend unter R¹ erwähnten Heterocyclen im Kohlenstoffgerüst zusätzlich durch eine Methoxygruppe monosubstituiert sein können,
und wobei alle in den unter R¹ definierten Resten erwähnten oder enthaltenen aromatischen und heteroaromatischen Reste und Molekülteile zusätzlich durch Halogenatome, durch Cyano- oder Hydroxygruppen mono-, di- oder trisubstituiert und die Substituenten gleich oder verschieden sein können,
und wobei bei dieser und allen voranstehend genannten Ausführungsformen jeweils den Verbindungen, in denen
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe und
R^{a}, R^{b} und R^{c} unabhängig voneinander jeweils ein Wasserstoff- oder Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₂-alkyl-, Hydroxy-C₃-alkenyl-, Hydroxy-C₃-alkinyl-, C₁₋₄-Alkoxy-, C₁₋₄-Alkoxy-C₁₋₂-alkyl-, Amino-, C₁₋₄-Alkyl-amino-, C₃₋₄-Alkenyl-amino-, C₃₋₄-Alkinyl-amino-, Di-(C₁₋₄-alkyl)-amino-, Di-(C₃₋₄-alkenyl)-amino-, Di-(C₃₋₄-alkinyl)-amino-, Amino-C₁₋₂-alkyl-, C₁₋₃-Alkyl-amino-C₁₋₂-alkyl-, Di-(C₁₋₃-alkyl)-amino-C₁₋₂-alkyl-, Amino-C₃-alkenyl-, C₁₋₃-Alkyl-amino-C₃-alkenyl-, Di-(C₁₋₃-alkyl)-amino-C₃-alkenyl-, Amino-C₃-alkinyl-, C₁₋₃-Alkyl-amino-C₃-alkinyl-, Di-(C₁-₃-alkyl)-amino-C₃-alkinyl-, Hydroxycarbonyl-, C₁₋₄-Alkyl-carbonyl-, Formyl-, C₁₋₄-Alkoxy-carbonyl-, Aminocarbonyl-, C₁₋₄-Alkyl-aminocarbonyl-, Di-(C₁₋₄-alkyl)-aminocarbonyl-, Formylamino-, C₁-₄-Alkyl-carbonylamino-, Formyl-C₁₋₄-alkyl-amino-, C₁₋₄-Alkylcarbonyl-C₁₋₄-alkyl-amino-, C₁₋₄-Alkyl-sulfonyl-, C₁₋₄-Alkyl-sulfinyl-, C₁₋₄-Alkyl-sulfonylamino-, C₁₋₄-Alkyl-sulfonyl-C₁-₄-alkylamino-, Aminosulfonyl-, C₁₋₄-Alkylaminosulfonyl- oder Di-(C₁₋₄-alkyl)-aminosulfonylgruppe darstellen,
wobei in den Definitionen der Reste R^{a}, R^{b} und R^{c} erwähnte oder enthaltene Alkyl-, Alkenyl- und Alkinylgruppen geradkettig oder verzweigt sein können, jede in diesen Resten enthaltene Methingruppe mit einem Fluoratom, jede Methylengruppe mit bis zu 2 Fluoratomen und jede Methylgruppe mit bis zu 3 Fluoratomen substituiert sein kann und
die Doppel- und Dreifachbindungen der in den für R^{a}, R^{b} und R^{c} vorstehend definierten Gruppen enthaltenen C₃₋₄-Alkenyl- oder C₃₋₄-Alkinylgruppen von in diesen Gruppen gegebenenfalls ebenfalls enthaltenen Heteroatomen isoliert sind,
eine herausragende Bedeutung zukommt,
den Verbindungen, in denen
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe und
R^{a}, R^{b} und R^{c} unabhängig voneinander jeweils ein Wasserstoff- oder Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₂-alkyl-, C₁₋₄-Alkoxy-, Amino-, C₁₋₄-Alkyl-amino-, Di-(C₁₋₄-alkyl)-amino-, Amino-C₁₋₂-alkyl-, C₁₋₃-Alkyl-amino-C₁₋₂-alkyl-, Di-(C₁₋₃-alkyl)-amino-C₁₋₂-alkyl-, Hydroxycarbonyl-, C₁₋₄-Alkyl-carbonyl-, Formyl-, C₁₋₄-Alkoxycarbonyl-, Aminocarbonyl-, C₁₋₄-Alkyl-aminocarbonyl-, Di-(C₁₋₄-alkyl)-aminocarbonyl-, Formylamino-, C₁₋₄-Alkyl-carbonylamino-, Formyl-C₁₋₄-alkyl-amino- oder C₁₋₄-Alkylcarbonyl-C₁₋₄-alkyl-aminogruppe darstellen,
wobei in den Definitionen der Reste R^{a}, R^{b} und R^{c} erwähnte oder enthaltene Alkyl-, Alkenyl- und Alkinylgruppen geradkettig oder verzweigt sein können und jede in diesen Resten enthaltene Methingruppe mit einem Fluoratom, jede Methylengruppe mit bis zu 2 Fluoratomen und jede Methylgruppe mit bis zu 3 Fluoratomen substituiert sein kann,
eine besonders herausragende Bedeutung zukommt, und
den Verbindungen, in denen
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe und
R^{a}, R^{b} und R^{c} unabhängig voneinander jeweils ein Wasserstoff- oder Halogenatom, eine Methyl-, Difluormethyl-, Trifluormethyl-, Ethyl-, Vinyl-, Ethinyl-, Cyano-, Hydroxy-, Methoxy-, Difluormethoxy-, Trifluormethoxy-, Amino-, Methylamino- oder Dimethylaminogruppe darstellen,
eine ganz besonders herausragende Bedeutung zukommt,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine vierzehnte Ausführungsform der vorliegenden Erfindung besteht in den Verbindungen der obigen allgemeinen Formel (I), in denen
A und X jeweils ein Sauerstoffatom bedeuten,
R¹ wie vorstehend unter der ersten Ausführungsform definiert ist,
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe und
R^{a}, R^{b} und R^{c} unabhängig voneinander jeweils ein Wasserstoff- oder Halogenatom, eine Methyl-, Difluormethyl-, Trifluormethyl-, Ethyl-, Vinyl-, Ethinyl-, Cyano-, Hydroxy-, Methoxy-, Difluormethoxy-, Trifluormethoxy-, Amino-, Methylamino- oder Dimethylaminogruppe darstellen,
und wobei bei dieser und allen voranstehend genannten Ausführungsformen jeweils den Verbindungen, in denen
R² und R³ wie vorstehend unter der zehnten oder elften Ausführungsform definiert sind, eine herausragende Bedeutung zukommt,
den Verbindungen, in denen R² und R³ wie vorstehend unter der zwölften Ausführungsform definiert sind, eine besonders herausragende Bedeutung zukommt,
und den Verbindungen, in denen R² und R³ wie vorstehend unter der dreizehnten Ausführungsform definiert sind, eine ganz besonders herausragende Bedeutung zukommt,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine fünfzehnte Ausführungsform der vorliegenden Erfindung besteht in den Verbindungen der obigen allgemeinen Formel (I), in denen
A und X jeweils ein Sauerstoffatom,
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe,
R^{a}, R^{b} und R^{c} unabhängig voneinander jeweils ein Wasserstoff- oder Halogenatom, eine C₁₋₃-Alkyl-, Trifluormethyl-, Cyano-, Hydroxy-, Methoxy-, Trifluormethoxy-, Amino-, Methylamino- oder Dimethylaminogruppe,
R¹ eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-c]chinolin-2-on-3-yl-, 1,3-Dihydro-naphth[1,2-*d*]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H-*thieno[3,2-*d*]pyrimidin-2-on-3-yl- oder 3,4-Dihydro-1*H*-thieno[3,4-*d*]pyrimidin-2-on-3-ylgruppe, die an einem ungesättigten Kohlenstoffatom des aromatischen oder heteroaromatischen Teils durch Halogenatome, durch Cyano- oder Hydroxygruppen mono-, di- oder trisubstituiert und die Substituenten gleich oder verschieden sein können, vorzugsweise jedoch unsubstituiert sind,
R² und R³ zusammen mit dem eingeschlossenen Stickstoffatom einen Rest der allgemeinen Formel 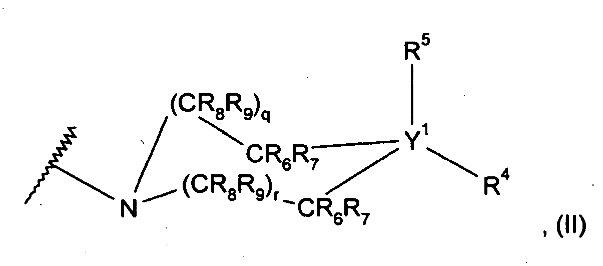 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, wobei die Summe aus q und r 1, 2 oder 3 beträgt,
q und r, wenn Y¹ das Stickstoffatom darstellt, die Zahlen 1 oder 2, wobei die Summe aus q und r 2 oder 3 beträgt,
R⁴ das Wasserstoffatom, eine Amino-, C₁₋₄-Alkylamino-, Di-(C₁₋₄-alkyl)-alkylamino-, C₁₋₆-Alky!-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl-, Amino-C₂₋₇-alkyl-, C₁₋₄-Alkylamino-C₂₋₇-alkyl-, Di-(C₁₋₄-alkyl-amino)-C₂₋₇-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl- oder Pyridylgruppe, die jeweils durch ein Halogenatom, durch eine C₁₋₃-Alkyl-, C₁₋₃-Alkoxy-, Amino-, C₁₋₄-Alkyl-amino-, oder Di-(C₁₋₄-alkyl)-aminogruppe substituiert sein kann,
ein Heterocyclus ausgewählt aus einer 5- bis 7-gliedrigen Azacycloalkyl- oder S,S-Dioxothiazagruppe und einer 6- bis 7-gliedrigen Diazacycloalkylgruppe,
wobei die vorstehend genannten Heterocyclen über ein Stickstoff- oder ein Kohlenstoffatom an Y¹ in Formel (II) gebunden sind und
durch eine oder zwei Hydroxy-, C₁₋₃-Alkyl- oder Hydroxy-C₁₋₃-alkylgruppen oder durch eine Cyclo-C₃₋₆-alkyl-, Hydroxy-C₃₋₆-cycloalkyl-, Cyclo-C₃₋₆-alkyl-C₁₋₃-alkyl, C₁₋₃-Alkylcarbonyl-C₁-₃-alkyl-, Amino-, C₁₋₄-Alkylamino-, Di-(C₁₋₄-alkyl)-amino-, Hydroxycarbonyl-carbonyl-, C₁₋₃-Alkoxycarbonyl-carbonyl-, Hydroxycarbonyl-C₁₋₃-alkylcarbonyl-, C₁₋₃-Alkoxycarbonyl-C₁₋₃-alkylcarbonyl-, Aminosulfonyl-, C₁₋₄-Alkylaminosulfonyl-, Di-(C₁₋₄-alkyl)-aminosulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)-aminocarbonyl-C₁₋₃-alkyl-, Hydroxyaminocarbonyl-C₁₋₃-alkyl-, C₁₋₃-Alkoxyaminocarbonyl-C₁-₃-alkyl- oder Hydroxy-(C₁₋₃-alkyl)-aminocarbonyl-C₁₋₃-alkylgruppen substituiert sein können,
R⁵ ein Wasserstoffatom oder, wenn Y¹ ein Stickstoffatom darstellt, auch ein freies Elektronenpaar und
R⁶, R⁷ R⁸ und R⁹, die gleich oder verschieden sein können, jeweils ein Wasserstoffatom oder eine C₁₋₃-Alkylgruppe darstellen,
bedeuten, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine sechzehnte Ausführungsform der vorliegenden Erfindung besteht in den Verbindungen der obigen allgemeinen Formel (I), in denen
A und X jeweils ein Sauerstoffatom,
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe,
R^{a}, R^{b} und R^{c} unabhängig voneinander jeweils ein Wasserstoff- oder Halogenatom, eine C₁₋₃-Alkyl-, Trifluormethyl-, Cyano-, Hydroxy-, Methoxy-, Trifluormethoxy-, Amino-, Methylamino- oder Dimethylaminogruppe,
R¹ eine 1,3,4,5-Tetrahydro-1,3-benzdiazepin-2-on-3-yl-, 3,4-Dihydro-1*H*-chinazolin-2- , on-3-yl-, 5-Phenyl-2,4-dihydro-1,2,4-triazol-3-on-2-yl-, 1,3-Dihydro-imidazo[4,5-c]chinolin-2-on-3-yl-, 1,3-Dihydro-naphth[1,2-*d*]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H-*thieno[3,2-*d*]pyrimidin-2-on-3-yl- oder 3,4-Dihydro-1*H*-thieno[3,4-*d*]pyrimidin-2-on-3-ylgruppe, die an einem ungesättigten Kohlenstoffatom des aromatischen oder heteroaromatischen Teils durch Halogenatome, durch Cyano- oder Hydroxygruppen mono-, di- öder trisubstituiert und die Substituenten gleich oder verschieden sein können, vorzugsweise jedoch unsubstituiert sind,
R² und R³ zusammen mit dem eingeschlossenen Stickstoffatom einen Rest der allgemeinen Formel 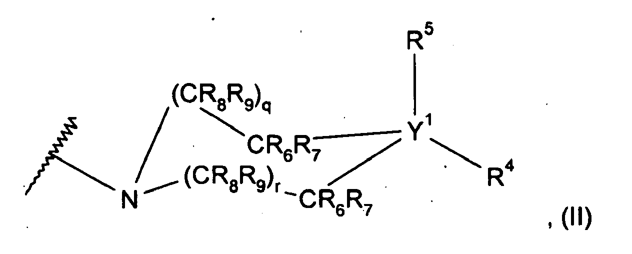 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, wobei die Summe aus q und r 1, 2 oder 3 beträgt,
q und r, wenn Y¹ das Stickstoffatom darstellt, die Zahlen 1 oder 2, wobei die Summe aus q und r 2 oder 3 beträgt,
R⁴ das Wasserstoffatom, eine Amino-, C₁₋₄-Alkyl-amino-, Di-(C₁₋₄-alkyl)-alkylamino-, C₁₋₆-Alkyl-, Cyclo-C₃₋₇-alkyl-, Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl- oder Pyridylgruppe, die jeweils durch ein Halogenatom, durch eine C₁₋₃-Alkyl-, C₁₋₃-Alkoxy-, Amino-, C₁₋₄-Alkyl-amino-, oder Di-(C₁₋₄-alkyl)-aminogruppe substituiert sein kann,
ein Heterocyclus ausgewählt aus einer 5- bis 7-gliedrigen Azacycloalkylgruppe und einer 6- bis 7-gliedrigen Diazacycloalkylgruppe,
wobei die vorstehend genannten Heterocyclen über ein Stickstoff- oder ein Kohlenstoffatom an Y¹ in Formel (II) gebunden sind und
durch eine C₁₋₃-Alkyl-, Cyclo-C₃₋₆-alkyl-, Cyclo-C₃₋₆-alkyl-C₁₋₃-alkyl, Amino-, C₁₋₄-Alkylamino- oder Di-(C₁₋₄-alkyl)-aminogruppen substituiert sein können,
R⁵ ein Wasserstoffatom oder, wenn Y¹ ein Stickstoffatom darstellt, auch ein freies Elektronenpaar und
R⁶, R⁷, R⁸ und R⁹, die gleich oder verschieden sein können, jeweils ein Wasserstoffatom oder eine C₁₋₃-Alkylgruppe darstellen,
bedeuten, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Als ganz besonders bevorzugte Verbindungen der obigen allgemeinen Formel (I) seien beispielsweise folgende Verbindungen genannt:

| | Struktur | Name |
|---|---|---|
| (1) | 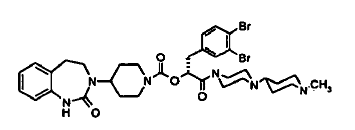 | 4-(2-Oxo-1,2,4,5-tetrahydro-1,3-benzdiazepin-3-yl)-piperidin-1-carbonsäure-(R)-1-(3,4-dibrom-benzyl)-2-[4-(1-methyl-piperidin-4-yl)-piperazin-1-yl]-2-oxo-ethylester |
| (2) | 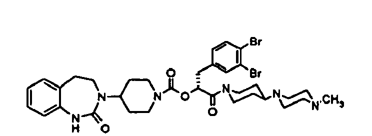 | 4-(2-Oxo-1,2,4,5-tetrahydro-1,3-benzdiazepin-3-yl)-piperidin-1-carbonsäure -(R)-1-(3,4-dibrom-benzyl)-2-[4-(4-methyl-piperazin-1-yl)-piperidin-1-yl]-2-oxo-ethylester |
| (3) | 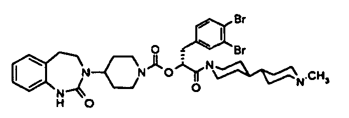 | 4-(2-Oxo-1,2,4,5-tetrahydro-1,3-benzdiazepin-3-yl)-piperidin-1-carbonsäure-(R)-1-(3,4-dibrom-benzyl)-2-(1'-methyl-4,4'-bipiperidinyl-1-yl)-2-oxo-ethylester |
| (4) | 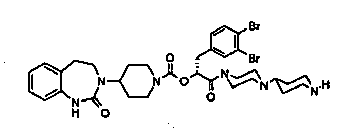 | 4-(2-Oxo-1,2,4, 5-tetrahydro-1,3-benzdiazepin-3-yl)-piperidin-1-carbonsäure-(R)-1-(3,4-dibrom-benzyl)-2-oxo-2-(4-piperidin-4-yl-piperazin-1-yl)-ethylester |
| (5) | 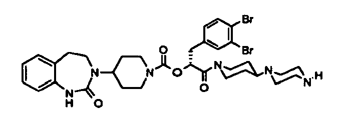 | 4-(2-Oxo-1,2,4, 5-tetrahydro-1, 3-benzdiazepin-3-yl)-piperidin-1-carbonsäure-(R)-1-(3,4-dibrom-benzyl)-2-oxo-2-(4-piperazin-1-yl-piperidin-1-yl)-ethylester |
| (6) | 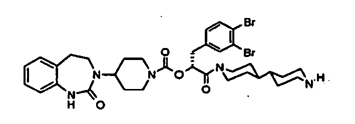 | 4-(2-Oxo-1,2,4, 5-tetrahydro-1,3-benzdiazepin⁼ 3-yl)-piperidin-1-carbonsäure-(R)-2-4,4'-bipiperidinyl-1-yl-1-(3,4-dibrom-benzyl)-2-oxo-ethylester |
| (7) | 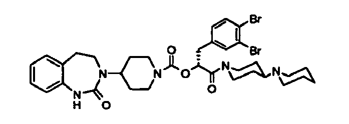 | 4-(2-Oxo-1,2,4, 5-tetrahydro-1,3-benzdiazepin-3-yl)-piperidin-l-carbonsäure-(R)-2-1,4'-bipiperidinyl-1'-yl-1-(3,4-dibrom-benzyl)-2-oxo-ethylester |
| (8) | 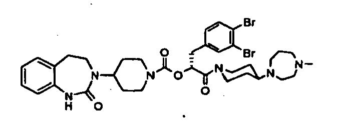 | 4-(2-Oxo-1,2,4,5-tetrahydro-1,3-benzdiazepin-3-yl)-piperidin-1-carbonsäure-(R)-1-(3,4-dibrom-benzyl)-2-[4-(4-methyl-perhydro-1,4-diazepin-1-yl)-piperidin-1-yl]-2-oxo-ethylester |
| (9) | 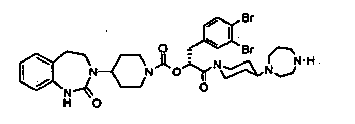 | 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-perhydro-1,4-diazepin-1-yl-piperidin-1-yl)-ethylester , |
| (10) | 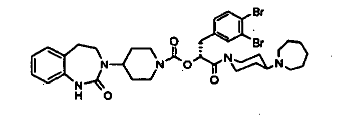 | 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-perhydro-azepin-1-yl-piperidin-1-yl)-ethylester |
| (11) | 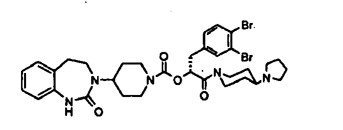 | 4-(2-Oxo-1,2,4,5-tetrahydro-1, 3-benzdiazepin-3-yl)-piperidin-l-carbonsäure-(R)-1-(3,4-dibrom-benzyl)-2-oxo-2-(4-pyrrolidin-1-yl-piperidin-1-yl)-ethylester |
| (12) | 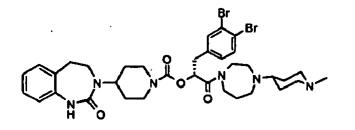 | 4-(2-Oxo-1,2,4,5-tetrahydro-1,3-benzdiazepin-3-yl)-piperidin-1-carbonsäure-(R)-1-(3,4-dibrom-benzyl)-2-[4-(1-methyl-piperidin-4-yl)-perhydro-1,4-diazepin-1-yl]-2-oxo-ethylester |
| (13) | 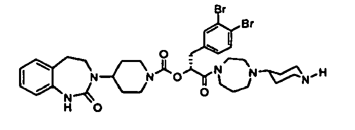 | 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-piperidin-4-yl-perhydro-l ,4-diazepin-1-yl)-ethylester |
| (14) | 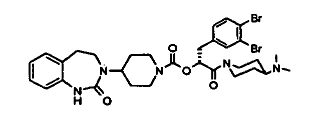 | 4-(2-Oxo-1,2,4, 5-tetrahydro-1,3-benzdiazepin-3-yl)-piperidin-1-carbonsäure-(R)-1-(3,4-dibrom-benzyl)-2-(4-dimethylamino-piperidin-1-yl)-2-oxo-ethylester |
| (15) | 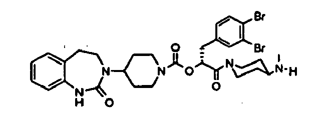 | 4-(2-Oxo-1,2,4, 5-tetrahydro-1,3-benzdiazepin-3-yl)-piperidin-1-carbonsäure-(R)-1-(3,4-dibrom-benzyl)-2-(4-methylamino-piperidin-1-yl)-2=oxo-ethylester . |
| (16) | 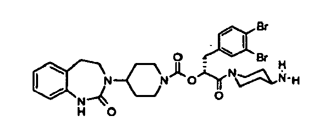 | 4-(2-Oxo-1,2,4, 5-tetrahydro-1,3-benzdiazepin-3-yl)-piperidin-1-carbonsäure-(R)-1-(3,4-dibrom-benzyl)-2-(4-amino-piperidin-1-yl)-2-oxo-ethylester |
| (17) | 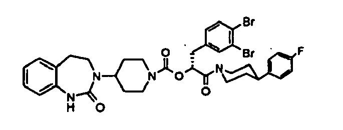 | 4-(2-Oxo-1,2,4,5-tetrahydro-1,3-benzdiazepin-3-yl)-piperidin-1-carbonsäure-(R)-1-(3,4-dibrom-benzyl)-2-[4-(4-fluor+phenyl)-piperidin-1-yl]-2-oxo-ethylester |
| (18) | 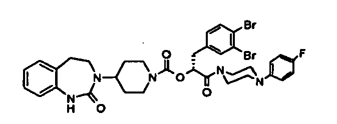 | 4-(2-Oxo-1,2,4,5-tetrahydro-1,3-benzdiazepin-3-yl)-piperidin-1-carbonsäure-(R)-1-(3,4-dibrom-benzyl)-2-[4-(4-fluor-phenyl)-piperazin-1-yl]-2-oxo-ethylester |
| (19) | 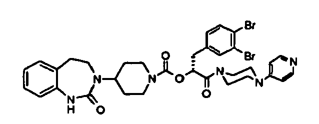 | 4-(2-Oxo-1,2,4,5-tetrahydro-1, 3-benzdiazepin-3-yl)-piperidin-1-carbonsäure-(R)-1-(3,4-dibrom-benzyl)-2-oxo-2-(4-pyridin-4-yl-piperazin-1-yl)-ethylester |
| (20) | 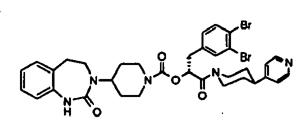 | 4-(2-Oxo-1,2,4,5-tetrahydro-1,3-benzdiazepin-3-yl)-piperidin-1-carbonsäure-(R)-1-(3,4-dibrom-benzyl)-2-oxo-2-(3,4,5,6-tetrahydro-2H-4,4'-bipyridinyl-1-yl)-ethylester |
| (21) | 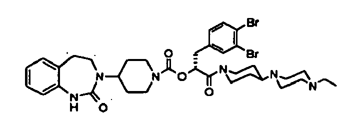 | 4-(2-Oxo-1,2,4,5-tetrahydro-1,3-benzdiazepin-3-yl)-piperidin-1-carbonsäure-(R)-1-(3,4-dibrom-benzyl)-2-[4-(4-ethyl-piperazin-1-yl)-piperidin-1-yl]-2-oxo-ethylester |
| (22) | 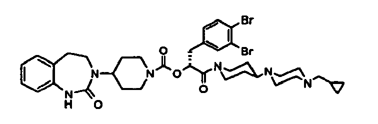 | 4-(2-Oxo-1,2,4,5-tetrahydro-1,3-benzdiazepin-3-yl)-piperidin-1-carbonsäure-(R)-2-[4-(4-cyclopropylmethyl-piperazin-1-yl)-piperidin-1-yl]-1-(3,4-dibrom-benzyl)-2-oxo-ethylester |
| (23) | 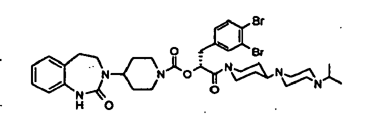 | 4-(2-Oxo-1,2,4,5-tetrahydro-1,3-benzdiazepin-3-yl)-piperidin-1-carbonsäure-(R)-1-(3,4-dibrom-benzyl)-2-[4-(4-isopropyl-piperazin-1-yl)-piperidin-1-yl]-2-oxo-ethylester |
| (24) | 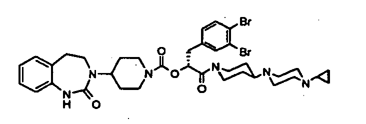 | 4-(2-Oxo-1,2,4, 5-tetrahydro-1,3-benzdiazepin-3-yl)-piperidin-1-carbonsäure-(R)-2-[4-(4-cyclopropyl-piperazin-1-yl)-piperid in-1-yl]-1-(3,4-dibrom-benzyl)-2-oxo-ethylester |
| (25) | 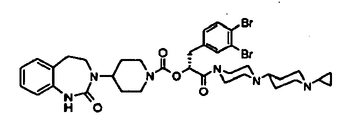 | 4-(2-Oxo-1,2,4,5-tetrahydro-1,3-benzdiazepin-3-yl)-piperidin-1-carbonsäure-(R)-2-[4-(1-cyclopropyl-piperidin-4-yl)-piperazin-1-yl]-1-(3,4-dibrom-benzyl)-2-oxo-ethylester |
| (26) | 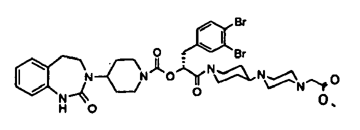 | 4-(2-Oxo-1,2,4,5-tetrahydro-1,3-benzdiazepin-3-yl)-piperidin-1-carbonsäure-(R)-1-(3,4-dibrom-benzyl)-2-[4-(4-methoxycarbonylmethyl-piperazin-1-yl)-piperidin-1-yl]-2-oxo-ethylester |
| (27) | 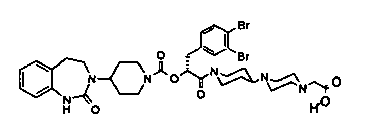 | 4-(2-Oxo-1,2,4,5-tetrahydro-1,3-benzdiazepin-3-yl)-piperidin-1-carbonsäure-(R)-2-[4-(4-carboxymethyl-piperazin-1-yl)-piperidin-1-yl]-1-(3,4-dibrom-benzyl)-2-oxo-ethylester |
| (28) | 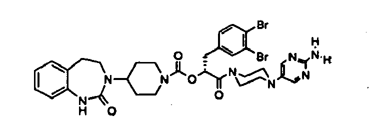 | 4-(2-Oxo-1,2,4,5-tetrahydro-1,3-benzdiazepin-3-yl)- piperidin-1-carbonsäure-(R)-2-[4-(2-amino-pyrimidin-5-yl)-piperazin-1-yl]-1-(3,4-dibrom-benzyl)-2-oxo-ethyl ester |
| (29) | 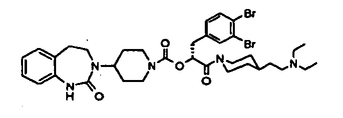 | 4-(2-Oxo-1,2,4,5-tetrahydro-1,3-benzdiazepin-3-yl)-piperidin-1-carbonsäure-(R)-1-(3,4-dibrom-benzyl)-2-[4-(2-diethylamino-ethyl)-piperidin-1-yl]-2-oxo-ethylester |
| (30) | 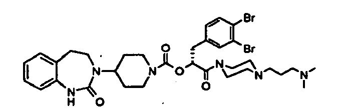 | 4-(2-Oxo-1,2,4,5-tetrahydro-1,3-benzdiazepin-3-yl)-piperidin-1-carbonsäure-(R)-1-(3,4-dibrom-benzyl)-2-[4-(3-dimethylamino-propyl)-piperazin-1-yl]-2-oxo-ethylester |
| (31) | 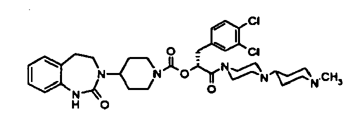 | 4-(2-Oxo-1,2,4, 5-tetrahydro-1,3-benzdiazepin-3-yl)-piperidin-1-carbonsäure-(R)-1-(3,4-dichlor-benzyl)-2-[4-(1-methyl-piperidin-4-yl)-piperazin-1-yl]-2-oxo-ethylester |
| (32) | 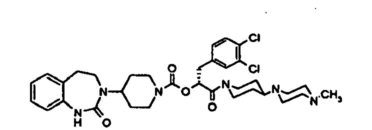 | 4-(2-Oxo-1,2,4,5-tetrahydro-1,3-benzdiazepin-3-yl)-piperidin-1-carbonsäure -(R)-1-(3,4-dichlor-benzyl)-2-[4-(4-methyl-piperazin-1-yl)-piperidin-1-yl]-2-oxo-ethylester |
| (33) | 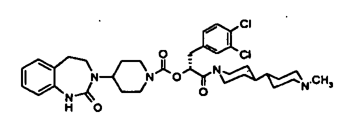 | 4-(2-Oxo-1,2,4,5-tetrahydro-1,3-benzdiazepin-3-yl)-piperidin-1-carbonsäure-(R)-1-(3,4-dichlor-benzyl)-2-(1'-methyl-4,4'-bipiperidinyl-1-yl)-2-oxo-ethylester |
| (34) | 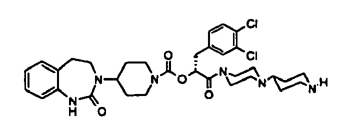 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-piperidin-4-yl-piperazin-1-yl)-ethylester |
| (35) | 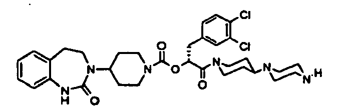 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-piperazin-1-yl-piperidin-1-yl)-ethylester |
| (36) | 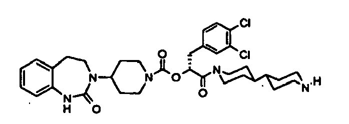 | 4-(2-Oxo-1,2,4, 5-tetrahydro-1,3-benzdiazepin-3-yl)-piperidin-1-carbonsäure-(R)-2-4,4'-bipiperidinyl-1-yl-1-(3,4-dichlor-benzyl)-2-oxo-ethylester |
| (37) | 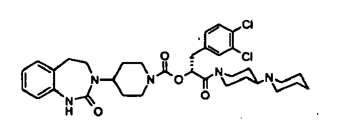 | 4-(2-Oxo-1,2,4,5-tetrahydro-1,3-benzdiazepin-3-yl)-piperidin-1-carbonsäure-(R)-2-1,4'-bipiperidinyl-l'-yl-1-(3,4-dichlor-benzyl)-2-oxo-ethylester |
| (38) | 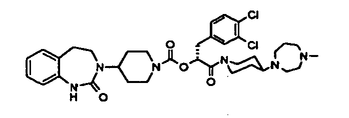 | 4-(2-Oxo-1,2,4, 5-tetrahydro-1,3-benzdiazepin-3-yl)-piperidin-1-carbonsäure-(R)-1-(3,4-dichlor-benzyl)-2-[4-(4-methyl-perhydro-1,4-diazepin-1-yl)-piperidin-1-yl]-2-oxo-ethylester |
| (39) | 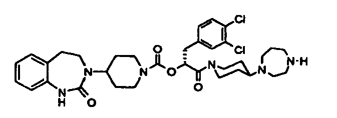 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-perhydro-1,4-diazepin-1-yl-piperidin-1-yl)-ethylester |
| (40) | 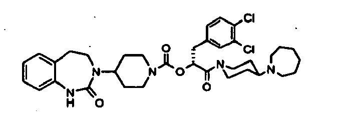 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-perhydro-azepin-1-yl-piperidin-1-yl)-ethylester |
| (41) | 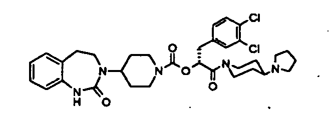 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-pyrrolidin-1-yl-piperidin-1-yl)-ethylester |
| (42) | 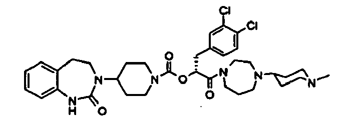 | 4-(2-Oxo-1,2,4, 5-tetrahydro-1,3-benzdiazepin-3-yl)-piperidin-1-carbonsäure-(R)-1-(3,4-dichlor-benzyl)-2-[4-(1-methyl-piperidin-4-yl)-perhydro-1,4-diazepin-1-yl]-2-oxo-ethylester |
| (43) | 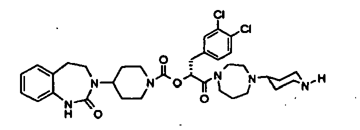 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-piperidin-4-yl-perhydro-1,4-diazepin-1-yl)-ethylester |
| (44) | 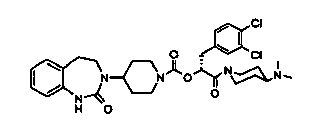 | 4-(2-Oxo-1,2,4,5-tetrahydro-1,3-benzdiazepin-3-yl)-piperidin-1-carbonsäure-(R)-1-(3,4-dichlor-benzyl)-2-(4-dimethylamino-piperidin-1-yl)-2-oxo-ethylester |
| (45) | 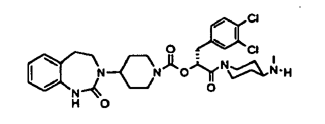 | 4-(2-Oxo-1,2,4,5-tetrahydro-1,3-benzdiazepin-3-yl)-piperidin-1-carbonsäure-(R)-1-(3,4-dichlor-benzyl)-2-(4-methylamino-piperidin-1-yl)-2-oxo-ethylester |
| (46) | 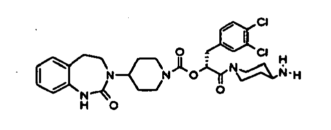 | 4-(2-Oxo-1,2,4,5-tetrahydro-1,3-benzdiazepin-3-yl)-piperidin-1-carbonsäure-(R)-1-(3,4-dichlor-benzyl)-2-(4-amino-piperidin-1-yl)-2-oxo-ethylester |
| (47) | 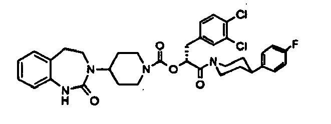 | 4-(2-Oxo-1,2,4,5-tetrahydro-1,3-benzdiazepin-3-yl)-piperidin-1-carbonsäure-(R)-1-(3,4-dichlor-benzyl)-2-[4-(4-fluor-phenyl)-piperidin-1-yl]-2-oxo-ethylester |
| (48) | 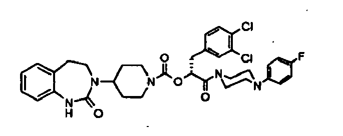 | 4-(2-0xo-1,2,4,5-tetrahydro-l ,3-benzdiazepin-3-yl)-piperidin-1-carbonsäure-(R)-1-(3,4-dichlor-benzyl)-2-[4-(4-fluor-phenyl)-piperazin-1-yl]-2-oxo-ethylester |
| (49) | 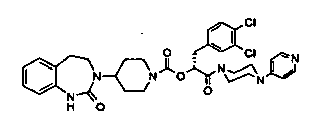 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-pyridin-4-yl-piperazin-1-yl)-ethylester |
| (50) | 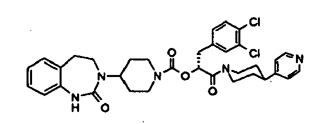 | 4-(2-Oxo-1,2,4,5-tetrahydro-1,3-benzdiazepin-3-yl)-piperidin-1-carbonsäure-(R)-1-(3,4-dichlor-benzyl)-2-oxo-2-(3,4,5,6-tetrahydro-2*H*-4,4'-bipyridinyl-1-yl)-ethylester |
| (51) | 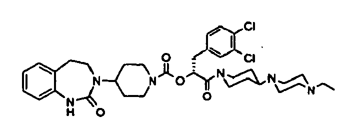 | 4-(2-Oxo-1,2,4,5-tetrahydro-1,3-benzdiazepin-3-yl)-piperidin-1-carbonsäure-(R)-1-(3,4-dichlor-benzyl)-2-[4-(4-ethyl-piperazin-1-yl)-piperidin-1-yl]-2-oxo-ethylester |
| (52) | 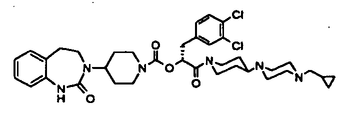 | 4-(2-Oxo-1,2,4,5-tetrahydro-1,3-benzdiazepin-3-yl)-piperidin-1-carbonsäure-(R)-2-[4-(4-cyclopropylmethyl-piperazin-1-yl)-piperidin-1-yl]-1-(3,4-dichlor-benzyl)-2-oxo-ethylester |
| (53) | 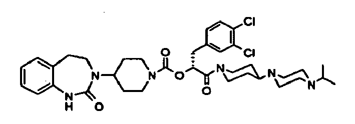 | 4-(2-Oxo-1,2,4,5-tetrahydro-1,3-benzdiazepin-3-yl)-piperidin-1-carbonsäure-(R)-1-(3,4-dichlor-benzyl)-2-[4-(4-isopropyl-piperazin-1-yl)-piperidin-1-yl]-2-oxo-ethylester |
| (54) | 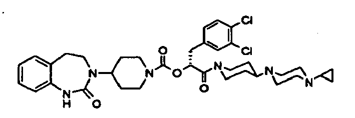 | 4-(2-Oxo-1,2,4,5-tetrahydro-1,3-benzdiazepin-3-yl)-piperidin-1-carbonsäure-(R)-2-[4-(4-cyclopropyl-piperazin-1-yl)-piperidin-1-yl]-1-(3,4-dichlor-benzyl)-2-oxo-ethylester |
| (55) | 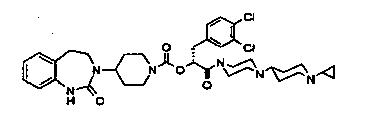 | 4-(2-Oxo-1,2,4,5-tetrahydro-1,3-benzdiazepin-3-yl)-piperidin-1-carbonsäure-(R)-2-[4-(1-cyclopropyl-piperidin-4-yl)-piperazin-1-yl]-1-(3,4-dichlor-benzyl)-2-oxo-ethylester |
| (56) | 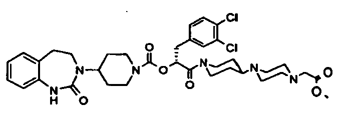 | 4-(2-Oxo-1,2,4,5-tetrahydro-1,3-benzdiazepin-3-yl)-piperidin-1-carbonsäure-(R)-1-(3,4-dichlor-benzyl)-2-[4-(4-methoxycarbonylmethyl-piperazin-1-yl)-piperidin-1-yl]-2-oxo-ethylester |
| (57) | 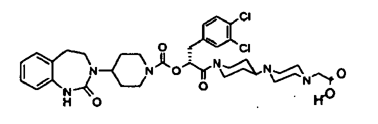 | 4-(2-Oxo-1,2,4,5-tetrahydro-1,3-benzdiazepin-3-yl)-piperidin-1-carbonsäure-(R)-2-[4-(4-carboxymethyl-piperazin-1-yl)-piperidin-1-yl]-1-(3,4-dichlor-benzyl)-2-oxo-ethylester |
| (58) | 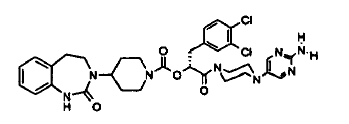 | 4-(2-Oxo-1,2,4,5-tetrahydro-1,3-benzdiazepin-3-yl)- piperidin-1-carbonsäure-(R)-2-[4-(2-amino-pyrimidin-5-yl)-piperazin-1-yl]-1-(3,4-dichlor-benzyl)-2-oxo-ethyl ester |
| (59) | 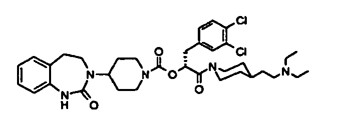 | 4-(2-Oxo-1,2,4,5-tetrahydro-1,3-benzdiazepin-3-yl)-piperidin-1-carbonsäure-(R)-1-(3,4-dichlor-benzyl)-2-[4-(2-diethylamino-ethyl)-piperidin-1-yl]-2-oxo-ethylester |
| (60) | 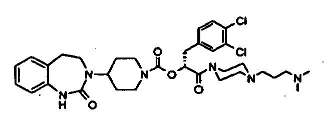 | 4-(2-Oxo-1,2,4,5-tetrahydro-1,3-benzdiazepin-3-yl)-piperidin-1-carbonsäure-(R)-1-(3,4-dichlor-benzyl)-2-[4-(3-dimethylamino-propyl)-piperazin-1-yl]-2-oxo-ethylester |
| (61) | 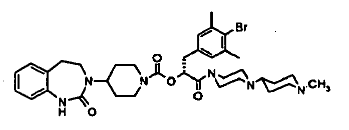 | 4-(2-Oxo-1,2,4,5-tetrahydro-1,3-benzdiazepin-3-yl)-piperidin-1-carbonsäure (R)-1-(4-brom-3,5-dimethyl-benzyl)-2-[4-(1-methyl-piperidin-4-yl)-piperazin-1-yl]-2-oxo-ethyl ester |
| (62) | 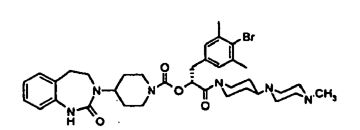 | 4-(2-Oxo-1,2,4,5-tetrahydro-1,3-benzdiazepin-3-yl)-piperidin-1-carbonsäure (R)-1-(4-brom- 3,5-dimethyl-benzyl)-2-[4-(4-methyl-piperazin-1-yl)-piperidin-1-yl]-2-oxo-ethyl ester |
| (63) | 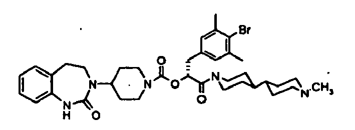 | 4-(2-Oxo-1,2,4,5-tetrahydro-1,3-benzdiazepin-3-yl)-piperidin-1-carbonsäure (R)-1-(4-brom-3,5-dimethyl-benzyl)-2-(1'-methyl-4,4'-bipiperidinyl-1-yl)-2-oxo-ethyl ester |
| (64) | 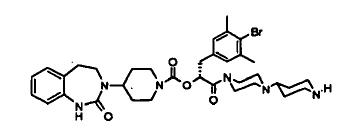 | 4-(2-Oxo-1,2,4,5-tetrahydro-1,3-benzdiazepin-3-yl)-piperidin-1-carbonsäure (R)-1-(4-brom-3,5-dimethyl-benzyl)-2-oxo-2-(4-piperidin-4-yl-piperazin-1-yl)-ethyl ester |
| (65) | 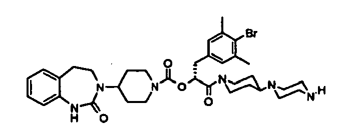 | 4-(2-Oxo-1,2,4,5-tetrahydro-1,3-benzdiazepin-3-yl)-piperidin-1-carbonsäure (R)-1-(4-brom-3,5-dimethyl-benzyl)-2-oxo-2-(4-piperazin-1-yl-piperidin-1-yl)-ethyl ester |
| (66) | 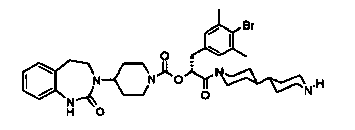 | 4-(2-Oxo-1,2,4,5-tetrahydro-1,3-benzdiazepin-3-yl)-piperidin-1-carbonsäure (R)-2-4,4'-bipiperidinyl-1-yl-1-(4-brom-3,5-dimethyl-benzyl)-2-oxo-ethyl ester |
| (67) | 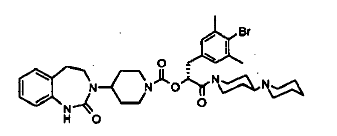 | 4-(2-Oxo-1,2,4,5-tetrahydro-1,3-benzdiazepin-3-yl)-piperidin-1-carbonsäure (R)-2-1,4'-bipiperidinyl-1'-yl-1-(4-brom-3,5-dimethyl-benzyl)-2-oxo-ethyl ester |
| (68) | 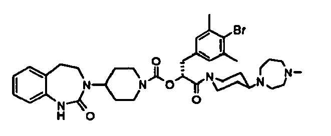 | 4-(2-Oxo-1,2,4,5-tetrahydro-1,3-benzdiazepin-3-yl)-piperidin-1-carbonsäure (R)-1-(4-brom-3,5-dimethyl-benzyl)-2-[4-(4-methyl-perhydro-1,4-diazepin-1-yl)-piperidin-1-yl]-2-oxo-ethyl ester |
| (69) | 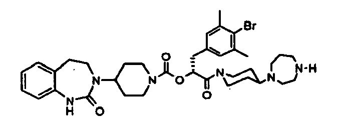 | 4-(2-Oxo-1,2,4,5-tetrahydro-1,3-benzdiazepin-3-yl)-piperidin-1-carbonsäure (R)-1-(4-brom-3,5-dimethyl-benzyl)-2-oxo-2-(4-perhydro-1,4-diazepin-1-yl-piperidin-1-yl)-ethyl ester |
| (70) | 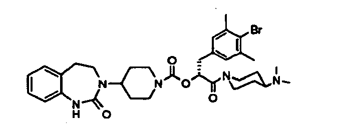 | 4-(2-Oxo-1,2,4,5-tetrahydro-1,3-benzdiazepin-3-yl)-piperidin-1-carbonsäure (R)-1-(4-brom-3,5-dimethyl-benzyl)-2-(4-dimethylamino-piperidin-1-yl)-2-oxo-ethyl ester |
| (71) | 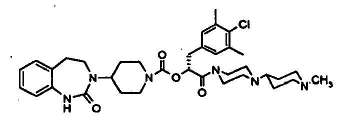 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-[4-(1-methyl-piperidin-4-yl)-piperazin-1-yl]-2-oxo-ethyl ester |
| (72) | 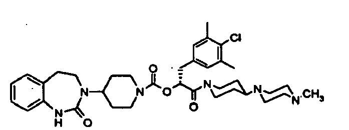 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-[4-(4-methyl-piperazin-1-yl)-piperidin-1-yl]-2-oxo-ethyl ester |
| (73) | 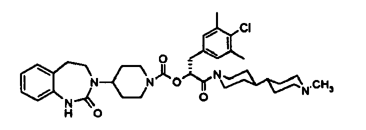 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-(1'-methyl-4,4'-bipiperidinyl-1-yl)-2-oxo-ethyl ester |
| (74) | 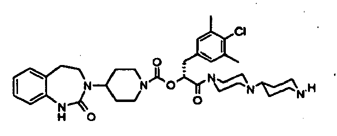 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-oxo-2-(4-piperidin-4-yl-piperazin-1-yl)-ethyl ester |
| (75) | 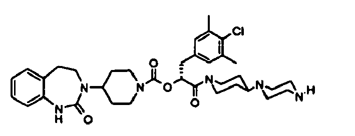 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-oxo-2-(4-piperazin-1-yl-piperidin-1-yl)-ethyl ester |
| (76) | 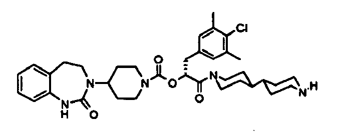 | 4-(2-Oxo-1,2,4,5-tetrahydro-1,3-benzdiazepin-3-yl)-piperidin-1-carbonsäure (R)-2-4,4'-bipiperidinyl-1-yl-1-(4-chlor-3,5-dimethyl-benzyl)-2-oxo-ethyl ester |
| (77) | 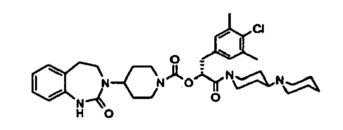 | 4-(2-Oxo-1,2,4,5-tetrahydro-1,3-benzdiazepin-3-yl)-piperidin-1-carbonsäure (R)-2-1,4'-bipiperidinyl-1'-yl-1-(4-chlor-3,5-dimethyl-benzyl)-2-oxo-ethyl ester |
| (78) | 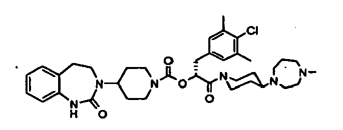 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-[4-(4-methyl-perhydro-1,4-diazepin-1-yl)-piperidin-1-yl]-2-oxo-ethyl ester |
| (79) | 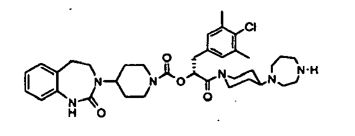 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-oxo-2-(4-perhydro-1,4-diazepin-1-yl-piperidin-1-yl)-ethyl ester |
| (80) | 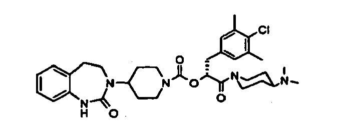 | 4-(2-Oxo-1,2,4,5-tetrahydro-1,3-benzdiazepin-3-yl)-piperidin-1-carbonsäure (R)-1-(4-chlor-3,5-dimethyl-benzyl)-2-(4-dimethylamino-piperidin-1-yl)-2-oxo-ethyl ester |
| (81) | 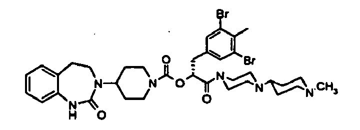 | 4-(2-Oxo-1,2,4,5-tetrahydro-1,3-benzdiazepin-3-yl)-piperidin-1-carbonsäure (R)-1-(3,5-dibrom-4-methyl-benzyl)-2-[4-(1-methyl-piperidin-4-yl)-piperazin-1-yl]-2-oxo-ethyl ester |
| (82) | 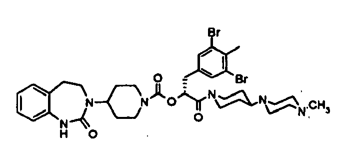 | 4-(2-Oxo-1,2,4,5-tetrahydro-1,3-benzdiazepin-3-yl)-piperidin-1-carbonsäure (R)-1-(3,5-dibrom-4-methyl-benzyl)-2-[4-(4-methyl-piperazin-1-yl)-piperidin-1-yl]-2-Oxo-ethyl ester |
| (83) | 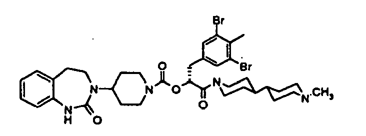 | 4-(2-Oxo-1,2,4,5-tetrahydro-1,3-benzdiazepin-3-yl)-piperidin-1-carbonsäure (R)-1-(3,5-dibrom-4-methyl-benzyl)-2-(1'-methyl-4,4'-bipiperidinyl-1-yl)-2-oxo-ethyl ester |
| (84) | 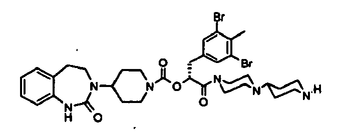 | 4-(2-Oxo-1,2,4,5-tetrahydro-1,3-benzdiazepin-3-yl)-piperidin-1-carbonsäure (R)-1-(3,5-dibrom-4-methyl-benzyl)-2-oxo-2-(4-piperidin-4-yl-piperazin-1-yl)-ethyl ester |
| (85) | 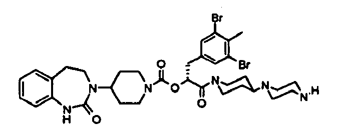 | 4-(2-Oxo-1,2,4,5-tetrahydro-1,3-benzdiazepin-3-yl)-piperidin-1-carbonsäure (R)-1-(3,5-dibrom-4-methyl-benzyl)-2-oxo-2-(4-piperazin-1-yl-piperidin-1-yl)-ethyl ester |
| (86) | 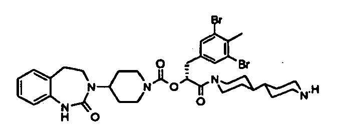 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dibrom-4-methyl-benzyl)-2-oxo-ethyl ester |
| (87) | 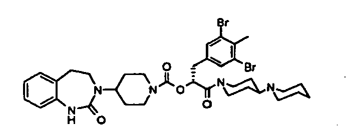 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dibrom-4-methyl-benzyl)-2-oxo-ethyl ester |
| (88) | 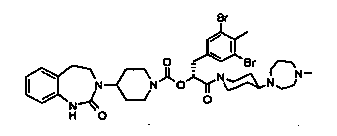 | 4-(2-Oxo-1,2,4,5-tetrahydro-1,3-benzdiazepin-3-yl)-piperidin-1-carbonsäure (R)-1-(3,5-dibrom-4-methyl-benzyl)-2-[4-(4-methyl-perhydro-1,4-diazepin-1-yl)-piperidin-1-yl]-2-oxo-ethyl ester |
| (89) | 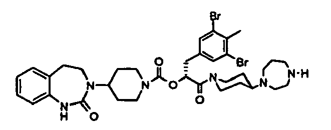 | 4-(2-Oxo-1,2,4,5-tetrahydro-1,3-benzdiazepin-3-yl)-piperidin-1-carbonsäure (R)-1-(3,5-dibrom-4-methyl-benzyl)-2-oxo-2-(4-perhydro-1,4-diazepin-1-yl-piperidin-1-yl)-ethyl ester |
| (90) | 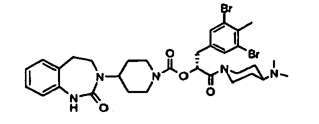 | 4-(2-Oxo-1,2,4,5-tetrahydro-1,3-benzdiazepin-3-yl)-piperidin-1-carbonsäure (R)-1-(3,5-dibrom-4-methyl-benzyl)-2-(4-dimethylamino-piperidin-1-yl)-2-oxo-ethyl ester |
| (91) | 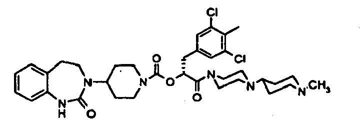 | 4-(2-Oxo-1,2,4,5-tetrahydro-1,3-benzdiazepin-3-yl)-piperidin-1-carbonsäure (R)-1-(3,5-dichlor-4-methyl-benzyl)-2-[4-(1-methyl-piperidin-4-yl)-piperazin-1-yl]-2-oxo-ethyl ester |
| (92) | 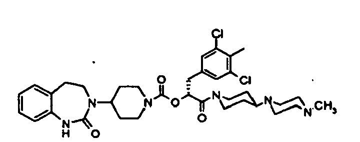 | 4-(2-Oxo-1,2,4,5-tetrahydro-1,3-benzdiazepin-3-yl)-piperidin-1-carbonsäure (R)-1-(3,5-dichlor-4-methyl-benzyl)-2-[4-(4-methyl-piperazin-1-yl)-piperidin-1-yl]-2-oxo-ethyl ester |
| (93) | 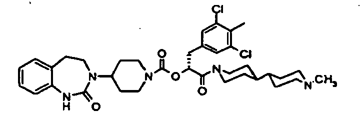 | 4-(2-Oxo-1,2,4,5-tetrahydro-1,3-benzdiazepin-3-yl)-piperidin-1-carbonsäure (R)-1-(3,5-dichlor-4-methyl-benzyl)-2-(1'-methyl-4,4'-bipiperidinyl-1-yl)-2-oxo-ethyl ester |
| (94) | 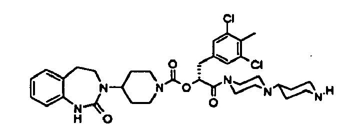 | 4-(2-Oxo-1,2,4,5-tetrahydro-1,3-benzdiazepin-3-yl)-piperidin-1-carbonsäure (R)-1-(3,5-dichlor-4-methyl-benzyl)-2-oxo-2-(4-piperidin-4-yl-piperazin-1-yl)-ethyl ester |
| (95) | 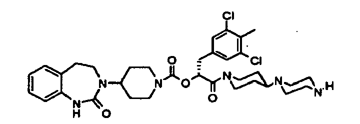 | 4-(2-Oxo-1,2,4,5-tetrahydro-1,3-benzdiazepin-3-yl)-piperidin-1-carbonsäure (R)-1-(3,5-dichlor-4-methyl-benzyl)-2-oxo-2-(4-piperazin-1-yl-piperidin-1-yl)-ethyl ester |
| (96) | 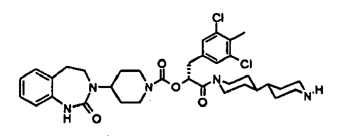 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dichlor-4-methyl-benzyl)-2-oxo-ethyl ester |
| (97) | 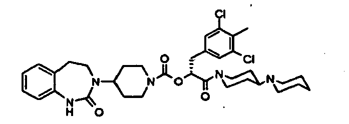 | 4-(2-Oxo-1,2,4,5-tetrahydro-1,3-benzdiazepin-3-yl)-piperidin-1-carbonsäure (R)-2-1,4'-bipiperidinyl-1 '-yl-1-(3,5-dichlor-4-methyl-benzyl)-2-oxo-ethyl ester |
| (98) | 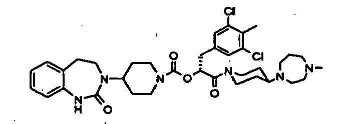 | 4-(2-Oxo-1,2,4,5-tetrahydro-1,3-benzdiazepin-3-yl)-piperidin-1-carbonsäure (R)-1-(3,5-dichlor-4-methyl-benzyl)-2-[4-(4-methyl-perhydro-1,4-diazepin-1-yl)-piperidin-1-yl]-2-oxo-ethyl ester |
| (99) | 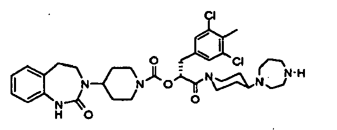 | 4-(2-Oxo-1,2,4,5-tetrahydro-1,3-benzdiazepin-3-yl)-piperidin-1-carbonsäure (R)-1-(3,5-dichlor-4-methyl-benzyl)-2-oxo-2-(4-perhydro-1,4-diazepin-1-yl-piperidin-1-yl)-ethyl ester |
| (100) | 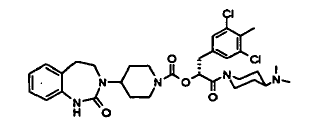 | 4-(2-Oxo-1,2,4,5-tetrahydro-1,3-benzdiazepin-3-yl)-piperidin-1-carbonsäure (R)-1-(3,5-dichlor-4-methyl-benzyl)-2-(4-dimethylamino-piperidin-1-yl)-2-oxo-ethyl ester |
| (101) | 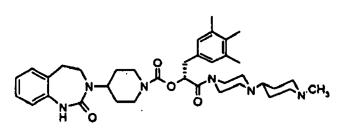 | 4-(2-Oxo-1,2,4,5-tetrahydro-1,3-benzdiazepin-3-yl)-piperidin-1-carbonsäure (R)-2-[4-(1-methyl-piperidin-4-yl)-piperazin-1-yl]-2-oxo-1-(3,4,5-trimethyl-benzyl)-ethyl ester |
| (102) | 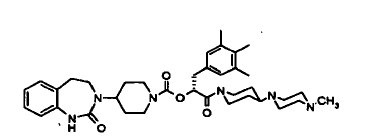 | 4-(2-Oxo-1,2,4,5-tetrahydro-1,3-benzdiazepin-3-yl)-piperidin-1-carbonsäure (R)-2-[4-(4-methyl-piperazin-1-yl)-piperidin-1-yl]-2-oxo-1-(3,4,5-trimethyl-benzyl)-ethyl ester |
| (103) | 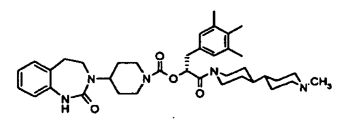 | 4-(2-Oxo-1,2,4,5-tetrahydro-1,3-benzdiazepin-3-yl)-piperidin-1-carbonsäure (R)-2-(1'-methyl-4,4'-bipiperidinyl-1-yl)-2-oxo-1-(3,4,5-trimethyl-benzyl)-ethyl ester |
| (104) | 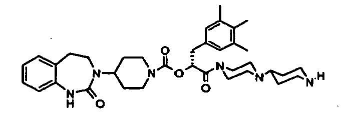 | 4-(2-Oxo-1,2,4,5-tetrahydro-1,3-benzdiazepin-3-yl)-piperidin-1-carbonsäure (R)-2-oxo-2-(4-piperidin-4-yl-piperazin-1-yl)-1-(3,4,5-trimethyl-benzyl)-ethyl ester |
| (105) | 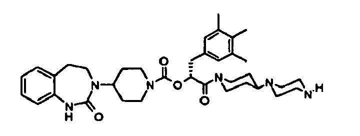 | 4-(2-Oxo-1,2,4,5-tetrahydro-1,3-benzdiazepin-3-yl)-piperidin-1-carbonsäure (R)-2-oxo-2-(4-piperazin-1-yl-piperidin-1-yl)-1-(3,4,5-trimethyl-benzyl)-ethyl ester |
| (106) | 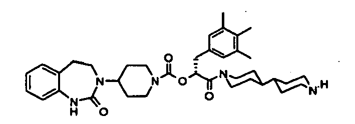 | 4-(2-Oxo-1,2,4,5-tetrahydro-1,3-benzdiazepin-3-yl)-piperidin-1-carbonsäure (R)-2-4,4'-bipiperidinyl-1-yl-2-oxo-1-(3,4,5-trimethyl-benzyl)-ethyl ester |
| (107) | 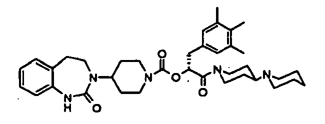 | 4-(2-Oxo-1,2,4,5-tetrahydro-1,3-benzdiazepin-3-yl)-piperidin-1-carbonsäure (R)-2-1,4'-bipiperidinyl-1'-yl-2-oxo-1-(3,4,5-trimethyl-benzyl)-ethyl ester |
| (108) | 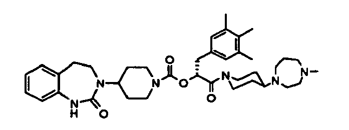 | 4-(2-Oxo-1,2,4,5-tetrahydro-1,3-benzdiazepin-3-yl)-piperidin-1-carbonsäure (R)-2-[4-(4-methyl-perhydro-1,4-diazepin-1-yl)-piperidin-1-yl]-2-oxo-1-(3,4,5-trimethyl-benzyl)-ethyl ester |
| (109) | 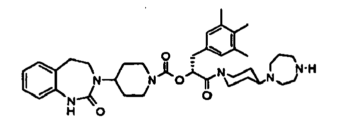 | 4-(2-Oxo-1,2,4,5-tetrahydro-1,3-benzdiazepin-3-yl)-piperidin-1-carbonsäure (R)-2-oxo-2-(4-perhydro-1,4-diazepin-1-yl-piperidin-1-yl)-1-(3,4,5-trimethyl-benzyl)-ethyl ester |
| (110) | 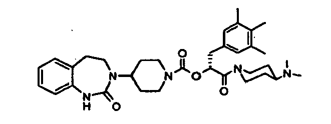 | 4-(2-Oxo-1,2,4,5-tetrahydro-1,3-benzdiazepin-3-yl)-piperidin-1-carbonsäure (R)-2-(4-dimethylamino-piperidin-1-yl)-2-oxo-1-(3,4,5-trimethyl-benzyl)-ethyl ester |
| (111) | 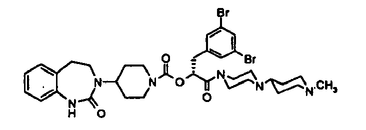 | 4-(2-Oxo-1,2,4,5-tetrahydro-1,3-benzdiazepin-3-yl)-piperidin-1-carbonsäure (R)-1-(3,5-dibrom-benzyl)-2-[4-(1-methyl-piperidin-4-yl)-piperazin-1-yl]-2-oxo-ethyl ester |
| (112) | 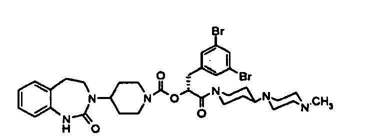 | 4-(2-Oxo-1,2,4,5-tetrahydro-1,3-benzdiazepin-3-yl)-piperidin-1-carbonsäure (R)-1-(3,5-dibrom-benzyl)-2-[4-(4-methyl-piperazin-1-yl)-piperidin-1-yl]-2-oxo-ethyl ester |
| (113) | 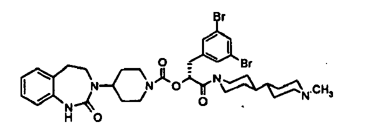 | 4-(2-Oxo-1,2,4,5-tetrahydro-1,3-benzdiazepin-3-yl)-piperidin-1-carbonsäure (R)-1-(3,5-dibrom-benzyl)-2-(1'-methyl-4,4'-bipiperidinyl-1-yl)-2-oxo-ethyl ester |
| (114) | 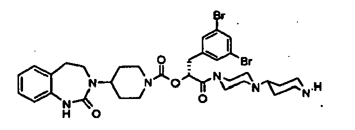 | 4-(2-Oxo-1,2,4,5-tetrahydro-1,3-benzdiazepin-3-yl)-piperidin-1-carbonsäure (R)-1-(3,5-dibrom-benzyl)-2-oxo-2-(4-piperidin-4-yl-piperazin-1-yl)-ethyl ester |
| (115) | 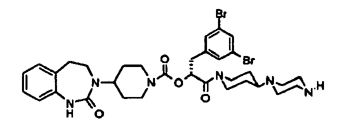 | 4-(2-Oxo-1,2,4,5-tetrahydro-1,3-benzdiazepin-3-yl)-piperidin-1-carbonsäure (R)-1-(3,5-dibrom-benzyl)-2-oxo-2-(4-piperazin-1-yl-piperidin-1-yl)-ethyl ester |
| (116) | 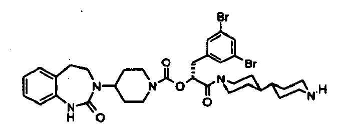 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dibrom-benzyl)-2-oxo-ethyl ester |
| (117) | 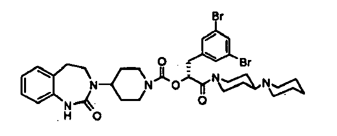 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dibrom-benzyl)-2-oxo-ethyl ester |
| (118) | 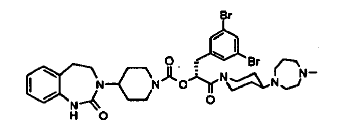 | 4-(2-Oxo-1,2,4,5-tetrahydro-1,3-benzdiazepin-3-yl)-piperidin-1-carbonsäure (R)-1-(3,5-dibrom-benzyl)-2-[4-(4-methyl-perhydro-1,4-diazepin-1-yl)-piperidin-1-yl]-2-oxo-ethyl ester |
| (119) | 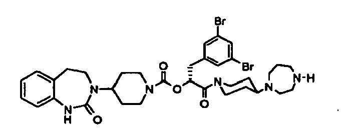 | 4-(2-Oxo-1,2,4,5-tetrahydro-1,3-benzdiazepin-3-yl)-piperidin-1-carbonsäure (R)-1-(3,5-dibrom-benzyl)-2-oxo-2-(4-perhydro-1,4-diazepin-1-yl-piperidin-1-yl)-ethyl ester |
| (120) | 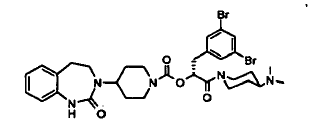 | 4-(2-Oxo-1,2,4,5-tetrahydro-1,3-benzdiazepin-3-yl)-piperidin-1-carbonsäure (R)-1-(3,5-dibrom-benzyl)-2-(4-dimethylamino-piperidin-1-yl)-2-oxo-ethyl ester |
| (121) | 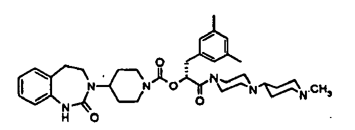 | 4-(2-Oxo-1,2,4,5-tetrahydro-1,3-benzdiazepin-3-yl)-piperidin-1-carbonsäure (R)-1-(3,5-dimethyl-benzyl)-2-[4-(1-methyl-piperidin-4-yl)-piperazin-1-yl]-2-oxo-ethyl ester |
| (122) | 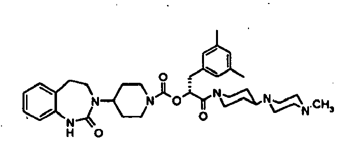 | 4-(2-Oxo-1,2,4,5-tetrahydro-1,3-benzdiazepin-3-yl)-piperidin-1-carbonsäure (R)-1-(3,5-dimethyl-benzyl)-2-[4-(4-methyl-piperazin-1-yl)-piperidin-1-yl]-2-oxo-ethyl ester |
| (123) | 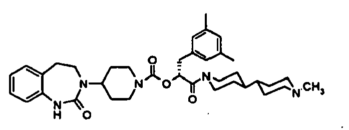 | 4-(2-Oxo-1,2,4,5-tetrahydro-1,3-benzdiazepin-3-yl)-piperidin-1-carbonsäure (R)-1-(3,5-dimethyl-benzyl)-2-(1'-methyl-4,4'-bipiperidinyl-1-yl)-2-oxo-ethyl ester |
| (124) | 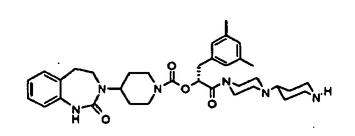 | 4-(2-Oxo-1,2,4,5-tetrahydro-1,3-benzdiazepin-3-yl)-piperidin-1-carbonsäure (R)-1-(3,5-dimethyl-benzyl)-2-oxo-2-(4-piperidin-4-yl-piperazin-1-yl)-ethyl ester |
| (125) | 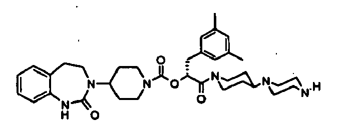 | 4-(2-Oxo-1,2,4,5-tetrahydro-1,3-benzdiazepin-3-yl)-piperidin-1-carbonsäure (R)-1-(3,5-dimethyl-benzyl)-2-oxo-2-(4-piperazin-1-yl-piperidin-1-yl)-ethyl ester |
| (126) | 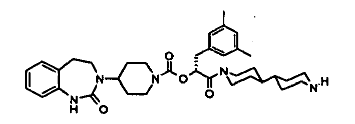 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dimethyl-benzyl)-2-oxo-ethyl ester |
| (127) | 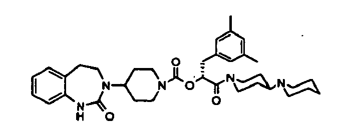 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dimethyl-benzyl)-2-oxo-ethyl ester |
| (128) | 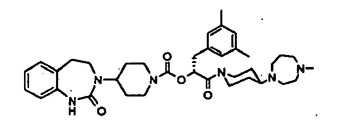 | 4-(2-Oxo-1,2,4,5-tetrahydro-1,3-benzdiazepin-3-yl)-piperidin-1-carbonsäure (R)-1-(3,5-dimethyl-benzyl)-2-[4-(4-methyl-perhydro-1,4-diazepin-1-yl)-piperidin-1-yl]-2-oxo-ethyl ester |
| (129) | 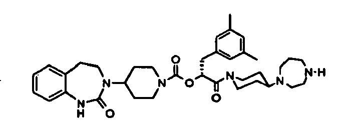 | 4-(2-Oxo-1,2,4,5-tetrahydro-1,3-benzdiazepin-3-yl)-piperidin-1-carbonsäure (R)-1-(3,5-dimethyl-benzyl)-2-oxo-2-(4-perhydro-1,4-diazepin-1-yl-piperidin-1-yl)-ethyl ester |
| (130) | 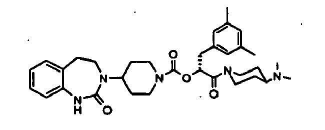 | 4-(2-Oxo-1,2,4,5-tetrahydro-1,3-benzdiazepin-3-yl)-piperidin-1-carbonsäure (R)-2-(4-dimethylamino-piperidin-1-yl)-1-(3,5-dimethyl-benzyl)-2-oxo-ethyl ester |
| (131) | 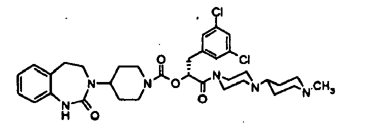 | 4-(2-Oxo-1,2,4,5-tetrahydro-1,3-benzdiazepin-3-yl)-piperidin-1-carbonsäure (R)-1-(3,5-dichlor-benzyl)-2-[4-(1-methyl-piperidin-4-yl)-piperazin-1-yl]-2-oxo-ethyl ester |
| (132) | 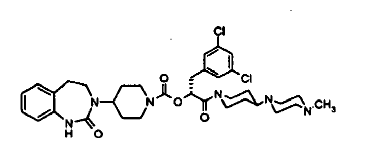 | 4-(2-Oxo-1,2,4,5-tetrahydro-1,3-benzdiazepin-3-yl)-piperidin-1-carbonsäure (R)-1-(3,5-dichlor-benzyl)-2-[4-(4-methyl-piperazin-1-yl)-piperidin-1-yl]-2-oxo-ethyl ester |
| (133) | 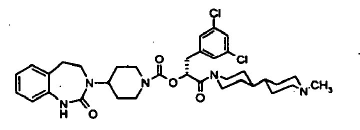 | 4-(2-Oxo-1,2,4,5-tetrahydro-1,3-benzdiazepin-3-yl)-piperidin-1-carbonsäure (R)-1-(3,5-dichlor-benzyl)-2-(1'-methyl-4,4'-bipiperidinyl-1-yl)-2-oxo-ethyl ester |
| (134) | 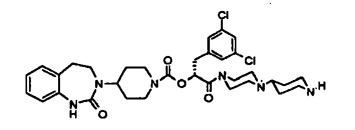 | 4-(2-Oxo-1,2,4,5-tetrahydro-1,3-benzdiazepin-3-yl)-piperidin-1-carbonsäure (R)-1-(3,5-dichlor-benzyl)-2-oxo-2-(4-piperidin-4-yl-piperazin-1-yl)-ethyl ester |
| (135) | 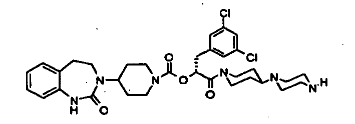 | 4-(2-Oxo-1,2,4,5-tetrahydro-1,3-benzdiazepin-3-yl)-piperidin-1-carbonsäure (R)-1-(3,5-dichlor-benzyl)-2-oxo-2-(4-piperazin-1-yl-piperidin-1-yl)-ethyl ester |
| (136) | 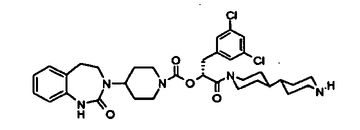 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dichlor-benzyl)-2-oxo-ethyl ester |
| (137) | 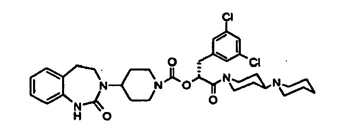 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dichlor-benzyl)-2-oxo-ethyl ester |
| (138) | 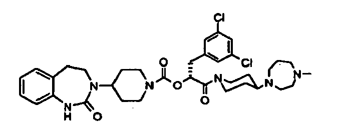 | 4-(2-Oxo-1,2,4,5-tetrahydro-1,3-benzdiazepin-3-yl)-piperidin-1-carbonsäure (R)-1-(3,5-dichlor-benzyl)-2-[4-(4-methyl-perhydro-1,4-diazepin-1-yl)-piperidin-1-yl]-2-oxo-ethyl ester |
| (139) | 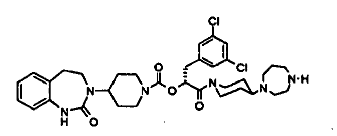 | 4-(2-Oxo-1,2,4,5-tetrahydro-1,3-benzdiazepin-3-yl)-piperidin-1-carbonsäure (R)-1-(3,5-dichlor-benzyl)-2-oxo-2-(4-perhydro-1,4-diazepin-1-yl-piperidin-1-yl)-ethyl ester |
| (140) | 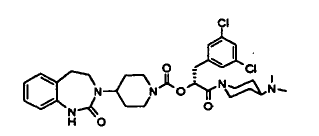 | 4-(2-Oxo-1,2,4,5-tetrahydro-1,3-benzdiazepin-3-yl)-piperidin-1-carbonsäure (R)-1-(3,5-dichlor-benzyl)-2-(4-dimethylamino-piperidin-1-yl)-2-oxo-ethyl ester |
| (141) | 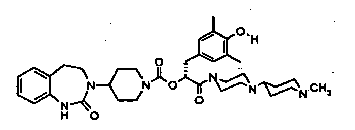 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-(1-methyl-piperidin-4-yl)-piperazin-1-yl]-2-oxo-ethyl ester |
| (142) | 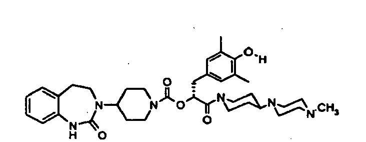 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-(4-methyl-piperazin-1-yl)-piperidin-1-yl]-2-oxo-ethyl ester |
| (143) | 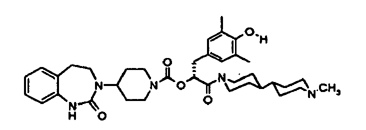 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-(1'-methyl-4,4'-bipiperidinyl-1-yl)-2-oxo-ethyl ester |
| (144) | 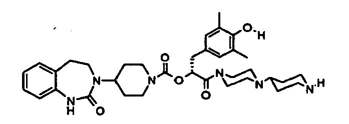 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-oxo-2-(4-piperidin-4-yl-piperazin-1-yl)-ethyl ester |
| (145) | 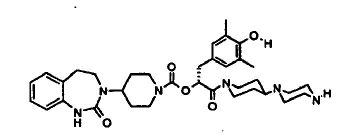 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-oxo-2-(4-piperazin-1-yl-piperidin-1-yl)-ethyl ester |
| (146) | 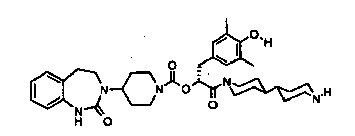 | 4-(2-Oxo-1,2,4,5-tetrahydro-1,3-benzdiazepin-3-yl)-piperidin-1-carbonsäure (R)-2-4,4'-bipiperidinyl-1-yl-1-(4-hydroxy-3,5-dimethyl-benzyl)-2-oxo-ethyl ester |
| (147) | 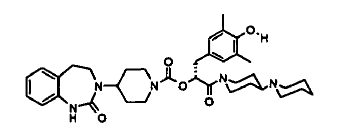 | 4-(2-Oxo-1,2,4,5-tetrahydro-1,3-benzdiazepin-3-yl)-piperidin-1-carbonsäure (R)-2-1,4'-bipiperidinyl-1'-yl-1-(4-hydroxy-3,5-dimethyl-benzyl)-2-oxo-ethyl ester |
| (148) | 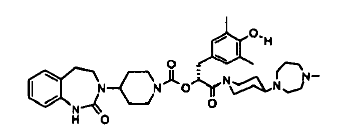 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-(4-methyl-perhydro-1,4-diazepin-1-yl)-piperidin-1-yl]-2-oxo-ethyl ester |
| (149) | 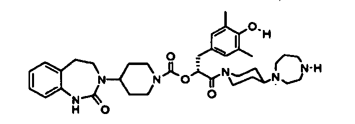 | 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-oxo-2-(4-perhydro-1,4-diazepin-1-yl-piperidin-1-yl)-ethyl ester |
| (150) | 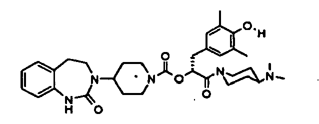 | 4-(2-Oxo-1,2,4,5-tetrahydro-1,3-benzdiazepin-3-yl)-piperidin-1-carbonsäure (R)-2-(4-dimethylamino-piperidin-1-yl)-1-(4-hydroxy-3,5-dimethyl-benzyl)-2-oxo-ethyl ester |
| (151) | 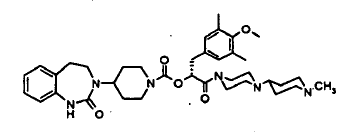 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-[4-(1-methyl-piperidin-4-yl)-piperazin-1-yl]-2-oxo-ethyl ester |
| (152) | 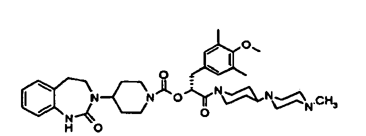 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-[4-(4-methyl-piperazin-1-yl)-piperidin-1-yl]-2-oxo-ethyl ester |
| (153) | 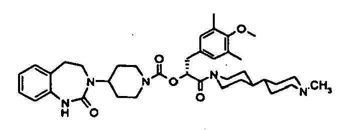 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-(1'-methyl-4,4'-bipiperidinyl-1-yl)-2-oxo-ethyl ester |
| (154) | 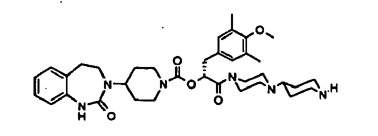 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-oxo-2-(4-piperidin-4-yl-piperazin-1-yl)-ethyl ester |
| (155) | 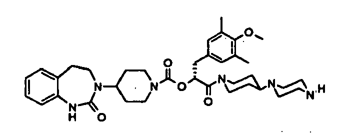 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-oxo-2-(4-piperazin-1-yl-piperidin-1-yl)-ethyl ester |
| (156) | 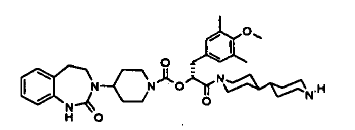 | 4-(2-Oxo-1,2,4,5-tetrahydro-1,3-benzdiazepin-3-yl)-piperidin-1-carbonsäure (R)-2-4,4'-bipiperidinyl-1-yl-1-(4-methoxy-3,5-dimethyl-benzyl)-2-oxo-ethyl ester |
| (157) | 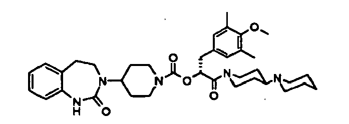 | 4-(2-Oxo-1,2,4,5-tetrahydro-1,3-benzdiazepin-3-yl)-piperidin-1-carbonsäure (R)-2-1,4'-bipiperidinyl-1'-yl-1-(4-methoxy-3,5-dimethyl-benzyl)-2-oxo-ethyl ester |
| (158) | 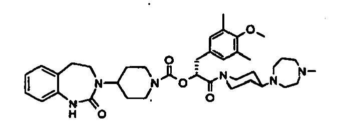 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-[4-(4-methyl-perhydro-1,4-diazepin-1-yl)-piperidin-1-yl]-2-oxo-ethyl ester |
| (159) | 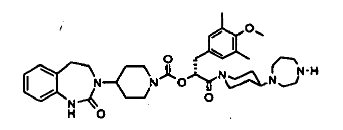 | 4-(2-Oxo-1,2,4,5-tetrahydro-1,3-benzdiazepin-3-yl)-piperidin-1-carbonsäure (R)-1-(4-methoxy-3,5-dimethyl-benzyl)-2-oxo-2-(4-perhydro-1,4-diazepin-1-yl-piperidin-1-yl)-ethyl ester |
| (160) | 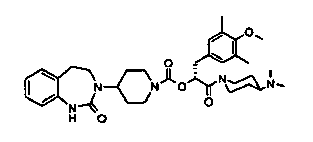 | 4-(2-Oxo-1,2,4,5-tetrahydro-1,3-benzdiazepin-3-yl)-piperidin-1-carbonsäure (R)-2-(4-dimethylamino-piperidin-1-yl)-1-(4-methoxy-3,5-dimethyl-benzyl)-2-oxo-ethyl ester |
| (161) | 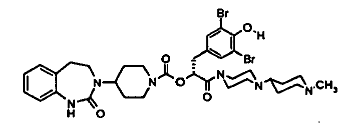 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(1-methyl-piperidin-4-yl)-piperazin-1-yl]-2-oxo-ethyl ester |
| (162) | 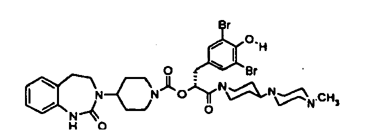 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(4-methyl-piperazin-1-yl)-piperidin-1-yl]-2-oxo-ethyl ester |
| (163) | 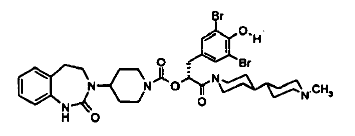 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-(1'-methyl-4,4'-bipiperidinyl-1-yl)-2-oxo-ethyl ester |
| (164) | 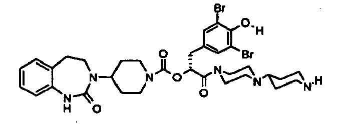 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-oxo-2-(4-piperidin-4-yl-piperazin-1-yl)-ethyl ester |
| (165) | 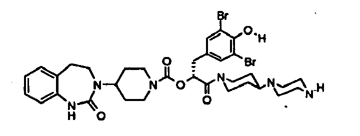 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-oxo-2-(4-piperazin-1-yl-piperidin-1-yl)-ethyl ester |
| (166) | 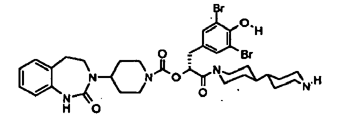 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dibrom-4-hydroxy-benzyl)-2-oxo-ethyl ester |
| (167) | 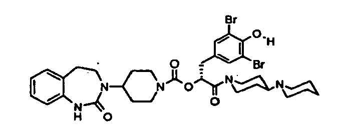 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dibrom-4-hydroxy-benzyl)-2-oxo-ethyl ester |
| (168) | 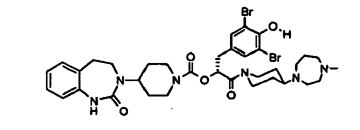 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-[4-(4-methyl-perhydro-1,4-diazepin-1-yl)-piperidin-1-yl]-2-oxo-ethyl ester |
| (169) | 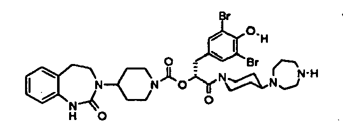 | 4-(2-Oxo-1,2,4,5-tetrahydro-1,3-benzdiazepin-3-yl)-piperidin-1-carbonsäure (R)-1-(3,5-dibrom-4-hydroxy-benzyl)-2-oxo-2-(4-perhydro-1,4-diazepin-1-yl-piperidin-1-yl)-ethyl ester |
| (170) | 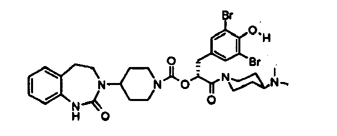 | 4-(2-Oxo-1,2,4,5-tetrahydro-1,3-benzdiazepin-3-yl)-piperidin-1-carbonsäure (R)-1-(3,5-dibröm-4-hydroxy-benzyl)-2-(4-dimethylamino-piperidin-1-yl)-2-oxo-ethyl ester |
| (171) | 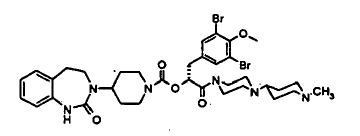 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-[4-(1-methyl-piperidin-4-yl)-piperazin-1-yl]-2-oxo-ethyl ester |
| (172) | 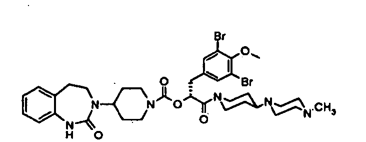 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-[4-(4-methyl-piperazin-1-yl)-piperidin-1-yl]-2-oxo-ethyl ester |
| (173) | 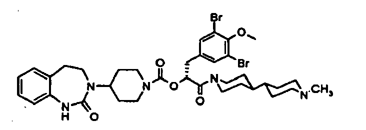 | 4-(2-Oxo-1,2,4,5-tetrahydro-1,3-benzdiazepin- 3-yl)-piperidin-1-carbonsäure (R)-1-(3,5-dibrom-4-methoxy-benzyl)-2-(1'-methyl-4,4'-bipiperidinyl-1-yl)-2-oxo-ethyl ester |
| (174) | 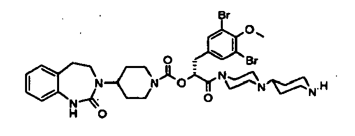 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-oxo-2-(4-piperidin-4-yl-piperazin-1-yl)-ethyl ester |
| (175) | 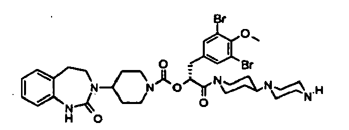 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-oxo-2-(4- piperazin-1-yl-piperidin-1-yl)-ethyl ester |
| (176) | 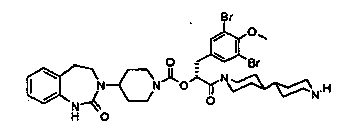 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-dibrom-4-methoxy-benzyl)-2-oxo-ethyl ester |
| (177) | 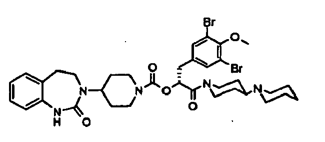 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-dibrom-4-methoxy-benzyl)-2-oxo-ethyl ester |
| (178) | 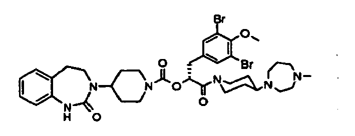 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-[4-(4-methyl-perhydro-1,4-diazepin-1-yl)-piperidin-1-yl]-2-oxo-ethyl ester |
| (179) | 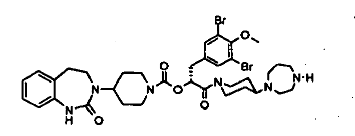 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-oxo-2-(4-perhydro-1,4-diazepin-1-yl-piperidin-1-yl)-ethyl ester |
| (180) | 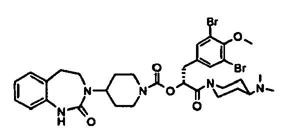 | 4-(2-Oxo-1,2,4,5-tetrahydro-1,3-benzdiazepin-3-yl)-piperidin-1-carbonsäure (R)-1-(3,5-dibrom-4-methoxy-benzyl)-2-(4-dimethylamino-piperidin-1-yl)-2-oxo-ethyl ester |
| (181) | 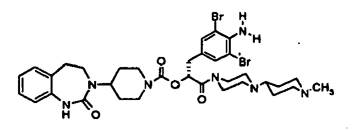 | 4-(2-Oxo-1,2,4,5-tetrahydro-1,3-benzdiazepin-3-yl)-piperidin-1-carbonsäure (R)-1-(4-amino-3,5-dibrom-benzyl)-2-[4-(1-methyl-piperidin-4-yl)-piperazin-1-yl]-2-oxo-ethyl ester |
| (182) | 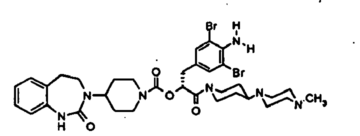 | 4-(2-Oxo-1,2,4,5-tetrahydro-1,3-benzdiazepin-3-yl)-piperidin-1-carbonsäure (R)-1-(4-amino-3,5-dibrom-benzyl)-2-[4-(4-methyl-piperazin-1-yl)-piperidin-1-yl]-2-oxo-ethyl ester |
| (183) | 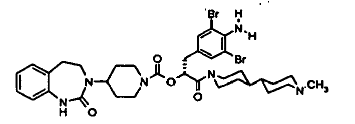 | 4-(2-Oxo-1,2,4,5-tetrahydro-1,3-benzdiazepin-3-yl)-piperidin-1-carbonsäure (R)-1-(4-amino-3,5-dibrom-benzyl)-2-(1'-methyl-4,4'-bipiperidinyl-1-yl)-2-oxo-ethyl ester |
| (184) | 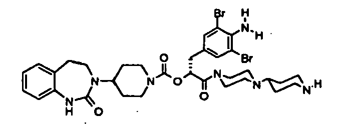 | 4-(2-Oxo-1,2,4,5-tetrahydro-1,3-benzdiazepin-3-yl)-piperidin-1-carbonsäure (R)-1-(4-amino-3,5-dibrom-benzyl)-2-oxo-2-(4-piperidin-4-yl-piperazin-1-yl)-ethyl ester |
| (185) | 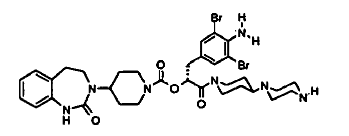 | 4-(2-Oxo-1,2,4,5-tetrahydro-1,3-benzdiazepin-3-yl)-piperidin-1-carbonsäure (R)-1-(4-amino-3,5-dibrom-benzyl)-2-oxo-2-(4-piperazin-1-yl-piperidin-1-yl)-ethyl ester |
| (186) | 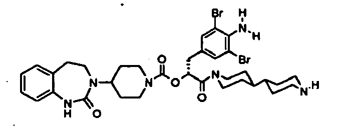 | 4-(2-Oxo-1,2,4,5-tetrahydro-1,3-benzdiazepin-3-yl)-piperidin-1-carbonsäure (R)-1-(4-amino-3,5-dibrom-benzyl)-2-4,4'-bipiperidinyl-1-yl-2-oxo-ethyl ester |
| (187) | 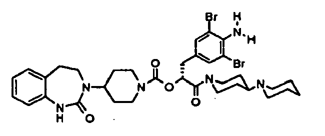 | 4-(2-Oxo-1,2,4,5-tetrahydro-1,3-benzdiazepin-3-yl)-piperidin-1-carbonsäure (R)-1-(4-amino-3,5-dibrom-benzyl)-2-1,4'-bipiperidinyl-1'-yl-2-oxo-ethyl ester |
| (188) | 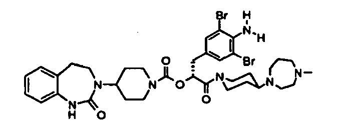 | 4-(2-Oxo-1,2,4,5-tetrahydro-1,3-benzdiazepin-3-yl)-piperidin-1-carbonsäure (R)-1-(4-amino-3,5-dibrom-benzyl)-2-[4-(4-methyl-perhydro-1,4-diazepin-1-yl)-piperidin-1-yl]-2-oxo-ethyl ester |
| (189) | 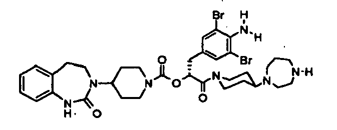 | 4-(2-Oxo-1,2,4,5-tetrahydro-1,3-benzdiazepin-3-yl)-piperidin-1-carbonsäure (R)-1-(4-amino-3,5-dibrom-benzyl)-2-oxo-2-(4-perhydro-1,4-diazepin-1-yl-piperidin-1-yl)-ethyl ester |
| (190) | 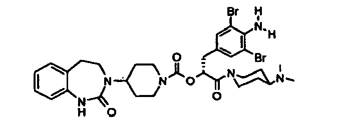 | 4-(2-Oxo-1,2,4,5-tetrahydro-1,3-benzdiazepin-3-yl)-piperidin-1-carbonsäure (R)-1-(4-amino-3,5-dibrom-benzyl)-2-(4-dimethylamino-piperidin-1-yl)-2-oxo-ethyl ester |
| (191) | 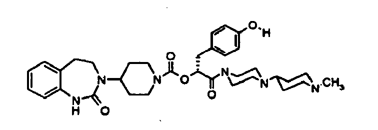 | 4-(2-Oxo-1,2,4,5-tetrahydro-1,3-benzdiazepin-3-yl)-piperidin-1-carbonsäure (R)-1-(4-hydroxy-benzyl)-2-[4-(1-methyl-piperidin-4-yl)-piperazin-1-yl]-2-oxo-ethyl ester |
| (192) | 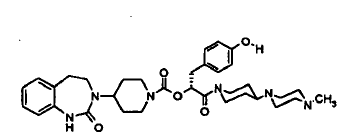 | 4-(2-Oxo-1,2,4,5-tetrahydro-1,3-benzdiazepin-3-yl)-piperidin-1-carbonsäure (R)-1-(4-hydroxy-benzyl)-2-[4-(4-methyl-piperazin-1-yl)-piperidin-1-yl]-2-oxo-ethyl ester |
| (193) | 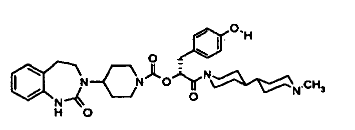 | 4-(2-Oxo-1,2,4,5-tetrahydro-1,3-benzdiazepin-3-yl)-piperidin-1-carbonsäure (R)-1-(4-hydroxy-benzyl)-2-(1'-methyl-4,4'-bipiperidinyl-1-yl)-2-oxo-ethyl ester |
| (194) | 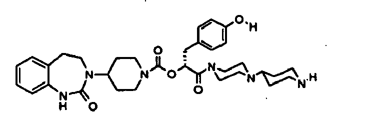 | 4-(2-Oxo-1,2,4,5-tetrahydro-1,3-benzdiazepin-3-yl)-piperidin-1-carbonsäure (R)-1-(4-hydroxy-benzyl)-2-oxo-2-(4-piperidin-4-yl-piperazin-1-yl)-ethyl ester |
| (195) | 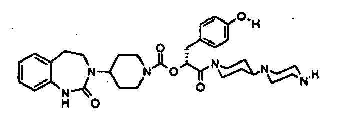 | 4-(2-Oxo-1,2,4,5-tetrahydro-1,3-benzdiazepin-3-yl)-piperidin-1-carbonsäure (R)-1-(4-hydroxy-benzyl)-2-oxo-2-(4-piperazin-1-yl-piperidin-1-yl)-ethyl ester |
| (196) | 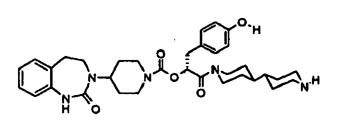 | 4-(2-Oxo-1,2,4,5-tetrahydro-1,3-benzdiazepin-3-yl)-piperidin-1-carbonsäure (R)-2-4,4'-bipiperidinyl-1-yl-1-(4-hydroxy-benzyl)-2-oxo-ethyl ester |
| (197) | 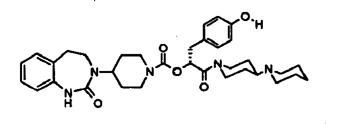 | 4-(2-Oxo-1,2,4,5-tetrahydro-1,3-benzdiazepin-3-yl)-piperidin-1-carbonsäure (R)-2-1,4'-bipiperidinyl-1'-yl-1-(4-hydroxy-benzyl)-2-oxo-ethyl ester |
| (198) | 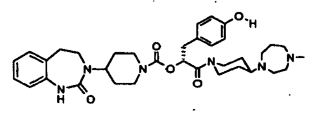 | 4-(2-Oxo-1,2,4,5-tetrahydro-1,3-benzdiazepin-3-yl)-piperidin-1-carbonsäure (R)-1-(4-hydroxy-benzyl)-2-[4-(4-methyl-perhydro-1,4-diazepin-1-yl)-piperidin-1-yl]-2-oxo-ethyl ester |
| (199) | 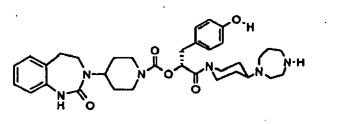 | 4-(2-Oxo-1,2,4,5-tetrahydro-1,3-benzdiazepin-3-yl)-piperidin-1-carbonsäure (R)-1-(4-hydroxy-benzyl)-2-oxo-2-(4-perhydro-1,4-d iazepin-1-yl-piperidin-1-yl)-ethyl ester |
| (200) | 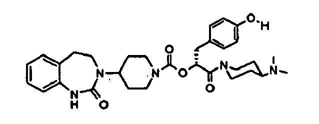 | 4-(2-Oxo-1,2,4,5-tetrahydro-1,3-benzdiazepin-3-yl)-piperidin-1-carbonsäure (R)-2-(4-dimethylamino-piperidin-1-yl)-1-(4-hydroxy-benzyl)-2-oxo-ethyl ester |
| (201) | 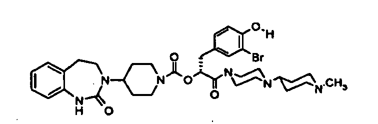 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(1-methyl-piperidin-4-yl)-piperazin-1-yl]-2-oxo-ethyl ester |
| (202) | 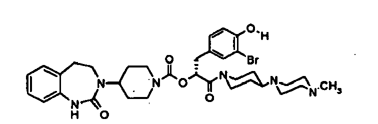 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-methyl-piperazin-1-yl)-piperidin-1-yl]-2-oxo-ethyl ester |
| (203) | 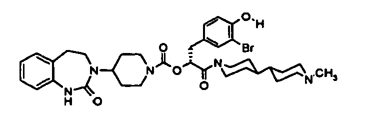 | 4-(2-Oxo-1,2,4,5-tetrahydro-1,3-benzdiazepin-3-yl)-piperidin-1-carbonsäure (R)-1-(3-brom-4-hydroxy-benzyl)-2-(1'-methyl-4,4'-bipiperidinyl-1-yl)-2-oxo-ethyl ester |
| (204) | 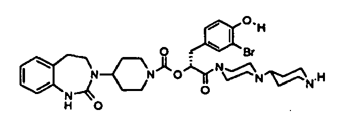 | 4-(2-Oxo-1,2,4,5-tetrahydro-1,3-benzdiazepin-3-yl)-piperidin-1-carbonsäure (R)-1-(3-brom-4-hydroxy-benzyl)-2-oxo-2-(4-piperidin-4-yl-piperazin-1-yl)-ethyl ester |
| (205) | 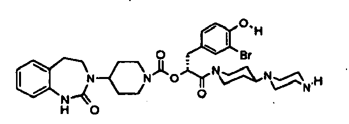 | 4-(2-Oxo-1,2,4,5-tetrahydro-1,3-benzdiazepin-3-yl)-piperidin-1-carbonsäure (R)-1-(3-brom-4-hydroxy-benzyl)-2-oxo-2-(4-piperazin-1-yl-piperidin-1-yl)-ethyl ester |
| (206) | 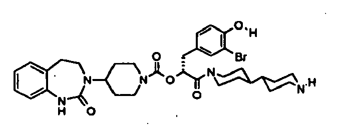 | 4-(2-Oxo-1,2,4,5-tetrahydro-1,3-benzdiazepin-3-yl)-piperidin-1-carbonsäure (R)-2-4,4'-bipiperidinyl-1-yl-1-(3-brom-4-hydroxy-benzyl)-2-oxo-ethyl ester |
| (207) | 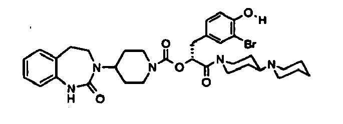 | 4-(2-Oxo-1,2,4,5-tetrahydro-1,3-benzdiazepin-3-yl)-piperidin-1-carbonsäure (R)-2-1,4'-bipiperidinyl-1'-yl-1-(3-brom-4-hydroxy-benzyl)-2-oxo-ethyl ester |
| (208) | 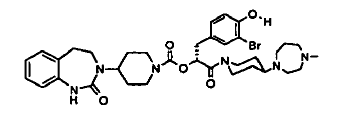 | 4-(2-Oxo-1,2,4,5-tetrahydro-1,3-benzdiazepin-3-yl)-piperidin-1-carbonsäure (R)-1-(3-brom-4-hydroxy-benzyl)-2-[4-(4-methyl-perhydro-1,4-diazepin-1-yl)-piperidin-1-yl]-2-oxo-ethyl ester |
| (209) | 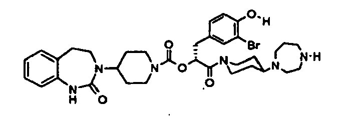 | 4-(2-Oxo-1,2,4,5-tetrahydro-1,3-benzdiazepin-3-yl)-piperidin-1-carbonsäure (R)-1-(3-brom-4-hydroxy-benzyl)-2-oxo-2-(4-perhydro-1,4-diazepin-1-yl-piperidin-1-yl)-ethyl ester |
| (210) | 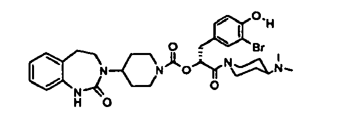 | 4-(2-Oxo-1,2,4,5-tetrahydro-1,3-benzdiazepin-3-yl)-piperidin-1-carbonsäure (R)-1-(3-brom-4-hydroxy-benzyl)-2-(4-dimethylamino-piperidin-1-yl)-2-oxo-ethyl ester |
| (211) | 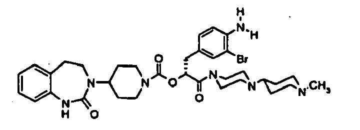 | 4-(2-Oxo-1,2,4,5-tetrahydro-1,3-benzdiazepin-3-yl)-piperidin-1-carbonsäure (R)-1-(4-amino-3-brom-benzyl)-2-[4-(1-methyl-piperidin-4-yl)-piperazin-1-yl]-2-oxo-ethyl ester |
| (212) | 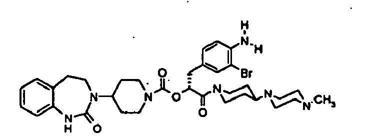 | 4-(2-Oxo-1,2,4,5-tetrahydro-1,3-benzdiazepin-3-yl)-piperidin-1-carbonsäure (R)-1-(4-amino-3-brom-benzyl)-2-[4-(4-methyl-piperazin-1-yl)-piperidin-1-yl]-2-oxo-ethyl ester |
| (213) | 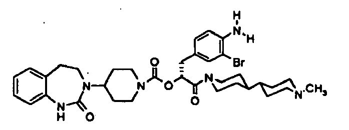 | 4-(2-Oxo-1,2,4,5-tetrahydro-1,3-benzdiazepin-3-yl)-piperidin-1-carbonsäure (R)-1-(4-amino-3-brom-benzyl)-2-(1'-methyl-4,4'-bipiperidinyl-1-yl)-2-oxo-ethyl ester |
| (214) | 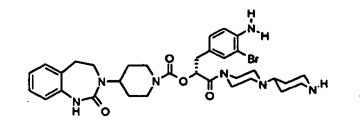 | 4-(2-Oxo-1,2,4,5-tetrahydro-1,3-benzdiazepin-3-yl)-piperidin-1-carbonsäure (R)-1-(4-amino-3-brom-benzyl)-2-oxo-2-(4-piperidin-4-yl-piperazin-1-yl)-ethyl ester |
| (215) | 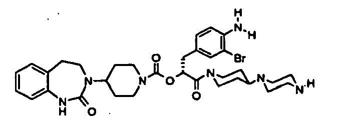 | 4-(2-Oxo-1,2,4,5-tetrahydro-1,3-benzdiazepin-3-yl)-piperidin-1-carbonsäure (R)-1-(4-amino-3-brom-benzyl)-2-oxo-2-(4-piperazin-1-yl-piperidin-1-yl)-ethyl ester |
| (216) | 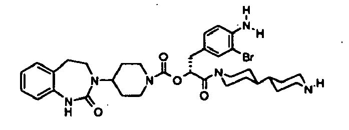 | 4-(2-Oxo-1,2,4,5-tetrahydro-1,3-benzdiazepin-3-yl)-piperidin-1-carbonsäure (R)-1-(4-amino-3-brom-benzyl)-2-4,4'-bipiperidinyl-1-yl-2-oxo-ethyl ester |
| (217) | 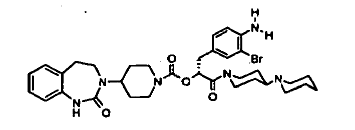 | 4-(2-Oxo-1,2,4,5-tetrahydro-1,3-benzdiazepin-3-yl)-piperidin-1-carbonsäure (R)-1-(4-amino-3-brom-benzyl)-2-1,4'-bipiperidinyl-1'-yl-2-oxo-ethyl ester |
| (218) | 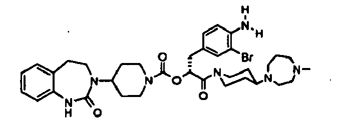 | 4-(2-Oxo-1,2,4,5-tetrahydro-1,3-benzdiazepin-3-yl)-piperidin-1-carbonsäure (R)-1-(4-amino-3-brom-benzyl)-2-[4-(4-methyl-perhydro-1,4-diazepin-1-yl)-piperidin-1-yl]-2-oxo-ethyl ester |
| (219) | 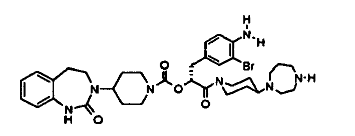 | 4-(2-Oxo-1,2,4,5-tetrahydro-1,3-benzdiazepin-3-yl)-piperidin-1-carbonsäure (R)-1-(4-amino-3-brom-benzyl)-2-oxo-2-(4-perhydro-1,4-diazepin-1-yl-piperidin-1-yl)-ethyl ester |
| (220) | 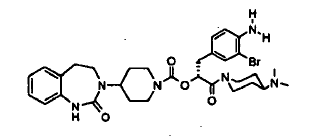 | 4-(2-Oxo-1,2,4,5-tetrahydro-1,3-benzdiazepin-3-yl)-piperidin-1-carbonsäure (R)-1-(4-amino-3-brom-benzyl)-2-(4-dimethylamino-piperidin-1-yl)-2-oxo-ethyl ester |
| (221) | 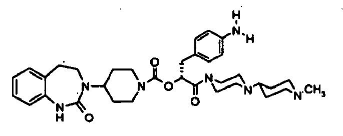 | 4-(2-Oxo-1,2,4,5-tetrahydro-1,3-benzdiazepin-3-yl)-piperidin-1-carbonsäure (R)-1-(4-amino-benzyl)-2-[4-(1-methyl-piperidin-4-yl)-piperazin-1-yl]-2-oxo-ethyl ester |
| (222) | 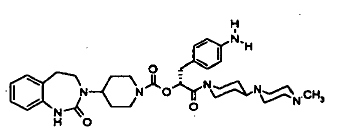 | 4-(2-Oxo-1,2,4,5-tetrahydro-1,3-benzdiazepin-3-yl)-piperidin-1-carbonsäure (R)-1-(4-amino-benzyl)-2-[4-(4-methyl-piperazin-1-yl)-piperidin-1-yl]-2-oxo-ethyl ester |
| (223) | 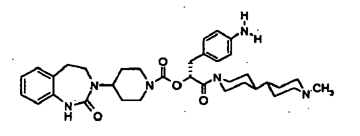 | 4-(2-Oxo-1,2,4,5-tetrahydro-1,3-benzdiazepin-3-yl)-piperidin-1-carbonsäure (R)-1-(4-amino-benzyl)-2-(1'-methyl-4,4'-bipiperidinyl-1-yl)-2-oxo-ethyl ester |
| (224) | 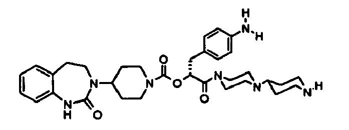 | 4-(2-Oxo-1,2,4,5-tetrahydro-1,3-benzdiazepin-3-yl)-piperidin-1-carbonsäure (R)-1-(4-amino-benzyl)-2-oxo-2-(4-piperidin-4-yl-piperazin-1-yl)-ethyl ester |
| (225) | 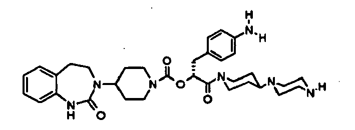 | 4-(2-Oxo-1,2,4,5-tetrahydro-1,3-benzdiazepin-3-yl)-piperidin-1-carbonsäure (R)-1-(4-amino-benzyl)-2-oxo-2-(4-piperazin-1-yl-piperidin-1-yl)-ethyl ester |
| (226) | 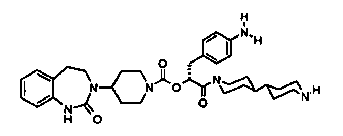 | 4-(2-Oxo-1,2,4,5-tetrahydro-1,3-benzdiazepin-3-yl)-piperidin-1-carbonsäure (R)-1-(4-amino-benzyl)-2-4,4'-bipiperidinyl-1-yl-2-oxo-ethyl ester |
| (227) | 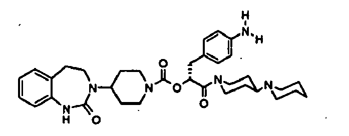 | 4-(2-Oxo-1,2,4,5-tetrahydro-1,3-benzdiazepin-3-yl)-piperidin-1-carbonsäure (R)-1-(4-amino-benzyl)-2-1 ,4'-bipiperidinyl-1 '-yl-2-oxo-ethyl ester |
| (228) | 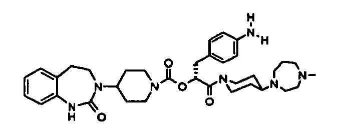 | 4-(2-Oxo-1,2,4,5-tetrahydro-1,3-benzdiazepin-3-yl)-piperidin-1-carbonsäure (R)-1-(4-amino-benzyl)-2-[4-(4-methyl-perhydro-1,4-diazepin-1-yl)-piperidin-1-yl]-2-oxo-ethyl ester |
| (229) | 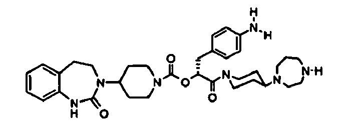 | 4-(2-Oxo-1,2,4,5-tetrahydro-1,3-benzdiazepin-3-yl)-piperidin-1-carbonsäure (R)-1-(4-amino-benzyl)-2-oxo-2-(4-perhydro-1,4-diazepin-1-yl-piperidin-1-yl)-ethyl ester |
| (230) | 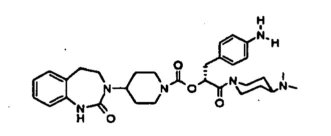 | 4-(2-Oxo-1,2,4,5-tetrahydro-1,3-benzdiazepin-3-yl)-piperidin-1-carbonsäure (R)-1-(4-amino-benzyl)-2-(4-dimethylamino-piperidin-1-yl)-2-oxo-ethyl ester |
| (231) | 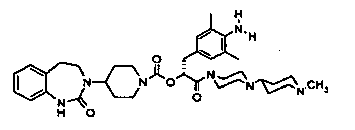 | 4-(2-Oxo-1,2,4,5-tetrahydro-1,3-benzdiazepin3-yl)-piperidin-1-carbonsäure (R)-1-(4-amino-3,5-dimethyl-benzyl)-2-[4-(1-methyl-piperidin-4-yl)-piperazin-1-yl]-2-oxo-ethyl ester |
| (232) | 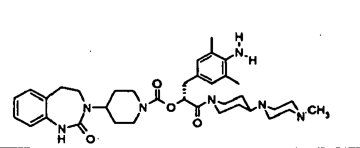 | 4-(2-Oxo-1,2,4,5-tetrahydro-1,3-benzdiazepin-3-yl)-piperidin-1-carbonsäure (R)-1-(4-amino-3,5-dimethyl-benzyl)-2-[4-(4-methyl-piperazin-1-yl)-piperidin-1-yl]-2-oxo-ethyl ester |
| (233) | 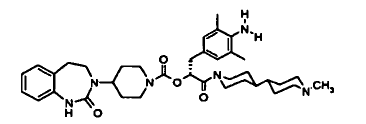 | 4-(2-Oxo-1,2,4,5-tetrahydro-1,3-benzdiazepin-3-yl)-piperidin-1-carbonsäure (R)-1-(4-amino- 3,5-dimethyl-benzyl)-2-(1'-methyl-4,4'-bipiperidinyl-1-yl)-2-oxo-ethyl ester |
| (234) | 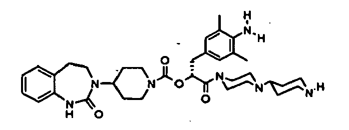 | 4-(2-Oxo-1,2,4,5-tetrahydro-1,3-benzdiazepin-3-yl)-piperidin-1-carbonsäure (R)-1-(4-amino-3,5-dimethyl-benzyl)-2-oxo-2-(4-piperidin-4-yl-piperazin-1-yl)-ethyl ester |
| (235) | 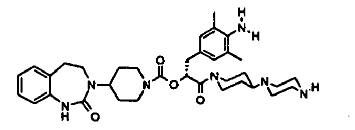 | 4-(2-Oxo-1,2,4,5-tetrahydro-1,3-benzdiazepin-3-yl)-piperidin-1-carbonsäure (R)-1-(4-amino-3,5-dimethyl-benzyl)-2-oxo-2-(4-piperazin-1-yl-piperidin-1-yl)-ethyl ester |
| (236) | 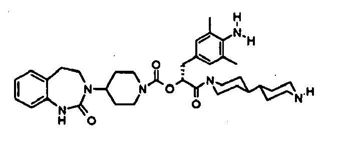 | 4-(2-Oxo-1,2,4, 5-tetrahydro-1,3-benzdiazepin-3-yl)-piperidin-1-carbonsäure (R)-1-(4-amino-3,5-dimethyl-benzyl)-2-4,4'-bipiperidinyl-1-yl-2-oxo-ethyl ester |
| (237) | 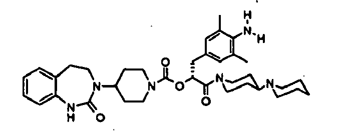 | 4-(2-Oxo-1,2,4,5-tetrahydro-1,3-benzdiazepin-3-yl)-piperidin-1-carbonsäure (R)-1-(4-amino-3,5-dimethyl-benzyl)-2-1,4'-bipiperidinyl-1'-yl-2-oxo-ethyl ester |
| (238) | 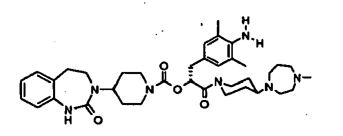 | 4-(2-Oxo-1,2,4,5-tetrahydro-1,3-benzdiazepin-3-yl)-piperidin-1-carbonsäure (R)-1-(4-amino-3,5-dimethyl-benzyl)-2-[4-(4-methyl-perhydro-1,4-diazepin-1-yl)-piperidin-1-yl]-2-oxo-ethyl ester |
| (239) | 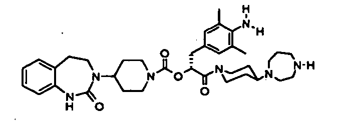 | 4-(2-Oxo-1,2,4,5-tetrahydro-1,3-benzdiazepin-3-yl)-piperidin-1-carbonsäure (R)-1-(4-amino-3,5-dimethyl-benzyl)-2-oxo-2-(4-perhydro-1,4-diazepin-1-yl-piperidin-1-yl)-ethyl ester |
| (240) | 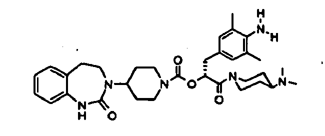 | 4-(2-Oxo-1,2,4,5-tetrahydro-1,3-benzdiazepin-3-yl)-piperidin-1-carbonsäure (R)-1-(4-amino-3,5-dimethyl-benzyl)-2-(4-dimethylamino-piperidin-1-yl)-2-oxo-ethyl ester |
| (241) | 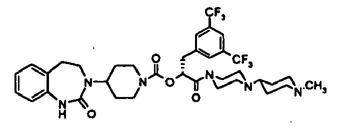 | 4-(2-Oxo-1,2,4,5-tetrahydro-1,3-benzdiazepin-3-yl)-piperidin-1-carbonsäure (R)-1-(3,5-bis-trifluormethyl-benzyl)-2-[4-(1-methyl-piperidin-4-yl)-piperazin-1-yl]-2-oxo-ethyl ester |
| (242) | 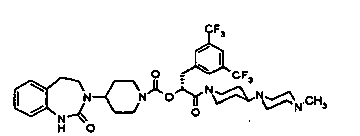 | 4-(2-Oxo-1,2,4,5-tetrahydro-1,3-benzdiazepin-3-yl)-piperidin-1-carbonsäure (R)-1-(3,5-bis-trifluormethyl-benzyl)-2-[4-(4-methyl-piperazin-1-yl)-piperidin-1-yl]-2-oxo-ethyl ester |
| (243) | 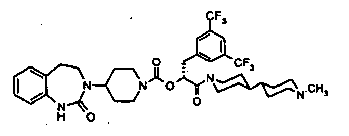 | 4-(2-Oxo-1,2,4,5-tetrahydro-1,3-benzdiazepin-3-yl)-piperidin-1-carbonsäure (R)-1-(3,5-bis-trifluormethyl-benzyl)-2-(1'-methyl-4,4'-bipiperidinyl-1-yl)-2-oxo-ethyl ester |
| (244) | 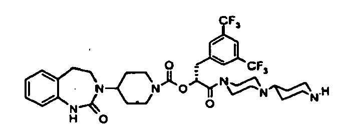 | 4-(2-Oxo-1,2,4,5-tetrahydro-1,3-benzdiazepin-3-yl)-piperidin-1-carbonsäure (R)-1-(3,5-bis-trifluormethyl-benzyl)-2-oxo-2-(4-piperidin-4-yl-piperazin-1-yl)-ethyl ester |
| (245) | 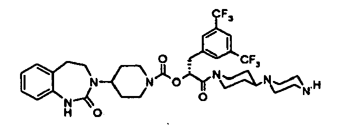 | 4-(2-Oxo-1,2,4,5-tetrahydro-1,3-benzdiazepin-3-yl)-piperidin-1-carbonsäure (R)-1-(3,5-bis-trifluormethyl-benzyl)-2-oxo-2-(4-piperazin-1-yl-piperidin-1-yl)-ethyl ester |
| (246) | 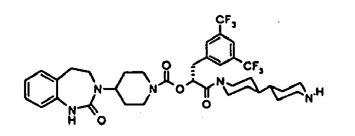 | 4-(2-Oxo-1,2,4,5-tetrahydro-1,3-benzdiazepin-3-yl)-piperidin-1-carbonsäure (R)-2-4,4'-bipiperidinyl-1-yl-1-(3,5-bis-trifluormethyl-benzyl)-2-oxo-ethylester |
| (247) | 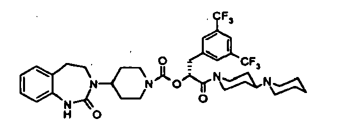 | 4-(2-Oxo-1,2,4,5-tetrahydro-1,3-benzdiazepin-3-yl)-piperidin-1-carbonsäure (R)-2-1,4'-bipiperidinyl-1'-yl-1-(3,5-bis-trifluormethyl-benzyl)-2-oxo-ethylester |
| (248) | 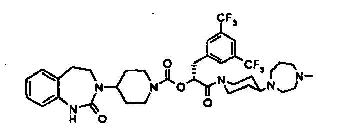 | 4-(2-Oxo-1,2,4,5-tetrahydro-1,3-benzdiazepin-3-yl)-piperidin-1-carbonsäure (R)-1-(3,5-bis-trifluormethyl-benzyl)-2-[4-(4-methyl-perhydro-1,4-diazepin-1-yl)-piperidin-1-yl]-2-oxo-ethylester |
| (249) | 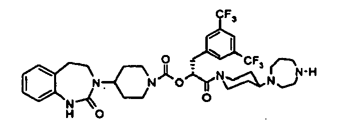 | 4-(2-Oxo-1,2,4,5-tetrahydro-1,3-benzdiazepin-3-yl)-piperidin-1-carbonsäure (R)-1-(3,5-bis-trifluormethyl-benzyl)-2-oxo-2-(4-perhydro-1,4-diazepin-1-yl-piperidin-1-yl)-ethylester |
| (250) | 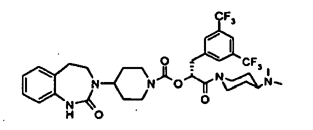 | 4-(2-Oxo-1,2,4,5-tetrahydro-1,3-benzdiazepin-3-yl)-piperidin-1-carbonsäure (R)-1-(3,5-bis-trifluormethyl-benzyl)-2-(4-dimethylamino-piperidin-1-yl)-2-oxo-ethylester |
| (251) | 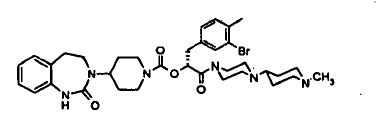 | 4-(2-Oxo-1,2,4,5-tetrahydro-1,3-benzdiazepin-3-yl)-piperidin-1-carbonsäure (R)-1-(3-brom-4-methyl-benzyl)-2-[4-(1-methyl-piperidin-4-yl)-piperazin-1-yl]-2-oxo-ethyl ester |
| (252) | 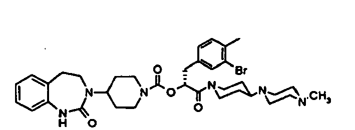 | 4-(2-Oxo-1,2,4,5-tetrahydro-1,3-benzdiazepin-3-yl)-piperidin-1-carbonsäure (R)-1-(3-brom-4-methyl-benzyl)-2-[4-(4-methyl-piperazin-1-yl)-piperidin-1-yl]-2-oxo-ethyl ester |
| (253) | 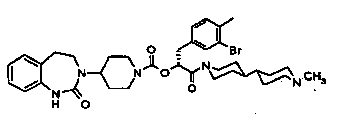 | 4-(2-Oxo-1,2,4,5-tetrahydro-1,3-benzdiazepin-3-yl)-piperidin-1-carbonsäure (R)-1-(3-brom-4-methyl-benzyl)-2-(1'-methyl-4,4'-bipiperidinyl-1-yl)-2-oxo-ethyl ester |
| (254) | 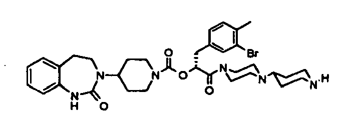 | 4-(2-Oxo-1,2,4,5-tetrahydro-1,3-benzdiazepin-3-yl)-piperidin-1-carbonsäure (R)-1-(3-brom-4-methyl-benzyl)-2-oxo-2-(4-piperidin-4-yl-piperazin-1-yl)-ethyl ester |
| (255) | 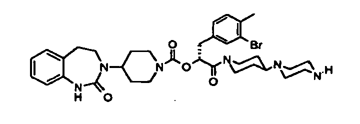 | 4-(2-Oxo-1,2,4,5-tetrahydro-1,3-benzdiazepin-3-yl)-piperidin-1-carbonsäure (R)-1-(3-brom-4-methyl-benzyl)-2-oxo-2-(4-piperazin-1-yl-piperidin-1-yl)-ethyl ester |
| (256) | 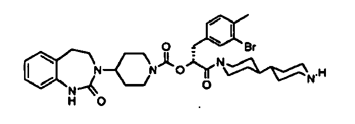 | 4-(2-Oxo-1,2,4,5-tetrahydro-1,3-benzdiazepin-3-yl)-piperidin-1-carbonsäure (R)-2-4,4'-bipiperidinyl-1-yl-1-(3-brom-4-methyl-benzyl)-2-oxo-ethyl ester |
| (257) | 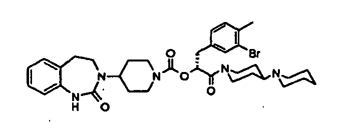 | 4-(2-Oxo-1,2,4,5-tetrahydro-1,3-benzdiazepin-3-yl)-piperidin-1-carbonsäure (R)-2-1,4'-bipiperidinyl-1'-yl-1-(3-brom-4-methyl-benzyl)-2-oxo-ethyl ester |
| (258) | 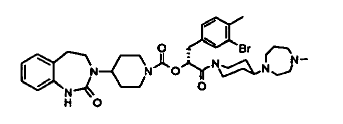 | 4-(2-Oxo-1,2,4,5-tetrahydro-1,3-benzdiazepin-3-yl)-piperidin-1-carbonsäure (R)-1-(3-brom-4-methyl-benzyl)-2-[4-(4-methyl-perhydro-1,4-diazepin-1-yl)-piperidin-1-yl]-2-oxo-ethyl ester |
| (259) | 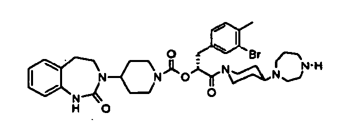 | 4-(2-Oxo-1,2,4,5-tetrahydro-1,3-benzdiazepin-3-yl)-piperidin-1-carbonsäure (R)-1-(3-brom-4-methyl-benzyl)-2-oxo-2-(4-perhydro-1,4-diazepin-1-yl-piperidin-1-yl)-ethyl ester |
| (260) | 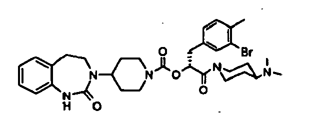 | 4-(2-Oxo-1,2,4,5-tetrahydro-1,3-benzdiazepin-3-yl)-piperidin-1-carbonsäure (R)-1-(3-brom-4-methyl-benzyl)-2-(4-dimethylamino-piperidin-1-yl)-2-oxo-ethyl ester |
| (261) | 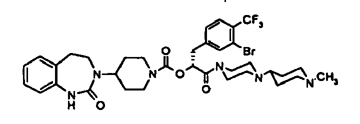 | 4-(2-Oxo-1,2,4,5-tetrahydro-1,3-benzdiazepin-3-yl)-piperidin-1-carbonsäure (R)-1-(3-brom-4-trifluormethyl-benzyl)-2-[4-(1-methyl-piperidin-4-yl)-piperazin-1-yl]-2-oxo-ethyl ester |
| (262) | 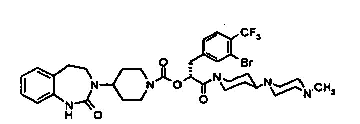 | 4-(2-Oxo-1,2,4,5-tetrahydro-1,3-benzdiazepin-3-yl)-piperidin-1-carbonsäure (R)-1-(3-brom-4-trifluormethyl-benzyl)-2-[4-(4-methyl-piperazin-1-yl)-piperidin-1-yl]-2-oxo-ethyl ester |
| (263) | 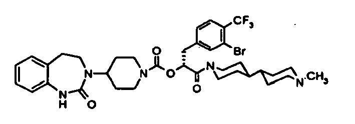 | 4-(2-Oxo-1,2,4,5-tetrahydro-1,3-benzdiazepin-3-yl)-piperidin-1-carbonsäure (R)-1-(3-brom-4-trifluormethyl-benzyl)-2-(1'-methyl-4,4'-bipiperidinyl-1-yl)-2-oxo-ethyl ester |
| (264) | 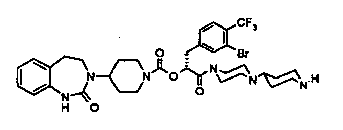 | 4-(2-Oxo-1,2,4,5-tetrahydro-1,3-benzdiazepin-3-yl)-piperidin-1-carbonsäure (R)-1-(3-brom-4-trifluormethyl-benzyl)-2-oxo-2-(4-piperidin-4-yl-piperazin-1-yl)-ethyl ester |
| (265) | 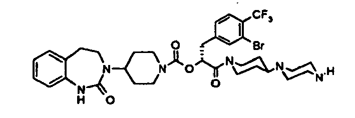 | 4-(2-Oxo-1,2,4,5-tetrahydro-1,3-benzdiazepin-3-yl)-piperidin-1-carbonsäure (R)-1-(3-brom-4-trifluormethyl-benzyl)-2-oxo-2-(4-piperazin-1-yl-piperidin-1-yl)-ethyl ester |
| (266) | 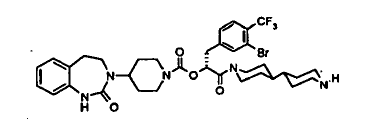 | 4-(2-Oxo-1,2,4,5-tetrahydro-1,3-benzdiazepin-3-yl)-piperidin-1-carbonsäure (R)-2-4,4'-bipiperidinyl-1-yl-1-(3-brom-4-trifluormethyl-benzyl)-2-oxo-ethyl ester |
| (267) | 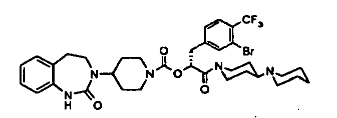 | 4-(2-Oxo-1,2,4,5-tetrahydro-1,3-benzdiazepin-3-yl)-piperidin-1-carbonsäure (R)-2-1,4'-bipiperidinyl-1'-yl-1-(3-brom-4-trifluormethyl-benzyl)-2-oxo-ethyl ester |
| (268) | 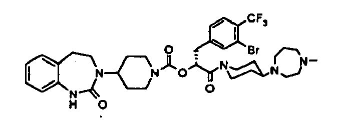 | 4-(2-Oxo-1,2,4,5-tetrahydro-1,3-benzdiazepin-3-yl)-piperidin-1-carbonsäure (R)-1-(3-brom-4-trifluormefhyl-benzyl)-2-[4-(4-methyl-perhydro-1,4-diazepin-1-yl)-piperidin-1-yl]-2-oxo-ethyl ester |
| (269) | 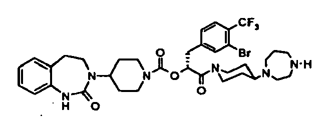 | 4-(2-Oxo-1,2,4,5-tetrahydro-1,3-benzdiazepin-3-yl)-piperidin-1-carbonsäure (R)-1-(3-brom-4-trifluormethyl-benzyl)-2-oxo-2-(4-perhydro-1,4-diazepin-1-yl-piperidin-1-yl)-ethyl ester |
| (270) | 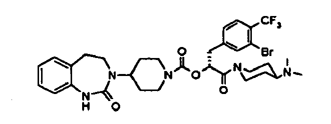 | 4-(2-Oxo-1,2,4,5-tetrahydro-1,3-benzdiazepin-3-yl)-piperidin-1-carbonsäure (R)-1-(3-brom-4-trifluormethyl-benzyl)-2-(4-dimethylamino-piperidin-1-yl)-2-oxo-ethyl ester |
| (271) | 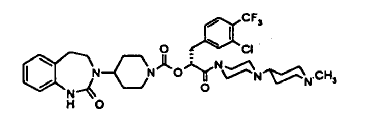 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-[4-(1-methyl-piperidin-4-yl)-piperazin-1-yl]-2-oxo-ethyl ester |
| (272) | 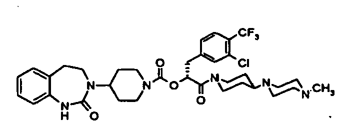 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-[4-(4-methyl-piperazin-1-yl)-piperidin-1-yl]-2-oxo-ethyl ester |
| (273) | 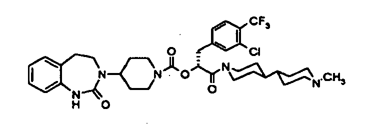 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-(1'-methyl-4,4'-bipiperidinyl-1-yl)-2-oxo-ethyl ester |
| (274) | 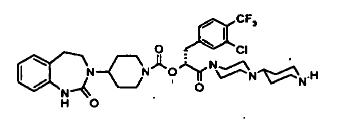 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-oxo-2-(4-piperidin-4-yl-piperazin-1-yl)-ethyl ester |
| (275) | 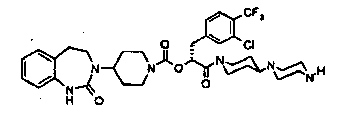 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-oxo-2-(4-piperazin-1-yl-piperidin-1-yl)-ethyl ester |
| (276) | 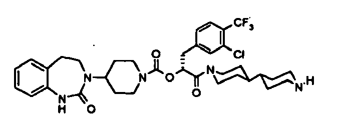 | 4-(2-Oxo-1,2,4,5-tetrahydro-1,3-benzdiazepin-3-yl)-piperidin-1-carbonsäure (R)-2-4,4'-bipiperidinyl-1-yl-1-(3-chlor-4-trifluormethyl-benzyl)-2-oxo-ethyl ester |
| (277) | 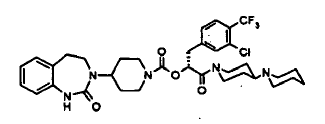 | 4-(2-Oxo-1,2,4,5-tetrahydro-1,3-benzdiazepin-3-yl)-piperidin-1-carbonsäure (R)-2-1,4'-bipiperidinyl-1'-yl-1-(3-chlor-4-trifluormethyl-benzyl)-2-oxo-ethyl ester |
| (278) | 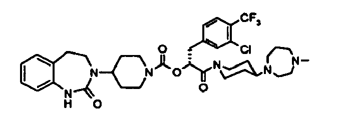 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-[4-(4-methyl-perhydro-1,4-diazepin-1-yl)-piperidin-1-yl]-2-oxo-ethyl ester |
| (279) | 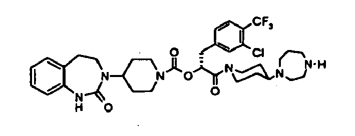 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-oxo-2-(4-perhydro-1,4-diazepin-1-yl-piperidin-1-yl)-ethyl ester |
| (280) | 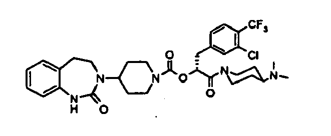 | 4-(2-Oxo-1,2,4,5-tetrahydro-1,3-benzdiazepin-3-yl)-piperidin-1-carbonsäure (R)-1-(3-chlor-4-trifluormethyl-benzyl)-2-(4-dimethylamino-piperidin-1-yl)-2-oxo-ethyl ester |
| (281) | 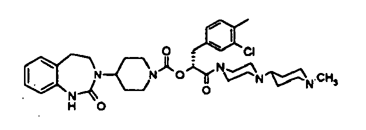 | 4-(2-Oxo-1,2,4,5-tetrahydro-1,3-benzdiazepin-3-yl)-piperidin-1-carbonsäure (R)-1-(3-chlor-4-methyl-benzyl)-2-[4-(1-methyl-piperidin-4-yl)-piperazin-1-yl]-2-oxo-ethyl ester |
| (282) | 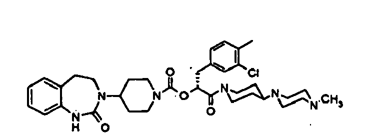 | 4-(2-Oxo-1,2,4,5-tetrahydro-1,3-benzdiazepin-3-yl)-piperidin-1-carbonsäure (R)-1-(3-chlor-4-methyl-benzyl)-2-[4-(4-methyl-piperazin-1-yl)-piperidin-1-yl]-2-oxo-ethyl ester |
| (283) | 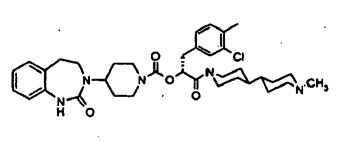 | 4-(2-Oxo-1,2,4,5-tetrahydro-1,3-benzdiazepin-3-yl)-piperidin-1-carbonsäure (R)-1-(3-chlor-4-methyl-benzyl)-2-(1'-methyl-4,4'-bipiperidinyl-1-yl)-2-oxo-ethyl ester |
| (284) | 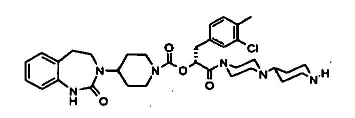 | 4-(2-Oxo-1,2,4,5-tetrahydro-1,3-benzdiazepin-3-yl)-piperidin-1-carbonsäure (R)-1-(3-chlor-4-methyl-benzyl)-2-oxo-2-(4-piperidin-4-yl-piperazin-1-yl)-ethyl ester |
| (285) | 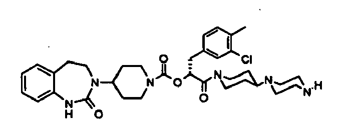 | 4-(2-Oxo-1,2,4,5-tetrahydro-1,3-benzdiazepin-3-yl)-piperidin-1-carbonsäure (R)-1-(3-chlor-4-methyl-benzyl)-2-oxo-2-(4-piperazin-1-yl-piperidin-1-yl)-ethyl ester |
| (286) | 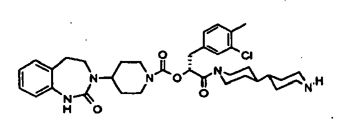 | 4-(2-Oxo-1,2,4,5-tetrahydro-1,3-benzdiazepin-3-yl)-piperidin-1-carbonsäure (R)-2-4,4'-bipiperidinyl-1-yl-1-(3-chlor-4-methyl-benzyl)-2-oxo-ethyl ester |
| (287) | 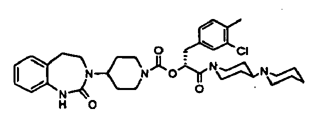 | 4-(2-Oxo-1,2,4,5-tetrahydro-1,3-benzdiazepin-3-yl)-piperidin-1-carbonsäure (R)-2-1,4'-bipiperidinyl-1'-yl-1-(3-chlor-4-methyl-benzyl)-2-oxo-ethyl ester |
| (288) | 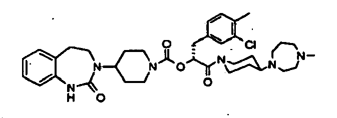 | 4-(2-Oxo-1,2,4,5-tetrahydro-1,3-benzdiazepin-3-yl)-piperidin-1-carbonsäure (R)-1-(3-chlor-4-methyl-benzyl)-2-[4-(4-methyl-perhydro-1,4-diazepin-1-yl)-piperidin-1-yl]-2-oxo-ethyl ester |
| (289) | 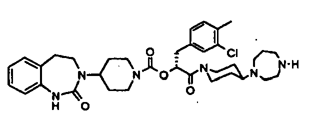 | 4-(2-Oxo-1,2,4,5-tetrahydro-1,3-benzdiazepin-3-yl)-piperidin-1-carbonsäure (R)-1-(3-chlor-4-methyl-benzyl)-2-oxo-2-(4-perhydro-1,4-diazepin-1-yl-piperidin-1-yl)-ethyl ester |
| (290) | 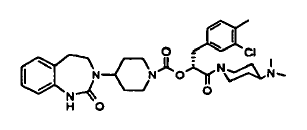 | 4-(2-Oxo-1,2,4,5-tetrahydro-1,3-benzdiazepin-3-yl)-piperidin-1-carbonsäure (R)-1-(3-chlor-4-methyl-benzyl)-2-(4-dimethylamino-piperidin-1-yl)-2-oxo-ethyl ester |
| (291) | 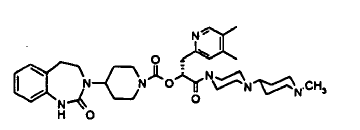 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-[4-(1-methyl-piperidin-4-yl)-piperazin-1-yl]-2-oxo-ethyl ester |
| (292) | 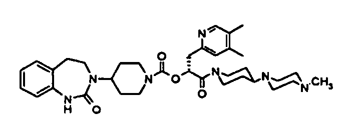 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-[4-(4-methyl-piperazin-1-yl)-piperidin-1-yl]-2-oxo-ethyl ester |
| (293) | 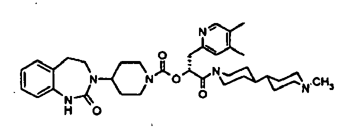 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-(1'-methyl-4,4'-bipiperidinyl-1-yl)-2-oxo-ethyl ester |
| (294) | 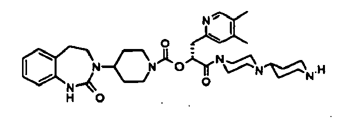 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-oxo-2-(4-piperidin-4-yl-piperazin-1-yl)-ethyl ester |
| (295) | 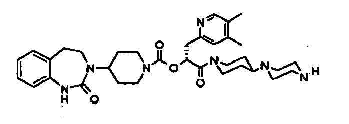 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-oxo-2-(4-piperazin-1-yl-piperidin-1-yl)-ethyl ester |
| (296) | 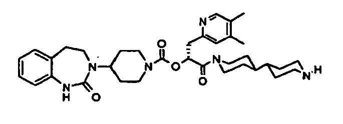 | 4-(2-Oxo-1,2,4,5-tetrahydro-1,3-benzdiazepin-3-yl)-piperidin-1-carbonsäure (R)-2-4,4'-bipiperidinyl-1-yl-1-(4,5-dimethyl-pyridin-2-ylmethyl)-2-oxo-ethyl ester |
| (297) | 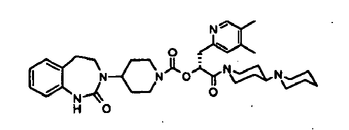 | 4-(2-Oxo-1,2,4,5-tetrahydro-1,3-benzdiazepin-3-yl)-piperidin-1-carbonsäure (R)-2-1,4'-bipiperidinyl-1'-yl-1-(4,5-dimethyl-pyridin-2-ylmethyl)-2-oxo-ethyl ester |
| (298) | 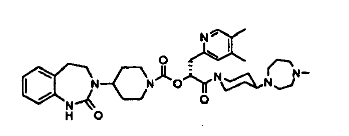 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-[4-(4-methyl-perhydro-1,4-diazepin-1-yl)-piperidin-1-yl]-2-oxo-ethyl ester |
| (299) | 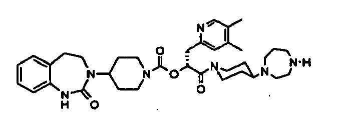 | 4-(2-Oxo-1,2,4,5-tetrahydro-1,3-benzdiazepin-3-yl)-piperidin-1-carbonsäure (R)-1-(4,5-dimethyl-pyridin-2-ylmethyl)-2-oxo-2-(4-perhydro-1,4-diazepin-1-yl-piperidin-1-yl)-ethyl ester |
| (300) | 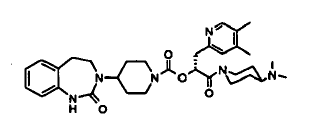 | 4-(2-Oxo-1,2,4,5-tetrahydro-1,3-benzdiazepin-3-yl)-piperidin-1-carbonsäure (R)-2-(4-dimethylamino-piperidin-1-yl)-1-(4,5-dimethyl-pyridin-2-ylmethyl)-2-oxo-ethyl ester |
| (301) | 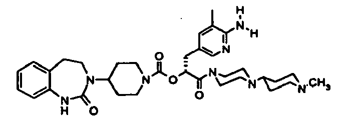 | 4-(2-Oxo-1,2,4,5-tetrahydro-1,3-benzdiazepin-3-yl)-piperidin-1-carbonsäure(R)-1-(6-amino-5-methyl-pyridin-3-ylmethyl)-2-[4-(1-methyl-piperidin-4-yl)-piperazin-1-yl]-2-oxo-ethyl ester |
| (302) | 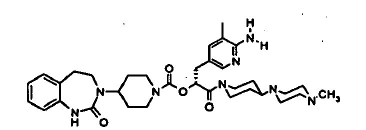 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-[4-(4-methyl-piperazin-1-yl)-piperidin-1-yl]-2-oxo-ethyl ester |
| (303) | 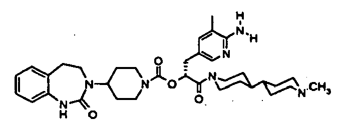 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-(1'-methyl-4,4'-bipiperidinyl-1-yl)-2-oxo-ethyl ester |
| (304) | 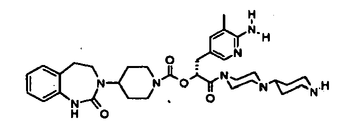 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-oxo-2-(4-piperidin-4-yl-piperazin-1-yl)-ethyl ester |
| (305) | 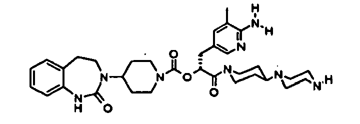 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-oxo-2-(4-piperazin-1-yl-piperidin-1-yl)-ethyl ester |
| (306) | 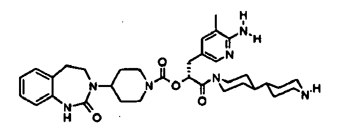 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-4,4'-bipiperidinyl-1-yl-2-oxo-ethyl ester |
| (307) | 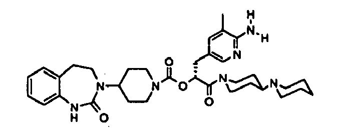 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-1,4'-bipiperidinyl-1'-yl-2-oxo-ethyl ester |
| (308) | 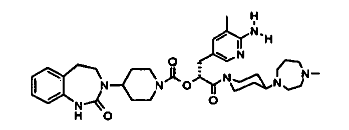 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-[4-(4-methyl-perhydro-1,4-diazepin-1-yl)-piperidin-1-yl]-2-oxo-ethyl ester |
| (309) | 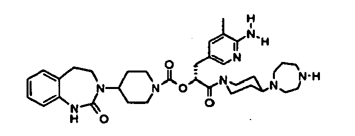 | 4-(2-Oxo-1,2,4,5-tetrahydro-1,3-benzdiazepin-3-yl)-piperidin-1-carbonsäure (R)-1-(6-amino-5-methyl-pyridin-3-ylmethyl)-2-oxo-2-(4-perhydro-1,4-diazepin-1-yl-piperidin-1-yl)-ethyl ester |
| (310) | 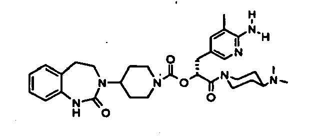 | 4-(2-Oxo-1,2,4,5-tetrahydro-1 3-benzdiazepin-3-yl)-piperidin-1-carbonsäure (R)-1-(6-amino-5-methyl-pyridin-3-ylmethyl)-2-(4-dimethylamino-piperidin-1-yl)-2-oxo-ethyl ester |
| (311) | 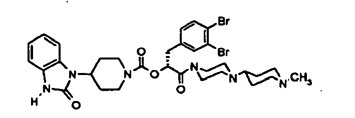 | 4-(2-Oxo-2,3-dihydro-benzimidazol-1-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (312) | 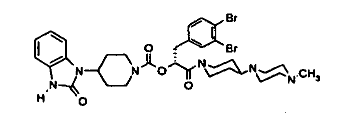 | 4-(2-Oxo-2,3-dihydro-benzimidazol-1-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (313) | 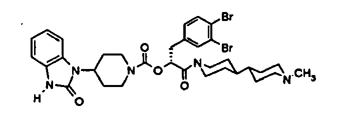 | 4-(2-Oxo-2,3-dihydro-benzimidazol-1-yl)-piperidin-1-carbonsäure (R)-1-(3,4-dibrom-benzyl)-2-(1'-methyl-4,4'-bipiperidinyl-1-yl)-2-oxo-ethyl ester |
| (314) | 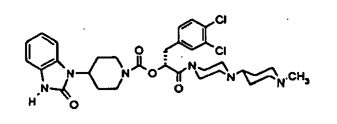 | 4-(2-Oxo-2,3-dihydro-benzimidazol-1-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (315) | 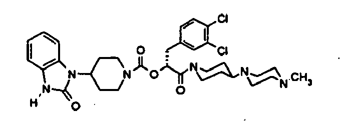 | 4-(2-Oxo-2,3-dihydro-benzimidazol-1-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (316) | 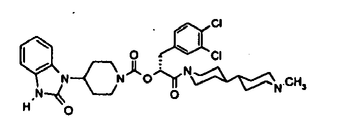 | 4-(2-Oxo-2,3-dihydro-benzimidazol-1-yl)-piperidin-1-carbonsäure (R)-1-(3,4-dichlor-benzyl)-2-(1'-methyl-4,4'-bipiperidinyl-1-yl)-2-oxo-ethyl ester |
| (317) | 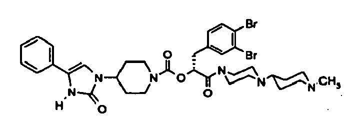 | 4-(2-Oxo-4-phenyl-2,3-dihydro-imidazol-1-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (318) | 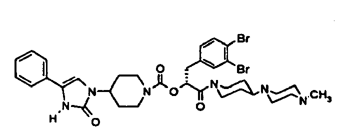 | 4-(2-Oxo-4-phenyl-2,3-dihydro-imidazol-1-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (319) | 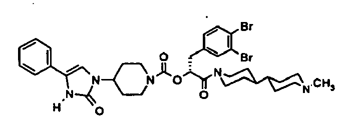 | 4-(2-Oxo-4-phenyl-2,3-dihydro-imidazol-1-yl)-piperidin-1-carbonsäure (R)-1-(3,4-dibrom-benzyl)-2-(1'-methyl-[4,4']bipiperidinyl-1-yl)-2-oxo-ethyl ester |
| (320) | 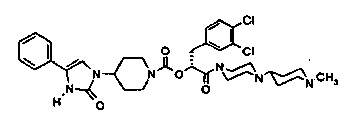 | 4-(2-Oxo-4-phenyl-2,3-dihydro-imidazol-1-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (321) | 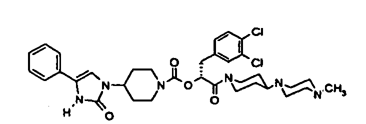 | 4-(2-Oxo-4-phenyl-2,3-dihydro-imidazol-1-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (322) | 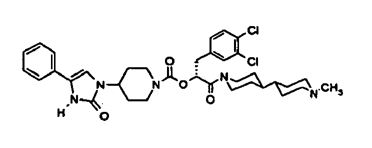 | 4-(2-Oxo-4-phenyl-2,3-dihydro-imidazol-1-yl)-piperidin-1-carbonsäure (R)-1-(3,4-dichlor-benzyl)-2-(1'-methyl-[4,4']bipiperidinyl-1-yl)-2-oxo-ethyl ester |
| (323) | 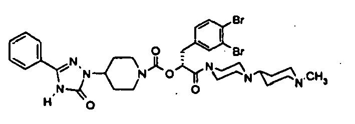 | 4-(5-Oxo-3-phenyl-4,5-dihydro-1,2,4-triazol-1 -yl)-piperidin-1-carbonsäure (R)-1-(3,4-dibrom-benzyl)-2-[4-(1-methyl-piperidin-4-yl)-piperazin-1-yl]-2-oxo-ethyl ester |
| (324) | 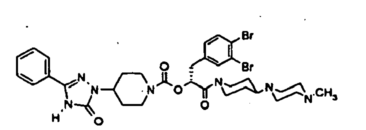 | 4-(5-Oxo-3-phenyl-4,5-dihydro-1,2,4-triazol-1-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (325) | 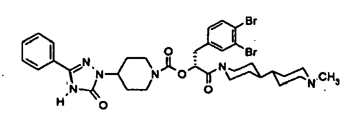 | 4-(5-Oxo-3-phenyl-4,5-dihydro-1,2,4-triazol-1-yl)-piperidin-1-carbonsäure (R)-1-(3,4-dibrom-benzyl)-2-(1'-methyl-4,4'-bipiperidinyl-1-yl)-2-oxo-ethyl ester |
| (326) | 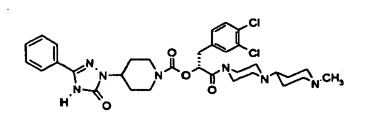 | 4-(5-Oxo-3-phenyl-4,5-dihydro-1,2,4-triazol-1-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (327) | 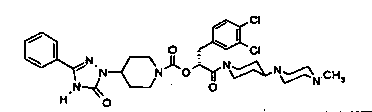 | 4-(5-Oxo-3-phenyl-4,5-dihydro-1,2,4-triazol-1-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (328) | 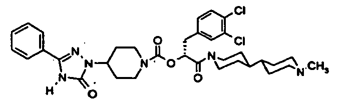 | 4-(5-Oxo-3-phenyl-4,5-dihydro-1,2,4-triazol-1-yl)-piperidin-1-carbonsäure (R)-1-(3,4-dichlor-benzyl)-2-(1'-methyl-4,4'-bipiperidinyl-1-yl)-2-oxo-ethyl ester |
| (329) | 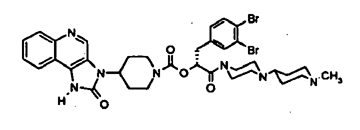 | 4-(2-Oxo-1,2-dihydro-imidazo[4,5-*c*]chinolin-3-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (330) | 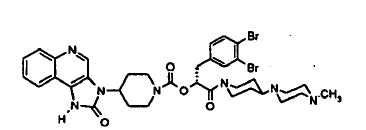 | 4-(2-Oxo-1,2-dihydro-imidazo[4,5-*c*]chinolin-3-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (331) | 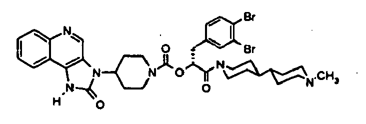 | 4-(2-Oxo-1,2-dihydro-imidazo[4,5-*c*]chinolin-3-yl)-piperidin-1-carbonsäure (R)-1-(3,4-dibrom-benzyl)-2-(1'-methyl-[4,4']bipiperidinyl-1-yl)-2-oxo-ethyl ester |
| (332) | 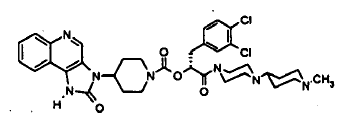 | 4-(2-Oxo-1,2-dihydro-imidazo[4,5-*c*]chinolin-3-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (333) | 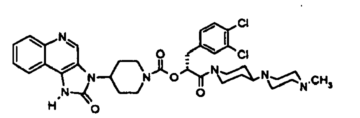 | 4-(2-Oxo-1,2-dihydro-imidazo[4,5-*c*]chinolin-3- yl)-piperidin-1-carbonsäure (R)-1-(3,4-dichlor-benzyl)-2-[4-(4-methyl-piperazin-1-yl)-piperidin-1-yl]-2-oxo-ethyl ester |
| (334) | 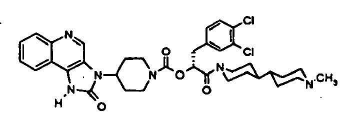 | 4-(2-Oxo-1,2-dihydro-imidazo[4,5-*c*]chinolin-3-yl)-piperidin-1-carbonsäure (R)-1-(3,4-dichlor-benzyl)-2-(1'-methyl-[4,4']bipiperidinyl-1-yl)-2-oxo-ethyl ester |
| (335) | 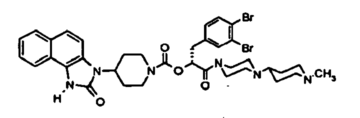 | 4-(2-Oxo-1,2-dihydro-naphth[1,2-*d*]imidazol-3-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (336) | 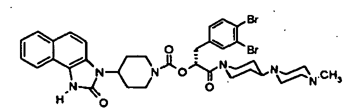 | 4-(2-Oxo-1,2-dihydro-naphth[1,2-*d*]imidazol-3-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (337) | 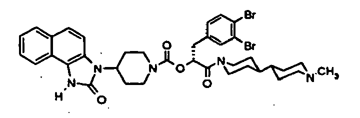 | 4-(2-Oxo-1,2-dihydro-naphth[1,2-*d*]imidazol-3-yl)-piperidin-1-carbonsäure (R)-1-(3,4-dibrom-benzyl)-2-(1'-methyl-4,4'-bipiperidinyl-1-yl)-2-oxo-ethyl ester |
| (338) | 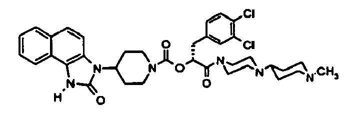 | 4-(2-Oxo-1,2-dihydro-naphth[1,2-*d*]imidazol-3-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (339) | 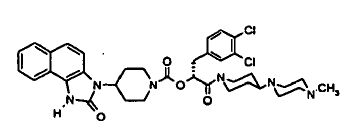 | 4-(2-Oxo-1,2-dihydro-naphth[1,2-d]imidazol-3-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (340) | 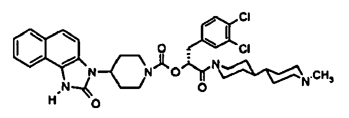 | 4-(2-Oxo-1,2-dihydro-naphth[1,2-*d*]imidazol-3-yl)-piperidin-1-carbonsäure (R)-1-(3,4-dichlor-benzyl)-2-(1'-methyl-4,4'-bipiperidinyl-1-yl)-2-oxo-ethyl ester |
| (341) | 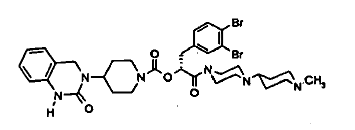 | 4-(2-Oxo-1,4-dihydro-2*H*-chinazolin-3-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (342) | 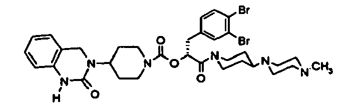 | 4-(2-Oxo-1,4-dihydro-2*H*-chinazolin-3-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (343) | 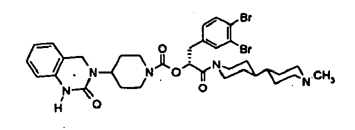 | 4-(2-Oxo-1,4-dihydro-2*H*-chinazolin-3-yl)-piperidin-1-carbonsäure (R)-1-(3,4-dibrom-benzyl)-2-(1'-methyl-[4,4']bipiperidinyl-1-yl)-2-oxo-ethyl ester |
| (344) | 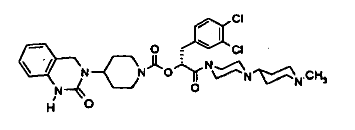 | 4-(2-Oxo-1,4-dihydro-2*H*-chinazolin-3-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (345) | 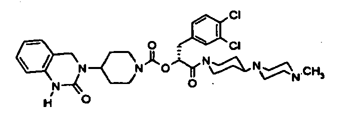 | 4-(2-Oxo-1,4-dihydro-2*H*-chinazolin-3-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (346) | 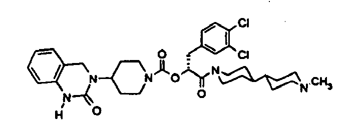 | 4-(2-Oxo-1,4-dihydro-2*H*-chinazolin-3-yl)-piperidin-1-carbonsäure (R)-1-(3,4-dichlor-benzyl)-2-(1'-methyl-[4,4']bipiperidinyl-1-yl)-2-oxo-ethyl ester |
| (347) | 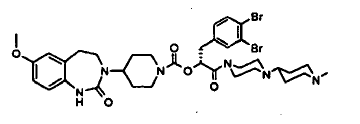 | 4-(7-Methoxy-2-oxo-1,2,4,5-tetrahydro-1, 3-benzdiazepin-3-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (348) | 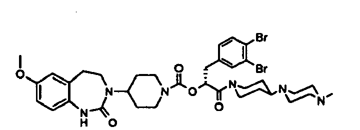 | 4-(7-Methoxy-2-oxo-1,2,4,5-tetrahydro-1,3-benzdiazepin-3-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (349) | 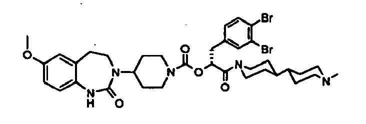 | 4-(7-Methoxy-2-oxo-1,2,4,5-tetrahydro-1,3-benzdiazepin-3-yl)-piperidin-1-carbonsäure (R)-1-(3,4-dibrom-benzyl)-2-(1'-methyl-4,4'-bipiperidinyl-1-yl)-2-oxo-ethyl ester |
| (350) | 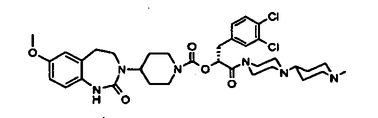 | 4-(7-Methoxy-2-oxo-1,2,4,5-tetrahydro-1,3-benzdiazepin-3-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (351) | 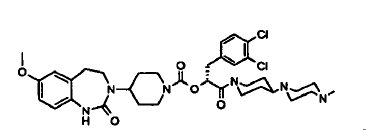 | 4-(7-Methoxy-2-oxo-1,2,4,5-tetrahydro-1,3-benzdiazepin-3-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (352) | 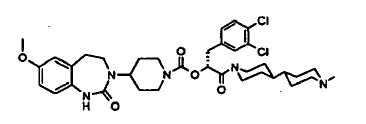 | 4-(7-Methoxy-2-oxo-1,2,4,5-tetrahydro-1,3-benzdiazepin-3-yl)-piperidin-1-carbonsäure (R)-1-(3,4-dichlor-benzyl)-2-(1'-methyl-4,4'-bipiperidinyl-1-yl)-2-oxo-ethyl ester |
| (353) | 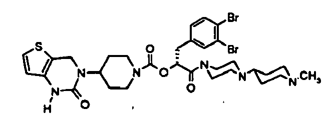 | 4-(2-Oxo-1,4-dihydro-2*H*-thieno[3,2-*d*]pyrimidin-3-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (354) | 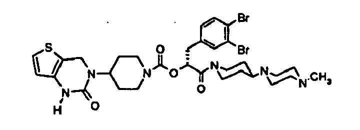 | 4-(2-Oxo-1,4-dihydro-2*H*-thieno[3,2-*d*]pyrimidin-3-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (355) | 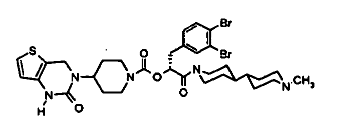 | 4-(2-Oxo-1,4-dihydro-2*H*-thieno[3,2-*d*]pyrimidin-3-yl)-piperidin-1-carbonsäure (R)-1-(3,4-dibrom-benzyl)-2-(1'-methyl-[4,4']bipiperidinyl-1-yl)-2-oxo-ethyl ester |
| (356) | 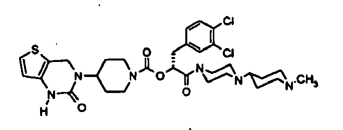 | 4-(2-Oxo-1,4-dihydro-2*H*-thieno[3,2-*d*]pyrimidin-3-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (357) | 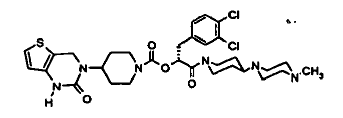 | 4-(2-Oxo-1,4-dihydro-2*H*-thieno[3,2-*d*]pyrimidin-3-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (358) | 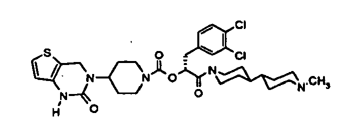 | 4-(2-Oxo-1,4-dihydro-2*H*-thieno[3,2-*d*]pyrimidin-3-yl)-piperidin-1-carbonsäure (R)-1-(3,4-dichlor-benzyl)-2-(1'-methyl-[4,4']bipiperidinyl-1-yl)-2-oxo-ethyl ester |
| (359) | 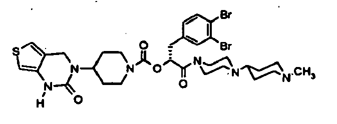 | 4-(2-Oxo-1,2-dihydro-4*H*-thieno[3,4-*d*]pyrimidin-3-yl)-piperidin-1-carbonsäure (R)-1-(3,4-dibrom-benzyl)-2-[4-(1-methyl-piperidin-4-yl)-piperazin-1-yl]-2-oxo-ethyl ester |
| (360) | 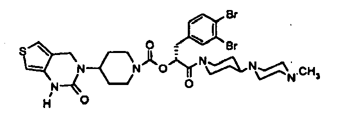 | 4-(2-Oxo-1,2-dihydro-4*H*-thieno[3,4-d]pyrimidin-3-yl)-piperidin-1-carbonsäure (R)-1-(3,4-dibrom-benzyl)-2-[4-(4-methyl-piperazin-1-yl)-piperidin-1-yl]-2-oxo-ethyl ester |
| (361) | 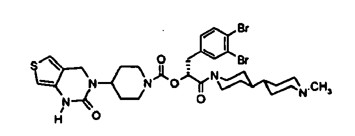 | 4-(2-Oxo-1,2-dihydro-4*H*-thieno[3,4-*d*]pyrimidin-3-yl)-piperidin-1-carbonsäure (R)-1-(3,4-dibrom-benzyl)-2-(1'-methyl-[4,4']bipiperidinyl-1-yl)-2-oxo-ethyl ester |
| (362) | 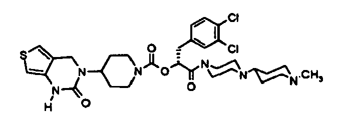 | 4-(2-Oxo-1,2-dihydro-4*H*-thieno[3,4-*d*]pyrimidin-3-yl)-piperidin-1-carbonsäure (R)-1-(3,4-dichlor-benzyl)-2-[4-(1-methyl-piperidin-4-yl)-piperazin-1-yl]-2-oxo-ethyl ester |
| (363) | 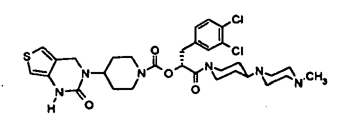 | 4-(2-Oxo-1,2-dihydro-4*H*-thieno[3,4-*d*]pyrimidin-3-yl)-piperidin-1-carbonsäure (R)-1-(3,4-dichlor-benzyl)-2-[4-(4-methyl-piperazin-1-yl)-piperidin-1-yl]-2-oxo-ethyl ester |
| (364) | 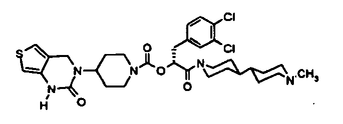 | 4-(2-Oxo-1,2-dihydro-4*H*-thieno[3,4-*d*]pyrimidin-3-yl)-piperidin-1-carbonsäure (R)-1-(3,4-dichlor-benzyl)-2-(1'-methyl-[4,4']bipiperidinyl-1-yl)-2-oxo-ethyl ester |

deren Enantiomere, deren Diastereomere und deren Salze.

Die Verbindungen der allgemeinen Formel (I) werden nach prinzipiell bekannten Methoden hergestellt. Die folgenden Verfahren haben sich zur Herstellung der erfindungsgemäßen Verbindungen der allgemeinen Formel (I) besonders bewährt:
(a) Zur Herstellung von Verbindungen der allgemeinen Formel (I), in der alle Reste wie eingangs erwähnt definiert sind:
   Umsetzung von Piperidinen der allgemeinen Formel 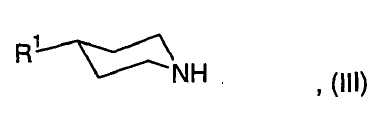 in der R¹ wie eingangs erwähnt definiert ist,
   mit Kohlensäurederivaten der allgemeinen Formel 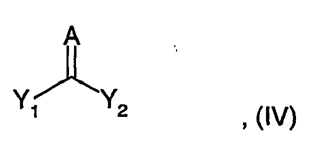 in der Y₁ und Y₂ nucleofuge Gruppen bedeuten, die gleich oder verschieden sein können, bevorzugt das Chloratom, die p-Nitrophenoxy- oder Trichlormethoxygruppe, falls A das Sauerstoffatom darstellt, oder das Chloratom, falls A das Schwefelatom bedeutet,
   und mit Verbindungen der allgemeinen Formel ' 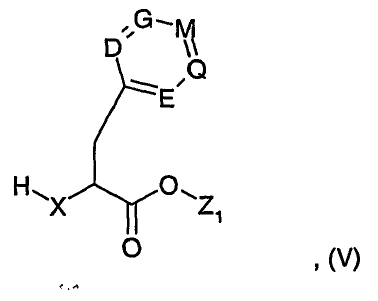 in der X, D, E, G, M und Q wie eingangs erwähnt definiert sind und Z₁ eine Schutzgruppe für eine Carboxygruppe darstellt, beispielsweise eine C₁₋₆-Alkyl- oder Benzylgruppe, wobei die Alkylgruppen linear oder verzweigt sein können und die Benzylgruppe durch ein oder zwei Methoxygruppen substituiert sein kann. Bevorzugt ist für Z₁ die Methyl-, Ethyl-, tert-Butyl oder Benzylgruppe. Vor Durchführung der Reaktion können im Rest R¹ einer Verbindung der Formel (III) und/oder in einer Verbindung der Formel (V) gegebenenfalls vorhandene Carbonsäurefunktionen, primäre oder sekundäre Aminofunktionen oder Hydroxyfunktionen durch übliche Schutzreste geschützt und gegebenenfalls verwendete Schutzreste nach Durchführung der Reaktion nach dem Fachmann geläufigen Methoden wieder abgespalten werden.
   In einer ersten Stufe werden die Verbindungen der allgemeinen Formel (III) in einem Lösungsmittel, beispielsweise in Dichlormethan, THF, Pyridin oder deren Mischungen, bei einer Temperaturzwischen -20 bis 50°C in Gegenwart einer Base, beispielsweise Triethylamin, Pyridin oder Ethyldiisopropylamin, mit den Kohlensäurederivaten der allgemeinen Formel (IV) zur Reaktion gebracht. Die dabei entstehende Zwischenstufe kann aufgereinigt oder ohne Reinigung weiter umgesetzt werden.
   Die Umsetzung dieser Zwischenstufen mit Verbindungen der allgemeinen Formel (V) erfolgt ebenfalls in einem der oben genannten Lösungmittel, und bei den oben genannten Temperaturen, in Gegenwart einer Base, wie Triethylamin oder Pyridin, mit oder ohne Zusatz eines Aktivierungsreagenz, wie z.B. 4-Dimethylaminopyridin. Zur Aktivierung können die Verbindungen der allgemeinen Formel (V) auch mittels eines Metallhydrides, wie z.B. NaH oder KH, deprotoniert werden, wobei in diesem Fall auf die Gegenwart der Base oder des Aktivierungsreagenzes verzichtet werden kann.
(b) Zur Herstellung von Verbindungen der allgemeinen Formel (I), in der alle Reste wie eingangs erwähnt definiert sind:
   Kupplung einer Carbonsäure der allgemeinen Formel 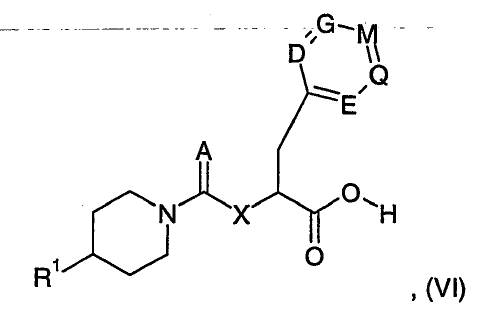 in der alle Reste wie eingangs erwähnt definiert sind, mit einem Amin der allgemeinen Formel HNR²R³, in der R² und R³ wie eingangs definiert sind. Vor Durchführung der Reaktion können in einer Verbindung der Formel (VI) und/oder in den Resten R² und R³ des Amins der Formel HNR²R³ gegebenenfalls vorhandene. Carbonsäurefunktionen, primäre oder sekundäre Aminofunktionen oder Hydroxyfunktionen durch übliche Schutzreste geschützt und gegebenenfalls verwendete Schutzreste nach Durchführung der Reaktion nach dem Fachmann geläufigen Methoden wieder abgespalten werden.
   Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z. B. Dicyclohexylcarbodiimid (DCC), Diisopropyl-carbodiimid (DIC) oderEthyl-(3-dimethylamino-propyl)-carbodiimid, O-(1*H*-Benzotriazol-1-yl)-*N,N-N',N'*-tetramethyluronium-hexa-fluorphosphat (HBTU) oder -tetrafluorborat (TBTU) oder 1H-Benzotriazol-1-yl-oxy-tris-(dimethylamino)-phosphoniumhexafluorphosphat (BOP) eingesetzt werden. Durch Zugabe von 1-Hydroxybenzotriazol (HOBt) oder von 3-Hydroxy-4-oxo-3,4-dihydro-1,2,3-benzotriazin (HOObt) kann die Reaktionsgeschwindigkeit gesteigert werden. Die Kupplungen werden normalerweise mit äquimolaren Anteilen der Kupplungskomponenten sowie des Kupplungsreagenz in Lösungsmitteln wie Dichlormethan, Tetrahydrofuran, Acetonitril, Dimethylformamid (DMF), Dimethylacetamid (DMA), *N*-Methylpyrrolidon (NMP) oder Gemischen aus diesen und bei Temperaturen zwischen -30 und +30°C, bevorzugt -20 und +25°C, durchgeführt. Sofern erforderlich wird als zusätzliche Hilfsbase N-Ethyldiisopropylamin (Hünig-Base) bevorzugt.
   Als weiteres Kupplungsverfahren zur Synthese von Verbindungen der allgemeinen Formel (I) wird das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden Carbonsäure der allgemeinen Formel (VIII) und dem Kohlensäure-monoisobutylester erhalten wird. Die Herstellung dieses gemischten Anhydrids und die Kupplung mit den Aminen der allgemeinen Formel HNR²R³ erfolgt im Eintopfverfahren, unter Verwendung der vorstehend genannten Lösungsmittel und bei Temperaturen zwischen -20°C und +25°C, bevorzugt zwischen 0°C und +25°C.
(c) Zur Herstellung von Verbindungen der allgemeinen Formel (I), in der alle Reste wie eingangs erwähnt definiert sind:
   Kupplung einer Verbindung der allgemeinen Formel 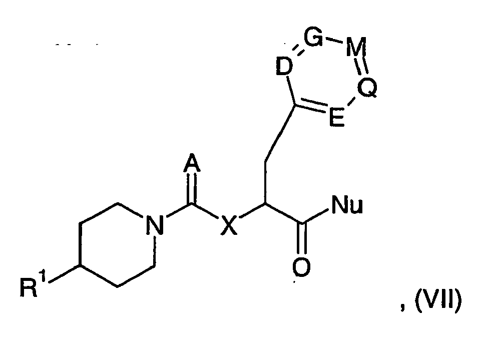 mit einem Amin der allgemeinen Formel HNR²R³,

in denen alle Reste wie eingangs erwähnt definiert sind und Nu eine Austrittsgruppe, beispielsweise ein Halogenatom, wie das Chlor-, Brom- oder Iodatom, eine Alkylsulfonyloxygruppe mit 1 bis 10 Kohlenstoffatomen im Alkylteil, eine gegebenenfalls durch Chlor- oder Bromatome, durch Methyl- oder Nitrogruppen mono-, di- oder trisubstituierte Phenylsulfonyloxy- oder Naphthylsulfonyloxygruppe, wobei die Substituenten gleich oder verschieden sein können, eine 1*H*-Imidazol-1-yl-, eine gegebenenfalls durch eine oder zwei Methylgruppen im Kohlenstoffgerüst substituierte 1*H*-Pyrazol-1-yl-, eine 1*H*-1,2,4-Triazol-1-yl-, 1*H-*1,2,3-Triazol-1-yl-, 1*H-*1,2,3,4-Tetrazol-1-yl-, eine Vinyl-, Propargyl-, p-Nitrophenyl-, 2,4-Dinitrophenyl-, Trichlorphenyl-, Pentachlorphenyl-, Pentafluorphenyl-, Pyranyl- oder Pyridinyl-, eine Dimethylaminyloxy-, 2(1*H*-Oxopyridin-1-yl-oxy-, 2,5-Dioxopyrrolidin-1-yloxy-, Phthalimidyloxy-, 1*H*-Benzo-triazol-1-yloxy- oder Azidgruppe bedeutet. Vor Durchführung der Reaktion können in einer Verbindung der Formel (VII) und/oder in den Resten R² und R³ des Amins der Formel HNR²R³ gegebenenfalls vorhandene Carbonsäurefunktionen, primäre oder sekundäre Aminofunktionen oder Hydroxyfunktionen durch übliche Schutzreste geschützt und gegebenenfalls verwendete Schutzreste nach Durchführung der Reaktion nach dem Fachmann geläufigen Methoden wieder abgespalten werden.

Die Umsetzung wird unter Schotten-Baumann- oder Einhorn-Bedingungen durchgeführt, das heißt, die Komponenten werden in Gegenwart von wenigstens einem Äquivalent einer Hilfsbase bei Temperaturen zwischen -50°C und +120°C, bevorzugt -10°C und +30°C; und gegebenenfalls in Gegenwart von Lösungsmitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, N-Ethyldiisopropylamin, N-Ethyldicyclohexylamin, 1,4-Di-azabicyclo[2,2,2]octan oder 1,8-Diaza-bicyclo[5,4,0]undec-7-en, als Lösungsmittel beispielsweise Dichlormethan, Tetrahydrofuran, 1,4-Dioxan, Acetonitril, Dimethylformamid, Dimethylacetamid, *N*-Methylpyrrolidon oder Gemische davon in Betracht; werden als Hilfsbasen Alkali- oder Erdalkalihydroxide, Alkalicarbonate oder -acetate verwendet, kann dem Reaktionsgemisch auch Wasser als Cosolvens zugesetzt werden.

Die erfindungsgemäßen neuen Verbindungen der allgemeinen Formel (I) enthalten ein oder mehrere Chiralitätszentren. Sind beispielsweise zwei Chiralitätszentren vorhanden, dann können die Verbindungen in Form zweier diastereomerer Antipodenpaare auftreten. Die Erfindung umfaßt die einzelnen Isomeren ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel (I) fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)- oder (-)-Camphersulfonsäure, bzw. einer optisch aktiven Base, beispielsweise mit (*R*)-(+)-1-Phenylethylamin, (*S*)-(-)-1-Phenylethylamin oder (S)-Brucin, entstehen.

Nach einem üblichen Verfahren zur Isomerentrennung wird das Racemat einer Verbindung der allgemeinen Formel (I) mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (*R*)- oder (*S*)-Enantiomer bzw. ein Gemisch zweier optisch aktiver, unter die allgemeine Formel (I) fallender, diastereomerer Verbindungen wird auch dadurch erhalten, dass man die oben beschriebenen Synthesen mit jeweils einer geeigneten (*R*)- bzw. (*S*)-konfigurierten Reaktionskomponente durchführt.

Falls die Gruppe X in Verbindungen der allgemeinen Formel (V) das Sauerstoffatom darstellt, können die für die Synthese benötigten Hydroxycarbonsäuren der allgemeinen Formel 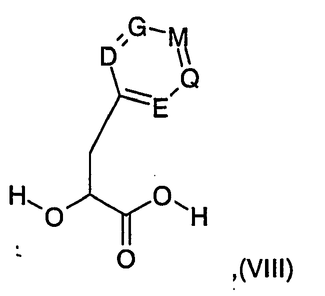 aus Verbindungen der allgemeinen Formel 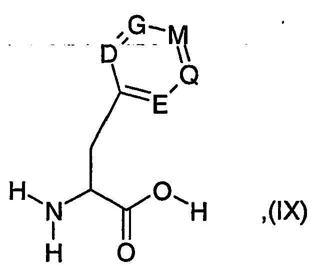 gewonnen werden, wobei D, E, G, M und Q in beiden Formeln wie eingangs erwähnt definiert sind.

Durch Diazotierung von Verbindungen der allgemeinen Formel (IX) mit einem geeignetem Diazotierungsreagenz, bevorzugt Natriumnitrit in sauren Milieu, können die Verbindungen der allgemeinen Formel (VIII) erhalten werden. Bei Einsatz enantiomerenreiner Verbindungen werden die entsprechenden enantiomerenreinen Hydroxycarbonsäureverbindungen erhalten, wobei die Reaktion unter Retention der Konfiguration abläuft.

Ein alternativer Zugang zu Verbindungen der allgemeinen Formel (VIII) besteht in der Umsetzung von Aldehyden der allgemeinen Formel (X) mit N-Acetylglycin in Acetanhydrid als Lösungsmittel in Gegenwart von Alkaliacetat, bevorzugt Natrium- oder Kaliumacetat bei geeigneter Temperatur, bevorzugt bei 80-130°C.

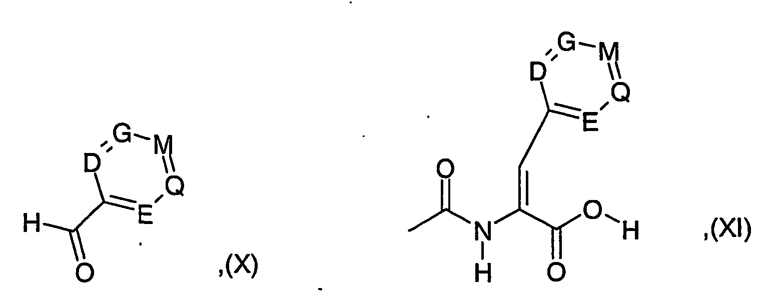

Die primär entstehenden Azlactone werden ohne Isolierung zu den Verbindungen der allgemeinen Formel (XI) hydrolysiert.
Durch weitere Umsetzung in Gegenwart von wässrigen Mineralsäuren, wie Schwefel, Phosphor- oder Chlorwasserstoffsäure, bevorzugt jedoch von Chlorwasserstoffsäure, werden Verbindungen der allgemeinen Formel (XII) erhalten. Diese werden dann mit geeigneten Reduktionsmitteln in die Verbindungen der allgemeinen Formel (VIII) überführt.

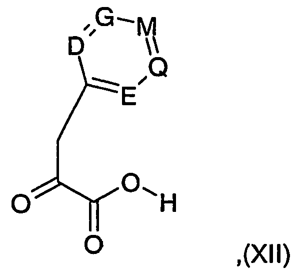

Als Reduktionsmittel können Alkaliborhydride, wie Natrium- oder Kaliumborhydrid verwendet werden. Weitere Reduktionsmittel stellen Chlordialkylborane, wie Chlordicyclohexylboran, dar. Werden chirale Chlordialkylborane, wie z.B. B-Chlordiisopinocampheylboran benutzt, können die Verbindungen der allgemeinen Formel (VIII) in enantiomerenreiner Form isoliert werden.

Ein weiterer Zugang zu Verbindungen der allgemeinen Formel (VIII) besteht in der Alkylierung der Verbindung (XIII) 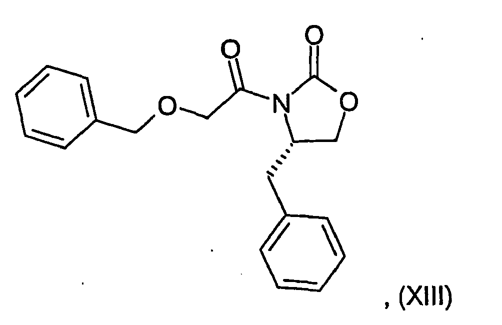 mit Aryl- oder Heteroaryl-methylhalogeniden der allgemeinen Formel 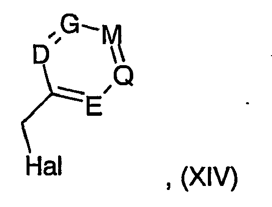 in der Hal ein Chlor-, Brom- oder Jodatom bedeutet, und D, E, G, Q und E wie eingangs erwähnt definiert sind, in Analogie zu literaturbekannten Methoden (Michael T. Crimmins, Kyle A. Emmitte und Jason D. Katz, Org. Lett. 2, 2165-2167 [2000]). Die entstehenden diastereomeren Produkte können dann mit Hilfe physikochemischer Methoden, bevorzugt mit Hilfe chromatographischer Methoden oder Umkristallisation, getrennt werden. Die hydrolytische Abspaltung des chiralen Auxiliars und Abspaltung der Benzylschutzgruppe eröffnet ebenfalls einen Zugang zu enantiomerenreinen Hydroxycarbonsäureverbindungen der allgemeinen Formel (V). Die weitere Umsetzung von Verbindungen der allgemeinen Formel (VIII) zu Verbindungen der allgemeinen Formel (V) erfolgt im alkoholischen Milieu, bevorzugt in Methanol oder Ethanol, in Gegenwart einer geeigneten Säure, wie Chlorwasserstoffsäure. Die Reaktion kann alternativ durch Umsetzung in alkoholischen Lösungsmitteln, bevorzugt Methanol, mit Thionychlorid erfolgen.

Falls die Gruppe X in Verbindungen der allgemeinen Formel (V) das Schwefelatom darstellt, können die für die Synthese benötigten Thiocarbonsäuren der allgemeinen Formel 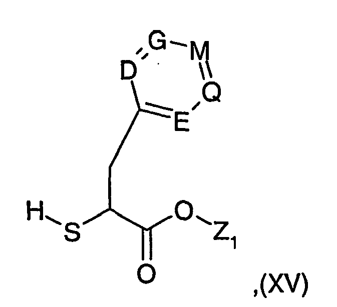 in der D, E, G, M und Q wie eingangs erwähnt definiert sind und Z₁ eine wie unter Verfahren (a) beschriebene Schutzgruppe für eine Carboxygruppe darstellt, aus Verbindungen der allgemeinen Formel (V) gewonnen werden, in derX das Sauerstoffatom darstellt.

Durch eine Mitsunobu-Reaktion der Verbindungen der allgemeinen Formel (V) mit C₁₋₆-Alkylthiocarbonsäuren, wobei die Alkylkette linear oder verzweigt sein kann diese bevorzugt jedoch die Methylgruppe darstellt, werden die entsprechenden Alkylthiocarbonsäureester dieser Verbindungen erhalten. Diese können nach bekannten Methoden zu den Verbindungen der allgemeinen Formel (XV) hydrolysiert werden (Bert Strijtveen und Richard M. Kellogg, J.Org. Chem. 51, 3664-3671 [1986]).

Alle Verbindungen der allgemeinen Formel (I), die primäre oder sekundäre Amino-, Hydroxy- oder Hydroxycarbonylfunktionen enthalten, werden bevorzugt aus mit Schutzgruppen versehenen Vorstufen gewonnen. Als Schutzgruppen für Aminofunktionen kommen beispielsweise eine Benzyloxycarbonyl-, 2-Nitro-benzyloxycarbonyl-, 4-Nitro-benzyloxycarbonyl-, 4-Methoxy-benzyloxycarbonyl-, 2-Chlor-benzyloxycarbonyl-, 3-Chlor-benzyloxycarbonyl-, 4-Chlor-benzyloxycarbonyl-, 4-Biphenylyl-α,α-dimethyl-benzyloxycarbonyl- oder 3,5-Dimethoxy-α,α-dimethyl-benzyloxycarbonylgruppe, eine Alkoxycarbonylgruppe mit insgesamt 1 bis 5 Kohlenstoffatomen im Alkylteil, beispielsweise die Methoxycarbonyl-, Ethoxycarbonyl-, *n*-Propoxycarbonyl-, Isopropoxycarbonyl-, *n*-Butoxycarbonyl-, 1-Methylpropoxycarbonyl-, 2-Methylpropoxy-carbonyl- oder *tert*-Butyloxycarbonylgruppe, die Allyloxycarbonyl-, 2,2,2-Trichlor-(1,1-dimethylethoxy)carbonyl- oder 9-Fluorenylmethoxycarbonylgruppe oder eine Formyl-, Acetyl- oder Trifluoracetylgruppe in Frage.
Als Schutzgruppe für Hydroxyfunktionen kommt beispielsweise eine Trimethylsilyl-, Triethylsilyl-, Triisopropyl-, *tert*-Butyldimethylsilyl- oder *tert*-Butyldiphenylsilylgruppe, eine tert-Butyl-, Benzyl-, 4-Methoxybenzyl- oder 3,4-Dimethoxybenzylgruppe in Frage.
Als Schutzgruppe für Hydroxycarbonylfunktionen kommt beispielsweise eine Alkylgruppe mit insgesamt 1 bis 5 Kohlenstoffatomen, beispielsweise die Methyl-, Ethyl-, *n*-Propyl-, Isopropyl-, *n*-Butyl-, *tert*-Butyl, Allyl-, 2,2,2-Trichlorethyl-, Benzyl- oder 4-Methoxybenzylgruppe in Frage.

Die erhaltenen Verbindungen der allgemeinen Formel (I) können, sofern sie geeignete basische Funktionen enthalten, insbesondere für pharmazeutische Anwendungen in ihre physiologisch verträglichen Salze mit anorganischen oder organischen Säuren übergeführt werden. Als Säuren kommen hierfür beispielsweise Salzsäure, Bromwasserstoffsäure, Phosphorsäure, Salpetersäure, Schwefelsäure, Methansulfonsäure, Ethansulfonsäure, Benzolsulfonsäure, *p*-Toluolsulfonsäure, Essigsäure, Fumarsäure, Bernsteinsäure, Milchsäure, Mandelsäure, Äpfelsäure, Zitronensäure, Weinsäure oder Maleinsäure in Betracht.

Außerdem lassen sich die neuen Verbindungen der Formel (I), falls sie Carbonsäurefunktion enthalten, in ihre Additionssalze mit anorganischen oder organischen Basen, insbesondere für die pharmazeutische Anwendung in ihre physiologisch verträglichen Additionssalze überführen. Als Basen kommen hierfür beispielsweise Natriumhydroxid, Kaliumhydroxid, Ammoniak, Cyclohexylamin, Dicyclohexylamin, Ethanolamin, Diethanolamin und Triethanolamin in Betracht.

Die vorliegende Erfindung betrifft Racemate, sofern die Verbindungen der allgemeinen Formel (I) nur ein Chiralitätselement besitzen. Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel (I) vorhanden ist, sowie die einzelnen optisch aktiven Enantiomeren, aus denen sich die erwähnten Racemate zusammensetzen.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Die neuen Verbindungen der allgemeinen Formel (I) und deren physiologisch verträgliche Salze weisen wertvolle pharmakologische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 ml "Balanced Salts Solution" [BSS (in mM): NaCl 120, KCI 5.4, NaHCO₃ 16.2, MgSO₄ 0.8, NaHPO₄ 1.0, CaCl₂ 1.8, D-Glucose 5.5, HEPES 30, pH 7.40] werden die Zellen zweimal bei 100 x g zentrifugiert und in BSS resuspendiert. Nach Bestimmung der Zellzahl werden die Zellen mit Hilfe eines Ultra-Turrax homogenisiert und für 10 Minuten bei 3000 x g zentrifugiert. Der Überstand wird verworfen und das Pellet in Tris-Puffer (10 mM Tris, 50 mM NaCl, 5 mM MgCl₂, 1 mM EDTA, pH 7.40), angereichert mit 1 % Rinderserum-Albumin und 0.1% Bacitracin, rezentrifugiert und resuspendiert (1 ml / 1000000 Zellen). Das Homogenat wird bei -80°C eingefroren. Die Membranpräparationen sind bei diesen Bedingungen für mehr als 6 Wochen stabil.

Nach Auftauen wird das Homogenat 1:10 mit Assay-Puffer (50 mM Tris, 150 mM NaCl, 5 mM MgCl₂, 1 mM EDTA, pH 7.40) verdünnt und 30 Sekunden lang mit einem Ultra-Turrax homogenisiert. 230 µl des Homogenats werden für 180 Minuten bei Raumtemperatur mit 50 pM ²⁵I-Iodotyrosyl-Calcitonin-Gene-Related Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM humanem CGRP-alpha während der Inkubation definiert.

Die Analyse der Konzentrations-Bindungskurven erfolgt mit Hilfe einer computergestützten nichtlinearen Kurvenanpassung.

Die eingangs erwähnten Verbindungen zeigen in dem beschriebenen Test IC₅₀-Werte ≤ 10000 nM.

### B. CGRP-Antagonismus in SK-N-MC-Zellen

SK-N-MC-Zellen (1 Mio. Zellen) werden zweimal mit 250 µl Inkubationspuffer (Hanks HEPES, 1 mM 3-Isobutyl-1-methylxanthin, 1% BSA, pH-7.4) gewaschen und bei 37°C für 15 Minuten vorinkubiert. Nach Zugabe von CGRP (10 µl) als Agonist in steigenden Konzentrationen (10⁻¹¹ bis 10⁻⁶ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1 M HCl und Zentrifugation (2000 x g, 4°C für 15 Minuten) extrahiert. Die Überstände werden in flüssigem Stickstoff eingefroren und bei -20°C gelagert.

Die cAMP-Gehalte der Proben werden mittels Radioimmunassay (Fa. Amersham) bestimmt und die pA₂-Werte antagonistisch wirkender Substanzen graphisch ermittelt.

Die erfindungsgemäßen Verbindungen zeigen in dem beschriebenen *in vitro*-Testmodell CGRP-antagonistische Eigenschaften in einem Dosisbereich zwischen 10⁻¹² bis 10⁻⁵ M_{.}

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne- bzw. Cluster-Kopfschmerz. Weiterhin beeinflussen die erfindungsgemäßen Verbindungen auch die folgenden Erkrankungen positiv: nicht-insulinabhängigen Diabetes mellitus ("NIDDM"), complex regional pain syndrome (CRPS1), cardiovaskuläre Erkrankungen, Morphintoleranz, Clostritiumtoxin-bedingte Durchfallerkrankungen, Erkrankungen der Haut, insbesondere thermische und strahlenbedingte Hautschäden inklusive Sonnenbrand, entzündliche Erkrankungen, z.B. entzündliche Gelenkerkrankungen (Arthritis), neurogene Entzündungen der oralen Mucosa, entzündliche Lungenerkrankungen, allergische Rhinitis, Asthma, Erkrankungen, die mit einer überschießenden Gefäßerweiterung und dadurch bedingter verringerter Gewebedurchblutung einhergehen, z.B. Schock und Sepsis. Darüber hinaus zeigen die erfindungsgemäßen Verbindungen eine lindernde Wirkung auf Schmerzzustände im allgemeinen.
Die Symptomatik menopausaler, durch Gefäßerweiterung und erhöhten Blutfluß verursachter Hitzewallungen östrogendefizienter Frauen sowie hormonbehandelter Prostatakarzinompatienten wird durch die CGRP-Antagonisten der vorliegenden Anwendung präventiv und akut-therapeutisch günstig beeinflußt, wobei sich dieser Therapieansatz vor der Hormonsubstitution durch Nebenwirkungsarmut auszeichnet.

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils 1 bis 3 x täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Die erfindungsgemäß hergestellten Verbindungen können entweder alleine oder gegebenenfalls in Kombination mit anderen Wirksubstanzen zur Behandlung von Migräne intravenös, subkutan, intramuskulär, intrarektal, intranasal, durch Inhalation, transdermal oder oral erfolgen, wobei zur Inhalation insbesondere Aerosolformulierungen geeignet sind. Die Kombinationen können entweder simultan oder sequentiell verabreicht werden.

Als Kombinationspartner denkbare Wirkstoffklassen sind z.B. Antiemetica, Prokinetica, Neuroleptica, Antidepressiva, Neurokinin-Antagonisten, Angiotensin Rezeptor Blocker (Angiotensin II Antagonisten), iNOS Inhibitoren, AMPA Antagonisten, Anticonvulsiva, Histamin-H1-Rezeptorantagonisten, Antimuscarinika, β-Blocker, α-Agonisten und α-Antagonisten, Ergotalkaloiden, schwachen Analgetica, nichtsteroidalen Antiphlogistica, Corticosteroiden, Calcium-Antagonisten, 5-HT_{1B/1D}-Agonisten oder andere Antimigränemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetylsalicylsäure, Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib und Celecoxib, in Betracht.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Topiramat, Riboflavin, Montelukast, Lisinopril, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

Ein weiterer Gegenstand der Erfindung sind Verdindungen zur Verwendung als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### Experimenteller Teil

Für hergestellte Verbindungen liegen in der Regel IR-, ¹H-NMR und/oder Massenspektren vor.

Wenn nicht anders angegeben, werden R_{f}-Werte unter Verwendung von DC-Fertigplatten Kieselgel 60 F254 (E. Merck, Darmstadt, Artikel-Nr. 1.05714) ohne Kammersättigung bestimmt.
Die unter der Bezeichnung Polygram ermittelten Rₜ-Werte werden unter Verwendung von DC-Fertigfolien Polygram SIL G/UV₂₅₄ (beschichtet mit 0.2 mm Kieselgel) der Firma Macherey-Nagel (Düren, Artikel-Nr. 805 021) erhoben.
Die unter der Bezeichnung Polygram-Alox ermittelten R_{f}-Werte werden unter Verwendung von DC-Fertigfolien Polygram Alox N/UV₂₅₄ (beschichtet mit 0.2 mm Aluminiumoxid) der Firma Macherey-Nagel (Düren, Artikel-Nr. 802 021) erhoben.
Die bei den Fliessmitteln angegebenen Verhältnisse beziehen sich auf Volumeneinheiten der jeweiligen Lösungsmittel. Die angegebenen Volumeneinheiten bei NH₃ beziehen sich auf eine konzentrierte Lösung von NH₃ in Wasser.
Soweit nicht anders vermerkt sind die bei den Aufarbeitungen der Reaktionslösungen verwendeten Säure-, Basen- und Salzlösungen wässrige Systeme der angebenen Konzentrationen.
Zu chromatographischen Reinigungen wird Kieselgel der Firma Millipore (MATREX*^{™}*, 35-70 µm) verwendet.
Zu chromatographischen Reinigungen wird Aluminiumoxid der Firma ICN Biomedicals (Eschwege, Artikelnummer 02090) verwendet. Gemäss Herstellerangaben wird vor Verwendung die benötigte Aktivitätsstufe erzeugt.
Die angegebenen HPLC-Daten werden unter nachstehend angeführten Parametern gemessen:

### Methode A:

| Zeit (min) | Volumenprozent Wasser (mit 0.1 % Ameisensäure) | Volumenprozent Acetonitril (mit 0.1 % Ameisensäure) |
|---|---|---|
| 0 | 95 | 5 |
| 9 | 10 | 90 |
| 10 | 10 | 90 |
| 11 | 95 | 5 |

Analytische Säule: Zorbax-Säule (Agilent Technologies), SB (Stable Bond) C18; 3.5 µm; 4.6 x 75 mm; Säulentemperatur: 30°C; Fluss: 0.8 mL / min; Injektionsvolumen: 5 µL; Detektion bei 254 nm

### Methode B:

| Zeit (min) | Volumenprozent Wasser (mit 0.1 % Ameisensäure) | Volumenprozent Acetonitril (mit 0.1 % Ameisensäure) |
|---|---|---|
| 0 | 95 | 5 |
| 9 | 10 | 90 |
| 10 | 10 | 90 |
| 11 | 95 | 5 |

Analytische Säule: Waters Symmetry C18; 3.5 µm; 4.6 x 75 mm; Säulentemperatur: 30°C; Fluss: 0.8 mL / min; Injektionsvolumen: 5 µL; Detektion bei 254 nm

### Methode C:

| Zelt (min) | Volumenprozent Wasser (mit 0.1 % Ameisensäure) | Volumenprozent Acetonitril (mit 0.1 % Ameisensäure) |
|---|---|---|
| 0 | 95 | 5 |
| 8 | 50 | 50 |
| 9 | 10 | 90 |
| 10 | 10 | 90 |
| 11 | 90 | 10 |

Analytische Säule: Zorbax-Säule (Agilent Technologies), Bonus-RP C14; 3.5 µm; 4.6 x 75 mm; Säulentemperatur: 30°C; Fluss: 0.8 mL / min; Injektionsvolumen: 5 µL; Detektion bei 254 nm

### Methode D:

| Zeit (min) | Volumenprozent Wasser (mit 0.1 % Ameisensäure) | Volumenprozent Acetonitril (mit 0.1 % Ameisensäure) |
|---|---|---|
| 0 | 95 | 5 |
| 8 | 50 | 50 |
| 9 | 10 | 90 |
| 10 | 10 | 90 |
| 11 | 90 | 10 |

Analytische Säule: Zorbax-Säule (Agilent Technologies), SB (Stable Bond) C18; 3.5 µm; 4.6 x 75 mm; Säulentemperatur: 30°C; Fluss: 0.8 mL / min; Injektionsvolumen: 5 µL; Detektion bei 254 nm

### Methode E:

| Zeit (min) | Volumenprozent Wasser (mit 0.1 % Ameisensäure) | Volumenprozent Acetonitril (mit 0.1 % Ameisensäure) |
|---|---|---|
| 0 | 95 | 5 |
| 4.5 | 10 | 90 |
| 5 | 10 | 90 |
| 5.5 | 90 | 10 |

Analytische Säule: Zorbax-Säule (Agilent Technologies), SB (Stable Bond) C18; 3.5 µm; 4.6 x 75 mm; Säulentemperatur: 30°C; Fluss: 1.6 mL / min; Injektionsvolumen: 5 µL; Detektion bei 254 nm

### Methode F:

| Zeit (min) | Volumenprozent Wasser (mit 0.04% Trifluoressigsäure) | Volumenprozent Acetonitril (mit 0.04% Trifluoressigsäure) |
|---|---|---|
| 0 | 80 | 20 |
| 30 | 20 | 80 |

Analytische Säule: Waters Symmetry C8; 5 µm; 4.6 x 150 mm; Säulentemperatur: 25°C; Fluss: 1.3 mL / min; Injektionsvolumen: 5 µL; Detektion bei 254 nm

Bei präparativen HPLC-Reinigungen werden in der Regel die gleichen Gradienten verwendet, die bei der Erhebung der analytischen HPLC-Daten benutzt wurden.
Die Sammlung der Produkte erfolgt massengesteuert, die Produkt enthaltenden Fraktionen werden vereinigt und gefriergetrocknet.
Falls nähere Angaben zur Konfiguration fehlen, bleibt offen, ob es sich um reine Enantiomere handelt oder ob partielle oder gar völlige Racemisierung eingetreten ist.

In den Versuchsbeschreibungen werden die folgenden Abkürzungen verwendet:
- Boc: *tert-*Butoxycarbonyl
- Cyc: Cyclohexan
- DCM: Dichlormethan
- DIPE: Diisopropylether
- DMF: *N,N*-Dimethylformamid
- EtOAc: Essigsäureethylester
- EtOH: Ethanol
- Fmoc: 9-Fluorenylmethoxycarbonyl
- halbkonz.: Halbkonzentriert
- HATU: O-(7-Azabenztriazol-1-yl)-N,N,N',N'-tetramethyluronium-hexafluoro- ' phosphat
- HCl: Salzsäure
- HOAc: Essigsäure
- HOBt: 1-Hydroxybenzotriazol-hydrat
- i. vac.: in vacuo (im Vakuum)
- KOH: Kaliumhydroxid
- konz.: konzentriert
- LiOH: Lithiumhydroxid
- MeOH: Methanol
- NaOAc: Natriumacetat
- NaCl: Natriumchlorid
- NaOH: Natriumhydroxid
- n.e.: nicht erhoben
- PE: Petrolether
- RT: Raumtemperatur
- TBME: *tert*-Butylmethylether
- TBTU: O-(Benzotriazol-1-yl)-N,N,N',N'-tetramethyluronium-tetrafluoroborat
- TFA: Trifluoressigsäure
- THF: Tetrahydrofuran

### Beispiel 1

### 4-(2-Oxo-1,2,4,5-tetrahydro-1,3-benzdiazepin-3-yl)-piperidin-carbonsäure-(R)-1-(3,4-dibrom-benzyl)-2-[4-(1-methyl-piperidin-4-yl)-piperazin-1-yl]-2-oxo-ethylester

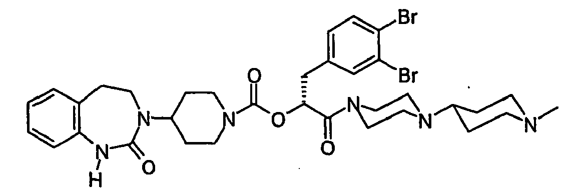

### 1a (Z,E)-2-Acetylamino-3-(3,4-dibrom-phenyl)-acrylsäure

Eine Mischung aus 22.1 g (83.7 mmol) 3,4-Dibrombenzaldehyd, 14.7 g (126 mmol) N-Acetylglycin und 10.3 g (126 mmol) NaOAc in 100 mL Acetanhydrid wurden 1.5 h auf 118°C (Innentemperatur) erhitzt. Nach beendeter Umsetzung wurde das Reaktionsgemisch auf 100°C gekühlt und dann portionenweise mit 20 g Eis versetzt (exotherme Reaktion), wobei die Innentemperatur unter 120°C gehalten wurde. Das Reaktionsgemisch wurde weitere 2 h auf 95°C erhitzt, dann zu einer Mischung aus 240 mL Wasser und 120 mL Toluol gegeben und 1 h bei RT nachgerührt. Der Niederschlag wurde abgesaugt, mit jeweils 50 mL Toluol und Wasser nach gewaschen und über Nacht bei 40°C im Umlufttrockenschrank getrocknet.

| | |
|---|---|
| Ausbeute: | 20.8 g (69% der Theorie) |
| ESI-MS: | (M+H)⁺ = 362/364/366 (2 Br) |
| R_{f} = | 0.19 (Kieselgel, EtOAc/MeOH/NH₃ 90:10:1) |

### 1 b 3-(3,4-Dibrom-phenyl)-2-oxo-propionsäure

Zu einer Lösung von 11.98 g (32.82 mmol) (Z,E)-2-Acetylamino-3-(3,4-dibromphenyl)-acrylsäure in 90 mL N-Methyl-2-pyrrolidinon wurden 125 mL eisgekühlte 4 M HCl zugegeben und das Reaktionsgemisch anschliessend für 2 h unter Rückfluss erhitzt. Die auf 40°C gekühlte Reaktionslösung wurde auf 450 mL Wasser gegossen, die entstandene Suspension mit 300 mL Toluol versetzt und über Nacht nachgerührt. Die organische Phase wurde solange mit Wasser extrahiert bis sich zwischen den Phasen ein Niederschlag bildete. Dieser wurde abgesaugt, die Phasen getrennt, die Toluolphase zur Hälfte eingeengt, diese erneut mit Wasser versetzt und der entstandene Niederschlag abgesaugt. Dieser wurde dann mit dem ersten Niederschlag vereinigt und bei 50°C im Umlufttrockenschrank getrocknet.

| | |
|---|---|
| Ausbeute: | 5.73 g (54% der Theorie) |
| ESI-MS: | (M+H)⁺ = 319/321/323 (2 Br) |
| R_{f} = | 0.17 (Kieselgel, EtOAc/MeOH/NH₃ 80:20:2) |

### 1c (R)-3-(3,4-Dibrom-phenyl)-2-hydroxy-propionsäure

Zu einer auf -35°C gekühlten Lösung von 5.1 g (15.8 mmol) 3-(3,4-Dibrom-phenyl)-2-oxo-propionsäure und 2.2 mL (15.8 mmol) Triethylamin in 20 mL THF wurden innerhalb von 30 min eine Lösung von 6.1 g (19.0 mmol) (1R)-B-Chlordiisopinocampheylboran in 40 mL THF zugetropft und das Reaktionsgemisch 1 h bei dieser Temperatur gehalten. Die Reaktionslösung wurde vorsichtig mit 30 mL 1 M NaOH (exotherm) und 30 mL TBME versetzt, 15 min nachgerührt, die organische Phase abgetrennt, diese dann mit 25 mL Wasser und 15 mL 1 M NaOH extrahiert. Die vereinigten wässrigen Phasen wurden mit 2 M HCl sauer gestellt, dreimal mit jeweils 40 mL TBME extrahiert und die vereinigten organischen Phasen über Na₂SO₄ getrocknet. Nach Entfernen des Trocken- und Lösungsmittels wurde der Rückstand chromatographisch (Kieselgel, EtOAc/MeOH/NH₃ 70:30:3) gereinigt.

| | |
|---|---|
| Ausbeute: | 3.2 g (63% der Theorie) |
| ESI-MS: | (M+H)⁺ = 321/323/325 (2 Br) |
| Retentionszeit (HPLC): | 7.0 min (Methode A) |

### 1d (R)-3-(3,4-Dibrom-phenyl)-2-hydroxy-propionsäureethylester

Zu einer auf 0°C gekühlten Lösung von 3.2 g (9.9 mmol) (R)-3-(3,4-Dibrom-phenyl)-2-hydroxy-propionsäure in 40 mL trockenem EtOH wurden tropfenweise 0.8 mL (10.9 mmol) Thionylchlorid zugegeben und das Reaktionsgemisch 1 h bei RT nachgerührt. Die Reaktionslösung wurde i.vac. eingeengt, der Rückstand mit 30 mL DCM versetzt und vom unlöslichen Niederschlag filtriert. Nach Entfernen des Lösungsmittels erhielt man das Produkt als zähes Öl, welches ohne Reinigung weiter umgesetzt wurde.

| | |
|---|---|
| Ausbeute: | 3.1 g (88% der Theorie) |
| ESI-MS: | (M+H)⁺ = 351/353/355 (2 Br) |
| Retentionszeit (HPLC): | 8.1 min (Methode A) |

### 1e 4-(2-Oxo-1,2,4,5-tetrahydro-1,3-benzdiazepin-3-yl)-piperidin-1-carbonylchlorid

Zu einer auf 0°C gekühlten Lösung von 2.5 g (10.2 mmol) 3-Piperidin-4-yl-1,3,4,5-tetrahydro-1,3-benzdiazepin-2-on und 2.6 mL (14.9 mmol) Ethyldiisopropylamin in 75 mL DCM wurden 6.0 g (12.1 mmol) Phosgen (20 Gewichtsprozent in Toluol) zugegeben und das Reaktionsgemisch 30 min bei dieser Temperatur nachgerührt. Die Reaktionslösung wurde auf RT erwärmt, i.vac. auf ca. 50 mL eingeengt, über Kieselgel filtriert, dieses mit 200 mL DCM/EtOAc (1:1) gewaschen und die vereinigten organischen Filtrate erneut i.vac. eingeengt. Der Rückstand wurde mit DIPE verrührt, abgesaugt und i.vac. getrocknet.

| | |
|---|---|
| Ausbeute: | 2.4 g (77% der Theorie) |
| R_{f} = | 0.43 (Kieselgel, DCM/EtOAc 1:1) |

### 1 f 4-(2-Oxo-1,2,4,5-tetrahydro-1,3-benzdiazepin-3-yl)-piperidin-1-carbonsäure-(R)-2-(3,4-dibrom-phenyl)-1-ethoxycarbonyl-ethylester

Zu einer auf 0°C gekühlten Lösung von 2.90 g (8.24 mmol) (R)-3-(3,4-Dibromphenyl)-2-hydroxy-propionsäureethylester in 50 mL trockenem THF wurden 362 mg (55% in Mineralöl, 9.06 mmol) NaH portionenweise zugegeben und weitere 30 min bei dieser Temperatur nachgerührt, wobei sich eine dunkelbraune Suspension bildete. Anschliessend wurden portionenweise unter Kühlung 2.15 g (6.99 mmol) 4-(2-Oxo-1,2,4,5-tetrahydro-1,3-benzdiazepin-3-yl)-piperidin-1-carbonylchlorid zugegeben und das Reaktionsgemisch 2 bei RT gerührt. Man versetzte mit 50 mL halbgesättigter NaHCO₃-Lösung, extrahierte zweimal mit jeweils 50 mL EtOAc, wusch die vereinigten organischen Phasen mit 50 mL gesättigter NaCl-Lösung und filtrierte die organische Phase über Na₂SO₄. Nach Entfernen des Lösungsmittels wurde der Rückstand chromatographisch (Kieselgel, EtOAc/Cyc 3:1) gereinigt.

| | |
|---|---|
| Ausbeute: | 3.64 g (84% der Theorie) |
| ESI-MS: | (M+H)⁺ = 622/624/626 (2 Br) |
| Retentionszeit (HPLC): | 10.0 min (Methode A) |

### 1 g 4-(2-Oxo-1,2,4,5-tetrahydro-1,3-benzdiazepin-3-yl)-piperidin-1-carbonsäure-(R)-1-carboxy-2-(3,4-dibrom-phenyl)-ethylester

Zu einer Lösung von 3.64 g (5.83 mmol) 4-(2-Oxo-1,2,4,5-tetrahydro-1,3-benzdiazepin-3-yl)-piperidin-1-carbonsäure-(R)-2-(3,4-dibrom-phenyl)-1-ethoxycarbonyl-ethylester in 70 mL THF wurde bei RT eine Lösung von 210 mg (9.0 mmol) LiOH*H₂O in 40 mL Wasser zugegeben und das Reaktionsgemisch 7 h bei RT gerührt. Man engte i.vac. ein, nahm den Rückstand in 100 mL Wasser auf, versetzte unter Rühren mit 1 M HCl bis zur sauren Reaktion, filtrierte den Niederschlag ab und trocknete diesen im Vakuumtrockenschrank bei 50°C. Das Produkt wurde ohne Reinigung weiter umgesetzt.

| | |
|---|---|
| Ausbeute: | 3.36 g (97% der Theorie) |
| ESI-MS: | (M+H)⁺ = 594/596/598 (2 Br) |
| Retentionszeit (HPLC): | 8.5 min (Methode A) |

### 1 h 4-(2-Oxo-1,2,4,5-tetrahydro-1,3-benzdiazepin-3-yl)-piperidin-1-carbonsäure-(R)-1-(3,4-dibrom-benzyl)-2-[4-(1-methyl-piperidin-4-yl)-piperazin-1-yl]-2-oxo-ethylester

Eine Lösung von 80 mg (0.13 mmol) 4-(2-Oxo-1,2,4,5-tetrahydro-1,3-benzdiazepin-3-yl)-piperidin-1-carbonsäure-(R)-1-carboxy-2-(3,4-dibrom-phenyl)-ethylester, 43.2 mg (0.13 mmol) TB-TU und 37-µL (0.27-mmol) -Triethylamin in 1.5 mL DMF wurde 1 h bei RT gerührt. Dann erfolgte die Zugabe von 24.9 mg (0.134 mmol) 1-(1-Methylpiperidin-4-yl)-piperazin und das Reaktionsgemisch wurde anschliessend über Nacht bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.

| | |
|---|---|
| Ausbeute: | 87.6 mg (87% der Theorie) |
| ESI-MS: | (M+H)⁺ = 759/761/763 (2 Br) |
| Retentionszeit (HPLC): | 5.0 min (Methode A) |

Analog wurden folgende Verbindungen aus jeweils 80 mg 4-(2-Oxo-1,2,4,5-tetrahydro-1,3-benzdiazepin-3-yl)-piperidin-1-carbonsäure-(R)-2-(3,4-dibrom-phenyl)-1-hydroxycarbonyl-ethylester und der entsprechenden Menge an Amin hergestellt:

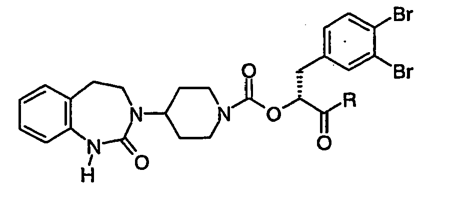

| **Beispiel** | **R** | **Ausbeute (%)** | **Massenspektrum** | **Retentionszeit HPLC (Methode)** |
|---|---|---|---|---|
| **2** | 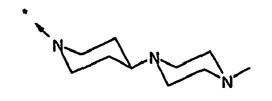 | 69 | 759/761/763 [M+H]⁺ | 5.6 min (A) |
| **3** | 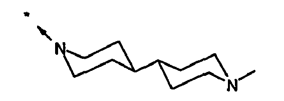 | 72 | 758/760/762 [M+H]⁺ | 6.0 min (A) |
| **4** | 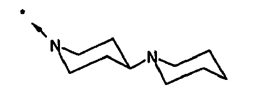 | 59 | 744/746/748 [M+H]⁺ | 6.2 min (A) |
| **5** | 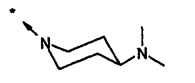 | 71 | 704/706/708 [M+H]⁺ | 5.8 min (A) |
| **6** | 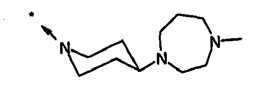 | 21 | 773/775/777 [M+H]⁺ | 5.4 min (A) |
| **7** | 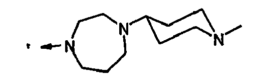 | 70 | 773/775/777 [M+H]⁺ | 5.1 min (A) |
| **8** | 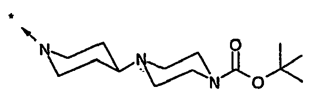 | 69 | 845/847/849 [M+H]⁺ | 6.6 min (A) |
| **9** | 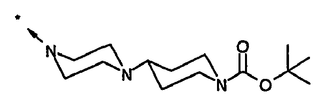 | 59 | 845/847/849 [M+H]⁺ | 6.7 min (A) |

Analog können folgende Verbindungen aus 4-(2-Oxo-1,2,4,5-tetrahydro-1,3-benzdiazepin-3-yl)-piperidin-1-carbonsäure-(R)-2-(3,4-dibrom-phenyl)-1-hydroxycarbonyl-ethylester und der entsprechenden Menge an Amin hergestellt werden:

| **Beispiel** | **R** |
|---|---|
| **10** | 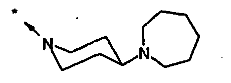 |
| **11** | 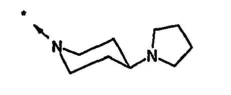 |
| **12** | 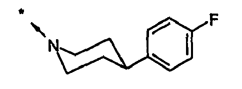 |
| **13** | 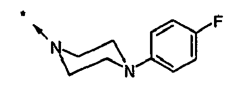 |
| **14** | 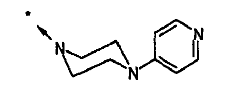 |
| **15** | 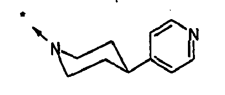 |
| **16** | 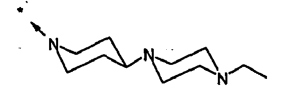 |
| **17** | 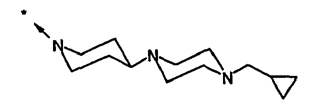 |
| **18** | 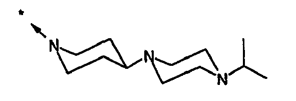 |

### Beispiel 19

### 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-piperidin-4-yl-piperazin-1-yl)-ethylester

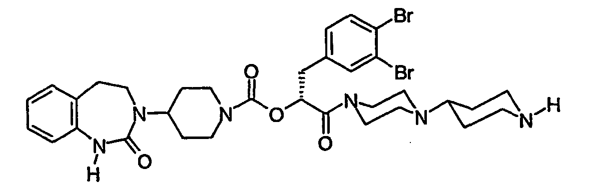

Eine Lösung von 125 mg (0.15 mmol) 4-(2-Oxo-1,2,4,5-tetrahydro-1,3-benzdiazepin-3-yl)-piperidin-1-carbonsäure-(R)-1-(3,4-dibrom-benzyl)-2-[4-(1-*tert*-butoxycarbonyl-piperidin-4-yl)-piperazin-1-yl]-2-oxo-ethylester in 20 mL 2 M HCl wurde 16 h bei RT gerührt. Das Reaktionsgemisch wurde lyophilisiert, wobei das Produkt als Bis-Hydrochlorid Salz anfiel.
- Ausbeute:: 110 mg (91% der Theorie)
- ESI-MS:: (M+H)⁺ = 745/747/749 (2 Br)
- Retentionszeit (HPLC):: 5.4 min (Methode A)

### Beispiel 20

### 4-(2-Oxo-1,2,4,5-tetrahydro-1,3-benzdiazepin-3-yl)-piperidin-1-carbonsäure-(R)-1-(3,4-dibrom-benzyl)-2-oxo-2-(4-piperazin-1-yl-piperidin-1-yl)-ethylester

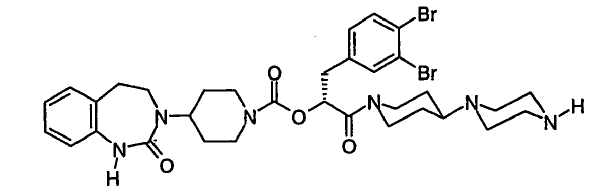

Eine Lösung von 79 mg (0.09 mmol) 4-(1-{(R)-3-(3,4-Dibrom-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-*tert*-butylester in 15 mL 2 M HCl wurde 16 h bei RT gerührt. Das Reaktionsgemisch wurde lyophilisiert, wobei das Produkt als Bis-Hydrochlorid Salz anfiel.
- Ausbeute:: 76 mg (100% der Theorie)
- ESI-MS:: (M+H)⁺ = 745/747/749 (2 Br)
- Retentionszeit (HPLC):: 5.7 min (Methode A)

### Beispiel 21

### 4-(2-Oxo-1,2,4,5-tetrahydro-1,3-benzdiazepin-3-yl)-piperidin-1-carbonsäure-(R)-1-(3,4-dichlor-benzyl)-2-[4-(1-methyl-piperidin-4-yl)-piperazin-1-yl]-2-oxo-ethylester

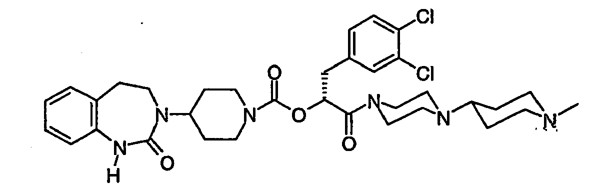

### 21a 2-Acetylamino-3-(3,4-dichlor-phenyl)-acrylsäure

Eine Mischung aus 20.0 g (112 mmol) 3,4-Dichlorbenzaldehyd, 19.7 g (168 mmol) N-Acetylglycin und 13.8 g (168 mmol) NaOAc in 80 mL Acetanhydrid wurden 5 h auf 120°C (Ölbadtemperatur) erhitzt. Nach beendeter Umsetzung wurde das Reaktionsgemisch mittels Eisbad abgekühlt und dann langsam mit 60 mL Wasser versetzt (leicht exotherme Reaktion). Das Reaktionsgemisch wurde weitere 1.5 h auf 80°C erhitzt, etwas abgekühlt, dann zu einer Mischung aus 400 mL Wasser und 200 mL Toluol gegeben und über Nacht bei RT nachgerührt. Der Niederschlag wurde abgesaugt, mit Toluol und Wasser nachgewaschen, anschließend mit Diethylether versetzt und abgesaugt.
- Ausbeute:: 21.0 g (68% der Theorie)
- ESI-MS:: (M+H)⁺ = 274/276/278 (2 Cl)
- R_{f} =: 0.16 (Kieselgel, DCM/MeOH/NH₃ 80:20:2)

### 21 b 3-(3,4-Dichlor-phenyl)-2-oxo-propionsäure

Zu einer Suspension von 21.0 g (76.6 mmol) 2-Acetylamino-3-(3,4-dichlor-phenyl)-acrylsäure in 100 mL N-Methyl-2-pyrrolidinon wurden 140 mL 4 M HCl zugegeben und das Reaktionsgemisch anschliessend für 4 h bei einer Ölbadtemperatur von 125°C erhitzt. Die abgekühlte Reaktionslösung wurde auf ein gekühltes Gemisch aus 350 mL Wasser und 120 mL Toluol gegossen. Die Phasen wurden getrennt, die wässrige Phase nochmals mit Toluol extrahiert, die vereinigten organischen Phasen mit Wasser extrahiert, über Na₂SO₄ filtriert und i. vac. eingeengt. Der Rückstand wurde in 1 M NaOH aufgenommen und zweimal mit Diethylether gewaschen. Die wässrige Phase wurde mit 2 M HCl sauer gestellt und dreimal mit EtOAc extrahiert. Die vereinigten organischen Phasen wurden über Na₂SO₄ filtriert und i. vac. eingeengt. Der Rückstand wurde mit Diethylether versetzt, abgesaugt und im Vakuumtrockenschrank getrocknet.
- Ausbeute:: 8.20 g (46% der Theorie)
- ESI-MS:: (M+H)⁺ = 231/233/235 (2 Cl)
- R_{f} =: 0.11 (Kieselgel, DCM/MeOH/Cyc/NH₃ 70:15:15:2)

### 21 c (R)-3-(3,4-Dichlor-phenyl)-2-hydroxy-propionsäure

Zu einer auf -35°C gekühlten Lösung von 8.0 g (34.3 mmol) 3-(3,4-Dichlor-phenyl)-2-oxo-propionsäure und 5.2 mL (38.0 mmol) Triethylamin in 40 mL THF wurden innerhalb von 30 min eine Lösung von 12.2 g (38.0 mmol) (1R)-B-Chlordiisopinocampheylboran in 20 mL THF zugetropft und das Reaktionsgemisch 1 h bei dieser Temperatur gehalten. Das Kühlbad wurde entfernt und die Reaktionslösung 4 h bei RT gerührt. Anschließend wurde die Reaktionslösung bei 5-10°C vorsichtig mit 50 mL 1 M NaOH (exotherm) und 30 mL TBME versetzt und 15 min nachgerührt. Die organische Phase wurde abgetrennt, mit 25 mL Wasser und 15 mL 1 M NaOH extrahiert. Die vereinigten wässrigen Phasen wurden mit 2 M HCl sauer gestellt und dreimal mit jeweils 40 mL TBME extrahiert. Die vereinigten organischen Phasen wurden über Na₂SO₄ getrocknet und i. vac. eingeengt. Der Rückstand wurde in 80 mL kochendem Wasser gelöst und über Celite abgesaugt. Das Filtrat wurde mit NaCl gesättigt und dreimal mit EtOAc extrahiert. Die vereinigten organischen Phasen wurden über Na₂SO₄ filtriert und erneut i. vac. eingeengt. Das Rohprodukt wurde ohne Reingung weiter umgesetzt.
- Ausbeute:: 3.9 g (48% der Theorie)
- ESI-MS:: (M+H)⁺ = 233/235/327 (2 Cl)
- Retentionszeit (HPLC):: 6.8 min (Methode A)
- R_{f} =: 0.87 (Kieselgel, DCM/MeOH/Cyc/NH₃ 70:15:15:2)

### 21d (R)-3-(3,4-Dichlor-phenyl)-2-hydroxy-propionsäureethylester

Zu einer Lösung von 3.5 g (14.9 mmol) (R)-3-(3,4-Dichlor-phenyl)-2-hydroxy-propionsäure in 50 mL EtOH wurden 50 mL ethanolische HCl zugegeben und das Reaktionsgemisch 4 h bei RT nachgerührt. Die Reaktionslösung wurde i. vac. eingeengt, der Rückstand mit DCM versetzt, mit 15% K₂CO₃-Lösung extrahiert und die organische Phase über Na₂SO₄ getrocknet. Nach Entfernen des Trocken- und Lösungsmittels erhielt man das Produkt als Öl, welches ohne Reinigung weiter umgesetzt wurde.
- Ausbeute:: 2.6 g (66% der Theorie)
- ESI-MS:: (M+H)⁺ = 263/265/267 (2 Cl)
- R_{f} =: 0.91 (Kieselgel, DCM/MeOH/Cyc/NH₃ 70:15:15:2)

### 21 e 4-(2-Oxo-1,2,4,5-tetrahydro-1,3-benzdiazepin-3-yl)-piperidin-1-carbonsäure-(R)-2-(3,4-dichlor-phenyl)-1-ethoxycarbonyl-ethylester

Zu einer auf 0°C gekühlten Lösung von 2.6 g (9.9 mmol) (R)-3-(3,4-Dichlor-phenyl)-2-hydroxy-propionsäureethylester in 50 mL THF wurden 450 mg (55% in Mineralöl, 10.3 mmol) NaH portionenweise zugegeben und weitere 30 min bei dieser Temperatur nachgerührt. Anschliessend wurden portionenweise unter Kühlung 3.7 g (11.9 mmol) 4-(2-Oxo-1,2,4,5-tetrahydro-1,3-benzdiazepin-3-yl)-piperidin-1-carbonylchlorid zugegeben und das Reaktionsgemisch über Nacht bei RT gerührt. Die Reaktionslösung wurde i.vac. eingeengt, der Rückstand mit DCM versetzt, die organische Phase abgetrennt, diese mit 10% Zitronensäure-Lösung und 15% K₂CO₃-Lösung gewaschen und über Na₂SO₄ getrocknet. Nach Entfernen des Trocken- und Lösungsmittels erhielt man das Produkt, welches ohne weitere Reinigung umgesetzt wurde.
- Ausbeute:: 5.2 g (98% der Theorie)
- ESI-MS:: (M+H)⁺ = 534/536/538 (2 Cl)
- R_{f} =: 0.77 (Kieselgel, DCM/MeOH/Cyc/NH₃ 70:15:15:2)

### 21f f 4-(2-Oxo-1,2,4,5-tetrahydro-1,3-benzdiazepin-3-yl)-piperidin-1-carbonsäure-1-carboxy-(R)-2-(3,4-dichlor-phenyl)-ethylester

Zu einer Lösung von 5.2 g (9.7 mmol) 4-(2-Oxo-1,2,4,5-tetrahydro-1,3-benzodiazepin-3-yl)-piperidin-1-(R)-carbonsäure-2-(3,4-dichlor-phenyl)-1-ethoxy-carbonyl-ethylester in 30 mL THF wurde bei RT eine Lösung von 348 mg (14.5 mmol) Lithiumhydroxid in 10 mL Wasser zugegeben und das Reaktionsgemisch über Nacht bei RT gerührt. Man engte i. vac. ein, nahm den Rückstand in 15% K₂CO₃-Lösung auf und wusch die wässrige Phase dreimal mit EtOAc. Die wässrige Phase versetzte man unter Rühren mit 5 M HCl bis zur sauren Reaktion und extrahierte erschöpfend mit DCM. Die vereinigten organischen Phasen filtrierte man über Na₂SO₄ und entfernte das Lösungsmittel i. vac. Man nahm den Rückstand in Isopropanol auf und filtrierte den Niederschlag ab. Das Filtrat engte man i.vac. ein, reinigte den Rückstand chromatographisch (Kieselgel, Gradient DCM/MeOH/NH₃ 10:0:0 zu 75:25:5), vereinigte die entsprechende Fraktionen, entfernte das Lösungsmittel, versetzte den Rückstand mit Diethylether und saugte ab.
- Ausbeute:: 2.2 g (45% der Theorie)
- ESI-MS:: (M+H)⁺ = 506/508/510 (2 Cl)
- R_{f}=: 0.51 (Kieselgel, DCM/MeOH/Cyc/NH₃ 70:15:15:2)

### 21g 4-(2-Oxo-1,2,4,5-tetrahydro-benzodiazepin-3-yl)-piperidin-1-carbonsäure-(R)-1-(3,4-dichlor-benzyl)-2-[4-(1-methyl-piperidin-4-yl)-piperazin-1-yl]-2-oxo-ethylester

Eine Lösung von 80 mg (0.16 mmol) 4-(2-Oxo-1,2,4,5-tetrahydro-1,3-benzdiazepin-3-yl)-piperidin-1-carbonsäure-1-carboxy-(R)-2-(3,4-dichlor-phenyl)-ethylester, 58 mg (0.18 mmol) TBTU und 25 µL (0.18 mmol) Triethylamin in 2 mL DMF wurde 10 min bei RT gerührt. Dann erfolgte die Zugabe von 33 mg (0.18 mmol) 1-(1-Methylpiperidin-4-yl)-piperazin und das Reaktionsgemisch wurde über Nacht bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.
- Ausbeute:: 35.0 mg (33% der Theorie)
- ESI-MS:: (M+H)⁺ = 671/673/675 (2 Cl)
- Retentionszeit (HPLC):: 5.3 min (Methode A)

Analog wurden folgende Verbindungen aus jeweils 80 mg (Beispiele 22 bis 24) bzw. aus jeweils 160 mg (Beispiele 25 bis 27) 4-(2-Oxo-1,2,4,5-tetra-hydro-1,3-benzdiazepin-3-yl)-piperidin-1-carbonsäure-1-carboxy-(R)-2-(3,4- dichlor-phenyl)-ethylester und der entsprechenden Menge an Amin hergestellt:

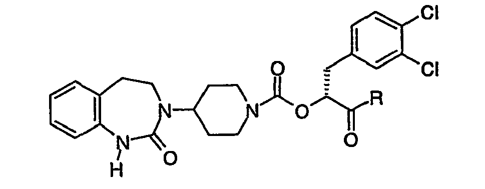

| **Beispiel** | **R** | **Ausbeute (%)** | **Massenspektrum** | **Retentionszeit HPLC (Methode)** |
|---|---|---|---|---|
| **22** | 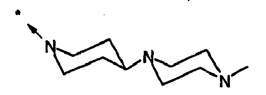 | 33 | 671/673/675 [M+H]⁺ | 5.8 min (A) |
| **23** | 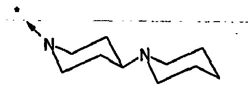 | 29 | 656/658/660 [M+H]⁺ | 6.5 min (A) |
| **24** | 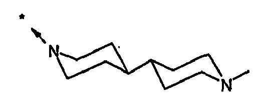 | 38 | 670/672/674 [M+H]⁺ | 6.2 min (A) |
| **25** | 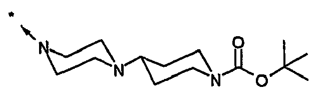 | 13 | 757/759/761 [M+H]⁺ | 7.0 min (A) |
| **26** | 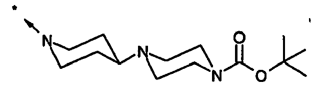 | 15 | 757/759/761 [M+H]⁺ | 6.9 min (A) |
| **27** | 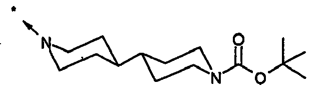 | 13 | n.e. | 5.0 min (A) |

### Beispiel 28

### 4-(2-Oxo-1,2,4,5-tetrahydro-1,3-benzdiazepin-3-yl)-piperidin-1-carbonsäure-(R)-1-(3,4-dichlor-benzyl)-2-oxo-2-(4-piperidin-4-yl-piperazin-1-yl)-ethylester

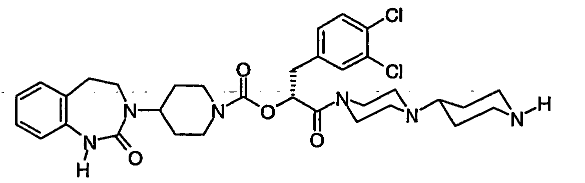

Eine Lösung von 30 mg (0.04 mmol) 4-(2-Oxo-1,2,4,5-tetrahydro-1,3-benzdiazepin-3-yl)-piperidin-1-carbonsäure-(R)-1-(3,4-dichlor-benzyl)-2-[4-(1-*tert*-butoxycarbonyl-piperidin-4-yl)-piperazin-1-yl]-2-oxo-ethylester (Beispiel 25) in 5 mL 4 M HCl wurde über Nacht bei RT gerührt. Das Reaktionsgemisch wurde lyophilisiert, wobei das Produkt als HCl-Salz anfiel.
- Ausbeute:: 18 mg (66% der Theorie)
- ESI-MS:: (M+H)⁺= 657/659/661 (2 Cl)
- R_{f} =: 0.33 (Kieselgel, DCM/MeOH/Cyc/NH₃ 70:15:15:2)

### Beispiel 29

### 4-(2-Oxo-1,2,4,5-tetrahydro-benzdiazepin-3-yl)-piperidin-1-carbonsäure-(R)-1-(3,4-dichlor-benzyl)-2-oxo-2-(4-piperazin-1-yl-piperidin-1-yl)-ethylester

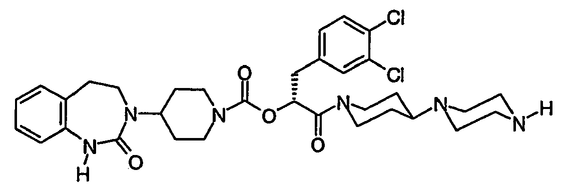

Eine Lösung von 35 mg (0.05 mmol) 4-(1-{(R)-3-(3,4-Dichlor-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-*tert*-butylester (Beispiel 26) in 5 mL 4 M HCl wurde über Nacht bei RT gerührt. Das Reaktionsgemisch wurde lyophilisiert, wobei das Produkt als HCl-Salz anfiel.
- Ausbeute:: 24 mg (75% der Theorie)
- ESI-MS:: (M+H)⁺ = 657/659/661 (2 Cl)
- R_{f} =: 0.30 (Kieselgel, DCM/MeOH/Cyc/NH₃ 70:15:15:2)

### Beispiel 30

### 4-(2-Oxo-1,2,4,5-tetrahydro-benzdiazepin-3-yl)-piperidin-1-carbonsäure-(R)-2-4,4'-bipiperidinyl-1-yl-1-(3,4-dichlor-benzyl)-2-oxo-ethylester

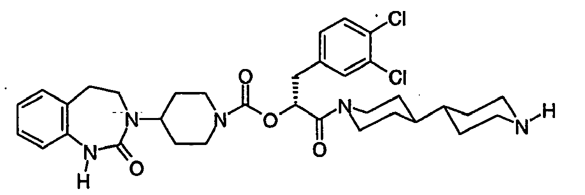

Eine Lösung von 30 mg (0.04 mmol) 1'-{(R)-3-(3,4-Dichlor-phenyl)-2-[4-(2-oxo-1,2,4,5-tetrahydro-1,3-benzdiazepin-3-yl)-piperidin-1-carbonyloxy]-propionyl}-4,4'-bipiperidinyl-1-carbonsäure-*tert*-butylester (Beispiel 27) in 5 mL 4 M HCl wurde über Nacht bei RT gerührt. Das Reaktionsgemisch wurde lyophilisiert, wobei das Produkt als HCl-Salz anfiel.
- Ausbeute:: 16 mg (58% der Theorie)
- ESI-MS:: (M+H)⁺ = 656/658/660 (2 Cl)

### Beispiel 31

### 4-(2-Oxo-1,2,4,5-tetrahydro-1,3-benzdiazepin-3-yl)-piperidin-1-carbonsäure-(R)-1-(4-chlor-3,5-dimethyl-benzyl)-2-[4-(4-methyl-piperazin-1-yl)-piperidin-1-yl]-2-oxo-ethylester

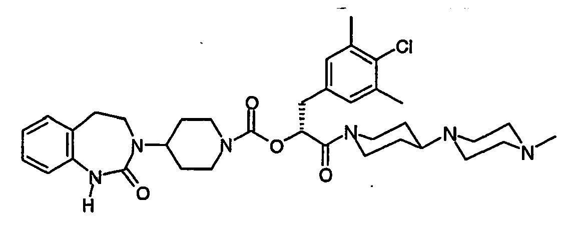

### 31a 5-Brom-2-chlor-1,3-dimethylbenzol

40.0 g (200 mmol) 4-Brom-2,6-dimethylamin in 100 mL halbkonz. HCl wurden bei 0°C mit 15.2 g (220 mmol) NaNO₂ in 90 mL Wasser versetzt, 20 min nachgerührt, mit einer Lösung aus 21.7 g (220 mmol) CuCl in 90 mL halbkonz. HCl versetzt und 2 h bei 70°C und 15 h bei RT gerührt. Nach beendeter Umsetzung wurde das Reaktionsgemisch auf 200 mL Wasser gegossen und mit TBME extrahiert. Die organischen Phasen wurden vereint, solange mit 2 M NaOH extrahiert, bis die organische Phase farblos blieb und die vereinigten organischen Phasen dann über Na₂SO₄ getrocknet. Nach Entfernen des Trocken- und Lösungsmittels wurde der Rückstand chromatographisch (Kieselgel, Cyc/DCM 1:1) gereinigt.
- Ausbeute:: 30.7 g (70% der Theorie)
- ESI-MS:: (M+H)⁺ = 218/220/222 (Br, Cl)
- R_{f} =: 0.84 (Kieselgel, Cyc/DCM 1:1)

### 31 b 2-Acetylamino-3-(4-chlor-3,5-dimethyl-phenyl)-acrylsäuremethylester

Unter Stickstoffatmosphäre wurden 30.0 g (136.7 mmol) 5-Brom-2-chlor-1,3-dimethylbenzol, 24.0 g (164.0 mmol) 2-Acetylamino-acrylsäuremethylester in 420 mL Triethylamin und 200 mL Acetonitril mit 3.4 g (10.9 mmol) Tri-o-tolyl-phosphan und 2.4 g (10.9 mmol) Pd(OAc)₂ versetzt und 18 h bei 80°C gerührt. Der Niederschlag wurde abgesaugt, das Filtrat i. vac. eingeengt, mit 800 mL DCM und 800 mL Wasser versetzt, die organische Phase abgetrennt und über Na₂SO₄ getrocknet. Nach Entfernen des Trocken- und Lösungsmittels wurde der Rückstand mit EtOAc verrührt, abgesaugt und i. vac getrocknet.
- Ausbeute:: 29.4 g (76% der Theorie)
- ESI-MS:: (M+H)⁺ = 282/284 (Cl)
- Retentionszeit (HPLC-MS):: 7.8 min (Methode A)

### 31c 3-(4-Chlor-3,5-dimethyl-phenyl)-2-oxo-propionsäure

29.4 g (105 mmol) 2-Acetylamino-3-(4-chlor-3,5-dimethylphenyl)-acrylsäuremethylester in 330 mL N-Methyl-2-pyrrolidinon wurden mit 500 mL gekühlter 4 M HCl versetzt, 6 h unter Rückfluß und 16 h bei RT gerührt. Nach beendeter Umsetzung wurde das Reaktionsgemisch auf 1650 mL Wasser gegossen, 1 h nachgerührt, abgesaugt und die Kristalle bei 50°C im Vakuumtrockenschrank getrocknet. Das Produkt wurde aus Toluol umkristallisiert.
- Ausbeute:: 12.3 g (52% der Theorie)
- ESI-MS:: (M+H)⁺ = 225/227 (Cl)
- Retentionszeit (HPLC-MS):: 7.9 min (Methode A)

### 31 d (R)-3-(4-Chlor-3,5-dimethyl-phenyl)-2-hydroxy-propionsäure

12.3 g (54.4 mmol) 3-(4-Chlor-3,5-dimethyl-phenyl)-2-oxo-propionsäure in 130 mL THF und 7.6 mL (54.4 mmol) Triethylamin wurden bei -35°C mit einer Lösung aus 21.0 g (65.3 mmol) (1 R)-B-Chlordiisopinocampheylboran in 65 mL THF innerhalb von 30 min versetzt und 2 h bei dieser Temperatur nachgerührt. Nach beendeter Umsetzung wurde das Reaktionsgemisch bei 0°C mit 50 mL 1 M NaOH alkalisch gestellt (exotherm), 3 h nachgerührt, mit 30 mL TBME versetzt und die Phasen getrennt. Die organische Phase wurde mit 50 mL Wasser und 30 mL 1 M NaOH gewaschen. Die vereinigten wässrigen Phasen wurden mit 2 M HCl sauer gestellt und mit TBME extrahiert. Die organischen Phasen wurden über Na₂SO₄ getrocknet und i. vac. eingeengt. Das Produkt wurde ohne Reinigung weiter umgesetzt.
- Ausbeute:: 12.5 g (100% der Theorie)
- ESI-MS:: (M+H)⁺ = 227/229 (Cl)
- Retentionszeit (HPLC-MS):: 7.1 min (Methode A)

### 31 e (R)-3-(4-Chlor-3,5-dimethyl-phenyl)-2-hydroxy-propionsäuremethylester

Zu einer auf 0°C gekühlten Lösung von 12.45 g (54.4 mmol) (R)-3-(4-Chlor-3,5-dimethyl-phenyl)-2-hydroxy-propionsäure in 300 mL MeOH wurden tropfenweise 4.4 mL (59.9 mmol) SOCl₂ zugegeben und das Reaktionsgemisch 1 h bei RT nachgerührt. Die Reaktionslösung wurde i. vac. eingeengt und der Rückstand chromatographisch (Kieselgel, Cyc/EtOAc 4:1) gereinigt.
- Ausbeute:: 10.1 g (76% der Theorie)
- ESI-MS:: (M+NH₄)⁺ = 260/262 (Cl)
- Retentionszeit (HPLC-MS):: 8.1 min (Methode A)

### 31 f 4-(2-Oxo-1,2,4,5-tetrahydro-1,3-benzdiazepin-3-yl)-piperidin-1-carbonsäure-(R)-2-(4-chlor-3,5-dimethyl-phenyl)-1-methoxycarbonyl-ethylester

Unter Stickstoffatmospäre wurden 1.0 g (8.2 mmol) 4-Dimethylaminopyridin in 30 mL Pyridin mit 1.7 g (8.2 mmol) Chlorameisensäure-4-nitrophenylester versetzt, 40 min bei RT gerührt, dann wurden 2.0 g (8.2 mmol) (R)-3-(4-Chlor-3,5-dimethyl-phenyl)-2-hydroxy-propionsäuremethylester zugegeben, nochmals 20 min bei RT gerührt und anschließend mit 2.0 g ( 8.2 mmol) 3-Piperidin-4-yl-1,3,4,5-tetrahydro-1,3-benzdiazepin-2-on versetzt und das Reaktionsgemisch 20 h bei RT gerührt. Man engte i.vac. ein, nahm den Rückstand in EtOAc auf, wusch mit 10% KHSO₄- und gesättigter NaHCO₃-Lösung und trocknete die organische Phase über Na₂SO₄. Nach Entfernen des Trocken- und Lösungsmittels wurde der Rückstand chromatographisch (Kieselgel,Cyc/EtOAc 1:1 bis 1:2 ) gereinigt.
- Ausbeute:: 2.16 g (51% der Theorie)
- ESI-MS:: (M+H)⁺ = 514/516 (Cl)
- Retentionszeit (HPLC-MS):: 10.1 min (Methode A)

### 31g 4-(2-Oxo-1,2,4,5-tetrahydro-1,3-benzdiazepin-3-yl)-piperidin-1-carbonsäure-(R)-1-carboxy-2-(4-chlor-3,5-dimethyl-phenyl)-ethylester

Zu einer Lösung von 2.15 g (4.18 mmol) 4-(2-Oxo-1,2,4,5-tetrahydro-1,3-benzdiazepin-3-yl)-piperidin-1-carbonsäure-(R)-2-(4-chlor-3,5-dimethyl-phenyl)-1-methoxycarbonyl-ethylester in 60 mL THF wurde eine Lösung von 150 mg (0.60 mmol) LiOH in 30 mL Wasser gegeben und das Reaktionsgemisch 1 h bei RT gerührt. Man engte i. vac. ein, nahm den Rückstand in 100 mL Wasser auf, säuerte mit 1 M HCl an, filtrierte den Niederschlag und trocknete diesen im Vakuumtrockenschrank bei 40°C.
- Ausbeute:: 2.05 g (98% der Theorie)
- ESI-MS:: (M+H)⁺ = 500/502 (Cl)
- Retentionszeit (HPLC-MS):: 8.8 min (Methode A)

### 31h 4-(2-Oxo-1,2,4,5-tetrahydro-1,3-benzdiazepin-3-yl)-piperidin-1-carbonsäure-(R)-1-(4-chlor-3,5-dimethyl-benzyl)-2-[4-(4-methyl-piperazin-1-yl)-piperidin-1-yl]-2-oxo-ethylester

Eine Lösung von 80 mg (0.16 mmol) 4-(2-Oxo-1,2,4,5-tetrahydro-benzdiazepin-3-yl)-piperidin-1-carbonsäure-(R)-2-(4-chlor-3,5-dimethyl-phenyl)-ethylester, 51 mg (0.16 mmol) TBTU und 28 µL (0.20 mmol) Triethylamin in 1.5 mL DMF wurde 1 h bei RT gerührt. Dann erfolgte die Zugabe von 30 mg (0.16 mmol) 1-Methyl-4-(piperidin-4-yl)-piperazin und das Reaktionsgemisch wurde 16 h bei RT gerührt. Die Reaktionslösung wurde über einen Spritzenfilter filtriert und ohne weitere Aufarbeitung direkt via HPLC gereinigt: Die das Produkt enthaltenden Fraktionen wurden vereinigt und lyophilisiert.
- Ausbeute:: 18 mg (17 % der Theorie)
- ESI-MS:: (M+H)⁺ = 665/667 (Cl)
- Retentionszeit (HPLC-MS):: 5.6 min (Methode A)

Analog wurden folgende Verbindungen aus jeweils 80 mg (Beispiele 32 bis 34) bzw. aus jeweils 140 mg (Beispiele 35 und 36) 4-(2-Oxo-1,2,4,5-tetrahydro-1,3-benzdiazepin-3-yl)-piperidin-1-carbonsäure-(R)-1-carboxy-2-(4-chlor-3,5-dimethylphenyl)-ethylester und der entsprechenden Menge an Amin hergestellt:

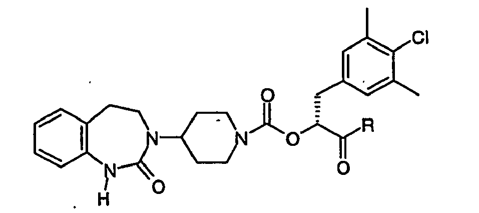

| **Beispiel** | **R** | **Ausbeute (%)** | **Massenspektrum** | **Retentionszeit HPLC (Methode)** |
|---|---|---|---|---|
| **32** | 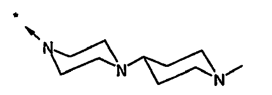 | 40 | 665/667(Cl) [M+H]⁺ | 5.3 min (A) |
| **33** | 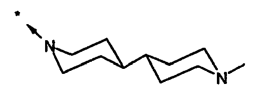 | 42 | 664/666(Cl) [M+H]⁺ | 6.8 min (A) |
| **34** | 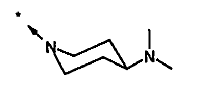 | 44 | 610/612(Cl) [M+H]⁺ | 6.5 min (A) |
| **35** | 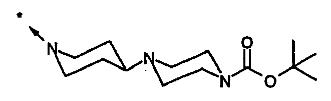 | 30 | 751/753(Cl) [M+H]+ | 7.5 min (A) |
| **36** | 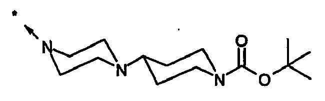 | 28 | 751/753(Cl) [M+H]+ | 7.7 min (A) |

### Beispiel 37

### 4-(2-Oxo-1,2,4,5-tetrahydro-1,3-benzdiazepin-3-yl)-piperidin-1-carbonsäure-(R)-1-(4-chlor-3,5-dimethyl-benzyl)-2-oxo-2-(4-piperazin-1-yl-piperidin-1-yl)-ethylester

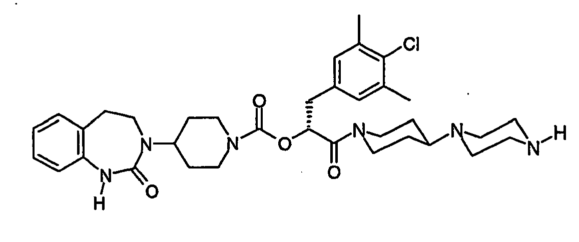

Eine Lösung von 64.0 mg (0.09 mmol) 4-(1-{(R)-3-(4-Chlor-3,5-dimethyl-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-*tert*-butylester (Beispiel 35) in 5 mL 2 M HCl wurde über Nacht bei RT gerührt. Das Reaktionsgemisch wurde lyophilisiert, wobei das Produkt als Bis-Hydrochlorid Salz anfiel.
- Ausbeute:: 61.2 mg (99% der Theorie)
- ESI-MS:: (M+H)⁺ = 651/653 (Cl)
- Retentionszeit (HPLC-MS):: 5.9 min (Methode A)

### Beispiel 38

### 4-(2-Oxo-1,2,4,5-tetrahydro-1,3-benzdiazepin-3-yl)-piperidin-1-carbonsäure-(R)-1-(4-chlor-3,5-dimethyl-benzyl)-2-oxo-2-(4-piperidin-4-yl-piperazin-1-yl)-ethylester

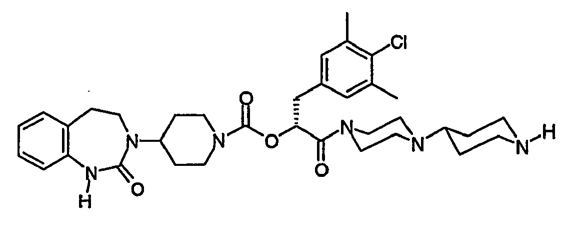

Eine Lösung von 59 mg (0.08 mmol) 4-(2-Oxo-1,2,4,5-tetrahydro-1,3-benzdiazepin-3-yl)-piperidin-1-carbonsäure-(R)-1-(4-chlor-3,5-di methyl-benzyl)-2-[4-(1-tert-butoxycarbonyl-piperidin-4-yl)-piperazin-1-yl]-2-oxo-ethylester (Beispiel 36) in 5 mL 2 M HCl wurde über Nacht bei RT gerührt. Das Reaktionsgemisch wurde lyophilisiert, wobei das Produkt als Bis-Hydrochlorid Salz anfiel.
- Ausbeute:: 55.7 mg (57% der Theorie)
- ESI-MS:: (M+H)⁺ = 651/653 (Cl)
- Retentionszeit (HPLC-MS):: 5.5 min (Methode A)

### Beispiel 39

### 4-(2-Oxo-1,2,4,5-tetrahydro-1,3-benzdiazepin-3-yl)-piperidin-1-carbonsäure-(R)-1-(3,5-dimethyl-benzyl)-2-[4-(1-methyl-peridin-4-yl)-piperazin-1-yl]-2-oxo-ethylester

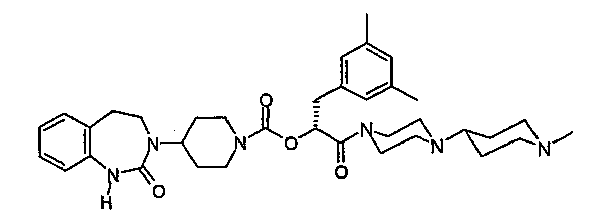

### 39a 4-(2-Oxo-1,2,4,5-tetrahydro-1,3-benzdiazepin-3-yl)-piperidin-1-carbonsäure-(R)-1-carboxy-2-(3,5-dimethyl-phenyl)-ethylester

500 mg (1.0 mmol) 4-(2-Oxo-1,2,4,5-tetrahydro-benzdiazepin-3-yl)-piperidin-1-carbonsäure-1-carboxy-(R)-2-(4-chlor-3,5-dimethyl-phenyl)-ethylester (Beispiel 31g) in 20 mL MeOH wurden mit 100 mg 10% Pd/C und 2 mL Triethylamin versetzt und 10 Tage bei RT und 3 bar hydriert. Nach beendeter Umsetzung wurde das Reaktionsgemisch i.vac. eingeengt, der Rückstand in 25 mL Wasser aufgenommen, mit 1 M HCI sauer gestellt, der Niederschlag abgesaugt und im Vakuumtrockenschrank bei 40°C getrocknet.
- Ausbeute:: 418 mg (90% der Theorie)
- ESI-MS:: (M+H)⁺ = 466
- Retentionszeit (HPLC-MS):: 8.3 min (Methode A)

### 39b 4-(2-Oxo-1,2,4,5-tetrahydro-1,3-benzdiazepin-3-yl)-piperidin-1-carbonsäure-(R)-1-(3,5-dimethyl-benzyl)-2-[4-(1-methyl-piperidin-4-yl)-piperazin-1-yl]-2-oxo-ethylester

Eine Lösung von 50 mg (0.16 mmol) 4-(2-Oxo-1,2,4,5-tetrahydro-1,3-benzdiazepin-3-yl)-piperidin-1-carbonsäure-(R)-1-carboxy-2-(3,5-dimethyl-phenyl)-ethylester, 35 mg (0.11 mmol) TBTU und 19 µL (0.13 mmol) Triethylamin in 1 mL DMF wurde 1 h bei RT gerührt. Dann erfolgte die Zugabe von 20 mg (0.11 mmol) 1-(1-Methyl-piperidin-4-yl)-piperazin und das Reaktionsgemisch wurde 16 h bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.
- Ausbeute:: 33 mg (49 % der Theorie)
- ESI-MS:: (M+H)⁺ = 631
- Retentionszeit (HPLC-MS):: 5.3 min (Methode A)

Analog wurden folgende Verbindungen aus jeweils 50 mg (Beispiele 40 und 41) bzw. aus jeweils 80 mg (Beispiele 42 und 43) 4-(2-Oxo-1,2,4,5-tetrahydro-1,3-benzdiazepin-3-yl)-piperidin-1-carbonsäure-(R)-1-carboxy-2-(3,5-dimethyl-phenyl)-ethylester und der entsprechenden Menge an Amin hergestellt:

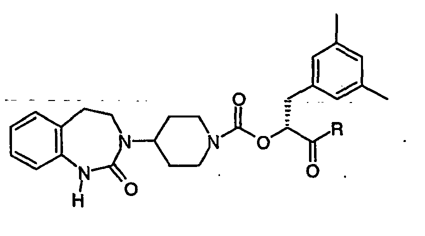

| **Beispiel** | **R** | **Ausbeute (%)** | **Massenspektrum** | **Retentionszeit HPLC (Methode) oder R_{f} (Kieselgel, Laufmittel)** |
|---|---|---|---|---|
| **40** | 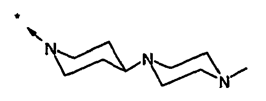 | 76 | 631 [M+H]⁺ | 5.8 min (A) |
| **41** | 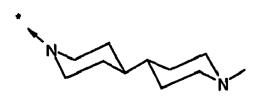 | 55 | 630 [M+H]⁺ | 6.5 min (A) |
| **42** | 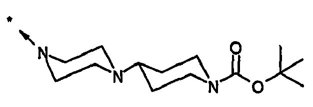 | 37 | 717 [M+H]⁺ | 4.7 min (A) |
| **43** | 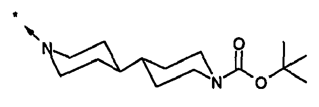 | 73 | 716 [M+H]⁺ | 0.59 (EtOAc) |

### Beispiel 44

### 4-(2-Oxo-1,2,4,5-tetrahvdro-1,3-benzdiazepin-3-yl)-piperidin-1-carbonsäure-(R)-1-(3,5-dimethyl-benzyl)-2-oxo-2-(4-piperidin-4-yl-piperazin-1-yl)-ethylester

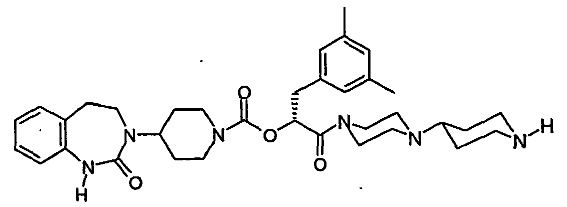

Eine Lösung von 45 mg (0.06 mmol) 4-(2-Oxo-1,2,4,5-tetrahydro-1,3-benzdiazepin-3-yl)-piperidin-1-carbonsäure-(R)-1-(3,5-dimethyl-benzyl)-2-[4-(1-tert-butoxy-carbonyl-piperidin-4-yl)-piperazin-1-yl]-2-oxo-ethylester (Beispiel 42) in 10 mL 2 M HCI wurde über Nacht bei RT gerührt. Das Reaktionsgemisch wurde lyophilisiert. Das Rohprodukt wurde in 1 mL DMF aufgenommen, mit 0.6 mL gesättigter K₂CO₃-Lösung alkalisch gestellt und via HPLC chromatographisch gereinigt.
- Ausbeute:: 26.8 mg (69% der Theorie)
- ESI-MS:: (M+H)⁺ = 617
- Retentionszeit (HPLC-MS):: 5.4 min (Methode A)

### Beispiel 45

### 4-(2-Oxo-1,2,4,5-tetrahydro-1,3-benzdiazepin-3-yl)-piperidin-1-carbonsäure-(R)-2-[4,4']bipiperidinyl-1-yl-1-(3,5-dimethyl-benzyl-2-oxo-ethylester

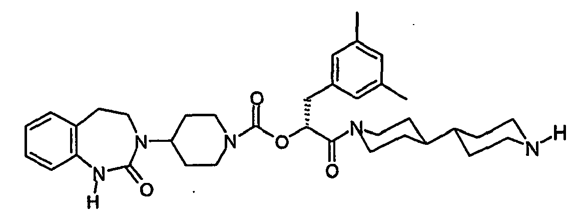

Eine Lösung von 101 mg (0.14 mmol) 1'-{(R)-3-(3,5-Dimethyl-phenyl)-2-[4-(2-oxo-1,2,4,5-tetrahydro-1,3-benzdiazepin-3-yl)-piperidin-1-carbonyloxy]-propionyl)-4,4'-bi-piperidinyl-1-carbonsäure-tert-butylester (Beispiel 43) in 10 mL 2 M HCl wurde über Nacht bei RT gerührt. Das Reaktionsgemisch wurde lyophilisiert. Das Rohprodukt wurde in 1 mL DMF aufgenommen, mit 0.6 mL gesättigter K₂CO₃-Lösung alkalisch gestellt und via HPLC chromatographisch gereinigt.
- Ausbeute:: 18.7 mg (22% der Theorie)
- ESI-MS:: (M+H)⁺ = 616
- Retentionszeit (HPLC-MS):: 6.5 min (Methode A)

### Beispiel 46

### 4-(2-Oxo-1,2,4,5-tetrahydro-1,3-benzdiazepin-3-yl)-piperidin-1-carbonsäure-(R)-1-(3,5-bis-trifluormethylbenzyl)-2-[4-(1-methyl-piperidin-4-yl)-piperazin-1-yl]-2-oxo-ethylester

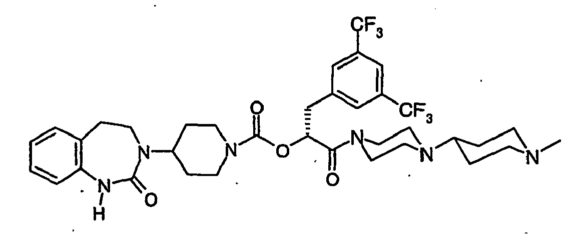

### 46a 2-Acetylamino-3-(3,5-bis-trifluormethyl-phenyl)acrylsäuremethylester

Unter Stickstoffatmosphäre wurden 50.0 g (171 mmol) 3,5-Bis-(trifluormethyl)-brombenzol, 25.0 g (171 mmol) 2-Acetylamino-acrylsäuremethylester in 475 mL Triethylamin und 250 mL Acetonitril mit 3.9 g (12.4 mmol) Tri-o-tolyl-phosphan und 2.8 g (12.5 mmol) Pd(OAc)₂ versetzt und 18 h bei 80°C gerührt. Nach beendeter Umsetzung wurde das Reaktionsgemisch i. vac. auf ca. 200 mL eingeengt, mit 400 mL EtOAc und 400 mL Wasser versetzt, der Niederschlag abgesaugt und die Phasen getrennt. Die organische Phase wurde über Na₂SO₄ getrocknet, mit Aktivkohle versetzt, filtriert und zur Trockene eingeengt. Der Rückstand wurde mit DIPE verrührt, abgesaugt und i. vac getrocknet.
- Ausbeute:: 19.5 g (32 % der Theorie)
- ESI-MS:: (M+H)⁺ = 356
- R_{f} =: 0.76 (Kieselgel, PE/EtOAc 1:1)

### 46b 3-(3,5-Bis-trifluormethyl-phenyl)-2-oxo-propionsäure

19.5 g (54.9 mmol) 2-Acetylamino-3-(3,5-bis-trifluormethyl-phenyl)-acrylsäuremethylester in 100 mL 1,4-Dioxan wurden auf 100°C Badtemperatur erwärmt, mit 100 mL 4 M HCI versetzt und 8 h bei 100°C Badtemperatur gerührt. Das Reaktionsgemisch wurde i. vac. eingeengt, die Kristalle abgesaugt, mit Wasser gewaschen und diese im Trockenschrank bei 50°C getrocknet.
- Ausbeute:: 16.1 g (98 % der Theorie)
- ESI-MS:: (M-H)⁻ = 299
- R_{f} =: 0.18 (Kieselgel, EtOAc)

### 46c (R)-3-(3,5-Bis-trifluormethyl-phenyl)-2-hydroxy -propionsäure

16.1 g (53.6 mmol) 3-(3,5-Bis-trifluormethyl-phenyl)-2-oxo-propionsäure in 9.5 (70.0 mmol) Triethylamin und 100 mL THF wurden bei -35°C mit einer Lösung aus 26.0 (81.1 mmol) (1 R)-B-Chlordiisopinocampheylboran in 40 mL THF innerhalb von 30 min versetzt, 1 h bei dieser Temperatur und über Nacht bei RT nachgerührt. Nach beendeter Umsetzung wurde das Reaktionsgemisch bei 0°C mit 160 mL 1 M NaOH alkalisch gestellt, 15 min nachgerührt, mit 100 mL TBME versetzt und die Phasen getrennt. Die organische Phase wurde mit 50 mL Wasser und 50 mL 1 M NaOH gewaschen. Die vereinigten wässrigen Phasen wurden mit 4 M HCI sauer gestellt, erschöpfend mit TBME extrahiert, die vereinigten organischen Phasen über Na₂SO₄ getrocknet, über Aktivkohle abgesaugt und i.vac. eingeengt. Das Produkt wurde ohne Reinigung weiter umgesetzt.
- Ausbeute:: 12.5 g (77% der Theorie)
- ESI-MS:: (M-H)⁻ = 301
- R_{f} =: 0.45 (Kieselgel, EtOAc)

### 46d (R)-3-(3,5-Bis-trifluormethyl-phenyl)-2-hydroxy-propionsäuremethylester

12.5 g (41.4 mmol) (R)-3-(3,5-Bis-trifluormethyl-phenyl)-2-hydroxy-propionsäure in 150 mL methanolischer HCI (1.25 M) wurden 4 h bei RT gerührt und anschliessend i. vac. eingeengt. Der Rückstand wurde in EtOAc aufgenommen und mit gesättigter NaHCO₃-Lösun gewaschen, die organische Phase über Na₂SO₄ getrocknet, über Aktivkohle abgesaugt und i. vac. eingeengt. Der Rückstand wurde mit PE verrührt, abgesaugt und i. vac. eingeengt. Das Produkt wurde ohne Reinigung weiter umgesetzt.
- Ausbeute:: 11.4 g (87% der Theorie)
- ESI-MS:: (M+H)⁺ = 316
- R_{f} =: 0.80 (Kieselgel, PE/EtOAc 1:1)

### 46e 4-(2-Oxo-1,2,4,5-tetrahydro-1,3-benzdiazepin-3-yl)-piperidin-1-carbonsäure-(R)-2-(3,5-bis-trifluormethyl-phenyl)-1-methoxycarbonyl-ethylester

Analog Beispiel 31f konnte aus 6.0 g (8.2 mmol) (R)-3-(3,5-Bis-trifluormethyl-phenyl)-2-hydroxy-propionsäuremethylester und 5.13 g (20.9 mmol) 3-Piperidin-4-yl-1,3,4,5-tetrahydro-1,3-benzdiazepin-2-on das Produkt erhalten werden. Die Reinigung erfolgte chromatographisch (Kieselgel, Gradient PE/EtOAc 1:1 zu 1:9 ).
- Ausbeute:: 5.1 g (46 % der Theorie)
- ESI-MS:: (M+H)⁺ = 588
- R_{f} =: 0.63 (Kieselgel, DCM/MeOH/Cyc/NH₃ 70:15:15:2)

### 46f 4-(2-Oxo-1,2,4,5-tetrahydro-1,3-benzdiazepin-3-yl)-piperidin-1-carbonsäure-(R)-2-(3,5-bis-trifluormethyl-phenyl)-1-carboxy-ethylester

Zu einer Lösung von 5.0 g (8.5 mmol) 4-(2-Oxo-1,2,4,5-tetrahydro-1,3-benzdiazepin-3-yl)-piperidin-1-carbonsäure-(R)-2-(3,5-bis-trifluormethyl-phenyl)-1-methoxycarbonyl-ethylester in 50 mL THF wurde eine Lösung von 307 mg (12.8 mmol) LiOH in 5 mL Wasser gegeben und das Reaktionsgemisch über Nacht bei RT gerührt. Man engte i. vac. ein, nahm den Rückstand in Wasser auf, säuerte mit 1 M HCl an, filtrierte den Niederschlag ab und trocknete diesen im Vakuumtrockenschrank bei 40°C.
- Ausbeute:: 4.5 g (92% der Theorie)
- ESI-MS:: (M+H)⁺ = 574
- R_{f} =: 0.32 (Kieselgel,DCM/MeOH/Cyc/NH₃ 70:15:15:2)

### 46g 4-(2-Oxo-1,2,4,5-tetrahydro-1,3-benzdiazepin-3-yl)-piperidin-1-carbonsäure-(R)-1-(3,5-bis-trifluormethyl-benzyl)-2-[4-(1-methyl-piperidin-4-yl)-piperazin-1-yl]-2-oxo-ethylester

Eine Lösung von 80 mg (0.14 mmol) 4-(2-Oxo-1,2,4,5-tetrahydro-benzdiazepin-3-yl)-piperidin-1-carbonsäure-(R)-2-(3,5-bis-trifluormethyl-phenyl)-1-carboxy-ethylester, 51 mg (0.16 mmol) TBTU und 22 µL (0.16 mmol) Triethylamin in 1 mL DMF wurde 1 h bei RT gerührt. Dann erfolgte die Zugabe von 29 mg (0.16 mmol) 1-(1-Methylpiperidin-4-yl)-piperazin und das Reaktionsgemisch wurde über Nacht bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.
- Ausbeute:: 56 mg (54% der Theorie)
- ESI-MS:: (M+H)⁺ = 739
- Retentionszeit (HPLC-MS):: 5.8 min (Methode A)

Analog wurden folgende Verbindungen aus jeweils 80 mg (Beispiele 47 bis 49) bzw. aus jeweils 100 mg (Beispiele 50 bis 53 ) 4-(2-Oxo-1,2,4,5-tetrahydro-benzdiazepin-3-yl)-piperidin-1-carbonsäure-(R)-2-(3,5-bis-trifluormethyl-phenyl)-1-carboxy-ethylester und der entsprechenden Menge an Amin hergestellt:

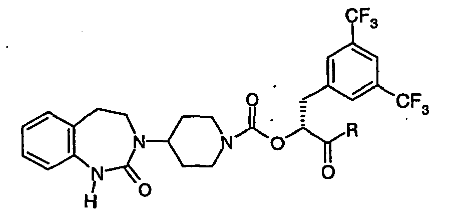

| **Beispiel** | **R** | **Ausbeute (%)** | **Massenspektrum** | **Retentionszeit HPLC (Methode)** |
|---|---|---|---|---|
| **47** | 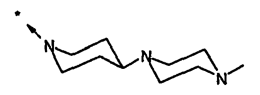 | 49 | 739 [M+H]⁺ | 6.5 min (A) |
| **48** | 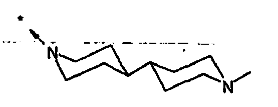 | 47 | 738 [M+H]⁺ | 7.1 min (A) |
| **49** | 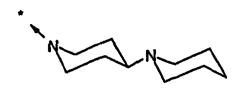 | 57 | 724 [M+H]⁺ | 7.1 min (A) |
| **50** | 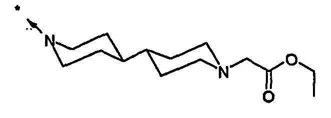 | 39 | 810 [M+H]+ | 7.3 min (A) |
| **51** | 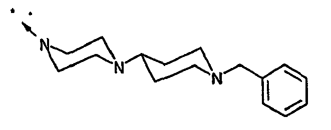 | 53 | 815 [M+H]+ | 6.4 min (A) |
| **52** | 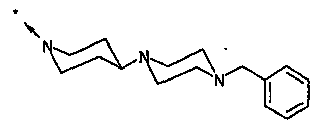 | 46 | 815 [M+H]+ | 6.1 min (A) |
| **53** | 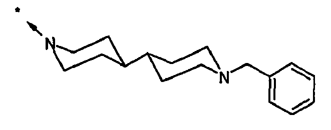 | 65 | 814 [M+H]+ | 7.9 min (A) |

### Beispiel 54

### 4-(2-Oxo-1,2,4,5-tetrahvdro-1,3-benzdiazepin-3-yl)-piperidin-1-carbonsäure-(R)-1-(3,5-bis-trifluormethyl-benzyl)-2-oxo-2-(4-piperidin-4-yl-piperazin-1-yl)-ethylester

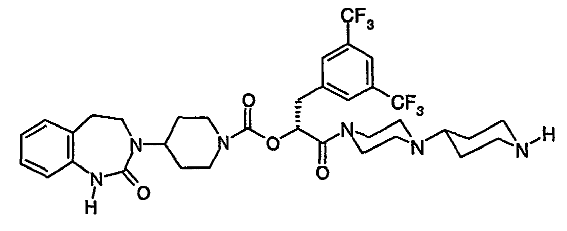

Eine Lösung von 77 mg (0.09 mmol) 4-(2-Oxo-1,2,4,5-tetrahydro-1,3-benzdiazepin-3-yl)-piperidin-1-carbonsäure-(R)-2-[4-(1-benzyl-piperidin-4-yl)-piperazin-1-yl]-1-(3,5-bis-trifluormethyl-benzyl)-2-oxo-ethylester (Beispiel 51) in 10 mL MeOH wurde mit 50 mg 10% Pd/C versetzt und 3 h bei RT und 50 psi Wasserstoff geschüttelt. Der Katalysator wurde abgesaugt, das Lösungsmittel i.vac. eingeengt, der Rückstand mit Acetonitril und Wasser versetzt und lyophilisiert.
- Ausbeute:: 46 mg (70% der Theorie)
- ESI-MS:: (M+H)⁺ = 725
- Retentionszeit (HPLC-MS):: 5.7 min (Methode A)

### Beispiel 55

### 4-(2-Oxo-1,2,4,5-tetrahydro-1,3-benzdiazepin-3-yl)-piperidin-1-carbonsäure-(R)-1-(3,5-bis-trifluormethyl-benzyl)-2-oxo-2-(4-piperazin-1-yl-piperidin-1-yl)-ethylester

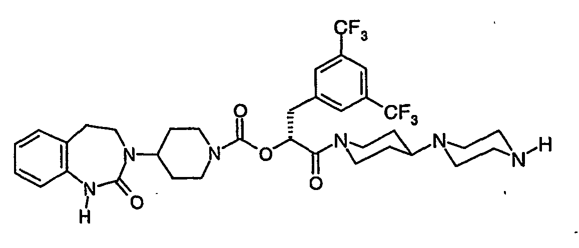

Eine Lösung von 64 mg (0.08 mmol) 4-(2-Oxo-1,2,4,5-tetrahydro-1,3-benzdiazepin-3-yl)-piperidin-1-carbonsäure-(R)-2-[4-(4-benzyl-piperazin-1-yl)-piperidin-1-yl]-1-(3,5-bis-trifluormethyl-benzyl)-2-oxo-ethylester (Beispiel 52) in 10 mL MeOH wurde mit 50 mg 10% Pd/C versetzt und 3 h bei RT und 50 psi Wasserstoff geschüttelt. Der Katalysator wurde abgesaugt, das Lösungsmittel i.vac. eingeengt, der Rückstand mit Acetonitril und Wasser versetzt und lyophilisiert.
- Ausbeute:: 43 mg (76% der Theorie)
- ESI-MS:: (M+H)⁺ = 725
- Retentionszeit (HPLC-MS):: 5.7 min (Methode A)

### Beispiel 56

### 4-(2-Oxo-1,2,4,5-tetrahydro-1,3-benzdiazepin-3yl)-piperidin-1-carbonsäure- R-2-4,4'-bipiperidinyl-1-yl-1-(3,5-bis-trifluormethyl-benzyl)-2-oxo-2-ethylester

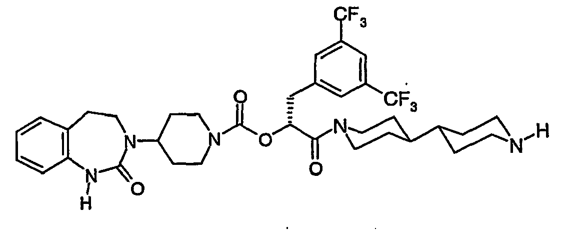

Eine Lösung von 92 mg (0.11 mmol) 4-(2-Oxo-1,2,4,5-tetrahydro-1,3-benzdiazepin-3-yl)-piperidin-1-carbonsäure-(R)-2-(1'-benzyl-4,4'-bipiperidinyl-1-yl)-1-(3,5-bis-trifluormethyl-benzyl)-2-oxo-ethylester (Beispiel 53) in 10 mL MeOH wurde mit 50 mg 10% Pd/C versetzt und 3 h bei RT und 50 psi Wasserstoff geschüttelt. Der Katalysator wurde abgesaugt, das Lösungsmittel i.vac. eingeengt, der Rückstand mit Acetonitril und Wasser versetzt und lyophilisiert.
- Ausbeute:: 55 mg (67 % der Theorie)
- ESI-MS:: (M+H)⁺ = 724
- Retentionszeit (HPLC-MS):: 5.6 min (Methode A)

### Beispiel 57

### 4-(2-Oxo-1,2,4,5-tetrahydro-1,3-benzdiazepin-3-yl)-piperidin-1-carbonsäure-(R)-1-4-(3,5-bis-trifluormethyl-benzyl)-2-(1'-carboxymethyl-4 4'-bipiperidinyl-1-yl)-2-oxo-ethylester

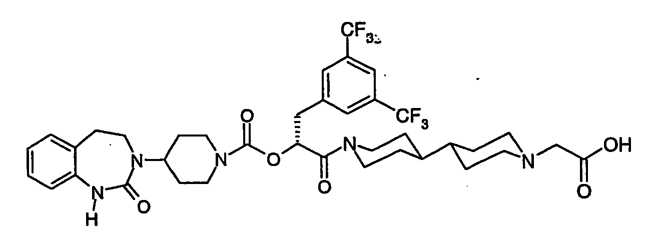

Zu einer Lösung von 35 mg (0.04 mmol) 4-(2-Oxo-1,2,4,5-tetrahydro-1,3-benzdiazepin-3-yl)-piperidin-1-carbonsäure-(R)-1-(3,5-bis-trifluormethyl-benzyl)-2-(1'-ethoxycarbonylmethyl-4,4'-bipiperidiny1-1-yl)-2-oxo-ethylester (Beispiel 50) in 5 mL THF wurde eine Lösung von 1.5 mg (0.06 mmol) LiOH in 1 mL Wasser gegeben und die Reaktionslösung über Nacht bei RT gerührt. Man engte i. vac. ein, nahm den Rückstand in Wasser auf, säuerte mit 1 N HCl an, filtrierte den Niederschlag ab und trocknete diesen im Vakuumtrockenschrank.
- Ausbeute:: 15 mg (44 % der Theorie)
- ESI-MS:: (M+H)⁺ = 782
- R_{f} =: 0.41 (Kieselgel, DCM/MeOH/Cyc/NH₃ 70:15:15:2)

### Beispiel 58

### 4-(2-Oxo-1,2,4,5-tetrahydro-1,3-benzdiazepin-3-yl)-piperidin-1-carbonsäure-(R)-1-(3,5-dibrom-benzyl)-2-(4-dimethylamino-piperidin-1-yl)-2-oxo-ethylester

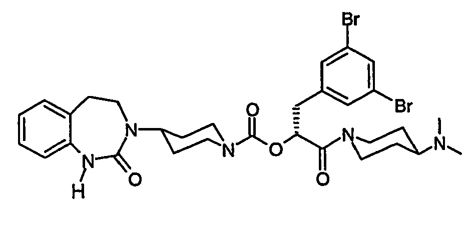

### 58a (Z,E)-2-Acetylamino-3-(3,5-dibrom-phenyl)-acrylsäure

Analog Beispiel 1a konnte aus 35.0 g (133 mmol) 3,5-Dibrom-benzaldehyd und 23.3 g (199 mmol) N-Acetyl-glycin das Produkt erhalten werden.
- Ausbeute:: 29.2 g (61 % der Theorie)
- Schmelzpunkt:: 248-249°C
- ESI-MS: (M+H)⁺ = 362/364/366 (2 Br)
- R_{f} =: 0.1 (Kieselgel, DCM/MeOH/AcOH 90:10:1)

### 58b 3-(3,5-Dibrom-phenyl)-2-oxo-propionsäure

Analog Beispiel 1b konnte aus 29.0 g (80.0 mmol) (Z,E)-2-Acetylamino-3-(3,5-dibrom-phenyl)-acrylsäure das Produkt erhalten werden.
- Ausbeute:: 14.0 g (54% der Theorie)
- ESI-MS: (M-H)⁻ = 318/320/322 (2 Br)
- R_{f} =: 0.4 (Kieselgel, DCM/MeOH/AcOH 90:10:1)

### 58c (R)-3-(3,5-Dibrom-phenyl)-2-hydroxy-propionsäure

Analog Beispiel 1 c konnte aus 12.0 g (37.3 mmol) 3-(3,5-Dibrom-phenyl)-2-oxo-propionsäure und 15.1 g (47.1 mmol) (1R)-B-Chlordiisopinocampheylboran das Produkt erhalten werden.
- Ausbeute:: 4.1 g (34% der Theorie)
- ESI-MS: (M-H)⁻ = 321/323/325 (2 Br)

### 58d (R)-3-(3,5-Dibrom-phenyl)-2-hydroxy-propionsäuremethylester

Analog Beispiel 46d konnte aus 4.0 g (12.4 mmol) (R)-3-(3,5-Dibrom-phenyl)-2-hydroxy-propionsäure das Produkt erhalten werden, wobei zur Veresterung methanolische HCl (6 M) verwendet wurde.
- Ausbeute:: 4.0 g (96% der Theorie)
- R_{f} =: 0.9 (Kieselgel, DCM/MeOH/AcOH 90:10:1)

### 58e 4-(2-Oxo-1,2,4,5-tetrahydro-1,3-benzdiazepin-3-yl)-piperidin-1-carbonsäure-(R)-2-(3,5-dibrom-phenyl)-1-methoxycarbonyl-ethylester

Analog Beispiel 31f konnte aus 3.40 g (10.06 mmol) (R)-3-(3,5-Dibrom-phenyl)-2-hydroxy-propionsäuremethylester und 2.46 g (10.03 mmol) 3-Piperidin-4-yl-1,3,4,5-tetrahydro-1,3-benzdiazepin-2-on das Produkt erhalten werden.
- Ausbeute:: 2.0 g (33% der Theorie)
- ESI-MS: (M+H)⁺ = 608/610/612 (2 Br)
- R_{f} =: 0.2 (Kieselgel, n-Hexan/EtOAc 3:7)
- Retentionszeit (HPLC):: 22.6 min (Methode F)

### 58f 4-(2-Oxo-1,2,4,5-tetrahydro-1,3-benzdiazepin-3-yl)-piperidin-1-carbonsäure-(R)-1-carboxy-2-(3,5-dibrom-phenyl)-ethylester

Zu einer Lösung von 2.0 g (3.3 mmol) 4-(2-Oxo-1,2,4,5-tetrahydro-1,3-benzdiazepin-3-yl)-piperidin-1-carbonsäure-(R)-2-(3,5-dibrom-phenyl)-1-methoxycarbonyl-ethylester in 12.5 mL THF wurde eine Lösung von 118 mg (4.9 mmol) LiOH in 5 mL Wasser gegeben und 2 h bei RT gerührt. Das Reaktionsgemisch wurde i. vac. eingeengt, der Rückstand mit Wasser und TBME versetzt, die wässrige Phase mit konz. HCI auf pH 2-3 eingestellt und mit DCM extrahiert. Die vereinigten organischen Phasen wurden mit gesättigter NaCl-Lösung gewaschen, über Na₂SO₄ getrocknet und i. vac. bis zur Trockene eingeengt.
- Ausbeute:: 1.9 g (97% der Theorie)
- ESI-MS: (M+H)⁺ = 594/596/598 (2 Br)
- R_{f} =: 0.25 (Kieselgel, DCM/MeOH 9:1)
- Retentionszeit (HPLC):: 19.1 min (Methode F)

### 58g 4-(2-Oxo-1,2,4,5-tetrahydro-1,3-benzdiazepin-3-yl)-piperidin-1-carbonsäure-(R)-1-(3,5-dibrom-benzyl)-2-(4-dimethylamino-piperidin-1-yl)-2-oxo-ethylester

Eine Lösung von 150 mg (0.25 mmol) 4-(2-Oxo-1,2,4,5-tetrahydro-1,3-benzdiazepin-3-yl)-piperidin-1-carbonsäure-(R)-1-carboxy-2-(3,5-dibrom-phenyl)-ethylester, 90 mg (0.28 mmol) TBTU, 95 µL (0.55 mmol) Ethyldiisopropylamin und 38 mg (0.28 mmol) HOBt in 6 mL DMF wurde 90 min bei RT gerührt. Dann erfolgte die Zugabe von 42 mg (0.33 mmol) 4-Dimethylamino-piperidin und das Reaktionsgemisch wurde 16 h bei RT gerührt. Die Reaktionslösung wurde mit Wasser versetzt, die organische Phase eingeengt und der Rückstand chromatographisch (Kieselgel, DCM/MeOH/NH₃ 95:5:0.5) gereinigt.
- Ausbeute:: 140 mg (79 % der Theorie)
- ESI-MS: (M+H)⁺ = 704/706/708 (2 Br)
- R_{f} =: 0.35 (Kieselgel, DCM/MeOH/NH₃ 90:10:1)
- Retentionszeit (HPLC):: 12.0 min (Methode F)

Analog wurden folgende Verbindungen aus jeweils 150 mg 4-(2-Oxo-1,2,4,5-tetrahydro-1,3-benzdiazepin-3-yl)-piperidin-1-carbonsäure-(R)-1-carboxy-2-(3,5-dibrom-phenyl)-ethylester und der entsprechenden Menge an Amin hergestellt:

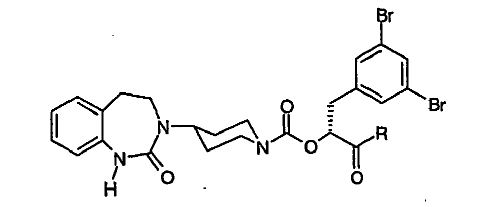

| **Beispiel** | **R** | **Ausbeute (%)** | **Massenspektrum** | **Retentionszeit HPLC (Methode)** |
|---|---|---|---|---|
| **59** | 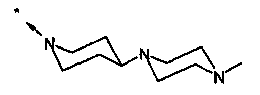 | 73 | 759/761/763 [M+H]⁺ | 10.4 min (F) |
| **60** | 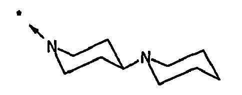 | 32 | 744/746/748 [M+H]⁺ | 12.8 min (F) |
| **61** | 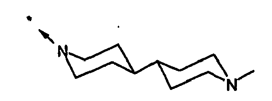 | 78 | 758/760/762 [M+H]⁺ | 12.9 min (F) |
| **62** | 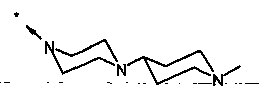 | 67 | 759/761/763 [M+H]⁺ | 9.3 min (F) |
| **63** | 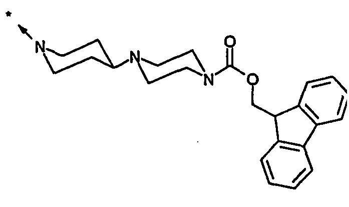 | 91 | 745/747/749 [M+H- Fmoc]⁺ | 17.6 min (F) |
| **64** | 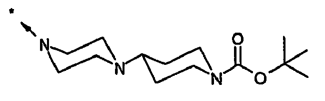 | 84 | 845/847/849 [M+H]⁺ | 14.9 min (F) |
| **65** | 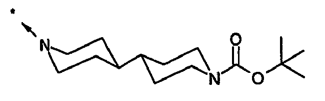 | 94 | 844/846/848 [M+H]⁺ | 27.2 min (F) |

### Beispiel 66

### 4-(2-Oxo-1,2,4,5-tetrahydro-1,3-benzdiazepin-3-yl)-piperidin-1-carbonsäure-(R)-1-(3,5-dibrom-benzyl)-2-oxo-2-(4-piperazin-1yl-piperidin-1-yl-ethylester

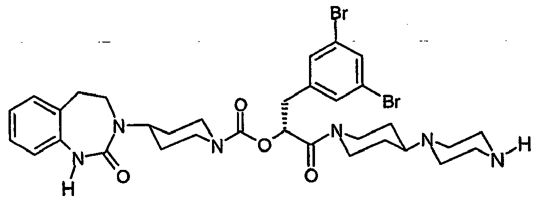

Eine Lösung von 300 mg (0.23 mmol) 4-(1-{(R)-3-(3,5-Dibrom-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-9*H*-fluoren-9-ylmethylester (Beispiel 63) in 4 mL Piperidin wurde 1 h bei RT gerührt. Die Reaktionslösung wurde zur Trockene eingeengt und der Rückstand chromatographisch (Kieselgel, Gradient DCM zu DCM/MeOH/NH₃ 90:10:1) gereinigt.
- Ausbeute:: 134 mg (79 % der Theorie)
- ESI-MS:: (M+H)⁺ = 745/747/749 (2 Br)
- Retentionszeit (HPLC):: 9.7 min (Methode F)

### Beispiel 67

### 4-(2-Oxo-1.2,4,5-tetrahydro-1,3-benzdiazepin-3-yl)-piperidin-1-carbonsäure-(R)-1-(3,5-dibrom-benzyl)-2-oxo-2-(4-piperidin-4-yl-piperazin-1-yl)-ethylester

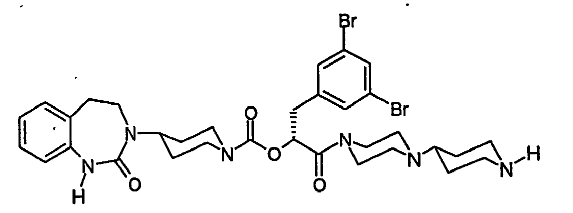

Zu einer Lösung von 180 mg (0.21 mmol) 4-(2-Oxo-1,2,4,5-tetrahydro-1,3-benzdiazepin-3-yl)-piperidin-1-carbonsäure-(R)-1-(3,5-dibrom-benzyl)-2-[4-(1-*tert-*butoxy-carbonyl-piperidin-4-yl)-piperazin-1-yl]-2-oxo-ethylester (Beispiel 64) in 2 mL Wasser wurden 1.8 mL HCI (3.2 M) gegeben und das Reaktionsgemisch 3 h bei RT gerührt. Man versetzte mit 25 mL EtOAc und 20 mL 17% Na₂CO₃-Lösung trennte die organische Phase ab, extrahierte die wässrige Phase erneut mit 25 mL EtOAc, wusch die vereinigten organischen Phasen mit 10 mL gesättigter NaCI-Lösung und trocknete über Na₂SO₄. Nach Entfernen des Trocken- und Lösungsmittels wurde der Rückstand in Diethylether suspendiert, die organische Phase abdekantiert und der Rückstand getrocknet.
- Ausbeute:: 130 mg (82% der Theorie)
- ESI-MS:: (M+H)⁺ = 745/747/749 (2 Br)
- Retentionszeit (HPLC):: 9.3 min (Methode F)

### Beispiel 68

### 4-(2-Oxo-1,2,4,5-tetrahydro-1,3-benzdiazepin-3-yl)-piperidin-1-carbonsäure-(R)-2-4,4'-bipiperidin-yl-1-(3,5-dibrom-benzyl)-2-oxo-2-ethylester

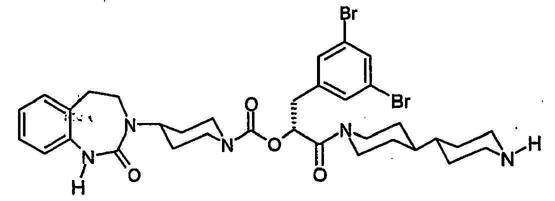

Eine Lösung von 160 mg (0.19 mmol) 1'-{(R)-3-(3,5-Dibrom-phenyl)-2-[4-(2-oxo-1,2,4,5-tetrahydro-1,3-benzdiazepin-3-yl)-piperidin-1-carbonyloxy]-propionyl}-4,4'-bi-piperidinyl-1-carbon-säure-*tert*-butylester (Beispiel 65) in 2 mL Ameisensäure wurde 1 h bei RT gerührt. Die Reaktionslösung wurde i.vac. eingeengt, der Rückstand in DCM aufgenommen, die organische Phase mit 10% Na₂CO₃-Lösung gewaschen, filtriert und zur Trockene eingeengt. Der Rückstand wurde in 10% NaOH suspendiert, 1 h bei RT gerührt, der Niederschlag filtriert, mit wenig Wasser und Diethylether gewaschen und i.vac. getrocknet.
- Ausbeute:: 86 mg (61 % der Theorie) .
- ESI-MS:: (M+H)⁺ = 744/746/748 (2 Br)
- Retentionszeit (HPLC):: 12.6 min (Methode F)

### Beispiel 69

### 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-(4-methyl-piperazin-1-yl)-piperidin-1-yl]-2-oxo-ethylester

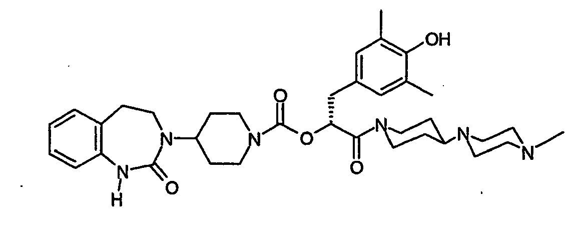

### 69a 2-Benzyloxy-5-brom-1,3-dimethylbenzol

Zu einer Lösung von 50.0 g (249 mmol) 2,6-Dimethyl-4-bromphenol in 500 mL DMF wurden 39.9 g (286 mmol) K₂CO₃ gegeben und 20 min nachgerührt. Dann wurden 34.0 mL (286 mmol) Benzylchlorid langsam zugetropft und das Reaktionsgemisch 3 h bei 100°C Badtemperatur gerührt. Nach beendeter Umsetzung wurde auf 500 mL Wasser gegossen und mit EtOAc erschöpfend extrahiert. Die organischen Phasen wurden vereint, über Na₂SO₄ getrocknet und i. vac. eingeengt.
- Ausbeute:: quantitativ
- GC-MS:: (M⁺) = 290/292 (Br)
- R_{f} =: 0.87 (Kieselgel, Cyc/EtOAc 3:1)

### 69b 2-Acetylamino-3-(4-benzyloxy-3,5-dimethyl-phenyl)-acrylsäuremethylester

Unter Stickstoffatmosphäre wurde eine Mischung aus 40.0 g (137 mmol) 2-Benzyloxy-5-brom-1,3-dimethylbenzol und 24.1 g (165 mmol) 2-Acetylamino-acrylsäuremethylester in 420 mL Triethylamin und 200 mL Acetonitril mit 3.5 g (11.2 mmol) Tri-*o*-tolyl-phosphan und 2.5 g (11.1 mmol) Pd(OAc)₂ versetzt und 18 h bei 80°C gerührt. Der Niederschlag wurde abgesaugt, das Filtrat i. vac. eingeengt und mit 800 mL DCM und 800 mL Wasser versetzt. Die organische Phase wurde abgetrennt, über Na₂SO₄ abgesaugt, das Lösungsmittel i.vac. entfernt, der Rückstand mit EtOAc verrührt, abgesaugt und i. vac getrocknet.
- Ausbeute:: 31.1 g (64% der Theorie)
- ESI-MS:: (M+H)⁺ = 354
- Retentionszeit (HPLC-MS):: 8.6 min (Methode A)

### 69c 3-(4-Benzyloxy-3,5-dimethyl-phenyl)-2-oxo-propionsäure

31.1 g (88.1 mmol) 2-Acetylamino-3-(4-benzyloxy-3,5-dimethyl-phenyl)-agrylsäure-methylester in 150 mL 1,4-Dioxan wurden mit 125 mL 4 M HCI versetzt, 7 h unter Rückfluß und über Nacht bei RT gerührt. Der Niederschlag wurde abgesaugt, mit Wasser gewaschen und bei 45°C im Vakuumtrockenschrank getrocknet.
- Ausbeute:: 14.3 g (54 % der Theorie)
- EI-MS:: (M)⁺ = 298
- Retentionszeit (HPLC-MS):: 9.0 min (Methode A)

### 69d (R)-3-(4-Benzoyl-3,5-dimethyl-phenyl)-2-hydroxy-propionsäure

Unter einer Stickstoffatmosphäre wurde eine Lösung von 14.3 g (47.8 mmol) 3-(4-Benzyloxy-3,5-dimethyl-phenyl)-2-oxo-propionsäure und 8.3 mL (59.8 mmol) Triethylamin in 170 mL THF bei -35°C mit einer Lösung von 22.1 (69.0 mmol) (1 R)-B-Chlordiisopinocampheylboran in 70 mL THF innerhalb von 30 min versetzt. Nach beendeter Zugabe wurde das Kältebad entfernt und die Reaktionslösung über Nacht bei RT gerührt. Das Reaktionsgemisch wurde bei 0°C mit 70 mL 1 M NaOH alkalische gestellt, mit 100 mL TBME versetzt, 15 min nachgerührt und die Phasen getrennt. Die organische Phase wurde mit 50 mL Wasser und dreimal mit je 50 mL 1 M NaOH gewaschen. Die vereinigten wässrigen Phasen wurden mit halbkonz. HCl sauer gestellt, mit EtOAc erschöpfend extrahiert und die vereinigten organischen Phasen über Na₂SO₄ getrocknet. Nach Entfernen des Trocken- und Lösungsmittels wurde der Rückstand ohne Reinigung weiter umgesetzt.
- Ausbeute:: 14.0 g (98% der Theorie)
- ESI-MS:: (M-H)- = 299
- Retentionszeit (HPLC-MS):: 7.9 min (Methode A)

### 69e (R)-3-(4-Benzyloxy-3,5-dimethyl-phenyl)-2-hydroxy-propionsäuremethylester

Zu einer auf 0°C gekühlten Lösung von 14.0 g (23.3 mmol) (R)-3-(4-Benzoyl-3,5-dimethyl-phenyl)-2-hydroxy-propionsäure in 150 mL MeOH wurden tropfenweise 2.0 mL (27.4 mmol) SOCl₂ zugegeben und das Reaktionsgemisch 1 h bei RT nachgerührt. Die Reaktionslösung wurde i. vac. eingeengt und der Rückstand chromatographisch (Kieselgel, Cyc/EtOAc 3:1) gereinigt.
- Ausbeute:: 5.7 g (78% der Theorie)
- ESI-MS:: (M+NH₄)⁺ = 332
- Retentionszeit (HPLC-MS):: 9.1 min (Methode A)

### 69f 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-methoxycarbonyl-ethylester

Unter Stickstoffatmosphäre wurde zu einer Lösung von 1.17 g (9.58 mmol) 4-Dimethylaminopyridin in 50 mL Pyridin 1.93 g (9.58 mmol) Chlorameisensäure-4-nitrophenylester gegeben, 1.5 h bei RT gerührt, mit 3.0 g (9.58 mmol) (R)-3-(4-Benzyloxy-3,5-dimethyl-phenyl)-2-hydroxy-propionsäuremethylester versetzt und 20 min bei RT gerührt. Anschließend wurden 2.35 g (9.58 mmol) 3-Piperidin-4-yl-1,3,4,5-tetrahydro-1,3-benzdiazepin-2-on zugegeben und 20 h bei RT gerührt. Das Reaktionsgemisch wurde i. vac. eingeengt, der Rückstand in EtOAc aufgenommen, die organische Phase mit 10% KHSO₄ und gesättigter NaHCO₃-Lösung gewaschen und über Na₂SO₄ getrocknet. Nach Entfernen des Trocken- und Lösungsmittels wurde der Rückstand chromatographisch (Kieselgel, Gradient Cyc/EtOAc 1:1 zu 1:2) gereinigt.
- Ausbeute:: 3.21 g (57% der Theorie)
- ESI-MS:: (M+H)⁺ = 586
- Retentionszeit (HPLC-MS):: 10.4 min (Methode A)

### 69g 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-carboxy-ethylester

Eine Lösung von 3.21 g (5.48 mmol) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-methoxycarbonyl-ethylester in 80 mL THF wurde mit einer Lösung von 200 mg (8.35 mmol) LiOH in 40 mL Wasser versetzt und 1 h bei RT gerührt. Das Reaktionsgemisch wurde i. vac. eingeengt, der Rückstand in 100 mL Wasser aufgenommen, mit 2 M HCI sauer gestellt, der Niederschlag abgesaugt und im Vakuumtrockenschrank bei 40°C getrocknet.
- Ausbeute:: quantitativ
- ESI-MS:: (M+H)⁺ = 572
- Retentionszeit (HPLC-MS):: 9.2 min (Methode A)

### 69h 4-(2-Oxo-1,2,4,5-tetrahydro-1,3-benzdiazepin-3-yl)-piperidin-1-carbonsäure-(R)-2-(4-hydroxy-3,5-dimethyl-phenyl)-1-carboxy-ethylester

3.72 g (6.51 mmol) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-carboxy-ethylester in 50 mL DCM wurden mit 300 mg 10% Pd/C versetzt und bei RT und 3 bar Wasserstoff bis zum Reaktionsstillstand geschüttelt. Der Katalysator wurde abgesaugt und das Lösungsmittel i. vac. eingeengt. Der Rückstand wurde mit DIPE verrieben und abgesaugt.
- Ausbeute:: 2.41 g (77% der Theorie)
- ESI-MS:: (M+H)⁺ = 482
- Retentionszeit (HPLC-MS):: 7.0 min (Methode A)

### 69i 4-(2-Oxo-1,2,4,5-tetrahydro-1,3-benzdiazepin-3-yl)-piperidin-1-carbonsäure (R)-1-(4-hydroxy-3,5-dimethyl-benzyl)-2-[4-(4-methyl-piperazin-1-yl)-piperidin-1-yl]-2-oxo-ethylester

Eine Lösung von 70 mg (0.15 mmol) 4-(2-Oxo-1,2,4,5-tetrahydro-1,3-benzdiazepin-3-yl)-piperidin-1-carbonsäure-(R)-2-(4-hydroxy-3,5-dimethyl-phenyl)-1-carboxy-ethylester, 51 mg (0.16 mmol) TBTU und 26 µL (0.18 mmol) Triethylamin in 1 mL DMF wurde 1 h bei RT gerührt. Dann erfolgte die Zugabe von 27 mg (0.15 mmol) 1-Methyl-4-piperidin-4-yl-piperazin und das Reaktionsgemisch wurde 16 h bei RT gerührt. Die Reaktionslösung wurde über einen Spritzenfilter filtriert und ohne weitere Aufarbeitung direkt via HPLC gereinigt. Die das Produkt enthaltenden Fraktionen wurden vereinigt und lyophilisiert.
- Ausbeute:: 39 mg (42 % der Theorie)
- ESI-MS:: (M+H)⁺ = 647
- Retentionszeit (HPLC-MS):: 5.3 min (Methode A)

Analog wurden folgende Verbindungen aus jeweils 70 mg (Beispiele 70 bis 76), aus jeweils 100 mg (Beispiele 77 und 78) bzw. aus 400 mg (Beispiel 79) 4-(2-Oxo-1,2,4,5-tetrahydro-1,3-benzdiazepin-3-yl)-piperidin-1-carbonsäure-(R)-2-(4-hydroxy-3,5-dimethyl-phenyl)-1-carboxy-ethylester und der entsprechenden Menge an Amin hergestellt:

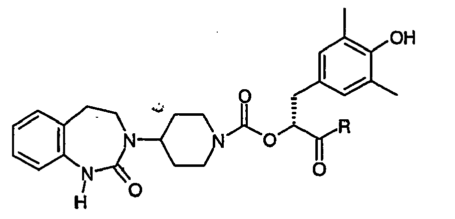

| **Beispiel** | **R** | **Ausbeute (%)** | **Massenspektrum** | **Retentionszeit HPLC (Methode)** |
|---|---|---|---|---|
| **70** | 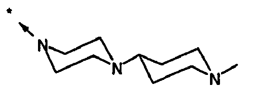 | 47 | 647 [M+H]⁺ | 4.9 min (A) |
| **71** | 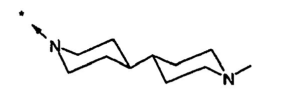 | 41 | 646 [M+H]⁺ | 5.8 min (A) |
| **72** | 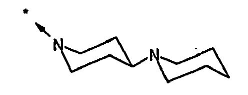 | 32 | 632 [M+H]⁺ | 5.8 min (A) |
| **73** | 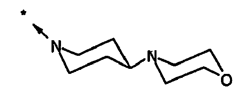 | 38 | 634 [M+H]⁺ | 5.6 min (A) |
| **74** | 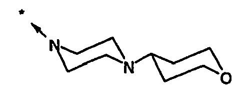 | 39 | 634 [M+H]⁺ | 5.7 min (A) |
| **75** | 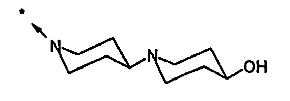 | 37 | 648 [M+H]⁺ | 5.5 min (A) |
| **76** | 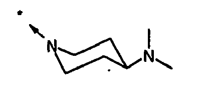 | 38 | 592 [M+H]⁺ | 5.6 min (A) |
| **77** | 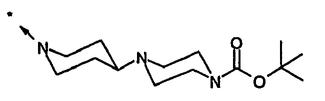 | 21 | 733 [M+H]⁺ | 6.4 min (A) |
| **78** | 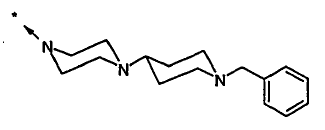 | 36 | 723 [M+H]⁺ | 5.3 min (A) |
| **79** | 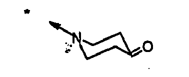 | 71 | 563 [M+H]⁺ | 6.9 min (A) |

### Beispiel 80

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-(4-piperazin-1-yl-piperidin-1-yl)-ethylester

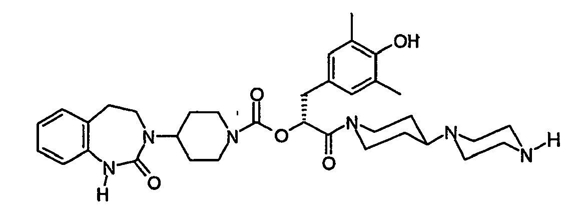

Eine Lösung von 32 mg (0.04 mmol) 4-(1-{(R)-3-(4-Hydroxy-3,5-dimethyl-phenyl)-2-[4-(2-oxo-1,2,4,5-tetrahydro-1,3-benzdiazepin-3-yl)-piperidin-1-carbonyloxy]-propionyl}-p)peridin-4-yl)-piperazin-1-carbonsäure-*tert*-butylester (Beispiel 77) in 5 mL 2 M HCI wurde 20 h bei RT gerührt und und anschliessend lyophilisiert, wobei das Produkt als Bis-Hydrochlorid anfiel.
- Ausbeute:: quantitativ
- ESI-MS:: (M+H)⁺ = 633
- Retentionszeit (HPLC-MS):: 5.0 min (Methode A)

### Beispiel 81

### 4-(2-Oxo-1,2,4.5-tetrahvdro-1,3-benzdiazepin-3-yl)-piperidin-1-carbonsäure-(R)-1-(4hydroxy-3,5-dimethyl-benzyl)-2-oxo-2-(4-piperidin-4-yl-piperazin-1-yl)-ethylester

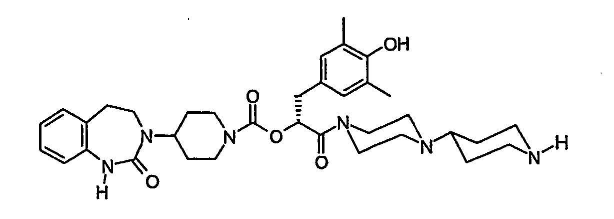

Eine Lösung von 54 mg (0.08 mmol) 4-(2-Oxo-1,2,4,5-tetrahydro-1,3-benzdiazepin-3-yl)-piperidin-1-carbonsäure-(R)-2-[4-(1-benzyl-piperidin-4-yl)-piperazin-1-yl]-1-(4-hydroxy-3,5-dimethyl-benzyl)-2-oxo-ethylester (Beispiel 78) in 5 mL MeOH wurden mit 20 mg 10% Pd/C versetzt und bei RT und 3 bar Wasserstoff bis zum Reaktionsstillstand geschüttelt. Der Katalysator wurde abgesaugt und das Lösungsmittel i.vac. eingeengt. Der Rückstand wurde mit DIPE verrieben, abgesaugt und im Hochvakuum getrocknet.
- Ausbeute:: 35.0 mg (74% der Theorie)
- ESI-MS:: (M+H)⁺ = 633
- Retentionszeit (HPLC-MS):: 4.9 min (Methode A)

### Beispiel 82

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(4-hydroxy-4-methyl-[1,4']bipiperidinyl-1'-yl)-2-oxo-ethylester

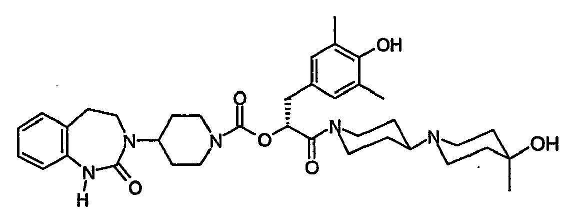

Eine Lösung von 50 mg (0.09 mmol) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-(4-oxo-piperidin-1-yl)-ethylester (Beispiel 79) in 1.5 mL DCM wurde mit 21 mg (0.18 mmol) 4-Methyl-piperidin-4-ol und 10.3 µl (0.19 mmol) AcOH versetzt, auf 0°C abgekühlt und 2 h gerührt. Dann wurden 28 mg (0.19 mmol) Natrium-triacetoxyborhydrid zugegeben und über Nacht bei 0°C gerührt. Nach Entfernen des Lösungsmittels wurde der Rückstand mit 2 mL DMF versetzt und via HPLC gereinigt. Die das Produkt enthaltenden Fraktionen wurden vereinigt und lyophilisiert.
- Ausbeute:: 25 mg (42% der Theorie)
- ESI-MS:: (M+H)⁺ = 662
- Retentionszeit (HPLC-MS):: 2.90 min (Methode E)

### Beispiel 83

### 4-(2-Oxo-1,2,4,5-tetrahydro-1,3-benzdiazepin-3-yl)-piperidin-1-carbonsäure-(R)-2-(4,4-dimethyl-[1,4']bipiperidinyl-1'-yl)-1-(4-hydroxy-3,5-dimethyl-benzyl)-2-oxo-ethylester

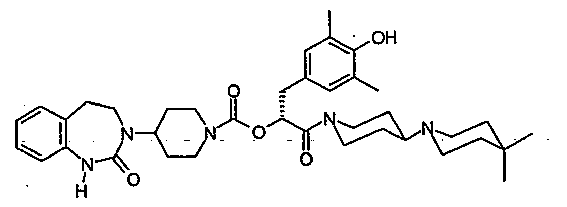

Analog Beispiel 82 konnte aus 50.0 mg (0.09 mmol) 4-(2-Oxo-1,2,4,5-tetrahydro-1,3-benzdiazepin-3-yl)-piperidin-1-carbonsäure-(R)-1-(4-hydroxy-3,5-di methyl-benzyl)-2-oxo-2-(4-oxo-piperidin-1-yl)-ethylester (Beispiel 79) und 31.0 mg (0.18 mmol) 4,4-Dimethylpiperidin das Produkt erhalten werden.
- Ausbeute:: 18.3 mg (31 % der Theorie)
- ESI-MS:: (M+H)⁺ = 660
- Retentionszeit (HPLC-MS):: 3.2 min (Methode E)

### Beispiel 84

### 4-(2-Oxo-1,2,4,5-tetrahydro-1,3-benzdiazepin-3-yl)-piperidin-1-carbonsäure-(R)-2-(4-amino-4-methyl-[1,4']biperidinyl-1'-yl)-1-(4-hydroxy-3,5-dimethyl-benzyl)-2-oxo-ethylester

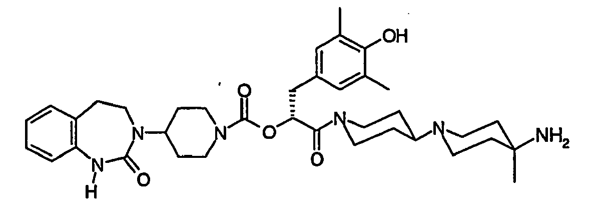

Eine Lösung von 150 mg (0.27 mmol) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-(4-oxo-piperidin-1-yl)-ethylester (Beispiel 79) in 4 mL DCM wurde mit 120 mg (0.53 mmol) (4-Methyl-piperidin-4-yl)-carbaminsäure-*tert*-butylester und 31 µL (0.56 mmol) AcOH versetzt, auf 0°C abgekühlt und 2 h gerührt. Dann wurden 85 mg (0.56 mmol) Natriumtriacetoxyborhydrid zugegeben und über Nacht bei 0°C gerührt. Dann wurde die Reaktionslösung mit 0.5 mL TFA versetzt und erneut über Nacht bei RT gerührt. Nach Entfernen der Lösemittel wurde der Rückstand in 2 mL DMF gelöst und via HPLC gereinigt. Die das Produkt enthaltenden Fraktionen wurden vereinigt und lyophilisiert, wobei das Produkt als TFA-Salz anfiel.
- Ausbeute:: 94 mg (46 % der Theorie)
- ESI-MS:: (M+H)⁺ = 661
- Retentionszeit (HPLC-MS):: 2.50 min (Methode E)

### Beispiel 85

### 4-(2-Oxo-1,2,4,5-tetrahyrdro-1,3-benzdiazepin-3-yl)-piperidin-1-carbonsäure-(R)-1-(4-amino-3,5-dimethyl-benzyl)-2-[4-(4-methyl-piperazin-1-yl)-piperidin-1-yl)-2-oxo-ethylester

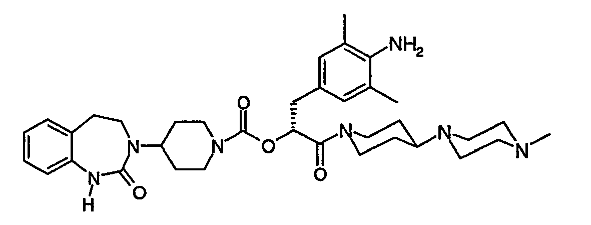

### 85a (Z,E)-2-Acetylamino-3-(4-amino-3,5-dimethyl-phenyl)-acrylsäuremethylester

Unter einer Stickstoffatmosphäre wurde zu einer Mischung aus 7.2 g (32.1 mmol) Pd(OAc)₂ und 10.1 g (32.1 mmol) Tri-o-tolyl-phosphan in 1.2 L Triethylamin und 600 mL Acetonitril zuerst eine Lösung von 90.0 g (441 mmol) 4-Brom-2,6-dimethyl-phenylamin in 200 mL Acetonitril gegeben und anschliessend eine Lösung von 65.0 g (445 mmol) 2-Acetylamino-acrylsäuremethylester in 200 mL Acetonitril zugetropft. Nach beendeter Zugabe wurde 18 h bei 80°C gerührt. Zur Vervollständigung der Reaktion wurde das Reaktionsgemisch erneut mit 4.0 g (17.8 mmol) Pd(OAc)₂ und 5.0 g (16.4 mmol) Tri-o-tolyl-phosphan versetzt und weitere 5 h bei 80°C gehalten. Man engt i.vac auf ca. 200 mL ein, versetzte den Rückstand mit 400 mL EtOAc, filtrierte den Rückstand (A) und trocknete die organische Phase über Na₂SO₄. Nach Entfernen des Trockenmittels durch Filtration über Aktivkohle wurde das Filtrat auf ca. 100 mL eingedampft, die ausgefallene Substanz abgesaugt, mit 30 mL EtOAc nach gewaschen und getrocknet.
Obiger Rückstand A wurde mit 1 L DCM, mit Na₂SO₄ und Aktivkohle versetzt und über Celite filtriert. Das Filtrat wurde eingeengt, der Rückstand mit 350 mL Diethylether versetzt, der entstandene Niederschlag abgesaugt, dieser mit 100 mL Diethylether gewaschen und getrocknet. Beide Produktfraktionen wurden vereinigt.
- Ausbeute:: 74.8 g (65% der Theorie)
- ESI-MS:: (M+H)⁺ = 263
- R_{f} =: 0.51 (Kieselgel, EtOAc)

### 85b 3-(4-Amino-3,5-dimethyl-phenyl)-2-oxo-propionsäure

Eine Suspension von 74.0 g (282 mmol) (Z,E)-2-Acetylamino-3-(4-amino-3,5-dimethyl-phenyl)-acrylsäuremethylester in 500 mL 1,4-Dioxan wurde auf 100°C erhitzt und mit 460 mL 4 M HCl versetzt, wobei sich eine Lösung bildete. Man erhitzte weitere 8 h bei 100°C und engte die abgekühlte Lösung i.vac. auf ca. 200 mL ein, wobei das Produkt kristallisierte. Man filtrierte, wusch den Rückstand mit 50 mL Wasser und trocknete das Produkt bei 50°C.
- Ausbeute:: 43.6 g (63% der Theorie)
- ESI-MS:: (M+H)⁺ = 208
- R_{f} =: 0.68 (Kieselgel, PE/EtOAc 1:1)

### 85c (R)-3-(4-Amino-3,5-dimethyl-phenyl)-2-hydroxy-propionsäuremethylester

Unter Stickstoffatmosphäre wurde eine Mischung aus 20.0 g (82.1 mmol) 3-(4-Amino-3,5-dimethyl-phenyl)-2-oxo-propionsäure und 25.7 mL (189 mmol) Triethylamin in 400 mL THF auf -35°C gekühlt. Dann wurde eine Lösung von 40.0 g (125 mmol) (1R) B-Chlordiisopinocampheylboran in 100 mL THF so zugetropft, dass die Reaktionstemperatur zwischen -35°C und -25°C blieb. Man hielt das Reaktionsgemisch 1 h bei dieser Temperatur, entfernte das Kältebad und ließ das Reaktionsgemisch über Nacht bei RT rühren. THF wurde i.vac. eingeengt, der Rückstand mit methanolischer HCI (1.25 M) versetzt und 2 h bei RT gerührt. Man engte i.vac. ein, nahm den Rückstand in 2 M HCI auf und extrahierte erschöpfend mit EtOAc. Die wässrige Phase wurde mit halbkonz. NaOH alkalisch gestellt und erschöpfend mit EtOAc extrahiert. Die vereinigten organischen Phasen wurden über Na₂SO₄ getrocknet, über Aktivkohle abgesaugt und eingeengt. Das Produkt konnte als braunes Öl erhalten werden.
- Ausbeute:: 8.3 g (45% der Theorie)
- ESI-MS:: (M+H)⁺ = 224
- R_{f} =: 0.46 (Kieselgel, PE/EtOAc 1:1)

### 85d 4-(2-Oxo-1,2,4,5-tetrahydro-1,3-benzdiazepin-3-yl)-piperidin-1-carbonsäure-(R)-2-(4-amino-3,5-dimethyl-phenyl)-1-methoxycarbonyl-ethylester

Analog Beispiel 31f konnte aus 4.0 g (17.9 mmol) (R)-3-(4-Amino-3,5-dimethylphenyl)-2-hydroxy-propionsäuremethylester und 4.8 g (19.6 mmol) 3-Piperidin-4-yl-1,3,4,5-tetrahydro-1,3-benzdiazepin-2-on das gewünschte Produkt erhalten werden.
- Ausbeute:: 3.2 g (36% der Theorie)
- ESI-MS:: (M+H)⁺ = 495
- R_{f} =: 0.35 (Kieselgel, DCM/MeOH/NH₃ 90:10:1)

### 85e 4-(2-Oxo-1,2,4,5-tetrahydro-1,3-benzdiazepin-3-yl)-piperidin-1-carbonsäure-(R)-2-(4-amino-3,5-dimethyl-phenyl)-1-carboxy-ethylester

Zu einer Lösung von 6.7 g (13.6 mmol) 4-(2-Oxo-1,2,4,5-tetrahydro-1,3-benzdiazepin-3-yl)-piperidin-1-carbonsäure-(R)-2-(4-amino-3,5-dimethyl-phenyl)-1-methoxycarbonyl-ethylester in 50 mL THF wurde eine Lösung von 500 mg (20.9 mmol) LiOH in 10 mL Wasser gegeben und das Reaktionsgemisch über Nacht bei RT gerührt. Zur Vervollständigung der Reaktion wurden weitere 300 mg (12.5 mmol) LiOH zugegeben und die Reaktionslösung 3 h bei 40°C gerührt. Man engte i.vac. ein, nahm den Rückstand in 15% K₂CO₃-Lösung auf und extrahierte erschöpfend mit DCM. Die wässrige Phase wurde mit 4 M HCI sauer gestellt, erschöpfend mit DCM extrahiert und die vereinigten organischen Phasen über Na₂SO₄ getrocknet. Nach Entfernen des Trocken- und Lösungsmittels wurde der Rückstand ohne Reinigung weiter umgesetzt.
- Ausbeute:: 4.2 g (65% der Theorie)
- ESI-MS:: (M+H)⁺ = 481
- R_{f} =: 0.21 (Kieselgel, DCM/MeOH/Cyc/NH₃ 70:15:15:2)

### 85f 4-(2-Oxo-1,2,4,5-tetrahydro-1,3-benzdiazepin-3-yl)-piperidin-1-carbonsäure-(R)-1-(4-amino-3,5-dimethyl-benzyl)-2-[4-(4-methyl-piperazin-1-yl)-piperidin-1-yl]-2-oxo-ethylester

Analog Beispiel 1h wurde aus 80 mg (0.17 mmol) 4-(2-Oxo-1,2,4,5-tetrahydro-1,3-benzdiazepin-3-yl)-piperidin-1-carbonsäure-(R)-2-(4-amino-3,5-dimethyl-phenyl)-1-carboxy-ethylester und 35 mg (0.19 mmol) 1-Methyl-4-piperidin-4-yl-piperazin das Produkt erhalten.
- Ausbeute:: 50 mg (47% der Theorie)
- ESI-MS:: (M+H)⁺ = 646
- Retentionszeit (HPLC):: 4.9 min (Methode B)

Analog wurden folgende Verbindungen aus jeweils 80 mg (Beispiele 86 bis 89) bzw. aus jeweils 100 mg (Beispiele 90 bis 92) 4-(2-Oxo-1,2,4,5-tetrahydro-1,3-benzdiazepin-3-yl)-piperidin-1-carbonsäure-(R)-2-(4-amino-3,5-dimethyl-phenyl)-1-carboxy-ethylester und der entsprechenden Menge an Amin hergestellt:

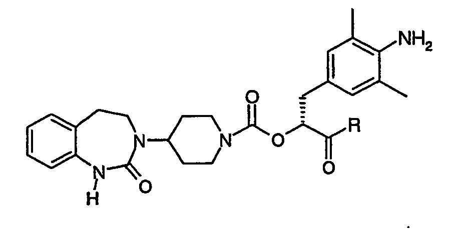

| **Beispiel** | **R** | **Ausbeute (%)** | **Massenspektrum** | **Retentionszeit HPLC (Methode)** |
|---|---|---|---|---|
| **86** | 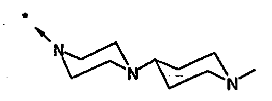 | 55 | 646 [M+H]⁺ | 3.6 min (B) |
| **87** | 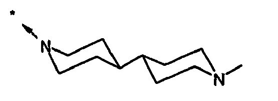 | 48 | 645 [M+H]⁺ | 4.5 min (B) |
| **88** | 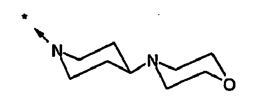 | 49 | 633 [M+H]⁺ | 4.3 min (B) |
| **89** | 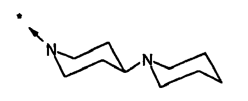 | 42 | 631 [M+H]⁺ | 4.6 min (B) |
| **90** | 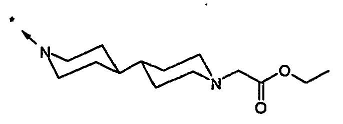 | 39 | 717 [M+H]⁺ | 4.7 min (B) |
| **91** | 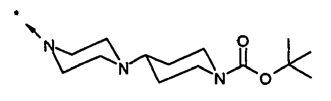 | 53 | 732 [M+H]⁺ | 5.2 min (B) |
| **92** | 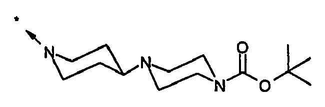 | 44 | 732 [M+H]⁺ | 5.0 min (B) |

### Beispiel 93

### 4-(2-Oxo-1,2,4,5-tetrahvdro-1,3-benzdiazepin-3-yl)-piperidin-1-carbonsäure-(R)-1-(4-amino-3,5-dimethyl-benzyl)-2-oxo-2-(4-piperidin-4-yl-piperazin-1-yl)-ethylester

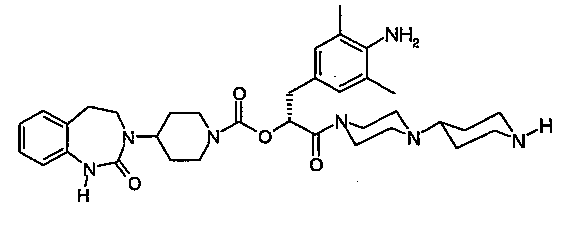

Eine Lösung von 80 mg (0.11 mmol) 4-(2-Oxo-1,2,4,5-tetrahydro-1,3-benzdiazepin-3-yl)-piperidin-1-carbonsäure-(R)-1-(4-amino-3,5-dimethyl-benzyl)-2-[4-(1-tert-butoxycarbonyl-piperidin-4-yl)-piperazin-1-yl]-2-oxo-ethylester (Beispiel 91) in 2 mL 4 M HCI wurde über Nacht bei RT gerührt. Das Reaktionsgemisch wurde mit festem K₂CO₃ alkalisch gestellt, erschöpfend mit DCM extrahiert und die vereinigten organischen Phasen i.vac. eingeengt. Weitere Reinigung erfolgte via HPLC. Die das Produkt enthaltenden Fraktionen wurden vereinigt und lyophilisiert.
- Ausbeute:: 4 mg (6% der Theorie)
- ESI-MS:: (M+H)⁺ = 632
- Retentionszeit (HPLC):: 3.6 min (Methode A)

### Beispiel 94

### 4-(2-Oxo-1,2,4,5-tetrahydro-1,3-benzdiazepin-3-yl)-piperidin-1-carbonsäure-(R)-1-(4-amino-3,5-dimethyl-benzyl)-2-oxo-2-(4-piperazin-1-yl-piperidin-1-yl)-ethylester

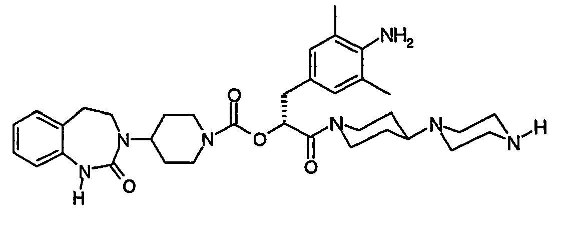

Analog Beispiel 93 wurde aus 65.0 mg (0.089 mmol) 4-(1-{(R)-3-(4-Amino-3,5-dimethyl-phenyl)-2-[4-(2-oxo-1,2,4,5-tetrahydro-1,3-benzdiazepin-3-yl)-piperidin-1-carbonyloxy]-propionyl)-piperidin-4-yl)-piperazin-1-carbonsäure-*tert*-butylester (Beispiel 92) das Produkt erhalten.
- Ausbeute:: 7 mg (12% der Theorie)
- ESI-MS:: (M+H)⁺ = 632
- Retentionszeit (HPLC):: 3.9 min (Methode A)

### Beispiel 95

### 4-(2-Oxo-1,2,4,5-tetrahydro-1,3-benzdiazepin-3-yl)-piperidin-1-carbonsäure-(R)-1-(4-amino-3,5-dimethyl-benzyl)-2-(1'-carboxymethyl-4,4'-bipiperidinyl-1-yl)-2-oxo-ethylester

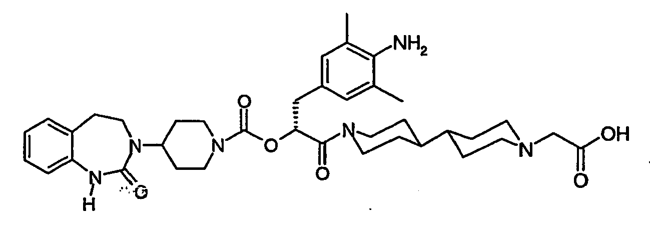

Zu einer Lösung von 55 mg (0.08 mmol) 4-(2-Oxo-1,2,4,5-tetrahydro-1,3-benzdiazepin-3-yl)-piperidin-1-carbonsäure-(R)-1-(4-amino-3,5-di methyl-benzyl)-2-(1'-ethoxycarbonylmethyl-4,4'-bipiperidinyl-1-yl)-2-oxo-ethylester (Beispiel 90) in 5 mL THF wurde eine Lösung von 3.1 mg (0.13 mmol) LiOH in 1 mL Wasser gegeben und das Reaktionsgemisch über Nacht bei RT gerührt. Man engte i.vac. ein, nahm den Rückstand in 1 mL DMF auf und reinigte das Rohprodukt via HPLC. Die das Produkt enthaltenden Fraktionen wurden vereinigt und lyophilisiert.
- Ausbeute:: 22 mg (42% der Theorie)
- ESI-MS:: (M+H)⁺ = 689
- Retentionszeit (HPLC):: 4.8 min (Methode A)

### Beispiel 96

### 4-(2-Oxo-1,2,4,5-tetrahydro-1,3-benzdiazepin-3-yl)-piperidin-1-carbonsäure-(R)-1-(3-chlor-4-methyl-benzyl)-2-[4-4-methyl-piperazin-1-yl)-piperidin-1-yl]-2-oxo-ethylester

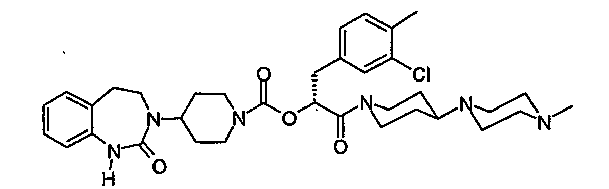

### 96a (Z,E)-2-Acetylamino-3-(3-chlor-4-methyl-phenyl)-acrylsäuremethylester

Unter Argonatmosphäre wurden zu einer Mischung aus 5.2 mL (39.0 mmol) 4-Brom-2-chlor-1-methyl-benzol und 10.0 g (69.9 mmol) 2-Acetylamino-acrylsäuremethylester in jeweils 100 mL Acetonitril und Triethylamin 0.65 g (2.9 mmol) Pd(OAc)₂ und 0.9 g (2.9 mmol) Tri-o-tolyl-phosphan zugegeben und das Reaktionsgemisch 20 h auf 90°C erhitzt. Nach dem Abkühlen wurde i.vac. eingeengt, der Rückstand mit DCM und Wasser versetzt, filtriert, die organische Phase abgetrennt und über Na₂SO₄ getrocknet. Nach Entfernen des Trocken- und Lösungsmittels wurde der Rückstand chromatographisch (Kieselgel, Gradient PE/EtOAc 1:1 zu EtOAc) gereinigt.
- Ausbeute:: 7.67 g (73% der Theorie)
- ESI-MS:: (M+H)⁺ = 268/270 (CI)
- Schmelzpunkt:: 144-145°C
- R_{f} =: 0.65 (Polygram, EtOAc)

### 96b 3-(3-Chlor-4-methyl-phenyl)-2-oxo-propionsäure

Zu einer Lösung von 7.67 g (28.7 mmol) (Z,E)-2-Acetylamino-3-(3-chlor-4-methylphenyl)-acrylsäuremethylester in 150 mL EtOH wurden 100 mL 4 M HCI zugegeben und die Reaktionslösung 4 h unter Rückfluss erhitzt. Das EtOH wurde i.vac. entfernt, der Rückstand im Eisbad gekühlt, wobei der ölige Rückstand erstarrte. Der Feststoff wurde filtriert, mit Wasser gewaschen, mit PE verrührt, erneut abgesaugt, mit wenig PE nachgewaschen und getrocknet.
Die vereinigten Filtrate wurden eingeengt, in 50 mL MeOH gelöst, mit 50 mL 4 M NaOH versetzt und 2 h unter Rückfluss erhitzt. MeOH wurde i.vac. abgezogen, der wässrige Rückstand mit konz. HCl angesäuert und erschöpfend mit EtOAc extrahiert. Die vereinigten organischen Phasen wurden mit gesättigter NaCI-Lösung gewaschen und über Na₂SO₄ getrocknet. Nach Entfernen des Trocken- und Lösungsmittels wurde der Rückstand aus DCM umkristallisiert und mit der ersten Produktfraktion vereinigt.
- Ausbeute:: 2.02 g (33% der Theorie)
- ESI-MS:: (M-H)⁻ = 211/213 (Cl)

### 96c (R)-3-(3-Chlor-4-methyl-phenyl)-2-hydroxy-propionsäure

Unter Stickstoffatmosphäre wurde eine Mischung aus 2.6 g (12.23 mmol) 3-(3-Chlor-4-methyl-phenyl)-2-oxo-propionsäure und 2.1 mL (15.1 mmol) Triethylamin in 40 mL THF auf -35°C gekühlt. Dann wurde eine Lösung von 5.87 g (18.30 mmol) (1R)-B-Chlordiisopinocampheylboran in 20 mL THF so zugetropft, dass die Reaktionstemperatur zwischen -35°C und -25°C blieb. Man entfernte das Kältebad und ließ das Reaktionsgemisch über Nacht bei RT rühren. Unter Eiskühlung wurden 30 mL 1 M NaOH und 60 mL Diethylether zugetropft und 15 min nachgerührt. Die wässrige Phase wurde abgetrennt, die organische Phase zweimal mit je 20 mL 1 M NaOH und einmal mit 20 mL Wasser extrahiert. Die vereinigten wässrigen Phasen wurden unter Eiskühlung mit halbkonz. HCI sauer gestellt, zweimal mit je 60 mL EtOAc extrahiert und die vereinigten organischen Phasen über Na₂SO₄ getrocknet. Nach Entfernen des Trocken- und Lösungsmittels wurde der Rückstand ohne Reinigung weiter umgesetzt.
- Ausbeute:: 2.8 g (85% der Theorie)

### 96d (R)-3-(3-Chlor-4-methyl-phenyl)-2-hydroxy-propionsäuremethylester

Zu einer Lösung von 2.8 g (10.4 mmol) (R)-3-(3-Chlor-4-methyl-phenyl)-2-hydroxy-propionsäure in 100 mL MeOH wurden unter Eiskühlung 2.0 mL (27.4 mmol) SOCI₂ langsam zugetropft und die Reaktionslösung 1 h bei 0°C und 1 h bei RT nachgerührt. Das Reaktionsgemisch wurde i.vac. eingeengt, der Rückstand in EtOAc aufgenommen, die organische Phase mit gesättigter NaHCO₃-Lösung gewaschen und über Na₂SO₄ getrocknet. Nach Entfernen des Trocken- und Lösungsmittels wurde der Rückstand chromatographisch (Kieselgel, Gradient DCM zu DCM/MeOH 50:1) gereinigt.
- Ausbeute:: 2.12 g (89% der Theorie)
- ESI-MS:: (M+H)⁺ = 229/231 (Cl)
- R_{f} =: 0.34 (Polygram, DCM)

### 96e 4-(2-Oxo-1,2,4,5-tetrahydro-1,3-benzdiazepin-3-yl)-piperidin-1-carbonsäure-(R)-2-(3-chlor-4-methyl-phenyl)-1-methoxycarbonyl-ethylester

Analog Beispiel 31 konnte aus 2.1 g (9.18 mmol) (R)-3-(3-Chlor-4-methyl-phenyl)-2-hydroxy-propionsäuremethylester und 2.45 g (9.99 mmol) 3-Piperidin-4-yl-1,3,4,5-tetrahydro-1,3-benzdiazepin-2-on das gewünschte Produkt erhalten werden.
- Ausbeute:: 3.4 g (74% der Theorie)
- ESI-MS:: (M+H)⁺ = 500/502 (Cl)
- R_{f} =: 0.52 (Polygram, EtOAc)

### 96f 4-(2-Oxo-1,2,4,5-tetrahydro-1,3-benzdiazepin-3-yl)-piperidin-1-carbonsäure-(R)-1-carboxy-2-(3-chlor-4-methyl-phenyl)-ethylester

Zu einer Lösung von 3.38 g (6.76 mmol) 4-(2-Oxo-1,2,4,5-tetrahydro-1,3-benzdiazepin-3-yl)-piperidin-1-carbonsäure-(R)-2-(3-chlor-4-methyl-phenyl)-1-methoxycarbonyl-ethylester in 30 mL THF wurde eine Lösung von 0.34 g (14.2 mmol) LiOH in 20 mL Wasser gegeben und das Reaktionsgemisch 7 h bei RT gerührt. Man engte i.vac. ein, verdünnte mit 80 mL Wasser, extrahierte die wässrige Phase zweimal mit je 50 mL Diethylether, säuerte die wässrige Phase mit 4 M HCl an und rührte 30 min nach. Das ausgefallene Produkt wurde abgesaugt, mit Wasser gewaschen und getrocknet.
- Ausbeute:: 3.2 g (97% der Theorie)
- ESI-MS:: (M+H)⁺ = 486/488 (Cl)

### 96g 4-(2-Oxo-1,2,4,5-tetrahydro-1,3-benzdiazepin-3-yl)-piperidin-1-carbonsäure-(R)-1-(3-chlor-4-methyl-benzyl)-2-[4-(4-methyl-piperazin-1-yl)-piperidin-1-yl]-2-oxo-ethylester

Eine Lösung von 100 mg (0.21 mmol) 4-(2-Oxo-1,2,4,5-tetrahydro-1,3-benzdiazepin-3-yl)-piperidin-1-carbonsäure-(R)-1-carboxy-2-(3-chlor-4-methyl-phenyl)-ethylester, 70.0 mg (0.22 mmol) TBTU und 35 µL (0.27 mmol) Triethylamin in 10 mL THF wurde 1 h bei RT gerührt. Dann erfolgte die Zugabe von 40 mg (0.22 mmol) 1-Methyl-4-piperidin-4-yl-piperazin und das Reaktionsgemisch wurde anschliessend über Nacht bei RT gerührt. Die Reaktionslösung wurde mit 20 mL halbgesättigter NaHCO₃-Lösung verrührt, zweimal mit je 20 mL EtOAc extrahiert und die vereinigten organischen Phasen über Na₂SO₄ getrocknet. Nach Entfernen des Trocken- und Lösungsmittels wurde der Rückstand chromatographisch (Alox, Aktivitätsstufe II-III DCM/MeOH 40:1) gereinigt.
- Ausbeute:: 123 mg (83% der Theorie)
- ESI-MS:: (M+H)⁺ = 651/653 (Cl)
- R_{f} =: 0.52 (Polygram-Alox, DCM/MeOH 25:1)

Analog wurden folgende Verbindungen aus jeweils 100 mg 4-(2-Oxo-1,2,4,5-tetrahydro-1,3-benzdiazepin-3-yl)-piperidin-1-carbonsäure-(R)-1-carboxy-2-(3-chlor-4-methyl-phenyl)-ethylester und der entsprechenden Menge an Amin hergestellt:

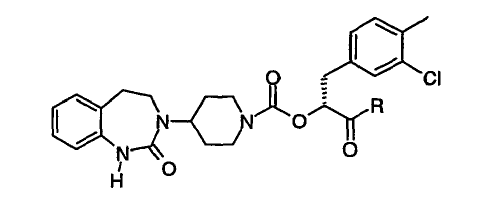

| **Beispiel** | **R** | **Ausbeute (%)** | **Massenspektrum** | **R_{f} (Polygram- Alox, Laufmittel)** |
|---|---|---|---|---|
| **97** | 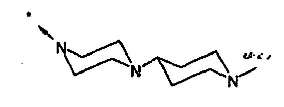 | 74 | 651/653 [M+H]⁺ | 0.48 DCM/MeOH 25:1 |
| **98** | 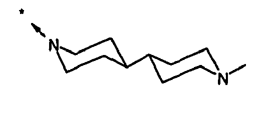 | 83 | 650/652 [M+H]⁺ | 0.55 (DCM/MeOH 25:1) |
| **99** | 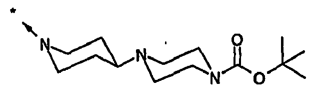 | 74 | 737/739 [M+H]⁺ | n.e |
| **100** | 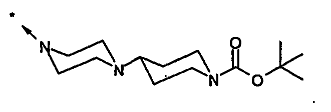 | 65 | 737/739 [M+H]⁺ | 0.33 (DCM/MeOH 50:1) |

### Beispiel 101

### 4-(2-Oxo-1,2,4,5-tetrahydro-1,3-benzdiazepin-3-yl)-piperidin-1-carbonsäure-(R)-1-(3-chlor-4-methyl-benzyl)-2-oxo-2-(4-piperazin-1-yl-piperidin-1-yl)-ethylester

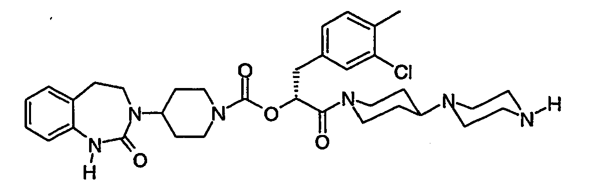

Zu einer Lösung von 130 mg (0.14 mmol) 4-(1-{(R)-3-(3-Chlor-4-methyl-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-*tert*-butylester (Beispiel 99) in 1 mL MeOH wurden 10 mL 1 M HCl zugegeben und das Reaktionsgemisch 24 h bei RT gerührt. Die Reaktionsmischung wurde ohne weitere Aufarbeitung direkt gefriergetrocknet. Das Produkt fiel als Bis-Hydrochlorid Salz an.
- Ausbeute:: 112 mg (95% der Theorie)
- ESI-MS:: (M+H)⁺ = 637/639 (Cl)

### Beispiel 102

### 4-(2-Oxo-1,2,4,5-tetrahydro-1,3-benzdiazepin-3-yl)-piperidin-1-carbonsäure-(R)-1-(3-chlor-4-methyl-benzyl)-2-oxo-2-(4-piperidin-4-yl-piperazin-1-yl)-ethylester

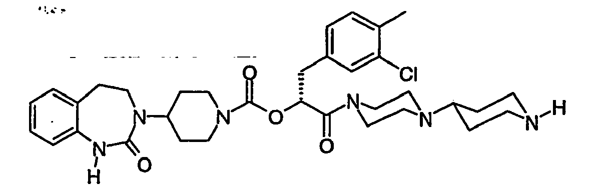

Analog Beispiel 101 konnte aus 110 mg (0.13 mmol) 4-(2-Oxo-1,2,4,5-tetrahydro-1,3-benzdiazepin-3-yl)-piperidin-1-carbonsäure-(R)-1-(3-chlor-4-methyl-benzyl)-2-[4-(1-*tert*-butoxy-carbonyl-piperidin-4-yl)-piperazin-1-yl]-2-oxo-ethylester (Beispiel 100) das Produkt erhalten werden, welches als Bis-Hydrochlorid Salz anfiel.
- Ausbeute:: 99 mg (93% der Theorie)
- ESI-MS:: (M+H)⁺ = 637/639 (CI)

### Beispiel 103

### 4-(2-Oxo-1,2,4,5-tetrahydro-1,3-benzdiazepin-3-yl)-piperidin-1-carbonsäure-(R)-1-(3-brom-4-methyl-benzyl)-2-[4-(4-methyl-piperazin-1-yl)-piperidin-1-yl]-2-oxo-ethylester

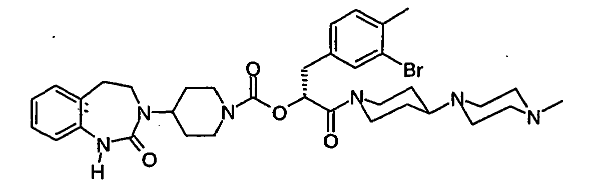

### 103a (Z,E)-2-Acetylamino-3-(3-brom-4-methyl-phenyl)-acrylsäure

Analog Beispiel 1a konnte aus 6.0 g (30.1 mmol) 3-Brom-4-methyl-benzaldehyd und 5.3 g (45.3 mmol) N-Acetylglycin das Produkt erhalten werden.
- Ausbeute:: 4.7 g (52% der Theorie)
- ESI-MS:: (M+H)⁺ = 298/300 (Br)
- R_{f} =: 0.12 (Kieselgel, DCM/MeOH/Cyc/NH₃ 70:15:15:2)

### 103b 3-(3-Brom-4-methyl-phenyl)-2-oxo-propionsäure

Zu einer auf 105°C erhitzten Lösung von 4.7 g (15.8 mmol) (Z,E)-2-Acetylamino-3-(3-brom-4-methyl-phenyl)-acrylsäure in 50 mL 1,4-Dioxan wurden 28 mL 4 M HCI gegeben und das Reaktionsgemisch weitere 5 h bei dieser Temperatur gehalten. Man entfernte das 1,4-Dioxan i.vac., versetzte den gekühlten Rückstand mit Wasser, filtrierte den entstandenen Niederschlag ab und trocknete diesen im Umlufttrockenschrank.
- Ausbeute:: 2.9 g (72% der Theorie)
- ESI-MS:: (M-H)⁻ = 255/257 (Br)
- R_{f} =: 0.18 (Kieselgel, DCM/MeOH/NH₃ 80:20:2)

### 103c (R)-3-(3-Brom-4-methyl-phenyl)-2-hydroxy-propionsäure

Unter Stickstoffatmosphäre wurde eine Mischung aus 2.9 g (11.3 mmol) 3-(3-Brom-4-methyl-phenyl)-2-oxo-propionsäure und 2.0 mL (15.1 mmol) Triethylamin in 40 mL THF auf -35°C gekühlt. Dann wurde eine Lösung von 5.44 g (17.0 mmol) (1R)-B-Chlordiisopinocampheylboran in 20 mL THF so zugetropft, dass die Reaktionstemperatur zwischen -35°C und -25°C blieb; man hielt das Reaktionsgemisch 1 h bei dieser Temperatur, entfernte dann das Kältebad und ließ über Nacht bei RT rühren. Zur Vervollständigung der Reaktion wurden weitere von 3.0 g (9.4 mmol) (1R)-B-Chlordiisopinocampheylboran zugegeben und weitere 5 h gerührt. Unter Eiskühlung wurden 30 mL 1 M NaOH und 30 mL TBME zugetropft und 15 min nachgerührt. Die wässrige Phase wurde abgetrennt, die organische Phase mit 15 mL 1 M NaOH und mit 25 mL Wasser extrahiert. Die vereinigten wässrigen Phasen wurden unter Eiskühlung mit 2 M HCI sauer gestellt, dreimal mit je 40 mL TBME extrahiert und die vereinigten organischen Phasen über Na₂SO₄ getrocknet. Nach Entfernen des Trocken- und Lösungsmittels wurde der Rückstand ohne Reinigung weiter umgesetzt.
- Ausbeute:: 3.0 g (ca. 70% Produktanteil, 72% der Theorie)
- ESI-MS:: (M-H)⁻ = 257/259 (Br)
- R_{f} =: 0.12 (Kieselgel, PE/EtOAc 1:1)

### 103d (R)-3-(3-Brom-4-methyl-phenyl)-2-hydroxy-propionsäuremethylester

Eine Lösung von 2.8 g (ca. 70%; 7.56 mmol) (R)-3-(3-Brom-4-methyl-phenyl)-2-hydroxy-propionsäure in methanolischer HCl (1.25 M) wurde 4 h bei RT gerührt. Man engte i.vac. ein und reinigte den Rückstand chromatographisch (Kieselgel, Gradient PE/EtOAc 9:1 zu PE/EtOAc 1:9).
- Ausbeute:: 1.6 g (77% der Theorie)
- ESI-MS:: (M+H)⁺ = 273/275 (Br)
- R_{f} =: 0.72 (Kieselgel, PE/EtOAc 1:1)

### 103e 4-(2-Oxo-1,2,4,5-tetrahydro-1,3-benzdiazepin-3-yl)-piperidin-1-carbonsäure-(R)-2-(3-brom-4-methyl-phenyl)-1-methoxycarbonyl-ethylester

Analog Beispiel 1f konnte aus 1.5 g (5.49 mmol) (R)-3-(3-Brom-4-methyl-phenyl)-2-hydroxy-propionsäuremethylester und 1.7 g (5.52 mmol) 4-(2-Oxo-1,2,4,5-tetrahydro-1,3-benzdiazepin-3-yl)-piperidin-1-carbonylchlorid das Rohprodukt erhalten werden. Dieses wurde chromatographisch gereinigt (Kieselgel, Gradient DCM/MeOH/NH₃ 100:0:0 zu DCM/MeOH/NH₃ 0:95:5). Die Produktfraktionen wurden vereinigt, i.vac. eingeengt, mit DIPE verrieben, abgesaugt und getrocknet.
- Ausbeute:: 1.1 g (37% der Theorie)
- ESI-MS:: (M+H)⁺ = 544/546 (Br)
- R_{f} =: 0.70 (Kieselgel, DCM/MeOH/Cyc/NH₃ 70:15:15:2)

### 103f 4-(2-Oxo-1,2,4,5-tetrahydro-1,3-benzdiazepin-3-yl)-piperidin-1-carbonsäure-(R)-2-(3-brom-4-methyl-phenyl)-1-carboxy-ethylester

Zu einer Lösung von 1.0 g (1.84 mmol) 4-(2-Oxo-1,2,4,5-tetrahydro-1,3-benzdiazepin-3-yl)-piperidin-1-carbonsäure-(R)-2-(3-brom-4-methyl-phenyl)-1-methoxycarbonyl-ethylester in 20 mL THF wurde eine Lösung von 70 mg (2.92 mmol) LiOH in 5 mL Wasser gegeben und das Reaktionsgemisch 5 h bei RT gerührt. Die Reaktionslösung wurde i.vac. eingeengt, der Rückstand mit DCM versetzt, die organische Phase mit 1 M KHSO₄-Lösung gewaschen und über Na₂SO4 getrocknet. Nach Entfernen des Trocken- und Lösungsmittels wurde der Rückstand mit DIPE verrieben, abgesaugt und getrocknet.
- Ausbeute:: 0.95 g (98% der Theorie)
- ESI-MS:: (M+H)⁺ = 530/532 (Br)
- R_{f} =: 0.29 (Kieselgel, DCM/MeOH/Cyc/NH₃ 70:15:15:2)

### 103g 4-(2-Oxo-1,2,4,5-tetrahydro-1,3-benzdiazepin-3-yl)-piperidin-1-carbonsäure-(R)-1-(3-brom-4-methyl-benzyl)-2-[4-(4-methyl-piperazin-1-yl)-piperidin-1-yl]-2-oxo-ethylester

Analog Beispiel 1 h konnte aus 100 mg (0.19 mmol) 4-(2-Oxo-1,2,4,5-tetrahydro-1,3-benzdiazepin-3-yl)-piperidin-1-carbonsäure-(R)-2-(3-brom-4-methyl-phenyl)-1-carboxy-ethylester und 38 mg (0.21 mmol) 1-Methyl-4-piperidin-4-yl-piperazin das Produkt erhalten werden.
- Ausbeute: ,: 90 mg (69% der Theorie)
- ESI-MS:: (M+H)⁺ = 695/697 (Br)
- R_{f} =: 0.59 (Kieselgel, DCM/MeOH/Cyc/NH₃ 70:15:15:2)

Analog wurden folgende Verbindungen aus jeweils 100 mg 4-(2-Oxo-1,2,4,5-tetrahydro-1,3-benzdiazepin-3-yl)-piperidin-1-carbonsäure-(R)-2-(3-brom-4-methylphenyl)-1-carboxy-ethylester und der entsprechenden Menge an Amin hergestellt:

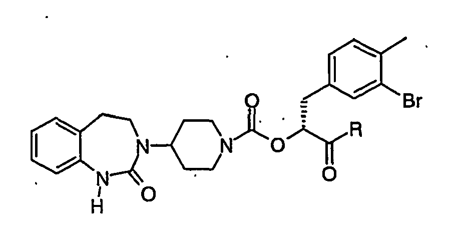

| **Beispiel** | **R** | **Ausbeute (%)** | **Massenspektrum** | **R_{f} (Kieselgel, Laufmittel) oder Retentionszeit HPLC (Methode)** |
|---|---|---|---|---|
| **104** | 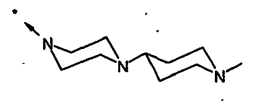 | 65 | 695/697 [M+H]⁺ | 0.58 (DCM/MeOH/ Cyc/NH₃ 70:15:15:2) |
| **105** | 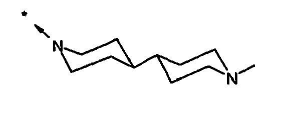 | 61 | 694/696 [M+H]⁺ | 0.57 (DCM/MeOH/ Cyc/NH₃ 70:15:15:2) |
| **105** | 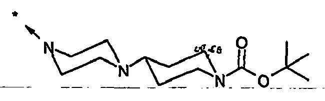 | 54 | 781/783 [M+H]⁺ | 7.1 min (A) |
| **106** | 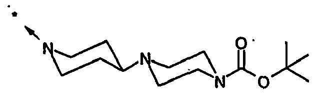 | 61 | 781/783 [M+H]⁺ | 6.9 min (A) |

### Beispiel 107

### 4-(2-Oxo-1,2,4,5-tetrahydro-1,3-benzdiazepin-3-yl)-piperidin-1-carbonsäure-(R)-1-(3-brom-4-methyl-benzyl)-2-oxo-2-(4-piperazin-1-yl-piperidin-1-yl)-ethylester

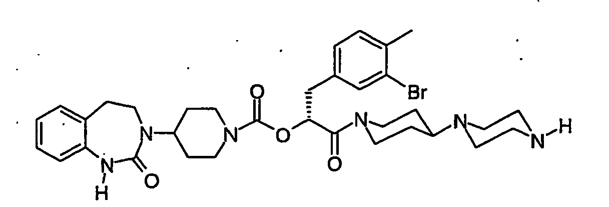

Analog Beispiel 93 konnte aus 90 mg (0.12 mmol) 4-(1-{(R)-3-(3-Brom-4-methylphenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-*tert*-butylester (Beispiel 106) das Produkt erhalten werden.
- Ausbeute:: 15 mg (19% der Theorie)
- ESI-MS:: (M+H)⁺ = 681/683 (Br)
- Retentionszeit (HPLC):: 5.7 min (Methode A)

### Beispiel 108

### 4-(2-Oxo-1,2,4.5-tetrahydro-1,3-benzdiazepin-3-yl)-piperidin-1-carbonsäure-(R)-1-(3-brom-4-methyl-benzyl)-2-oxo-2-(4-piperidin-4-yl-piperazin-1-yl)-ethylester

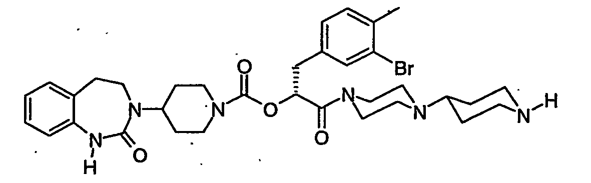

Analog Beispiel 93 konnte aus 80 mg (0.12 mmol) 4-(2-Oxo-1,2,4,5-tetrahydro-1,3-benzdiazepin-3-yl)-piperidin-1-carbonsäure-(R)-1-(3-brom-4-methyl-benzyl)-2-[4-(1-*tert*-butoxy-carbonyl-piperidin-4-yl)-piperazin-1-yl]-2-oxo-ethylester (Beispiel 105) das Produkt erhalten werden.
- Ausbeute:: 44 mg (63% der Theorie)
- ESI-MS:: (M+H)⁺= 681/683 (Br)
- Retentionszeit (HPLC):: 5.5 min (Methode A)

### Beispiel 109

### 4-(2-Oxo-1,2,4,5-tetrahydro-1,3-benzdiazepin-3-yl)-piperidin-1-carbonsäure-(R)-1-(6-amino-5-methyl-pyridin-3-ylmethyl)-2-[4-(4-methyl-piperazin-1-yl)-piperidin-1-yl]-2-oxo-ethylester

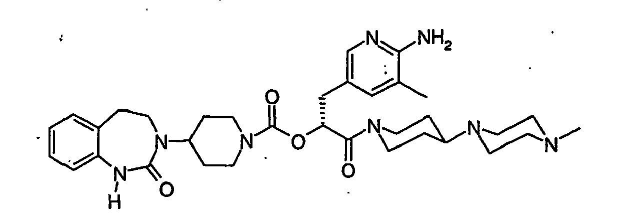

### 109a (Z,E)-2-Acetylamino-3-(6-amino-5-methyl-pyridin-3-yl)-acrylsäuremethylester

Unter Stickstoffatmosphäre wurde zu einer Mischung aus 33.6 g (180 mmol) 5-Brom-3-methyl-pyridin-2-ylamin, 28.9 g (198 mmol) 2-Acetylamino-acrylsäuremethylester, 4.42 g (14.4 mmol) Tri-o-tolyl-phosphan und 30.9 mL (180 mmol) Ethyldiisopropylamin in 500 mL Butyronitril 6.58 g (7.19 mmol) Tris-(dibenzylidenaceton)-Palladium gegeben und das Reaktionsgemisch 17 h auf 110°C erhitzt. Die Reaktionslösung wurde i.vac. eingeengt und der Rückstand mit ca. 500 mL Wasser verrührt. Der Niederschlag wurde filtriert, aus Acetonitril umkristallisiert und getrocknet.
Die wässrige Mutterlauge wurde eingeengt und der Rückstand chromatographisch (Kieselgel, EtOAc/MeOH/NH₃ 90:10:1) gereinigt. Die das Produkt enthaltenden Fraktionen wurden eingeengt, der Rückstand mit wenig Acetonitril verrieben, filtriert, getrocknet und mit obiger Produktfraktion vereinigt.
- Ausbeute:: 16.6 g (37% der Theorie)
- ESI-MS:: (M+H)⁺ = 250
- R_{f} =: 0.46 (Kieselgel, EtOAc/MeOH/NH₃ 90:10:1)

### 109b 3-(6-Amino-5-methyl-pyridin-3-yl)-2-oxo-propionsäure

Zu einer Lösung von 15.57 g (62.46 mmol) (Z,E)-2-Acetylamino-3-(6-amino-5-methyl-pyridin-3-yl)-acrylsäuremethylester in 250 mL 1,4-Dioxan wurden 230 mL 4 M HCI gegeben, das Reaktionsgemisch für 1.5 h unter Rückfluss erhitzt und weitere 16 h bei RT gerührt. Man engte i.vac. ein, verrieb den Rückstand mit EtOAc/DIPE (1:1), filtrierte und trocknete im Umlufttrockenschrank. Das Produkt fiel als HydrochloridSalz an.
- Ausbeute:: 14.4 g (100% der Theorie)
- ESI-MS:: (M+H)⁺ = 195
- Retentionszeit (HPLC):: 2.7 min (Methode A)

### 109c (R)-3-(6-Amino-5-methyl-pyridin=3-yl)-2-hydroxy-propionsäuremethylester

Unter Argonatmosphäre wurde eine Mischung aus 13.8 g (59.9 mmol) 3-(6-Amino-5-methyl-pyridin-3-yl)-2-oxo-propionsäure und 17.5 mL (125.7 mmol) Triethylamin in 140 mL THF auf -35°C gekühlt. Dann wurde eine Lösung von 40.3 g (126 mmol) (1R)-B-Chlordiisopinocampheylboran in 210 mL THF so zugetropft, dass die Reaktionstemperatur zwischen -35°C und -25°C blieb; man hielt das Reaktionsgemisch 3 h bei dieser Temperatur ehe man bei 0-5°C mit 150 mL 1 M NaOH versetzte und das Reaktionsgemisch 2 h bei RT nachrührte. Man versetzte mit 200 mL TBME, trennte die organische Phase ab und säuerte diese mit 200 mL 2 M HCI an. Die wässrige Phase wurde abgetrennt, eingeengt, der Rückstand in THF/MeOH (1:1) aufgenommen, filtriert und das Filtrat anschliessend eingeengt. Das so erhaltene Rohprodukt (12.5 g) wurde in 300 mL MeOH gelöst, unter Eiskühlung tropfenweise mit 4.3 mL (59.3 mmol) SOCl₂ versetzt und weitere 2 h bei RT gerührt. Man engte i.vac ein und reinigte den Rückstand chromatographisch (Kieselgel, EtOAc/MeOH/NH₃ 90:10:1).
- Ausbeute:: 5.62 g (45% der Theorie)
- ESI-MS:: (M+H)⁺ = 211
- Retentionszeit (HPLC):: 2.4 min (Methode A)

### 109d 4-(2-Oxo-1,2,4,5-tetrahydro-1,3-benzdiazepin-3-yl)-piperidin-1-carbonsäure-(R)-2-(6-amino-5-methyl-pyridin-3-yl)-1-methoxycarbonyl-ethylester

Analog Beispiel 31f konnte aus 2.75 g (13.10 mmol) (R)-3-(6-Amino-5-methyl-pyridin-3-yl)-2-hydroxy-propionsäuremethylester und 3.21 g (13.10 mmol) 3-Piperidin-4-yl-1,3,4,5-tetrahydro-1,3-benzdiazepin-2-on das gewünschte Produkt erhalten werden.
- Ausbeute:: 1.38 g (22% der Theorie)
- ESI-MS:: (M+H)⁺ = 482
- Retentionszeit (HPLC):: 4.9 min (Methode C)

### 109e 4-(2-Oxo-1,2,4,5-tetrahydro-1,3-benzdiazepin-3-yl)-piperidin-1-carbonsäure-(R)-2-(6-amino-5-methyl-pyridin-3-yl)-1-carboxy-ethylester

Zu einer Lösung von 1.27 g (2.64 mmol) 4-(2-Oxo-1,2,4,5-tetrahydro-1,3-benzdiazepin-3-yl)-piperidin-1-carbonsäure-(R)-2-(6-amino-5-methyl-pyridin-3-yl)-1-methoxycarbonyl-ethylester in 30 mL THF wurde eine Lösung von 100 mg (4.18 mmol) LiOH in 25 mL Wasser zugegeben und die Reaktionslösung für 1 h bei RT gerührt. Man engte i.vac. ein, nahm den Rückstand in Wasser auf, versetzte unter Rühren mit 2 M KHSO₄-Lösung, dekantierte die überstehende Lösung ab, trocknete den Rückstand, verrührte diesen mit THF und filtrierte das Produkt.
- Ausbeute:: 0.92 g (74% der Theorie)
- ESI-MS:: (M+H)⁺ = 468
- Retentionszeit (HPLC):: 4.8 min (Methode A)

### 109f 4-(2-Oxo-1,2,4,5-tetrahydro-1,3-benzdiazepin-3-yl)-piperidin-1-carbonsäure-(R)-1-(6-amino-5-methyl-pyridin-3-ylmethyl)-2-[4-(4-methyl-piperazin-1-yl)-piperidin-1-yl]-2-oxo-ethylester

Analog Beispiel 1h konnte aus 50 mg (0.11 mmol) 4-(2-Oxo-1,2,4,5-tetrahydro-1,3-benzdiazepin-3-yl)-piperidin-1-carbonsäure-(R)-2-(6-amino-5-methyl-pyridin-3-yl)-1-carboxy-ethylester und 20 mg (0.11 mmol) 1-Methyl-4-piperidin-4-yl-piperazin das Produkt erhalten werden.
- Ausbeute:: 6 mg (9% der Theorie)
- ESI-MS:: (M+H)⁺ = 633
- Retentionszeit (HPLC):: 4.4 min (Methode A)

Analog wurden folgende Verbindungen aus jeweils 50 mg 4-(2-Oxo-1,2,4,5-tetrahydro-1,3-benzdiazepin-3-yl)-piperidin-1-carbonsäure-(R)-2-(6-amino-5-methyl-pyridin-3-yl)-1-carboxy-ethylester und der entsprechenden Menge an Amin hergestellt:

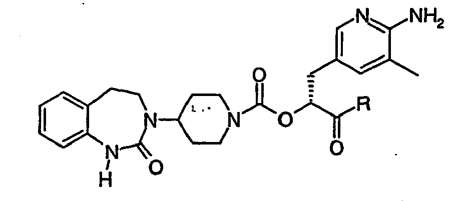

| **Beispiel** | **R** | **Ausbeute (%)** | **Massenspektrum** | **Retentionszeit HPLC (Methode)** |
|---|---|---|---|---|
| **110** | 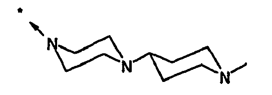 | 19 | 633 [M+H]⁺ | 4.4 min (D) |
| **111** | 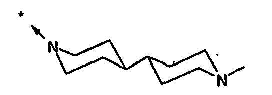 | 29 | 632 [M+H]⁺ | 5.6 min (D) |
| **112** | 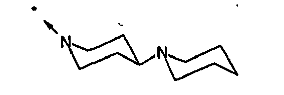 | 25 | 618 [M+H]⁺ | 5.4 min (D) |
| **113** | 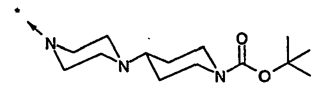 | 54 | 719 [M+H]⁺ | 6.8 min (A) |
| **114** | 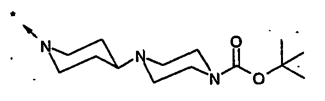 | 61 | 719 [M+H]⁺ | 6.5 min (D) |

### Beispiel 115

### 4-(2-Oxo-1,2,4,5-tetrahydro-1,3-benzdiazepin-3-yl)-piperidin-1-carbonsäure-(R)-1-(6-amino-5-methyl-pyridin-3-ylmethyl)-2-oxo-2-(4-piperazin-1-yl-piperidin-1-yl)-ethylester

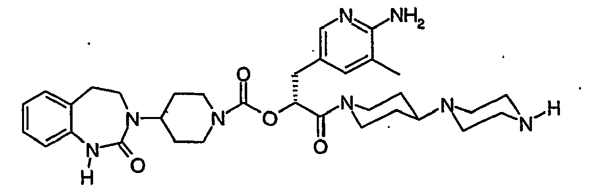

Eine Lösung von 75 mg (0.10 mmol) 4-(1-{(R)-3-(6-Amino-5-methyl-pyridin-3-yl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-*tert*-butylester (Beispiel 114) in 5 mL 2 M HCI wurde 20 h bei RT gerührt. Nach Lyophilisation des Reaktionsgemisches wurde der Rückstand in 1 mL DMF gelöst, mit 0.6 mL gesättigter K₂CO₃-Lösung alkalisch gestellt und via HPLC gereinigt. Die das Produkt enthaltenden Fraktionen wurden vereinigt und erneut lyophilisiert.
- Ausbeute:: 28 mg (44% der Theorie)
- ESI-MS:: (M+H)⁺= 619
- Retentionszeit (HPLC):: 3.8 min (Methode A)

### Beispiel 116

### 4-(2-Oxo-1,2,4,5-tetrahydro-1,3-benzdiazepin-3-yl)-piperidin-1-carbonsäure-(R)-1-(6-amino-5-methyl-pyridin-3-ylmethyl)-2-oxo-2-(4-yl-piperidin-4-yl-piperazin-1-yl)-ethylester

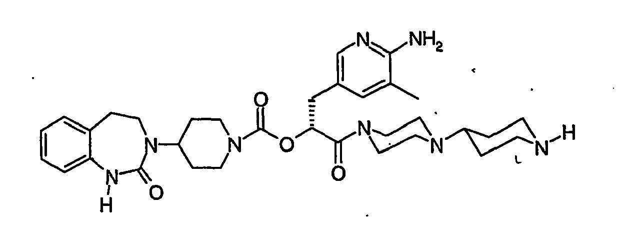

Analog Beispiel 115 konnte aus 66 mg (0.09 mmol) 4-(2-Oxo-1,2,4,5-tetrahydro-1,3-benzdiazepin-3-yl)-piperidin-1-carbonsäure-(R)-1-(6-amino-5-methyl-pyridin-3-ylmethyl)-2-[4-(1-*tert*-butoxy-carbonyl-piperidin-4-yl)-piperazin-1-yl]-2-oxo-ethylester (Beispiel 113) das Produkt erhalten werden.
- Ausbeute:: 29 mg (51 % der Theorie)
- ESI-MS:: (M+H)⁺=619
- Retentionszeit (HPLC):: 3.5 min (Methode A)

### Beispiel 117

### 4-(2-Oxo-1,2,4,5-tetrahydro-1,3-benzdiazepin-3-yl)-piperidin-1-carbonsäure-(R)-1-(4-hydroxy-benzyl)-2-[4-(4-methyl-piperazin-1-yl)-piperidin-1-yl]-2-oxo-ethylester

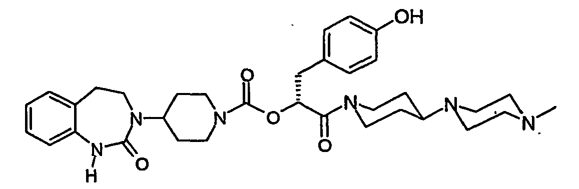

### 117a (Z,E)-2-Acetylamino-3-(4-benzyloxy-phenyl)-acrylsäure

Analog Beispiel 1 a konnte aus 30.0 g (141 mmol) 4-Benzyloxy-benzaldehyd und 24.8 g (212 mmol) N-Acetyl-glycin das Produkt erhalten werden.
- Ausbeute:: 32.0 g (73% der Theorie)
- ESI-MS:: (M+H)⁺ = 312
- R_{f} =: 0.35 (Kieselgel, DCM/MeOH/AcOH 90:10:1)

### 117b 3-(4-Benzyloxy-phenyl)-2-oxo-propionsäure

Analog Beispiel 1b konnte aus 32.0 g (103 mmol) (Z,E)-2-Acetylamino-3-(4-benzyloxy-phenyl)-acrylsäure das Produkt erhalten werden.
- Ausbeute:: 12.4 g (45% der Theorie)
- ESI-MS:: (M-H)⁻ = 269
- R_{f} =: 0.30 (Kieselgel, DCM/MeOH/AcOH 80:20:2)

### 117c (R)-3-(4-Benzyloxy-phenyl)-2-hydroxy-propionsäure

Analog Beispiel 1 c konnte aus 11.5 g (42.6 mmol) 3-(4-Benzyloxy-phenyl)-2-oxo-propionsäure und 16.7 g (52.1 mmol) (1R)-B-Chlordiisopinocampheylboran das Produkt erhalten werden.
- Ausbeute:: 7.43 g (64% der Theorie)
- ESI-MS:: (M+Na)⁺ = 294
- Retentionszeit (HPLC):: 13.3 min (Methode F)

### 117d (R)-3-(4-Benzyloxy-phenyl)-2-hydroxy-propionsäuremethylester

Analog Beispiel 46d konnte aus 7.3 g (26.8 mmol) (R)-3-(4-Benzyloxy-phenyl)-2-hydroxy-propionsäure das Produkt erhalten werden.
- Ausbeute:: 7.6 g (99% der Theorie)
- Retentionszeit (HPLC):: 16.7 min (Methode F)

### 117e 4-(2-Oxo-1,2,4,5-tetrahydro-1,3-benzdiazepin-3-yl)-piperidin-1-carbonsäure-(R)-2-(4-benzyloxy-phenyl)-1-methoxycarbonyl-ethylester

Analog Beispiel 31f konnte aus 7.6 g (26.5 mmol) (R)-3-(4-Benzyloxy-phenyl)-2-hydroxy-propionsäuremethylester und 6.5 g (26.5 mmol) 3-Piperidin-4-yl-1,3,4,5-tetrahydro-1,3-benzdiazepin-2-on das Produkt erhalten werden.
- Ausbeute:: 4.1 g (28% der Theorie)
- ESI-MS:: (M+H)⁺ = 558
- R_{f} =: 0.25 (Kieselgel, *n*-Hexan/EtOAc 3:7)
- Retentionszeit (HPLC):: 22.0 min (Methode F)

### 117f 4-(2-Oxo-1,2,4,5-tetrahydro-1,3-benzdiazepin-3-yl)-piperidin-1-carbonsäure-(R)-1-carboxy-2-(4-benzyloxy-phenyl)-ethylester

Analog Beispiel 58f konnte aus 4.1 g (7.4 mmol) (4-(2-Oxo-1,2,4,5-tetrahydro-1,3-benzdiazepin-3-yl)-piperidin-1-carbonsäure-(R)-2-(4-benzyloxy-phenyl)-1-methoxycarbonyl-ethylester und 264 mg (11.0 mmol) LiOH das Produkt erhalten werden.
- Ausbeute:: 2.7 g (68% der Theorie)
- ESI-MS:: (M+H)⁺ = 544
- Retentionszeit (HPLC):: 18.8 min (Methode F)

### 117g 4-(2-Oxo-1,2,4,5-tetrahydro-1,3-benzdiazepin-3-yl)-piperidin-1-carbonsäure-(R)-1-carboxy-2-(4-hydroxy-phenyl)-ethylester

Zu einer Lösung von 2.2 g (4.1 mmol) 4-(2-Oxo-1,2,4,5-tetrahydro-1,3-benzdiazepin-3-yl)-piperidin-1-carbonsäure-(R)-1-carboxy-2-(4-benzyloxy-phenyl)-ethylester und 450 mg (4.4 mmol) Triethylamin in 90 mL MeOH wurden 220 mg 10% Pd/C gegeben und das Reaktionsgemisch bei 3 bar H₂ 24 h hydriert. Der Katalysator wurde filtriert, zweimal mit MeOH gewaschen und das Filtrat i.vac. eingeengt. Der Rückstand wurde in 20 mL Wasser aufgenommen und mit 10% HCI auf pH 2-3 eingestellt. Der ausgefallene Niederschlag wurde filtriert, mit wenig Wasser gewaschen und bei 50°C getrocknet.
- Ausbeute:: 1.4 g (76% der Theorie)
- ESI-MS:: (M+H)⁺ = 454
- R_{f} =: 0.65 (Kieselgel, DCM/MeOH/AcOH 80:20:2)

### 117h 4-(2=Oxo-1,2,4,5-tetrahydro-1,3-benzdiazepin-3-yl)-piperidin-1-carbonsäure-(R)-1-(4-hydroxy-benzyl)-2-[4-(4-methyl-piperazin-1-yl)-piperidin-1-yl]-2-oxo-ethylester

Unter Stickstoffatmosphäre wurde zu einer Lösung von 140 mg (0.31 mmol) 4-(2-Oxo-1,2,4,5-tetrahydro-1,3-benzdiazepin-3-yl)-piperidin-1-carbonsäure-(R)-1-carboxy-2-(4-hydroxy-phenyl)-ethylester, 150 mg (0.39 mmol) HATU und 80 µL (0.47 mmol) Ethyldiisopropylamin in 5 mL DMF 74 mg (0.40 mmol) 1-Methyl-4-piperidin-4-yl-piperazin gegeben und das Reaktionsgemisch 6 h bei RT gerührt. Die Reaktionslösung wurde i.vac. eingeengt und der Rückstand chromatographisch (Kieselgel, DCM/MeOH/NH₃ 93:7:0.7) gereinigt.
- Ausbeute:: 100 mg (52% der Theorie)
- ESI-MS:: (M+H)⁺ = 619
- R_{f} =: 0.6 (Kieselgel, DCM/MeOH/NH₃ 80:20:2)

Analog wurden folgende Verbindungen aus jeweils 140 mg (Beispiele 118 und 119), 150 mg (Beispiele 120 und 121), 200 mg (Beispiele 122 und 123) oder 230 mg (Beispiel 124) 4-(2-Oxo-1,2,4,5-tetrahydro-1,3-benzdiazepin-3-yl)-piperidin-1-carbonsäure-(R)-1-carboxy-2-(4-hydroxy-phenyl)-ethylester und der entsprechenden Menge an Amin hergestellt:

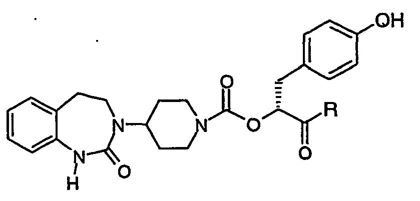

| **Beispiel** | **R** | **Ausbeute (%)** | **Massenspektrum** | **R_{f} (Kieselgel, Laufmittel)** |
|---|---|---|---|---|
| **118** | 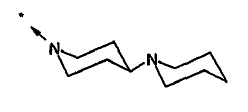 | 27 | 604 [M+H]⁺ | 0.70 (DCM/MeOH/ NH₃ 80:20:2) |
| **119** | 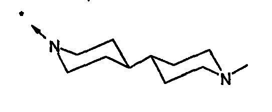 | 47 | 618 [M+H]⁺ | 0.65 (DCM/MeOH/ NH₃ 80:20:2) |
| **120** | 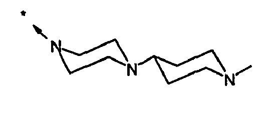 | 64 | 619 [M+H]⁺ | 0.35 (DCM/MeOH/ NH₃ 80:20:2) |
| **121** | 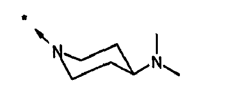 | 54 | 564 [M+H]⁺ | 0.40 (DCM/MeOH/ NH₃ 80:20:2) |
| **122** | 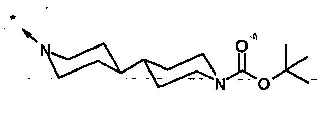 | 45 | 704 [M+H]⁺ | 0.65 (DCM/MeOH/ NH₃ 80:20:2) |
| **123** | 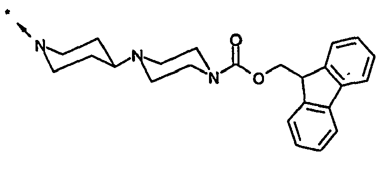 | 43 | 827 [M+H]⁺ | 0.40 (DCM/MeOH/ AcOH 90:10:1) |
| **124** | 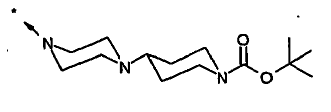 | 53 | 705 [M+H]⁺ | 0.65 (DCM/MeOH/ NH₃ 80:20:2) |

### Beispiel 125

### 4-(2-Oxo-1,2,4,5-tetrahydro-1,3-benzdiazepin-3-yl)-piperidin-1-carbonsäure-(R)-1-(4-hydroxy-benzyl)-2-oxo-2-(4-piperazin-1-yl-piperidin-1-yl-ethylester

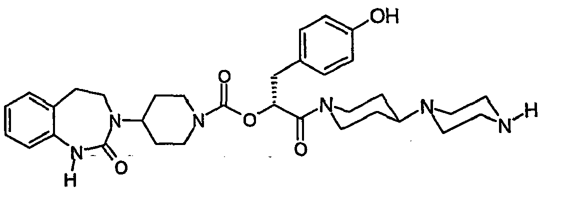

Eine Lösung von 250 mg (0.30 mmol) 4-(1-{(R)-3-(4-Hydroxy-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-9H-fluoren-9-ylmethylester (Beispiel 123) in 4 mL Piperidin wurde bei RT 30 min gerührt. Das Reaktionsgemisch wurde i.vac. eingeengt und der Rückstand chromatographisch (Kieselgel, DCM/MeOH/NH₃ 90:10:1) gereinigt.
- Ausbeute:: 40 mg (22% der Theorie)
- ESI-MS:: (M+H)⁺ = 605
- Retentionszeit (HPLC):: 4.7 min (Methode F)

### Beispiel 126

### 4-(2-Oxo-1,2,4,5-tetrahydro-1,3-benzdiazepin-3-yl)-piperidin-1-carbonsäure-(R)-1-(4-hydroxy-benzyl)-2-oxo-2-(4-piperidin-4-yl-piperazin-1-yl)-ethylester

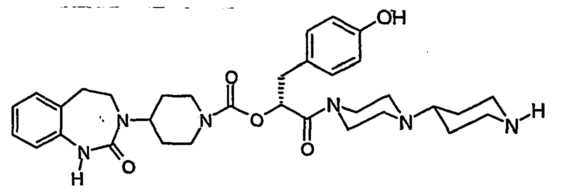

Zu einer Lösung von 210 mg (0.30 mmol) 4-(2-Oxo-1,2,4,5-tetrahydro-1,3-benzdiazepin-3-yl)-piperidin-1-carbonsäure-(R)-1-(4-hydroxy-benzyl)-2-[4-(1-*tert-*butoxy-carbonyl-piperidin-4-yl)-piperazin-1-yl]-2-oxo-ethylester (Beispiel 124) in 1.5 mL Ameisensäure wurde 3 h bei RT gerührt. Das Reaktionsgemisch wurde i.vac. eingeengt und der Rückstand chromatographisch (Kieselgel, DCM/MeOH/NH₃ 90:10:1) gereinigt.
- Ausbeute:: 40 mg (22% der Theorie)
- ESI-MS:: (M+H)⁺ = 605
- R_{f} =: 0.45 (Kieselgel, DCM/MeOH/NH₃ 80:20:2)
- Retentionszeit (HPLC):: 4.6 min (Methode F)

### Beispiel 127

### 4-(2-Oxo-1,2,4,5-tetrahydro-1,3-benzdiazepin-3-yl)-piperin-1-carbonsäure-(R)-2 4,4'-bipiperidinyl-1-yl-1-(4-hydroxy-benzyl)-2-oxo-ethylester

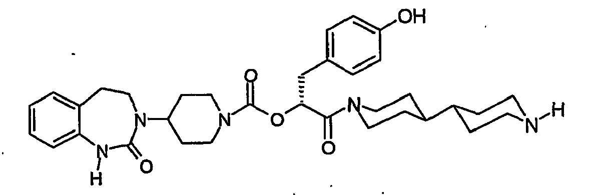

Analog Beispiel 126 konnte aus 130 mg (0.19 mmol) 1'-{(R)-3-(4-Hydroxy-phenyl)-2-[4-(2-oxo-1,2,4,5-tetrahydro-1,3-benzdiazepin-3-yl)-piperidin-1-carbonyloxy]-propionyl}-4,4'-bipiperidinyl-1-carbonsäure-*tert*-butylester (Beispiel 122) das Produkt erhalten werden.
- Ausbeute:: 70 mg (63% der Theorie)
- ESI-MS:: (M+H)⁺ = 604
- R_{f} =: 0.20 (Kieselgel, DCM/MeOH/NH₃ 80:20:2)
- Retentionszeit (HPLC):: 6.9 min (Methode F)

### Beispiel 128

### 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-[4-(4-methyl-piperazin-1-yl)-piperidin-1-yl]-2-oxo-ethylester

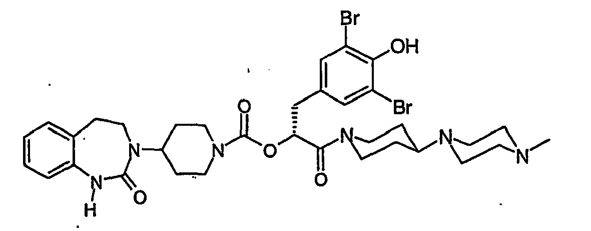

### 128a (Z,E)-3-(4-Acetoxy-3,5-dibrom-phenyl)-2-acetylamino-acrylsäure

Analog Beispiel 1a konnte aus 30 g (107 mmol) 3,5-Dibrom-4-hydroxy-benzaldehyd und 18.8 g (161 mmol) N-Acetyl-glycin das Produkt erhalten werden.
- Ausbeute:: 35.7 g (79% der Theorie)
- ESI-MS:: (M+H)⁺ = 420/422/424 (2 Br)
- R_{f} =: 0.20 (Kieselgel, DCM/MeOH/AcOH 90:10:1)

### 128b 3-(3,5-Dibrom-4-hydroxy-phenyl)-2-oxo-propionsäure

Analog Beispiel 1b konnte aus 35.7 g (84.8 mmol) (Z,E)-3-(4-Acetoxy-3,5-dibromphenyl)-2-acetylamino-acrylsäure das Produkt erhalten werden.
- Ausbeute:: 20.5 g (72% der Theorie)
- ESI-MS:: (M-H)⁻ = 335/337/339 (2 Br)
- R_{f} =: 0.35 (Kieselgel, DCM/MeOH/AcOH 80:20:2)

### 128c (R)-3-(3,5-Dibrom-4-hydroxy-phenyl)-2-hydroxy-propionsäure

Analog Beispiel 1c konnte aus 14.5 g (42.9 mmol) 3-(3,5-Dibrom-4-hydroxy-phenyl)-2-oxo-propionsäure und 30.9 g (96.33 mmol) (1R)-B-Chlordiisopinocampheylboran das Produkt erhalten werden.
- Ausbeute:: 12.7 g (87% der Theorie)
- ESI-MS:: (M-H)⁻ = 337/339/341 (2 Br)
- R_{f} =: 0.4 (Kieselgel, DCM/MeOH/AcOH 80:20:2)
- Retentionszeit (HPLC):: 6.4 min (Methode F)

### 128d (R)-3-(3,5-Dibrom-4-hydroxy-phenyl)-2-hydroxy-propionsäuremethylester

Analog Beispiel 46d konnte aus 14.0 g (34.8 mmol) 3(R)-3-(3,5-Dibrom-4-hydroxyphenyl)-2-hydroxy-propionsäure das Produkt erhalten werden.
- Ausbeute:: 7.0 g (57% der Theorie)
- ESI-MS:: (M-H)⁻ = 351/353/355 (2 Br)
- Retentionszeit (HPLC):: 9.8 min (Methode F)

### 128e (R)-3-[3,5-Dibrom-4-(2-trimethylsilanyl-ethoxymethoxy)-phenyl]-2-hydroxy-propionsäuremethylester

Unter Stickstoffatmosphäre wurden zu einer Lösung von 6.78 g (19.2 mmol) (R)-3-(3,5-Dibrom-4-hydroxy-phenyl)-2-hydroxy-propionsäuremethylester in 100 mL Acetonitril 11.1 g (76.6 mmol) 40% KF/Al₂O₃ gegeben und die resultierende Suspension für einige min bei RT gerührt. Anschliessend wurde eine Lösung von 4.07 mL (23.0 mmol) (2-Chlormethoxy-ethyl)-trimethyl-silan in 20 mL Acetonitril zugegeben und das Reaktionsgemisch 20 h bei RT gerührt. Man filtrierte über Celite, engte das Lösungsmittel i.vac. ein und reinigte Rückstand- chromatographisch (Kieselgel, *n*-Hexan/EtOAc 7:3).
- Ausbeute:: 5.49 g (59% der Theorie)
- R_{f} =: 0.45 (Kieselgel, *n*-Hexan/EtOAc 1:1)

### 128f 4-(2-Oxo-1,2,4,5-tetrahydro-1,3-benzdiazepin-3-yl)-piperidin-1-carbonsäure-(R)-2-[3,5-dibrom-4-(2-trimethylsilanyl-ethoxymethoxy)-phenyl]-1-methoxycarbonyl-ethylester

Analog Beispiel 31f konnte aus 4:63 g (9.56 mmol) (R)-3-[3,5-Dibrom-4-(2-trimethylsilanyl-ethoxymethoxy)-phenyl]-2-hydroxy-propionsäuremethylester und 2.35 g (9.56 mmol) 3-Piperidin-4-yl-1,3,4,5-tetrahydro-1,3-benzdiazepin-2-on das Produkt erhalten werden.
- Ausbeute:: 4.35 g (69% der Theorie)
- ESI-MS:: (M+H)⁺ = 754/756/758 (2 Br)
- Retentionszeit (HPLC):: 29.2 min (Methode F)

### 128g 4-(2-Oxo-1,2,4,5-tetrahydro-1,3-benzdiazepin-3-yl)-piperidin-1-carbonsäure-(R)-2-(3,5-dibrom-4-hydroxy-phenyl)-1-methoxycarbonyl-ethylester

Unter Stickstoffatmosphäre wurden zu einer Lösung von 4.30 g (5.69 mmol) 4-(2-Oxo-1,2,4,5-tetrahydro-1,3-benzdiazepin-3-yl)-piperidin-1-carbonsäure-(R)-2-[3,5-dibrom-4-(2-trimethylsilanyl-ethoxymethoxy)-phenyl]-1-methoxy-carbonyl-ethylester in 40 mL THF und 40 mL MeOH 5.46 mL methanolische H₂SO₄ (0.5 M) gegeben und die Reaktionslösung 6 h bei RT gerührt. Das Reaktionsgemisch wurde i.vac eingeengt und der Rückstand ohne Reinigung weiter umgesetzt.
- Ausbeute:: quantitativ
- ESI-MS:: (M+H)⁺ = 624/626/628 (2 Br)
- Retentionszeit (HPLC):: 17.3 min (Methode F)

### 128h 4-(2-Oxo-1,2,4,5-tetrahydro-1,3-benzdiazepin-3-yl)-piperidin-1-carbonsäure-(R)-1-carboxy-2-(3,5-dibrom-4-hydroxy-phenyl)-ethylester

Zu einer Lösung von 4-(2-Oxo-1,2,4,5-tetrahydro-1,3-benzdiazepin-3-yl)-piperidin-1-carbonsäure-(R)-2-(3,5-dibrom-4-hydroxy-phenyl)-1-methoxycarbonyl-ethylester (Rohprodukt aus Beispiel 128g) in 80 mL THF wurde eine Lösung von 0.51 g (21.3 mmol) LiOH gegeben und das Reaktionsgemisch 3 h bei RT gerührt. Man zog i.vac. das THF ab, wusch die wässrige Phase mit EtOAc, säuerte mit 10% HCI an und extrahierte die wässrige Phase erschöpfend mit EtOAc. Die vereinigten organischen Phasen wurden i.vac. eingeengt, in Diethylether suspendiert, filtriert, der Rückstand getrocknet und anschliessend chromatographisch (Kieselgel, DCM/MeOH/AcOH 90:10:1) gereinigt.
- Ausbeute:: 3.5 g (100% der Theorie)
- ESI-MS:: (M+H)⁺ = 610/612/614 (2 Br)
- Retentionszeit (HPLC):: 14.1 min (Methode F)

### 128i 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-[4-(4-methyl-piperazin-1-yl)-piperidin-1-yl]-2-oxo-ethylester

Unter Stickstoffatmosphäre wurde zu einer Lösung von 151 mg (0.25 mmol) 4-(2-Oxo-1,2,4,5-tetrahydro-1,3-benzdiazepin-3-yl)-piperidin-1-carbonsäure-(R)-1-carboxy-2-(3,5-dibrom-4-hydroxy-phenyl)-ethylester, 104 mg (0.27 mmol) HATU und 47 µL (0.27 mmol) Ethyldiisopropylamin in 5 mL DMF 55 mg (0.30 mmol) 1-Methyl-4-piperidin-4-yl-piperazin gegeben und das Reaktionsgemisch 3 h bei RT gerührt. Die Reaktionslösung wurde i.vac. eingeengt und der Rückstand chromatographisch (Kieselgel, DCM/MeOH/NH₃ 80:20:2) gereinigt.
- Ausbeute:: 190 mg (99% der Theorie)
- ESI-MS:: (M+H)⁺ = 775/777/779 (2 Br)
- R_{f} =: 0.3 (Kieselgel, DCM/MeOH/AcOH 80:20:2)
- Retentionszeit (HPLC):: 7.2 min (Methode F)

Analog wurden folgende Verbindungen aus jeweils 151 mg 4-(2-Oxo-1,2,4,5-tetrahydro-1,3-benzdiazepin-3-yl)-piperidin-1-carbonsäure-(R)-1-carboxy-2-(3,5-dibrom-4-hydroxy-phenyl)-ethylester und der entsprechenden Menge an Amin hergestellt:

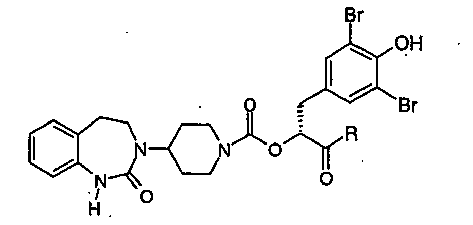

| **Beispiel** | **R** | **Ausbeute (%)** | **Massenspektrum** | **Retentionszeit HPLC (Methode)** |
|---|---|---|---|---|
| **129** | 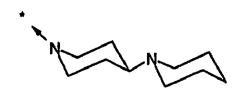 | 95 | 760/762/764 [M+H]⁺ | 9.5 min (F) |
| **130** | 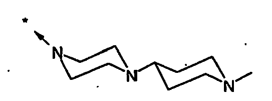 | 89 | 775/777/779 [M+H]⁺ | 6.7 min (F) |
| **131** | 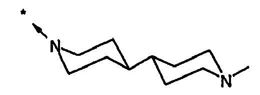 | 93 | 774/776/778 [M+H]⁺ | 9.6 min (F) |
| **132** | 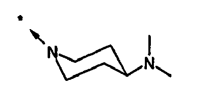 | 82 | 720/722/724 [M+H]⁺ | 8.7 min (F) |
| **133** | 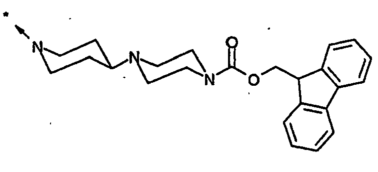 | 91 | 761/763/765 [M+H- Fmoc]⁺ | 15.0 min (F) |

### Beispiel 134

### 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-oxo-2-(4-piperidin-4-yl-piperazin-1-yl)-ethylester

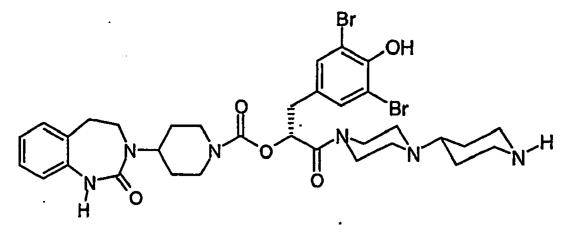

Analog Beispiel 128i wurden aus 200 mg (0.33 mmol) 4-(2-Oxo-1,2,4,5-tetrahydro-1,3-benzdiazepin-3-yl)-piperidin-1-carbonsäure-(R)-1-carboxy-2-(3,5-dibrom-4-hydroxy-phenyl)-ethylester, 140 µL (0.82 mmol) Ethyldiisopropyamin und 135 mg (0.44 mmol) 4-Piperazin-1-yl-piperidin-1-carbonsäure-*tert-*butylester (eingesetzt als Hydrochlorid Salz) das Rohprodukt erhalten. Dieses wurde in 2 mL Ameisensäure gelöst und 2 h bei RT gerührt. Das Reaktionsgemisch wurde i.vac, eingeengt und der Rückstand chromatographisch (Kieselgel, Eluation zuerst mit DCM/MeOH/NH₃ 80:20:2 dann mit DCM/MeOH/NH₃ 50:50:5) gereinigt.
- Ausbeute:: 20 mg (8% der Theorie)
- ESI-MS:: (M+H)⁺ = 761/763/765 (2 Br)
- R_{f} =: 0.35 (Kieselgel, DCM/MeOH/NH₃ 80:20:2)
- Retentionszeit (HPLC):: 6.5 min (Methode F)

### Beispiel 135

### 4-(2-Oxo-1,2,4,5-tetrahydro-1,3-benzdiazepin-3-yl)piperidin-1-carbonsäure-(R)-1-(3,5-dibrom-4-hydroxy-benzyl)-2-oxo-2-(4-piperazin-1-yl-piperidin-1-yl)-ethylester

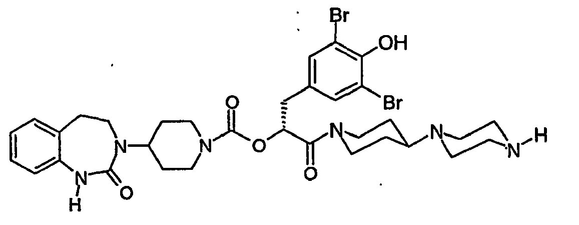

Eine Lösung von 200 mg (0.20 mmol) 4-(1-{(R)-3-(3,5-Dibrom-4-hydroxy-phenyl)-2-[4-(2-oxo-1,2,4,5-tetrahydro-1,3-benzdiazepin-3-yl)-piperidin-1-carbonyloxy]-propionyl}-piperidin-4-yl)-piperazin-1-carbonsäure-9*H*-fluoren-9-ylmethylester (Beispiel 133) in 4 mL Piperidin wurde 30 min bei RT gerührt. Das Reaktionsgemisch wurde i.vac. eingeengt und der Rückstand chromatographisch (Kieselgel, DCM/MeOH/NH₃ 80:20:2) gereinigt.
- Ausbeute:: 20 mg (8% der Theorie)
- ESI-MS:: (M+H)⁺ = 761/763/765 (2 Br)
- Retentionszeit (HPLC):: 6.8 min (Methode F)

### Beispiel 136

### 4-(2-Oxo-1,2,4,5-tetrahydro-1,3-benzdiazepin-3-yl)-carbonsäure-R-1- (3,5-dibrom-4-hydroxy-benzyl)-2-(1'-methansulfonyl-4,4'-bipiperidinyl-1-yl)-2-oxo-ethylester

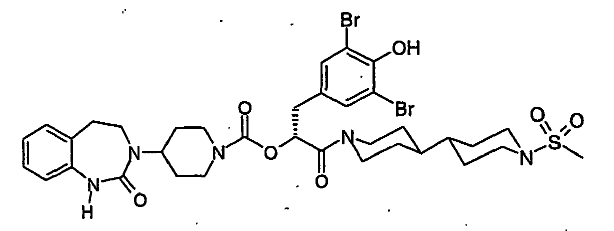

Eine Lösung von 130 mg (0.21 mmol) 4-(2-Oxo-1,2,4,5-tetrahydro-1,3-benzdiazepin-3-yl)-piperidin-1-carbonsäure-(R)-1-carboxy-2-(3,5-dibrom-4-hydroxy-phenyl)-ethylester, 80 mg (0.25 mmol) TBTU und 50 µL (0.27 mmol) Ethyldiisopropylamin in 10 mL THF wurde 50 min bei RT gerührt. Dann erfolgte die Zugabe von 60 mg (0.24 mmol) 1-Methansulfonyl-[4,4']bipiperidinyl und das Reaktionsgemisch wurde über Nacht bei RT gerührt. Die Reaktionslösung wurde mit 50 mL EtOAc verdünnt, zweimal mit je 30 mL 15% K₂CO₃-Lösung extrahiert, die organische Phase abgetrennt und über MgSO₄ getrocknet. Nach Entfernen des Trocken- und Lösungsmittels wurde der Rückstand mit Wasser verrieben und filtriert. Der Feststoff wurde mit 5 mL 1 M HCI versetzt und über Nacht gerührt. Weitere Reinigung des Rohproduktes erfolgte chromatographisch (Kieselgel, Gradient DCM zu DCM/MeOH/NH₃ 50:45:5). Die das Produkt enthaltenden Fraktionen wurden vereinigt, i.vac. eingeengt, mit DIPE verrieben, abgesaugt und bei 40°C getrocknet.
- Ausbeute:: 80 mg (45% der Theorie)
- ESI-MS:: (M+H)⁺ = 838/840/842 (2 Br)
- R_{f} =: 0.42 (Kieselgel, DCM/MeOH/Cyc/NH₃ 70:15:15:2)

### Beispiel 137

### 4-(2-Oxo-1,2,4,5-tetrahydro-1,3-benzdiazepin-3-yl)-piperidin-1-carbonsäure-(R)-2-(4-amino-4-methyl-1,4'-bipiperidinyl-1'-yl)-1-(3,5-dibrom-4-hydroxy-benzyl)-2-oxo-ethylester

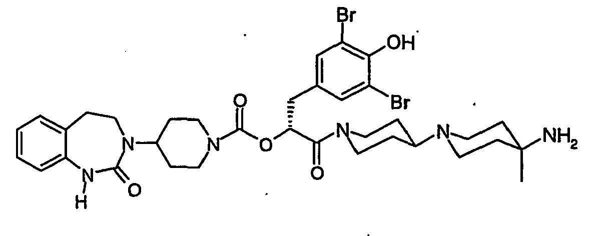

Analog Beispiel 1h konnte aus 80 mg (0.13 mmol) 4-(2-Oxo-1,2,4,5-tetrahydro-1,3-benzdiazepin-3-yl)-piperidin-1-carbonsäure-(R)-1-carboxy-2-(3,5-dibrom-4-hydroxyphenyl)-ethylester und 36 mg (0.14 mmol) 4-Methyl-[1,4']bipiperidinyl-4-ylamin (eingesetzt als Bis-Hydrochlorid Salz) das Produkt erhalten werden, welches als Formiat-Satz anfiel.
- Ausbeute:: 6 mg (6% der Theorie)
- ESI-MS:: (M+H)⁺ = 789/791/793 (2 Br)
- Retentionszeit (HPLC):: 4.9 min (Methode A)

### Beispiel 138

### 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-(1'-ethoxycarbonylmethyl-4,4'-bipiperidinyl-1-yl)-2-oxo-ethylester

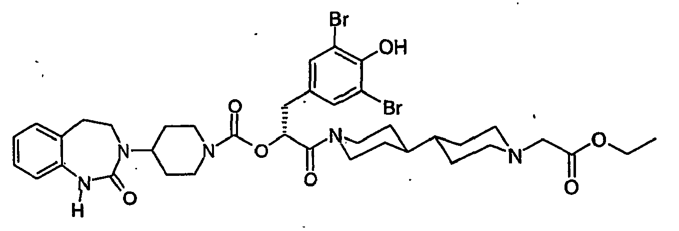

Zu einer Lösung von 500 mg (0.82 mmol) 4-(2-Oxo-1,2,4,5-tetrahydro-1,3-benzdiazepin-3-yl)-piperidin-1-carbonsäure-(R)-1-carboxy-2-(3,5-dibrom-4-hydroxyphenyl)-ethylester, 315 mg (0.98 mmol) TBTU und 150 µL (1.06 mmol) Triethylamin in 5 mL DMF wurden 208 mg (0.82 mmol) [4,4']Bipiperidinyl-1-yl-essigsäureethylester gegeben und das Reaktionsgemisch über Nacht bei RT gerührt. Zur Vervollständigung der Reaktion wurden weitere 315 mg (0.98 mmol) TBTU, 150 µL (1.06 mmol) Triethylamin und 208 mg (0.82 mmol) [4,4']Bipiperidinyl-1-yl-essigsäureethylester zugegeben und erneut über Nacht bei RT gerührt. Das Reaktionsgemisch wurde ohne weitere Aufarbeitung direkt via HPLC gereinigt. Die das Produkt enthaltenden Fraktionen wurden vereinigt, i.vac. eingeengt, mit gesättigter NaHCO₃-Lösung neutralisiert, die wässrige Phase zweimal mit je 100 mL DCM extrahiert und die vereinigten organischen Phasen über Na₂SO₄ getrocknet. Nach Entfernen des Trocken- und Lösungsmittels wurde der Rückstand mit DIPE verrieben, abgesaugt und an der Luft getrocknet.
- Ausbeute:: 204 mg (29% der Theorie)
- ESI-MS:: (M+H)⁺ = 846/848/850 (2 Br)
- Retentionszeit (HPLC):: 6.5 min (Methode A)

### Beispiel 139

### 4-(2-Oxo-1,2,4,5-tetrahydro-1,3-benzdiazepin-3-yl-piperidin-1-carbonsäure-(R)-2-(1'-carboxymethyl-4,4'-bipiperidinyl-1-yl)-1-(3,5-dibrom-4-hydroxy-benzyl)-2-oxo-ethyl-ester

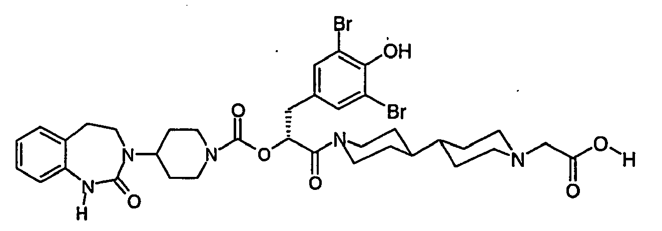

Zu einer Lösung von 30 mg (0.04 mmol) 4-(2-Oxo-1,2,4,5-tetrahydro-1,3-benzdiazepin-3-yl)-piperidin-1-carbonsäure-(R)-1-(3,5-dibrom-4-hydroxy-benzyl)-2-(1'-ethoxycarbonylmethyl-4,4'-bipiperidinyl-1-yl)-2-oxo-ethylester (Beispiel 138) in 3 mL THF wurde eine Lösung von 1.4 mg (0.06 mmol) LiOH in 1 mL Wasser gegeben und die Reaktionslösung über Nacht bei RT gerührt. Man entfernte das Lösungsmittel im Stickstoffstrom, nahm den Rückstand in 1 mL Wasser auf, säuerte mit Ameisensäure an, saugte den entstandenen Niederschlag ab und trocknete diesen im Vakuum.
- Ausbeute:: 20 mg (69% der Theorie)
- ESI-MS:: (M+H)⁺ = 818/820/822 (2 Br)
- Retentionszeit (HPLC):: 6.5 min (Methode A)

### Beispiel 140

### 4-(2-Oxo-1,2,4,5-tetrahydro-1,3-benzdiazepin-3-yl)-piperidin-1-carbonsäure-(R)-2-(4-amino-4-methyl-1,4'-bipiperidinyl-1'-yl)-1-(3,5-bis-trifluormethyl-benzyl)-2-oxo-ethylester

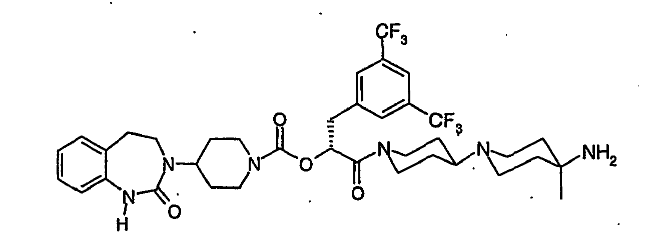

Analog Beispiel 1 h konnte aus 80 mg (0.14 mmol) 4-(2-Oxo-1,2,4,5-tetrahydro-1,3-benzdiazepin-3-yl)-piperidin-1-carbonsäure-(R)-2-(3,5-bis-trifluormethyl-phenyl)-1-carboxy-ethylester (Beispiel 46f) und 38 mg (0.14 mmol) 4-Methyl-[1,4']bipiperidinyl-4-ylamin (eingesetzt als Bis-Hydrochlorid Salz) das Produkt erhalten werden, welches als Formiat-Salz anfiel.
- Ausbeute:: 47 mg (42% der Theorie)
- ESI-MS:: (M+H)⁺ = 753
- Retentionszeit (HPLC):: 5.4 min (Methode A)

### Beispiel 141

### 4-(2-Oxo-1,2,4,5-tetrahydro-1,3-benzdiazepin-3-yl)-piperidin-1-carbonsäure-(R)-2-(1'-ethoxycarbonylmethyl-4,4'-bipiperidinyl-1-yl)-1-(4-hydroxy-3,5-dimethyl-benzyl)-2-oxo-ethylester

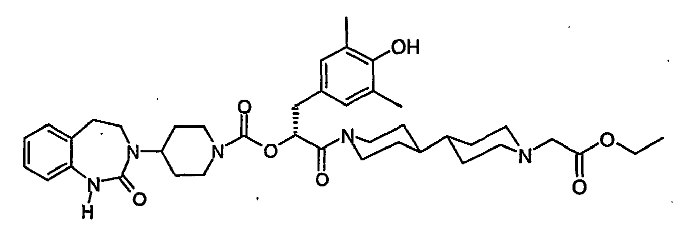

Analog Beispiel 46g konnte aus 200 mg (0.42 mmol) 4-(2-Oxo-1,2,4,5-tetrahydro-1,3-benzdiazepin-3-yl)-piperidin-1-carbonsäure-(R)-1-carboxy-2-(4-hydroxy-3,5-dimethylphenyl)-ethylester (Beispiel 69h) und 117 mg (0.46 mmol) [4,4']Bipiperidinyl-1-yl-essigsäureethylester das Produkt erhalten werden.
- Ausbeute:: 222 mg (74% der Theorie)
- ESI-MS:: (M+H)⁺ = 718
- Retentionszeit (HPLC):: 3.1 min (Methode E)

### Beispiel 142

### 4-(2-Oxo-1,2,4,5-tetrahydro-1,3-benzdiazepin-3-yl)-piperidin-1-carbonsäure-(R)-2-(1'-carboxymethyl-4 4'-bipiperidinyl-1-yl)-1-(4-hydroxy-3,5-dimethyl-benyl)-2-oxo-ethyl-ester

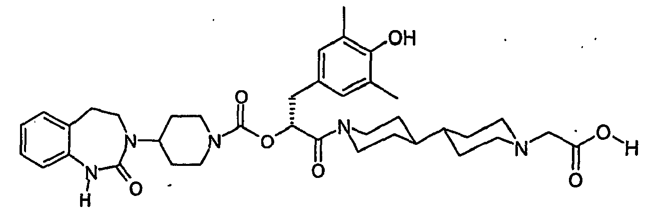

Analog Beispiel 139 konnte aus 100 mg (0.14 mmol) 4-(2-Oxo-1,2,4,5-tetrahydro-1,3-benzdiazepin-3-yl)-piperidin-1-carbonsäure-(R)-2-(1'-ethoxycarbonylmethyl-4,4'-bipiperidinyl-1-yl)-1-(4-hydroxy-3,5-dimethyl-benzyl)-2-oxo-ethylester (Beispiel 141) und 3.8 mg (0.16 mmol) LiOH das Produkt erhalten werden.
- Ausbeute:: 56 mg (58% der Theorie)
- ESI-MS:: (M-H)⁻ = 688
- Retentionszeit (HPLC):: 3.0 min (Methode E)

### Beispiel 143

### 4-(2-Oxo-1,2,4,5-tetrahydro-1,3-benzdiazepin-3-yl)-piperidin-1-carbonsäure-(R)-2-(4-cyclohexyl-piperazin-1-yl)-1-(4-hydroxy-3 5-dimethyl-benzyl)-2-oxo-ethylester

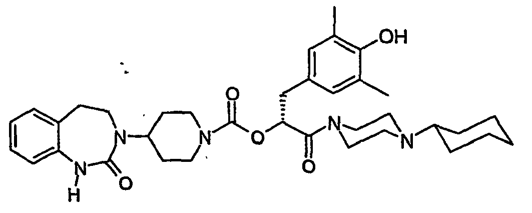

Analog Beispiel 1h konnte aus 69 mg (0.14 mmol) 4-(2-Oxo-1,2,4,5-tetrahydro-1,3-benzdiazepin-3-yl)-piperidin-1-carbonsäure-(R)-1-carboxy-2-(4-hydroxy-3,5-dimethylphenyl)-ethylester (Beispiel 69h) und 24 mg (0.14 mmol) 1-Cyclohexyl-piperazin das Produkt erhalten werden.
- Ausbeute:: 51 mg (91 % der Theorie)
- ESI-MS:: (M+H)⁺ = 632
- Retentionszeit (HPLC):: 3.1 min (Methode E)

### Beispiel 144

### 4-(2-Oxo-1,2,4,5-tetrahydro-1,3-benzdiazepin-3-yl)-piperidin-1-carbonsäure-(R)-2-[4-(4-methyl-piperazin-1-yl)-piperidin-1-yl]-2-oxo-1-(3-trifluormethyl-benzyl)-ethylester

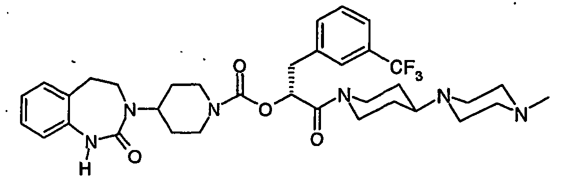

### 144a (Z,E)-2-Acetylamino-3-(3-trifluormethyl-phenyl)-acrylsäure

Analog Beispiel 1a konnte aus 15.8 g (115 mmol) 3-Trifluormethyl-benzaldehyd und 21.3 g (182 mmol) N-Acetyl-glycin das Produkt erhalten werden.
- Ausbeute:: 16.7 g (53% der Theorie)
- ESI-MS:: (M+H)⁺ = 274
- R_{f} =: 0.4 (Kieselgel, DCM/MeOH/AcOH 90:10:1)

### 144b 2-Oxo-3-(3-trifluormethyl-phenyl)-propionsäure

Analog Beispiel 46b konnte aus 16.6 g (60.8 mmol) (Z,E)-2-Acetylamino-3-(3-trifluormethyl-phenyl)-acrylsäure das Produkt erhalten werden.
- Ausbeute:: 5.7 g (40% der Theorie)
- ESI-MS:: (M-H)⁻ = 231
- R_{f} =: 0.19 (Kieselgel, DCM/MeOH/Cyc/NH₃ 70:15:15:2)

### 144c (R)-2-Hydroxy-3-(3-trifluormethyl-phenyl)-propionsäure

Analog Beispiel 1 c konnte aus 5.70 g (24.6 mmol) 2-Oxo-3-(3-trifluormethyl-phenyl)-propionsäure und 11.8 g (36.8 mmol) (1R)-B-Chlordiisopinocampheylboran das Produkt erhalten werden.
- Ausbeute:: 4.25 g (74% der Theorie)
- ESI-MS:: (M-H)⁻ = 233
- R_{f} =: 0.35 (Kieselgel, DCM/MeOH/Cyc/NH₃ 70:15:15:2)

### 144d (R)-2-Hydroxy-3-(3-trifluormethyl-phenyl)-propionsäuremethylester

Analog Beispiel 46d konnte aus 4.20 g (17.9 mmol) (R)-2-Hydroxy-3-(3-trifluormethylphenyl)-propionsäure das Produkt erhalten werden.
- Ausbeute:: 2.47 g (55% der Theorie)
- ESI-MS:: (M+H)⁺ = 249
- R_{f} =: 0.73 (Kieselgel, DCM/MeOH/Cyc/NH₃ 70:15:15:2)

### 144e 4-(2-Oxo-1,2,4,5-tetrahydro-1,3-benzdiazepin-3-yl)-piperidin-1-carbonsäure-(R)-1-methoxycarbonyl-2-(3-trifluormethyl-phenyl)-ethylester

Analog Beispiel 31f konnte aus 2.47 g (9.95 mmol) (R)-2-Hydroxy-3-(3-trifluormethylphenyl)-propionsäuremethylester und 4.10 g (65% Reinheit, 10.9 mmol) 3-Piperidin-4-yl-1,3,4,5-tetrahydro-1,3-benzdiazepin-2-on das Produkt erhalten werden.
- Ausbeute:: 3.16 g (61% der Theorie)
- ESI-MS:: (M+H)⁺ = 520
- R_{f} =: 0.93 (Kieselgel, EtOAc/MeOH/NH₃ 80:20:2)

### 144f 4-(2-Oxo-1,2,4,5-tetrahydro-1,3-benzdiazepin-3-yl)-piperidin-1-carbonsäure-(R)-1-carboxy-2-(3-trifluormethyl-phenyl)-ethylester

Analog Beispiel 46f konnte aus 3.10 g (5.97 mmol) 4-(2-Oxo-1,2,4,5-tetrahydro-1,3-benzdiazepin-3-yl)-piperidin-1-carbonsäure-(R)-1-methoxycarbonyl-2-(3-trifluormethyl-phenyl)-ethylester und 0.22 g (9.00 mmol) LiOH das Produkt erhalten werden.
- Ausbeute:: 2.80 g (93% der Theorie)
- ESI-MS:: (M+H)⁺ = 506
- R_{f} =: 0.58 (Kieselgel, EtOAc/MeOH/NH₃ 70:30:3)

### 144g 4-(2-Oxo-1,2,4,5-tetrahydro-1,3-benzdiazepin-3-yl)-piperidin-1-carbonsäure-(R)-2-[4-(4-methyl-piperazin-1-yl)-piperidin-1-yl]-2-oxo-1-(3-trifluormethyl-benzyl)-ethylester

Analog Beispiel 1 h konnte aus 80.0 mg (0.16 mmol) 4-(2-Oxo-1,2,4,5-tetrahydro-1,3-benzdiazepin-3-yl)-piperidin-1-carbonsäure-(R)-1-carboxy-2-(3-trifluormethyl-phenyl)-ethylester und 29.6 mg (0.16 mmol) 1-Methyl-4-piperidin-4-yl-piperazin das Produkt erhalten werden.
- Ausbeute:: 67 mg (63% der Theorie)
- ESI-MS:: (M+H)⁺ = 671
- R_{f} =: 0.4 (Kieselgel, DCM/MeOH/Cyc/NH₃ 70:15:15:2)

Analog wurden folgende Verbindungen aus jeweils 80 mg (Beispiele 145 bis 148) bzw. 140 mg (Beispiele 149 und 150) 4-(2-Oxo-1,2,4,5-tetrahydro-1,3-benzdiazepin-3-yl)-pi peridi n-1-carbonsäure-(R)-1-carboxy-2-(3-trifl uor-methyl-phenyl)-ethylester und der entsprechenden Menge an Amin hergestellt:

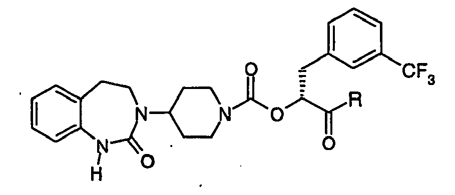

| **Beispiel** | **R** | **Ausbeute (%)** | **Massenspektrum** | **R_{f} (Kieselgel, DCM/MeOH/ Cyc/NH₃ 70:15:15:2) oder Retentionszeit HPLC (Methode)** |
|---|---|---|---|---|
| **145** | 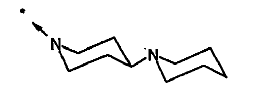 | 93 | 656 [M+H]⁺ | 0.68 |
| **146** | 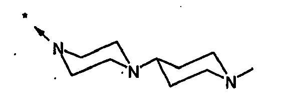 | 64 | 671 [M+H]⁺ | 0.35 |
| **147** | 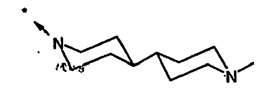 | 74 | 670 [M+H]⁺ | 0.53 |
| **148** | 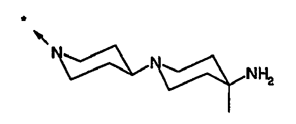 | 48 | 685 [M+H]⁺ | 5.1 min (A) |
| **149** | 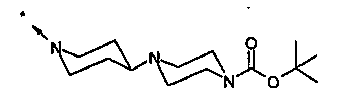 | 62 | 757 [M+H]⁺ | 0.51 |
| **150** | 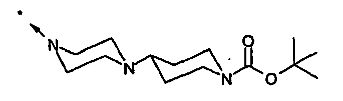 | 71 | 757 [M+H]⁺ | 0.50 |

### Beispiel 151

### 4-(2-Oxo-1,2;4,5-tetrahydro-1,3-benzdiazepin-3-yl)-piperidin-1-carbonsäure-(R)-1-(3-methyl-benzyl)-2-oxo-2-(4-piperazin-1-yl-piperidin-1-yl)-ethylester

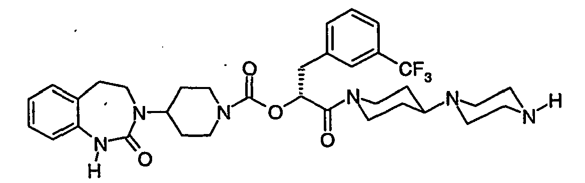

Eine Lösung von 131 mg (0.17 mmol) 4-{1-[(R)-2-[4-(2-Oxo-1,2,4,5-tetrahydro-1,3-benzdiazepin-3-yl)-piperidin-1-carbonyloxy]-3-(3-trifluormethyl-phenyl)-propionyl]-piperidin-4-yl}-piperazin-1-carbonsäure-*tert*-butylester (Beispiel 149) in 1.5 mL 4 M HCl wurde über Nacht bei RT gerührt. Die Reaktionslösung wurde ohne weitere Aufarbeitung via HPLC gereinigt. Die das Produkt enthaltenden Fraktionen wurden vereinigt und lyophilisiert.
- Ausbeute:: 75 mg (67% der Theorie)
- ESI-MS:: (M+H)⁺ = 657
- R_{f} =: 0.38 (Kieselgel, DCM/MeOH/Cyc/NH₃ 70:15:15:2)

### Beispiel 152

### 4-(2-Oxo-1,2,4,5-tetrahydro-1,3-benzdiazepin-3-yl)-piperidin-1-carbonsäure-(R)-2-oxo-2-(4-piperidin-4-yl-piperazin-1-yl)-1-(3-trifluormethyl-benzyl)-ethylester

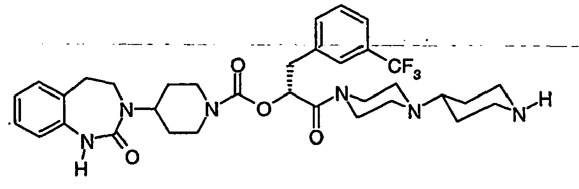

Analog Beispiel 151 wurde aus 149 mg (0.20 mmol) 4-(2-Oxo-1,2,4,5-tetrahydro-1,3-benzdiazepin-3-yl)-piperidin-1-carbonsäure-(R)-1-(3-trifluormethyl-benzyl)-2-[4-(1-*tert*-butoxy-carbonyl-piperidin-4-yl)-piperazin-1-yl]-2-oxo-ethylester (Beispiel 150) das Produkt erhalten.
- Ausbeute:: 66 mg (51% der Theorie)
- ESI-MS:: (M+H)⁺ = 657
- R_{f} =: 0.18 (Kieselgel, DCM/MeOH/Cyc/NH₃ 70:15:15:2)

### Beispiel 153

### 4-(2-Oxo-1,2,4,5-tetrahydro-1,3-benzdiazepin-3-yl)-piperidin-1-carbonsäure-(R)-2-[4-(4-methyl-piperazin-1-yl)-piperidin-1-yl]-2-oxo-1-(3-methyl-benzyl)-ethylester

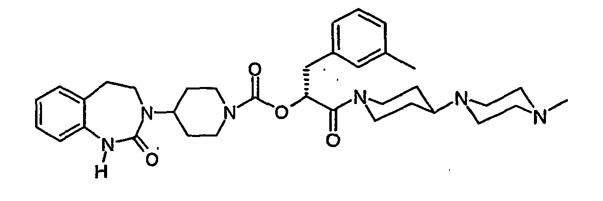

### 153a (Z,E)-2-Acetylamino-3-m-tolyl-acrylsäure

Analog Beispiel 1a konnte aus 25.0 g (212 mmol) 3-Methyl-benzaldehyd und 24.9 g (212 mmol) N-Acetyl-glycin das Produkt erhalten werden.
- Ausbeute:: 26.0 g (56% der Theorie)
- ESI-MS:: (M+H)⁺ = 220
- Retentionszeit (HPLC):: 5.4 min (Methode A)

### 153b 2-Oxo-3-m-tolyl-propionsäure

Analog Beispiel 46b konnte aus 13.0 g (59.3 mmol) (Z,E)-2-Acetylamino-3-*m-*tolyl-acrylsäure das Produkt erhalten werden.
- Ausbeute:: 9.2 g (88% der Theorie)
- ESI-MS:: (M-H)⁻ = 177
- Retentionszeit (HPLC):: 7.3 min (Methode A)

### 153c (R)-2-Hydroxy-3-m-tolyl-propionsäure

Analog Beispiel 1 c konnte aus 9.24 g (51.9 mmol) 2-Oxo-3-*m*-tolyl-propionsäure und 24.0 g (74.8 mmol) (1R)-B-Chlordiisopinocampheylboran das Produkt erhalten werden.
- Ausbeute:: 8.4 g (90% der Theorie)
- ESI-MS:: (M-H)⁻ = 179
- Retentionszeit (HPLC):: 7.2 min (Methode A)

### 153d (R)-2-Hydroxy-3-m-tolyl-propionsäuremethylester

Analog Beispiel 31 e konnte aus 8.40 g (46.6 mmol) (R)-2-Hydroxy-3-*m*-tolyl-propionsäure und 3.74 mL (51.28 mmol) SOCl₂ das Produkt erhalten werden.
- Ausbeute:: 6.28 g (69% der Theorie)
- ESI-MS:: (M+H)⁺ = 195
- Retentionszeit (HPLC):: 6.9 min (Methode A)

### 153e 4-(2-Oxo-1,2,4,5-tetrahydro-1,3-benzdiazepin-3-yl)-piperidin-1-carbonsäure-(R)-1-methoxycarbonyl-2-m-tolyl-ethylester

Analog Beispiel 31f konnte aus 1.12 g (5.76 mmol) (R)-2-Hydroxy-3-*m*-tolyl-propion-säuremethylester und 1.41 g (5.76 mmol), 3-Piperidin-4-yl-1,3,4,5-tetrahydro-1,3-benzdiazepin-2-on das Produkt erhalten werden.
- Ausbeute:: 2.07 g (77% der Theorie)
- ESI-MS:: (M+H)⁺ = 466
- Retentionszeit (HPLC):: 9.0 min (Methode A)

### 153f 4-(2-Oxo-1,2,4,5-tetrahydro-1,3-benzdiazepin-3-yl)-piperidin-1-carbonsäure-(R)-1-carboxy-2-m-tolyl-ethylester

Analog Beispiel 46f konnte aus 2.07 g (4.45 mmol) 4-(2-Oxo-1,2,4,5-tetrahydro-1,3-benzdiazepin-3-yl)-piperidin-1-carbonsäure-(R)-1-methoxycarbonyl-2-*m*-tolyl-ethylester und 0.16 g (6.72 mmol) LiOH das Produkt erhalten werden.
- Ausbeute:: 1.86 g (93% der Theorie)
- ESI-MS:: (M+H)⁺ = 452
- Retentionszeit (HPLC):: 8.0 min (Methode A)

### 153g 4-(2-Oxo-1,2,4,5-tetrahydro-1,3-benzdiazepin-3-yl)-piperidin-1-carbonsäure-(R)-2-[4-(4-methyl-piperazin-1-yl)-piperidin-1-yl]-2-oxo-1-(3-methyl-benzyl)-ethylester

Analog Beispiel 1h konnte aus 80.0 mg (0.18 mmol) 4-(2-Oxo-1,2,4,5-tetrahydro-1,3-benzdiazepin-3-yl)-piperidin-1-carbonsäure-(R)-1-carboxy-2-*m*-tolyl-ethylester und 33.1 mg (0.18 mmol) 1-Methyl-4-piperidin-4-yl-piperazin das Produkt erhalten werden.
- Ausbeute:: 46.7 mg (43% der Theorie)
- ESI-MS:: (M+H)⁺ = 617
- Retentionszeit (HPLC):: 5.5 min (Methode A)

Analog wurden folgende Verbindungen aus jeweils 80 mg 4-(2-Oxo-1,2,4,5-tetrahydro-1,3-benzdiazepin-3-yl)-piperidin-1-carbonsäure-(R)-1-carboxy-2-*m*-tolyl-ethylester und der entsprechenden Menge an Amin hergestellt:

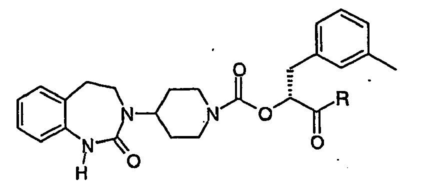

| **Beispiel** | **R** | **Ausbeute (%)** | **Massenspektrum** | **Retentionszeit HPLC (Methode)** |
|---|---|---|---|---|
| **154** | 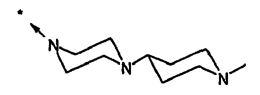 | 80 | 617 [M+H]⁺ | 5.0 min (A) |
| **155** | 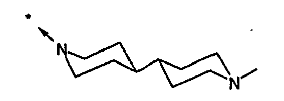 | 75 | 616 [M+H]⁺ | 6.2 min (A) |

Die nachfolgenden Beispiele beschreiben die Herstellung pharmazeutischer Anwendungsformen, die als Wirkstoff eine beliebige Verbindung der allgemeinen Formel (I) enthalten:

### Beispiel I

| Kapseln zur Pulverinhalation mit 1 mg Wirkstoff | |
|---|---|
| Zusammensetzung: | |
| 1 Kapsel zur Pulverinhalation enthält: | |
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II

| Inhalationslösung für Respimat^{®} mit 1 mg Wirkstoff | |
|---|---|
| Zusammensetzung: | |
| 1 Hub enthält: | |
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren:

Der Wirkstoff und Benzalkoniumchlorid werden in Wasser gelöst und in Respimat^{®}-Kartuschen abgefüllt.

### Beispiel III

| Inhalationslösung für Vernebler mit 1 mg Wirkstoff | |
|---|---|
| Zusammensetzung: | |
| 1 Fläschchen enthält: | |
| Wirkstoff | 0.1 g |
| Natriumchlorid | 0.18 g |
| Benzalkoniumchlorid | 0.002 g |
| Wasser gereinigt ad | 20.0 ml |

### Herstellunasverfahren:

Wirkstoff, Natriumchlorid und Benzalkoniumchlorid werden in Wasser gelöst.

### Beispiel IV

| Treibgas-Dosieraerosol mit 1 mg Wirkstoff | |
|---|---|
| Zusammensetzung: | |
| 1 Hub enthält: | |
| Wirkstoff | 1.0mg |
| Lecithin | 0.1 % |
| Treibgas ad | 50.0 µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel V

| Nasalspray mit 1 mg Wirkstoff | |
|---|---|
| Zusammensetzung: | |
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellungsverfahren:

Der Wirkstoff und die Hilfsstoffe werden in Wasser gelöst und in ein entsprechendes Behältnis abgefüllt.

### Beispiel VI

| Injektionslösung mit 5 mg Wirksubstanz pro 5 ml | |
|---|---|
| Zusammensetzung: | |
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII

| Injektionslösung mit 100 mg Wirksubstanz pro 20 ml | |
|---|---|
| Zusammensetzung: | |
| Wirksubstanz | 100 mg |
| Monokaliumdihydrogenphosphat = KH₂PO₄ | 12 mg |
| Dinatriumhydrogenphosphat = Na₂HPO₄·2H₂O | 2 mg |
| Natriumchlorid | 180 mg |
| Human-Serum-Albumin | 50 mg |
| Polysorbat 80 | 20 mg |
| Wasser für Injektionszwecke ad | 10 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel VIII

| Lyophilisat mit 10 mg Wirksubstanz | |
|---|---|
| Zusammensetzung: | |
| Wirksubstanz | 10 mg |
| Mannit | 300 mg |
| Human-Serum-Albumin | 20 mg |
| Wasser für Injektionszwecke ad | 2 ml |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

| Lösungsmittel für Lyophilisat: | |
|---|---|
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (Wfl) auflösen; in Ampullen abfüllen.

### Beispiel IX

| Tabletten mit 20 mg Wirksubstanz | |
|---|---|
| Zusammensetzung: | |
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg.

### Beispiel X

| Kapseln mit 20 mg Wirksubstanz | |
|---|---|
| Zusammensetzung: | |
| Wirksubstanz | 20 mg |
| Maisstärke | 80 mg |
| Kieselsäure, hochdispers | 5 mg |
| Magnesiumstearat | 2.5 mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Größe 3 abfüllen.

### Beispiel XI

| Zäpfchen mit 50 mg Wirksubstanz | |
|---|---|
| Zusammensetzung: | |
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

### Beispiel XII

| Injektionslösung mit 10 mg Wirksubstanz pro 1 mL | |
|---|---|
| Zusammensetzung: | |
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. CGRP-Antagonisten der allgemeinen Formel 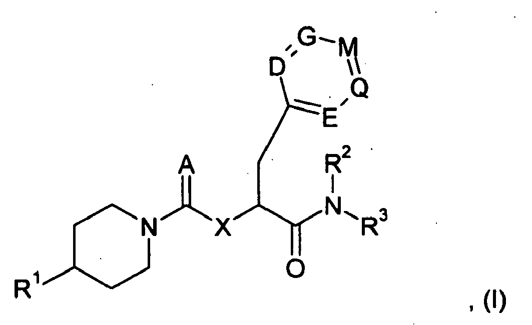 in der
A ein Sauerstoff- oder Schwefelatom,
X ein Sauerstoff- oder Schwefelatom,
(a) D, E unabhängig voneinander jeweils eine Methingruppe oder das Stickstoffatom und
G eine durch die Gruppe R^{a} substituierte Methingruppe,
M eine durch die Gruppe R^{b} substituierte Methingruppe,
Q eine durch die Gruppe R^{c} substituierte Methingruppe,
wobei eine oder zwei der Gruppen G, M und Q jeweils auch ein Stickstoffatom bedeuten können,
oder
(b) D und E jeweils eine Methingruppe, wobei eine der Gruppen D und E auch ein Stickstoffatom bedeuten kann, und
G, M und Q jeweils ein Stickstoffatom,
wobei R^{a}, R^{b} und R^{c} unabhängig voneinander jeweils ein Wasserstoff oder Halogenatom, eine C₁-₆-Alkyl-, C₂₋₆-Alkenyl-, C₂₋₆-Alkinyl-, Cyclo-C₃₋₇-alkyl-, Cyclo-C₃₋₇-alkenyl-, Cyano-, Hydroxy-, Hydroxy-C₁₋₆-alkyl-, Hydroxy-C₃₋₆-alkenyl-, Hydroxy-C₃₋₆-alkinyl-, C₁-₆-Alkoxy-, C₁₋₆-Alkoxy-C₁₋₆-alkyl-, C₁₋₆-Alkoxy-C₃₋₆-alkenyl-, C₁₋₆-Alkoxy-C₃₋₆-alkinyl-, C₃₋₆-Alkenoxy-C₁₋₆-alkyl-, C₃₋₆-Alkenoxy-C₃₋₆-alkenyl-, C₃₋₆-Alkenoxy-C₃₋₆-alkinyl-, C₃₋₆-Alkinoxy-C₁₋₆-alkyl-, C₃₋₆-Alkinoxy-C₃₋₆-alkenyl-, C₃₋₆-A)kinoxy-C₃₋₆-alkinyl)-, Thiohydroxy-, C₁₋₆-Alkylthio-, C₃₋₆-Alkenylthio-, C₃₋₆-Alkinylthio-, Amino-, C₁₋₆-Alkyl-amino-, C₃₋₆-Alkenyl-amino-, C₃₋₆-Alkinyl-amino-, Di-(C₁₋₆-alkyl)-amino-, Di-(C₃₋₆-alkenyl)-amino-, Di-(C₃₋₆-alkinyl)-amino-, Amino-C₁₋₆-alkyl-, C₁₋₃-Alkyl-amino-C₁₋₆-alkyl-, Di(C₁₋₃-Alkyl)-amino-C₁₋₆-alkyl-, Amino-C₃₋₆-alkenyl-, C₁₋₃-Alkyl-amino-C₃₋₆-alkenyl-, Di-(C₁₋₃-alkyl)-amino-C₃₋₆-alkenyl-, Amino-C₃₋₆-alkinyl-, C₁₋₃-Alkyl-amino-C₃₋₆-alkinyl-, Di-(C₁₋₃-alkyl)-amino-C₃₋₆-alkinyl-, Hydroxycarbonyl-, Phenylcarbonyl-, Pyridylcarbonyl-, C₁₋₆-Alkyl-carbonyl-, C₂₋₆-Alkenyl-carbonyl-, C₂₋₆-Akinyl-carbonyl-, Formyl-, C₁₋₆-Alkoxy-carbonyl-, C₃₋₆-Alkenoxy-carbonyl-, C₃₋₆-Alkinoxy-carbonyl-, Aminocarbonyl-, C₁₋₆-Alkyl-aminocarbonyl-, C₃₋₆-Alkenyl-aminocarbonyl-, C₃₋₆-Alkinyl-aminocarbonyl-, Di-(C₁₋₆-alkyl)-aminocarbonyl-, Di-(C₃₋₆-alkenyl)-aminocarbonyl-, Di-(C₃₋₆-alkinyl)-aminocarbonyl-, Formylamino-, C₁₋₆-Alkyl-carbonylamino-, C₂₋₆-Alkenyl-carbonylamino-, C₂₋₆-Alkinyl-carbonylamino-, Formyl-C₁₋₆-alkyl-amino-, Formyl-C₃₋₆-alkenyl-amino-, Formyl-C₃₋₆-alkinyl-amino-, C₁₋₆-Alkyl-carbonyl-C₁₋₆-alkyl-amino-, C₂₋₆-Alkenyl-carbonyl-C₁₋₆-alkyl-amino-, C₂₋₆-Alkinyl-carbonyl-C₁₋₆-alkyl-amino-, C₁₋₆-Alkyl-carbonyl-C₃₋₆-alkenyl-amino-, C₂₋₆-Alkenyl-carbonyl-C₃₋₆-alkenyl-amino-, C₂₋₆-Alkinyl-carbonyl-C₃₋₆-alkenyl-amino-, C₁₋₆-Alkyl-carbonyl-C₃₋₆-alkinyl-amino-, C₂₋₆-Alkenyl-carbonyl-C₃₋₆-alkinyl-amino-, C₂₋₆-Alkinyl-carbonyl-C₃₋₆-alkinyl-amino-, C₁₋₆-Alkyl-sulfonyl-, C₂₋₆-Alkenyl-s ulfonyl-, C₂₋₆-Alkinyl-sulfonyl-, C₁₋₆-Alkyl-sulfinyl-, C₂₋₆-Alkenyl-sulfinyl-, C₂₋₆-Alkinyl-sulfinyl-, C₁₋₆-Alkyl-sulfonylamino-, C₂₋₆-Alkenyl-sulfonylamino-, C₂₋₆-Alkinyl-sulfonylamino-, C₁₋₆-Alkyl-sulfonyl-C₁₋₆-alkylamino-, C₁₋₆-Alkyl-sulfonyl-C₃₋₆-alkenylamino-, C₁₋₆-Alkyl-sulfonyl-C₃₋₆-alkinylamino-, C₂₋₆-Alkenyl-sulfonyl-C₁₋₆-alkylamino-, C₂₋₆-Alkenyl-sulfonyl-C₃₋₆-alkenylamino-, C₂₋₆-Alkenyl-sulfonyl-C₃₋₆-alkinylamino-, C₂₋₆-Alkinyl-sulfonyl-C₁₋₆-alkylamino-, C₂₋₆-Alkinyl-sulfonyl-C₃₋₆-alkenylamino-, C₂₋₆-Alkinyl-sulfonyl-C₃₋₆-alkinylamino-, Aminosulfonyl-, C₁₋₆-Alkylaminosulfonyl-, Di-(C₁₋₆-alkyl)-aminosulfonyl-, C₃₋₆-Alkenyl-aminosulfonyl-, Di-(C₃₋₆-alkenyl)-aminosulfonyl-, C₃₋₆-Alkinylaminosulfonyl- oder Di-(C₃₋₆alkinyl)-aminosulfonylgruppe bedeuten
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₆-Alkylgruppe darstellen,
(ii) R^{c} kein Wasserstoffatom bedeutet, wenn R^{a} sowie R^{b} jeweils eine C₁₋₆-Alkylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine C₁₋₆-Alkyl-, C₂₋₆-Alkenyl- oder C₂₋₆-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₆-Alkyl-, C₂₋₆-Alkenyl- oder C₂₋₆-Alkinylgruppe und R^{b} ein Chlor- oder Bromatom, eine Amino-, Methylamino- oder Hydroxygruppe, darstellen,
R¹ eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-c]chinolin-2-on-3-yl-, 1,3-Dihydro-naphth[1,2-d]imidazol-2-on-3-y)-, 1,3-Dihydro-benzimidazol-2-on-3-yl-, 4-Phenyl-1,3-dihydro-imidazol-2-on-1-yl-, 3,4-Dihydro-1*H*-thieno-[3.2-d]pyrimidin-2-on-3-y)- oder 3,4-Dihydro-1*H*-thieno[3,4-d]pyrimidin-2-on-3-y)gruppe,
wobei die vorstehend unter R¹ erwähnten Heterocyclen im Kohlenstoffgerüst zusätzlich durch eine Methoxygruppe monosubstituiert sein können,
wobei alle in den vorstehend unter R¹ definierten Resten erwähnten oder enthaltenen aromatischen und heteroaromatischen Reste zusätzlich durch Halogenatome, durch Cyano- oder Hydroxygruppen mono-, di- oder trisubstituiert und die Substituenten gleich oder verschieden sein können, und
wobei die Doppel-,und Dreifachbindungen der in den für R^{a}, R^{b}, R^{c} und R¹ vorstehend definierten Gruppen enthaltenen C₃₋₆-Alkenyl- oder C₃₋₆-Alkinylgruppen von in diesen Gruppen gegebenenfalls ebenfalls enthaltenen Heteroatomen isoliert sind,
R² das Wasserstoffatom,
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Diazinyl-, Hydroxy-, Amino-, C₁₋₆-Alkylamino-, Di-(C₁₋₆-alkyl)-amino-, C₃₋₆-Alkenylamino-, Di-(C₃₋₆-alkenyl)amino-, C₃₋₆-Alkinylamino-, Di-(C₃₋₆-alkinyl)amino-, Hydroxycarbonyl-, C₁₋₆-Alkoxycarbonyl-, Aminocarbonyl-, Aminocarbonylamino-, C₁₋₆-Alkylcarbonylamino-, C₂₋₆-Alkenyl-carbonylamino-, C₂₋₆-Alkinylcarbonylamino-, 4-Morpholinyl-, [Bis-(2-hydroxyethyl)]amino-, 4-(C₁₋₆-Alkyl)-1-piperazinyl- oder 4-(w-Hydroxy-C₂₋₇-alkyl)₋1-piperazinylgruppe substituiert sein kann,
eine Phenyl- oder Pyridinylgruppe,
wobei die in den für R² vorstehend definierten Gruppen genannten oder als Substituenten enthaltenen Phenyl-, Pyridinyl- und Diazinylgruppen zusätzlich im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkyl-amino-C₁₋₃-alkyl-, Di-(C₁₋₃-alkyl)-amino-C₁₋₃-alkyl-, C₁₋₃-Alkylcarbonylamino-, C₁₋₃-Alkylcarbonylamino-C₁₋₃-alkyl-, Aminocarbonyl-, C₁₋₃-Alkyl-aminocarbonyl-, Di-(C₁₋₃-alkyl)-aminocarbonyl-, Cyano-, Aminosulfonyl-, C₁₋₃-Alkyl-aminosulfonyl-, Di-(C₁₋₃-alkyl)-aminosulfonyl-, C₁₋₃-Alkyl-thio-, C₁₋₃-Alkyl-sulfinyl-, oder C₁₋₃-Alkyl-sulfonyl- mono-, di- oder trisubstituiert und die Substituenten gleich oder verschieden sein können,
R³ das Wasserstoffatom oder eine durch eine Phenyl- oder Pyridinylgruppe substituierte C₁₋₃-Alkylgruppe,
wobei die C₁₋₃-Alkylgruppe mit einer in R² enthaltenen Alkylgruppe oder einem in R² enthaltenen Phenyl- oder Pyridylring unter Einschluss des Stickstoffatoms, an welches R² und R³ gebunden sind, unter Ausbildung eines 4- 7-gliedrigen Ringes verbunden sein kann,
oder
R² und R³ zusammen mit dem eingeschlossenen Stickstoffatom einen Rest der allgemeinen Formel 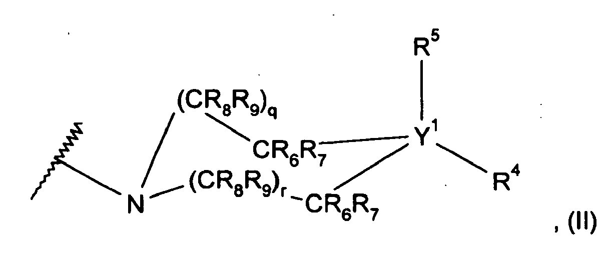 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, oder
q und r, wenn Y¹ das Stickstoffatom darstellt, die Zahlen 1 oder 2,
R⁴ das Wasserstoffatom, eine Amino-, C₁₋₄-Alkylamino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl- oder Cycto-C₃₋₇-atkenytgruppe, eine Amino-C₂₋₇-alkyl-, C₁₋₄-Alkyl-amino-C₂₋₇-alkyl-, Di-(C₁₋₄-alkyl-amino)-C₂₋₇-alkyl-, Aminoiminomethyl-, Aminocarbonylamino-, C₁₋₄-Alkyl-aminocarbonylamino-, Di-(C₁₋₄-alkyl)-aminocarbonylamino-, C₁₋₄-Alkyl-aminocarbonyl-C₁₋₄-alkyl-amino-, Di-(C₁₋₄-alkyl)-aminocarbonyl-C₁₋₄-alkyl-amino-, Phenylaminocarbonylamino-, Aminocarbonyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, C₃₋₆-Alkenoxycarbonyl-, C₃₋₆-Alkinoxycarbonyl-, C₁₋₆-Alkoxycarbonyl-C₁₋₃₋alkyl-, C₁₋₆-Alkenoxycarbonyl-C₁₋₃-alkyl-, C₁₋₆-Alkinoxycarbonyl-C₁₋₃-alkyl- oder Hydroxycarbonyl-C₁₋₃-alkylgruppe,
eine Phenyl-, Pyridinyl-, Diazinyl-, 1-Naphthyl-, 2-Naphthyl-, Pyridinylcarbonyl- oder Phenylcarbonylgruppe, die jeweils im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkyl-amino-C₁₋₃-alkyl-, Di-(C₁₋₄-alkyl)-amino-C₁₋₃-alkyl-, C₁₋₄-Alkylcarbonylamino-, C₁₋₄-Alkylcarbonylamino-C₁₋₃-alkyl-, Aminocarbonyl-, C₁₋₄-Alkyl-aminocarbonyl-, Di-(C₁₋₄-alkyl)-aminocarbonyl-, Cyano-, Aminosulfonyl-, C₁₋₄-Alkyl-aminosulfonyl-, . Di-(C₁₋₄-alkyl)-aminosulfonyl-, C₁₋₄-Alkyl-thio-, C₁₋₄-Alkyl-sulfinyl-, oder C₁₋₄-Alkylsulfonyl- mono-, di- oder trisubstituiert und die Substituenten gleich oder verschieden sein können,
ein Heterocyclus ausgewählt aus einer 4- bis 10-gliedrigen Azacycloalkylgruppe, einer 6- bis 10-gliedrige Oxaza-, Thiaza-, S,S-Dioxothiaza- und Diazacycloalkylgruppe sowie einer 6- bis 10-gliedrigen Azabicycloalkylgruppe,
eine 1-Alkyl-4-piperidinylcarbonyl- oder 4-Alkyl-1-piperazinylcarbonylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methingruppe durch ein Fluoratom sowie eine nicht direkt mit einem Stickstoff-, Sauerstoff- oder Schwefelatom verknüpfte Methylengruppe durch ein oder zwei Fluoratome substituiert sein kann,
die vorstehend genannten mono- und bicyclischen Heterocyclen sowie die 1-(C₁₋₆-Alkyl)-4-piperidinylcarbonyl- und 4-(C₁₋₆-Alkyl)-1-piperazinylcarbonylgruppe im Ring ein- bis vierfach durch Hydroxy-, C₁₋₆-Alkyl- oder Hydroxy-C₁₋₃-alkylgruppen, oder, gegebenenfalls zusätzlich, einfach durch eine Cyclo-C₃₋₇-alkyl-, Hydroxy-C₃₋₇-cycloalkyl-, Cyclo-C₃₋₇-alkenyl-, Cyclo-C₃₋₇-alkyl-C₁₋₃-alkyl-, Phenyl-C₁₋₃-alkyl-, Pyridyl-C₁₋₃-alkyl-, C₁₋₆-Alkylcarbonyl-, C₁₋₆-Alkylcarbonyl-C₁₋₃-alkyl-, Hydroxy-, C₁₋₆-Alkoxy-, Amino-, C₁₋₄-Alkylamino-, Di-(C₁₋₄-alkyl)amino-, Phenylcarbonyl-, Pyridinylcarbonyl-, C₁₋₆-Alkoxycarbonyl-, Hydroxycarbonyl-carbonyl-, C₁₋₆-Alkoxycarbonyl-ca rbonyl-, Hydroxycarbonyl-C₁₋₃-alkyl-, C₁₋₆-Alkoxycarbonyl-C₁₋₃-alkyl, Hydroxycarbonyl-C₁₋₃-alkylcarbonyl-, C₁₋₆-Alkoxycarbonyl-C₁₋₃-alkylcarbonyl-, Aminocarbonyl-, C₁₋₄-Alkylaminocarbonyl-, Di-(C₁₋₄-alkyl)aminocarbonyl-, Aminosulfonyl-, C₁₋₄-Alkylaminosulfonyl-, Di-(C₁₋₄-alkyl)aminosulfonyl-, C₁₋₃-Alkylsulfonyl-, Cyclo-C₃₋₇alkylsulfonyl-, Aminocarbonyl-C₁₋₃-alkyl-, C₁₋₄-Alkylaminocarbonyl-C₁₋₃-alkyl-, Di-(C₁₋₄-alkyl)aminocarbonyl-C₁₋₃-alkyl-, Hydroxyaminocarbonyl-C₁₋₃-alkyl-, C₁₋₃-Alkoxyaminocarbonyl-C₁-₃-alkyl- oder Hydroxy-(C₁₋₃-alkyl)-aminocarbonyl-C₁₋₃-alkylgruppe, durch eine im Ring gegebenenfalls C₁₋₃-alkylsubstituierte Cyclo-C₃₋₇-alkylca rbonyl-, Azacydo-C₄₋₇-alkylcarbonyl-, Diazacyclo-C₅₋₇-alkylcarbonyl- oder Oxazacyclo-C₅₋₇-alkylcarbonylgruppe substituiert sein können, wobei die Substituenten gleich oder verschieden und an ein Ring-Kohlenstoff- oder Ring-Stickstoffatom gebunden sein können,
wobei die in den für R⁴ vorstehend definierten Resten enthaltenen Phenyl- und Pyridinyl-Reste ihrerseits durch Halogenatome, durch d₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkylamino-C₁₋₃-alkyl-, Di-(C₁₋₄-alkyl)-amino-C₁₋₃-alkyl-, C₁₋₄-Alkylcarbonylamino-, C₁₋₄-Alkylcarbonylamino-C₁₋₃-alkyl-, Aminocarbonyl-, C₁₋₃-Alkyl-aminocarbonyl-, Di-C₁₋₄-alkyl-aminocarbonyl-, Cyano-, Aminosulfonyl-, C₁₋₄-Alkyl-aminosulfonyl-, Di-(C₁₋₄-alkyl)-aminosulfonyl-, C₁₋₃-Alkylthio-, C₁₋₃-Alkyl-sulfinyl-, oder C₁₋₃-Alkyl-sulfonyl- mono-, di- oder trisubstituiert sein können, wobei die Substituenten gleich oder verschieden sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom oder eine Hydroxygruppe,
einen C₁₋₄-Alkylrest, wobei ein unverzweigter Alkylrest in ω-Stellung durch eine Phenyl-, Pyridinyl-, Diazinyl-, Amino-, C₁₋₄-Alkylamino-, Di-(C₁₋₄-alkyl)-amino-, 4 C₁₋₄-Alkyl-1-piperazinyl- oder 4-Morpholinylgruppe substituiert sein kann,
eine C₁₋₆-Alkoxycarbonyl-, Cyano- oder Aminocarbonylgruppe oder auch, wenn Y¹ ein Stickstoffatom darstellt, ein freies Elektronenpaar,
oder, wenn Y¹ das Kohlenstoffatom darstellt, auch das Fluoratom, oder
R⁴ zusammen mit R⁵ und Y¹ einen 4- bis 7-gliedrigen cycloaliphatischen Ring, in dem eine Methylengruppe durch eine Gruppe -NH-, -N(C₁₋₄-Alkyl)-, -N(C₃₋₄-Alkenyl)-, -N(C₃₋₄-Alkinyl)-. -N(Cyclo-C₃₋₇-alkyl)-, -N(C₃₋₇-Cycloalkyl-C₁₋₃-alkyl)-, -N(Hydroxycarbonyl-C₁₋₃-alkyl)- oder -N(C₁₋₆-Alkoxycarbonyl-C₁₋₃-alkyl)- ersetzt sein kann,
wobei ein an ein Stickstoffatom in einer der für R⁴ vorstehend definierten Gruppen gebundenes Wasserstoffatom durch einen Schutzrest ersetzt sein kann,
R⁶ und R⁷, die gleich oder verschieden sein können, jeweils ein Wasserstoffatom, eine C₁₋₄-Alkylgruppe oder auch, wenn Y¹ ein Kohlenstoffatom darstellt, das Fluoratom, eine Amino-, C₁₋₄-Alkylamino- oder Di-(C₁₋₄-alkyl)-aminogruppe, wobei die beiden C₁₋₄-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen, bedeuten,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können und
unter den in den vor- und nachstehenden Definitionen genannten Schutzresten die aus der Peptidchemie geläufigen Schutzgruppen zur verstehen sind, insbesondere
eine im Phenylkern gegebenenfalls durch ein Halogenatom, durch eine Nitro- oder Phenylgruppe, durch eine oder zwei Methoxygruppen substituierte Phenylalkoxycarbonylgruppe mit 1 bis 3 Kohlenstoffatomen im Alkoxyteil,
beispielsweise die Benzyloxycarbonyl-, 2-Nitrobenzyloxycarbonyl-, 4-Nitro-benzyloxycarbonyl-, 4-Methoxy-benzyloxycarbonyl-, 2-Chlor-benzyloxycarbonyl-, 3-Chlor-benzyloxycarbonyl-, 4-Chlor-benzyloxycarbonyl-, 4-Biphenylyl-α,α-dimethyl-benzyloxycarbonyl- oder 3,5-Dimethoxy-α,α-dimethyl-benzyloxycarbonylgruppe,
eine Alkoxycarbonylgruppe mit insgesamt 1 bis 5 Kohlenstoffatomen im Alkylteil,
beispielsweise die Methoxycarbonyl-, Ethoxycarbonyl-, n-Propoxycarbonyl-, Isopropoxycarbonyl-, n-Butoxycarbonyl-, 1-Methylpropoxycarbonyl-, 2-Methylpropoxy-carbonyl- oder fert.Butyloxycarbonylgruppe,
die Allyloxycarbonyl-, 2,2,2-Trichlor-(1,1-dimethylethoxy)carbonyl- oder 9-Fluorenylmethoxycarbonylgruppe oder
die Formyl-, Acetyl- oder Trifluoracetylgruppe,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

2. CGRP-Antagonisten der allgemeinen Formel (I) gemäß Anspruch 1, in denen
A, X, D, E, G, M, Q und R¹ wie in Anspruch 1 erwähnt definiert sind und
R² das Wasserstoffatom,
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Diazinyl-, Hydroxy-, Amino-, C₁₋₆-Alkylamino-, Di-(C₁₋₆-alkyl)-amino-, C₃₋₆-Alkenylamino-, Di-(C₃₋₆-alkenyl)amino-, C₃₋₆-Alkinylamino-, Di-(C₃₋₆-alkinyl)amino-, Hydroxycarbonyl-, C₁₋₆-Alkoxycarbonyl-, Aminocarbonyl-, Aminocarbonylamino-, C₁₋₆-Alkylcarbonylamino-, C₂₋₆-Alkenyl-carbonylamino-, C₂₋₆-Alkinylcarbonylamino-, 4-Morpholinyl-, [Bis-(2-hydroxyethyl)]amino-, 4-(C₁₋₆-Alkyl)-1-piperazinyl- oder 4-(ω-Hydroxy-C₂₋₇-alkyl)-1-piperazinylgruppe substituiert sein kann,
eine Phenyl- oder Pyridinylgruppe,
wobei die in den für R² vorstehend definierten Gruppen genannten oder als Substituenten enthaltenen Phenyl-, Pyridinyl- und Diazinylgruppen zusätzlich im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkyl-amino-C₁₋₃-alkyl-, Di-(C₁₋₃-alkyl)-amino-C₁₋₃-alkyl-, C₁₋₃-, Alkylcarbonylamino-, C₁₋₃-Alkylcarbonylamino-C₁₋₃-alkyl-, Aminocarbonyl-, C₁₋₃-Alkyl-aminocarbonyl-, Di-(C₁₋₃-alkyl)-aminocarbonyl-, Cyano-, Aminosulfonyl-, C₁₋₃-Alkyl-aminosulfonyl-, Di-(C₁₋₃-alkyl)-aminosulfonyl-, C₁₋₃-Alkyl-thio-, C₁₋₃-Alkyl-sulfinyl-, oder C₁₋₃-Alkyl-sulfonyl- mono-, di- oder trisubstituiert und die Substituenten gleich oder verschieden sein können,
R³ das Wasserstoffatom oder eine durch eine Phenyl- oder Pyridinylgruppe substituierte C₁₋₃-Alkylgruppe,
wobei die C₁₋₃-Alkylgruppe mit einer in R² enthaltenen Alkylgruppe oder einem in R² enthaltenen Phenyl- oder Pyridylring unter Einschluss des Stickstoffatoms, an welches R² und R³ gebunden sind, unter Ausbildung eines 4- 7-gliedrigen Ringes verbunden sein kann,
oder
R² und R³ zusammen mit dem eingeschlossenen Stickstoffatom einen Rest der allgemeinen Formel 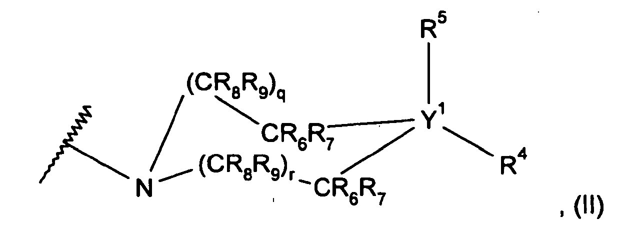 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, eine Cyclo-C₃₋₇-alkyl-, Cyclo-C₃₋₇-alkenyl-, Amino-C₂-₇-alkyl-, C₁₋₄-Alkyl-amino-C₂₋₇-alkyl-, Di-(C₁₋₄-alkyl-amino)-C₂₋₇-alkyl-, Aminoiminomethyl-, Aminocarboriylamino-, C₁₋₄-Alkyl-aminocarbonylamino-, Di-(C₁₋₄-alkyl)-aminocarbonylamino-, C₁₋₄-Alkyl-aminocarbonyl-C₁₋₄-alkyl-amino-, Di-(C₁₋₄-alkyl)-aminocarbonyl-C₁₋₄-alkyl-amino-, Phenylaminocarbonylamino-, Aminocarbonyl-, C₁₋₄-Alkyl-aminocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl- Di-(C₁₋₄-alkyl)-aminocarbonyl-C₁₋₃-alkyl-, Aminocarbonylamino-C₁₋₃-alkyl-, C₁₋₆-Alkoxycarbonyl-, C₃₋₆-Alkenoxycarbonyl-, C₃₋₆-Alkinoxycarbonyl-, C₁₋₆-Alkoxycarbonyl-C₁₋₃-alkyl-, C₁₋₆-Alkenoxycarbonyl-C₁₋₃-alkyl-, C₁₋₆-Alkinoxycarbonyl-C₁₋₃-alkyl- oder Hydroxycarbonyl-C₁₋₃-alkylgruppe,
eine Phenyl-, Pyridinyl-, Diazinyl-, 1-Naphthyl-, 2-Naphthyl-, Pyridinylcarbonyl- oder Phenylcarbonylgruppe, die jeweils im Kohlenstoffgerüst durch Halogen, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkyl-amino-C₁₋₃-alkyl-, Di-(C₁₋₄-alkyl)-amino-C₁₋₃-alkyl-, C₁₋₄-Alkylcarbonylamino-, C₁₋₄-Alkylcarbonylamino-C₁₋₃-alkyl-, Aminocarbonyl-, C₁₋₄-Alkyl-aminocarbonyl-, Di-(C₁₋₄-alkyl)-aminocarbonyl-, Cyano-, Aminosulfonyl-, C₁₋₄-Alkyl-aminosulfonyl-, Di-(C₁₋₄-alkyl)-aminosulfonyl-, C₁₋₄-Alkyl-thio-, C₁₋₄-Alkyl-sulfinyl-, oder C₁₋₄-Alkyl-sulfonyl- mono-, di- oder trisubstituiert und die Substituenten gleich oder verschieden sein können,
ein Heterocyclus ausgewählt aus einer 4- bis 10-gliedrigen Azacycloalkylgruppe, einer 6- bis 10-gliedrige Oxaza-, Thiaza- und Diazacycloalkylgruppe sowie einer 6- bis 10-gliedrigen Azabicycloalkylgruppe,
eine 1-Alkyl-4-piperidinylcarbonyl- oder 4-Alkyl-1-piperazinylcarbonylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methingruppe durch ein Fluoratom sowie eine nicht direkt mit einem Stickstoff-, Sauerstoff- oder Schwefelatom verknüpfte Methylengruppe durch ein oder zwei Fluoratome substituiert sein kann,
die vorstehend genannten mono- und bicyclischen Heterocyclen sowie die 1-(C₁₋₆-Alkyl)-4-piperidinylcarbonyl- und 4-(C₁₋₆-Alkyl)-1-piperazinylcarbonylgruppe im Ring ein- bis vierfach durch C₁₋₆-Alkylgruppen, oder, gegebenenfalls zusätzlich, einfach durch eine Cyclo-C₃₋₇-alkyl-, Cyclo-C₃₋₇-alkenyl-, Cyclo-C₃₋₇-alkyl-C₁₋₃-alkyl-, Phenyl-C₁₋₃-alkyl-, Pyridyl-C₁₋₃-alkyl-, C₁₋₆-Alkylcarbonyl-, Hydroxy-, C₁₋₆-Alkoxy-, Amino-, C₁-₄-Alkylamino-, Di-(C₁₋₄-alkyl)amino-, Phenylcarbonyl-, Pyridinylcarbonyl-, Hydroxycarbonyl-, Hydroxycarbonyl-C₁₋₃-alkyl-, C₁₋₆-Alkoxycarbonyl-, C₁₋₆-Alkoxycarbonyl-C₁₋₃-alkyl, Aminocarbonyl-, C₁₋₄-Alkylaminocarbonyl-, Di-(C₁₋₄-alkyl)aminocarbonyl-, C₁₋₃-Alkylsulfonylgruppe, durch eine im Ring gegebenenfalls C₁₋₃-alkylsubstituierte Cyclo-C₃₋₇-alkylcarbonyl-, Azacyclo-C₄₋₇-alkylcarbonyl-, Diazacyclo-C₅₋₇alkylcarbonyl- oder Oxazacyclo-C₅₋₇-alkylcarbonylgruppe substituiert sein können, wobei die Substituenten gleich oder verschieden und an ein Ring-Kohlenstoff- oder Ring-Stickstoffatom gebunden sein können,
wobei die in den für R⁴ vorstehend definierten Resten enthaltenen Phenyl- und Pyridinyl-Reste ihrerseits durch Halogenatome, durch C₁₋₃-Alkyl-, C₂₋₃-Alkenyl-, C₂₋₃-Alkinyl-, C₁₋₃-Alkoxy-, Hydroxy-, Amino-, C₁₋₄-Alkylamino-, Di-(C₁₋₄-alkyl)-amino-, Amino-C₁₋₃-alkyl-, C₁₋₄-Alkylamino-C₁₋₃-alkyl-, Di-(C₁₋₄-alkyl)-amino-C₁₋₃-alkyl-, C₁₋₄-Alkylcarbonylamino-, C₁₋₄-Alkylcarbonylamino-C₁₋₃-alkyl-, Aminocarbonyl-, C₁₋₃-Alkyl-aminocarbonyl-, Di-C₁₋₄-alkyl-aminocarbonyl-, Cyano-, Aminosulfonyl-, C₁₋₄-Alkyl-aminosulfonyl-, Di-(C₁₋₄-alkyl)-aminosulfonyl-, C₁₋₃-Alkylthio-, C₁₋₃-Alkyl-sulfinyl-, oder C₁₋₃-Alkyl-sulfonyl- mono-, di- oder trisubstituiert sein können, wobei die Substituenten gleich oder verschieden sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom,
einen C₁₋₄-Alkylrest, wobei ein unverzweigter Alkylrest in ω-Stellung durch eine Phenyl-, Pyridinyl-, Diazinyl-, Amino-, C₁₋₄-Alkylamino-, Di-(C₁₋₄-alkyl)-amino-, 4 C₁₋₄-Alkyl-1-piperazinyl- oder 4-Morpholinylgruppe substituiert sein kann,
eine C₁₋₆-Alkoxycarbonyl-, Cyano- oder Aminocarbonylgruppe oder auch, wenn Y¹ ein Stickstoffatom darstellt, ein freies Elektronenpaar,
oder, wenn Y¹ das Kohlenstoffatom darstellt, auch das Fluoratom, oder
R⁴ zusammen mit R⁵ und Y¹ einen 4- bis 7-gliedrigen cycloaliphatischen Ring, in dem eine Methylengruppe durch eine Gruppe -NH-, -N(C₁₋₄-Alkyl)-, -N(C₃₋₄-Alkenyl)-, -N(C₃₋₄-Alkinyl)-, -N(Cyclo-C₃₋₇-alkyl)- oder -N(C₃₋₇-Cycloalkyl-C₁₋₃-alkyl)- ersetzt sein kann,
wobei ein an ein Stickstoffatom in einer der für R⁴ vorstehend definierten Gruppen gebundenes Wasserstoffatom durch einen Schutzrest ersetzt sein kann,
R⁶ und R⁷, die gleich oder verschieden sein können, jeweils ein Wasserstoffatom, eine C₁₋₄-Alkylgruppe oder auch, wenn Y¹ ein Kohlenstoffatom darstellt, das Fluoratom, eine C₁₋₄-Alkylamino- oder Di-(C₁₋₄-alkyl)-aminogruppe, wobei die beiden C₁₋₄-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen, bedeuten,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen , und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

3. CGRP-Antagonisten der allgemeinen Formel (I) gemäß Anspruch 1, in denen
A, X, R¹, R² und R³ wie in einem der Ansprüche 1 bis 2 erwähnt definiert sind und
(a) D, E unabhängig voneinander jeweils eine Methingruppe oder das Stickstoffatom und
G eine durch die Gruppe R^{a} substituierte Methingruppe,
M eine durch die Gruppe R^{b} substituierte Methingruppe,
Q eine durch die Gruppe R^{c} substituierte Methingruppe,
wobei eine oder zwei der Gruppen G, M und Q jeweils auch ein Stickstoffatom bedeuten kann,
oder
(b) D und E jeweils eine Methingruppe, wobei eine der Gruppen D und E auch ein Stickstoffatom bedeuten kann, und
G, M und Q jeweils ein Stickstoffatom bedeuten,
wobei R^{a}, R^{b} und R^{c} unabhängig voneinander jeweils ein Wasserstoff- oder - Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₄-alkyl-, Hydroxy-C₃₋₄-alkenyl-, Hydroxy-C₃₋₄-alkinyl-, C₁₋₄-Alkoxy-, C₁₋₄-Alkoxy-C₁₋₄-alkyl-, C₁₋₄-Alkoxy-C₃₋₄-alkenyl-, C₁₋₄-Alkoxy-C₃₋₄-alkinyl-, Thiohydroxy-, C₁₋₄-Alkylthio-, Amino-, C₁₋₄-Alkyl-amino-, C₃₋₄-Alkenyl-amino-, C₃₋₄-Alkinyl-amino-, Di-(C₁₋₄-alkyl)-amino-, Di-(C₃₋₄-alkenyl)-amino-, Di-(C₃₋₄-alkinyl)-amino-, Amino-C₁₋₄-alkyl-, C₁₋₃-Alkyl-amino-C₁₋₄-alkyl-, Di-(C₁₋₃-alkyl)-amino-C₁₋₄-alkyl-, Amino-C₃₋₄-alkenyl-, C₁₋₃-Alkyl-amino-C₃₋₄-alkenyl-, Di-(C₁₋₃-alkyl)-amino-C₃₋₄-alkenyl-, Amino-C₃₋₄-alkinyl-, C₁₋₃-Alkyl-amino-C₃₋₄-alkinyl-, Di-(C₁₋₃-alkyl)-amino-C₃₋₄-alkinyl-, Hydroxycarbonyl-, Phenylcarbonyl-, Pyridylcarbonyl-, C₁₋₄-Alkyl-carbonyl-, Formyl-, C₁₋₄-Alkoxy-Carbonyl-, C₃₋₄-Alkenoxy-carbonyl-, C₃₋₄-Alkinoxy-carbonyl-, Aminocarbonyl-, C₁₋₄-Alkyl-aminocarbonyl-, C₃₋₄-Alkenyl-aminocarbonyl-, C₃₋₄-Alkinyl-aminocarbonyl-, Di-(C₁₋₄-alkyl)-aminocarbonyl-, Di-(C₃₋₄-alkenyl)-aminocarbonyl-, Di-C₃₋₄-(alkinyl)-aminocarbonyl-, Formylamino-, C₁₋₄-Alkyl-carbonylamino-, Formyl-C₁₋₄-alkyl-amino-, Formyl-C₃₋₄-alkenyl-amino-, Formyl-C₃₋₄-alkinyl-amino-, C₁₋₄-Alkyl-carbonyl-C₁₋₄-alkyl-amino-, C₁₋₄-Alkyl-carbonyl-C₃₋₄-alkenyl-amino-, C₁₋₄-Alkyl-carbonyl-C₃₋₄-alkinyl-amino-, C₁₋₄-Alkyl-sulfonyl-, C₂₋₄-Alkenyl-sulfonyl-, C₂₋₄-Alkinyl-sulfonyl-, C₁₋₄-Alkyl-sulfinyl-, C₂₋₄-Alkenyl-sulfinyl-, C₂₋₄-Alkinyl-sulfinyl-, C₁₋₄-Alkyl-sulfonylamino-, C₁₋₄-Alkyl-sulfonyl-C₁₋₄-alkylamino-, C₁₋₄-Alkyl-sulfonyl-C₃₋₄-alkenylamino-, C₁₋₄-Alkyl-sulfonyl-C₃₋₄-alkinylamino-, Aminosulfonyl-, C₁₋₄-Alkylaminosulfonyl-, Di-(C₁₋₄-alkyl)-aminosulfonyl-, C₃₋₄-Alkenylamino-sulfonyl-, Di-(C₃₋₄alkenyl)-aminosulfonyl-, C₃₋₄-Alkinylaminosulfonyl- oder Di-(C₃₋₄-alkinyl)-aminosulfonylgruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₄-Alkylgruppe darstellen,
(ii) R^{c} kein Wasserstoffatom bedeutet, wenn R^{a} sowie R^{b} jeweils eine C₁₋₄-Alkylgruppe darstellen,
(iii) R^{a} nicht die Bedeutungen eines Wasserstoff-, Fluor-, Chlor-, Brom- oder Iodatoms oder einer Difluor- oder Trifluormethylgruppe annimmt, wenn R^{c} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder Iodatoms oder einer Difluor- oder Trifluormethylgruppe annimmt, wenn R^{a} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
wobei, soweit nichts anderes erwähnt wurde, alle in den vorstehend für R^{a}, R^{b} und R^{c} definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können,
die Doppel- und Dreifachbindungen der in den für R^{a}, R^{b} und R^{c} vorstehend definierten Gruppen enthaltenen C₃₋₄-Alkenyl- oder C₃₋₄-Alkinylgruppen von in diesen Gruppen gegebenenfalls ebenfalls enthaltenen Heteroatomen isoliert sind,
und alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

4. CGRP-Antagonisten der allgemeinen Formel (I) gemäß Anspruch 1, in denen
A, X, R¹, R² und R³ wie in einem der Ansprüche 1 bis 2 erwähnt definiert sind und
(a) D, E unabhängig voneinander jeweils eine Methingruppe oder das Stickstoffatom und
G eine durch die Gruppe R^{a} substituierte Methingruppe,
M eine durch die Gruppe R^{b} substituierte Methingruppe,
Q eine durch die Gruppe R^{c} substituierte Methingruppe,
wobei eine oder zwei der Gruppen G, M und Q jeweils auch ein Stickstoffatom bedeuten kann,
oder
(b) D und E jeweils eine Methingruppe, wobei eine der Gruppen D und E auch ein Stickstoffatom bedeuten kann, und
G, M und Q jeweils ein Stickstoffatom bedeuten,
wobei R^{a}, R^{b} und R^{c} unabhängig voneinander jeweils ein Wasserstoff- oder Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₂-alkyl-, Hydroxy-C₃-alkenyl-, Hydroxy-C₃-alkinyl-, C₁₋₄-Alkoxy-, C₁₋₄-Alkoxy-C₁₋₂-alkyl-, Amino-, C₁₋₄-Alkyl-amino-, C₃₋₄-Alkenyl-amino-, C₃₋₄-Alkinyl-amino-, Di-(C₁₋₄-alkyl)-amino-, Di-(C₃₋₄-alkenyl)-amino-, Di-(C₃₋₄-alkinyl)-amino-, Amino-C₁₋₂-alkyl-, C₁₋₃-Alkyl-amino-C₁₋₂-alkyl-, Di-(C₁₋₃-alkyl)-amino-C₁₋₂-alkyl-, Amino-C₃-alkenyl-, C₁₋₃-Alkyl-amino-C₃-alkenyl-, Di-(C₁₋₃-alkyl)-amino-C₃-alkenyl-, Amino-C₃-alkinyl-, C₁₋₃-Alkyl-amino-C₃-alkinyl-, Di-(C₁₋₃-alkyl)-amino-C₃-alkinyl-, Hydroxycarbonyl-, C₁₋₄-Alkyl-carbonyl-, Formyl-, C₁₋₄-Alkoxy-carbonyl-, Aminocarbonyl-, C₁₋₄-Alkyl-aminocarbonyl-, Di-(C₁₋₄-alkyl)-aminocarbonyl-, Formylamino-, C₁₋₄-Alkyl-carbonylamino-, Formyl-C₁₋₄-alkyl-amino-, C₁₋₄-Alkyl-carbonyl-C₁₋₄-alkyl-amino-, C₁₋₄-Alkyl-sulfonyl-, C₁₋₄-Alkyl-sulfinyl-, C₁₋₄-Alkyl-sulfonylamino-, C₁₋₄-Alkyl-sulfonyl-C₁₋₄-alkylamino-, Aminosulfonyl-, C₁₋₄-Alkylaminosulfonyl- oder Di-(C₁₋₄-alkyl)-aminosulfonylgruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₄-Alkylgruppe darstellen,
(ii) R^{c} kein Wasserstoffatom bedeutet, wenn R^{a} sowie R^{b} jeweils eine C₁₋₄-Alkylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
wobei, soweit nichts anderes erwähnt wurde, alle in den vorstehend für R^{a}, R^{b} und R^{c} definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können, und
die Doppel- und Dreifachbindungen der in den für R^{a}, R^{b} und R^{c} vorstehend definierten Gruppen enthaltenen C₃₋₄-Alkenyl- oder C₃₋₄-Alkinylgruppen von in diesen Gruppen gegebenenfalls ebenfalls enthaltenen Heteroatomen isoliert sind,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

5. CGRP-Antagonisten der allgemeinen Formel (I) gemäß Anspruch 1, in denen
A, X, R¹, R² und R³ wie in einem der Ansprüche 1 bis 2 erwähnt definiert sind und
(a) D, E unabhängig voneinander jeweils eine Methingruppe oder das Stickstoffatom und
G eine durch die Gruppe R^{a} substituierte Methingruppe,
M eine durch die Gruppe R^{b} substituierte Methingruppe,
Q eine durch die Gruppe R^{c} subsituierte Methingruppe,
wobei eine oder zwei der Gruppen G, M und Q jeweils auch ein Stickstoffatom bedeuten kann,
oder
(b) D und E jeweils eine Methingruppe, wobei eine der Gruppen D und E auch ein Stickstoffatom bedeuten kann, und
G, M und Q jeweils ein Stickstoffatom bedeuten,
wobei R^{a}, R^{b} und R^{c} unabhängig voneinander jeweils ein Wasserstoff- oder Halogenatom, eine C₁₋₄-Alkyl-, C₂₋₄-Alkenyl-, C₂₋₄-Alkinyl-, Cyclo-C₃₋₆-alkyl-, Cyclo-C₃₋₆-alkenyl-, Cyano-, Hydroxy-, Hydroxy-C₁₋₂-alkyl-, C₁₋₄-Alkoxy-, Amino-, C₁₋₄-Alkylamino-, Di-(C₁₋₄-alkyl)-amino-, Amino-C₁₋₂-alkyl-, C₁₋₃-Alkyl-amino-C₁₋₂-alkyl-, Di-(C₁₋₃-alkyl)-aminO-C₁₋₂-alkyl-, Hydroxycarbonyl-, C₁₋₄-Alkyl-carbonyl-, Formyl-, C₁₋₄-Alkoxy-carbonyl-, Aminocarbonyl-, C₁₋₄-Alkyl-aminocarbonyl-, Di-(C₁₋₄-alkyl)-aminocarbonyl-, Formylamino-, C₁₋₄-Alkyl-carbonylamino-, Formyl-C₁₋₄-alkyl-amino- oder C₁₋₄-Alkyl-carbonyl-C₁₋₄-alkyl-aminogruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine C₁₋₄-Alkylgruppe darstellen,
(ii) R^{c} kein Wasserstoffatom bedeutet, wenn R^{a} sowie R^{b} jeweils eine C₁₋₄-Alkylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine C₁₋₄-Alkyl-, C₂₋₄-Alkenyl- oder C₂₋₄-Alkinylgruppe und R^{b} ein Chlor- oder Bromatom, eine Amino-, Methylamino- oder Hydroxygruppe darstellen,
wobei, soweit nichts anderes erwähnt wurde, alle in den vorstehend für R^{a}, R^{b} und R^{c} definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4- bis 7-gliedrigen, gesättigten oder ungesättigten heterocyclischen Ringes miteinander verbunden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

6. CGRP-Antagonisten der allgemeinen Formel (I) gemäß Anspruch 1, in denen
A, X, R¹, R² und R³ wie in einem der Ansprüche 1 bis 2 erwähnt definiert sind und
(a) D, E unabhängig voneinander jeweils eine Methingruppe oder das Stickstoffatom und
G eine durch die Gruppe R^{a} substituierte Methingruppe,
M eine durch die Gruppe R^{b} substituierte Methingruppe,
Q eine durch die Gruppe R^{c} substituierte Methingruppe,
wobei eine oder zwei der Gruppen G, M und Q jeweils auch ein Stickstoffatom bedeuten kann,
oder
(b) D und E jeweils eine Methingruppe, wobei eine der Gruppen D und E auch ein Stickstoffatom bedeuten kann, und
G, M und Q jeweils ein Stickstoffatom bedeuten,
wobei R^{a}, R^{b} und R^{c} unabhängig voneinander jeweils ein Wasserstoff- oder Halogenatom, eine Methyl-, Difluormethyl-, Trifluormethyl-, Ethyl-, Vinyl-, Ethinyl-, Cyano-, Hydroxy-, Methoxy-, Difluormethoxy-, Trifluormethoxy-, Amino-, Methylamino- oder Dimethylaminogruppe darstellen,
mit den Maßgaben, dass, sofern keine der Gruppen D, E, G, M und Q ein Stickstoffatom darstellt,
(i) R^{a} kein Wasserstoffatom bedeutet, wenn R^{b} sowie R^{c} jeweils eine Methyl- oder Ethylgruppe darstellen,
(ii) R^{c} kein Wasserstoffatom bedeutet, wenn R^{a} sowie R^{b} jeweils eine Methyl- oder Ethylgruppe darstellen,
(iii) R^{a} nicht die Bedeutungen eines Wasserstoff-, Fluor-, Chlor-, Brom- oder lodatoms oder einer Difluor- oder Trifluormethylgruppe annimmt, wenn R^{c} eine Methyl-, Ethyl-, Vinyl- oder Ethinylgruppe und R^{b} ein Chlor- oder Bromatom, eine Amino-, Methylamino- oder Hydroxygruppe darstellen,
(iv) R^{c} nicht die Bedeutungen eines Wasserstoff-, Fluor-, Chlor-, Brom- oder lodatoms oder einer Difluor- oder Trifluormethylgruppe annimmt, wenn R^{a} eine Methyl-, Ethyl-, Vinyl- oder Ethinylgruppe und R^{b} ein Chlor- oder Bromatom, eine Amino-, Methylamino- oder Hydroxygruppe darstellen,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

7. CGRP-Antagonisten der allgemeinen Formel (I) gemäß Anspruch 1, in denen
A, X, D, E, G, M, Q und R¹ wie in einem der Ansprüche 1 bis 6 erwähnt definiert sind und
R² das Wasserstoffatom oder
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Hydroxy-, Amino-, C₁₋₆-Alkylamino-, Di-(C₁₋₆-alkyl)-amino-, Hydroxycarbonyl-, C₁₋₆-Alkoxycarbonyl-, Aminocarbonyl-, Aminocarbonylamino-, C₁₋₆-Alkylamino-, 4-Morpholinylgruppe substituiert sein kann,
wobei die in den für R² vorstehend definierten Gruppen genannten oder als Substituenten enthaltenen Phenyl- und Pyridinylreste zusätzlich im Kohlenstoffgerüst durch Halogen, durch C₁₋₃-Alkyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkylamino-C₁₋₃-alkyl-, Di-(C₁₋₃-alkyl)-amino-C₁₋₃-alkyl-, C₁₋₃-Alkylcarbonylamino-, C₁₋₃-Alkylcarbonyl-C₁₋₃-alkylamino-, Aminocarbonyl-, C₁₋₃-Alkylaminocarbonyl- oder Di-(C₁₋₃-alkyl)-aminocarbonylgruppen mono-, di- oder trisubstituiert und die Substituenten gleich oder verschieden sein können,
R³ das Wasserstoffatom oder eine C₁₋₃-Alkylgruppe darstellen oder
R² und R³ zusammen mit dem eingeschlossenen Stickstoffatom einen Rest der allgemeinen Formel 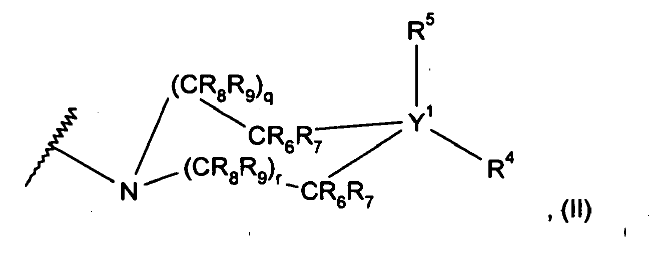 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl- oder Cyclo-C₃₋₇-alkenylgruppe, eine Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₄-Alkyl-a minocarbonyl-, Di-(C₁₋₄-alkyl)-aminocarbonyl-, Aminocarbonyl-C₁₋₃-alkyl-, C₁₋₄-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl-, Pyridinyl- oder Diazinylgruppe, die jeweils durch ein Halogen, durch eine C₁₋₃-Alkyl-, C₁₋₃-Alkoxy-, Hydroxy-, Amino-, C₁₋₄-Alkyl-amino-, Di-(C₁₋₄-alkyl)-amino- Amino-C₁₋₃-alkyl-, C₁₋₄-Alkyl-amino-C₁₋₃-alkyl-, Di-(C₁₋₄-alkyl)-amino-C₁₋₃-alkylgruppe substituiert sein können,
ein Heterocyclus ausgewählt aus einer 4- bis 7-gliedrigen Azacycloalkylgruppe, einer 6- bis 7-gliedrigen Oxaza-, S,S-Dioxothiaza- und Diazacycloalkylgruppe und einer 7- bis 9-gliedrigen Azabicycloalkylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methylengruppe durch ein oder zwei Fluoratome substituiert sein kann und
die vorstehend genannten mono- und bicyclischen Heterocyclen ein- oder zweifach durch Hydroxy-, C₁₋₃-Alkyl- oder Hydroxy-C₁₋₃-alkylgruppen oder einfach durch eine Benzyl-, Cyclo-C₃₋₆-alkyl-, Hydroxycydo-C₃₋₆-alkyl-, Cyclo-C₃₋₆-alkyl-C₁₋₃-alkyl, C₁₋₄-Alkylcarbonyl-, C₁₋₄-Alkylcarbonyl-C₁₋₃-alkyl-, Hydroxy-, C₁₋₄-Alkoxy-, Amino-, C₁₋₄-Alkylamino-, Di-(C₁₋₄-alkyl)-amino-, C₁₋₃-Alkoxycarbonyl-, Hydroxycarbonyl-carbonyl-, C₁₋₃-Alkoxycarbonyl-carbonyl-, Hydroxycarbonyl-C₁₋₃-alkyl-, C₁₋₃-Alkoxycarbonyl-C₁₋₃-alkyl-, Hydroxycarbonyl-C₁₋₃-alkylcarbonyl-, C₁₋₃-Alkoxycarbonyl-C₁₋₃-alkylcarbonyl-, Aminosulfonyl-, C₁₋₄-Alkylaminosulfonyl-, Di-(C₁₋₄-alkyl)-aminosulfonyl-, C₁₋₃-Alkylsulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)-aminocarbonyl-C₁₋₃-alkyl-, Hydroxyaminocarbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppen substituiert sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom, einen C₁₋₃-Alkylrest oder, wenn Y¹ ein Stickstoffatom darstellt, auch ein freies Elektronenpaar,
R⁶ und R⁷, die gleich oder verschieden sein können, jeweils ein Wasserstoffatom, eine C₁₋₃-Alkylgruppe oder auch, wenn Y¹ ein Kohlenstoffatom darstellt, eine Amino-, C₁₋₃-Alkylamino- oder Di-(C₁₋₃-alkyl)-aminogruppe, wobei die beiden C₁₋₃-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
bedeuten,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

8. CGRP-Antagonisten der allgemeinen Formel (I) gemäß Anspruch 1, in denen
A, X, D, E, G, M, Q und R¹ wie in einem der Ansprüche 1 bis 6 erwähnt definiert sind und
R² das Wasserstoffatom oder
eine Phenylmethylgruppe oder eine C₂₋₇-Alkylgruppe, die in ω-Stellung durch eine Cyclo-C₃₋₇-alkyl-, Cyclo-C₃₋₇-alkenyl-, Phenyl-, Pyridinyl-, Hydroxy-, Amino-, C₁₋₆-Alkylamino-, Di-(C₁₋₆-alkyl)-amino-, Hydroxycarbonyl-, C₁₋₆-Alkoxycarbonyl-, Aminocarbonyl-, Aminocarbonylamino-, C₁₋₆-Alkylamino-, 4-Morpholinylgruppe substituiert sein kann,
wobei die in den für R² vorstehend definierten Gruppen genannten oder als Substituenten enthaltenen Phenyl- und Pyridinylreste zusätzlich im Kohlenstoffgerüst durch Halogen, durch C₁₋₃-Alkyl-, C₁₋₃-Alkoxy-, Hydroxy-, Amino-, C₁₋₃-Alkylamino-, Di-(C₁₋₃-alkyl)-amino-, Amino-C₁₋₃-alkyl-, C₁₋₃-Alkylamino-C₁₋₃-alkyl-, Di-(C₁₋₃-alkyl)-amino-C₁₋₃-alkyl-, C₁₋₃-Alkylcarbonylamino-, C₁₋₃-Alkylcarbonyl-C₁₋₃-alkylamino-, Aminocarbonyl-, C₁₋₃-Alkylaminocarbonyl- oder Di-(C₁₋₃-alkyl)-aminocarbonylgruppen mono-, di- oder trisubstituiert und die Substituenten gleich oder verschieden sein können,
R³ das Wasserstoffatom oder eine C₁₋₃-Alkylgruppe darstellen oder
R² und R³ zusammen mit dem eingeschlossenen Stickstoffatom einen Rest der allgemeinen Formel 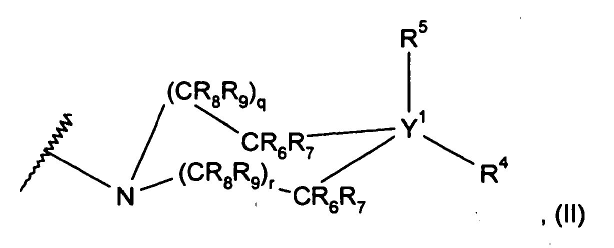 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, Cyclo-C₃₋₇-alkyl-, Cyclo-C₃₋₇-alkenyl-, Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₄-Alkyl-aminocarbonyl-, Di-(C₁₋₄-alkyl)-aminocarbonyl-, Aminocarbonyl-C₁₋₃-alkyl-, C₁₋₋₄-Alkyl-aminocarbonyl-C₁₋₃-alkyl-, Di-(C₁₋₄-alkyl)-aminocarbonyl-C₁₋₃-alkyl-, Aminocarbonylamino-C₁₋₃-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl-, Pyridinyl- oder Diazinylgruppe, die jeweils durch ein Halogen, durch eine C₁₋₃-Alkyl-, C₁₋₃-Alkoxy-, Hydroxy-, Amino-, C₁₋₄-Alkyl-amino-, Di-(C₁₋₄-alkyl)-amino- Amino-C₁₋₃-alkyl-, C₁₋₄-Alkyl-amino-C₁₋₃-alkyl-, Di-(C₁₋₄-alkyl)-amino-C₁₋₃-alkylgruppe substituiert sein können,
ein Heterocyclus ausgewählt aus einer 4- bis 7-gliedrigen Azacycloalkylgruppe, einer 6- bis 7-gliedrigen Oxaza- und Diazacycloalkylgruppe und einer 7- bis 9-gliedrigen Azabicycloalkylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methylengruppe durch ein oder zwei Fluoratome substituiert sein kann und
die vorstehend genannten mono- und bicyclischen Heterocyclen ein- oder mehrfach, beispielsweise ein- bis dreifach, durch C₁₋₃-Alkylgruppen oder einfach durch eine Benzyl-, Cyclo-C₃₋₆-alkyl-, Cyclo-C₃₋₆-alkyl-C₁₋₃-alkyl, C₁₋₄-Alkylcarbonyl-, Hydroxy-, C₁₋₄-Alkoxy-, Amino-, C₁₋₄-Alkylamino-, Di-(C₁₋₄-alkyl)-amino-, Hydroxycarbonyl-, C₁₋₃-Alkoxycarbonyl-, Hydroxycarbonyl-carbonyl-, C₁₋₃-Alkoxycarbonyl-carbonyl-, Hydroxycarbonyl-C₁₋₃-alkyl-, C₁₋₃-Alkoxycarbonyl-C₁₋₃-alkyl- oder C₁₋₃-Alkylsulfonylgruppen substituiert sein können,
oder auch, wenn Y¹ das Kohlenstoffatom darstellt, die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom, einen C₁₋₃-Alkylrest oder, wenn Y¹ ein Stickstoffatom darstellt, auch ein freies Elektronenpaar,
R⁶ und R⁷, die gleich oder verschieden sein können, jeweils ein Wasserstoffatom, eine C₁₋₃-Alkylgruppe oder auch, wenn Y¹ ein Kohlenstoffatom darstellt, eine C₁₋₃-Alkylamino- oder Di-(C₁₋₃-alkyl)-aminogruppe, wobei die beiden C₁₋₃-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
bedeuten,
wobei, soweit nichts anderes erwähnt wurde, alle in den vorstehend definierten Resten erwähnten oder enthaltenen Alkyl-, Alkenyl- und Alkinylgruppen geradkettig oder verzweigt sein können, jede in den vorstehend definierten Resten enthaltene Methingruppe mit einem Fluoratom, jede Methylengruppe mit bis zu 2 Fluoratomen und jede Methylgruppe mit bis zu 3 Fluoratomen substituiert sein kann sowie zwei an ein Stickstoffatom gebundene Alkyl- und Alkenylgruppen unter Ausbildung eines 4-bis 7-gliedrigen, gesättigten oder ungesättigten heterocyclischen Ringes miteinander verbunden sein können,
alle in den vorstehend definierten Resten erwähnten oder enthaltenen aromatischen und heteroaromatischen Reste zusätzlich durch Halogen, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

9. CGRP-Antagonisten der allgemeinen Formel (I) gemäß Anspruch 1, in denen
A, X, D, E, G, M, Q und R¹ wie in einem der Ansprüche 1 bis 6 erwähnt definiert sind und
R² das Wasserstoffatom oder
eine Phenylmethylgruppe oder eine C₂₋₇-Alkylgruppe, die in ω-Stellung durch eine Phenyl-, Amino-, C₁₋₆-Alkylamino-, Di-(C₁₋₆-alkyl)-aminogruppe substituiert sein kann,
wobei die vorstehend erwähnte Phenyl- und Phenylmethylgruppe zusätzlich an einem aromatischen Kohlenstoffatom durch Halogen, durch C₁₋₃-Alkyl-, C₁₋₃-Alkoxy-, Amino-C₁₋₃-alkyl-, C₁₋₃-Alkylamino-C₁₋₃-alkyl- oder Di-(C₁₋₃-alkyl)-amino-C₁₋₃-alkylgruppen mono- oder disubstituiert und die Substituenten gleich oder verschieden sein können,
R³ das Wasserstoffatom oder eine C₁₋₃-Alkylgruppe darstellen oder
R² und R³ zusammen mit dem eingeschlossenen Stickstoffatom einen Rest der allgemeinen Formel 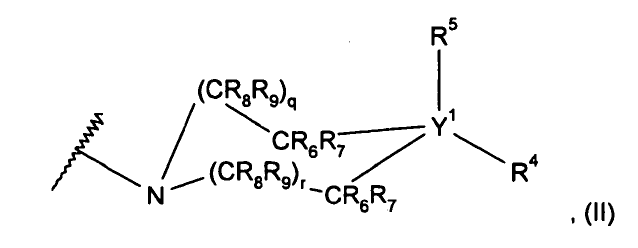 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0 oder 1 oder
q und r, wenn Y¹ das Stickstoffatom darstellt, die Zahlen 1 oder 2,
R⁴ das Wasserstoffatom, eine Amino-, C₁₋₄-Alkyl-amino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl- oder Cyclo-C₃₋₇-alkenylgruppe, eine Amino-C₂₋₇-alkyl-, C₁₋₄-Alkyl-amino-C₂₋₇alkyl-, Di-(C₁₋₄-alkyl-amino)-C₂₋₇-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl- oder Pyridylgruppe, die jeweils durch ein Halogen, durch eine C₁₋₃-Alkyl-, C₁₋₃-Alkoxy-, Amino-, C₁₋₄-Alkyl-amino-, Di-(C₁₋₄-alkyl)-aminogruppe substituiert sein kann,
ein Heterocyclus ausgewählt aus einer 6- bis 7-gliedrige Azacycloalkylgruppe, einer 6- bis 7-gliedrige S,S-Dioxothiaza- und Diazacycloalkylgruppe und einer 7- bis 9-gliedrige Azabicycloalkylgruppe,
wobei die vorstehend genannten mono- und bicyclischen Heterocyclen über ein Stickstoff- oder ein Kohlenstoffatom an Y¹ in Formel (II) gebunden sind,
in den vorstehend genannten mono- und bicyclischen Heterocyclen eine nicht direkt mit einem Stickstoff-, Sauerstoff- oder Schwefelatom verknüpfte Methylengruppe durch ein oder zwei Fluoratome substituiert sein kann und die vorstehend genannten mono- und bicyclischen Heterocyclen ein- oder zweifach durch eine Hydroxy-, C₁₋₃-Alkyl- oder Hydroxy-C₁₋₃-alkylgruppe, durch eine Benzyl-, Cyclo-C₃₋₆-alkyl-, Hydroxy-C₃₋₆-cycloalkyl-, Cyclo-C₃₋₆-alkyl-C₁₋₃-alkyl, C₁₋₃-Alkylcarbonyl-C₁₋₃-alkyl-, Amino-, C₁₋₄-Alkylamino- oder Di-(C₁₋₄-alkyl)-amino-, Hydroxycarbonyl-carbonyl-, C₁₋₆-Alkoxycarbonyl-carbonyl-, Hydroxycarbonyl-C₁₋₃-alkylcarbonyl-, C₁₋₃-Alkoxycarbonyl-C₁₋₃-alkylcarbonyl-, Aminosulfonyl-, C₁₋₄-Alkylaminosulfonyl-, Di-(C₁₋₄-alkyl)-aminosulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)-aminocarbonyl-C₁₋₃-alkyl-, Hydroxyaminocarbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppen substituiert sein können,
oder auch, wenn Y¹ das Kohlenstoffatom darstellt die Hydroxycarbonyl-, Aminomethyl-, C₁₋₄-Alkyl-aminomethyl- oder Di-(C₁₋₄-alkyl)-aminomethylgruppe,
R⁵ ein Wasserstoffatom oder, wenn Y¹ ein Stickstoffatom darstellt, auch ein freies Elektronenpaar,
R⁶ und R⁷, die gleich oder verschieden sein können, jeweils ein Wasserstoffatom, eine C₁₋₃-Alkylgruppe oder auch, wenn Y¹ ein Kohlenstoffatom darstellt, eine C₁₋₃-Alkylamino- oder Di-(C₁₋₃-alkyl)-aminogruppe, wobei die beiden C₁₋₃-Alkylgruppen unter Ausbildung eines Ringes miteinander verbunden sein können und
R⁸ und R⁹, die gleich oder verschieden sein können, jeweils ein Wasserstoffatom oder eine C₁₋₃-Alkylgruppe darstellen,
bedeuten, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

10. CGRP-Antagonisten der allgemeinen Formel (I) gemäß Anspruch 1, in denen
A, X, D, E, G, M, Q und R¹ wie in einem der Ansprüche 1 bis 6 erwähnt definiert sind und
R² eine Phenylmethylgruppe oder eine C₂₋₇-Alkylgruppe, die in ω-Stellung durch eine Phenyl-, Amino-, C₁₋₆-Alkylamino-, Di-(C₁₋₆-alkyl)-aminogruppe substituiert sein kann,
wobei die vorstehend genannte Phenyl- und Phenylmethylgruppe an einem aromatischen Kohlenstoffatom durch eine Amino-C₁₋₃-alkyl-, C₁₋₃-Alkylamino-C₁₋₃-alkyl- oder Di-(C₁₋₃-alkyl)-amino-C₁₋₃-alkylgruppe substituiert sein kann,
R³ das Wasserstoffatom oder eine C₁₋₃-Alkylgruppe oder
R² und R³ zusammen mit dem eingeschlossenen Stickstoffatom einen Rest der allgemeinen Formel 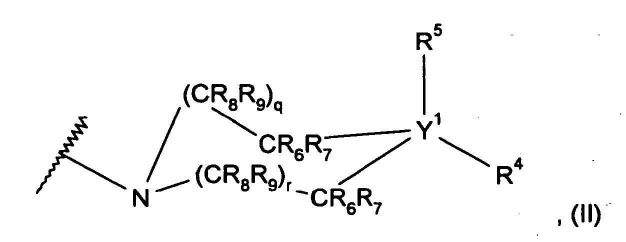 in der
R⁶ und R⁷ jeweils ein Wasserstoffatom oder eine Dimethylaminogruppe,
R⁸ und R⁹ jeweils das Wasserstoffatom und
(a) Y¹ das Kohlenstoffatom,
q und r die Zahlen 0 oder 1,
R⁴ das Wasserstoffatom,
eine Phenyl-, Pyridinyl- oder Pyrimidinylgruppe, die jeweils durch ein Halogen, durch eine Amino-, Methylamino-, Dimethylamino-, Methyl- oder Methoxygruppe substituiert sein kann,
eine Hydroxy-, 2-Diethylamino-ethyl-, Amino-, Methylamino-, Dimethylamino-, Diethylamino-, Pyrrolidin-1-yl-, 3-Hydroxy-pyrrolidin-1-yl-, 2-Hydroxycarbonyl-pyrrolidin-1-yl-, 2-Methoxycarbonyl-pyrrolidin-1-yl-, Piperidin-1-yl-, 4,4-Dimethylpiperidin-1-yl-, 4-Amino-4-methyl-piperidin-1-yl-, 2-Hydroxycarbonyl-piperidin-1-yl-, 2-Methoxycarbonyl-piperidin-1-yl-, 4-Hydroxymethyl-piperidin-1-yl-, 4-(1-Hydroxycyclopropyl)-piperidin-1-yl-, 4-Amino-piperidin-1-yl-, 4-Methylamino-piperidin-1-yl-, 4-Dimethylamino-piperidin-1-yl-, 4-Hydroxy-4-methyl-piperidin-1-yl-, 4-Hydroxy-4-ethyl-piperidin-1-yl-, 4-Hydroxy-4-trifluormethyl-piperidin-1-yl-, 4-Hydroxy-4-hydroxymethyl-piperidin-1-yl-, 3-Amino-piperidin-1-yl-, 3-Methylamino-piperidin-1-yl-, 3-Dimethylamino-piperidin-1-yl-, 3-Hydroxy-piperidin-1-yl-, 4-Hydroxypiperidin-1-yl-, 4-Hydroxycarbonylmethyl-piperidin-1-yl-, 4-Ethoxycarbonylmethyl-piperidin-1-yl-, Perhydro-azepin-1-yl-, Perhydro-1,4-diazepin-1-yl-, 4-Methyl-perhydro-1,4-diazepin-1-yl-, 1-Methyl-piperidin-4-yl-, Piperidin-4-yl-, 1-Ethylpiperidin-4-yl-, 1-(2-Hydroxyethyl)-piperidin-4-yl-, 1-Cyclopropyl-piperidin-4-yl-, 1-Cyclopropylmethyl-piperidin-4-yl-, 1-Methylsulfonyl-piperidin-4-yl-, 1-Ethylsulfonyl-piperidin-4-yl-, 1-Isopropylsulfonyl-piperidin-4-yl-, 1-Cyclopropylsulfonyl-piperidin-4-yl-, 4-Hydroxy-1-methylsulfonyl-piperidin-4-yl-, 1-Aminosulfonyl-piperidin-4-yl-, 1-(Methylaminosulfonyl)-piperidin-4-yl-, 1-(Dimethylaminosulfonyl)-piperidin-4-yl-, 1-Hydroxycarbonylmethyl-piperidin-4-yl-, 1-Ethoxycarbonylmethyl-piperidin-4-yl-, 1-(2-Hydroxycarbonylethyl)-piperidin-4-yl-, 1-(2-Ethoxycarbonylethyl)-piperidin-4-yl-, 1-(3-Hydroxycarbonyl-propionyl)-piperidin-4-yl-, 1-(3-Ethoxycarbonyl-propionyl)-piperidin-4-yl-, 1-(Hydroxycarbamoyl-methyl)-piperidin-4-yl-, 1-(Hydroxy-methylcarbamoyl-methyl)-piperidin-4-yl-, 1-(Methoxycarbamoyl-methyl)-piperidin-4-yl-, 1-Oxalyl-piperidin-4-yl-, 1-Ethoxyoxalyl-piperidin-4-yl-, Piperazin-1-yl-, 4-Methyl-piperazin-1-yl-, 4-Cyclopropylmethyl-piperazin-1-yl-, 4-Ethyl-piperazin-1-yl-, 4-(2-Hydroxyethyl)-piperazin-1-yl-, 4-Cyclopropyl-piperazin-1-yl-, 4-Methylsulfonyl-piperazin-1-yl-, 4-Aminosulfonyl-piperazin-1-yl-, 4-(Methylaminosulfonyl)-piperazin-1-yl-, 4-(Dimethylaminosulfonyl)-piperazin-1-yl-, 4-Hydroxycarbonylmethyl-piperazin-1-yl-, 4-Ethoxycarbonylmethyl-piperazin-1-yl-, 4-(2-Hydroxycarbonylethyl)-piperazin-1-yl-, 4-(2-Ethoxycarbonylethyl)-piperazin-1-yl-, 4-(3-Hydroxycarbonyl-propionyl)-piperazin-1-yl-, 4-(3-Ethoxycarbonyl-propionyl)-piperazin-1-yl-, 4-(Hydroxycarbamoyl)-methyl-piperazin-1-yl-, 4-(Hydroxy-methyl-carbamoyl)-methyl-piperazin-1-yl-, 4-(Methoxycarbamoyl)-methyl-piperazin-1-yl-, 1,2-Dimethyl-piperazin-1-yl-, 3-Methyl-piperazin-1-yl-, 3,4,5-Trimethyl-piperazin-1-yl-, 3,5-Dimethyl-piperazin-1-yl-, 3,3,4-Trimethyl-piperazin-1-yl-, 3,3-Dimethyl-piperazin-1-yl-, 3,3,4,5,5-Pentamethyl-piperazin-1-yl-, 3,3,5,5-Tetramethyl-piperazin-1-yl-, 3,3,3-Trifluor-2-oxo-propyl)-piperazin-1-yl-, Morpholin-4-yl-, 1,1-Dioxo-1λ⁶-thiomorpholin-4-yl-, Tetrahydropyran-4-yl-, 4,4-Difluor-piperidin-1-yl-, 8-Methyl-8-aza-bicyclo[3.2.1]oct-3-yl-, 8-Aza-bicyclo[3.2.1]oct-3-yl-, Azetidin-1-yl-, 1-(Methoxycarbonylmethyl)-piperidin-4-yl-, 1-(Ethoxycarbonylmethyl)-piperidin-4-yl-, 4-(Ethoxycarbonylmethyl)-piperazin-1-yl-, 1-Hydroxycarbonylmethyl-piperidin-4-yl- oder 4-Hydroxycarbonylmethyl-piperazin-1-ylgruppe, und
R⁵ ein Wasserstoffatom, oder
(b) Y¹ ein Stickstoffatom,
q und r die Zahlen 1 oder 2,
R⁴ das Wasserstoffatom,
eine Phenyl-, Pyridinyl- oder Pyrimidinylgruppe, die jeweils durch ein Halogen, durch eine Amino-, Methylamino-, Dimethylamino-, Methyl- oder Methoxygruppe substituiert sein kann,
eine Methyl-, Ethyl-, Isopropyl-, Cyclopropyl-, Cyclopentyl-, Cyclohexyl-, Cycloheptyl-, 4-Hydroxycarbonylmethyl-cylohexyl-, 4-Ethoxycarbonylmethyl-cyclohexyl-, Cyclopropylmethyl-, 2-Diethylamino-propyl-, 1-Chinuclidin-3-yl-, Tetrahydropyran-4-yl-, 1-Piperidin-4-yl-, 1-Methyl-piperidin-4-yl-, 1-Ethyl-piperidin-4-yl-, 1-(2-Hydroxyethyl)-piperidin-4-yl-, 1-Methylsulfonyl-piperidin-4-yl-, 1-Aminosulfonyl-piperidin-4-yl-, 1-(Methylaminosulfonyl)-piperidin-4-yl-, 1-(Dimethylaminosulfonyl)-piperidin-4-yl-, 1-Hydroxycarbonylmethyl-piperidin-4-yl-, 1-Ethoxycarbonylmethyl-piperidin-4-yl-, 1-(2-Hydroxycarbonylethyl)-piperidin-4-yl-, 1-(2-Ethoxycarbonylethyl)-piperidin-4-yl-, 1-(3-Hydroxycarbonyl-propionyl)-piperidin-4-yl-, 1-(3-Ethoxycarbonyl-propionyl)-piperidin-4-yl-, 1-(Hydroxycarbamoyl-methyl)-piperidin-4-yl-, 1-(Hydroxy-methyl-carbamoyl-methyl)-piperidin-4-yl-, 1-(Methoxycarbamoyl-methyl)-piperidin-4-yl-, 1-Cyclopropyl-piperidin-4-yl-, 1-Cyclopropylmethyl-piperidin-4-yl-, 1-Hydroxycarbonylmethyl-piperidin-4-yl- oder 1-Ethoxycarbonylmethyl-piperidin-4-ylgruppe und
R⁵ ein freies Elektronenpaar darstellen,
bedeuten, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

11. CGRP-Antagonisten der allgemeinen Formel (I) gemäß Anspruch 1, in denen
A, X, D, E, G, M, Q und R¹ wie in einem der Ansprüche 1 bis 6 erwähnt definiert sind und
R² eine Phenylmethylgruppe oder eine C₂₋₇-Alkylgruppe, die in ω-Stellung durch eine Phenyl-, Amino-, C₁₋₆-Alkylamino-, Di-(C₁₋₆-alkyl)-aminogruppe substituiert sein kann,
wobei die vorstehend genannte Phenyl- und Phenylmethylgruppe an einem aromatischen Kohlenstoffatom durch eine Amino-C₁₋₃-alkyl-, C₁₋₃-Alkylamino-C₁₋₃-alkyl- oder Di-(C₁₋₃-alkyl)-amino-C₁₋₃-alkylgruppe substituiert sein kann,
R³ das Wasserstoffatom oder eine C₁₋₃-Alkylgruppe oder
R² und R³ zusammen mit dem eingeschlossenen Stickstoffatom einen Rest der allgemeinen Formel 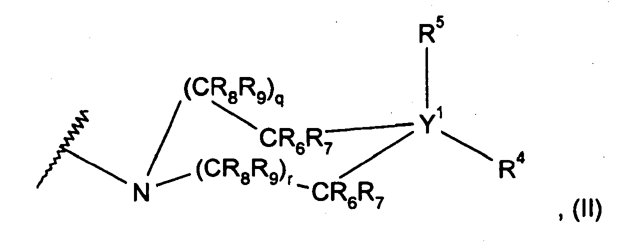 in der
R⁶ und R⁷ jeweils ein Wasserstoffatom oder eine Dimethylaminogruppe,
R⁸ und R⁹ jeweils das Wasserstoffatom und
(a) Y¹ das Kohlenstoffatom,
q und r die Zahlen 0 oder 1,
R⁴ das Wasserstoffatom,
eine Phenyl-, Pyridinyl- oder Pyrimidinylgruppe, die jeweils durch ein Halogen, durch eine Amino-, Methylamino-, Dimethylamino-, Methyl- oder Methoxygruppe substituiert sein kann,
eine Hydroxy-, 2-Diethylamino-ethyl-, Amino-, Methylamino-, Dimethylamino-, Diethylamino-, Pyrrolidin-1-yl-, Piperidin-1-yl-, 4-Amino-piperidin-1-yl-, 4-Methylamino-piperidin-1-yl-, 4-Dimethylamino-piperidin-1-yl-, 3-Amino-piperidin-1-yl-, 3-Methylamino-piperidin-1-yl-, 3-Dimethylamino-piperidin-1-yl-, Perhydro-azepin-1-yl-, Perhydro-1,4-diazepin-1-yl-, 4-Methyl-perhydro-1,4-diazepin-1-yl-, 1-Methyl-piperidin-4-yl-, Piperidin-4-yl-, 1-Ethylpiperidin-4-yl-, 1-Cyclopropyl-piperidin-4-yl-, 1-Cyclopropylmethyl-piperidin-4-yl-, Piperazin-1-yl-, 4-Methyl-piperazin-1-yl-, 4-Cyclopropylmethyl-piperazin-1-yl-, 4-Ethyl-piperazin-1-yl-, 4-Cyclopropyl-piperazin-1-yl-, 1,2-Dimethyl-piperazin-1-yl-, 3-Methyl-piperazin-1-yl-, 3,4,5-Trimethyl-piperazin-1-yl-, 3,5-Dimethyl-piperazin-1-yl-, 3,3,4-Trimethyl-piperazin-1-yl-, 3,3-Dimethyl-piperazin-1-yl-, 3,3,4,5,5-Pentamethyl-piperazin-1-yl-, 3,3,5,5-Tetramethyl-piperazin-1-yl-, Morpholin-4-yl-, 4,4-Difluor-piperidin-1-yl-, 8-Methyl-8-aza-bicyclo[3.2.1]oct-3-yl-, 8-Aza-bicyclo[3.2.1]oct-3-yl-, Azetidin-1-yl-, 1-(Methoxycarbonylmethyl)-piperidin-4-yl-, 1-(Ethoxycarbonylmethyl)-piperidin-4-yl-, 4-(Ethoxycarbonylmethyl)-piperazin-1-yl-, 1-Hydroxycarbonylmethyl-piperidin-4-yl- oder 4-Hydroxycarbonylmethyl-piperazin-1-ylgruppe, und
R⁵ ein Wasserstoffatom, oder
(b) Y¹ ein Stickstoffatom,
q und r die Zahlen 1 oder 2,
R⁴ das Wasserstoffatom,
eine Phenyl-, Pyridinyl- oder Pyrimidinylgruppe, die jeweils durch ein Halogen, durch eine Amino-, Methylamino-, Dimethylamin-, Methyl- oder Methoxygruppe substituiert sein kann,
eine Methyl-, Ethyl-, Isopropyl-, Cyclopropyl-, Cyclopropylmethyl-, 2-Diethylamino-propyl-, 1-Chinuclidin-3-yl-, 1-Piperidin-4-yl-, 1-Methylpiperidin-4-yl-, 1-Ethyl-piperidin-4-yl-, 1-Cyclopropyl-piperidin-4-yl-, 1-Cyclopropylmethyl-piperidin-4-yl-, 1-Hydroxycarbonylmethyl-piperidin-4-yl- oder 1-Ethoxycarbonylmethyl-piperidin-4-ylgruppe und
R⁵ ein freies Elektronenpaar darstellen,
bedeuten, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

12. CGRP-Antagonisten der allgemeinen Formel (I) gemäß Anspruch 1, in denen
D, E, G, M, Q, R¹, R² und R³ wie in einem der Ansprüche 1 bis 10 erwähnt definiert sind und
A und X jeweils ein Sauerstoffatom darstellen,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

13. CGRP-Antagonisten der allgemeinen Formel (I) gemäß Anspruch 1, in denen
A und X jeweils ein Sauerstoffatom darstellen,
R¹ eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-*c*]-chinolin-2-on-3-yl-, 1,3-Dihydro-naphth[1,2-*d*]imidazol-2-on-3-yl-, 1,3-Dihydro-benzimidazol-2-on-3-yl-, 4-Phenyl-1,3-dihydro-imidazol-2-on-1-yl-3,4-Dihydro-1*H*-thieno-[3,2-*d*]pyrimidin-2-on-3-yl- oder 3,4-Dihydro-1*H*-thieno[3,4-*d*]pyrimidin-2-on-3-ylgruppe bedeutet,
und R² und R³ wie in Anspruch 1 oder 2 erwähnt definiert sind,
wobei die vorstehend unter R¹ erwähnten Heterocyclen im Kohlenstoffgerüst zusätzlich durch eine Methoxygruppe monosubstituiert sein können,
und wobei alle in den unter R¹ definierten Resten erwähnten oder enthaltenen aromatischen und heteroaromatischen Reste und Molekülteile zusätzlich durch Halogenatome, durch Cyano- oder Hydroxygruppen mono-, di- oder trisubstituiert und die Substituenten gleich oder verschieden sein können,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

14. CGRP-Antagonisten der allgemeinen Formel (I) gemäß Anspruch 1, in denen
A und X jeweils ein Sauerstoffatom bedeuten,
R¹ wie in Anspruch 1 definiert ist,
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe und
R^{a}, R^{b} und R^{c} unabhängig voneinander jeweils ein Wasserstoff- oder Halogenatom, eine Methyl-, Difluormethyl-, Trifluormethyl-, Ethyl-, Vinyl-, Ethinyl-, Cyano-, Hydroxy-, Methoxy-, Difluormethoxy-, Trifluormethoxy-, Amino-, Methylamino- oder Dimethylaminogruppe darstellen,
deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

15. CGRP-Antagonisten der allgemeinen Formel (I) gemäß Anspruch 1, in denen
A und X jeweils ein Sauerstoffatom,
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe,
R^{a}, R^{b} und R^{c} unabhängig voneinander jeweils ein Wasserstoff- oder Halogenatom, eine C₁₋₃-Alkyl-, Trifluormethyl-, Cyano-, Hydroxy-, Methoxy-, Trifluormethoxy-, Amino-, Methylamino- oder Dimethylaminogruppe,
R¹ eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-*c*]chinolin-2-on-3-yl-, 1,3-Dihydro-naphth[1,2-*d*]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H-*thieno[3,2-*d*]pyrimidin-2-on-3-yl- oder 3,4-Dihydro-1*H*-thieno[3,4-*d*]pyrimidin-2-on-3-ylgruppe, die an einem ungesättigten Kohlenstoffatom des aromatischen oder heteroaromatischen Teils durch Halogenatome, durch Cyano- oder Hydroxygruppen mono-, di- oder trisubstituiert und die Substituenten gleich oder verschieden sein können, vorzugsweise jedoch unsubstituiert sind,
R² und R³ zusammen mit dem eingeschlossenen Stickstoffatom einen Rest der allgemeinen Formel 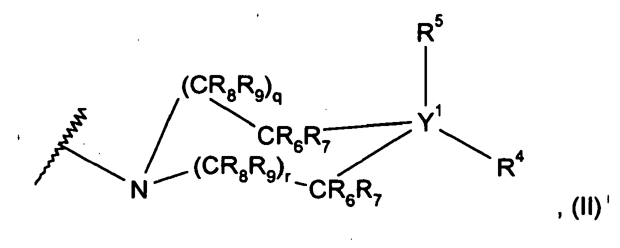 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, wobei die Summe aus q und r 1, 2 oder 3 beträgt,
q und r, wenn Y¹ das Stickstoffatom darstellt, die Zahlen 1 oder 2, wobei die Summe aus q und r 2 oder 3 beträgt,
R⁴ das Wasserstoffatom, eine Amino-, C₁₋₄-Alkyl-amino-, Di-(C₁₋₄-alkyl)-alkylamino-, C₁₋₆-Alkyl-, eine gegebenenfalls durch eine Hydroxycarbonyl-, C₁₋₆-Alkoxycarbonyl-, Hydroxycarbonyl-C₁₋₃-alkyl- oder C₁₋₆-Alkoxycarbonyl-C₁₋₃-alkylgruppe substituierte Cyclo-C₃₋₇-alkyl-, Amino-C₂₋₇-alkyl-, C₁₋₄-Alkylamino-C₂₋₇-alkyl-, Di-(C₁₋₄-alkyl-amino)-C₂₋₇-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl- oder Pyridylgruppe, die jeweils durch ein Halogenatom, durch eine C₁₋₃-Alkyl-, C₁₋₃-Alkoxy-, Amino-, C₁₋₄-Alkyl-amino-, oder Di-(C₁₋₄-alkyl)-aminogruppe substituiert sein kann,
ein Heterocyclus ausgewählt aus einer 5- bis 7-gliedrigen Azacycloalkyl- oder S,S-Dioxothiazagruppe und einer 6- bis 7-gliedrigen Diazacycloalkylgruppe,
wobei die vorstehend genannten Heterocyclen über ein Stickstoff- oder ein Kohlenstoffatom an Y¹ in Formel (II) gebunden sind und
durch eine oder zwei Hydroxy-, C₁₋₃-Alkyl- oder Hydroxy-C₁₋₃-alkylgruppen oder durch eine Cyclo-C₃₋₆-alkyl-, Hydroxy-C₃₋₆-cycloalkyl-, Cyclo-C₃₋₆-alkyl-C₁₋₃-alkyl, C₁₋₃-Alkylcarbonyl-C₁₋₃-alkyl-, Amino-, C₁₋₄-Alkylamino-, Di-(C₁₋₄-alkyl)-amino-, Hydroxycarbonyl-carbonyl-, C₁₋₃-Alkoxycarbonyl-carbonyl-, Hydroxycarbonyl-C₁₋₃-alkylcarbonyl-, C₁₋₃-Alkoxycarbonyl-C₁₋₃-alkylcarbonyl-, Aminosulfonyl-, C₁₋₄-Alkylaminosulfonyl-, Di-(C₁₋₄-alkyl)-aminosulfonyl-, Cyclo-C₃₋₇-alkylsulfonyl-, Aminocarbonyl-C₁₋₃-alkyl-, C₁₋₄-Alkylaminocarbonyl-C₁₋₃-alkyl-, Di-(C₁₋₄-alkyl)-aminocarbonyl-C₁₋₃-alkyl-, Hydroxyaminocarbonyl-C₁₋₃-alkyl-, C₁₋₃-Alkoxyaminocarbonyl-C₁₋₃-alkyl- oder Hydroxy-(C₁₋₃-alkyl)-aminocarbonyl-C₁₋₃-alkylgruppen substituiert sein können,
R⁵ ein Wasserstoffatom oder, wenn Y¹ ein Stickstoffatom darstellt, auch ein freies Elektronenpaar und
R⁶, R⁷, R⁸ und R⁹, die gleich oder verschieden sein können, jeweils ein Wasserstoffatom oder eine C₁₋₃-Alkylgruppe darstellen,
bedeuten, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

16. CGRP-Antagonisten der allgemeinen Formel (I) gemäß Anspruch 1, in denen
A und X jeweils ein Sauerstoffatom,
D und E jeweils eine Methingruppe,
G eine durch die Gruppe R^{a} substituierte Methingruppe,
M eine durch die Gruppe R^{b} substituierte Methingruppe,
Q eine durch die Gruppe R^{c} substituierte Methingruppe,
R^{a}, R^{b} und R^{c} unabhängig voneinander jeweils ein Wasserstoff- oder Halogenatom, eine C₁₋₃-Alkyl-, Trifluormethyl-, Cyano-, Hydroxy-, Methoxy-, Trifluormethoxy-, Amino-, Methylamino- oder Dimethylaminogruppe,
R¹ eine 1,3,4,5-Tetrahydro-1,3-benzdiazepin-2-on-3-yl-, 3,4-Dihydro-1*H*-chinazolin-2-on-3-yl-, 5-Phenyl-2,4-dihydro-1,2,4-triazol-3-on-2-yl-, 1,3-Dihydro-imidazo[4,5-*c*]chinolin-2-on-3-yl-, 1,3-Dihydro-naphth[1,2*-d*]imidazol-2-on-3-yl-, 1,3-Dihydro-benzimidazol-2-on-3-yl-, 4-Phenyl-1,3-dihydro-imidazol-2-on-1-yl-, 3,4-Dihydro-1*H-*thieno[3,2-*d*]pyrimidin-2-on-3-yl- oder 3,4-Dihydro-1*H*-thieno[3,4-*d*]pyrimidin-2-on-3-ylgruppe, die an einem ungesättigten Kohlenstoffatom des aromatischen oder heteroaromatischen Teils durch Halogenatome, durch Cyano- oder Hydroxygruppen mono-, di- oder trisubstituiert und die Substituenten gleich oder verschieden sein können, vorzugsweise jedoch unsubstituiert sind,
R² und R³ zusammen mit dem eingeschlossenen Stickstoffatom einen Rest der allgemeinen Formel 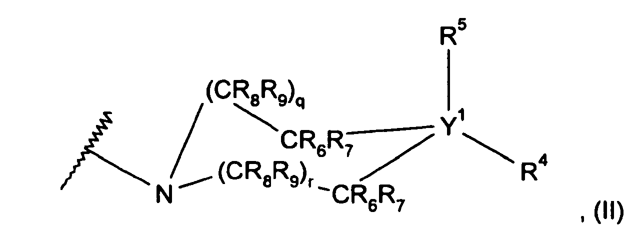 in der
Y¹ das Kohlenstoffatom oder, wenn R⁵ ein freies Elektronenpaar darstellt, auch das Stickstoffatom,
q und r, wenn Y¹ das Kohlenstoffatom darstellt, die Zahlen 0, 1 oder 2, wobei die Summe aus q und r 1, 2 oder 3 beträgt,
q und r, wenn Y¹ das Stickstoffatom darstellt, die Zahlen 1 oder 2, wobei die Summe aus q und r 2 oder 3 beträgt,
R⁴ das Wasserstoffatom, eine Amino-, C₁₋₄-Alkyl-amino-, Di-(C₁₋₄-alkyl)-alkylamino-, C₁₋₆-Alkyl-, Cyclo-C₃₋₇-alkyl-, Amino-C₂₋₇-alkyl-, C₁₋₄-Alkyl-amino-C₂₋₇-alkyl-, Di-(C₁₋₄-alkyl-amino)-C₂₋₇-alkyl-, C₁₋₆-Alkoxycarbonyl-, C₁₋₆-Alkoxycarbonyl-C₁₋₃-alkyl- oder Hydroxycarbonyl-C₁₋₃-alkylgruppe,
eine Phenyl- oder Pyridylgruppe, die jeweils durch ein Halogenatom, durch eine C₁₋₃-Alkyl-, C₁₋₃-Alkoxy-, Amino-, C₁₋₄-Alkyl-amino-, oder Di-(C₁₋₄-alkyl)-aminogruppe substituiert sein kann,
ein Heterocyclus ausgewählt aus einer 5- bis 7-gliedrigen Azacycloalkylgruppe und einer 6- bis 7-gliedrigen Diazacycloalkylgruppe,
wobei die vorstehend genannten Heterocyclen über ein Stickstoff- oder ein Kohlenstoffatom an Y¹ in Formel (II) gebunden sind und
durch eine C₁₋₃-Alkyl-, Cyclo-C₃₋₆-alkyl-, Cyclo-C₃₋₆-alkyl-C₁₋₃-alkyl, Amino-, C₁₋₄-Alkylamino- oder Di-(C₁₋₄-alkyl)-aminogruppen substituiert sein können,
R⁵ ein Wasserstoffatom oder, wenn Y¹ ein Stickstoffatom darstellt, auch ein freies Elektronenpaar und
R⁶, R⁷, R⁸ und R⁹, die gleich oder verschieden sein können, jeweils ein Wasserstoffatom oder eine C₁₋₃-Alkylgruppe darstellen,
bedeuten, deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

17. Folgende Verbindungen der allgemeinen Formel I gemäß Anspruch 1:
| | Struktur |
|---|---|
| (1) | 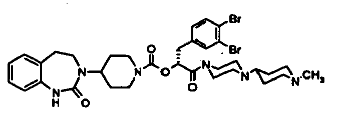 |
| (2) | 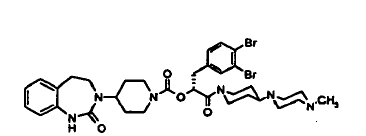 |
| (3) | 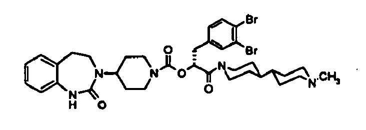 |
| (4) | 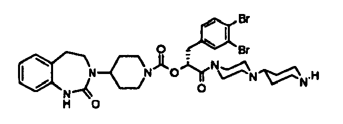 |
| (5) | 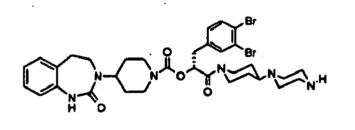 |
| (6) | 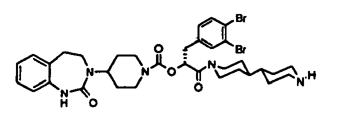 |
| (7) | 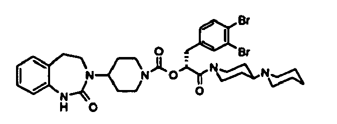 |
| (8) | 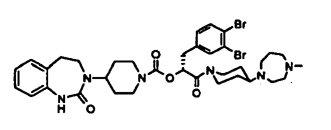 |
| (9) | 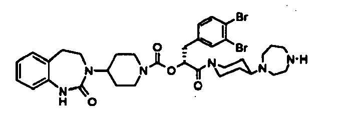 |
| (10) | 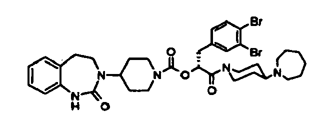 |
| (11) | 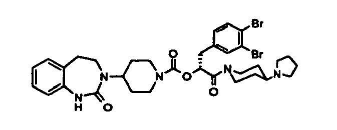 |
| (12) | 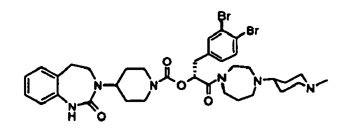 |
| (13) | 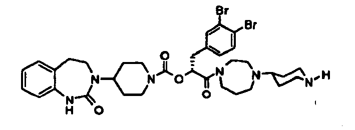 |
| (14) | 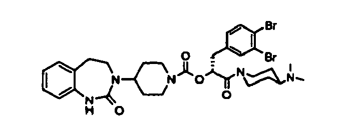 |
| (15) | 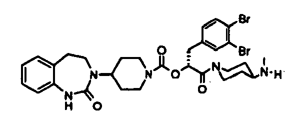 |
| (16) | 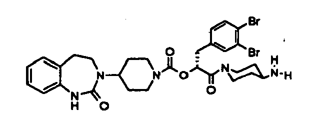 |
| (17) | 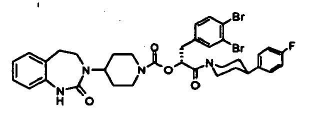 |
| (18) | 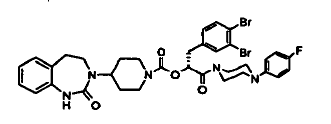 |
| (19) | 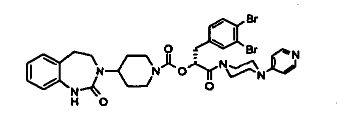 |
| (20) | 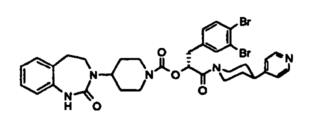 |
| (21) | 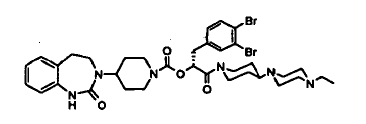 |
| (22) | 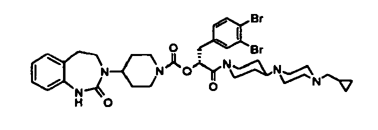 |
| (23) | 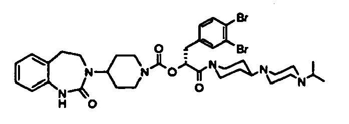 |
| (24) | 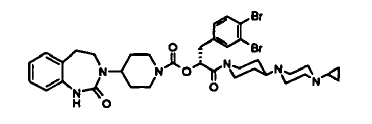 |
| (25) | 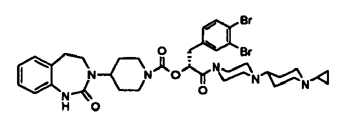 |
| (26) | 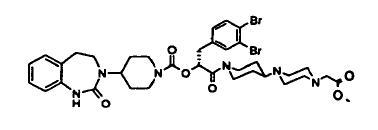 |
| (27) | 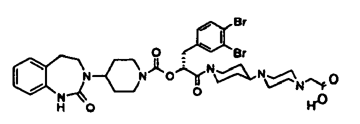 |
| (28) | 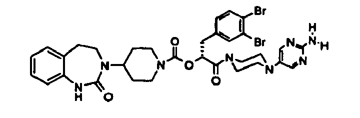 |
| (29) | 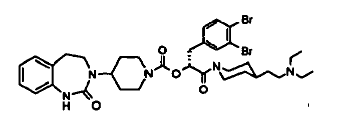 |
| (30) | 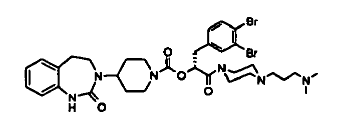 |
| (31) | 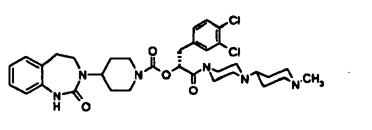 |
| (32) | 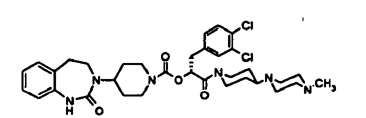 |
| (33) | 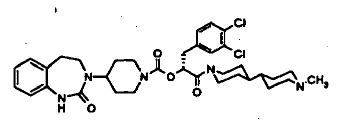 |
| (34) | 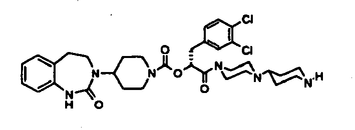 |
| (35) | 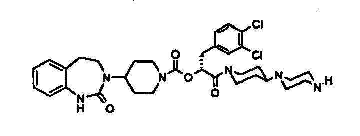 |
| (36) | 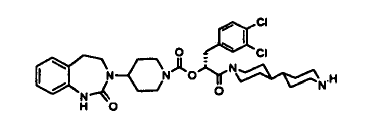 |
| (37) | 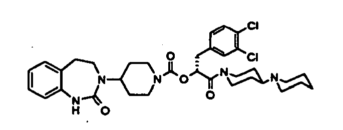 |
| (38) | 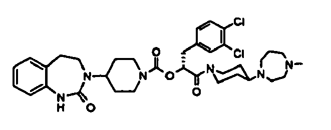 |
| (39) | 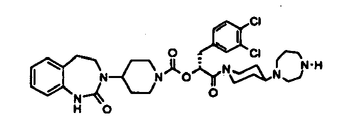 |
| (40) | 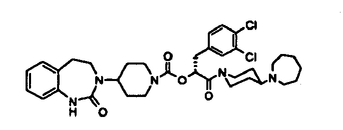 |
| (41) | 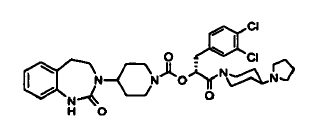 |
| (42) | 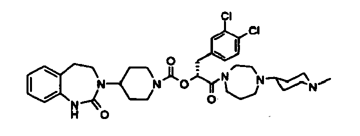 |
| (43) | 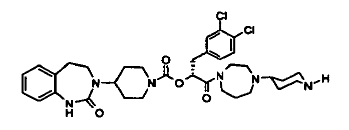 |
| (44) | 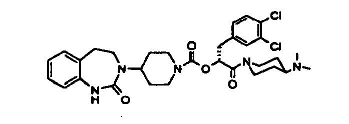 |
| (45) | 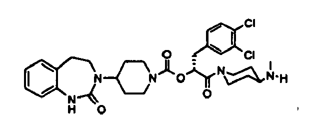 |
| (46) | 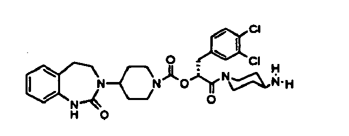 |
| (47) | 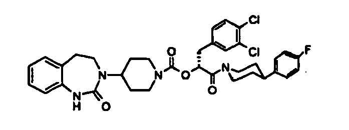 |
| (48) | 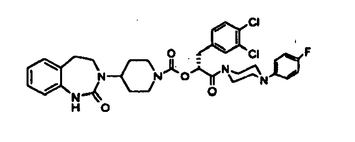 |
| (49) | 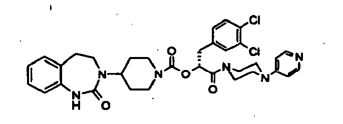 |
| (50) | 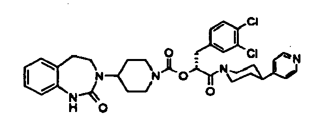 |
| (51) | 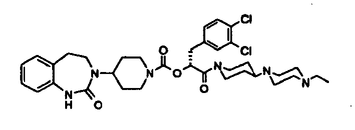 |
| (52) | 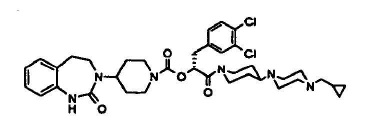 |
| (53) | 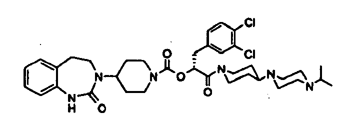 |
| (54) | 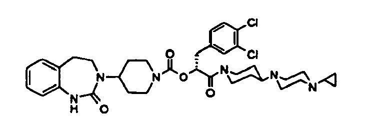 |
| (55) | 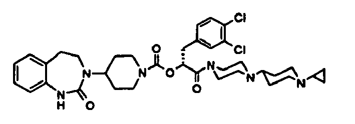 |
| (56) | 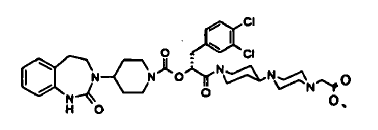 |
| (57) | 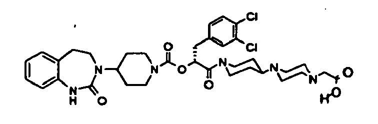 |
| (58) | 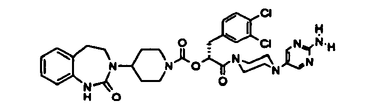 |
| (59) | 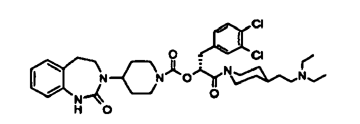 |
| (60) | 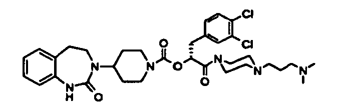 |
| (61) | 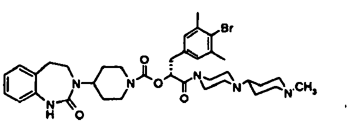 |
| (62) | 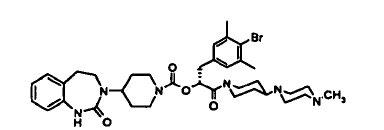 |
| (63) | 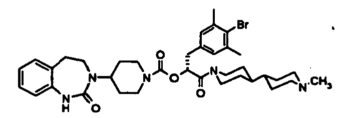 |
| (64) | 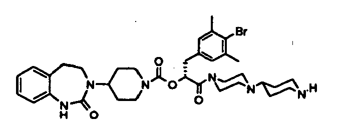 |
| (65) | 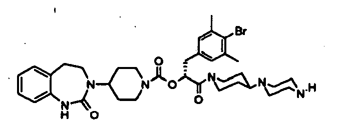 |
| (66) | 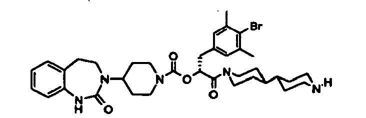 |
| (67) | 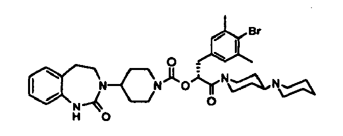 |
| (68) | 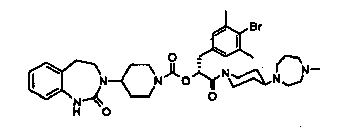 |
| (69) | 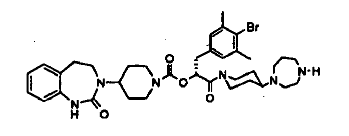 |
| (70) | 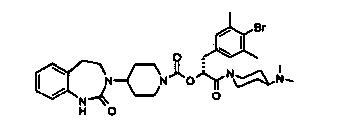 |
| (71) | 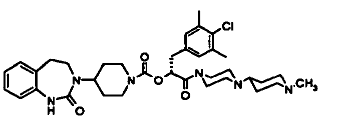 |
| (72) | 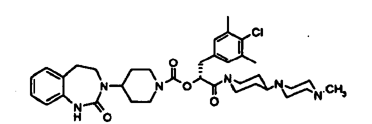 |
| (73) | 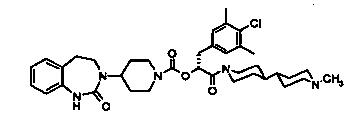 |
| (74) | 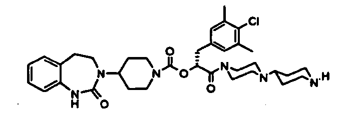 |
| (75) | 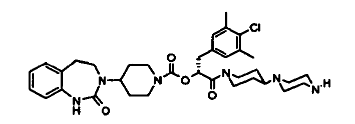 |
| (76) | 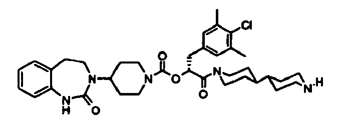 |
| (77) | 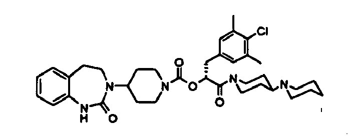 |
| (78) | 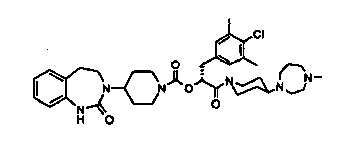 |
| (79) | 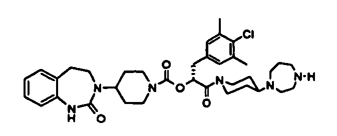 |
| (80) | 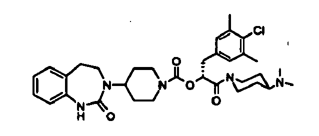 |
| (81) | 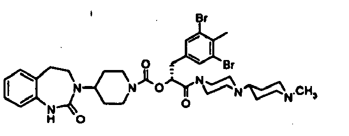 |
| (82) | 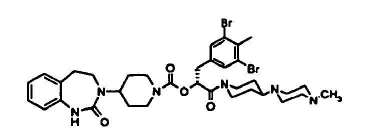 |
| (83) | 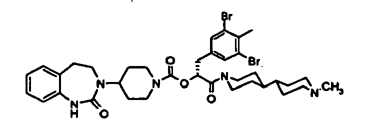 |
| (84) | 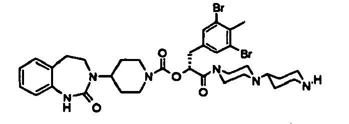 |
| (85) | 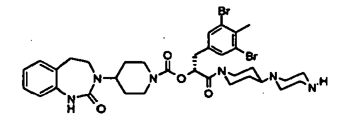 |
| (86) | 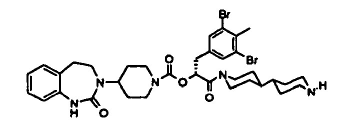 |
| (87) | 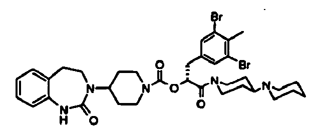 |
| (88) | 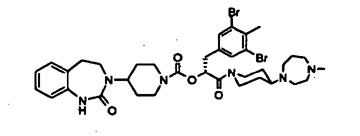 |
| (89) | 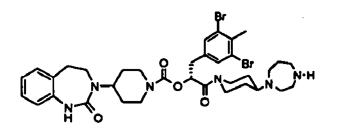 |
| (90) | 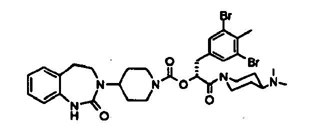 |
| (91) | 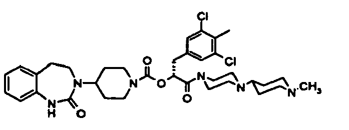 |
| (92) | 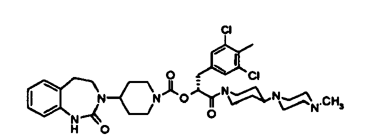 |
| (93) | 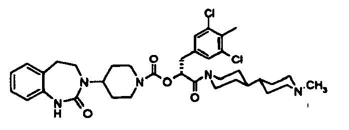 |
| (94) | 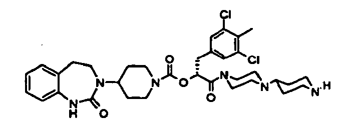 |
| (95) | 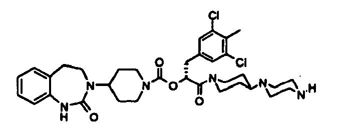 |
| (96) | 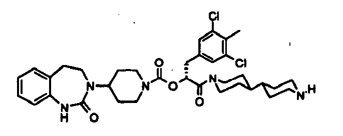 |
| (97) | 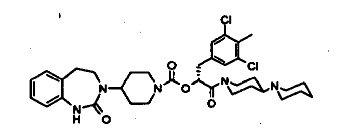 |
| (98) | 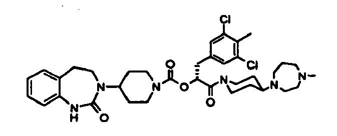 |
| (99) | 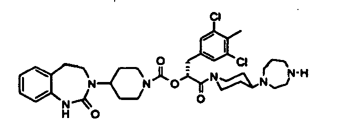 |
| (100) | 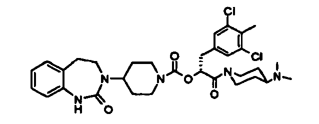 |
| (101) | 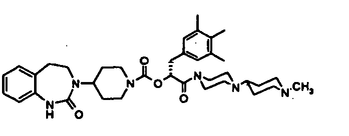 |
| (102) | 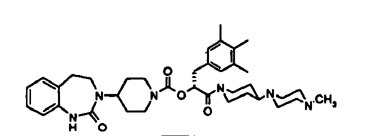 |
| (103) | 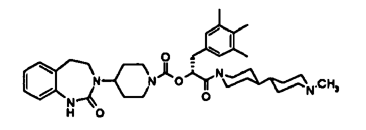 |
| (104) | 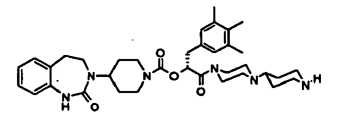 |
| (105) | 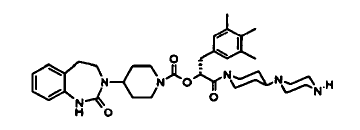 |
| (106) | 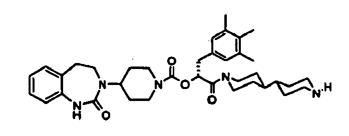 |
| (107) | 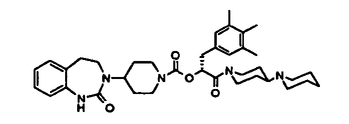 |
| (108) | 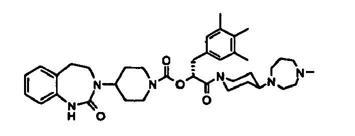 |
| (109) | 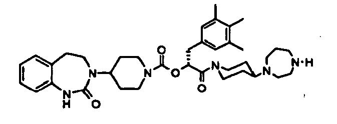 |
| (110) | 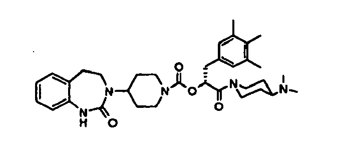 |
| (111) | 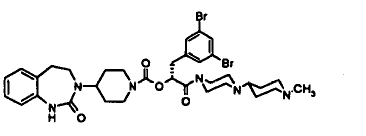 |
| (112) | 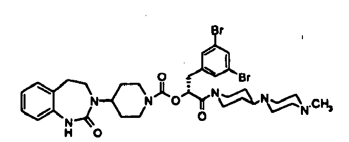 |
| (113) | 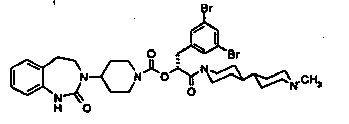 |
| (114) | 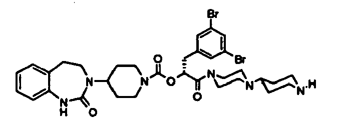 |
| (115) | 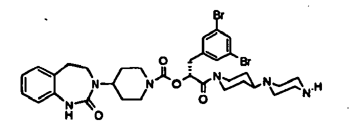 |
| (116) | 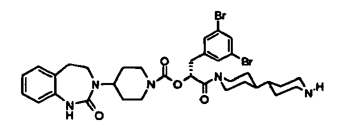 |
| (117) | 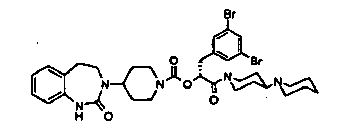 |
| (118) | 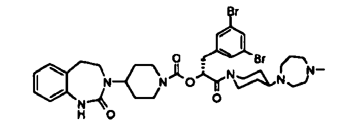 |
| (119) | 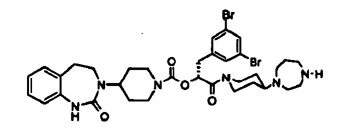 |
| (120) | 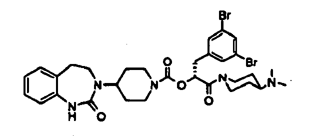 |
| (121) | 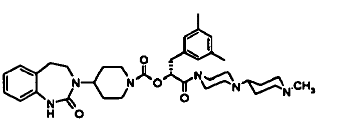 |
| (122) | 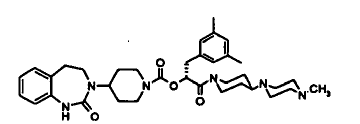 |
| (123) | 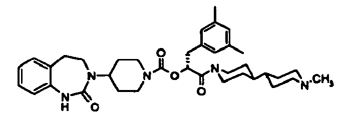 |
| (124) | 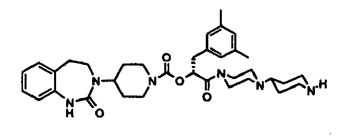 |
| (125) | 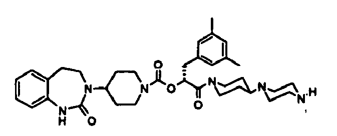 |
| (126) | 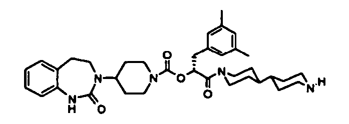 |
| (127) | 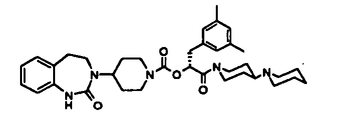 |
| (128) | 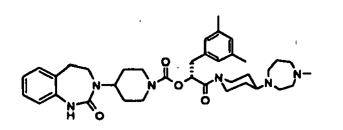 |
| (129) | 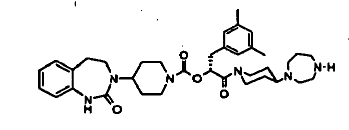 |
| (130) | 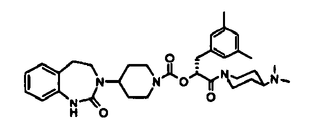 |
| (131) | 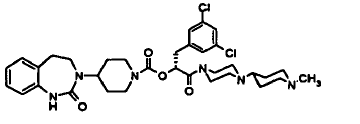 |
| (132) | 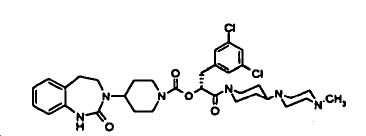 |
| (133) | 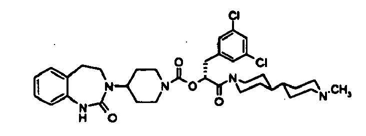 |
| (134) | 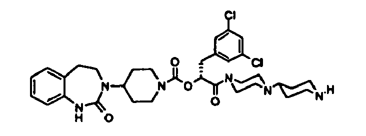 |
| (135) | 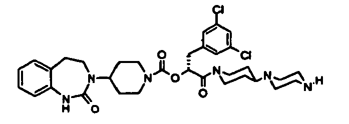 |
| (136) | 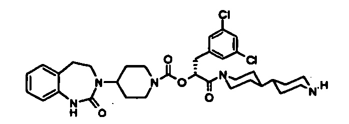 |
| (137) | 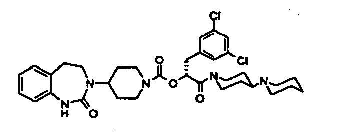 |
| (138) | 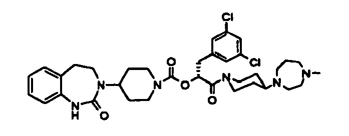 |
| (139) | 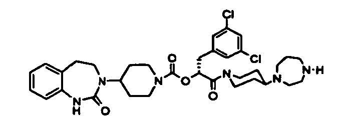 |
| (140) | 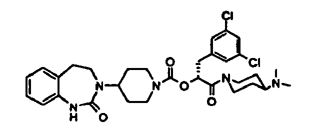 |
| (141) | 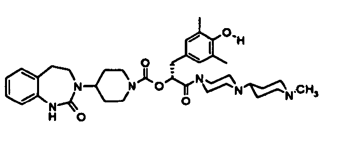 |
| (142) | 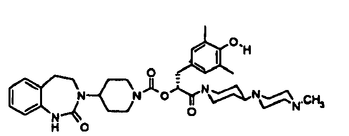 |
| (143) | 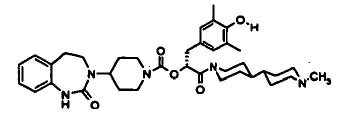 |
| (144) | 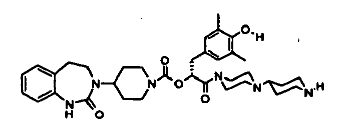 |
| (145) | 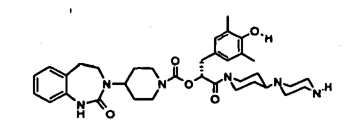 |
| (146) | 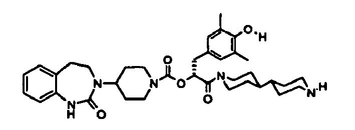 |
| (147) | 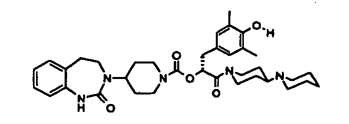 |
| (148) | 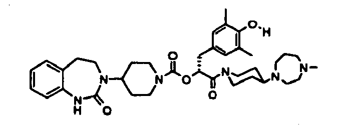 |
| (149) | 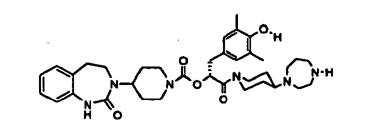 |
| (150) | 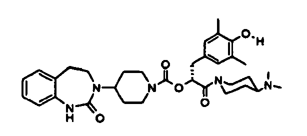 |
| (151) | 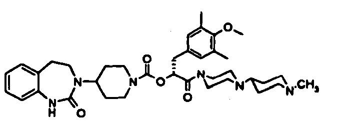 |
| (152) | 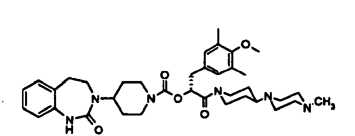 |
| (153) | 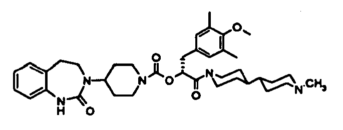 |
| (154) | 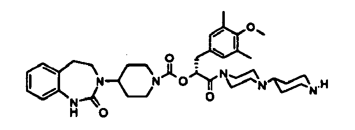 |
| (155) | 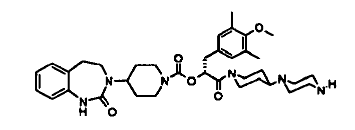 |
| (156) | 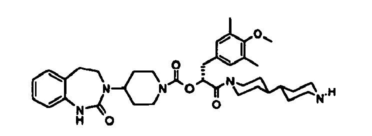 |
| (157) | 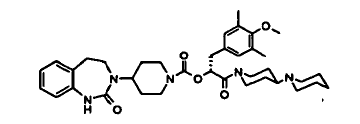 |
| (158) | 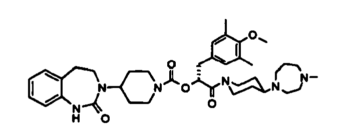 |
| (159) | 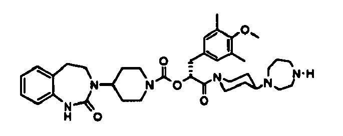 |
| (160) | 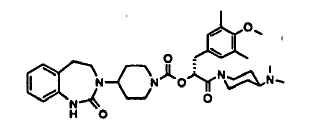 |
| (161) | 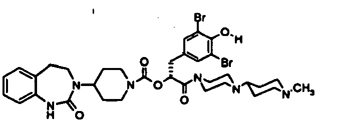 |
| (162) | 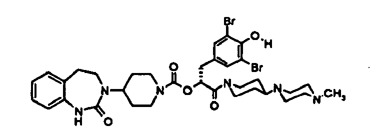 |
| (163) | 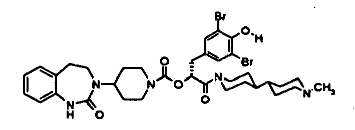 |
| (164) | 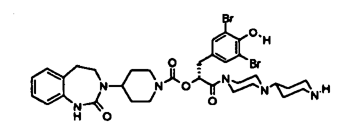 |
| (165) | 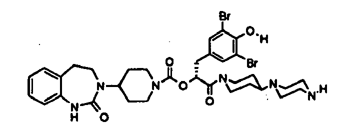 |
| (166) | 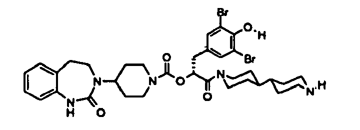 |
| (167) | 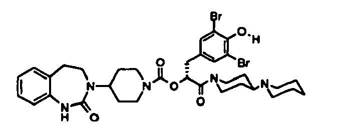 |
| (168) | 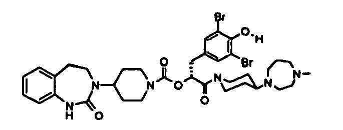 |
| (169) | 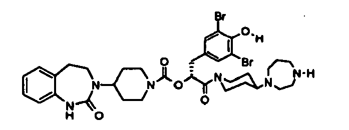 |
| (170) | 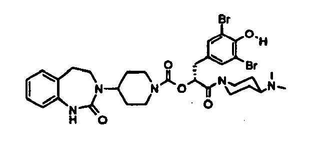 |
| (171) | 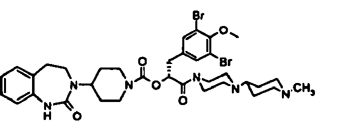 |
| (172) | 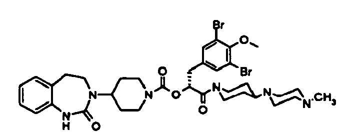 |
| (173) | 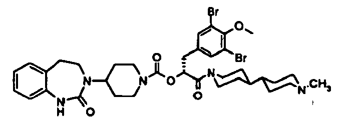 |
| (174) | 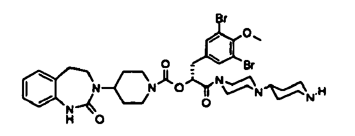 |
| (175) | 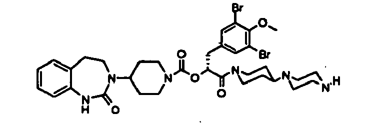 |
| (176) | 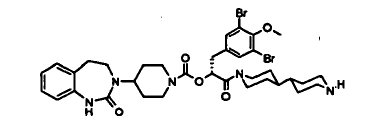 |
| (177) | 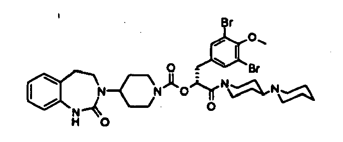 |
| (178) | 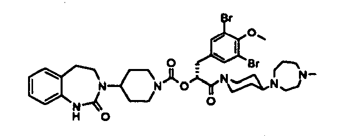 |
| (179) | 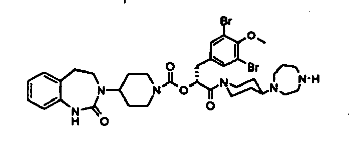 |
| (180) | 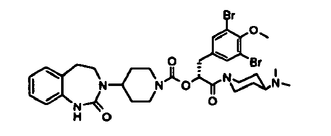 |
| (181) | 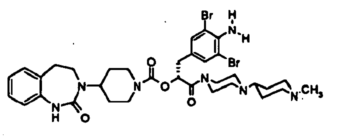 |
| (182) | 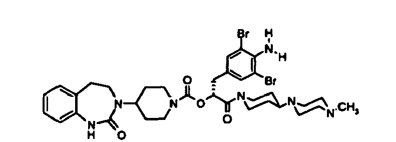 |
| (183) | 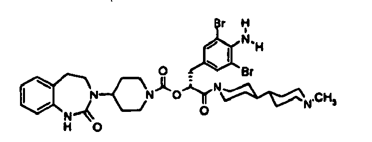 |
| (184) | 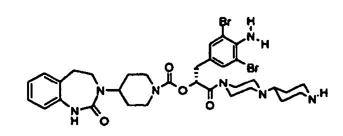 |
| (185) | 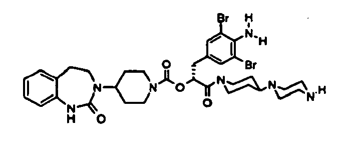 |
| (186) | 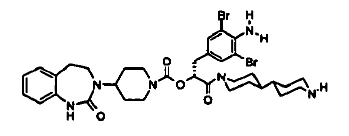 |
| (187) | 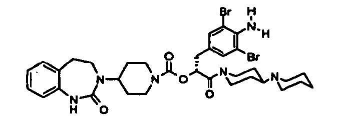 |
| (188) | 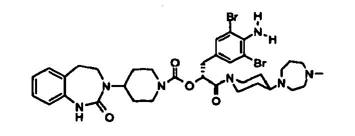 |
| (189) | 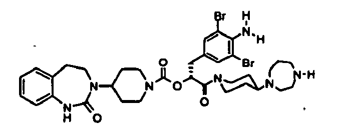 |
| (190) | 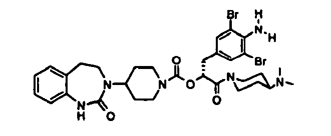 |
| (191) | 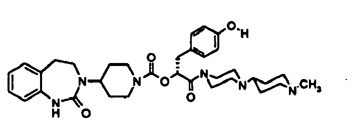 |
| (192) | 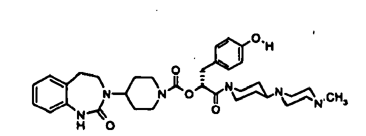 |
| (193) | 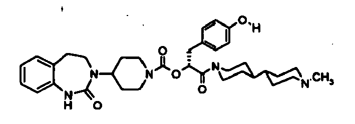 |
| (194) | 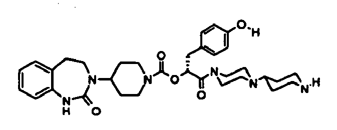 |
| (195) | 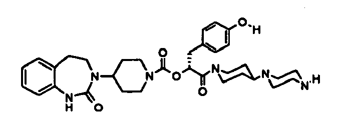 |
| (196) | 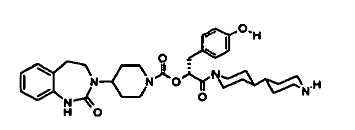 |
| (197) | 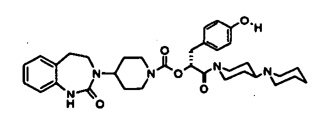 |
| (198) | 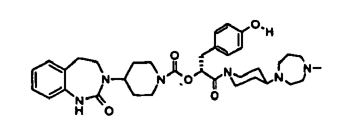 |
| (199) | 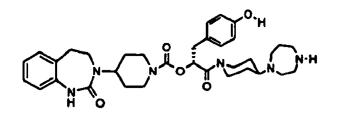 |
| (200) | 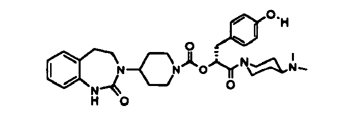 |
| (201) | 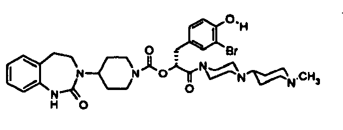 |
| (202) | 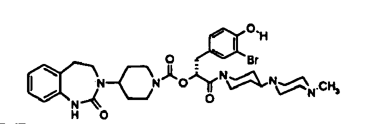 |
| (203) | 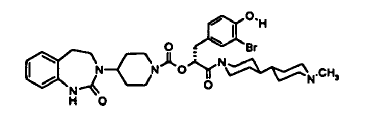 |
| (204) | 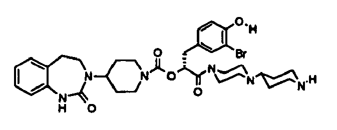 |
| (205) | 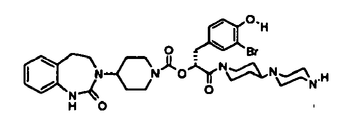 |
| (206) | 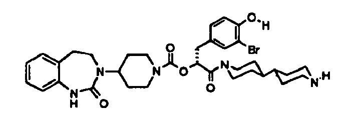 |
| (207) | 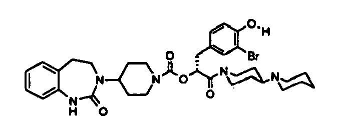 |
| (208) | 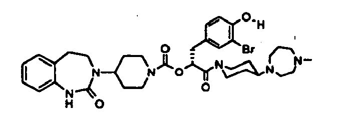 |
| (209) | 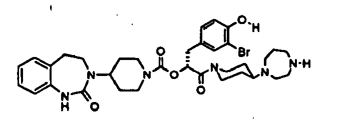 |
| (210) | 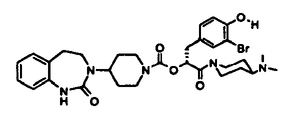 |
| (211) | 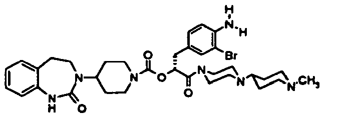 |
| (212) | 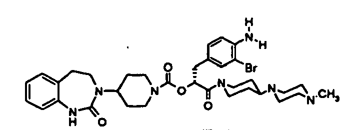 |
| (213) | 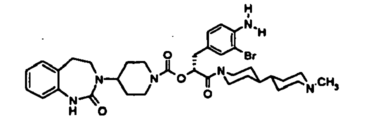 |
| (214) | 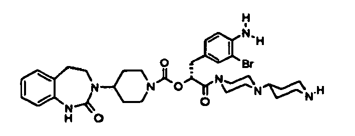 |
| (215) | 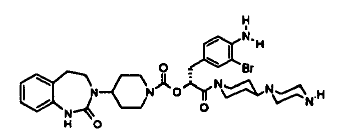 |
| (216) | 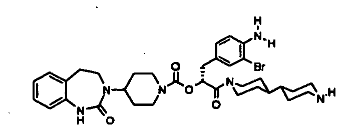 |
| (217) | 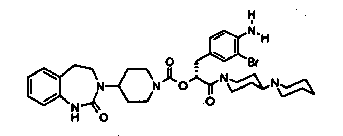 |
| (218) | 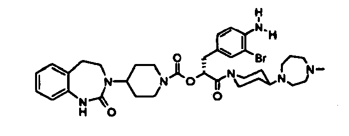 |
| (219) | 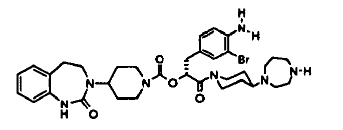 |
| (220) | 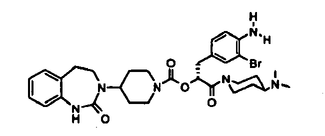 |
| (221) | 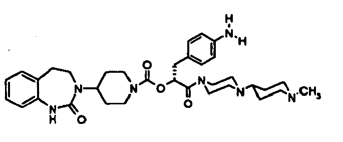 |
| (222) | 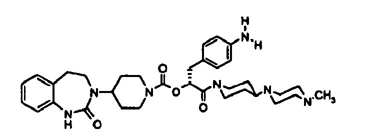 |
| (223) | 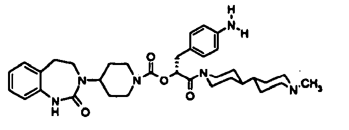 |
| (224) | 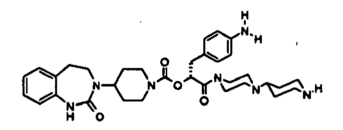 |
| (225) | 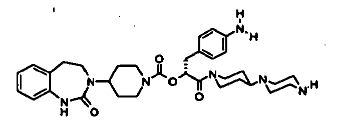 |
| (226) | 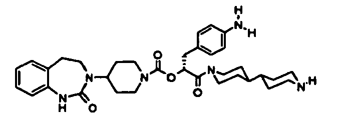 |
| (227) | 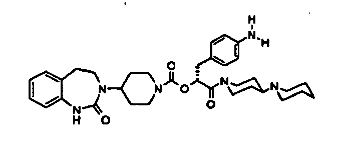 |
| (228) | 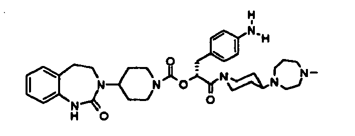 |
| (229) | 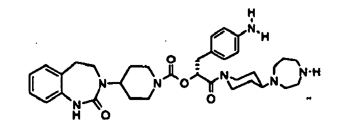 |
| (230) | 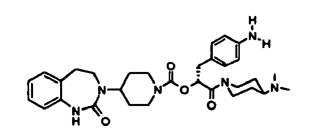 |
| (231) | 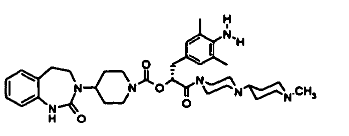 |
| (232) | 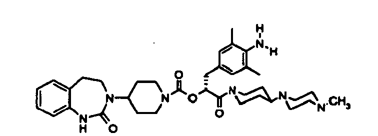 |
| (233) | 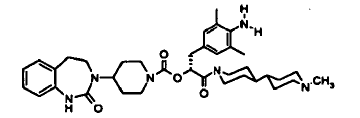 |
| (234) | 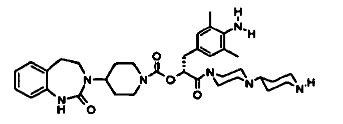 |
| (235) | 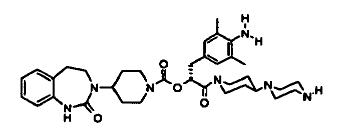 |
| (236) | 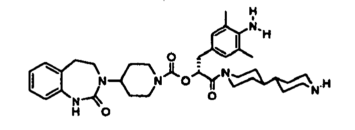 |
| (237) | 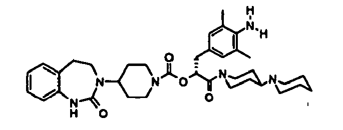 |
| (238) | 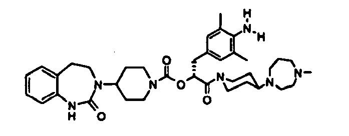 |
| (239) | 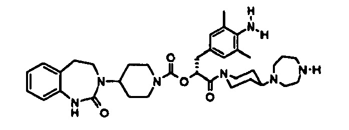 |
| (240) | 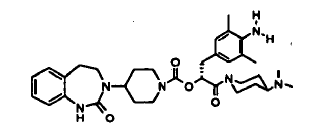 |
| (241) | 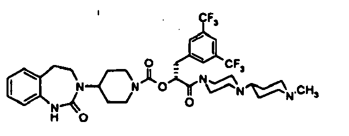 |
| (242) | 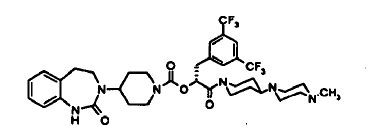 |
| (243) | 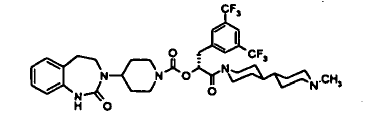 |
| (244) | 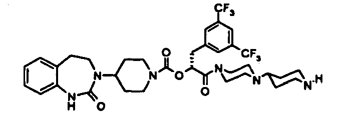 |
| (245) | 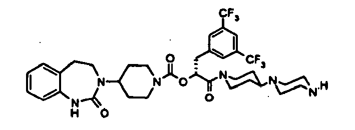 |
| (246) | 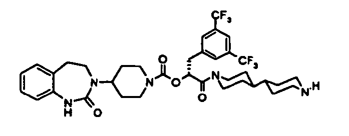 |
| (247) | 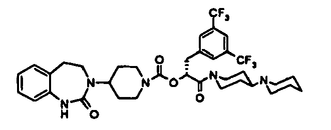 |
| (248) | 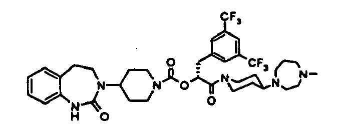 |
| (249) | 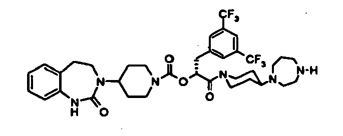 |
| (250) | 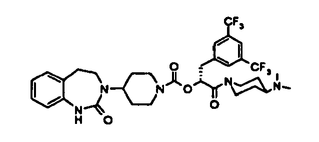 |
| (251) | 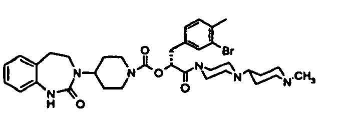 |
| (252) | 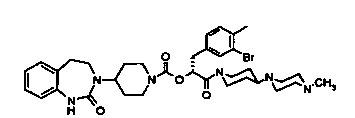 |
| (253) | 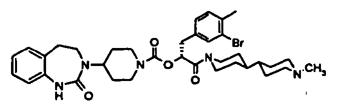 |
| (254) | 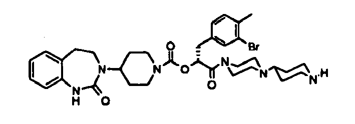 |
| (255) | 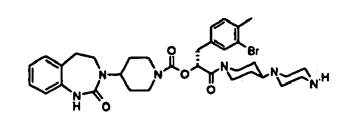 |
| (256) | 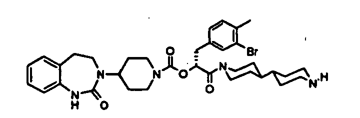 |
| (257) | 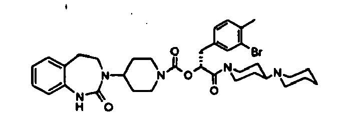 |
| (258) | 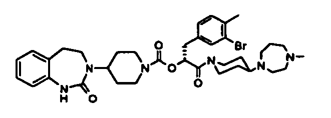 |
| (259) | 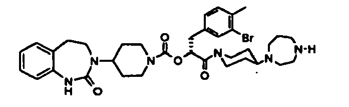 |
| (260) | 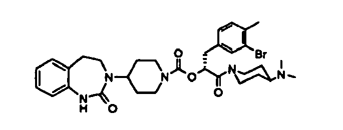 |
| (261) | 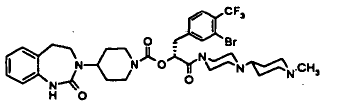 |
| (262) | 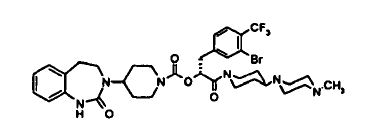 |
| (263) | 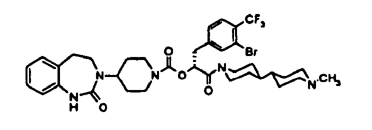 |
| (264) | 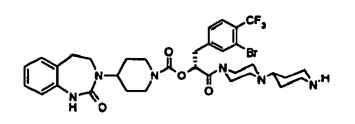 |
| (265) | 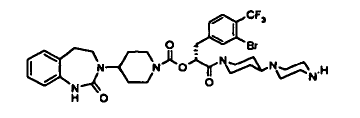 |
| (266) | 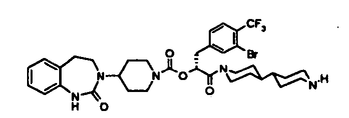 |
| (267) | 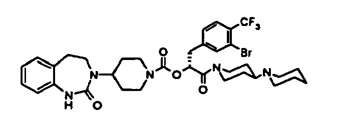 |
| (268) | 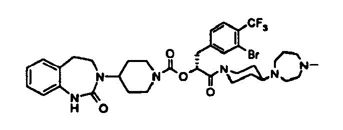 |
| (269) | 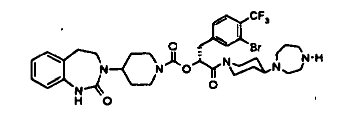 |
| (270) | 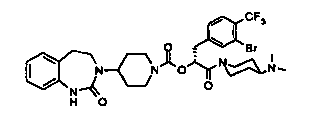 |
| (271) | 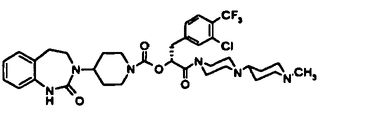 |
| (272) | 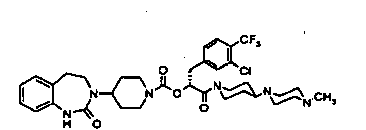 |
| (273) | 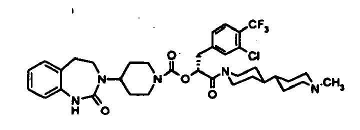 |
| (274) | 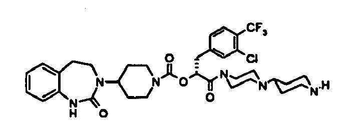 |
| (275) | 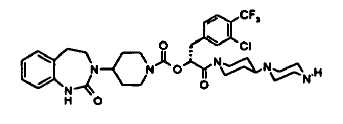 |
| (276) | 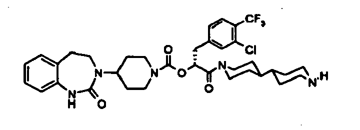 |
| (277) | 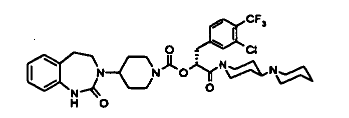 |
| (278) | 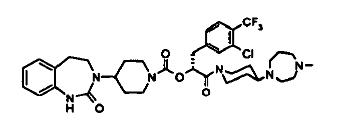 |
| (279) | 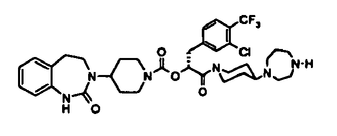 |
| (280) | 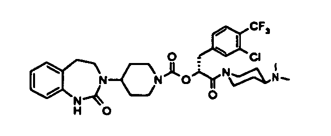 |
| (281) | 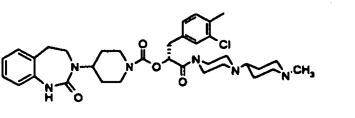 |
| (282) | 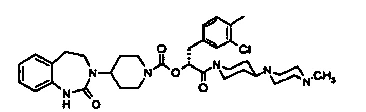 |
| (283) | 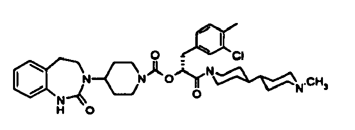 |
| (284) | 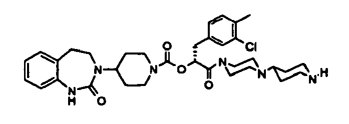 |
| (285) | 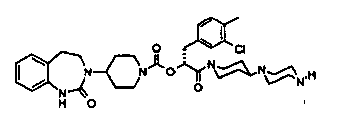 |
| (286) | 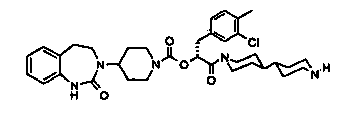 |
| (287) | 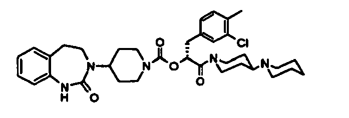 |
| (288) | 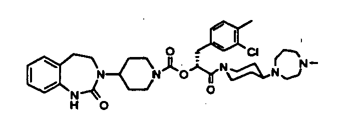 |
| (289) | 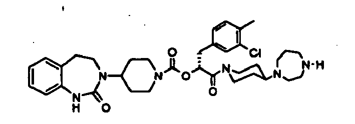 |
| (290) | 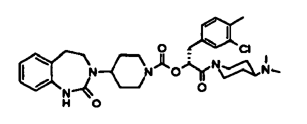 |
| (291) | 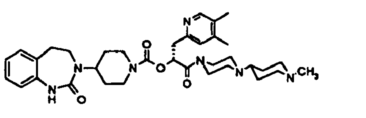 |
| (292) | 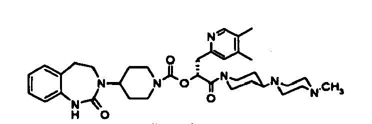 |
| (293) | 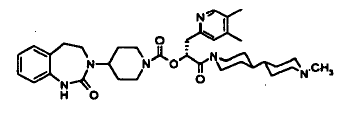 |
| (294) | 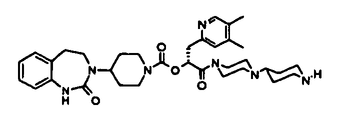 |
| (295) | 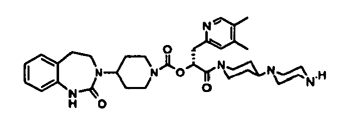 |
| (296) | 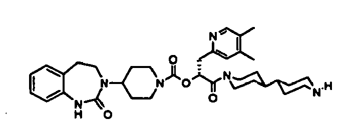 |
| (297) | 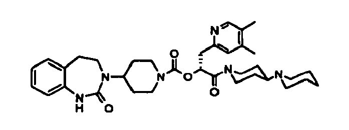 |
| (298) | 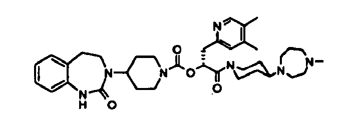 |
| (299) | 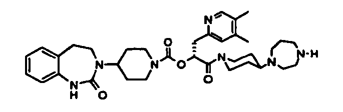 |
| (300) | 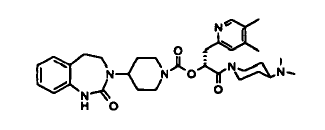 |
| (301) | 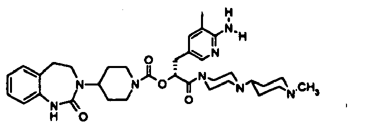 |
| (302) | 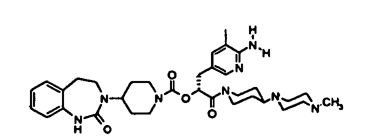 |
| (303) | 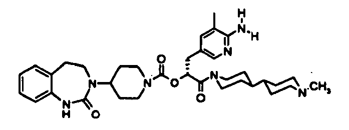 |
| (304) | 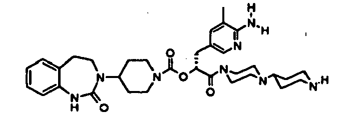 |
| (305) | 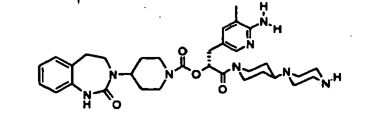 |
| (306) | 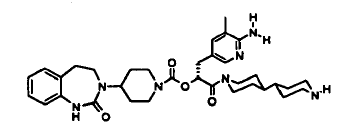 |
| (307) | 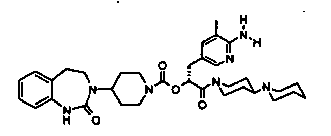 |
| (308) | 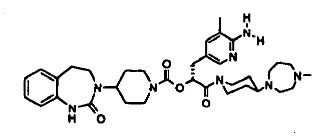 |
| (309) | 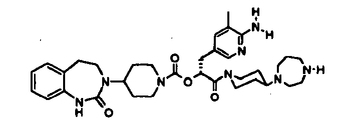 |
| (310) | 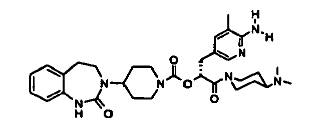 |
| (311) | 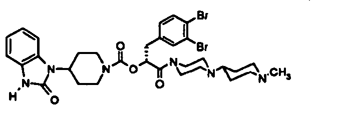 |
| (312) | 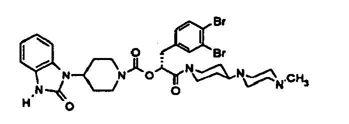 |
| (313) | 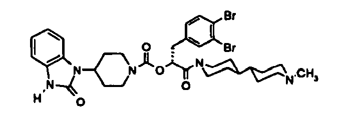 |
| (314) | 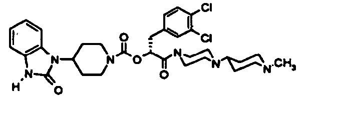 |
| (315) | 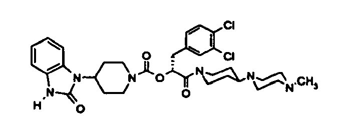 |
| (316) | 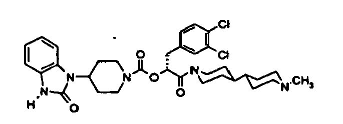 |
| (317) | 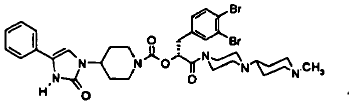 |
| (318) | 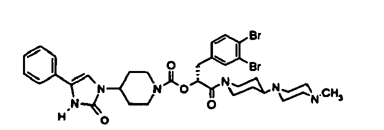 |
| (319) | 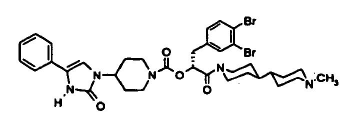 |
| (320) | 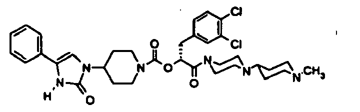 |
| (321) | 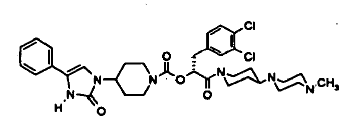 |
| (322) | 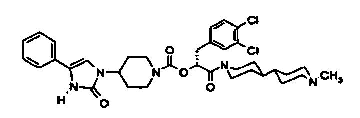 |
| (323) | 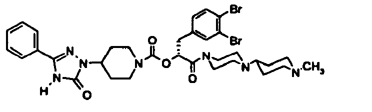 |
| (324) | 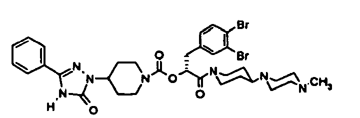 |
| (325) | 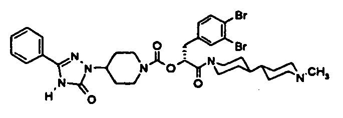 |
| (326) | 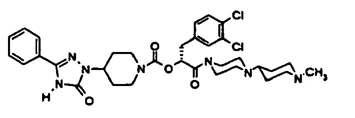 |
| (327) | 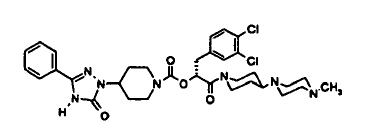 |
| (328) | 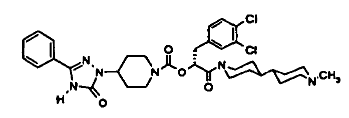 |
| (329) | 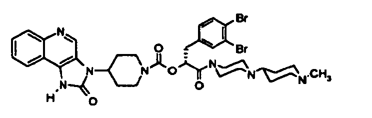 |
| (330) | 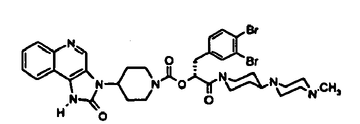 |
| (331) | 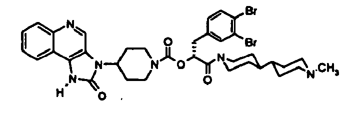 |
| (332) | 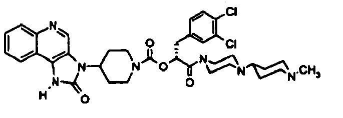 |
| (333) | 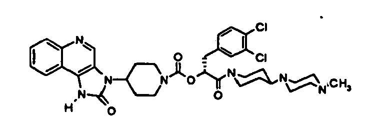 |
| (334) | 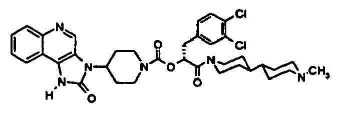 |
| (335) | 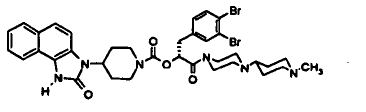 |
| (336) | 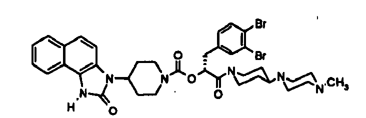 |
| (337) | 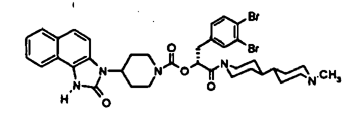 |
| (338) | 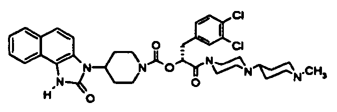 |
| (339) | 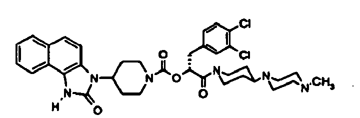 |
| (340) | 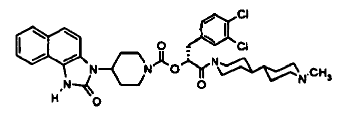 |
| (341) | 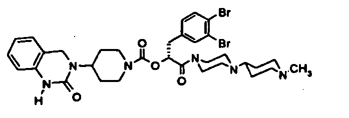 |
| (342) | 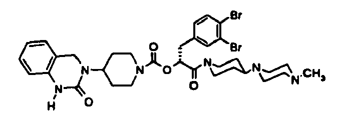 |
| (343) | 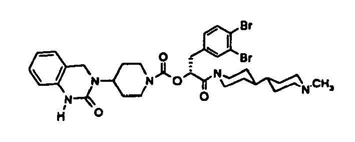 |
| (344) | 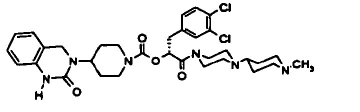 |
| (345) | 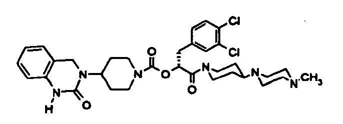 |
| (346) | 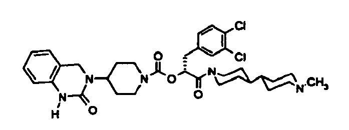 |
| (347) | 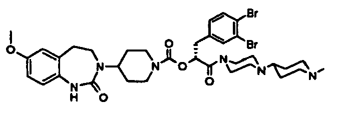 |
| (348) | 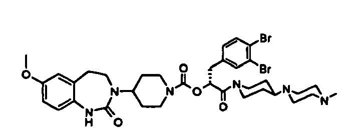 |
| (349) | 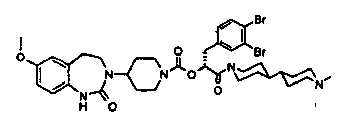 |
| (350) | 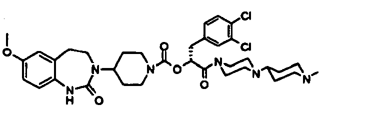 |
| (351) | 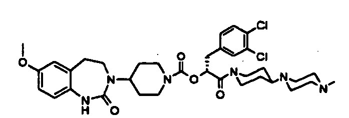 |
| (352) | 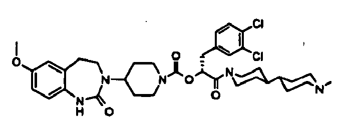 |
| (353) | 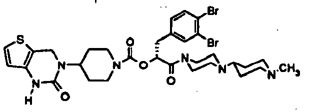 |
| (354) | 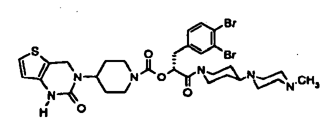 |
| (355) | 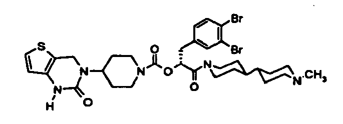 |
| (356) | 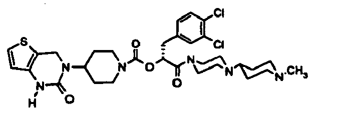 |
| (357) | 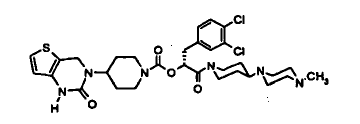 |
| (358) | 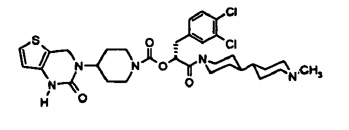 |
| (359) | 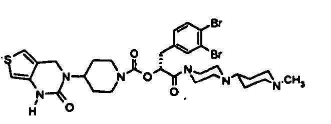 |
| (360) | 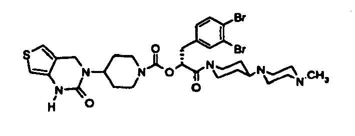 |
| (361) | 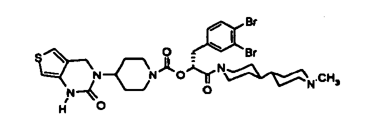 |
| (362) | 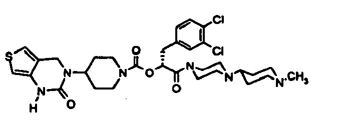 |
| (363) | 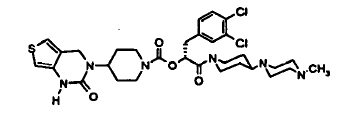 |
| (364) | 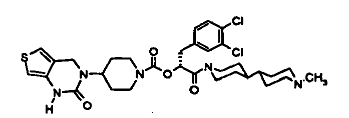 |
deren Enantiomere, deren Diastereomere und deren Salze.

18. Physiologisch verträgliche Salze der Verbindungen nach einem der Ansprüche 1 bis 17 mit anorganischen oder organischen Säuren oder Basen.

19. Arzneimittel, enthaltend eine Verbindung nach einem der Ansprüche 1 bis 17 oder ein physiologisch verträgliches Salz gemäß Anspruch 18 neben gegebenenfalls einem oder mehreren inerten Trägerstoffen und/oder Verdünnungsmitteln.

20. Verwendung einer Verbindung nach einem der Ansprüche 1 bis 18 zur Herstellung eines Arzneimittels zur akuten und prophylaktischen Behandlung von Kopfschmerzen, insbesondere Migräne- bzw. Cluster-Kopfschmerz, zur Behandlung von nicht-insulinabhängigem Diabetes mellitus ("NIDDM"); des complex regional pain syndrome (CRPS1), cardiovaskulärer Erkrankungen, Morphintoleranz, Clostritiumtoxin-bedingten Durchfallerkrankungen, Erkrankungen der Haut, insbesondere thermische und strahlenbedingte Hautschäden inklusive Sonnenbrand, entzündlicher Erkrankungen, z.B. entzündlicher Gelenkerkrankungen (Arthritis), neurogenen Entzündungen der oralen Mucosa, entzündlichen Lungenerkrankungen, allergischer Rhinitis, Asthma, Erkrankungen, die mit einer überschießenden Gefäßerweiterung und **dadurch** bedingter verringerter Gewebedurchblutung einhergehen, z.B. Schock und Sepsis, zur Linderung von Schmerzzuständen, oder zur präventiven oder akut-therapeutisch Behandlung der Symptomatik menopausaler, durch Gefäßerweiterung und erhöhten Blutfluss verursachter Hitzewallungen östrogendefizienter Frauen sowie hormonbehandelter Prostatakarzinompatienten.

21. Verfahren zur Herstellung eines Arzneimittels gemäß Anspruch 19, **dadurch gekennzeichnet, dass** auf nichtchemischem Weg eine Verbindung nach einem der Ansprüche 1 bis 18 in einen oder mehrere inerte Trägerstoffe und/oder Verdünnungsmittel eingearbeitet wird.

22. Verfahren zur Herstellung der Verbindungen der allgemeinen Formel (I) nach einem der Ansprüche 1 bis 18, **dadurch gekennzeichnet, dass**
(a) ein Piperidin der allgemeinen Formel 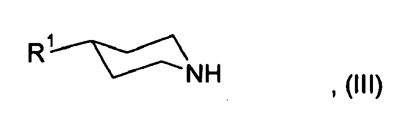 in der R¹ wie im Anspruch 1 definiert ist,
mit einem Kohlensäurederivat der allgemeinen Formel 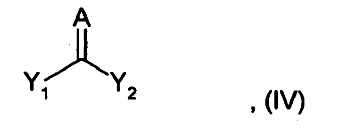 in der A wie im Anspruch 1 definiert ist und Y₁ und Y₂ nucleofuge Gruppen bedeuten,
sowie mit einer Verbindung der allgemeinen Formel 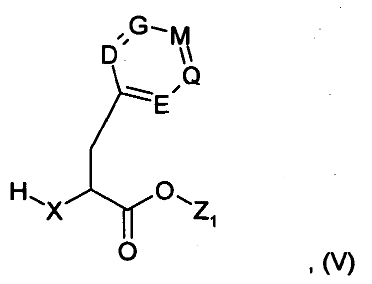 in der X, D, E, G, M und Q wie im Anspruch 1 definiert sind und Z₁ eine Schutzgruppe für eine Carboxygruppe darstellt, umgesetzt wird, wobei vor Durchführung der Reaktion im Rest R¹ einer Verbindung der Formel (III) und/oder in einer Verbindung der Formel (V) gegebenenfalls vorhandene Carbonsäurefunktionen, primäre oder sekundäre Aminofunktionen oder Hydroxyfunktionen, falls erforderlich, durch Schutzreste geschützt und gegebenenfalls verwendete Schutzreste nach Durchführung der Reaktion wieder abgespalten werden, oder
(b) eine Carbonsäure der allgemeinen Formel 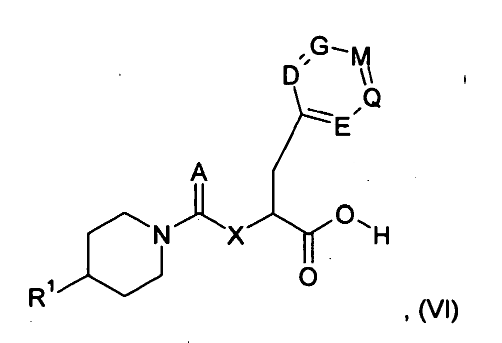 in der alle Reste wie im Anspruch 1 definiert sind, mit einem Amin der allgemeinen Formel HNR²R³, in der R² und R³ wie wie im Anspruch 1 definiert sind, gekuppelt wird, wobei vor Durchführung der Reaktion in einer Verbindung der Formel (VI) und/oder in den Resten R² und R³ des Amins der Formel HNR²R³ gegebenenfalls vorhandene Carbonsäurefunktionen, primäre oder sekundäre Aminofunktionen oder Hydroxyfunktionen, falls erforderlich, durch Schutzreste geschützt und gegebenenfalls verwendete Schutzreste nach Durchführung der Reaktion wieder abgespalten werden, oder
(c) eine Verbindung der allgemeinen Formel 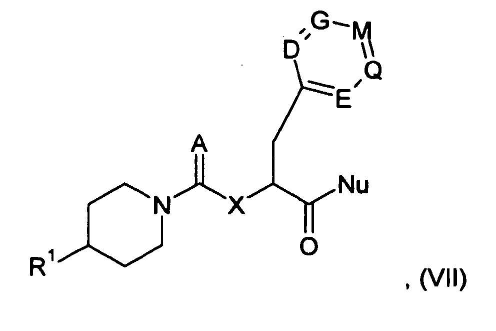 mit einem Amin der allgemeinen Formel HNR²R³, in denen alle Reste wie im Anspruch 1 definiert sind und Nu eine Austrittsgruppe, bedeutet, gekuppelt wird,
wobei vor Durchführung der Reaktion in einer Verbindung der Formel (VII) und/oder in den Resten R² und R³ des Amins der Formel HNR²R³ gegebenenfalls vorhandene Carbonsäurefunktionen, primäre oder sekundäre Aminofunktionen oder Hydroxyfunktionen, falls erforderlich, durch Schutzreste geschützt und gegebenenfalls verwendete Schutzreste nach Durchführung der Reaktion wieder abgespalten werden,
gewünschtenfalls eine so erhaltene Verbindung der allgemeinen Formel (I) in ihre Stereoisomere aufgetrennt wird und/oder
eine so erhaltene Verbindung der allgemeinen Formel (I) in ihre Salze, insbesondere für die pharmazeutische Anwendung in ihre physiologisch verträglichen Salze übergeführt wird.

## Claims

1. CGRP-antagonists of general formula 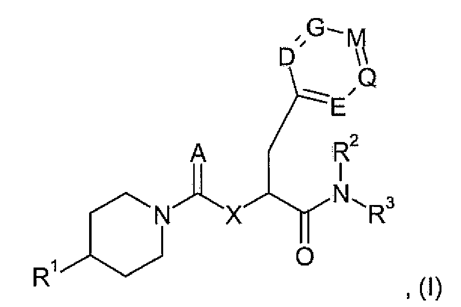 wherein
A denotes an oxygen or sulphur atom,
X denotes an oxygen or sulphur atom,
(a) D, E independently of one another each denote a methyne group or the nitrogen atom and
G denotes a methyne group substituted by the group R^{a},
M denotes a methyne group substituted by the group R^{b},
Q denotes a methyne group substituted by the group R^{c},
while one or two of the groups G, M and Q in each case may also represent a nitrogen atom,
or
(b) D and E each denote a methyne group, while one of the groups D and E may also represent a nitrogen atom, and
G, M and Q each denote a nitrogen atom,
while R^{a}, R^{b} and R^{c} independently of one another each denote a hydrogen or halogen atom, a C₁-₆-alkyl, C₂₋₆-alkenyl, C₂₋₆-alkynyl, cyclo-C₃₋₇-alkyl, cyclo-C₃₋₇-alkenyl, cyano, hydroxy, hydroxy-C₁₋₆-alkyl, hydroxy-C₃₋₆-alkenyl, hydroxy-C₃₋₆-alkynyl, C₁₋₆-alkoxy, C₁₋₆-alkoxy-C₁₋₆-alkyl, C₁₋₆-alkoxy-C₃₋₆-alkenyl, C₁₋₆-alkoxy-C₃₋₆-alkynyl, C₃₋₆-alkenoxy-C₁₋₆-alkyl, C₃₋₆-alkenoxy-C₃₋₆-alkenyl, C₃₋₆-alkenoxy-C₃₋₆-alkynyl, C₃₋₆-alkynoxy-C₁₋₆-alkyl, C₃₋₆-alkynoxy-C₃₋₆-alkenyl, C₃₋₆-alkynoxy-C₃₋₆-alkynyl, thiohydroxy, C₁₋₆-alkylthio, C₃₋₆-alkenylthio, C₃₋₆-alkynylthio, amino, C₁₋₆-alkyl-amino, C₃₋₆-alkenyl-amino, C₃₋₆-alkynyl-amino, di-(C₁₋₆-alkyl)-amino, di-(C₃₋₆-alkenyl)-amino, di-(C₃₋₆-alkynyl)-amino, amino-C₁₋₆-alkyl, C₁₋₃-alkyl-amino-C₁₋₆-alkyl, di(C₁₋₃-alkyl)-amino-C₁₋₆-alkyl, amino-C₃₋₆-alkenyl, C₁₋₃-alkyl-amino-C₃₋₆-alkenyl, di-(C₁₋₃-alkyl)-amino-C₃₋₆-alkenyl, amino-C₃₋₆-alkynyl, C₁₋₃-alkylamino-C₃₋₆-alkynyl, di-(C₁₋₃-alkyl)-amino-C₃₋₆-alkynyl, hydroxycarbonyl, phenylcarbonyl, pyridylcarbonyl, C₁₋₆-alkyl-carbonyl, C₂₋₆-alkenyl-carbonyl, C₂₋₆-alkynyl-carbonyl, formyl, C₁₋₆-alkoxy-carbonyl, C₃₋₆-alkenoxy-carbonyl, C₃₋₆-alkynoxy-carbonyl, aminocarbonyl, C₁₋₆-alkyl-aminocarbonyl, C₃₋₆-alkenyl-aminocarbonyl, C₃₋₆-alkynyl-aminocarbonyl, di-(C₁₋₆-alkyl)-aminocarbonyl, di-(C₃₋₆-alkenyl)-aminocarbonyl, di-(C₃₋₆-alkynyl)-aminocarbonyl, formylamino, C₁₋₆-alkyl-carbonylamino, C₂₋₆-alkenyl-carbonylamino, C₂₋₆-alkynyl-carbonylamino, formyl-C₁₋₆-alkyl-amino, formyl-C₃₋₆-alkenyl-amino, formyl-C₃₋₆-alkynyl-amino, C₁₋₆-alkyl-carbonyl-C₁₋₆-alkylamino, C₂₋₆-alkenyl-carbonyl-C₁₋₆-alkyl-amino, C₂₋₆-alkynyl-carbonyl-C₁₋₆-alkylamino, C₁₋₆-alkyl-carbonyl-C₃₋₆-alkenyl-amino, C₂₋₆-alkenyl-carbonyl-C₃₋₆-alkenyl-amino, C₂₋₆-alkynyl-carbonyl-C₃₋₆-alkenyl-amino, C₁₋₆-alkylcarbonyl-C₃₋₆-alkynyl-amino, C₂₋₆-alkenyl-carbonyl-C₃₋₆-alkynyl-amino, C₂₋₆-alkynyl-carbonyl-C₃₋₆-alkynyl-amino, C₁₋₆-alkyl-sulphonyl, C₂₋₆-alkenyl-sulphonyl, C₂-₆-alkynyi-sulphonyl, C₁₋₆-alkyl-sulphinyl, C₂₋₆-alkenyl-sulphinyl, C₂₋₆-alkynyl-sulphinyl, C₁₋₆-alkyl-sulphonylamino, C₂₋₆-alkenyl-sulphonylamino, C₂₋₆-alkynyl-sulphonylamino, C₁₋₆-alkyl-sulphonyl-C₁₋₆-alkylamino, C₁₋₆-alkyl-sulphonyl-C₃₋₆-alkenylamino, C₁₋₆-alkyl-sulphonyl-C₃₋₆-alkynylamino, C₂₋₆-alkenyl-sulphonyl-C₁₋₆-alkylamino, C₂₋₆-alkenyl-sulphonyl-C₃₋₆-alkenylamino, C₂₋₆-alkenyl-sulphonyl-C₃₋₆-alkynylamino, C₂₋₆-alkynyl-sulphonyl-C₁₋₆-alkylamino, C₂₋₆-alkynyl-sulphonyl-C₃₋₆-alkenylamino, C₂₋₃-alkynyl-sulphonyl-C₃₋₆-alkynylamino, aminosulphonyl, C₁₋₆-alkylaminosulphonyl, di-(C₁₋₆-alkyl)-aminosulphonyl, C₃₋₆-alkenyl-aminosulphonyl, di-(C₃₋₆-alkenyl)-aminosulphonyl, C₃₋₆-alkynylaminosulphonyl or di-(C₃₋₆-alkynyl)-aminosulphonyl group
with the provisos that, if none of the groups D, E, G, M and Q denotes a nitrogen atom,
(i) R^{a} does not denote a hydrogen atom, if both R^{b} and R^{c} each denote a C₁₋₆-alkyl group,
(ii) R^{c} does not denote a hydrogen atom if both R^{a} and R^{b} each denote a C₁₋₆-alkyl group,
(iii) R^{a} does not take on the meanings of a hydrogen, fluorine, chlorine, bromine or iodine atom or a difluoro- or triftuoromethyl group if R^{c} denotes a C₁₋₆-alkyl, C₂₋₆-alkenyl or C₂₋₆-alkynyl group and R^{b} denotes a chlorine or bromine atom, an amino, methylamino or hydroxy group,
(iv) R^{c} does not take on the meanings of a hydrogen, fluorine, chlorine, bromine or iodine atom or a difluoro- or trifluoromethyl group if R^{a} denotes a C₁₋₆-alkyl, C₂₋₆-alkenyl or C₂₋₆-alkynyl group and R^{b} denotes a chlorine or bromine atom, an amino, methylamino or hydroxy group, R¹ denotes a 1,3,4,5-tetrahydro-1,3-benzodiazepin-2-on-3-yl, 3,4-dihydro-1H-quinazolin-2-on-3-yl, 5-phenyl-2,4-dihydro-1,2,4-triazol-3-on-2-yl, 1,3-dihydro-imidazo[4,5-c]quinalin-2-on-3-yl, 1,3-dihydro-naphth[1,2-d]imidazol-2-on-3-yl, 1,3-dihydro-benzimidazol-2-on-3-yl, 4-phenyl-1,3-dihydro-imidazol-2-on-1-yl, 3,4-dihydro-1*H*-thieno[3,2-d]pyrimidin-2-on-3-yl or 3,4-dihydro-1*H*-thieno[3,4-d]pyrimidin-2-on-3-yl group,
while the heterocycle in the carbon skeleton mentioned under R¹ hereinbefore may additionally be monosubstituted by a methoxy group,
all the aromatic and heteroaromatic groups mentioned or contained in the groups defined hereinbefore under R¹ may additionally be mono-, di- or trisubstituted by halogen atoms, by cyano or hydroxy groups and the substituents may be identical or different, and
the double and triple bonds of the C₃₋₆-alkenyl or C₃₋₆-alkynyl groups contained in the groups given as definitions for R^{a}, R^{b}, R^{c} and R¹ hereinbefore may be isolated from any heteroatoms optionally also contained in these groups,
R² denotes the hydrogen atom,
a phenylmethyl group or a C₂₋₇-alkyl group which may be substituted in the ω position by a cyclo-C₃₋₇-alkyl, cyclo-C₃₋₇-alkenyl, phenyl, pyridinyl, diazinyl, hydroxy, amino, C₁₋₆-alkylamino, di-(C₁₋₆-alkyl)-amino, C₃₋₆-alkenylamino, di-(C₃₋₆-alkenyl)amino, C₃₋₆-alkynylamino, di-(C₃₋₆-alkynyl)amino, hydroxycarbonyl, C₁₋₆-alkoxycarbonyl, aminocarbonyl, aminocarbonylamino, C₁₋₆-alkylcarbonylamino, C₂₋₆-alkenylcarbonylamino, C₂₋₆-alkynylcarbonylamino, 4-morpholinyl, [bis-(2-hydroxyethyl)]amino, 4-(C₁₋₆-alkyl)-1-piperazinyl or 4-(ω-hydroxy-C₂₋₇-alkyl)-1-piperazinyl group,
a phenyl or pyridinyl group,
while the phenyl, pyridinyl and diazinyl groups mentioned in the groups defined hereinbefore for R² or contained as substituents may additionally be mono-, di- or trisubstituted in the carbon skeleton by halogen, by C₁₋₃-alkyl, C₂₋₃-alkenyl, C₂₋₃-alkynyl, C₁₋₃-alkoxy, hydroxy, amino, C₁₋₃-alkylamino, di-(C₁₋₃-alkyl)-amino, amino-C₁₋₃-alkyl, C₁₋₃-alkyl-amino-C₁₋₃-alkyl, di-(C₁₋₃-alkyl)-amino-C₁₋₃-alkyl, C₁₋₃alkylcarbonylamino, C₁₋₃-alkylcarbonylamino-C₁₋₃-alkyl, aminocarbonyl, C₁₋₃-alkylaminocarbonyl, di-(C₁₋₃-alkyl)-aminocarbonyl, cyano, aminosulphonyl, C₁₋₃-alkyl-aminosulphonyl, di-(C₁₋₃-alkyl)-aminosulphonyl, C₁₋₃-alkyl-thio, C₁₋₃-alkyl-sulphinyl or C₁₋₃-alkyl-sulphonyl and the substituents may be identical or different,
R³ denotes the hydrogen atom or a C₁₋₃-alkyl group substituted by a phenyl, or pyridinyle group,
while the C₁₋₃-alkyl group may be connected to an alkyl group contained in R² or to a phenyl or pyridyl ring contained in R² including the nitrogen atom to which R² and R are bound, forming a 4- to 7-membered ring,
or
R² and R³ together with the enclosed nitrogen atom denote a group of general formula 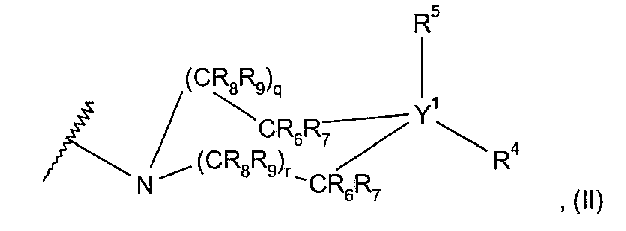 wherein
Y¹ denotes the carbon atom or, if R⁵ denotes a free electron pair, it may also denote the nitrogen atom,
q and r, if Y¹ denotes the carbon atom, denote the numbers 0, 1 or 2, or
q and r, if Y¹ denotes the nitrogen atom, denote the numbers 1 or 2,
R⁴ denotes the hydrogen atom, an amino, C₁₋₄-alkyl-amino, di-(C₁₋₄-alkyl)-alkylamino, C₁₋₆-alkyl, a cyclo-C₃₋₇-alkyl or cyclo-C₃₋₇-alkenyl group optionally substituted by a hydroxycarbonyl, C₁₋₆-alkoxycarbonyl, hydroxycarbonyl-C₁₋₃-alkyl or C₁₋₆-alkoxycarbonyl-C₁₋₃-alkyl group, an amino-C₂₋₇-alkyl, C₁₋₄-alkyl-amino-C₂₋₇-alkyl, di-(C₁₋₄-alikylamino)-C₂₋₇-alkyl, aminoiminomethyl, aminocarbonylamino, C₁₋₄-alkyl-aminocarbonylamino, di-(C₁₋₄-alkyl)-aminocarbonylamino, C₁₋₄-alkylaminocarbonyl-C₁₋₄-alkylamino, di-(C₁₋₄-alkyl)-aminocarbonyl-C₁₋₄-alkylamino, phenylaminocarbonylamino, aminocarbonyl, C₁₋₄-alkylaminocarbonyl, di-(C₁₋₄-alkyl)-aminocarbonyl, aminocarbonyl-C₁₋₃-alkyl, C₁₋₄-alkyl-aminocarbonyl-C₁₋₃-alkyl, di-(C₁₋₄-alkyl)-aminocarbonyl-C₁₋₃-alkyl, aminocarbonylamino-C₁₋₃-alkyl, C₁₋₆-alkoxycarbonyl, C₃₋₆-alkenoxycarbonyl, C₃₋₆-alkynoxycarbonyl, C₁₋₆-alkoxycarbonyl-C₁₋₃-alkyl, C₁₋₆-alkenoxycarbonyl-C₁₋₃-alkyl, C₁₋₆-alkynoxycarbonyl-C₁₋₃-alkyl or hydroxycarbonyl-C₁₋₃-alkyl group,
a phenyl, pyridinyl, diazinyl, 1-naphthyl, 2-naphthyl, pyridinylcarbonyl or phenylcarbonyl group which may be mono-, di- or trisubstituted in each case in the carbon skeleton by halogen, by C₁₋₃-alkyl, C₂₋₃-alkenyl, C₂₋₃-alkynyl, C₁₋₃-alkoxy, hydroxy, amino, C₁₋₄-alkylamino, di-(C₁₋₄-alkyl)-amino, amino-C₁₋₃-alkyl, C₁₋₄-alkyl-amino-C₁₋₃-alkyl, di-(C₁₋₄alkyl)-amino-C₁₋₃-alkyl, C₁₋₄-alkylcarbonylamino, C₁₋₄-alkylcarbonylamino-C₁₋₃-alkyl, aminocarbonyl, C₁₋₄-alkyl-aminocarbonyl, di-(C₁₋₄-alkyl)-aminocarbonyl, cyano, aminosulphonyl, C₁₋₄-alkyl-aminosulphonyl, di-(C₁₋₄-alkyl)-aminosulphonyl, C₁₋₄-alkyl-thio, C₁₋₄-alkyl-sulphinyl, or C₁₋₄-alkyl-sulphonyl and the substituents may be identical or different,
a heterocycle selected from a 4- to 10-membered azacycloalkyl group, a 6- to 10-membered oxaza-, thiaza-, S,S-dioxothiaza- and diazacycloalkyl group and a 6- to 10-membered azabicycloalkyl group,
a 1-alkyl-4-piperidinylcarbonyl or 4-alkyl-1-piperazinylcarbonyl group,
while the above-mentioned mono- and bicyclic heterocycles are bound to Y¹ in formula (II) via a nitrogen or a carbon atom,
in the above-mentioned mono- and bicyclic heterocycles a methyne group not directly linked to a nitrogen, oxygen or sulphur atom may be substituted by a fluorine atom and a methylene group not directly linked to a nitrogen, oxygen or sulphur atom may be substituted by one or two fluorine atoms,
the above-mentioned mono- and bicyclic heterocycles and the 1-(C₁₋₆-alkyl)-4-piperidinylcarbonyl and 4-(C₁₋₆-alkyl)-1-piperazinylcarbonyl group in the ring may be mono- to tetrasubstituted by hydroxy, C₁₋₆-alkyl or hydroxy-C₁₋₃-alkyl groups, or, optionally additionally, monosubstituted by a cyclo-C₃₋₇-alkyl, hydroxy-C₃₋₇-cycloalkyl, cyclo-C₃₋₇-alkenyl, cyclo-C₃₋₇-alkyl-C₁₋₃-alkyl, phenyl-C₁₋₃-alkyl, pyridyl-C₁₋₃-alkyl, C₁₋₆-alkylcarbonyl, C₁₋₆-alkylcarbonyl-C₁₋₃-alkyl, hydroxy, C₁₋₆-alkoxy, amino, C₁₋₄-alkylamino, di-(C₁₋₄-alkyl)amino, phenylcarbonyl, pyridinylcarbonyl, C₁₋₆-alkoxycarbonyl, hydroxycarbonyl-carbonyl, C₁₋₆-alkoxycarbonyl-carbonyl, hydroxycarbonyl-C₁₋₃-alkyl, C₁₋₆-alkoxycarbonyl-C₁₋₃-alkyl, hydroxycarbonyl-C₁₋₃-afkylcarbonyl, C₁₋₆-alkoxycarbonyl-C₁₋₃-alkylcarbonyl, aminocarbonyl, C₁₋₄-alkylaminocarbonyl, di-(C₁₋₄-alkyl)aminocarbonyl, aminosulphonyl, C₁₋₄-alkylaminosulphonyl, di-(C₁₋₄-alkyl)aminosulphonyl, C₁₋₃-alkylsulphonyl, cyclo-C₃₋₇-alkylsulphonyl, aminocarbonyl-C₁₋₃-alkyl, C₁₋₄-alkylaminocarbonyl-C₁₋₃-alkyl, di-(C₁₋₄-alkyl)aminocarbonyl- , C₁₋₃-alkyl, hydroxyaminocarbonyl-C₁₋₃-alkyl, C₁₋₃-alkoxyaminocarbonyl-C₁₋₃-alkyl or hydroxy-(C₁₋₃-alkyl)-aminocarbonyl-C₁₋₃-alkyl group, by a cyclo-C₃₋₇-alkyl-carbonyl, azacyclo-C₄₋₇-alkyl-carbonyl diazacyclo-C₅₋₇-alkyl-carbonyl or oxazacyclo-C₅₋₇-alkyl-carbonyl group optionally C₁₋₃-alkyl-substituted in the ring, while the substituents may be identical or different and may be bound to a cyclic carbon or cyclic nitrogen atom,
while the phenyl and pyridinyl groups contained in the groups defined above for R⁴ may in turn be mono-, di- or trisubstituted by halogen atoms, by C₁₋₃-alkyl, C₂₋₃-alkenyl, C₂₋₃-alkynyl, C₁₋₃-alkoxy, hydroxy, amino, C₁₋₄-alkylamino, di-(C₁₋₄-alkyl)-amino, amino-C₁₋₃-alkyl, C₁₋₄-alkyl-amino-C₁₋₃-alkyl, di-(C₁₋₄-alkyl)-amino-C₁₋₃-alkyl, C₁₋₄-alkylcarbonylamino, C₁₋₄-alkylcarbonylamino-C₁₋₃-alkyl, aminocarbonyl, C₁₋₃-alkylaminocarbonyl, di-C₁₋₄-alkyl-aminocarbonyl, cyano, aminosulphonyl, C₁₋₄-alkyl-aminosulphonyl, di-(C₁₋₄-alkyl)-aminosulphonyl, C₁₋₃-alkyl-thio, C₁₋₃-alkyl-sulphinyl, or C₁₋₃-alkylsulphonyl, while the substituents may be identical or different,
or also, if Y¹ denotes the carbon atom, the hydroxycarbonyl, aminomethyl, C₁₋₄-alkyl-aminomethyl or di-(C₁₋₄-alkyl)-aminomethyl group,
R⁵ denotes a hydrogen atom or a hydroxy group,
a C₁₋₄-alkyl group, while an unbranched alkyl group may be substituted in the ω position by a phenyl, pyridinyl, diazinyl, amino, C₁₋₄-alkylamino, di-(C₁₋₄-alkyl)-amino, 4-C₁₋₄-alkyl-1-piperazinyl or 4-morpholinyl group,
a C₁₋₆-alkoxycarbonyl, cyano or aminocarbonyl group or also, if Y¹ denotes a nitrogen atom, a free electron pair,
or, if Y¹ denotes the carbon atom, it may also denote the fluorine atom, or
R⁴ together with R⁵ and Y¹ denote a 4- to 7-membered cycloaliphatic ring wherein a methylene group may be replaced by a group -NH-, -N(C₁₋₄-alkyl)-, -N(C₃₋₄-alkenyl)-, -N(C₃₋₄-alkynyl)-, -N(cyclo-C₃₋₇-alkyl)-, -N(C₃₋₇-cycloalkyl-C₁₋₃-alkyl)-, -N(hydroxycarbonyl-C₁₋₃-alkyl)- or -N(C₁₋₆-alkoxycarbonyl C₁₋₃-alkyl)-,
while a hydrogen atom bound to a nitrogen atom in one of the groups defined for R⁴ hereinbefore may be replaced by a protecting group,
R and R⁷, which may be identical or different, each denote a hydrogen atom, a C₁₋₄-alkyl group or also, if Y¹ denotes a carbon atom, the fluorine atom, an amino, C₁₋₄-alkylamino or di-(C₁₋₄-alkyl)-amino group, while the two C₁₋₄-alkyl groups may be joined together, forming a ring and
R⁸ and R⁹, which may be identical or different, each denote a hydrogen atom or a C₁₋₃-alkyl group,
while, unless otherwise stated, all the alkyl, alkenyl and alkynyl groups mentioned or contained in the groups defined hereinbefore may be straight-chain or branched, every methyne group contained in the groups defined hereinbefore may be substituted by a fluorine atom, every methylene group may be substituted by up to 2 fluorine atoms and every methyl group may be substituted by up to 3 fluorine atoms and two alkyl and alkenyl groups bound to a nitrogen atom may be joined together forming a 4- to 7-membered, saturated or unsaturated heterocyclic ring,
all the aromatic and heteroaromatic groups mentioned or contained in the groups defined hereinbefore may additionally be mono-, di- or trisubstituted by halogen, by cyano or hydroxy groups and the substituents may be identical or different and
by the protecting groups mentioned in the definitions above and hereinafter are meant the protective groups familiar from peptide chemistry, particularly
a phenylalkoxycarbonyl group with 1 to 3 carbon atoms in the alkoxy moiety, optionally substituted in the phenyl nucleus by a halogen atom, by a nitro or phenyl group or by one or two methoxy groups,
for example the benzyloxycarbonyl, 2-nitro-benzyloxycarbonyl, 4-nitro-benzyloxycarbonyl, 4-methoxy-benzyloxycarbanyl, 2-chloro-benzyloxycarbonyl, 3-chloro-benzyloxycarbonyl, 4-chloro-benzyloxycarbonyl, 4-biphenylyl-α,αdimethyl-benzyloxycarbonyl or 3,5-dimethoxy-α,α-dimethyl-benzyloxycarbonyl group,
an alkoxycarbonyl group with a total of 1 to 5 carbon atoms in the alkyl moiety,
for example the methoxycarbonyl, ethoxycarbonyl, n-propoxycarbonyl, isopropoxycarbonyl, n-butoxycarbonyl, 1-methylpropoxycarbonyl, 2-methylpropoxy-carbonyl or tert.butyloxycarbonyl group,
the allyloxycarbonyl, 2,2,2-trichloro-(1,1-dimethylethoxy)carbonyl or 9-fluorenylmethoxycarbonyl group or
the formyl, acetyl or trifluoracetyl group,
the tautomers, the isomers, the diastereomers, the enantiomers, the hydrates, the mixtures thereof and the salts thereof as well as the hydrates of the salts, particularly the physiologically acceptable salts thereof with inorganic or organic acids or bases.

2. CGRP-antagonists of general formula (I) according to claim 1, wherein
A, X, D, E, G, M, Q and R¹ are defined as in claim 1 and
R² denotes the hydrogen atom,
a phenylmethyl group or a C₂₋₇-alkyl group which may be substituted in the ω position by a cyclo-C₃₋₇alkyl, cyclo-C₃₋₇-alkenyl, phenyl, pyridinyl, diazinyl, hydroxy, amino, C₁₋₆-alkylamino, di-(C₁₋₆-alkyl)-amino, C₃₋₆-alkenylamino, di-(C₃₋₆-alkenyl)amino, C₃₋₆-alkynylamino, di-(C₃₋₆-alkynyl)amino, hydroxycarbonyl, C₁₋₆-alkoxycarbonyl, aminocarbonyl, aminocarbonylamino, C₁₋₆-alkylcarbonylamino, C₂₋₆-alkenylcarbonylamino, C₂₋₆-alkynylcarbonylamino, 4-morpholinyl, [bis-(2-hydroxyethyl)]amino, 4-(C₁₋₆-alkyl)-1-piperazinyl or 4-(ω-hydroxy-C₂₋₇alkyl)-1-piperazinyl group,
a phenyl or pyridinyl group,
while the phenyl, pyridinyl and diazinyl groups mentioned in the groups defined for R² hereinbefore or contained as substituents therein may additionally be mono-, di- or trisubstituted in the carbon skeleton by halogen, by C₁₋₃-alkyl, C₂₋₃-alkenyl, C₂₋₃-alkynyl, C₁₋₃-alkoxy, hydroxy, amino, C₁₋₃-alkylamino, di-(C₁₋₃-alkyl)-amino, amino-C₁₋₃-alkyl, C₁₋₃-alkylamino-C₁₋₃-alkyl, di-(C₁₋₃-alkyl)-amino-C₁₋₃-alkyl, C₁₋₃alkylcarbonylamino, C₁₋₃-alkylcarbonylamino-C₁₋₃-alkyl, aminocarbonyl, C₁₋₃-alkylaminocarbonyl, di-(C₁₋₃-alkyl)-aminocarbonyl, cyano, aminosulphonyl, C₁₋₃-alkyl-aminosulphonyl, di-(C₁₋₃-alkyl)-aminosulphonyl, C₁₋₃-alkyl-thio, C₁₋₃-alkyl-sulphinyl or C₁₋₃-alkyl-sulphonyl and the substituents may be identical or different,
R³ denotes the hydrogen atom or a C₁₋₃-alkyl group substituted by a phenyl or pyridinyl group,
while the C₁₋₃-alkyl group may be linked to an alkyl group contained in R² or a phenyl or pyridyl contained in R² including the nitrogen atom to which R² and R are bound, forming a 4- to 7-membered ring,
or
R² and R³ together with the enclosed nitrogen atom denote a group of general formula 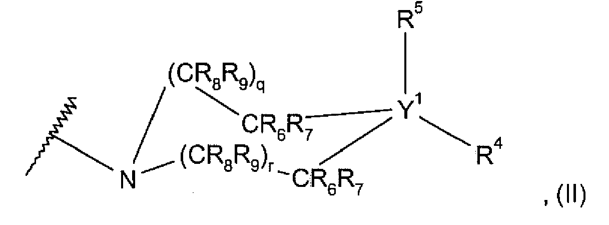 wherein
Y¹ denotes the carbon atom or, if R⁵ denotes a free electron pair, may also denote the nitrogen atom,
q and r, if Y¹ denotes the carbon atom, denote the numbers 0, 1 or 2, or
q and r, if Y¹ denotes the nitrogen atom, denote the numbers 1 or 2,
R⁴ denotes the hydrogen atom, an amino, C₁₋₄-alkyl-amino, di-(C₁₋₄-alkyl)-alkylamino, C₁₋₆-alkyl, a cyclo-C₃₋₇-alkyl, cyclo-C₃₋₇-alkenyl, amino-C₂₋₇-alkyl, C₁₋₄-alkyl-amino-C₂₋₇-alkyl, di-(C₁₋₄-alkylamino)-C₂-₇-alkyl, aminoiminomethyl, aminocarbonylamino, C₁₋₄-alkyl-aminocarbonylamino, di-(C₁₋₄-alkyl)-aminocarbonylamino, C₁₋₄-alkyl-aminocarbonyl-C₁₋₄-alkylamino, di-(C₁₋₄-alkyl)-aminocarbonyl-C₁₋₄-alkylamino, phenylaminocarbonylamino, aminocarbonyl, C₁₋₄-alkyl-aminocarbonyl, di-(C₁₋₄-alkyl)-aminocarbonyl, aminocarbonyl-C₁₋₃-alkyl, C₁₋₄-alkylaminocarbonyl-C₁₋₃-alkyl, di-(C₁₋₄-alkyl)-aminocarbonyl-C₁₋₃-alkyl, aminocarbonylamino-C₁₋₃-alkyl, C₁₋₆-alkoxycarbonyl, C₃₋₆-alkenoxycarbonyl, C₃₋₆-alkynoxycarbonyl, C₁₋₆-alkoxycarbonyl-C₁₋₃-alkyl, C₁₋₆-alkenoxycarbonyl-C₁₋₃-alkyl, C₁₋₆-alkynoxycarbonyl-C₁₋₃-alkyl or hydroxycarbonyl-C₁₋₃-alkyl group,
a phenyl, pyridinyl, diazinyl, 1-naphthyl, 2-naphthyl, pyridinylcarbonyl or phenylcarbonyl group which may be mono-, di- or trisubstituted in each case in the carbon skeleton by halogen, by C₁₋₃-alkyl, C₂₋₃-alkenyl, C₂₋₃-alkynyl, C₁₋₃-alkoxy, hydroxy, amino, C₁₋₄-alkylamino, di-(C₁₋₄-alkyl)-amino, amino-C₁₋₃-alkyl, C₁₋₄-alkyl-amino-C₁₋₃-alkyl, di-(C₁₋₄-alkyl)-amino-C₁₋₃-alkyl, C₁₋₄-alkylcarbonylamino, C₁₋₄-alkylcarbonylamino-C₁₋₃-alkyl, aminocarbonyl, C₁₋₄-alkyl-aminocarbonyl, di-(C₁₋₄-alkyl)-aminocarbonyl, cyano, aminosulphonyl, C₁₋₄-alkyl-aminosulphonyl, di-(C₁₋₄-alkyl)-aminosulphonyl, C₁₋₄-alkyl-thio, C₁₋₄-alkyl-sulphinyl or C₁₋₄-alkyl-sulphonyl and the substituents may be identical or different,
a heterocycle selected from a 4- to 10-membered azacycloalkyl group, a 6- to 10-membered oxaza-, thiaza- and diazacycloalkyl group as well as a 6- to 10-membered azabicycloalkyl group,
a 1-alkyl-4-piperidinylcarbonyl or 4-alkyl-1-piperazinylcarbonyl group,
while the above-mentioned mono- and bicyclic heterocycles are bound to Y¹ in formula (II) via a nitrogen or carbon atom,
in the above-mentioned mono- and bicyclic heterocycles a methyne group not directly attached to a nitrogen, oxygen or sulphur atom may be substituted by a fluorine atom and a methylene group not directly attached to a nitrogen, oxygen or sulphur atom may be substituted by one or two fluorine atoms,
the above-mentioned mono- and bicyclic heterocycles as well as the 1-(C₁₋₆-alkyl)-4-piperidinylcarbonyl and 4-(C₁₋₆-alkyl)-1-piperazinylcarbonyl group may be mono- to tetrasubstituted in the ring by C₁₋₆-alkyl groups, or, optionally additionally, monosubstituted by a cyclo-C₃₋₇-alkyl, cyclo-C₃₋₇-alkenyl, cyclo-C₃₋₇-alkyl-C₁₋₃-alkyl, phenyl-C₁₋₃-alkyl, pyridyl-C₁₋₃-alkyl, C₁₋₆.alkylcarbonyl, hydroxy, C₁₋₆-alkoxy, amino, C₁₋₄-alkylamino, di-(C₁₋₄-alkyl)amino, phenylcarbonyl, pyridinylcarbonyl, hydroxycarbonyl, hydroxycarbonyl-C₁₋₃-alkyl, C₁₋₆-alkoxycarbonyl, C₁₋₆-alkoxycarbonyl-C₁₋₃alkyl, aminocarbonyl, C₁₋₄-alkylaminocarbonyl, di-(C₁₋₄-alkyl)aminocarbonyl, C₁₋₃-alkylsulphonyl group, by a cyclo-C₃₋₇-alkylcarbonyl, azacyclo-C₄₋₇-alkylcarbonyl, diazacyclo-C₅₋₇-alkylcarbonyl or oxazacyclo-C₅₋₇-alkylcarbonyl group optionally C₁₋₃-alkylsubstituted in the ring, while the substituents may be identical or different and may be bound to a cyclic carbon or cyclic nitrogen atom,
while the phenyl and pyridinyl groups contained in the groups given as definitions for R⁴ hereinbefore may in turn be mono-, di- or trisubstituted by halogen atoms, by C₁₋₃-alkyl, C₂₋₃-alkenyl, C₂₋₃-alkynyl, C₁₋₃-alkoxy, hydroxy, amino, C₁₋₄-alkylamino, di-(C₁₋₄-alkyl)-amino, amino-C₁₋₃-alkyl, C₁₋₄-alkyl-amino-C₁₋₃-alkyl, di-(C₁₋₄-alkyl)-amino-C₁₋₃-alkyl, C₁₋₄-alkylcarbonylamino, C₁₋₄-alkylcarbonylamino-C₁₋₄-alkyl, aminocarbonyl, C₁₋₃-alkylaminocarbonyl, di-C₁₋₄-alkyl-aminocarbonyl, cyano, aminosulphonyl, C₁₋₄-alkyl-aminosulphonyl, di-(C₁₋₄-alkyl)-aminosulphonyl, C₁₋₃-alkylthio, C₁₋₃-alkyl-sulphinyl or C₁₋₃-alkylsulphonyl, while the substituents may be identical or different,
or also, if Y¹ denotes the carbon atom, the hydroxycarbonyl, aminomethyl, C₁₋₄-alkyl-aminomethyl or di-(C₁₋₄-alkyl)-aminomethyl group,
R⁵ denotes a hydrogen atom,
a C₁₋₄-alkyl group, while an unbranched alkyl group may be substituted in the ω position by a phenyl, pyridinyl, diazinyl, amino, C₁₋₄-alkylamino, di-(C₁₋₄-alkyl)-amino, 4-C₁₋₄-alkyl-1-piperazinyl or 4-morpholinyl group,
a C₁₋₆-alkoxycarbonyl, cyano or aminocarbonyl group or, if Y¹ denotes a nitrogen atom, a free electron pair,
or, if Y¹ denotes the carbon atom, also the fluorine atom, or
R⁴ together with R⁵ and Y¹ denotes a 4- to 7-membered cycloaliphatic ring wherein a methylene group may be replaced by an -NH-, -N(C₁₋₄-alkyl)-, -N(C₃₋₄-alkenyl)-, -N(C₃₋₄-alkynyl)-, -N(cyclo-C₃₋₇-alkyl)- or -N(C₃₋₇cycloalkyl-C₁₋₃-alkyl)- group,
while a hydrogen atom bound to a nitrogen atom in one of the groups defined for R⁴ hereinbefore may be replaced by a protecting group,
R⁶ and R⁷, which may be identical or different, each denote a hydrogen atom, a C₁₋₄-alkyl group or, if Y¹ denotes a carbon atom, the fluorine atom, a C₁₋₄-alkylamino or di-(C₁₋₄-alkyl)-amino group, while the two C₁₋₄-alkyl groups may be joined together, forming a ring, and
R⁸ and R⁹, which may be identical or different, each denote a hydrogen atom or a C₁₋₃-alkyl group,
while, unless otherwise stated, all the alkyl, alkenyl and alkynyl groups mentioned or contained in the groups defined hereinbefore may be straight-chain or branched, each methyne group contained in the groups defined hereinbefore may be substituted by a fluorine atom, each methylene group may be substituted by up to 2 fluorine atoms and each methyl group may be substituted by up to 3 fluorine atoms and two alkyl and alkenyl groups bound to a nitrogen atom may be joined together, forming a 4- to 7-membered, saturated or unsaturated heterocyclic ring,
all the aromatic and heteroaromatic groups mentioned or contained in the groups defined hereinbefore may additionally be mono-, di- or trisubstituted by halogen, by cyano or hydroxy groups and the substituents may be identical or different,
the tautomers, the isomers, the diastereomers, the enantiomers, the hydrates, the mixtures thereof and the salts thereof as well as the hydrates of the salts, particularly the physiologically acceptable salts thereof with inorganic or organic acids or bases.

3. CGRP-antagonists of general formula (I) according to claim 1, wherein
A, X, R¹, R² and R³ are defined as in one of claims 1 to 2 and
(a) D, E independently of one another each denote a methyne group or the nitrogen atom and
G denotes a methyne group substituted by the group R^{a},
M denotes a methyne group substituted by the group R^{b},
Q denotes a methyne group substituted by the group R^{c},
while one or two of the groups G, M and Q in each case may also represent a nitrogen atom,
or
(b) D and E each denote a methyne group, while one of the groups D and E may also represent a nitrogen atom, and
G, M and Q each denote a nitrogen atom,
while R^{a}, R^{b} and R^{c} independently of one another each denote a hydrogen or halogen atom, a C₁₋₄-alkyl, C₂₋₄-alkenyl, C₂₋₄-alkynyl, cyclo-C₃₋₆-alkyl, cyclo-C₃₋₆-alkenyl, cyano, hydroxy, hydroxy-C₁₋₄-alkyl, hydroxy-C₃₋₄-alkenyl, hydroxy-C₃₋₄-alkynyl, C₁₋₄-alkoxy, C₁₋₄-alkoxy-C₁₋₄-alkyl, C₁₋₄-alkoxy-C₃₋₄-alkenyl, C₁₋₄-alkoxy-C₃₋₄-alkynyl, thiohydroxy, C₁₋₄-alkylthio, amino, C₁₋₄-alkyl-amino, C₃₋₄-alkenyl-amino, C₃₋₄-alkynyl-amino, di-(C₁₋₄-alkyl)-amino, di-(C₃₋₄-alkenyl)-amino, di-(C₃₋₄-alkynyl)-amino, amino-C₁₋₄-alkyl, C₁₋₃-alkylamino-C₁₋₄-alkyl, di-(C₁₋₃-alkyl)-amino-C₁₋₄-alkyl, amino-C₃-₄-alkenyl, C₁₋₃-alkylamino-C₃₋₄-alkenyl, di-(C₁₋₃-alkyl)-amino-C₃₋₄-alkenyl, amino-C₃₋₄-alkynyl, C₁₋₃-alkyl-amino-C₃₋₄-alkynyl, di-(C₁₋₃-alkyl)-amino-C₃₋₄-alkynyl, hydroxycarbonyl, phenylcarbonyl, pyridylcarbonyl, C₁₋₄-alkyl-carbonyl, formyl, C₁₋₄-alkoxy-carbonyl, C₃₋₄-alkenoxy-carbonyl, C₃₋₄-alkynoxy-carbonyl, aminocarbonyl, C₁₋₄-alkyl-aminocarbonyl, C₃₋₄-alkenyl-aminocarbonyl, G₃₋₄-alkynyl-aminocarbonyl, di-(C₁₋₄-alkyl)-aminocarbonyl, di-(C₃₋₄-alkenyl)-aminocarbonyl, di-C₃₋₄-(alkynyl)-aminocarbonyl, formylamino, C₁₋₄-alkylcarbonylamino, formyl-C₁₋₄-alkyl-amino, formyl-C₃₋₄-alkanyl-amino, formyl-C₃₋₄-alkynyl-amino, C₁₋₄-alkyl-carbonyl-C₁₋₄-alkyl-amino, C₁₋₄-alkyl-carbonyl-C₃₋₄-alkenyl-amino, C₁₋₄-alkyl-carbonyl-C₂₋₄-alkynyl-amino, C₁₋₄-alkylsulphonyl, C₂₋₄-alkenyl-sulphonyl, C₂₋₄-alkynyl-sulphonyl, C₁₋₄-alkyl-sulphinyl, C₂-₄-alkenyl-sulphinyl, C₂₋₄-alkynyl-sulphinyl, C₁₋₄-alkyl-sulphonylamino, C₁₋₄-alkyl-sulphonyl-C₁₋₄-alkylamino, C₁₋₄-alkyl-sulphonyl-C₃₋₄-alkenylamino, C₁₋₄-alkyl-sulphonyl-C₃₋₄-alkynylamino, aminosulphonyl, C₁₋₄-alkylaminosulphonyl, di-(C₁₋₄-alkyl)-aminosulphonyl, C₃₋₄-alkenylamino-sulphonyl, di-(C₃₋₄.-alkenyl)-aminosulphonyl, C₃₋₄-alkynylaminosulphonyl or di-(C₃₋₄-alkynyl)-aminosulphonyl group,
with the provisos that, if none of the groups D, E, G, M and Q denotes a nitrogen atom,
(i) R^{a} does not denote a hydrogen atom, if R^{b} and R^{c} each denote a C₁₋₄-alkyl group,
(ii) R^{c} does not denote a hydrogen atom, if R^{a} and R^{b} each denote a C₁₋₄-alkyl group,
(iii) R^{a} does not take on the meanings of a hydrogen, fluorine, chlorine, bromine or iodine atom or a difluoro- or trifluoromethyl group, if R^{c} denotes a C₁₋₄-alkyl, C₂₋₄-alkenyl or C₂₋₄-alkynyl group and R^{b} denotes a chlorine or bromine atom or an amino, methylamino, or hydroxy, group,
(iv) R^{c} does not take on the meanings of a hydrogen, fluorine, chlorine, bromine or iodine atom or a difluoro- or trifluoromethyl group, if R^{a} denotes a C₁₋₄-alkyl, C₂₋₄-alkenyl or C₂₋₄-alkynyl group and R^{b} denotes a chlorine or bromine atom, an amino, methylamino, or hydroxy group,
while, unless otherwise stated, all the alkyl, alkenyl and alkynyl groups mentioned or contained in the groups defined hereinbefore for R^{a}, R and R^{c} may be straight-chain or branched, every methyne group contained in the groups defined hereinbefore may be substituted by a fluorine atom, every methylene group may be substituted by up to 2 fluorine atoms and every methyl group may be substituted by up to 3 fluorine atoms and two alkyl and alkenyl groups bound to a nitrogen atom may be joined together forming a 4-to 7-membered, saturated or unsaturated heterocyclic ring,
the double and triple bonds of the C₃₋₄-alkenyl or C₃₋₄-alkynyl groups contained in the groups given as definitions for R^{a}, R^{b} and R^{c} hereinbefore may be isolated from any heteroatoms optionally also contained in these groups,
and all the aromatic and heteroaromatic groups mentioned or contained in the groups defined hereinbefore may additionally be mono-, di- or trisubstituted by halogen, by cyano or hydroxy groups and the substituents may be identical or different,
the tautomers, the isomers, the diastereomers, the enantiomers, the hydrates thereof, the mixtures thereof and the salts thereof and the hydrates of the salts, particularly the physiologically acceptable salts thereof with inorganic or organic acids or bases.

4. CGRP-antagonists of general formula (I) according to claim 1, wherein
A, X, R¹, R² and R³ are defined as in one of claims 1 to 2 and
(a) D, E independently of one another each denote a methyne group or the nitrogen atom and
G denotes a methyne group substituted by the group R^{a},
M denotes a methyne group substituted by the group R^{b},
Q denotes a methyne group substituted by the group R^{c},
while one or two of the groups G, M and Q in each case may also represent a nitrogen atom,
or
(b) D and E each denote a methyne group, while one of the groups D and E may also represent a nitrogen atom, and
G, M and Q each denote a nitrogen atom,
while R^{a}, R^{b} and R^{c} independently of one another each denote a hydrogen or halogen atom, a C₁₋₄-alkyl, C₂₋₄-alkenyl, C₂₋₄-alkynyl, cyclo-C₃₋₆-alkyl, cyclo-C₃₋₆-alkenyl, cyano, hydroxy, hydroxy-C₁₋₂-alkyl, hydroxy-C₃-alkenyl, hydroxy-C₃-alkynyl, C₁₋₄-alkoxy, C₁₋₄-alkoxy-C₁₋₂-alkyl, amino, C₁₋₄-alkyl-amino, C₃₋₄-alkenyl-amino, C₃₋₄-alkynyl-amino, di-(C₁₋₄-alkyl)-amino, di-(C₃₋₄-alkenyl)-amino, di-(C₃₋₄-alkynyl)-amino, amino-C₁₋₂-alkyl, C₁₋₃-alkyl-amino-C₁₋₂-alkyl, di-(C₁₋₃-alkyl)-amino-C₁₋₂-alkyl, amino-C₃-alkenyl, C₁₋₃-alkyl-amino-C₃-alkenyl, di-(C₁₋₃-alkyl)-amino-C₃-alkenyl, amino-C₃-alkynyl, C₁₋₃-alkyl-amino-C₃-alkynyl, di-(C₁₋₃-alkyl)-amino-C₃-alkynyl, hydroxycarbonyl, C₁₋₄-alkylcarbonyl, formyl, C₁₋₄-alkoxy-carbonyl, aminocarbonyl, C₁₋₄-alkylaminocarbonyl, di-(C₁₋₄-alkyl)-aminocarbonyl, formylamino, C₁₋₄-alkylcarbonylamino, formyl-C₁₋₄-alkyl-amino, C₁₋₄-alkyl-carbonyl-C₁₋₄-alkyl-amino, C₁₋₄-alkyl-sulphonyl, C₁₋₄-alkyl-sulphinyl, C₁₋₄-alkyl-sulphonylamino, C₁₋₄-alkylsulphonyl-C₁₋₄-alkylamino, aminosulphonyl, C₁₋₄-alkylaminosulphonyl or di-(C₁₋₄-alkyl)-aminosulphonyl group,
with the provisos that, if none of the groups D, E, G, M and Q denotes a nitrogen atom,
(i) R^{a} does not denote a hydrogen atom if R^{b} and R^{c} each denote a C₁₋₄-alkyl group,
(ii) R^{c} does not denote a hydrogen atom if R^{a} and R^{b} each denote a C₁₋₄-alkyl group,
(iii) R^{a} does not take on the meanings of a hydrogen, fluorine, chlorine, bromine or iodine atom or a difluoro- or trifluoromethyl group if R^{c} denotes a C₁₋₄-alkyl, C₂₋₄-alkenyl or C₂₋₄-alkynyl group and R^{b} denotes a chlorine or bromine atom, an amino, methylamino or hydroxy group,
(iv) R^{c} does not take on the meanings of a hydrogen, fluorine, chlorine, bromine or iodine atom or a difluoro- or trifluoromethyl, group if R^{a} denotes a C₁₋₄-alkyl, C₂₋₄-alkenyl or C₂₋₄-alkynyl group and R^{b} denotes a chlorine or bromine atom, an amino, methylamino or hydroxy group,
while, unless otherwise stated, all the alkyl, alkenyl and alkynyl groups mentioned or contained in the groups defined hereinbefore for R^{a}, R and R^{c} may be straight-chain or branched, every methyne group contained in the groups defined hereinbefore may be substituted by a fluorine atom, every methylene group may be substituted by up to 2 fluorine atoms and every methyl group may be substituted by up to 3 fluorine atoms and two alkyl and alkenyl groups bound to a nitrogen atom may be joined together forming a 4-to 7-membered, saturated or unsaturated heterocyclic ring,
the double and triple bonds of the C₃₋₄-alkenyl or C₃₋₄-alkynyl groups contained in the groups given as definitions for R^{a}, R^{b} and R^{e} hereinbefore may be isolated from any heteroatoms optionally also contained in these groups,
the tautomers, the isomers, the diastereomers, the enantiomers, the hydrates thereof, the mixtures thereof and the salts thereof and the hydrates of the salts, particularly the physiologically acceptable salts thereof with inorganic or organic acids or bases.

5. CGRP-antagonists of general formula (I) according to claim 1, wherein
A, X, R¹, R² and R³ are defined as in one of claims 1 to 2 and
(a) D, E independently of one another each denote a methyne group or the nitrogen atom and
G denotes a methyne group substituted by the group R^{a},
M denotes a methyne group substituted by the group R^{b},
Q denotes a methyne group substituted by the group R^{c},
while one or two of the groups G, M and Q in each case may also represent a nitrogen atom,
or
(b) D and E each denote a methyne group, while one of the groups D and E may also represent a nitrogen atom, and
G, M and Q each denote a nitrogen atom,
while R^{a}, R^{b} and R^{c} independently of one another each denote a hydrogen or halogen atom, a C₁₋₄-alkyl, C₂₋₄-alkenyl, C₂₋₄-alkynyl, cyclo-C₃₋₆-alkyl, cyclo-C₃₋₆-alkenyl, cyano, hydroxy, hydroxy-C₁₋₂-alkyl, C₁₋₄-alkoxy, amino, C₁₋₄-alkyl-amino, di-(C₁₋₄-alkyl)-amino, amino-C₁₋₂-alkyl, C₁₋₃-alkyl-amino-C₁₋₂-alkyl, di-(C₁₋₃-alkyl)-amino-C₁₋₂-alkyl, hydroxycarbonyl, C₁₋₄-alkylcarbonyl, formyl, C₁₋₄-alkoxy-carbonyl, aminocarbonyl, C₁₋₄-alkylaminocarbonyl, di-(C₁₋₄-alkyl)-aminocarbonyl, formylamino, C₁₋₄-alkylcarbonylamino, formyl-C₁₋₄-alkyl-amino or C₁₋₄-alkyl-carbanyl-C₁₋₄-alkyl-amino group,
with the provisos that, if none of the groups D, E, G, M and Q denotes a nitrogen atom,
(i) R^{a} does not denote a hydrogen atom if R^{b} and R^{c} each denote a C₁₋₄-alkyl group,
(ii) R^{c} does not denote a hydrogen atom if R^{a} and R^{b} each denote a C₁₋₄-alkyl group,
(iii) R^{a} does not take on the meanings of a hydrogen, fluorine, chlorine, bromine or iodine atom or a difluoro- or trifluoromethyl group if R^{c} denotes a C₁₋₄-alkyl, C₂₋₄-alkenyl or C₂₋₄-alkynyl group and R^{b} denotes a chlorine or bromine atom, an amino, methylamino or hydroxy group,
(iv) R^{c} does not take on the meanings of a hydrogen, fluorine, chlorine, bromine or iodine atom or a difluoro- or trifluoromethyl group if R^{a} denotes a C₁₋₄-alkyl, C₂₋₄-alkenyl or C₂₋₄-alkynyl group and R^{b} denotes a chlorine or bromine atom, an amino, methylamino or hydroxy group,
while, unless otherwise stated, all the alkyl, alkenyl and alkynyl groups mentioned or contained in the groups defined hereinbefore for R^{a}, R and R^{c} may be straight-chain or branched, every methyne group contained in the groups defined hereinbefore may be substituted by a fluorine atom, every methylene group may be substituted by up to 2 fluorine atoms and every methyl group may be substituted by up to 3 fluorine atoms and two alkyl and alkenyl groups bound to a nitrogen atom may be joined together forming a 4-to 7-membered, saturated or unsaturated heterocyclic ring,
the tautomers, the isomers, the diastereomers, the enantiomers, the hydrates thereof, the mixtures thereof and the salts thereof and the hydrates of the salts, particularly the physiologically acceptable salts thereof with inorganic or organic acids or bases.

6. CGRP-antagonists of general formula (I) according to claim 1, wherein
A, X, R¹, R² and R³ are defined as in one of claims 1 to 2 and
(a) D, E independently of one another each denote a methyne group or the nitrogen atom and
G denotes a methyne group substituted by the group R^{a},
M denotes a methyne group substituted by the group R^{b},
Q denotes a methyne group substituted by the group R^{c},
while one or two of the groups G, M and Q in each case may also represent a nitrogen atom,
or
(b) D and E each denote a methyne group, while one of the groups D and E may also represent a nitrogen atom, and
G, M and Q each denote a nitrogen atom,
while R^{a}, R^{b} and R^{c} independently of one another each denote a hydrogen or halogen atom, a methyl, difluoromethyl, trifluoromethyl, ethyl, vinyl, ethynyl, cyano, hydroxy, methoxy, difluoromethoxy, trifluoromethoxy, amino, methylamino or dimethylamino group,
with the provisos that, if none of the groups D, E, G, M and Q denotes a nitrogen atom,
(i) R^{a} does not denote a hydrogen atom if R^{b} and R^{c} each denote a methyl or ethyl group,
(ii) R^{c} does not denote a hydrogen atom if R^{a} and R^{b} each denote a methyl or ethyl group,
(iii) R^{a} does not take on the meanings of a hydrogen, fluorine, chlorine, bromine or iodine atom or a difluoro- or trifluoromethyl group if R^{c} denotes a methyl, ethyl, vinyl or ethynyl group and R denotes a chlorine or bromine atom, an amino, methylamino or hydroxy group,
(iv) R^{c} does not take on the meanings of a hydrogen, fluorine, chlorine, bromine or iodine atom or a difluoro- or trifluoromethyl group if R^{a} denotes a methyl, ethyl, vinyl or ethynyl group and R denotes a chlorine or bromine atom, an amino, methylamino or hydroxy group,
the tautomers, the isomers, the diastereomers, the enantiomers, the hydrates thereof, the mixtures thereof and the salts thereof and the hydrates of the salts, particularly the physiologically acceptable salts thereof with inorganic or organic acids or bases.

7. CGRP-antagonists of general formula (I) according to claim 1, wherein
A, X, D, E, G, M, Q and R¹ are defined as in one of claims 1 to 6 and
R² denotes the hydrogen atom or
a phenylmethyl group or a C₂₋₇-alkyl group which may be substituted in the ω position by a cyclo-C₃₋₇-alkyl, cyclo-C₃₋₇-alkenyl, phenyl, pyridinyl, hydroxy, amino, C₁₋₆-alkylamino, di-(C₁₋₆-alkyl)-amino, hydroxycarbonyl, C₁₋₆-alkoxycarbonyl, aminocarbonyl, aminocarbonylamino, C₁₋₆-alkylamino, 4-morpholinyl group,
while the phenyl and pyridinyl groups mentioned in the groups defined for R² hereinbefore or contained as substituents may additionally be mono-, di- or trisubstituted in the carbon skeleton by halogen, by C₁₋₃-alkyl, C₁₋₃-alkoxy, hydroxy, amino, C₁₋₃-alkylamino, di-(C₁₋₃-alkyl)-amino, amino-C₁₋₃-alkyl, C₁₋₃-alkylamino-C₁₋₃-alkyl, di-(C₁₋₃-alkyl)-amino-C₁₋₃-alkyl, C₁₋₃-alkylcarbonylamino, C₁₋₃-alkylcarbonyl-C₁₋₃-alkylamino, aminocarbonyl, C₁₋₃-alkylaminocarbonyl or di-(C₁₋₃-alkyl)-aminocarbonyl groups and the substituents may be identical or different,
R³ denotes the hydrogen atom or a C₁₋₃-alkyl group or
R² and R³ together with the enclosed nitrogen atom denote a group of general formula 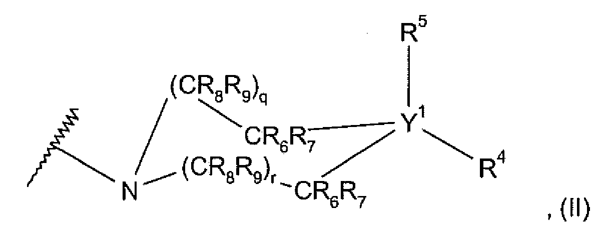 wherein
Y¹ denotes the carbon atom or, if R⁵ denotes a free electron pair, it may also denote the nitrogen atom,
q and r, if Y¹ denotes the carbon atom, denote the numbers 0 or 1 or
q and r, if Y¹ denotes the nitrogen atom, denote the numbers 1 or 2,
R⁴ denotes the hydrogen atom, an amino, C₁₋₄-alkyl-amino, di-(C₁₋₄-alkyl)-alkylamino, C₁₋₆-alkyl, a cyclo-C₃₋₇-alkyl or cyclo-C₃₋₇-alkenyl group optionally substituted by a hydroxycarbonyl, C₁₋₆-alkoxycarbonyl, hydroxycarbonyl-C₁₋₃-alkyl or C₁₋₆-alkoxycarbonyl-C₁₋₃-alkyl group, an amino-C₂₋₇-alkyl, C₁₋₄-alkyl-amino-C₂₋₇-alkyl, di-(C₁₋₄-alkylamino)-C₂₋₇-alkyl, C₁₋₄-alkyl-aminocarbonyl, di-(C₁₋₄-alkyl)-aminocarbonyl, aminocarbonyl-C₁₋₃-alkyl, C₁₋₄-alkyl-aminocarbonyl-C₁₋₃-alkyl, di-(C₁₋₄-alkyl)-aminocarbonyl-C₁₋₃-alkyl, aminocarbonylamino-C₁₋₃-alkyl, C₁₋₆-alkoxycarbonyl , C₁₋₆-alkoxycarbonyl-C₁₋₃-alkyl or hydroxycarbonyl-C₁₋₃-alkyl group,
a phenyl, pyridinyl or diazinyl group which may be substituted in each case by a halogen, a C₁₋₃-alkyl, C₁₋₃-alkoxy, hydroxy, amino, C₁₋₄-alkylamino, di-(C₁₋₄-alkyl)-amino, amino-C₁₋₃-alkyl, C₁₋₄-alkyl-amino-C₁₋₃-alkyl, di-(C₁₋₄-alkyl)-amino-C₁₋₃-alkyl group,
a heterocycle selected from a 4- to 7-membered azacycloalkyl group, a 6-to 7-membered oxaza-, S,S-dioxothiaza- and diazacycloalkyl group and a 7- to 9-membered azabicycloalkyl group,
while the above-mentioned mono- and bicyclic heterocycles are bound to Y¹ in formula (II) via a nitrogen or a carbon atom, in the above-mentioned mono- and bicyclic heterocycles a methylene group not directly linked to a nitrogen, oxygen or sulphur atom may be substituted by one or two fluorine atoms and
the above-mentioned mono- and bicyclic heterocycle may be mono- or disubstituted by hydroxy, C₁₋₃-alkyl or hydroxy-C₁₋₃-alkyl groups or monosubstituted by a benzyl, cyclo-C₃₋₆-alkyl, hydroxycyclo-C₃₋₆-alkyl, cydo-C₃₋₆-alkyl-C₁₋₃-alkyl, C₁₋₄-alkylcarbonyl, C₁₋₄-alkyloarbonyl-C₁₋₃-alkyl, hydroxy, C₁₋₄-alkoxy, amino, C₁₋₄-alkylamino, di-(C₁₋₄-alkyl)-amino, C₁₋₃-alkoxycarbonyl, hydroxycarbonyl-carbonyl, C₁₋₃-alkoxycarbonyl-carbonyl, hydroxycarbonyl-C₁₋₃-alkyl, C₁₋₃-alkoxycarbonyl-C₁₋₃-alkyl, hydroxycarbonyl-C₁₋₃-alkylcarbonyl, C₁₋₃-alkoxycarbonyl-C₁₋₃-alkylcarbonyl, aminosulphonyl, C₁₋₄-alkylaminosulphonyl, di-(C₁₋₄-alkyl)-aminosulphonyl, C₁₋₃-alkylsulphonyl, cyclo-C₃₋₇-alkylsulphonyl, aminocarbonyl-C₁₋₃-alkyl, C₁₋₄-alkylaminocarbonyl-C₁₋₃-alkyl, di-(C₁₋₄-alkyl)-aminocarbonyl-C₁₋₃-alkyl, hydroxyaminocarbonyl-C₁₋₃-alkyl, C₁₋₃-alkoxyaminocarbonyl-C₁₋₃-alkyl or hydroxy-(C₁₋₃-alkyl)-aminocarbonyl-C₁₋₃-alkyl group,
or also, if Y¹ denotes the carbon atom, the hydroxycarbonyl, aminomethyl, C₁₋₄-alkyl-aminomethyl or di-(C₁₋₄-alkyl)-aminomethyl group,
R⁵ denotes a hydrogen atom, a C₁₋₃-alkyl group or, if Y¹ denotes a nitrogen atom, also a free electron pair,
R⁶ and R⁷, which may be identical or different, each denote a hydrogen atom, a C₁₋₃-alkyl group or also, if Y¹ denotes a carbon atom, an amino, C₁₋₃-alkylamino or di-(C₁₋₃-alkyl)-amino group, while the two C₁₋₃-alkyl groups may be joined together, forming a ring and
R⁸ and R⁹, which may be identical or different, each denote a hydrogen atom or a C₁₋₃-alkyl group,
while, unless otherwise stated, all the alkyl, alkenyl and alkynyl groups mentioned or contained in the groups defined hereinbefore may be straight-chain or branched, every methyne group contained in the groups defined hereinbefore may be substituted by a fluorine atom, every methylene group may be substituted by up to 2 fluorine atoms and every methyl group may be substituted by up to 3 fluorine atoms and two alkyl and alkenyl groups bound to a nitrogen atom may be joined together forming a 4- to 7-membered, saturated or unsaturated heterocyclic ring,
all the aromatic and heteroaromatic groups mentioned or contained in the groups defined hereinbefore may additionally be mono-, di- or trisubstituted by halogen, by cyano or hydroxy groups and the substituents may be identical or different,
the tautomers, the isomers, the diastereomers, the enantiomers, the hydrates thereof, the mixtures thereof and the salts thereof and the hydrates of the salts, particularly the physiologically acceptable salts thereof with inorganic or organic acids or bases.

8. CGRP-antagonists of general formula (I) according to claim 1, wherein
A, X, D, E, G, M, Q and R¹ are defined as in one of claims 1 to 6 and
R² denotes the hydrogen atom or
a phenylmethyl group or a C₂₋₇-alkyl group which may be substituted in the ω position by a cyclo-C₃₋₇-alkyl, cyclo-C₃₋₇-alkenyl, phenyl, pyridinyl, hydroxy, amino, C₁₋₆-alkylamino, di-(C₁₋₆-alkyl)-amino, hydroxycarbonyl, C₁₋₆-alkoxycarbonyl, aminocarbonyl, aminocarbonylamino, C₁₋₆-alkylamino, 4-morpholinyl group,
while the phenyl and pyridinyl groups mentioned in the groups given as definitions for R² or contained as substituents therein may additionally be mono-, di- or trisubstituted in the carbon skeleton by halogen, by C₁₋₃-alkyl, C₁₋₃-alkoxy, hydroxy, amino, C₁₋₃-alkylamino, di-(C₁₋₃-alkyl)-amino, amino-C₁₋₃-alkyl, C₁₋₃-alkylamino-C₁₋₃-alkyl, di-(C₁₋₃-alkyl)-amino-C₁₋₃-alkyl, C₁₋₃-alkylcarbonylamino, C₁₋₃-alkylcarbonyl-C₁₋₃-alkylamino, aminocarbonyl, C₁₋₃-alkylaminocarbonyl or di-(C₁₋₃-alkyl)-aminocarbonyl groups and the substituents may be identical or different,
R³ denotes the hydrogen atom or a C₁₋₃-alkyl group or
R² and R³ together with the enclosed nitrogen atom denote a group of general formula 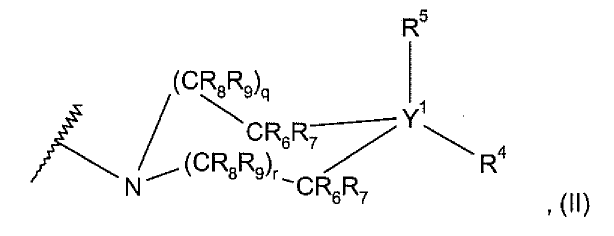 wherein
Y¹ denotes the carbon atom or, if R⁵ denotes a free electron pair, it may also denote the nitrogen atom,
q and r, if Y¹ denotes the carbon atom, denote the numbers 0 or 1 or
q and r, if Y¹ denotes the nitrogen atom, denote the numbers 1 or 2,
R⁴ denotes the hydrogen atom, an amino, C₁₋₄-alkyl-amino, di-(C₁₋₄-alkyl)-alkylamino, C₁₋₆-alkyl, cyclo-C₃₋₇-alkyl, cyclo-C₃₋₇-alkenyl, amino-C₂₋₇-alkyl, C₁₋₄-alkyl-amino-C₂₋₇-alkyl, di-(C₁₋₄-alkylamino)-C₂₋₇-alkyl, C₁₋₄-alkylaminocarbonyl, di-(C₁₋₄-alkyl)-aminocarbonyl, aminocarbonyl-C₁₋₃-alkyl, C₁₋₄-alkyl-aminocarbonyl-C₁₋₃-alkyl, di-(C₁₋₄-alkyl)-aminocarbonyl-C₁₋₃-alkyl, aminocarbonylamino-C₁₋₃-alkyl, C₁₋₆-alkoxycarbonyl, C₁₋₆-alkoxycarbonyl-C₁₋₃-alkyl or hydroxycarbonyl-C₁₋₃-alkyl group,
a phenyl, pyridinyl or diazinyl group which may be substituted in each case by a halogen, by a C₁₋₃-alkyl, C₁₋₃-alkoxy, hydroxy, amino, C₁₋₄-alkylamino, di-(C₁₋₄-alkyl)-amino, amino-C₁₋₃-alkyl, C₁₋₄-alkyl-amino-C₁₋₃-alkyl, di-(C₁₋₄-alkyl)-amino-C₁₋₃-alkyl group,
a heterocycle selected from a 4- to 7-membered azacycloalkyl group, a 6-to 7-membered oxaza- and diazacycloalkyl group and a 7- to 9-membered azabicycloalkyl group,
while the above-mentioned mono- and bicyclic heterocycles are bound to Y¹ in formula (II) via a nitrogen or carbon atom,
in the above-mentioned mono- and bicyclic heterocycles a methylene group not directly attached to a nitrogen, oxygen or sulphur atom may be substituted by one or two fluorine atoms and
the above-mentioned mono- and bicyclic heterocycles may be mono- or polysubstituted, for example mono- to trisubstituted, by C₁₋₃-alkyl groups or monosubstituted by a benzyl, cyclo-C₃₋₆-alkyl, cyclo-C₃₋₆-alkyl-C₁₋₃-alkyl, C₁₋₄-alkylcarbonyl, hydroxy, C₁₋₄-alkoxy, amino, C₁₋₄-alkylamino, di-(C₁₋₄-alkyl)-amino, hydroxycarbonyl, C₁₋₃-alkoxycarbonyl, hydroxycarbonyl-carbonyl, C₁₋₃-alkoxycarbonyl-carbonyl, hydroxycarbonyl-C₁₋₃-alkyl, C₁₋₃-alkoxycarbonyl-C₁₋₃-alkyl or C₁₋₃-alkylsulphonyl group,
or also, if Y¹ denotes the carbon atom, the hydroxycarbonyl, aminomethyl, C₁₋₄-alkyl-aminomethyl or di-(C₁₋₄-alkyl)-aminomethyl group,
R⁵ denotes a hydrogen atom, a C₁₋₃-alkyl group or, if Y¹ denotes a nitrogen atom, it may also denote a free electron pair,
and R⁷, which may be identical or different, each denote a hydrogen atom, a C₁₋₃-alkyl group or also, if Y¹ denotes a carbon atom, a C₁₋₃-alkylamino or di-(C₁₋₃-alkyl)-amino group, while the two C₁₋₃-alkyl groups may be joined together forming a ring and
R⁸ and R⁹, which may be identical or different, each denote a hydrogen atom or a C₁₋₃-alkyl group,
while, unless otherwise stated, all the alkyl, alkenyl and alkynyl groups mentioned or contained in the groups defined hereinbefore may be straight-chain or branched, each methyne group contained in the groups defined hereinbefore may be substituted by a fluorine atom, each methylene group may be substituted by up to 2 fluorine atoms and each methyl group may be substituted by up to 3 fluorine atoms and two alkyl and alkenyl groups bound to a nitrogen atom may be joined together forming a 4- to 7-membered, saturated or unsaturated heterocyclic ring,
all the aromatic and heteroaromatic groups mentioned or contained in the groups defined hereinbefore may additionally be mono-, di- or trisubstituted by halogen or by cyano or hydroxy groups and the substituents may be identical or different,
the tautomers, the isomers, the diastereomers, the enantiomers, the hydrates thereof, the mixtures thereof and the salts thereof as well as the hydrates of the salts, particularly the physiologically acceptable salts thereof with inorganic or organic acids or bases.

9. CGRP-antagonists of general formula (I) according to claim 1, wherein
A, X, D, E, G, M, Q and R¹ are defined as in one of claims 1 to 6 and
R² denotes the hydrogen atom or
a phenylmethyl group or a C₂₋₇-alkyl group which may be substituted in the ω position by a phenyl, amino, C₁₋₆-alkylamino, di-(C₁₋₆-alkyl)-amino group,
while the phenyl and phenylmethyl group mentioned hereinbefore may additionally be mono- or disubstituted at an aromatic carbon atom by halogen, by C₁₋₃-alkyl, C₁₋₃-alkoxy, amino-C₁₋₃-alkyl, C₁₋₃-alkylamino-C₁₋₃-alkyl or di-(C₁₋₃-alkyl)-amino-C₁₋₃-alkyl groups and the substituents may be identical or different,
R³ denotes the hydrogen atom or a C₁₋₃-alkyl group or
R² and R³ together with the enclosed nitrogen atom denote a group of general formula 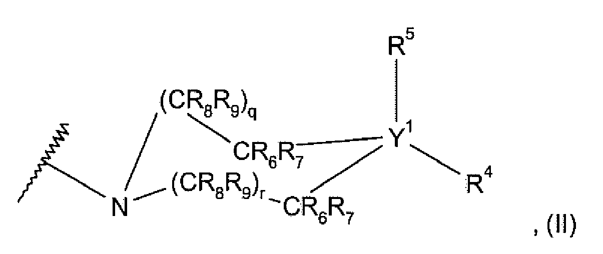 wherein
Y¹ denotes the carbon atom or, if R⁵ denotes a free electron pair, it may also denote the nitrogen atom,
q and r, if Y¹ denotes the carbon atom, denote the numbers 0 or 1 or
q and r, if Y¹ denotes the nitrogen atom, denote the numbers 1 or 2,
R⁴ denotes the hydrogen atom, an amino, C₁₋₄-alkyl-amino, di-(C₁₋₄-alkyl)-alkylamino, C₁₋₆-alkyl, a cyclo-C₃₋₇-alkyl or cyclo-C₃₋₇-alkenyl group optionally substituted by a hydroxycarbonyl, C₁₋₆-alkoxycarbonyl, hydroxycarbonyl-C₁₋₃-alkyl or C₁₋₆-alkoxycarbonyl-C₁₋₃-alkyl group, an amino-C₂₋₇-alkyl, C₁₋₄-alkyl-amino-C₂₋₇-alkyl, di-(C₁₋₄-alkylamino)-C₂₋₇-alkyl, C₁₋₆-alkoxycarbonyl, C₁₋₆-alkoxycarbonyl-C₁₋₃-alkyl or hydroxycarbonyl-C₁₋₃-alkyl group,
a phenyl or pyridyl group which may be substituted in each case by a halogen, by a C₁₋₃-alkyl, C₁₋₃-alkoxy, amino, C₁₋₄-alkyl-amino, di-(C₁₋₄-alkyl)-amino group,
a heterocycle selected from a 6- to 7-membered azacycloalkyl group, a 6-to 7-membered S,S-dioxothiaza- and diazacycloalkyl group and a 7- to 9-membered azabicycloalkyl group,
while the above-mentioned mono- and bicyclic heterocycles are bound to Y¹ in formula (II) via a nitrogen or a carbon atom,
in the above-mentioned mono- and bicyclic heterocycles a methylene group not directly linked to a nitrogen, oxygen or sulphur atom may be substituted by one or two fluorine atoms and
the above-mentioned mono- and bicyclic heterocycles may be mono- or disubstituted by a hydroxy, C₁₋₃-alkyl or hydroxy-C₁₋₃-alkyl group, by a benzyl, cyclo-C₃₋₆-alkyl, hydroxy-C₃₋₆-cycloalkyl, cyclo-C₃₋₆-alkyl-C₁₋₃-alkyl, C₁₋₃-alkylcarbonyl-C₁₋₃-alkyl, amino, C₁₋₄-alkylamino or di-(C₁₋₄-alkyl)-amino, hydroxycarbonyl-carbonyl, C₁₋₆-alkoxycarbonyl-carbonyl, hydroxycarbonyl-C₁₋₃-alkylcarbonyl, C₁₋₃-alkoxycarbonyl-C₁₋₃-alkylcarbonyl, aminosulphonyl, C₁₋₄-alkylaminosulphonyl, di-(C₁₋₄-alkyl)-aminosulphonyl, cyclo-C₃₋₇-alkylsulphonyl, aminocarbonyl-C₁₋₃-alkyl, C₁₋₄-alkylaminocarbonyl-C₁₋₃-alkyl, di-(C₁₋₄-alkyl)-aminocarbonyl-C₁₋₃-alkyl, hydroxyaminocarbonyl-C₁₋₃-alkyl, C₁₋₃-alkoxyaminocarbonyl-C₁₋₃-alkyl or hydroxy-(C₁₋₃-alkyl)-aminocarbonyl-C₁₋₃-alkyl groups,
or also, if Y¹ denotes the carbon atom, the hydroxycarbonyl, aminomethyl, C₁₋₄-alkyl-aminomethyl or di-(C₁₋₄-alkyl)-aminomethyl group,
R⁵ denotes a hydrogen atom or, if Y¹ denotes a nitrogen atom, may also denote a free electron pair,
R⁶ and R⁷, which may be identical or different, each denote a hydrogen atom, a C₁₋₃-alkyl group or also, if Y¹ denotes a carbon atom, a C₁₋₃-alkylamino or di-(C₁₋₃-alkyl)-amino group, while the two C₁₋₃-alkyl groups may be joined together, forming a ring and
R⁸ and R⁹, which may be identical or different, each denote a hydrogen atom or a C₁₋₃-alkyl group,
the tautomers, the isomers, the diastereomers, the enantiomers, the hydrates thereof, the mixtures thereof and the salts thereof and the hydrates of the salts, particularly the physiologically acceptable salts thereof with inorganic or organic acids or bases.

10. CGRP-antagonists of general formula (I) according to claim 1, wherein
A, X, D, E, G, M, Q and R¹ are defined as in one of claims 1 to 6 and
R² denotes a phenylmethyl group or a C₂₋₇-alkyl group which may be substituted in the ω position by a phenyl, amino, C₁₋₆-alkylamino, di-(C₁₋₆-alkyl)-amino group,
while the phenyl and phenylmethyl group mentioned hereinbefore may be substituted at an aromatic carbon atom by an amino-C₁₋₃-alkyl, C₁₋₃-alkylamino-C₁₋₃-alkyl or di-(C₁₋₃-alkyl)-amino-C₁₋₃-alkyl group,
R³ denotes the hydrogen atom or a C₁₋₃-alkyl group or
R² and R³ together with the enclosed nitrogen atom denote a group of general formula 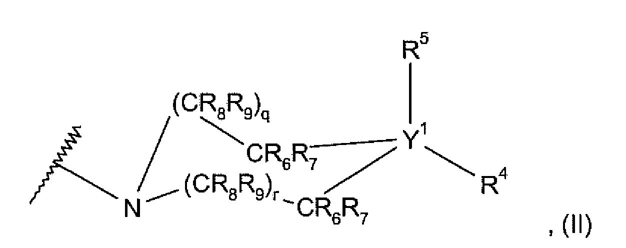 wherein
R⁶ and R⁷ each denote a hydrogen atom or a dimethylamino group,
R⁸ and R⁹ each denote the hydrogen atom and
(a) Y¹ denotes the carbon atom,
q and r denote the numbers 0 or 1,
R⁴ denotes the hydrogen atom,
a phenyl, pyridinyl or pyrimidinyl group which may be substituted in each case by a halogen, by an amino, methylamino, dimethylamino, methyl or methoxy group,
a hydroxy, 2-diethylamino-ethyl, amino, methylamino, dimethylamino, diethylamino, pyrrolidin-1-yl, 3-hydroxy-pyrrolidin-1-yl, 2-hydroxycarbonyl-pyrrolidin-9-yl, 2-methoxycarbonyl-pyrrolidin-1-yl, piperidin-1-yl, 4,4-dimethylpiperidin-1-yl, 4-amino-4-methyl-piperidin-1-yl, 2-hydroxycarbonyl-piperidin-1-yl, 2-methoxycarbonyl-piperidin-1-yl, 4-hydroxymethyl-piperidin-1-yl, 4-(1-hydroxycyclopropyl)-piperidin-1-yl, 4-amino-piperidin-1-yl, 4-methylamino-piperidin-1-yl, 4-dimethylamino-piperidin-1-yl, 4-hydroxy-4-methyl-piperidin-1-yl, 4-hydroxy-4-ethyl-piperidin-1-yl, 4-hydroxy-4-trifluoromethyl-piperidin-9-yl, 4-hydroxy-4-hydroxymethyl-piperidin-1-yl, 3-amino-piperidin-1-yl, 3-methylamino-piperidin-1-yl, 3-dimethylamino-piperidin-1-yl, 3-hydroxy-piperidin-1-yl, 4-hydroxy-piperidin-1-yl, 4-hydroxycarbonylmethyl-piperidin-1-yl, 4-ethoxycarbonylmethyl-piperidin-1-yl, perhydro-azepin-1-yl, perhydro-1,4-diazepin-1-yl, 4-methyl-perhydro-1,4-dfazepin-1-yl, 1-methyl-piperidin-4-yl, piperidin-4-yl, 1-ethylpiperidin-4-yl, 1-(2-hydroxyethyl)-piperidin-4-yl, 1-cyclopropyl-piperidin-4-yl, 1-cyclopropylmethyl-piperidin-4-yl, 1-methylsulphonyl-piperidin-4-yl, 1-ethylsulphonyl-piperidin-4-yl, 1-isopropylsulphonyl-piperidin-4-yl, 1-cyclopropylsulphonyl-piperidin-4-yl, 4-hydroxy-1-methylsulphonyl-piperidin-4-yl, 1-aminosulphonyl-piperidin-4-yl, 1-(methylaminosulphonyl)-piperidin-4-yl, 1-(dimethylaminosulphonyl)-piperidin-4-yl, 1-hydroxycarbonylmethyl-piperidin-4-yl, 1-ethoxycarbonylmethyl-piperidin-4-yl, 1-(2-hydroxycarbonylethyl)-piperidin-4-yl, 1-(2-ethoxycarbonylethyl)-piperidin-4-yl, 1-(3-hydroxycarbonyl-propionyl)-piperidin-4-yl, 1-(3-ethoxycarbonyl-propionyl)-piperidin-4-yl, 1-(hydroxycarbamoyl-methyl)-piperidin-4-yl, 1-(hydroxy-methylcarbamoyl-methyl)-piperidin-4-yl, 1-(methoxycarbamoyl-methyl)-piperidin-4-yl, 1-oxalyl-piperidin-4-yl, 1-ethoxyoxalyl-piperidin-4-yl, piperazin-1-yl, 4-methyl-piperazin-1-yl, 4-cyclopropylmethyl-piperazin-1-yl, 4-ethyl-piperazin-1-yl, 4-(2-hydroxyethyl)-piperazin-1-yl, 4-cyclopropyl-piperazin-1-yl, 4-methylsulphonyl-piperazin-1-yl, 4-aminosulphonyl-piperazin-1-yl, 4-(methylaminosulphonyl)-piperazin-1-yl, 4-(dimethylaminosulphonyl)-piperazin-1-yl, 4-hydroxycarbonylmethyl-piperazin-1-yl, 4-ethoxycarbonylmethyl-piperazin-1-yl, 4-(2-hydroxycarbonylethyl)-piperazin-1-yl, 4-(2-ethoxycarbonylethyl)-piperazin-1-yl, 4-(3-hydroxycarbonyl-propionyl)-piperazin-1-yl, 4-(3-ethoxycarbonyl-propionyl)-piperazin-1-yl, 4-(hydroxycarbamoyl)-methyl-piperazin-1-yl, 4-(hydroxy-methyl-carbamoyl)-methyl=piperazin-1-yl, 4-(methoxycarbamoyl)-methyl-piperazin-1-yl, 1,2-dimethyl-piperazin-1-yl, 3-methyl-piperazin-1-yl, 3,4,5-trimethyl-piperazin-1-yl, 3,5-dimethyl-piperazin-1-yl, 3,3,4-trimethyl-piperazin-1-yl, 3,3-dimethyl-piperazin-1-yl, 3,3,4,5,5-pentamethyl-piperazin-1-yl, 3,3,5,5-tetramethyl-piperazin-1-yl, 3,3,3-trifluoro-2-oxopropyl)-piperazin-1-yl, morpholin-4-yl, 1,1-dioxo-1λ⁶-thiomorpholin-4-yl, tetrahydropyran-4-yl, 4,4-difluoro-piperidin-1-yl, 8-methyl-8-aza-bicyclo[3.2.1]oct-3-yl, 8-aza-bicyclo[3.2.1]oct-3-yl, azetidin-1-yl, 1-(methoxycarbonylmethyl)-piperidin-4-yl, 1-(ethoxycarbonylmethyl)-piperidin-4-yl, 4-(ethoxycarbonylmethyl)-piperazin-1-yl, 1-hydroxycarbonylmethyl-piperidin-4-yl or 4-hydroxycarbonylmethyl-piperazin-1-yl group, and
R⁵ denotes a hydrogen atom, or
(b) Y¹ denotes a nitrogen atom,
q and r denote the numbers 1 or 2,
R⁴ denotes the hydrogen atom,
a phenyl, pyridinyl or pyrimidinyl group which may be substituted in each case by a halogen, by an amino, methylamino, dimethylamino, methyl or methoxy group,
a methyl, ethyl, isopropyl, cyclopropyl, cyclopentyl, cyclohexyl, cycloheptyl, 4-hydroxycarbonylmethyl-cylohexyl, 4-ethoxycarbonylmethyl-cyclohexyl, cyclopropylmethyl, 2-diethylamino-propyl, 1-quinuclidin-3-yl, tetrahydropyran-4-yl, 1-piperidin-4-yl, 1-methyl-piperidin-4-yl, 1-ethyl-piperidin-4-yl, 1-(2-hydroxyethyl)-piperidin-4-yl, 1-methylsulphonyl-piperidin-4-yl, 1-aminosulphonyl-piperidin-4-yl, 1-(methylaminosulphonyl)-piperidin-4-yl, 1-(dimethylaminosulphonyl)-piperidin-4-yl, 1-hydroxycarbonylmethyl-piperidin-4-yl, 1-ethoxycarbonytmethyl-piperidin-4-yl, 1-(2-hydroxycarbonylethyl)-piperidin-4-yl, 1-(2-ethoxycarbonylethyl)-piperidin-4-yl, 1-(3-hydroxycarbonyl-propionyl)-piperidin-4-yl, 1-(3-ethoxycarbonyl-propionyl)-piperidin-4-yl, 1-(hydroxycarbamoyl-methyl)-piperidin-4-yl, 1-(hydroxy-methylcarbamoyl-methyl)-piperidin-4-yl, 1-(methoxycarbamoyl-methyl)-piperidin-4-yl, 1-cyclopropyl-piperidin-4-yl, 1-cyclopropylmethyl-piperidin-4-yl, 1-hydroxycarbonylmethyl-piperidin-4-yl or 1-ethoxycarbonylmethyl-piperidin-4-yl group and
R⁵ denotes a free electron pair,
the tautomers, the isomers, the diastereomers, the enantiomers, the hydrates thereof, the mixtures thereof and the salts thereof and the hydrates of the salts, particularly the physiologically acceptable salts thereof with inorganic or organic acids or bases.

11. CGRP-antagonists of general formula (I) according to claim 1, wherein
A, X, D, E, G, M, Q and R¹ are defined as in one of claims 1 to 6 and
R² denotes a phenylmethyl group or a C₂₋₇-alkyl group which may be substituted in the ω position by a phenyl, amino, C₁₋₆-alkylamino, di-(C₁₋₆-alkyl)-amino group,
while the above-mentioned phenyl and phenylmethyl group may be substituted at an aromatic carbon atom by an amino-C₁₋₃-alkyl, C₁₋₃-alkylamino-C₁₋₃-alkyl or di-(C₁₋₃-alkyl)-amino-C₁₋₃-alkyl group,
R³ denotes the hydrogen atom or a C₁₋₃-alkyl group or
R² and R³ together with the enclosed nitrogen atom denote a group of general formula 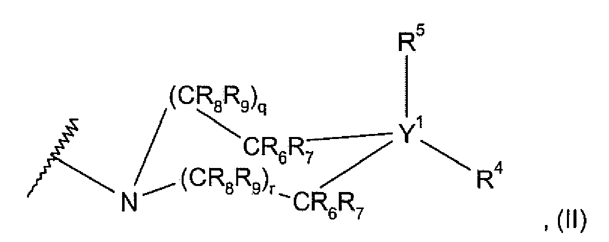 wherein
R⁶ and R⁷ each denote a hydrogen atom or a dimethylamino group,
R⁸ and R⁹ each denote the hydrogen atom and
(a) Y¹ denotes the carbon atom,
q and r denote the numbers 0 or 1,
R⁴ denotes the hydrogen atom,
a phenyl, pyridinyl or pyrimidinyl group which may be substituted in each case by a halogen or by an amino, methylamino, dimethylamino, methyl or methoxy group,
a hydroxy, 2-diethylamino-ethyl , amino, methylamino, dimethylamino, dimethylamino, pyrrolidin-1-yl, piperidin-1-yl, 4-amino-piperidin-1-yl, 4-methylamino-piperidin-1-yl, 4-dimethylamino-piperidin-1-yl, 3-amino-piperidin-1-yl, 3-methylamino-piperidin-1-yl, 3-dimethylamino-piperidin-1-yl, perhydro-azepin-1-yl, perhydro-1,4-diazepin-1-yl, 4-methyl-perhydro-1,4-diazepin-1-yl, 1-methyl-piperidin-4-yl, piperidin-4-yl, 1-ethylpiperidin-4-yl, 1-cyclopropyl-piperidin-4-yl, 1-cyclopropylmethyl-piperidin-4-yl, piperazin-1-yl, 4-methyl-piperazin-1-yl, 4-cyclopropylmethyl-piperazin-1-yl, 4-ethyl-piperazin-1-yl, 4-cyclopropyl-piperazin-1-yl, 1,2-dimethyl-piperazin-1-yl, 3-methyl-piperazin-1-yl, 3,4,5-trimethyl-piperazin-1-yl, 3,5-dimethyl-piperazin-1-yl, 3,3,4-trimethyl-piperazin-1-yl, 3,3-dimethyl-piperazin-1-yl, 3,3,4,5,5-pentamethyl-piperazin-1-yl, 3,3,5,5-tetramethyl-piperazin-1-yl, morpholin-4-yl, 4,4-difluoro-piperidin-1-yl, 8-methyl-8-aza-bicyclo[3.2.1]oct-3-yl, 8-aza-bicyclo[3.2.1]oct-3-yl, azetidin-1-yl, 1-(methoxycarbonylmethyl)-piperidin-4-yl, 1-(ethoxycarbonylmethyl)-piperidin-4-yl, 4-(ethoxycarbonylmethyl)-piperazin-1-yl, 1-hydroxycarbonylmethyl-piperidin-4-yl or 4-hydroxycarbonylmethyl-piperazin-1-yl group, and
R⁵ denotes a hydrogen atom, or
(b) Y¹ denotes a nitrogen atom,
q and r denote the numbers 1 or 2,
R⁴ denotes the hydrogen atom,
a phenyl, pyridinyl or pyrimidinyl group which may be substituted in each case by a halogen or by an amino, methylamino, dimethylamino, methyl or methoxy group,
a methyl, ethyl, isopropyl, cyclopropyl, cyclopropylmethyl, 2-diethylarnino-propyl, 1-quinuclidin-3-yl, 1-piperidin-4-yl, 1-methyl-piperidin-4-yl, 1-ethyl-piperidin-4-yl, 1-cyclopropyl-piperidin-4-yl, 1-cyclopropylmethyl-piperidin-4-yl, 1-hydroxycarbonylmethyl-piperidin-4-yl or 1-ethoxycarbonylmethyl-piperidin-4-yl group and
R⁵ denotes a free electron pair,
the tautomers, the diastereomers, the enantiomers, the hydrates, the mixtures thereof and the salts thereof as well as the hydrates of the salts, particularly the physiologically acceptable salts thereof with inorganic or organic acids or bases.

12. CGRP-antagonists of general formula (I) according to claim 1, wherein
D, E, G, M, Q, R¹, R² and R³ are defined as in one of claims 1 to 10 and
A and X each denote an oxygen atom,
the tautomers, the diastereomers, the enantiomers, the hydrates, the mixtures thereof and the salts thereof as well as the hydrates of the salts, particularly the physiologically acceptable salts thereof with inorganic or organic acids or bases.

13. CGRP-antagonists of general formula (I) according to claim 1, wherein,
A and X each denote an oxygen atom,
R¹ denotes a 1,3,4,5-tetrahydro-1,3-benzodiazepin-2-on-3-yl, 3,4-dihydro-1*H-*quinazolin-2-on-3-yl, 5-phenyl-2,4-dihydro-1,2,4-triazol-3-on-2-yl, 1,3-dihydro-imidazo[4,5-c]quinolin-2-on-3-yl, 1,3-dihydro-naphth[1,2-*d*]imidazol-2-on-3-yl, 1,3-dihydro-benzimidazol-2-on-3-yl, 4-phenyl-1,3-dihydro-imidazol-2-on-1-yl, 3,4-dihydro-1*H*thieno[3,2-*d*]pyrimidin-2-on-3-yl or 3,4-dihydro-1*H*-thieno[3,4-*d*]pyrimidin-2-on-3-yl group,
and R² and R³ are defined as in claim 1 or 2,
while the heterocycles in the carbon skeleton mentioned hereinbefore under R¹ may additionally be monosubstituted by a methoxy group,
and all the aromatic and heteroaromatic groups and parts of molecules mentioned or contained in the groups defined under R¹ may additionally be mono-, di- or trisubstituted by halogen atoms, by cyano or hydroxy groups and the substituents may be identical or different,
the tautomers, the diastereomers, the enantiomers, the hydrates, the mixtures thereof and the salts thereof as well as the hydrates of the salts, particularly the physiologically acceptable salts thereof with inorganic or organic acids or bases.

14. CGRP-antagonists of general formula (I) according to claim 1, wherein
A and X each denote an oxygen atom,
R¹ is defined as in claim 1,
D and E each denote a methyne group,
G denotes a methyne group substituted by the group R^{a},
M denotes a methyne group substituted by the group R^{b},
Q denotes a methyne group substituted by the group R^{c} and
R^{a}, R^{b} and R^{c} independently of one another each denote a hydrogen or halogen atom, a methyl, difluoromethyl, trifluoromethyl, ethyl, vinyl, ethynyl, cyano, hydroxy, methoxy, difluoromethoxy, trifluoromethoxy, amino, methylamino, or dimethylamino, group,
the tautomers, the diastereomers, the enantiomers, the hydrates, the mixtures thereof and the salts thereof as well as the hydrates of the salts, particularly the physiologically acceptable salts thereof with inorganic or organic acids or bases.

15. CGRP-antagonists of general formula (I) according to claim 1, wherein
A and X each denote an oxygen atom,
D and E each denote a methyne group,
G denotes a methyne group substituted by the group R^{a},
M denotes a methyne group substituted by the group R^{b},
Q denotes a methyne group substituted by the group R^{c},
R^{a}, R^{b} and R^{c} independently of one another each denote a hydrogen or halogen atom, a C₁₋₃-alkyl, trifluoromethyl, cyano, hydroxy, methoxy, trifluoromethoxy, amino, methylamino or dimethylamino group,
R¹ denotes a 1,3,4,5-tetrahydro-1,3-benzodiazepin-2-on-3-yl, 3,4-dihydro-1*H-*quinazolin-2-on-3-yl, 5-phenyl-2,4-dihydro-1,2,4-triazol-3-on-2-yl, 1,3-dihydro-imidazo[4,5-c]quinolin-2-on-3-yl, 1,3-dihydro-naphth[1,2-*d*]imidazol-2-on-3-yl, 1,3-dihydro-benzimidazol-2-on-3-yl, 4-phenyl-1,3-dihydro-imidazol-2-on-1-yl, 3,4-dihydro-1*H*-thieno[3,2-*d*]pyrimidin-2-on-3-yl or 3,4-dihydro-1*H*-thieno[3,4-*d*]pyrimidin-2-on-3-yl group, which is mono-, di- or trisubstituted at an unsaturated carbon atom of the aromatic or heteroaromatic moiety by halogen atoms or by cyano or hydroxy groups and the substituents may be identical or different, but are preferably unsubstituted,
R² and R³ together with the enclosed nitrogen atom denote a group of general formula 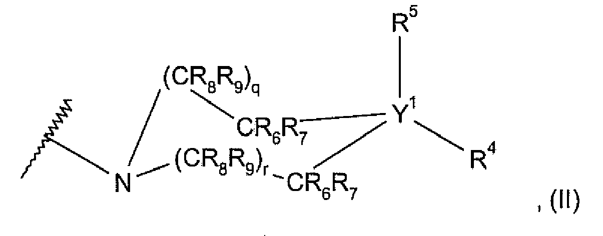 wherein
Y¹ denotes the carbon atom or, if R⁹ denotes a free electron pair, it may also denote the nitrogen atom,
q and r, if Y¹ denotes the carbon atom, denote the numbers 0, 1 or 2, while the total of q and r is 1, 2 or 3,
q and r, if Y¹ denotes the nitrogen atom, denote the numbers 1 or 2, while the total of q and r is 2 or 3,
R⁴ denotes the hydrogen atom, an amino, C₁₋₄-alkyl-amino, di-(C₁₋₄-alkyl)-alkylamino, C₁₋₆-alkyl, a cydo-C₃₋₇-alkyl, amino-C₂₋₇-alkyl, C₁₋₄-alkylamino-C₂₋₇-alkyl, di-(C₁₋₄-alkylamino)-C₂₋₇-alkyl, C₁₋₆-alkoxycarbonyl, C₁₋₆-alkoxycarbonyl-C₁₋₃-alkyl or hydroxycarbonyl-C₁₋₃-alkyl group optionally substituted by a hydroxycarbonyl, C₁₋₆-alkoxycarbonyl, hydroxycarbonyl[-C₁₋₃-alkyl or C₁₋₆-alkoxycarbonyl-C₁₋₃-alkyl group, a phenyl or pyridyl group which may be substituted in each case by a halogen atom, by a C₁₋₃-alkyl, C₁₋₃-alkoxy, amino, C₁₋₄-alkyl-amino or di-(C₁₋₄-alkyl)-amino group,
a heterocycle selected from a 5- to 7-membered azacycloalkyl or S,S-dioxothiaza group and a 6- to 7-membered diazacycloalkyl group,
while the above-mentioned heterocycles are bound to Y¹ in formula (II) via a nitrogen or a carbon atom and
may be substituted by one or two hydroxy, C₁₋₃-alkyl or hydroxy-C₁₋₃-alkyl groups or by a cyclo-C₃₋₆-alkyl, hydroxy-C₃₋₆-cycloalkyl, cyclo-C₃₋₆-alkyl-C₁₋₃-alkyl, C₁₋₃-alkylcarbonyl-C₁₋₃-alkyl, amino, C₁₋₄-alkylamino, di-(C₁₋₄-alkyl)-amino, hydroxycarbonyl-carbonyl, C₁₋₃-alkoxycarbonyl-carbonyl, hydroxycarbonyl-C₁₋₃-alkylcarbonyl, C₁₋₃-alkoxycarbonyl-C₁₋₃-alkylcarbonyl, aminosulphonyl, C₁₋₄-alkylaminosulphonyl, di-(C₁₋₄-alkyl)-aminosulphonyl, cyclo-C₃₋₇-alkylsulphonyl, aminocarbonyl-C₁₋₃-alkyl, C₁₋₄-alkylaminocarbonyl-C₁₋₃-alkyl, di-(C₁₋₄-alkyl)-aminocarbonyl-C₁₋₃-alkyl, hydroxyaminocarbonyl-C₁₋₃-alkyl, C₁₋₃-alkoxyaminocarbonyl-C₁₋₃-alkyl or hydroxy-(C₁₋₃-alkyl)-aminocarbonyl-C₁₋₃-alkyl group,
R⁵ denotes a hydrogen atom or, if Y¹ denotes a nitrogen atom, it may also denote a free electron pair and
R⁶, R⁷, R⁸ and R⁹, which may be identical or different, each denote a hydrogen atom or a C₁₋₃-alkyl group,
the tautomers, the isomers, the diastereomers, the enantiomers, the hydrates, thereof, the mixtures thereof and the salts thereof and the hydrates of the salts, particularly the physiologically acceptable salts thereof with inorganic or organic acids or bases.

16. CGRP-antagonists of general formula (I) according to claim 1, wherein
A and X each denote an oxygen atom,
D and E each denote a methyne group,
G denotes a methyne group substituted by the group R^{a},
M denotes a methyne group substituted by the group R^{b},
Q denotes a methyne group substituted by the group R^{c},
R^{a}, R^{b} and R^{c} independently of one another each denote a hydrogen or halogen atom, a C₁₋₃-alkyl, trifluoromethyl, cyano, hydroxy, methoxy, trifluoromethoxy, amino, methylamino or dimethylamino group,
R¹ denotes a 1,3,4,5-tetrahydro-1,3-benzodiazepin-2-on-3-yl, 3,4-dihydro-1*H-*quinazolin-2-on-3-yl, 5-phenyl-2,4-dihydro-1,2,4-triazol-3-on-2-yl, 1,3-dihydro-imidazo[4,5-c]quinolin-2-on-3-yl, 1,3-dihydro-naphth[1,2-*d*]imidazol-2-on-3-yl, 1,3-dihydro-benzimidazol-2-on-3-yl, 4-phenyl-1,3-dihydro-imidazol-2-on-1-yl, 3,4-dihydro-1*H*-thieno[3,2-*d*]pyrimidin-2-on-3-yl or 3,4-dihydro-1*H*-thieno[3,4-*d*]pyrimidin-2-on-3-yl group, which may be mono-, di- or trisubstituted at an unsaturated carbon atom of the aromatic or heteroaromatic moiety by halogen atoms or by cyano or hydroxy groups and the substituents may be identical or different, but are preferably unsubstituted,
R² and R³ together with the enclosed nitrogen atom denote a group of general formula 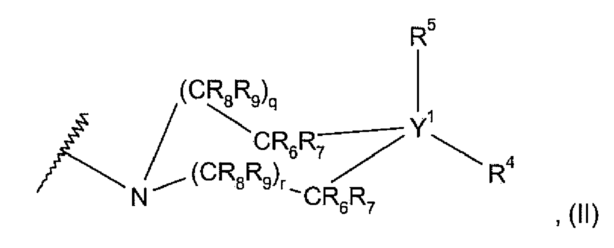 wherein
Y¹ denotes the carbon atom or, if R⁵ denotes a free electron pair, it may also denote the nitrogen atom,
q and r, if Y¹ denotes the carbon atom, denote the numbers 0, 1 or 2, the sum of q and r being 1, 2 or 3,
q and r, if Y¹ denotes the nitrogen atom, denote the numbers 1 or 2, the sum of q and r being 2 or 3,
R⁴ denotes the hydrogen atom, an amino, C₁₋₄-alkyl-amino, di-(C₁₋₄-alkyl)-alkylamino, C₁₋₆-alkyl, cyclo-C₃₋₇-alkyl, amino-C₂₋₇-alkyl, C₁₋₄-alkyl-amino-C₂₋₇-alkyl, di-(C₁₋₄-alkylamino)-C₂₋₇-alkyl, C₁₋₆-alkoxycarbonyl, C₁₋₆-alkoxycarbonyl-C₁₋₃-alkyl or hydroxycarbonyl-C₁₋₃-alkyl group,
a phenyl or pyridyl group which may be substituted in each case by a halogen atom or by a C₁₋₃-alkyl, C₁₋₃-alkoxy, amino, C₁₋₄-alkyl-amino or di-(C₁₋₄-alkyl)-amino group,
a heterocycle selected from a 5- to 7-membered azacycloalkyl group and a 6- to 7-membered diazacycloalkyl group,
while the above-mentioned heterocycles are bound to Y¹ in formula (II) via a nitrogen or carbon atom and
may be substituted by a C₁₋₃-alkyl, cyclo-C₃₋₆-alkyl, cyclo-C₃₋₆-alkyl-C₁₋₃-alkyl, amino, C₁₋₄-alkylamino or di-(C₁₋₄-alkyl)-amino group,
R⁵ denotes a hydrogen atom or, if Y¹ denotes a nitrogen atom, it may also denote a free electron pair and
R⁶, R⁷, R⁸ and R⁹, which may be identical or different, each denote a hydrogen atom or a C₁₋₃-alkyl group,
the tautomers, the diastereomers, the enantiomers, the hydrates, the mixtures thereof and the salts thereof as well as the hydrates of the salts, particularly the physiologically acceptable salts thereof with inorganic or organic acids or bases.

17. The following compounds of general formula I according to claim 1:
| | Structure |
|---|---|
| (1) | 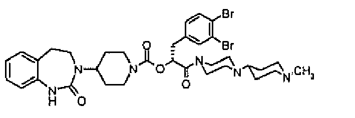 |
| (2) | 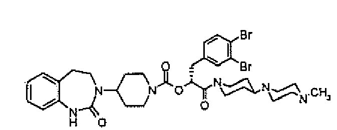 |
| (3) | 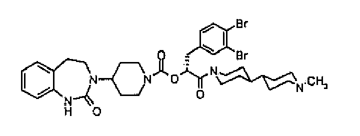 |
| (4) | 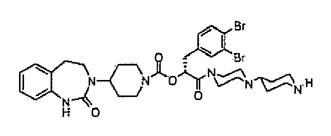 |
| (5) | 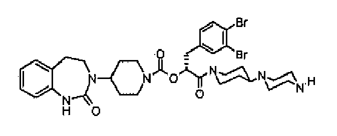 |
| (6) | 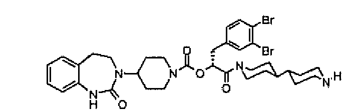 |
| (7) | 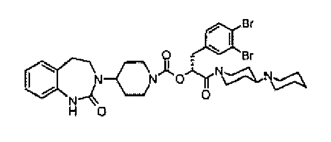 |
| (8) | 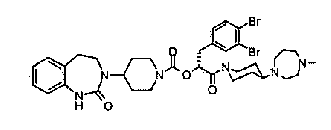 |
| (9) | 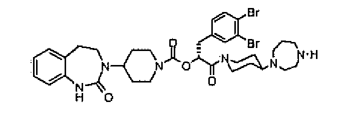 |
| (10) | 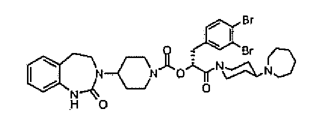 |
| (11) | 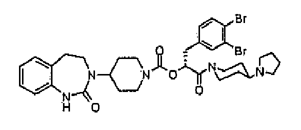 |
| (12) | 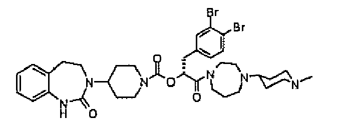 |
| (13) | 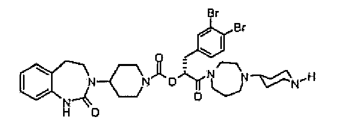 |
| (14) | 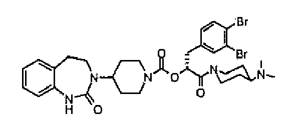 |
| (15) | 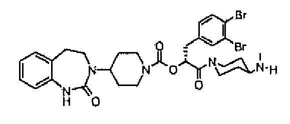 |
| (16) | 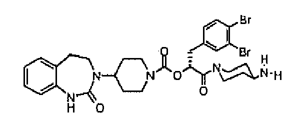 |
| (17) | 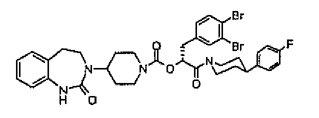 |
| (18) | 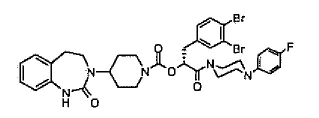 |
| (19) | 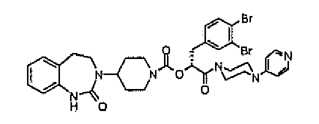 |
| (20) | 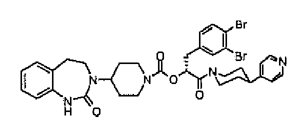 |
| (21) | 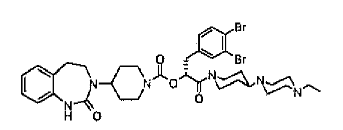 |
| (22) | 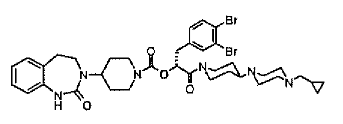 |
| (23) | 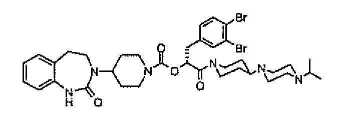 |
| (24) | 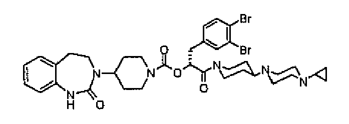 |
| (25) | 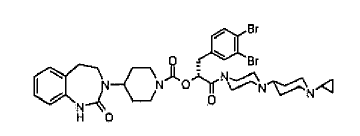 |
| (26) | 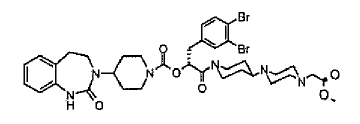 |
| (27) | 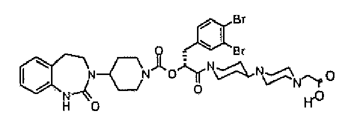 |
| (28) | 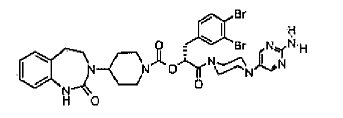 |
| (29) | 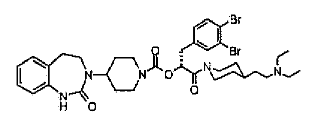 |
| (30) | 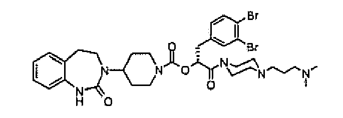 |
| (31) | 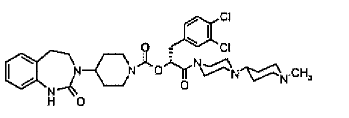 |
| (32) | 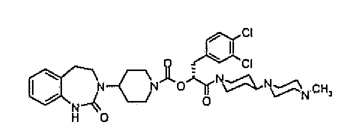 |
| (33) | 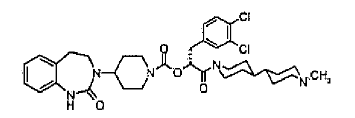 |
| (34) | 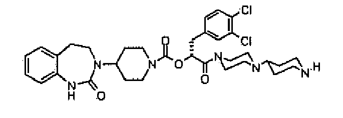 |
| (35) | 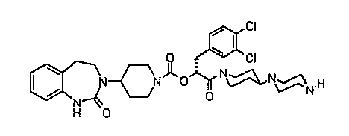 |
| (36) | 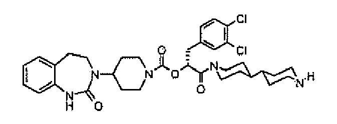 |
| (37) | 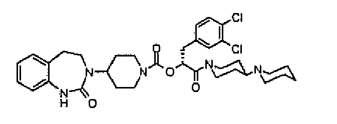 |
| (38) | 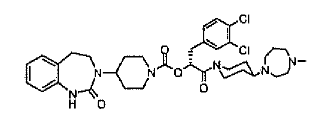 |
| (39) | 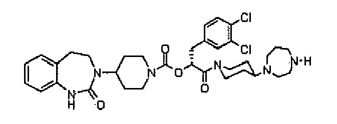 |
| (40) | 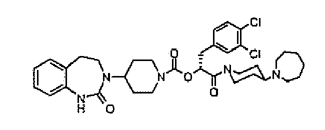 |
| (41) | 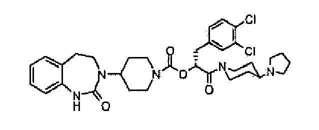 |
| (42) | 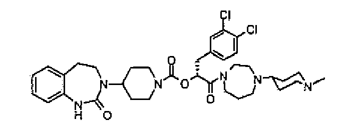 |
| (43) | 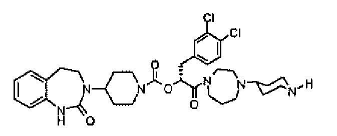 |
| (44) | 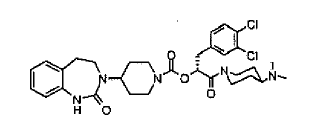 |
| (45) | 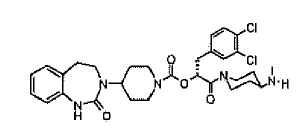 |
| (46) | 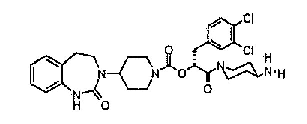 |
| (47) | 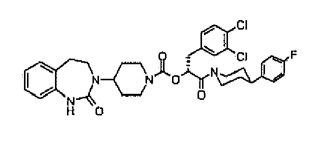 |
| (48) | 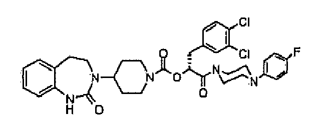 |
| (49) | 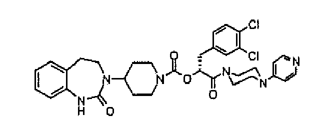 |
| (50) | 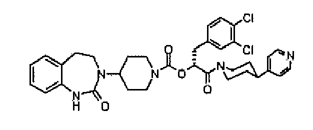 |
| (51) | 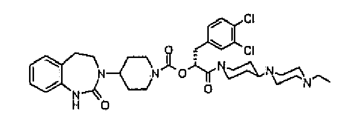 |
| (52) | 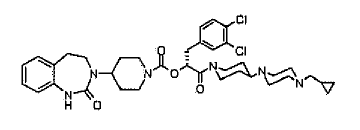 |
| (53) | 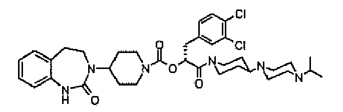 |
| (54) | 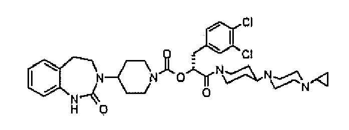 |
| (55) | 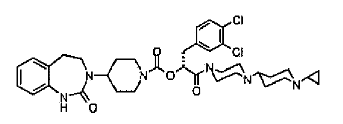 |
| (56) | 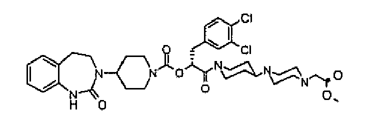 |
| (57) | 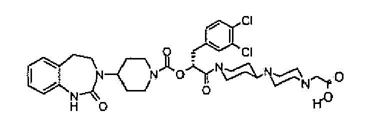 |
| (58) | 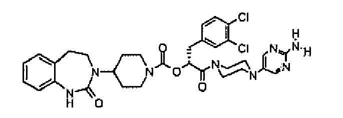 |
| (59) | 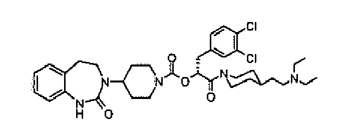 |
| (60) | 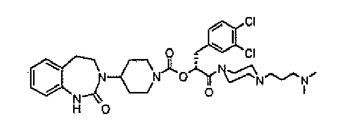 |
| (61) | 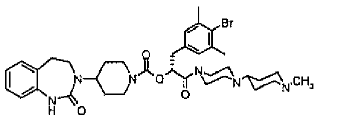 |
| (62) | 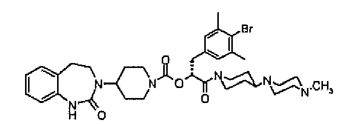 |
| (63) | 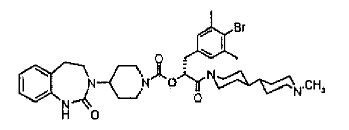 |
| (64) | 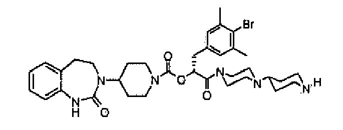 |
| (65) | 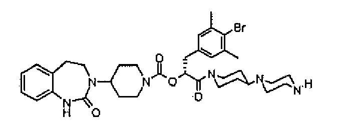 |
| (66) | 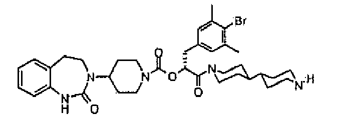 |
| (67) | 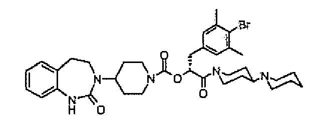 |
| (68) | 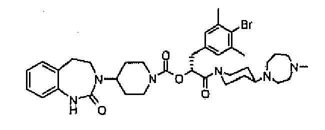 |
| (69) | 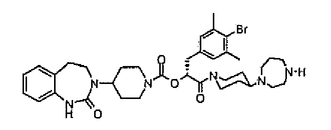 |
| (70) | 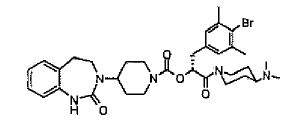 |
| (71) | 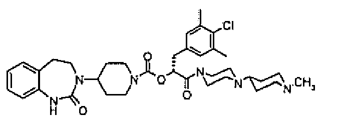 |
| (72) | 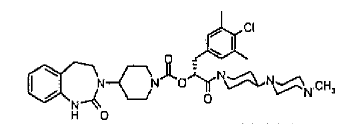 |
| (73) | 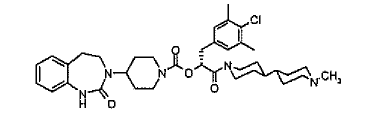 |
| (74) | 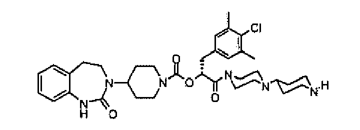 |
| (75) | 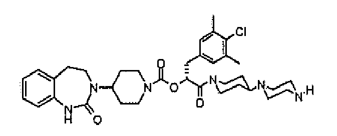 |
| (76) | 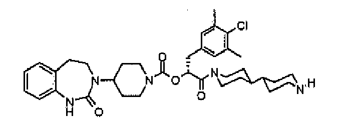 |
| (77) | 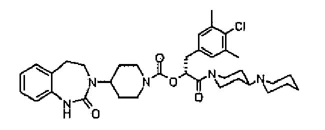 |
| (78) | 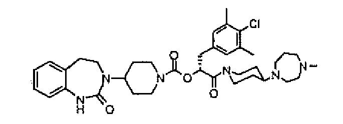 |
| (79) | 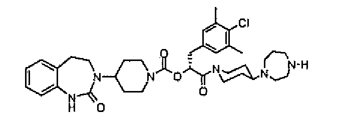 |
| (80) | 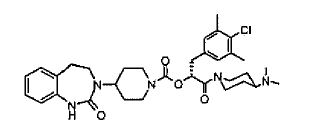 |
| (81) | 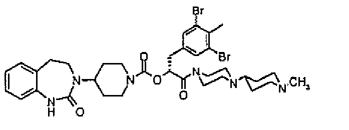 |
| (82) | 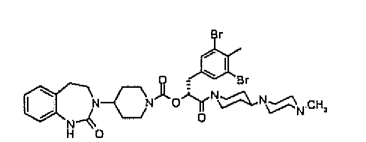 |
| (83) | 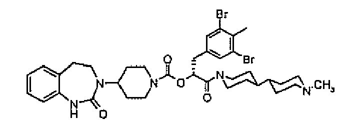 |
| (84) | 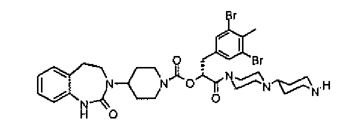 |
| (85) | 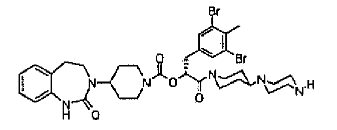 |
| (86) | 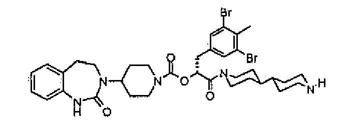 |
| (87) | 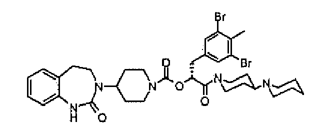 |
| (88) | 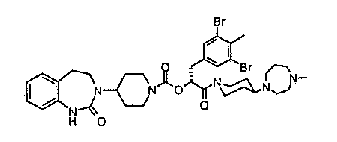 |
| (89) | 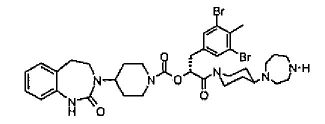 |
| (90) | 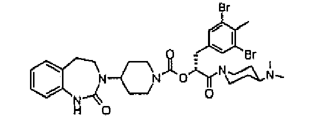 |
| (91) | 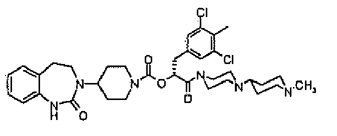 |
| (92) | 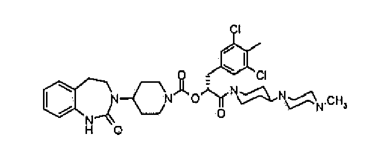 |
| (93) | 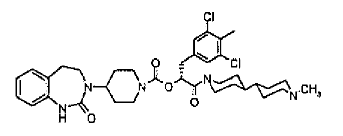 |
| (94) | 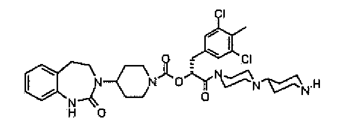 |
| (95) | 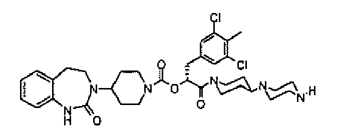 |
| (96) | 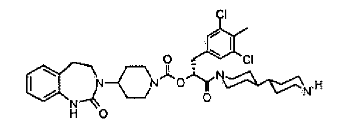 |
| (97) | 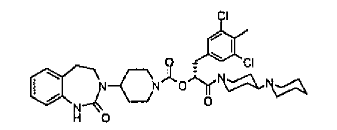 |
| (98) | 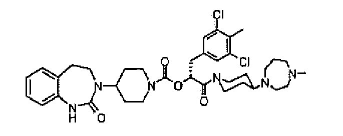 |
| (99) | 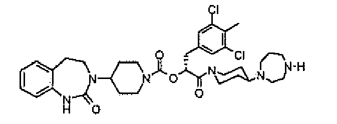 |
| (100) | 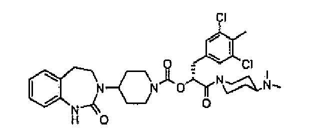 |
| (101) | 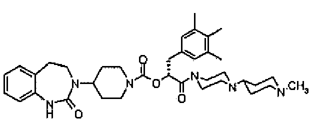 |
| (102) | 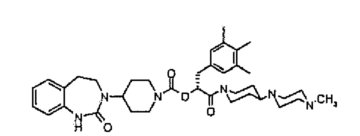 |
| (103) | 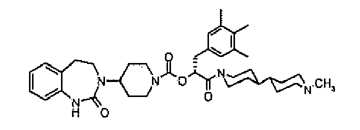 |
| (104) | 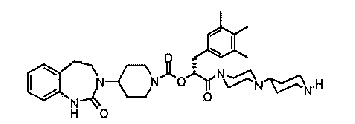 |
| (105) | 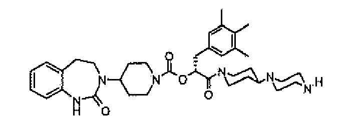 |
| (106) | 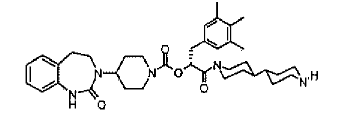 |
| (107) | 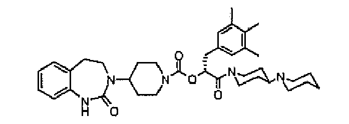 |
| (108) | 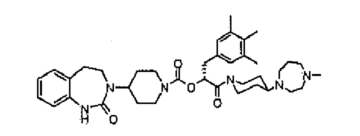 |
| (109) | 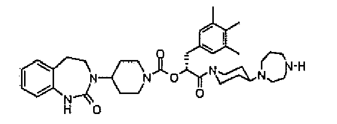 |
| (110) | 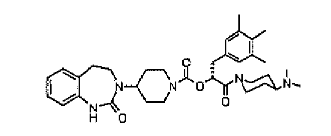 |
| (111) | 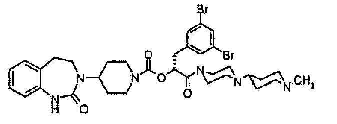 |
| (112) | 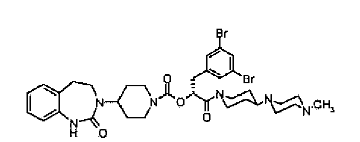 |
| (113) | 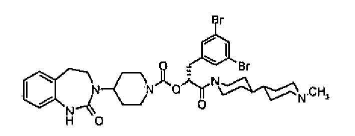 |
| (114) | 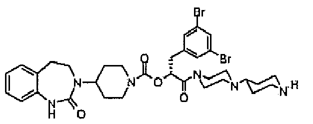 |
| (115) | 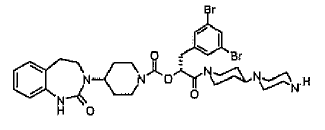 |
| (116) | 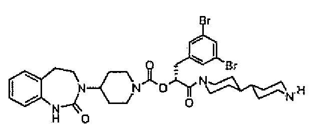 |
| (117) | 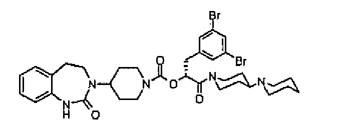 |
| (118) | 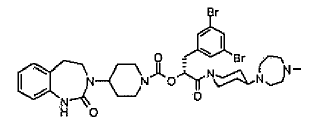 |
| (119) | 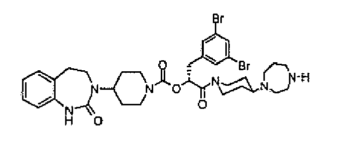 |
| (120) | 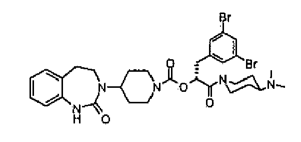 |
| (121) | 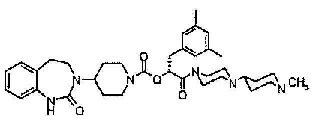 |
| (122) | 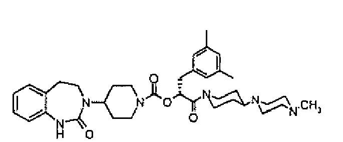 |
| (123) | 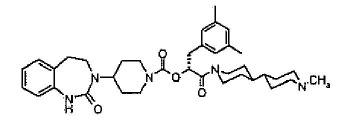 |
| (124) | 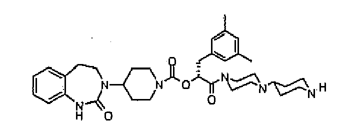 |
| (125) | 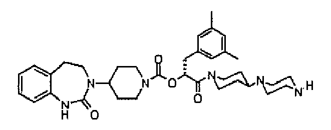 |
| (126) | 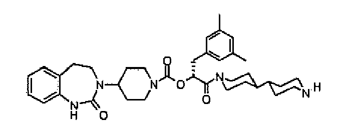 |
| (127) | 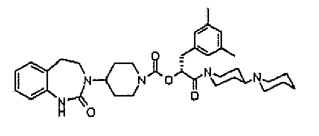 |
| (128) | 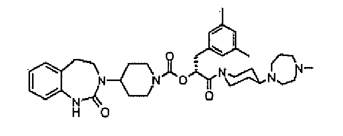 |
| (129) | 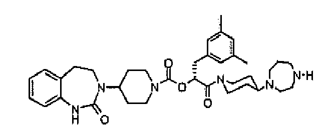 |
| (130) | 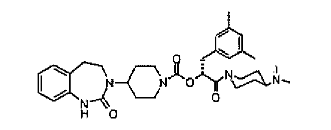 |
| (131) | 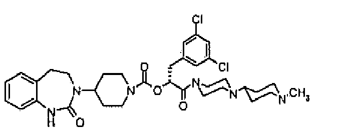 |
| (132) | 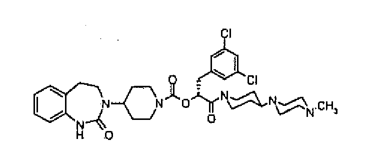 |
| (133) | 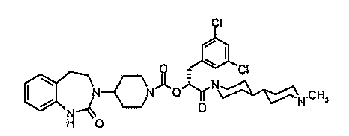 |
| (134) | 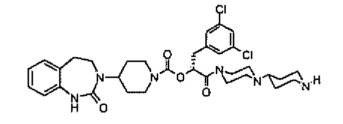 |
| (135) | 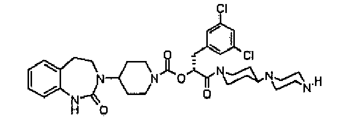 |
| (136) | 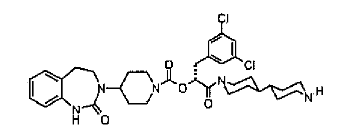 |
| (137) | 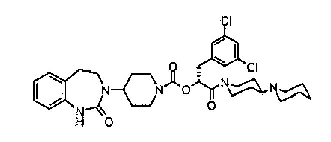 |
| (138) | 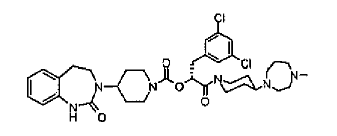 |
| (139) | 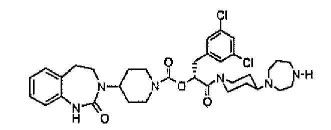 |
| (140) | 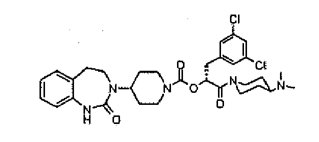 |
| (141) | 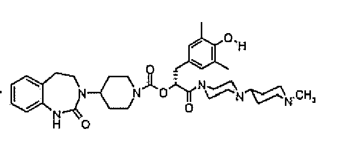 |
| (142) | 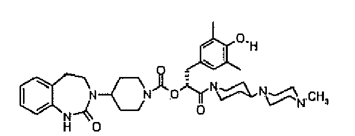 |
| (143) | 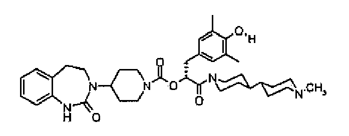 |
| (144) | 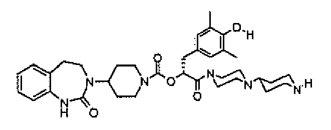 |
| (145) | 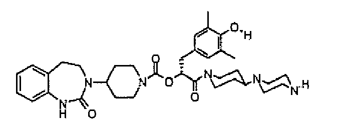 |
| (146) | 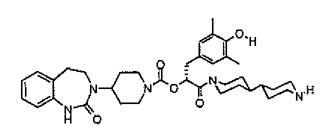 |
| (147) | 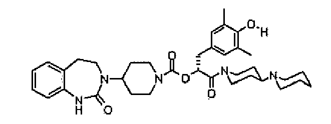 |
| (148) | 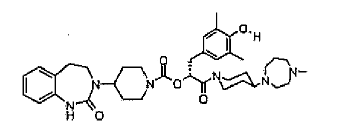 |
| (149) | 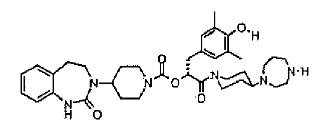 |
| (150) | 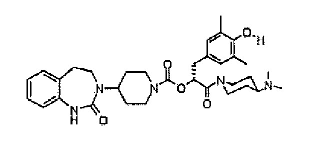 |
| (151) | 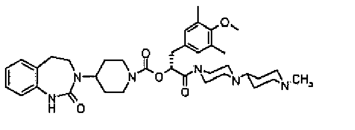 |
| (152) | 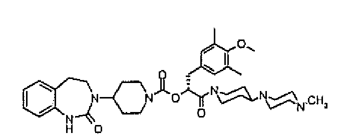 |
| (153) | 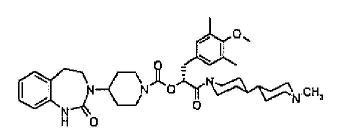 |
| (154) | 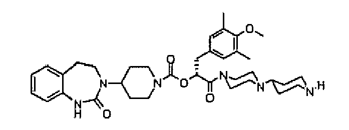 |
| (155) | 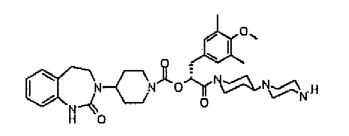 |
| (156) | 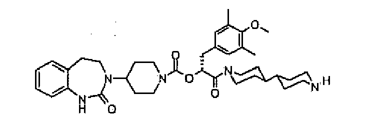 |
| (157) | 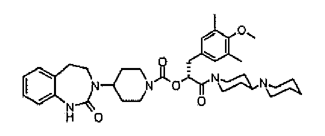 |
| (158) | 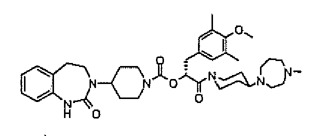 |
| (159) | 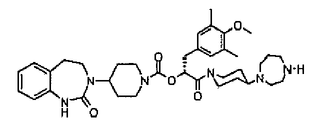 |
| (160) | 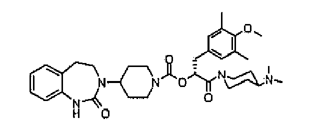 |
| (161) | 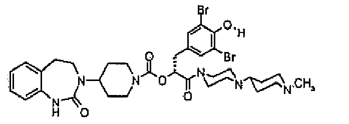 |
| (162) | 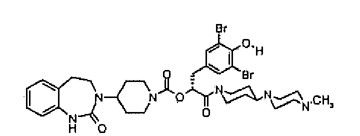 |
| (163) | 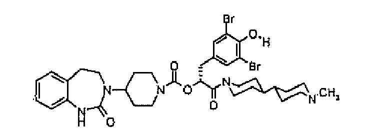 |
| (164) | 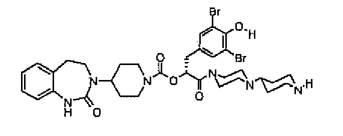 |
| (165) | 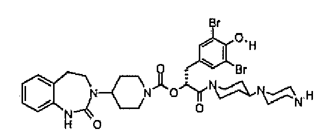 |
| (166) | 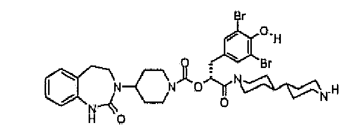 |
| (167) | 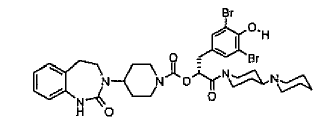 |
| (168) | 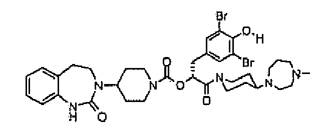 |
| (169) | 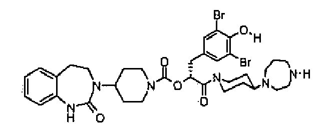 |
| (170) | 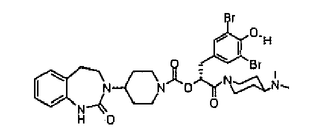 |
| (171) | 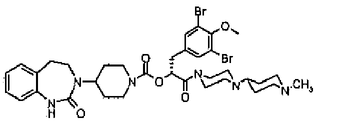 |
| (172) | 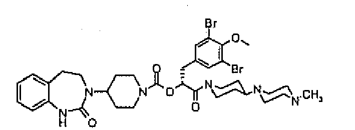 |
| (173) | 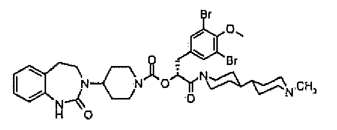 |
| (174) | 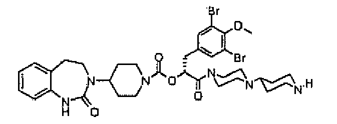 |
| (175) | 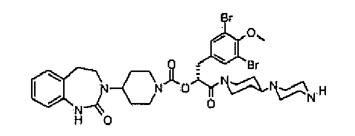 |
| (176) | 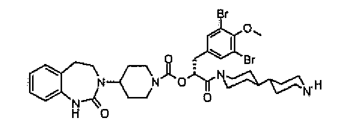 |
| (177) | 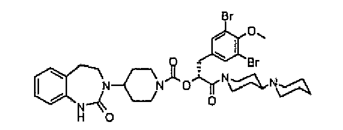 |
| (178) | 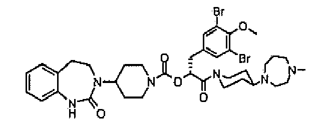 |
| (179) | 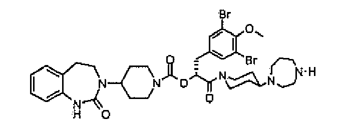 |
| (180) | 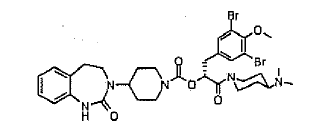 |
| (181) | 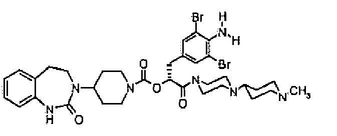 |
| (182) | 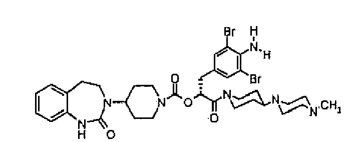 |
| (183) | 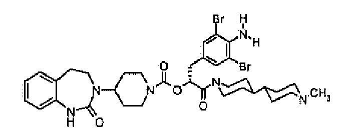 |
| (184) | 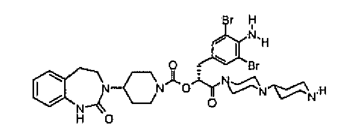 |
| (185) | 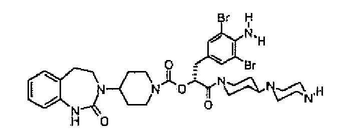 |
| (186) | 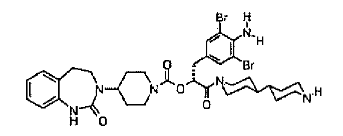 |
| (187) | 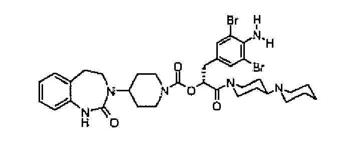 |
| (188) | 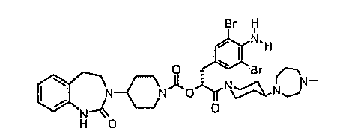 |
| (189) | 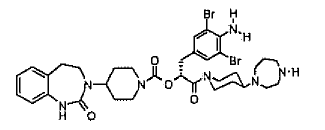 |
| (190) | 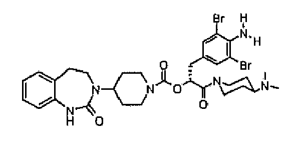 |
| (191) | 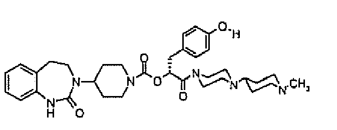 |
| (192) | 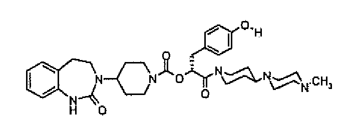 |
| (193) | 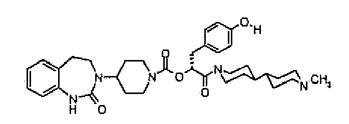 |
| (194) | 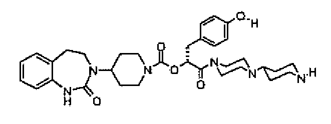 |
| (195) | 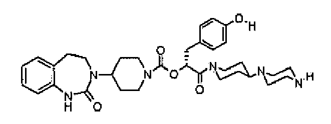 |
| (196) | 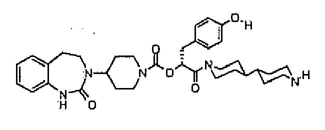 |
| (197) | 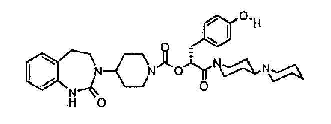 |
| (198) | 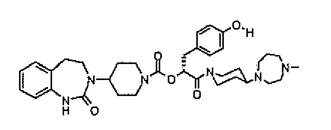 |
| (199) | 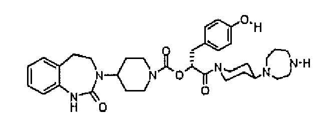 |
| (200) | 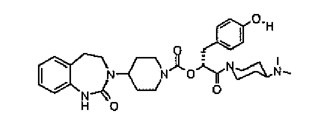 |
| (201) | 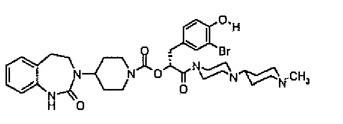 |
| (202) | 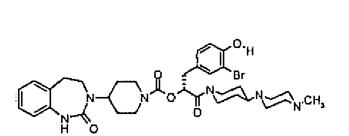 |
| (203) | 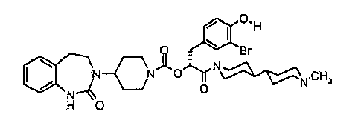 |
| (204) | 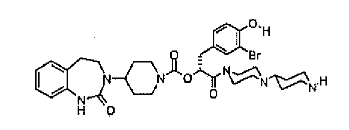 |
| (205) | 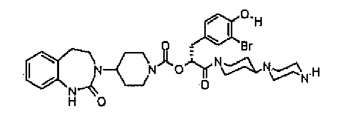 |
| (206) | 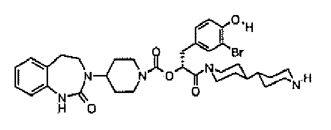 |
| (207) | 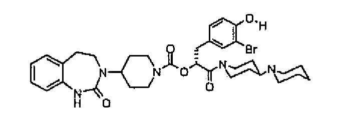 |
| (208) | 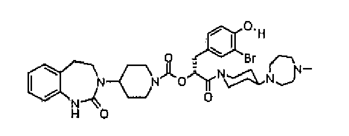 |
| (209) | 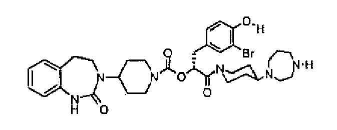 |
| (210) | 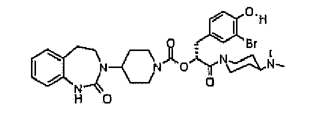 |
| (211) | 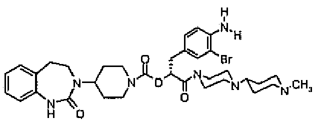 |
| (212) | 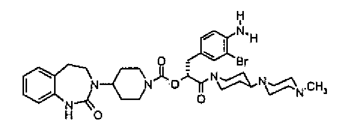 |
| (213) | 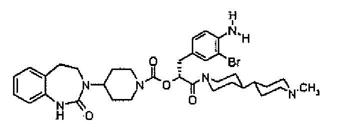 |
| (214) | 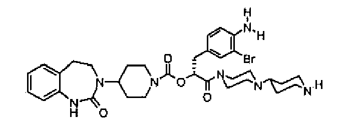 |
| (215) | 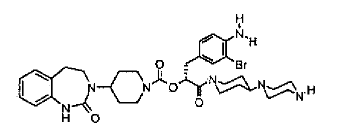 |
| (216) | 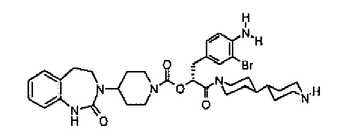 |
| (217) | 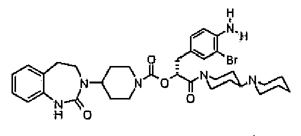 |
| (218) | 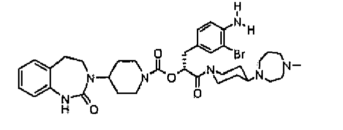 |
| (219) | 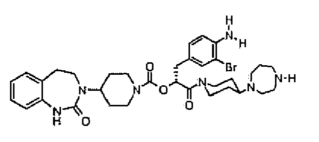 |
| (220) | 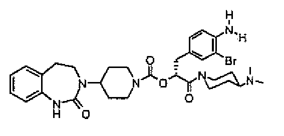 |
| (221) | 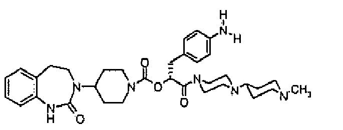 |
| (222) | 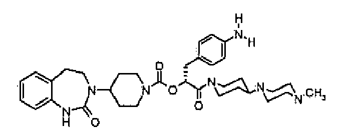 |
| (223) | 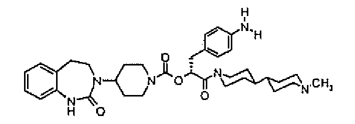 |
| (224) | 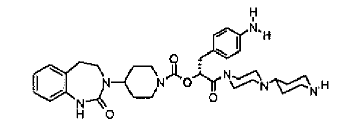 |
| (225) | 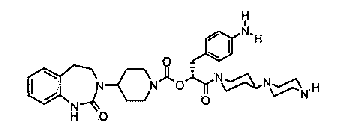 |
| (226) | 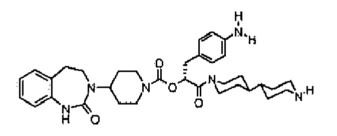 |
| (227) | 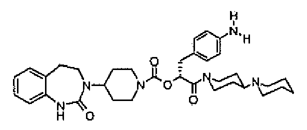 |
| (228) | 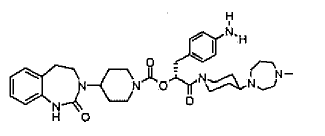 |
| (229) | 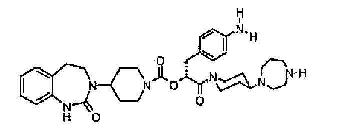 |
| (230) | 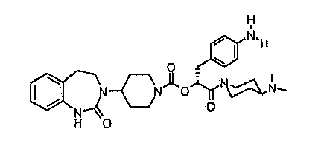 |
| (231) | 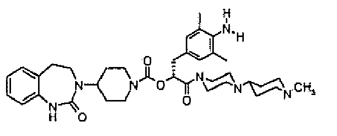 |
| (232) | 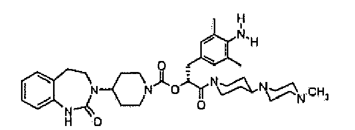 |
| (233) | 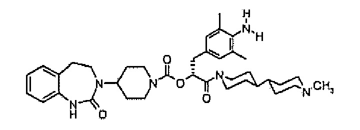 |
| (234) | 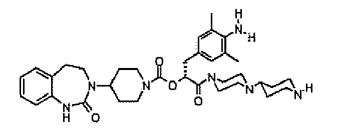 |
| (235) | 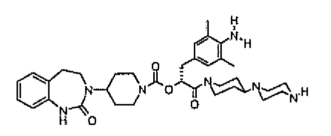 |
| (236) | 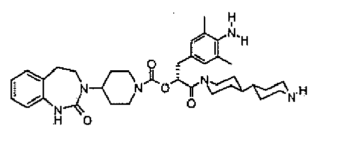 |
| (237) | 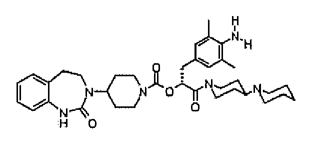 |
| (238) | 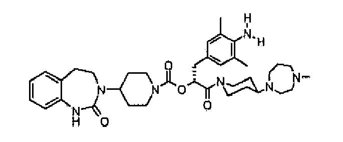 |
| (239) | 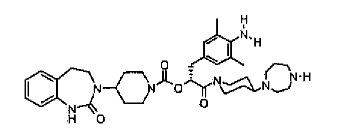 |
| (240) | 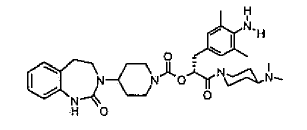 |
| (241) | 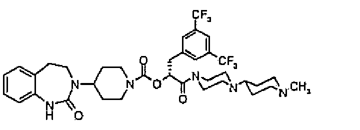 |
| (242) | 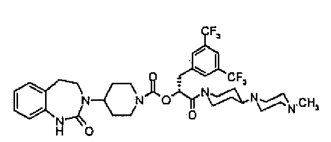 |
| (243) | 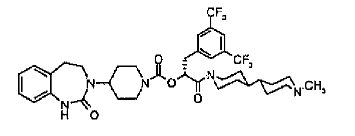 |
| (244) | 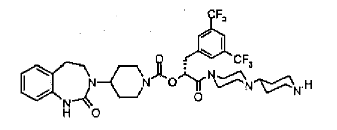 |
| (245) | 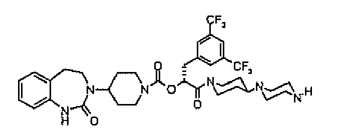 |
| (246) | 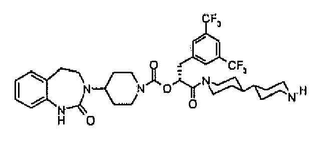 |
| (247) | 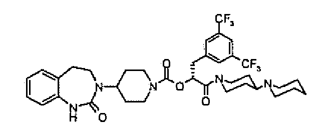 |
| (248) | 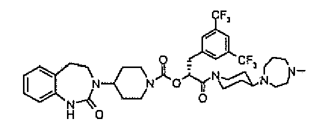 |
| (249) | 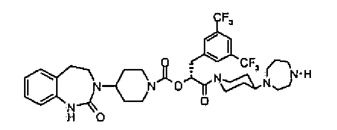 |
| (250) | 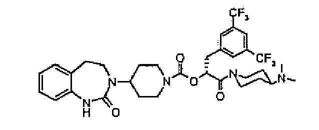 |
| (251) | 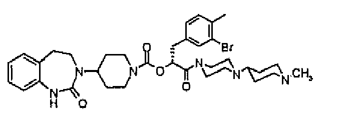 |
| (252) | 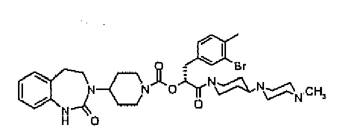 |
| (253) | 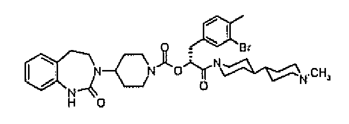 |
| (254) | 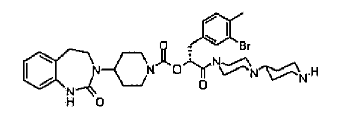 |
| (255) | 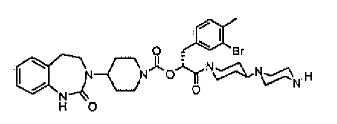 |
| (256) | 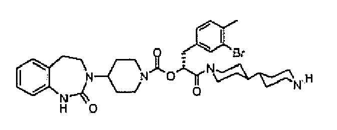 |
| (257) | 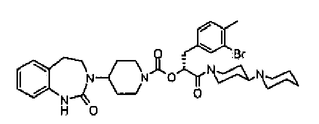 |
| (258) | 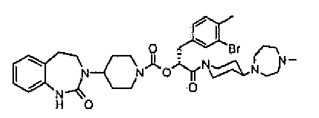 |
| (259) | 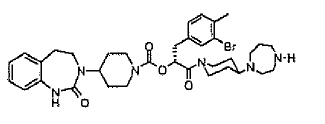 |
| (260) | 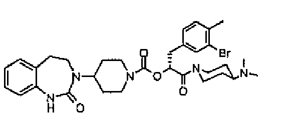 |
| (261) | 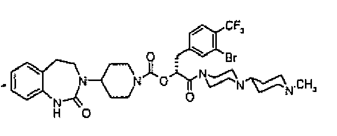 |
| (262) | 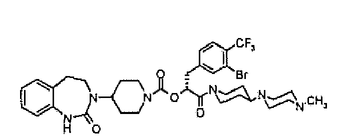 |
| (263) | 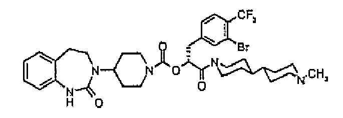 |
| (264) | 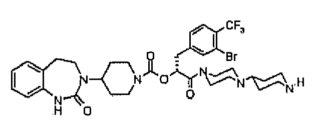 |
| (265) | 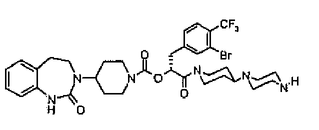 |
| (266) | 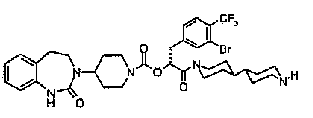 |
| (267) | 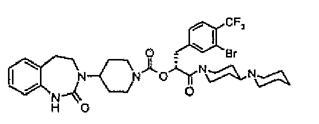 |
| (268) | 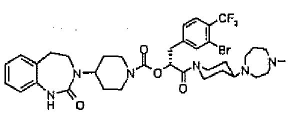 |
| (269) | 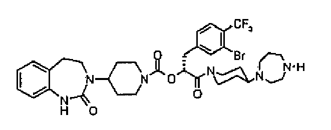 |
| (270) | 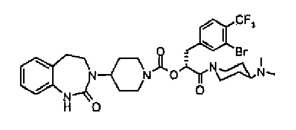 |
| (271) | 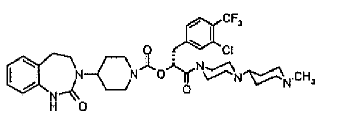 |
| (272) | 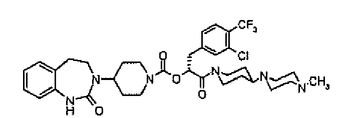 |
| (273) | 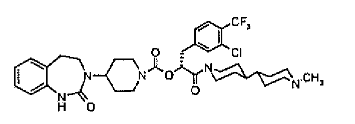 |
| (274) | 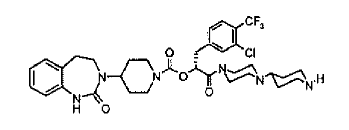 |
| (275) | 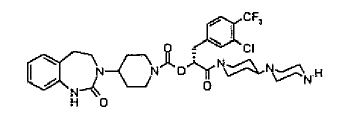 |
| (276) | 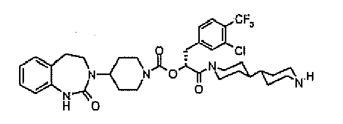 |
| (277) | 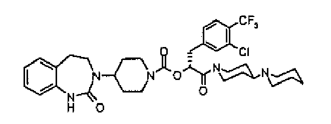 |
| (278) | 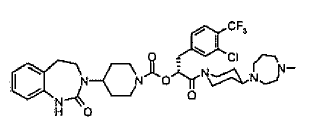 |
| (279) | 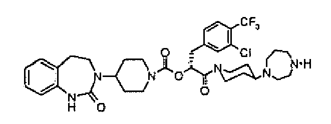 |
| (280) | 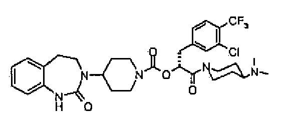 |
| (281) | 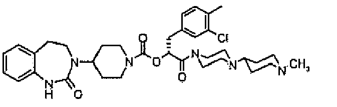 |
| (282) | 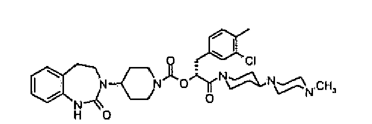 |
| (283) | 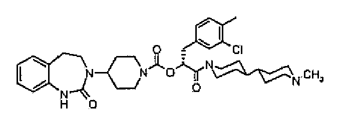 |
| (284) | 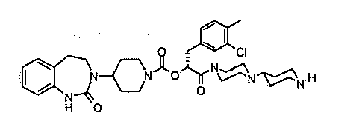 |
| (285) | 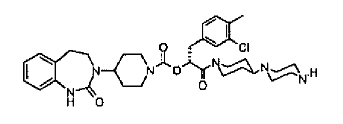 |
| (286) | 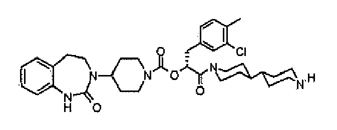 |
| (287) | 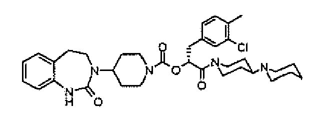 |
| (288) | 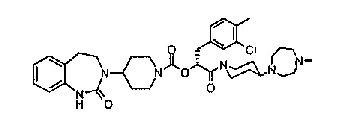 |
| (289) | 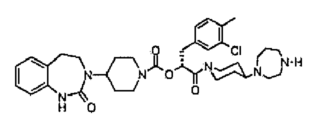 |
| (290) | 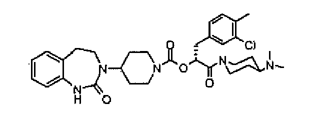 |
| (291) | 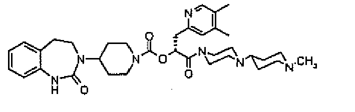 |
| (292) | 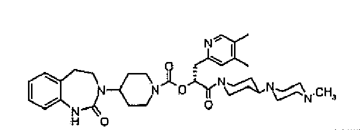 |
| (293) | 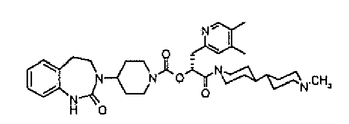 |
| (294) | 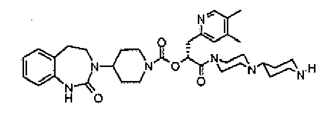 |
| (295) | 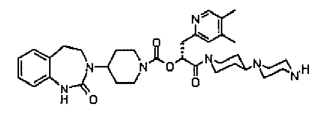 |
| (296) | 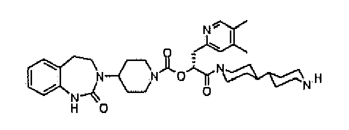 |
| (297) | 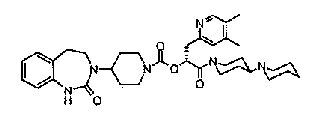 |
| (298) | 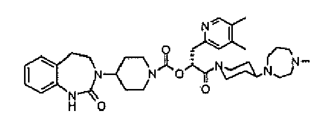 |
| (299) | 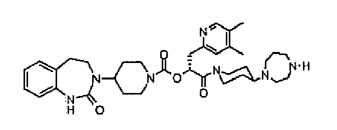 |
| (300) | 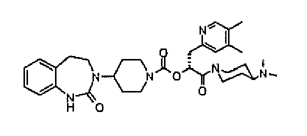 |
| (301) | 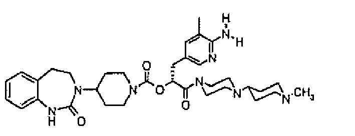 |
| (302) | 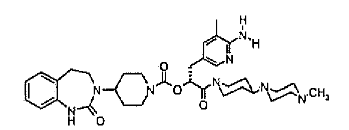 |
| (303) | 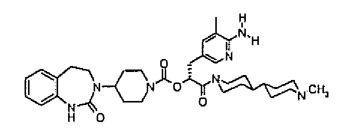 |
| (304) | 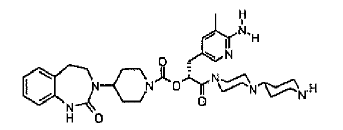 |
| (305) | 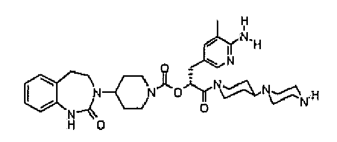 |
| (306) | 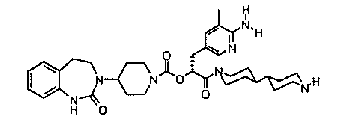 |
| (307) | 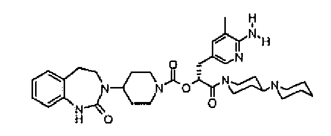 |
| (308) | 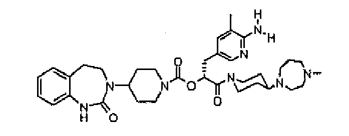 |
| (309) | 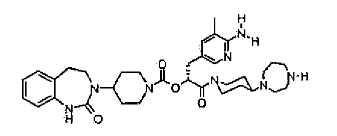 |
| (310) | 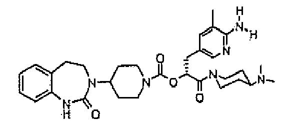 |
| (311) | 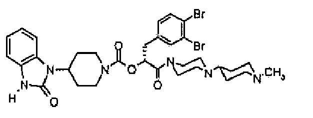 |
| (312) | 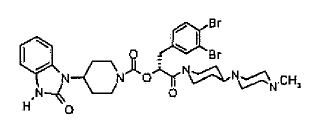 |
| (313) | 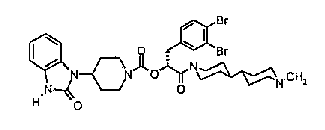 |
| (314) | 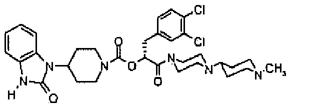 |
| (315) | 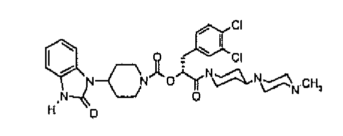 |
| (316) | 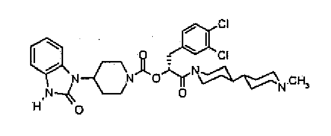 |
| (317) | 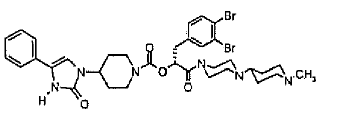 |
| (318) | 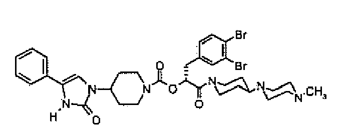 |
| (319) | 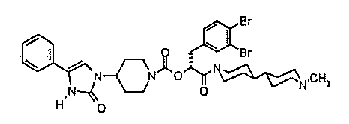 |
| (320) | 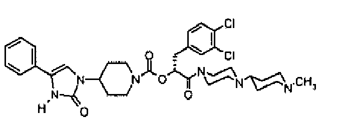 |
| (321) | 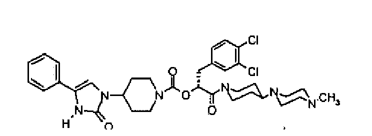 |
| (322) | 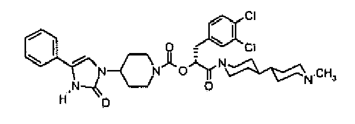 |
| (323) | 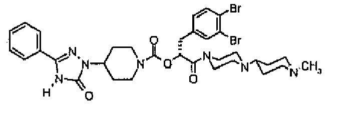 |
| (324) | 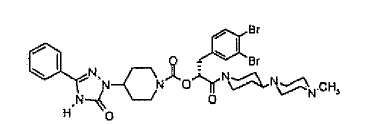 |
| (325) | 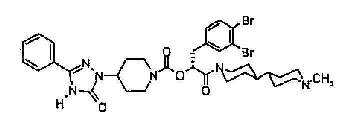 |
| (326) | 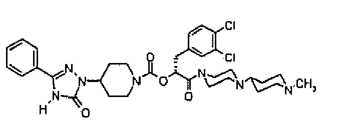 |
| (327) | 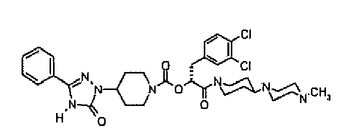 |
| (328) | 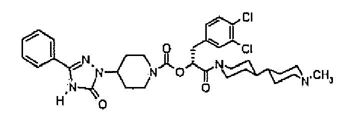 |
| (329) | 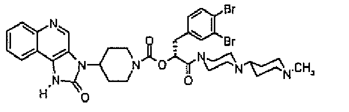 |
| (330) | 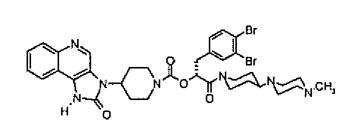 |
| (331) | 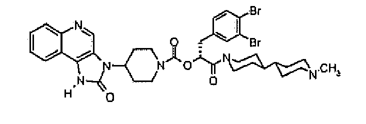 |
| (332) | 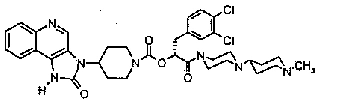 |
| (333) | 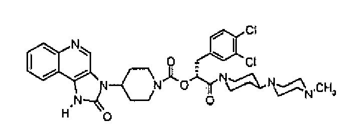 |
| (334) | 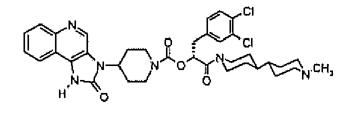 |
| (335) | 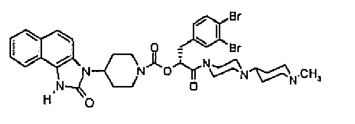 |
| (336) | 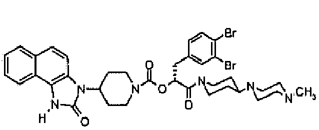 |
| (337) | 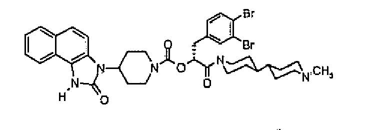 |
| (338) | 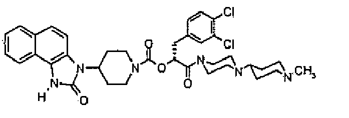 |
| (339) | 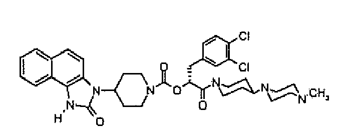 |
| (340) | 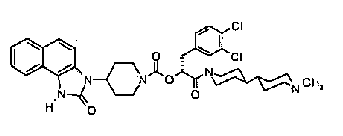 |
| (341) | 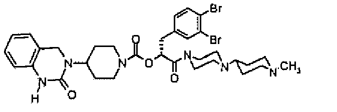 |
| (342) | 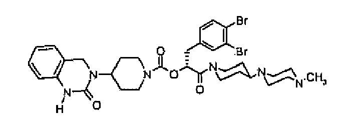 |
| (343) | 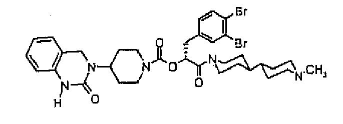 |
| (344) | 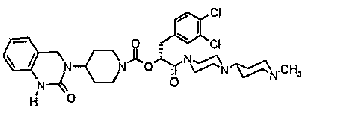 |
| (345) | 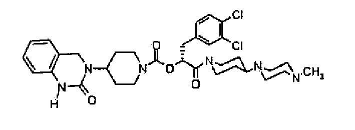 |
| (346) | 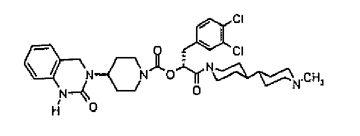 |
| (347) | 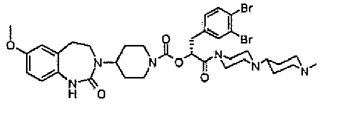 |
| (348) | 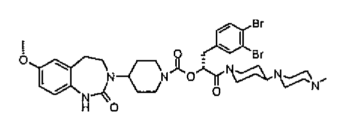 |
| (349) | 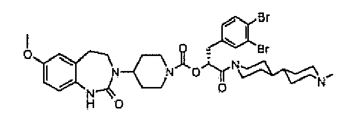 |
| (350) | 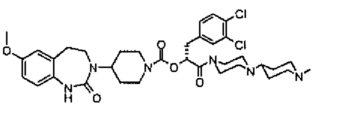 |
| (351) | 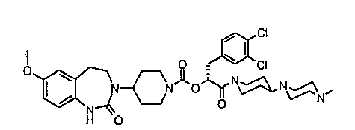 |
| (352) | 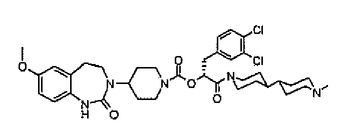 |
| (353) | 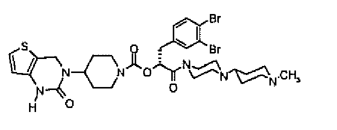 |
| (354) | 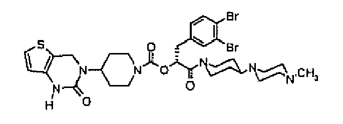 |
| (355) | 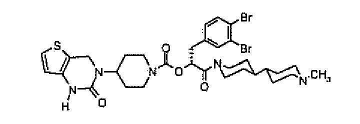 |
| (356) | 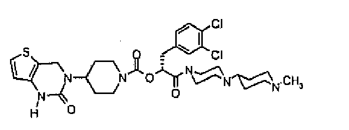 |
| (357) | 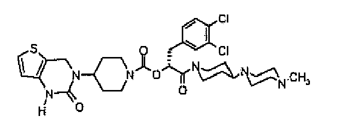 |
| (358) | 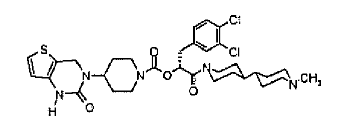 |
| (359) | 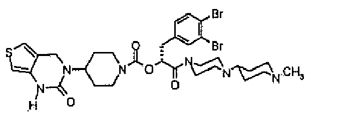 |
| (360) | 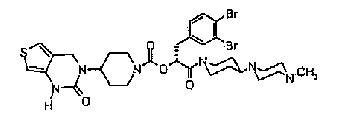 |
| (361) | 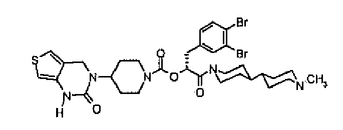 |
| (362) | 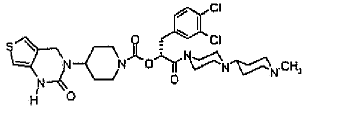 |
| (363) | 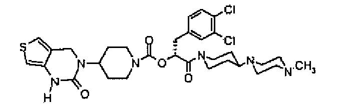 |
| (364) | 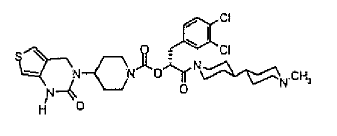 |
the enantiomers, the diastereomers and the salts thereof.

18. Physiologically acceptable salts of the compounds according to one of claims 1 to 17 with inorganic or organic acids or bases.

19. Pharmaceutical compositions containing a compound according to one of claims 1 to 17 or a physiologically acceptable salt according to claim 18 optionally together with one or more inert carriers and/or diluents.

20. Use of a compound according to one of claims 1 to 18 for preparing a pharmaceutical composition for the acute and prophylactic treatment of headaches, particularly migraine or cluster headaches, for the treatment of non-insulin-dependent diabetes mellitus ("NIDDM"), complex regional pain syndrome (CRPS1), cardiovascular diseases, morphine tolerance, diarrhoea caused by clostridium toxin, skin diseases, particularly thermal and radiation-induced skin damage including sunburn, inflammatory diseases, e.g. inflammatory diseases of the joints (arthritis), neurogenic inflammation of the oral mucosa, inflammatory lung diseases, allergic rhinitis, asthma, diseases accompanied by excessive vasodilatation and resultant reduced circulation of the blood, e.g. shock and sepsis, for alleviating pain or for preventive or acute therapeutic treatment of the symptoms of menopausal hot flushes caused by vasodilatation and increased blood flow in oestrogen-deficient women and hormone-treated patients with prostate carcinoma.

21. Process for preparing a pharmaceutical composition according to claim 19, **characterised in that** a compound according to one of claims 1 to 18 is incorporated in one or more inert carriers and/or diluents by a non-chemical method.

22. Process for preparing the compounds of general formula (I) according to one of claims 1 to 18, **characterised in that**
(a) a piperidine of general formula 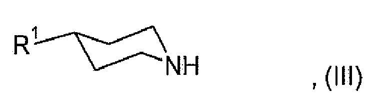 wherein R¹ is defined as in claim 1,
is reacted with a carbonic acid derivative of general formula 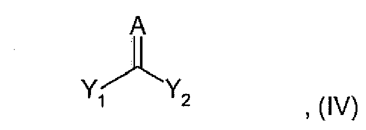 wherein A is defined as in claim 1 and Y₁ and Y₂ denote nucleofugic groups, and with a compound of general formula 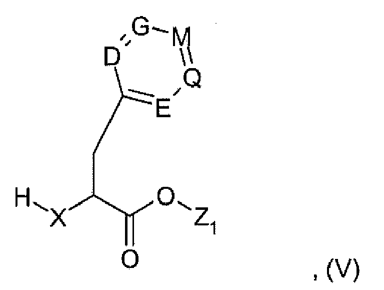 wherein X, D, E, G, M and Q are defined as in claim 1 and Z₁ denotes a protective group for a carboxy group, while before the reaction is carried out any carboxylic acid functions, primary or secondary amino functions or hydroxy functions which may be present in the group R¹ of a compound of formula (III) and/or in a compound of formula (V) are protected, if necessary, by protective groups and any protective groups used are cleaved after the reaction is complete, or
(b) a carboxylic acid of general formula 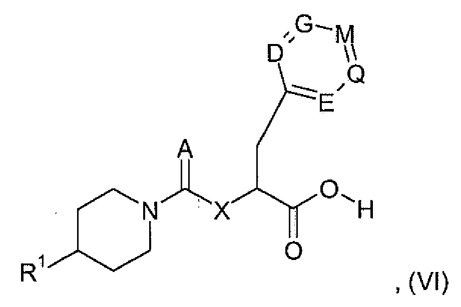 wherein all the groups are defined as in claim 1, is coupled to an amine of general formula HNR²R³, wherein R² and R³ are defined as in claim 1, while before the reaction is carried out any carboxylic acid functions, primary or secondary amino functions or hydroxy functions which may be present in a compound of formula (VI) and/or in the groups R² and R³ of the amine of formula HNR²R³ are protected, if necessary, by protective groups and any protective groups used are cleaved after the reaction is complete, or
(c) a compound of general formula 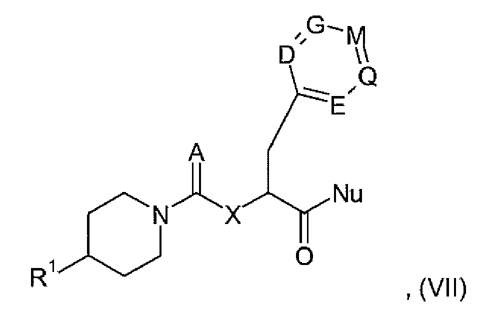 is coupled with an amine of general formula HNR²R³ wherein all the groups are defined as in claim 1 and Nu denotes a leaving group, while before the reaction is carried out any carboxylic acid functions, primary or secondary amino functions or hydroxy functions which may be present in a compound of formula (VII) and/or in the groups R² and R³ of the amine of formula HNR²R³ are protected, if necessary, by protective groups and any protective groups used are cleaved after the reaction is complete,
if desired a compound of general formula (I) thus obtained is resolved into the stereoisomers thereof and / or
a compound of general formula (I) thus obtained is converted into the salts thereof, particularly for pharmaceutical use into the physiologically acceptable salts thereof.

## Revendications

1. Antagonistes CGRP de formule générale 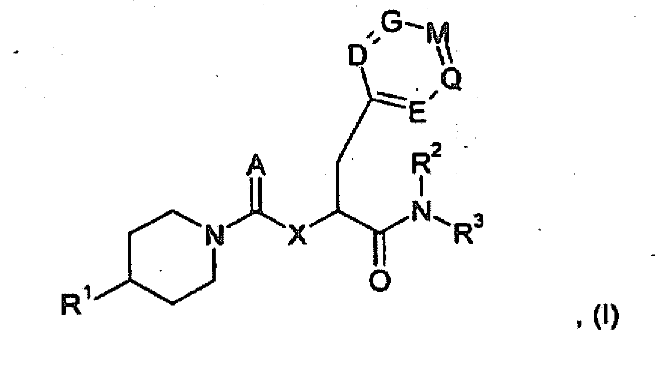 dans laquelle
A est un atome d'oxygène ou un atome de soufre,
X est un atome d'oxygène ou un atome de soufre,
(a) D, E indépendamment l'un de l'autre sont respectivement un groupe méthine ou l'atome d'azote et
G est un groupe méthine substitué par le groupe R^{a},
M est un groupe méthine substitué par le groupe R^{b},
Q est un groupe méthine substitué par le groupe R^{c},
un ou deux des groupes G, M et Q pouvant être également un atome d'azote,
ou
(b) D et E sont respectivement un groupe méthine, l'un des groupes D et E pouvant également désigner un atome d'azote, et
G, M et Q sont respectivement un atome d'azote,
où R^{a}, R^{b} et R^{c} indépendamment les uns des autres sont respectivement un atome d'hydrogène ou d`halogène, un groupe alkyle en C₁ à C₆, alcényle en C₂ à C₆, alcinyle en C₂ à C₆, cyclo-alkyle C₃ à C₇, cyclo-alcényle C₃ à C₇, cyano, hydroxy, hydroxy-alkyle C₁ à C₆, hydroxy-alcényle C₃ à C₆, hydroxy-alcinyle C₃ à C₆, alcoxy en C₁ à C₆, alcoxy C₁ à C₆-alkyle C₁ à C₆, alcoxy en C₁ à C₆-alcényle C₃ à C₆, alcoxy C₁ à C₆-alcinyle C₃ à C₆, alcénoxy C₃ à C₆-alkyle C₁ à C₆, alcénoxy en C₃ à C₆-alcényle en C₃ à C₆, alcénoxy en C₃ à C₆-alcinyle C₃ à C₆, alcinoxy C₃ à C₆-alkyle en C₁ à C₆, alcinoxy C₃ à C₆-alcényle C₃ à C₆, alcinoxy en C₃ à C₆-alcinyle en C₃ à C₆, thiohydroxy, alkylthio en C₁ à C₆, alcénylthio en C₃ à C₆, alcinylthio en C₃ à C₆, amino, alkyle en C₁ à C₆-amino, alcényle en C₃ à C₆-amino, alcinyle en C₃ à C₆-amino, di-(alkyle en C₁ à C₆)-amino, di-(alcényle en C₃ à C₆)-amino, di-(alcinyle en C₃ à C₆)-amino, amino-alkyle en C₁ à C₆, alkyle en C₁ à C₃-amino-alkyle en C₁ à C₆, di(alkyle en C₁ à C₃)-amino-alkyle en C₁ à C₆, amino-alcényle en C₃ à C₆, alkyle en C₁ à C₃-amino-alcényle en C₃ à C₆, di-(alkyle en C₁ à C₃)-amino-alcényle en C₃ à C₆, amino-alcinyle en C₃ à C₆, alkyle en C₁ à C₃-amino-alcinyle en C₃ à C₆, di-(alkyle en C₁ à C₃)-amino-alcinyle en C₃ à C₆, hydroxycarbonyle, phénylcarbonyle, pyridylcarbonyle, alkyle en C₁ à C₆-carbonyle, alcényle en C₂ à C₆-carbonyle, alcinyle en C₂ à C₆-carbonyle, formyle, alcoxy en C₁ à C₆-carbonyle, alcénoxy en C₃ à C₆-carbonyle, alcinoxy en C₃ à C₆-carbonyle, aminocarbonyle, alkyle en C₁ à C₆-aminocarbonyle, alcényle en C₃ à C₆-aminocarbonyle, alcinyle en C₃ à C₆-aminocarbonyle, di-(alkyle en C₁ à C₆)-aminocarbonyle, di-(alcényle en C₃ à C₆)-aminocarbonyle, di-(alcinyle en C₃ à C₆)-aminocarbonyle, formylamino, alkyle en C₁ à C₆-carbonylamino, alcényle en C₂ à C₆-carbonylamino, alcinyle en C₂ à C₆-carbonylamino, formyl-alkyle en C₁ à C₆-amino, formyle-alcényle en C₃ à C₆-amino, formyle-alcinyle en C₃ à C₆-amino, alkyle en C₁ à C₆-carbonyl-alkyle en C₁ à C₆-amino, alcényle en C₂ à C₆-carbonyle-alkyle en C₁ à C₆-amino, alcinyle en C₂ à C₆-carbonyle-alkyle en C₁ à C₆-amino, alkyle en C₁ à C₆-carbonyle-alcényle en C₃ à C₆-amino, alcényle en C₂ à C₆-carbonyl-alcényle en C₃ à C₆-amino, alcinyle en C₂ à C₆-carbnyl-alcényle en C₃ à C₆-amino, alkyle en C₁ à C₆-carbonyl-alcinyle en C₃ à C₆-amino, alcényle en C₂ à C₆-carbonyl-alcinyle en C₃ à C₆-amino, alcényle en C₂ à C₆-carbonyl-alcinyle en C₃ à C₆-amino, alkyle en C₁ à C₆-sulfonyle, alcényle en C₂ à C₆-sulfonyle, alcinyle en C₂ à C₆-sulfonyle, alkyle en C₁ à C₆-sulfinyle, alcényle en C₂ à C₆-sulfinyle, alcinyle en C₂ à C₆-sulfinyle, alkyle en C₁ à C₆-sulfonylamino, alcényle en C₂ à C₆-sulfonylamino, alcinyle en C₂ à C₆-sulfonylamino, alkyle en C₁ à C₆-sulfonyl-alkylamino en C₁ à C₆, alkyle en C₁ à C₆-sulfonyl-alcénylamino en C₃ à C₆, alkyle en C₁ à C₆-sulfonyl-alcinylamino en C₃ à C₆, alcényle en C₂ à C₆-sulfonyl-alkylamino en C₁ à C₆, alcényle en C₂ à C₆-sulfonyl-alcénylamino en C₃ à C₆, alcényle en C₂ à C₆-sulfonyl-alcinylamino en C₃ à C₆, alcinyle en C₂ à C₆-sulfonyl-alkylamino en C₁ à C₆, alcinyle en C₂ à C₆-sulfonyl-alcénylamino en C₃ à C₆, alcinyle en C₂ à C₆-sulfonyl-alcinylamino en C₃ à C₆, aminosulfonyle, alkylaminosulfonyle en C₁ à C₆, di-(alkyle en C₁ à C₆)-aminosulfonyle, alcényle en C₃ à C₆-aminosulfonyle, di-(alcényle en C₃ à C₆)-aminosulfonyle, alcinylaminosulfonyle en C₃ à C₆, ou di-(alcinyle en C₃ à C₆)-aminosulfonyle,
à condition que, dans la mesure où aucun des groupes D, E, G, M et Q ne représente un atome d]azote,
(i) R^{a} n'est pas un atome d'hydrogène si R^{b} et R^{c} désignent respectivement un groupe alkyle en C₁ à C₆,
(ii) R^{c} n'est pas un atome d'hydrogène si R^{a} et R^{b} représentent respectivement un groupe alkyle en C₁ à C₆,
(iii) R^{a} n'est pas un atome d'hydrogène, de fluor, de chlore, de brome ou d'iode ou un groupe difluoro- ou trifluorométhyle si R^{c} est un groupe alkyle en C₁ à C₆, alcényle en C₂ à C₆, ou alcinyle en C₂ à C₆, et R^{b} est un atome de chlore ou de brome, un groupe amino, méthylamino ou hydroxy,
(iv) R^{c} n'est pas un atome d'hydrogène, de fluor, de chlore, de brome ou d'iode ou un groupe difluoro- ou trifluorométhyle, si R^{a} désigne un groupe alkyle en C₁ à C₆, alcényle en C₂ à C₆ ou alcinyle en C₂ à C₆, et R^{b} est un atome de chlore ou de brome, un groupe amino, méthylamino ou hydroxy,
R¹ désigne un groupe 1,3,4,5-tétrahydro-1,3-benzodiazépin-2-on-3-yle, 3,4-dihydro-1H-quinazalin-2-on-3-yle, 5-phényl-2,4-dihydro-1,2,4-triazol-3-on-2-yle, 1,3-dihydro-imidazo[4,5-c]quinolin-2-on-3-yle, 1,3-dihydro-napht[1,2-d]imidazol-2-on-3-yle, 1,3-dihydro-benzimidazol-2-on-3-yle, 4-phényl-1,3-dihydro-imidazol-2-on-1-yle, 3,4-dihydro-1H-thiéno-[3,2-d]pyrimidin-2-on-3-yle ou 3,4-dihydro-1H-thiéno[3,4-d]pyrimidin-2-on-3-yle,
les hétérocycles mentionnés précédemment pour R¹ dans le squelette de carbone pouvant être en outre mono-substitués par un groupe méthoxy,
tous les radicaux aromatiques et hétéroaromatiques mentionnés ou contenus dans les radicaux définis précédemment pour R¹, pouvant en outre être mono-, di ou tri- substitués par des atomes d'halogène, des groupes cyano ou hydroxy et les substituants pouvant être identiques ou différents, et
les liaisons doubles ou triples des groupes alcényle en C₃ à C₆ ou alcinyle en C₃ à C₆ contenus dans les groupes définis précédemment pour R^{a}, R^{b}, R^{c} et R¹ étant isolées des hétéroatomes contenus également éventuellement dans ces groupes,
R² désigne l'atome d'hydrogène,
un groupe phénylméthyle ou un groupe alkyle en C₂ à C₇, qui peut être substitué en position ω par un groupe cyclo-alkyle en C₃ à C₇, cyclo-alcényle en C₃ à C₇, phényle, pyridinyle, diazinyle, hydroxy, amino, alkylamino en C₁ à C₆, di-(alkyle en C₁ à C₆)-amino, alcénylamino en C₃ à C₆, di-(alcényle en C₃ à C₆)amino, alcinylamino en C₃ à C₆, di-(alcinyle en C₃ à C₆)-amino, hydroxycarbonyle, alcoxycarbonyle en C₁ à C₆, aminocarbonyle, aminocarbonylamino, alkylcarbonylamino en C₁ à C₆, alcénylcarbonylamino en C₂ à C₆, alcinylcarbonylamino en C₂ à C₆, 4-morpholinyle, [bis-(2-hydroxyéthyl)]amino, 4-(alkyle en C₁ à C₆)-1-pipérazinyle ou 4-(ω-hydroxy-alkyle en C₂ à C₇)-1-pipérazinyle,
un groupe phényle ou pyridinyle,
les groupes phényle, pyridinyle et diazinyle cités dans les groupes définis précédemment pour R² ou contenus comme substituants étant en outre mono-, di- ou trisubstitués dans le squelette de carbone par un atome d'halogène, un groupe alkyle en C₁ à C₃, alcényle en C₂ à C₃, alcinyle en C₂ à C₃, alcoxy en C₁ à C₃, hydroxy, amino, alkylamino en C₁ à C₃, di-(alkyle en C₁ à C₃)-amino, amino-alkyle en C₁ à C₃, alkyle en C₁ à C₃-amino-alkyle en C₁ à C₃, di-(alkyle en C₁ à C₃)-amino-alkyle en C₁ à C₃, alkylcarbonylamino en C₁ à C₃, alkylcarbonylamino en C₁ à C₃-alkyle en C₁ à C₃, aminocarbonyle, alkyle en C₁ à C₃-aminocarbonyle, di-(alkyle en C₁ à C₃)-aminocarbonyle, cyano, aminosulfonyle, alkyle en C₁ à C₃-aminosulfonyle, di-(alkyle en C₁ à C₃)-aminosulfonyle, alkyle en C₁ à C₃-thio, alkyle en C₁ à C₃-sulfinyle ou alkyle en C₁ à C₃-sulfonyle et les substituants pouvant être identiques ou différents,
R³ désigne l'atome d'hydrogène ou un groupe alkyle en C₁ à C₃ substitué par un groupe phényle ou pyridinyle,
le groupe alkyle en C₁ à C₃ pouvant être lié à un groupe alkyle contenu dans R² ou à un cycle phényle ou pyridyle contenu dans R² en incluant l'atome d'azote, auquel R² et R³ sont liés, en formant un cycle de 4 à 7 chaînons,
ou
R² et R³ conjointement avec l'atome d'azote inclus forment un radical de formule générale 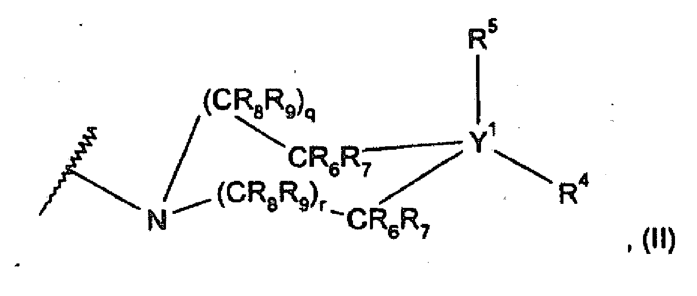 dans laquelle
Y¹ désigne l'atome de carbone ou si R⁵ désigne une paire d'électrons libre, également l'atome d'azote,
q et r, lorsque Y¹ représentent l'atome de carbone, sont les chiffres 0, 1 ou 2 ou
q et r, si Y¹ représente l'atome d'azote, sont les chiffres 1 ou 2,
R⁴ désigne l'atome d'hydrogène, un groupe amino, alkylamino C₁ à C₄, di-(alkyle en C₁ à C₄)-alkylamino, alkyle en C₁ à C₆, un groupe cyclo-alkyle en C₃ à C₇ ou cyclo-alcényle en C₃ à C₇ éventuellement substitué par un groupe hydroxycarbonyle, alcoxycarbonyle en C₁ à C₆, hydroxycarbonyl-alkyle en C₁ à C₃ ou alcoxycarbonyle en C₁ à C₆-alkyle C₁ à C₃, un groupe amino-alkyle en C₂ à C₇, alkyle C₁ à C₄-amino-alkyle en C₂ à C₇, di-(alkylamino en C₁ à C₄), alkyle en C₂ à C₇, amino-iminométhyle, aminocarbonylamino, alkyle C₁ à C₄-aminocarbonylamino, di(alkyle en C₁ à C₄)-aminocarbonylamino, alkyle C₁ à C₄-aminocarbonyl-alkyle en C₁ à C₄-amino, di-(alkyle en C₁ à C₄)-aminocarbonyl-alkyle en C₁ à C₄-amino, phénylaminocarbonylamino, aminocarbonyle, alkyle en C₁ à C₄-aminocarbonyle, di-(alkyle en C₁ à C₄)-aminocarbonyle, aminocarbonyl-alkyle en C₁ à C₃, alkyle en C₁ à C₄-aminocarbonyl-alkyle en C₁ à C₃, di-(alkyle en C₁ à C₄)-aminocarbonyl-alkyle en C₁ à C₃, aminocarbonylamino-alkyle en C₁ à C₃, alcoxycarbonyle en C₁ à C₆, alcénoxycarbonyle en C₃ à C₆, alcinoxycarbonyle en C₃ à C₆, alcoxycarbonyle en C₁ à C₆-alkyle en C₁ à C₃, alcénoxycarbonyle en C₁ à C₆-alkyle en C₁ à C₃, alcinoxycarbonyle en C₁ à C₆-alkyle en C₁ à C₃, ou hydroxycarbonyl-alkyle en C₁ à C₃,
un groupe phényle, pyridinyle, diazinyle, 1-naphtyle, 2-naphtyle, pyridinylcarbonyle ou phénylcarbonyle qui peut être mono-, di- ou trisubstitués dans le squelette de carbone par un atome d'halogène, un groupe alkyle en C₁ à C₃, alcényle en C₂ à C₃, alcinyle en C₂ à C₃, alcoxy en C₁ à C₃, hydroxy, amino, alkylamino en C₁ à C₄, di-(alkyle en C₁ à C₄)-amino, amino-alkyle en C₁ à C₃, alkyle en C₁ à C₄-amino-alkyle en C₁ à C₃, di-(alkyle en C₁ à C₄)-amino-alkyle en C₁ à C₃, alkylcarbonylamino en C₁ à C₄, alkylcarbonylamino en C₁ à C₄-alkyle en C₁ à C₃, aminocarbonyle, alkyle en C₁ à C₄-aminocarbonyle, di-(alkyle en C₁ à C₄)-aminocarbonyle, cyano, aminosulfonyle, alkyle en C₁ à C₄-aminosulfonyle, di(alkyle en C₁ à C₄)-aminosulfonyle, alkyle en C₁ à C₄-thio, alkyle en C₁ à C₄-sulfinyle ou alkyle C₁ à C₄ et les substituants peuvent être identiques ou différents,
un hétérocycle choisi dans un groupe azacycloalkyle de 4 à 10 chaînons, un groupe oxaza, thiaza, S,S-dioxothiaza et diazacycloalkyle de 6 à 10 chaînons et un groupe azabicycloalkyle de 6 à 10 chaînons,
un groupe 1-alkyl-4-pipéridinylcarbonyle ou 4-alkyl-1-pipérazinylcarbonyle,
les hétérocycles mono- et bicycliques précédemment cités étant liés par un atome d'azote ou un atome de carbone à Y¹ dans la formule (II),
dans les hétérocycles mono- et bicycliques précédemment cités, un groupe méthine relié de façon indirecte avec un atome d'azote, d'oxygène ou de soufre, pouvant être substitué par un atome de fluor et un groupe méthylène relié de façon indirecte avec un atome d'azote, d'oxygène ou de soufre, par un ou deux atomes de fluor,
les hétérocycles mono- et bicycliques précédemment cités et les groupes 1-(alkyle en C₁ à C₆)-4-pipéridinylcarbonyle et 4-(alkyle en C₁ à C₆)-1-pipérazinylcarbonyle pouvant être substitués une à quatre fois dans le cycle par un groupe hydroxy, alkyle en C₁ à C₆ ou hydroxy-alkyle en C₁ à C₃, ou éventuellement, en outre mono-substitué par un groupe cyclo-alkyle en C₃ à C₇, hydroxy-cycloalkyle en C₃ à C₇, cyclo-alcényle en C₃ à C₇, cyclo-alkyle en C₃ à C₇-alkyle en C₁ à C₃, phényl-alkyle en C₁ à C₃, pyridyl-alkyle en C₁ à C₃, alkylcarbonyle en C₁ à C₆, alkylcarbonyle en C₁ à C₆-alkyle en C₁ à C₃, hydroxy, alcoxy en C₁ à C₆, amino, alkylamino en C₁ à C₄, di-(alkyle en C₁ à C₄)-amino, phénylcarbonyle, pyridinylcarbonyle, alcoxycarbonyle en C₁ à C₆, hydroxycarbonyl-carbonyle, alcoxycarbonyle en C₁ à C₆-carbonyle, hydroxycarbonyl-alkyle en C₁ à C₃, alcoxycarbonyle en C₁ à C₆-alkyle en C₁ à C₃, hydroxycarbonyl-alkylcarbonyle en C₁ à C₃, alcoxycarbonyle en C₁ à C₆-alkylcarbonyle en C₁ à C₃, aminocarbonyle, alkylaminocarbonyle en C₁ à C₄, di-(alkyle n C₁ à C₄)-aminocarbonyle, aminosulfonyle, alkylaminosulfonyle en C₁ à C₄, di-(alkyle en C₁ à C₄)aminosulfonyle, alkylsulfonyle en C₁ à C₃, cyclo-alkylsulfonyle en C₃ à C₇, aminocarbonyl-alkyle en C₁ à C₃, alkylaminocarbonyle en C₁ à C₄-alkyle en C₁ à C₃, di-(alkyle en C₁ à C₄)aminocarbonyl-alkyle en C₁ à C₃, hydroxyaminocarbonyl-alkyle en C₁ à C₃, alcoxyaminocarbonyle en C₁ à C₃-alkyle en C₁ à C₃, ou hydroxy-(alkyle en C₁ à C₃)-aminocarbonyl-alkyle en C₁ à C₃, par un groupe cyclo-alkylcarbonyle en C₃ à C₇, azacyclo-alkylcarbonyle en C₄ à C₇, diazacyclo-alkylacarbonyle en C₅ à C₇, ou oxazacyclo-alkylcarbonyle en C₅ à C₇ éventuellement alkyl-substitués en C₁ à C₃, dans le cycle, les substituants étant identiques ou différents et pouvant être liés sur un atome de carbone du cycle ou un atome d'azote du cycle,
les radicaux phényle et pyridinyle contenus dans les radicaux définis précédemment pour R⁴ pouvant être mono- di ou trisubstitués de leurs côtés par des atomes d'halogène, par un groupe alkyle en C₁ à C₃, alcényle en C₂ à C₃, alcinyle en C₂ à C₃, alcoxy en C₁ à C₃, hydroxy, amino, alkylamino en C₁ à C₄, di-(alkyle en C₁ à C₄)-amino, amino-alkyle en C₁ à C₃, alkyle en C₁ à C₄-aminoalkyle en C₁ à C₃, di-(alkyle en C₁ à C₄)-amino-alkyle en C₁ à C₃, alkylcarbonylamino en C₁ à C₄, alkylcarbonylamino en C₁ à C₄-alkyle en C₁ à C₃, aminocarbonyle, alkyle en C₁ à C₃-aminocarbonyle, di-alkyle en C₁ à C₄-aminocarbonyle, cyano, aminosulfonyl-alkyle en C₁ à C₄-aminosulfonyle, di-(alkyle en C₁ à C₄)-aminosulfonyle, alkylthio en C₁ à C₃, alkyle en C₁ à C₃-sulfinyle ou alkyle en C₁ à C₃-sulfonyle, les substituants pouvant être identiques ou différents,
ou également, si Y¹ désigne l'atome de carbone, le groupe hydroxycarbonyle, aminométhyle, alkyle en C₁ à C₄-aminométhyle ou di-(alkyle en C₁ à C₄)-aminométhyle,
R⁵ est un atome d'hydrogène ou un groupe hydroxy,
un radical alkyle en C₁ à C₄, un radical alkyle non ramifié pouvant être substitué en position ω par un groupe phényle, pyridinyle, diazinyle, amino, alkylamino en C₁ à C₄, di-(alkyle en C₁ à C₄)-amino, 4-allyle en C₁ à C₄-1-pipérazinyle ou 4-morpholinyle,
un groupe alcoxycarbonyle en C₁ à C₆, cyano ou aminocarbonyle ou également, si Y¹ désigne un atome d'azote, une paire d'électrons libres,
ou si Y¹ désigne l'atome de carbone, également l'atome de fluor, ou
R⁴ conjointement avec R⁵ et Y¹ désignent un cycle cycloaliphatique de 4 à 7 chaînons dans lequel un groupe méthylène peut être substitué par un groupe NH-, -N(alkyle en C₁ à C₄)-, -N(alcényle en C₃ à C₄)-, -N(alcinyle en C₃ à C₄), -N(cyclo-alkyle en C₃ à C₇)-, -N(cycloalkyle en C₃ à C₇-alkyle en C₁ à C₃) -N(hydraxycarbonyl-alkyle en C₁ à C₃)- ou -N(alcoxycarbonyle en C₁ à C₆-alkyle en C₁ à C₃),
un atome d'hydrogène lié à un atome d'azote dans l'un des groupes définis précédemment pour R⁴ pouvant être substitué par un radical protecteur,
R⁶ et R⁷ qui peuvent être identiques ou différents, représentent respectivement un atome d'hydrogène, un groupe alkyle en C₁ à C₄ ou également, si Y¹ représente un atome de carbone, l'atome de fluor, un groupe amino, alkylamino en C₁ à C₄, ou di-(alkyle en C₁ à C₄)-amino, les deux groupes alkyle en C₁ à C₄ pouvant être liés l'un à l'autre en formant un cycle, et
R⁸ et R⁹ qui peuvent être identiques ou différents, représentent respectivement un atome d'hydrogène ou un groupe alkyle en C₁ à C₃,
où sauf indication contraire, tous les groupes alkyle, alcényle et alcinyle mentionnés ou contenus dans les radicaux définis précédemment peuvent être linéaires ou ramifiés, chaque groupe méthine contenu dans les radicaux définis précédemment pouvant être substitué par un atome de fluor, chaque groupe méthylène pouvant être substitué par jusqu'à 2 atomes de fluor et chaque groupe méthyle pouvant être substitué par jusqu'à 3 atomes de fluor et deux groupes alkyle et alcényle liés à un atome d'azote pouvant être liés l'un à l'autre en formant un cycle hétérocyclique saturé ou insaturé de 4 à 7 chaînons,
tous les radicaux aromatiques ou hétéroaromatiques mentionnés ou contenus dans les radicaux définis précédemment pouvant être mono-, di ou trisubstitués en outre par un atome d'halogène, un groupe cyano ou hydroxy et les substituants pouvant être identiques ou différents, et
désignent, parmi les radicaux protecteurs cités dans les définitions précédentes et suivantes, les groupes protecteurs courants de la chimie des peptides, en particulier un groupe phénylalcoxycarbonyle substitué dans le noyau phényle par un atome d'halogène, par un groupe nitro ou phényle, par un ou deux groupes méthoxy, comportant 1 à 3 atomes de carbone dans la partie alcoxy,
par exemple, les groupes benzyloxycarbonyle, 2-nitrobenzyloxycarbonyle, 4-nitrobenzyloxycarbonyle, 4-méthoxy-benzyloxycarbonyle, 2-chloro-benzyloxycarbonyle, 3-chloro-benzyloxycarbonyle, 4-chlara-benzyloxycarbonyle, 4-biphénylyl-α,α-diméthyl-benzyloxycarbonyle ou 3,5-diméthoxy-α,α-diméthyl-benzyloxycarbonyle, un groupe alcoxycarbonyle comportant au total 1 à 5 atomes de carbone dans la partie alkyle,
par exemple, le groupe méthoxycarbonyle, éthoxycarbonyle, n-propoxycarbonyle, isopropoxycarbonyle, n-butoxycarbonyle, 1-méthylpropoxycarbonyle, 2-méthylpropoxy-carbonyle ou tert-butyloxycarbonyle,
le groupe, allyloxycarbonyle, 2,2,2-trichloro-(1,1-diméthyléthoxy)carbonyle ou 9-fluorénylméthoxycarbonyl ou
le groupe formyle, acétyle ou trifluoracétyle,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

2. Antagonistes CGRP de formule générale (I) selon la revendication 1, dans lesquels A, X, D, E, G, M, Q et R¹ sont tels que définis dans la revendication 1 et
R² est l'atome d'hydrogène,
un groupe phénylméthyle ou un groupe alkyle en C₂ à C₇, qui peut être substitué en position ω par un groupe cyclo-alkyle en C₃ à C₇, cyclo-alcényle en C₃ à C₇, phényle, pyridinyle, diazinyle, hydroxy, amino, alkylamino en C₁ à C₆, di-(alkyl en C₁ à C₆)-amino, alcénylamino en C₃ à C₆, di-(alcényle en C₃ à C₆)amino, alcinylamino en C₃ à C₆, di-(alcinyle en C₃ à C₆)amino, hydroxycarbonyle, alcoxycarbonyle en C₁ à C₆, aminocarbonyle, aminocarbonylamino, alkylcarbonylamino en C₁ à C₆, alcénylcarbonylamino en C₂ à C₆, alcinylcarbonylamino en C₂ à C₆, 4-morpholinyle, [Bis-(2-hydroxyéthyl)]amino, 4-(alkyle en C₁ à C₆)-1-pipérazinyle ou 4-(ω-hydroxy-alkyle en C₂ à C₇)-1-pipérazinyle,
un groupe phényle ou pyridinyle,
les groupes phényle, pyridinyle et diazinyle cités dans les groupes définis précédemment pour R² ou contenus comme substituants étant mono- di- ou trisubstitués en outre dans le squelette de carbone par un atome d'halogène, par un groupe alkyle en C₁ à C₃, alcényle en C₂ à C₃, alcinyle en C₂ à C₃, alcoxy en C₁ à C₃, hydroxy, amino, alkylamino, en C₁ à C₃, di-(alkyle en C₁ à C₃)-amino-, amino-alkyle en C₁ à C₃, alkyle en C₁ à C₃-amino-alkyle en C₁ à C₃, di-(alkyle en C₁ à C₃)-amino-alkyle en C₁ à C₃, alkylcarbonylamino en C₁ à C₃, alkylcarbonylamino en C₁ à C₃-alkyle en C₁ à C₃, aminocarbonyle, alkyl en C₁ à C₃-aminocarbonyle, di-(alkyl en C₁ à C₃)-aminocarbonyle, cyano, aminosulfonyle, alkyle en C₁ à C₃-aminosulfonyle, di-(alkyle en C₁ à C₃)-aminosulfonyle, alkyle en C₁ à C₃-thio-, alkyle en C₁ à C₃-sulfinyle, ou alkyle en C₁ à C₃-sulfonyle et les substituants peuvent être identiques ou différents,
R³ désigne l'atome d'hydrogène ou un groupe alkyle en C₁ à C₃ substitué par un groupe phényle ou pyridinyle,
le groupe alkyle en C₁ à C₃ pouvant être lié à un groupe alkyle contenu dans R² ou un cycle phényle ou pyridyle contenu dans R² en incluant l'atome d'azote auquel R² et R³ sont liés, en formant un cycle de 4 à 7 chaînons,
ou
R² et R³ conjointement avec l'atome d'azote inclus forment un radical de formule générale 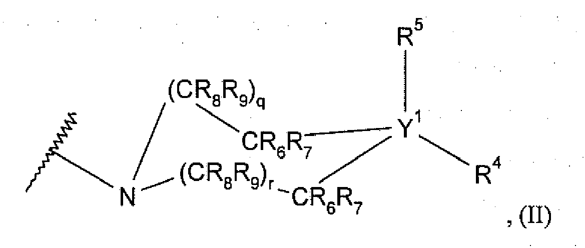 dans laquelle
Y¹ désigne l'atome de carbone ou, si R⁵ représente une paire d'électrons libres, également l'atome d'azote,
q et r, si Y¹ représentent l'atome de carbone, sont les chiffres 0, 1 ou 2 ou
q et r, si Y¹ représente l'atome d'azote, sont les chiffres 1 ou 2,
R⁴ désigne l'atome d'hydrogène, un groupe amino, alkylamino C₁ à C₄, di-(alkyle en C₃ à C₄)-alkylamino, alkyle en C₁ à C₆, cyclo-alkyle en C₃ à C₇, cyclo-alcényle en C₃ à C₇, amino-alkyle en C₂ à C₇, alkyle C₁ à C₄-amino-alkyle en C₂ à C₇, di-(alkylamino en C₁ à C₄)-alkyle en C₂ à C₇, amino-iminométhyle, aminocarbonylamino, alkyle C₁ à C₄-aminocarbonylamino, di(alkyle en C₁ à C₄)-aminocarbonylamino, alkyle C₁ à C₄-aminocarbonyl-alkyle en C₁ à C₄-amino, di-(alkyle en C₁ à C₄)-aminocarbonyl-alkyle en C₁ à C₄-amino, phénylaminocarbonylamino, aminocarbonyle, alkyle en C₁ à C₄-aminocarbonyle, di-(alkyle en C₁ à C₄)-aminocarbonyle, aminocarbonyl-alkyle en C₁ à C₃, alkyle en C₁ à C₄-aminocarbonyl-alkyle en C₁ à C₃, di-(alkyle en C₁ à C₄)-aminocarbonyl-alkyle en C₁ à C₃, aminocarbonylamino-alkyle en C₁ à C₃, alcoxycarbonyle en C₁ à C₆, alcénoxycarbonyle en C₃ à C₆, alcinoxycarbonyle en C₃ à C₆, alcoxycarbonyle en C₁ à C₆-alkyle en C₁ à C₃, alcénoxycarbonyle en C₁ à C₆-alkyle en C₁ à C₃, alcinoxyearbonyle en C₁ à C₆-alkyle en C₁ à C₃, ou hydroxycarbonyl-alkyle en C₁ à C₃,
un groupe phényle, pyridinyle, diazinyle, 1-naphtyle, 2-naphtyle, pyridinylcarbonyle ou phénylcarbonyle qui peuvent être mono-, di- ou trisubstitués dans le squelette de carbone par un atome d'halogène, un groupe alkyle en C₁ à C₃, alcényle en C₂ à C₃, alcinyle en C₂ à C₃, alcoxy en C₁ à C₃, hydroxy, amino, alkylamino en C₁ à C₄, di-(alkyle en C₁ à C₄)-amino, amino-alkyle en C₁ à C₃, alkyle en C₁ à C₄-amino-alkyle en C₁ à C₃, di-(alkyle en C₁ à C₄)-amino-alkyle en C₁ à C₃, alkylcarbonylamino en C₁ à C₄, alkylcarbonylamino en C₁ à C₄-alkyle en C₁ à C₃, aminocarbonyle, alkyle en C₁ à C₄-aminocarbonyle, di-(alkyle en C₁ à C₄)-aminocarbonyle, cyano, aminosulfonyle, alkyle en C₁ à C₄-aminosulfonyle, di(alkyle en C₁ à C₄)-aminosulfonyle, alkyle en C₁ à C₄-thio, alkyle en C₁ à C₄-sulfinyle ou alkyle C₁ à C₄-sulfonyle et les substituants peuvent être identiques ou différents,
un hétérocycle choisi parmi un groupe azacycloalkyle de 4 à 10 chaînons, un groupe oxaza, thiaza, et diazacycloalkyle de 6 à 10 chaînons et un groupe azabicycloalkyle de 6 à 10 chaînons,
un groupe 1-alkyl-4-pipéridinylcarbonyle ou 4-alkyl-1-pipérazinylcarbonyle,
les hétérocycles mono- et bicycliques précédemment cités étant liés par un atome d'azote ou un atome de carbone à Y¹ dans la formule (II),
dans les hétérocycles mono- et bicycliques précédemment cités, un groupe méthine relié de façon indirecte avec un atome d'azote, d'oxygène ou de soufre, pouvant être substitué par un atome de fluor et un groupe méthylène relié de façon indirecte avec un atome d'azote, d'oxygène ou de soufre, par un ou deux atomes de fluor,
les hétérocycles mono- et bicycliques précédemment cités et les groupes 1-(alkyle en C₁ à C₆)-4-pipéridinylcarbonyle et 4-(alkyle en C₁ à C₆)-1-pipérazinylcarbonyle pouvant être substitués une à quatre fois dans le cycle par un groupe alkyle en C₁ à C₆ ou éventuellement, en outre monosubstitués par un groupe cyclo-alkyle en C₃ à C₇, cyclo-alcényle en C₃ à C₇, cyclo-alkyle en C₃ à C₇-alkyle en C₁ à C₃, phényl-alkyle en C₁ à C₃, pyridyl-alkyle en C₁ à C₃, alkylcarbonyle en C₁ à C₆, hydroxy, alcoxy en C₁ à C₆, amino, alkylamino en C₁ à C₄, di-(alkyle en C₁ à C₄)-amino, phénylcarbonyle, pyridinylcarbonyle, hydroxycarbonyle, hydroxycarbonyl-alkyle en C₁ à C₃, alcoxycarbonyle en C₁ à C₆, alcoxycarbonyle en C₁ à C₆-alkyle en C₁ à C₃, aminocarbonyle, alkylaminocarbonyle en C₁ à C₄, di-(alkyle en C₁ à C₄)-aminocarbonyle, alkylsulfonyle en C₁ à C₃, par un groupe cyclo-alkyle-substitué en C₁ à C₃-alkylcarbonyle en C₃ à C₇, azacyclo-alkylcarbonyle en C₄ à C₇, diazacyclo-alkylcarbonyle en C₅ à C₇, ou oxazacyclo-alkylcarbonyle en C₅ à C₇, les substituants étant identiques ou différents et pouvant être liés sur un atome de carbone du cycle
ou un atome d'azote du cycle,
les radicaux phényle et pyridinyle contenus dans les radicaux définis précédemment pour R⁴ pouvant être mono- di ou trisubstitués de leurs côtés par des atomes d'halogène, par un groupe alkyle en C₁ à C₃, alcényle en C₂ à C₃, alcinyle en C₂ à C₃, alcoxy en C₁ à C₃, hydroxy, amino, alkylamino en C₁ à C₄, di-(alkyle en C₁ à C₄)-amino, amino-alkyle en C₁ à C₃, alkyle en C₁ à C₄-aminoalkyle en C₁ à C₃, di-(alkyle en C₁ à C₄)-amino-alkyle en C₁ à C₃, alkylcarbonylamino en C₁ à C₄, alkylcarbonylamino en C₁ à C₄-alkyle en C₁ à C₃, aminocarbonyle, alkyle en C₁ à C₃-aminocarbonyle, di-alkyle en C₁ à C₄-aminocarbonyle, cyano, aminosulfonyl-alkyle en C₁ à C₄-aminosulfonyle, di-(alkyle en C₁ à C₄)-aminosulfonyle, alkylthio en C₁ à C₃, alkyle en C₁ à C₃-sulfinyle ou alkyle en C₁ à C₃-sulfonyle, les substituants pouvant être identiques ou différents,
ou également, si Y¹ désigne l'atome de carbone, le groupe hydroxycarbonyle, aminométhyle, alkyle en C₁ à C₄-aminométhyle ou di-(alkyle en C₁ à C₄)-aminométhyle,
R⁵ est un atome d'hydrogène,
un radical alkyle en C₁ à C₄, un radical alkyle non ramifié pouvant être substitué en position ω par un groupe phényle, pyridinyle, diazinyle, amino, alkylamino en C₁ à C₄, di-(alkyle en C₁ à C₄)-amino, 4-alkyle en C₁ à C₄-1-pipérazinyle ou 4-morpholinyle,
un groupe alcoxycarbonyle en C₁ à C₆, cyano ou aminocarbonyle ou également, si Y¹ désigne un atome d'azote, une paire d'électrons libres,
ou si Y¹ désigne l'atome de carbone, également l'atome de fluor, ou
R⁴, conjointement avec R⁵ et Y¹, désigne un cycle cycloaliphatique de 4 à 7 chaînons dans lequel un groupe méthylène peut être substitué par un groupe NH-, -N(alkyle en C₁ à C₄)-, -N(alcényle en C₃ à C₄)-, -N(alcinyle en C₃ à C₄)-, -N(cyclo-alkyle en C₃ à C₇)-, ou -N(cycloalkyle C₃ à C₇-alkyle en C₁ à C₃)-,
un atome d'hydrogène lié à un atome d'azote dans l'un des groupes définis précédemment pour R⁴, pouvant être substitué par un radical protecteur,
R⁶ et R⁷, qui peuvent être identiques ou différents, représentent respectivement un atome d'hydrogène, un groupe alkyle en C₁ à C₄ ou également, si Y¹ représente un atome de carbone, l'atome de fluor, un groupe alkylamino en C₁ à C₄, ou di-(alkyle en C₁ à C₄)-amino, les deux groupes alkyle en C₁ à C₄ pouvant être liés l'un à l'autre en formant un cycle, et
R⁸ et R⁹, qui peuvent être identiques ou différents, représentent respectivement un atome d'hydrogène ou un groupe alkyle en C₁ à C₃,
où sauf indication contraire, tous les groupes alkyle, alcényle et alcinyle mentionnés ou contenus dans les radicaux définis précédemment peuvent être linéaires ou ramifiés, chaque groupe méthine contenu dans les radicaux définis précédemment pouvant être substitué par un atome de fluor, chaque groupe méthylène pouvant être substitué par jusqu'à 2 atomes de fluor et chaque groupe méthyle pouvant être substitué par jusqu'à 3 atomes de fluor et deux groupes alkyle et alcényle liés à un atome d'azote pouvant être liés l'un à l'autre en formant un cycle hétérocyclique saturé ou insaturé de 4 à 7 chaînons,
tous les radicaux aromatiques ou hétéroaromatiques mentionnés ou contenus dans les radicaux définis précédemment pouvant être mono-, di ou trisubstitués en outre par un atome d'halogène, un groupe cyano ou hydroxy et les substituants pouvant être identiques ou différents,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

3. Antagonistes CGRP de formule générale (I) selon la revendication 1, dans lesquels A, X, R¹, R² et R³ sont tels que définis dans l'une des revendications 1 à 2 et
(a) D, E sont indépendamment l'un de l'autre un groupe méthine ou l'atome d'azote et
G est un groupe méthine substitué par le groupe R^{a},
M est un groupe méthine substitué par le groupe R^{b},
Q est un groupe méthine substitué par le groupe R^{c},
un ou deux des groupes G, M et Q pouvant désigner également un atome d'azote,
ou
(b) D et E sont respectivement un groupe méthine, l'un des groupes D et E pouvant également désigner un atome d'azote, et
G, M et Q sont respectivement un atome d'azote,
où R^{a}, R^{b} et R^{c} indépendamment les uns des autres sont respectivement un atome d'hydrogène ou d'halogène, un groupe alkyle en C₁ à C₄, alcényle en C₂ à C₄, alcinyle en C₂ à C₄, cyclo-alkyle C₃ à C₆, cyclo-alcényle C₃ à C₆, cyano, hydroxy, hydroxy-alkyle C₁ à C₄, hydroxy-alcényle C₃ à C₄, hydroxy-alcinyle C₃ à C₄, alcoxy en C₁ à C₄, alcoxy C₁ à C₄-alkyle C₁ à C₄, alcoxy en C₁à C₄-alcényle C₃ à C₄, alcoxy en C₁ à C₄-alcinyle C₃ à C₄, thiohydroxy, alkylthio en C₁ à C₄, amino, alkyle en C₁ à C₄-amino, alcényle en C₃ à C₄-amino, alcinyle en C₃ à C₄-amino, di-(alkyle en C₁ à C₄)-amino, di-(alcényle en C₃ à C₄)-amino, di-(alcinyle en C₃ à C₄)-amino, amino-alkyle en C₁ à C₄, alkyle en C₁ à C₃-amino-alkyle en C₁ à C₄, di(alkyle en C à C₃)-amino-alkyle en C₁ à C₄, amino-alcényle en C₃ à C₄, alkyle en C₁ à C₃-amino-alcényle en C₃ à C₄, di-(alkyle en C₁ à C₃)-amino-alcényle en C₃ à C₄, amino-alcinyle en C₃ à C₄, alkyle en C₁ à C₃-amino-alcinyle en C₃ à C₄, di-(alkyle en C₁ à C₃)-amino-alcinyle en C₃ à C₄, hydroxycarbonyle, phénylcarbonyle, pyridylcarbonyle, alkyle en C₁ à C₄-carbonyle, formule, alcoxy en C₁ à C₄-carbonyle, alcénoxy en C₃ à C₄-carbonyle, alcinoxy en C₃ à C₄-carbonyle, aminocarbonyle, alkyle en C₁ à C₄-aminocarbonyle, alcényle en C₃ à C₄-aminocarbonyle, alcinyle en C₃ à C₄-aminocarbonyle, di-(alkyle en C₁ à C₄)-aminocarbonyle, di-(alcényle en C₃ à C₄)-aminocarbonyle, di-(alcinyle en C₃ à C₄)-aminocarbonyle, formylamino, alkyle en C₁ à C₄-carbonylamino, formyl-alkyle en C₁ à C₄-amino, formyle-alcényle en C₃ à C₄-amino, formyl-alcinyle en C₃ à C₄-amino, alkyle en C₁ à C₄-carbonyl-alkyle en C₁ à C₄-amino, alkyle en C₁ à C₄-carbonyle, alcényle en C₃ à C₄-amino, alkyle en C₁ à C₄-carbonyle-alcinyle en C₃ à C₄-amino, alkyle en C₁ à C₄-sulfonyle, alcényle en C₂ à C₄-sulfonyle, alcinyle en C₂ à C₄-sulfonyle, alkyle en C₁ à C₄-sulfinyle, alcényle en C₂ à C₄-sulfinyle, alcinyle en C₂ à C₄-sulfinyle, alkyle en C₁ à C₄-sulfonylamino, alkyle en C₁ à C₄-sulfonyle-alkylamino en C₁à C₄, alkyle en C₁ à C₄-sulfonyl-alcénylamino en C₃ à C₄, alkyle en C₁ à C₄-sulfonyl-alcinylamino en C₃ à C₄, aminosulfonyle, alkylaminosulfonyle en C₁ à C₄, di-(alkyle en C₁ à C₄)-aminosulfonyle, alcényle en C₃ à C₄-aminosulfonyle, di-(alcényle en C₃ à C₄)-aminosulfonyle, alcinylaminosulfonyle en C₃ à C₄ ou di-(alcinyle en C₃ à C₄)-aminosulfonyle,
à condition que, dans la mesure ou aucun des groupes D, E, G, M et Q ne représente un atome d'azote,
(i) R^{a} n'est pas un atome d'hydrogène si R^{b} et R^{c} désignent respectivement un groupe alkyle en C₁ à C₄,
(ii) R^{c} n'est pas un atome d'hydrogène si R^{a} et R^{b} représentent respectivement un groupe alkyle en C₁ à C₄,
(iii) R^{a} n'est pas un atome d'hydrogène, de fluor, de chlore, de brome ou d'iode ou un groupe difluoro- ou trifluorométhyle si R^{c} est un groupe alkyle en C₁ à C₄, alcényle en C₂ à C₄, ou alcinyle en C₂ à C₄, et R^{b} est un atome de chlore ou de brome, un groupe amino, méthylamino ou hydroxy,
(iv) R^{c} n'est pas un atome d'hydrogène, de fluor, de chlore, de brome ou d'iode ou un groupe difluoro- ou trifluorométhyle, si R^{a} désigne un groupe alkyle en C₁ à C₄, alcényle en C₂ à C₄ ou alcinyle en C₂ à C₄, et R^{b} est un atome de chlore ou de brome, un groupe amino, méthylamino ou hydroxy,
où sauf indication contraire, tous les groupes alkyle, alcényle et alcinyle mentionnés ou contenus dans les radicaux définis précédemment pour R^{a}, R^{b} et R^{c} peuvent être linéaires ou ramifiés, chaque groupe méthine contenu dans les radicaux définis précédemment pouvant être substitué par un atome de fluor, chaque groupe méthylène pouvant être substitué par jusqu'à 2 atomes de fluor et chaque groupe méthyle pouvant être substitué par jusqu'à 3 atomes de fluor et deux groupes alkyle et alcényle liés à un atome d'azote pouvant être liés l'un à l'autre en formant un cycle hétérocyclique saturé ou insaturé de 4 à 7 chaînons,
les liaisons doubles ou triples des groupes alcényle en C₃ à C₄ ou alcinyle en C₃ à C₄ contenus dans les groupes définis précédemment pour R^{a}, R^{b}, et R^{c} étant isolées des hétéroatomes contenus éventuellement dans ces groupes,
et tous les radicaux aromatiques et hétéroaromatiques mentionnés ou contenus dans les radicaux définis précédemment sont mono-, di- ou trisubstitués en outre par un atome d'halogène, des groupes cyano ou hydroxy et les substituants pouvant être identiques ou différents,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

4. Antagonistes CGRP de formule générale (I) selon la revendication 1, dans lesquels A, X, Ruz, R² et R³ sont tels que définis dans l'une des revendications 1 à 2 et
(a) D, E indépendamment l'un de l'autre sont respectivement, un groupe méthine ou l'atome d'azote et
G est uns groupe méthine substitué par le groupe R^{a},
M est un groupe méthine substitué par le groupe R^{b},
Q est un groupe méthine substitué par le groupe R^{c},
un ou deux des groupes G, M et Q pouvant être respectivement également un atome d'azote,
zou
(b) D et E sont respectivement un groupe méthine, l'un des groupes D et E pouvant être également un atome d'azote et
G, M et Q sont respectivement un atome d'azote,
R^{a}, R^{b} et R^{c} étant indépendamment les uns des autres un atome d'hydrogène ou d'halogène, un groupe alkyle en C₁ à C₄, alcényle en C₂ à C₄, alcinyle en C₂ à C₄, cyclo-alkyle en C₃ à C₆, cyclo-alcényle en C₃ à C₆, cyano, hydroxy, hydroxy-alkyle en C₁ à C₂, hydroxy-alcényle en C₃, hydroxy-alcinyle en C₃, alcoxy en C₁ à C₃, alcoxy en C₁ à C₄-alkyle en C₁ à C₂, amino, alkyle en C₁ à C₄-amino-, alcényle en C₃ à C₄-amino, alcinyle en C₃ à C₄-amino-, di-(alkyle en C₁ à C₄)-amino, di-(alcényle en C₃ à C₄)-amino, di-(alcinyle en C₃ à C₄)-amino, amino-alkyle en C₁ à C₂, alkyle en C₁ à C₃-amino-alkyle en C₁ à C₂, di-(alkyle en C₁ à C₃)-amino-alkyle en C₁ à C₂, amino-alcényle en C₃, alkyle en C₁ E à C₃-amino-alcényle en C₃, di-(alkyle en C₁ à C₃)-amino-alcényle en C₃, amino-alcinyle en C₃, alkyle en C₁ à C₃-amino-alcinyle en C₃, di-(alkyle en C₁ à C₃)-amino-alcinyle en C₃, hydroxycarbonyle, alkyle en C₁ à C₄-carbonyle, formyle, alcoxy en C₁ à C₄-carbonyle, aminocarbonyle, alkyle en C₁ à C₄-aminocarbonyle, di-(alkyle en C₁ à C₄)-aminocarbonyle, formylamino, alkyle en C₁ à C₄-carbonylamino-, formyl-alkyle en C₁ à C₄-amino, alkyle en C₁ à C₄-carbonyl-alkyle en C₁ à C₄-alkyl-amino, alkyle en C₁ à C₄-sulfonyle, alkyle en C₁ à C₄-sulfinyle, alkyle en C₁ à C₄-sulfonylamino, alkyle en C₁ à C₄-sulfonyl-alkylamino en C₁ à C₄, aminosulfonyle, alkylaminosulfonyle en C₁ à C₄, ou di-(alkyle en C₁ à C₄)-aminosulfonyle,
à condition que, dans la mesure où aucun des groupes D, E, G, M et Q ne représente un atome d'azote,
(i) R^{a} n'est pas un atome d'hydrogène si R^{b} et R^{c} désignent respectivement un groupe alkyle en C₁ à C₄,
(ii) R^{c} n'est pas un atome d'hydrogène si R^{a} et R^{b} représentent respectivement un groupe alkyle en C₁ à C₄,
(iii) R^{a} n'est pas un atome d'hydrogène, de fluor, de chlore, de brome ou d'iode ou un groupe difluoro- ou trifluorométhyle si R^{c} est un groupe alkyle en C₁ à C₄, alcényle en C₂ à C₄, ou alcinyle en C₂ à C₄, et R^{b} est un atome de chlore ou de brome, un groupe amino, méthylamino ou hydroxy,
(iv) R^{c} n'est pas un atome d'hydrogène, de fluor, de chlore, de brome ou d'iode ou un groupe difluoro- ou trifluorométhyle, si R^{a} désigne un groupe alkyle en C₁ à C₄, alcényle en C₂ à C₄ ou alcinyle en C₂ à C₄, et R^{b} est un atome de chlore ou de brome,
un groupe amino, méthylamino, ou hydroxy,
où sauf indication contraire, tous les groupes alkyle, alcényle et alcinyle mentionnés ou contenus dans les radicaux définis précédemment pour R^{a}, R^{b} et R^{c} peuvent être linéaires ou ramifiés, chaque groupe méthine contenu dans les radicaux définis précédemment pouvant être substitué par un atome de fluor, chaque groupe méthylène pouvant être substitué par jusqu'à 2 atomes de fluor et chaque groupe méthyle pouvant être substitué par jusqu'à 3 atomes de fluor et deux groupes alkyle et alcényle liés à un atome d'azote pouvant être liés l'un à l'autre en formant un cycle hétérocyclique saturé ou insaturé de 4 à 7 chaînons, et
les liaisons doubles ou triples des groupes alcényle en C₃ à C₄ ou alcinyle en C₃ à C₄ obtenus dans les groupes définis précédemment pour R^{a}, R^{b} et R^{c} étant isolées des hétéroatomes contenus éventuellement dans ces groupes,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

5. Antagonistes CGRP de formule générale (I) selon la revendication 1, dans lesquels A, X, R¹, R² et R³ sont tels que définis dans l'une des revendications 1 à 2 et
(a) D, E indépendamment l'un de l'autre sont respectivement, un groupe méthine ou l'atome d'azote et
G est un groupe méthine substitué par le groupe R^{a},
M est un groupe méthine substitué par le groupe R^{b}
Q est un groupe méthine substitué par le groupe R^{c},
un ou deux-des groupes G, M et Q pouvant être respectivement également un atome d'azote,
ou
(b) D et E sont respectivement un groupe méthine, l'un des groupes D et E pouvant être également un atome d'azote et
G, M et Q sont respectivement un atome d'azote,
R^{a}, R^{b} et R^{c} étant indépendamment les uns des autres un atome d'hydrogène ou d'halogène, un groupe alkyle en C₁ à C₄, alcényle en C₂ à C₄, alcinyle en C₂ à C₄, cyclo-alkyle en C₃ à C₆, cyclo-alcényle en C₃ à C₆, cyano, hydroxy, hydroxy-alkyle en C₁ à C₂, alcoxy en C₁ à C₄, amino, alkyle en C₁ à C₄-amino-, di-(alkyle en C₁ à C₄)-amino, amino-alkyle en C₁ à C₂, alkyle en C₁ à C₃-amino-alkyle en C₁ à C₂, di-(alkyle en C₁ à C₃)-amino-alkyle en C₁ à C₂, hydroxycarbonyle, alkyle en C₁ à C₄-carbonyle, formyle, alcoxy en C₁ à C₄-carbonyle, aminocarbonyle, alkyle en C₁ à C₄-aminocarbonyle, di-(alkyle en C₁ à C₄)-aminocarbonyle, formylamino, alkyle en C₁ à C₄-carbonylamino, formyl-alkyle en C₁ à C₄-amino ou alkyle en C₁ à C₄-carbonyl-alkyle en C₁ à C₄-amino,
à condition que, dans la mesure où aucun des groupes D, E, G, M et Q ne représente un atome d'azote,
(i) R^{a} n'est pas un atome d'hydrogène si R^{b} et R^{c} désignent respectivement un groupe alkyle en C₁ à C₄,
(ii) R^{c} n'est pas un atome d'hydrogène si R^{a} et R^{b} représentent respectivement un groupe alkyle en C₁ à C₄,
(iii) R^{a} n'est pas un atome d'hydrogène, de fluor, de chlore, de brome ou d'iode ou un groupe difluoro- ou trifluorométhyle si R^{c} est un groupe alkyle en C₁ à C₄, alcényle en C₂ à C₄, ou alcinyle en C₂ à C₄, et R^{b} est un atome de chlore ou de brome, un groupe amino, méthylamino ou hydroxy,
(iv) R^{c} n'est pas un atome d'hydrogène, de fluor, de chlore, de brome ou d'iode ou un groupe difluoro- ou trifluorométhyle, si R^{a} désigne un groupe alkyle en C₁ à C₄, alcényle en C₂ à C₄ ou alcinyle en C₂ à C₄, et R^{b} est un atome de chlore ou de brome, un groupe amino, méthylamino ou hydroxy,
où sauf indication contraire, tous les groupes alkyle, alcényle et alcinyle mentionnés ou contenus dans les radicaux définis précédemment pour R^{a}, R^{b} et R^{c} peuvent être linéaires ou ramifiés, chaque groupe méthine, contenu dans les radicaux définis précédemment pouvant être substitué par un atome de fluor, chaque groupe méthylène pouvant être substitué par jusqu'à 2 atomes de fluor et chaque groupe méthyle pouvant être substitué par jusqu'à 3 atomes de fluor et deux groupes alkyle et alcényle liés à un atome d'azote pouvant être liés l'un à l'autre en formant un cycle hétérocyclique saturé ou insaturé de 4 à 7 chaînons,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

6. Antagonistes CGRP de formule générale (I) selon la revendication 1, dans lesquels A, X, R¹, R² et R³ sont tels que définis dans l'une des revendications 1 à 2 et
(a) D, E indépendamment l'un de l'autre sont respectivement, un groupe méthine ou l'atome d'azote et
G est un groupe méthine substitué par le groupe R^{a},
M est un groupe méthine substitué par le groupe R^{b},
Q est un groupe méthine substitué par le groupe R^{c},
un ou deux des groupes G, M et Q pouvant être respectivement également un atome d`azote,
ou
(b) D et E sont respectivement un groupe méthine, l'un des groupes D et E pouvant être également un atome d'azote et
G, M et Q sont respectivement un atome d'azote,
R^{a}, R^{b} et R^{c} étant indépendamment les uns des autres un atome d'hydrogène ou d'halogène, un groupe méthyle, difluorométhyle, trifluorométhyle, éthyle, vinyle, éthinyle, cyano, hydroxy, méthoxy, difluorométhoxy, trifluorométhoxy, amino, méthylamino ou diméthylamino,
à condition que, dans la mesure où aucun des groupes D, E, G, M et Q ne représente pas un atome d'azote,
(i) R^{a} n'est pas un atome d'hydrogène si R^{b} et R^{c} désignent respectivement un groupe méthyle ou éthyle,
(ii) R^{c} n'est pas un atome d'hydrogène si R^{a} et R^{b} représentent respectivement un groupe méthyle ou éthyle,
(iii) R^{a} n'est pas un atome d'hydrogène, de fluor, de chlore, de brome ou d'iode ou un groupe difluoro- ou trifluorométhyle si R^{c} est un groupe méthyle, éthyle, vinyle ou éthinyle, et R^{b} est un atome de chlore ou de brome, un groupe amino, méthylamino ou hydroxy,
(iv) R^{c} n'est pas un atome d'hydrogène, de fluor, de chlore, de brome ou d'iode ou un groupe difluoro- ou trifluorométhyle, si R^{a} désigne un groupe méthyle, éthyle, vinyle ou éthinyle et R^{b} est un atome de chlore ou de brome, un groupe amino, méthylamino ou hydroxy,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

7. Antagonistes CGRP de formule générale (I) selon la revendication 1, dans lesquels A, X, D, E, G, M, Q et R¹ sont tels que définis dans l'une des revendications 1 à 6 et R² est l'atome d'hydrogène ou
un groupe phénylméthyle ou un groupe alkyle en C₂ à C₇ qui peut être substitué en position ω par un groupe cyclo-alkyle en C₃ à C₇, cyclo-alcényle en C₃ à C₇, phényle, pyridinyle, hydroxy, amino, alkylamino en C₁ à C₆, di-(alkyle en C₁ à C₆)-amino, hydroxycarbonyle, alkoxycarbonyle en C₁ à C₆, aminocarbonyle, aminocarbonylamino, alkylamino en C₁ à C₆, 4-morpholinyle,
les radicaux phényle et pyridinyle contenus dans les groupes définis précédemment pour R² ou comme substituants, pouvant être en outre mono- di ou trisubstitués dans le squelette de carbone par un atome d'halogène, par un groupe alkyle en C₁ à C₃, alcoxy en C₁ à C₃, hydroxy, amino, alkylamino en C₁ à C₃, di-(alkyle en C₁ à C₃)-amino, amino-alkyle en C₁ à C₃, alkyle en C₁ à C₃-aminoalkyle en C₁ à C₃, di-(alkyle en C₁ à C₃)-amino-alkyle en C₁ à C₃, alkylcarbonylamino en C₁ à C₃, alkylcarbonyle en C₁ à C₃-alkylamino en C₁ à C₃, aminocarbonyle, alkylaminocarbonyle en C₁ à C₃, ou di-(alkyle en C₁ à C₃)-aminocarbonyle et les substituants pouvant être identiques ou différents,
R³ est l'atome d'hydrogène ou un groupe alkyle en C₁ à C₃ ou
R² et R³ forment conjointement avec l'atome d'azote inclus, un radical de formule générale 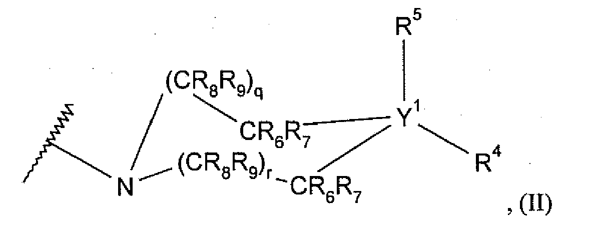 où
Y¹ est l'atome de carbone ou, si R⁵ représente une paire d'électrons libres, est également l'atome d'azote,
q et r, si Y¹ est l'atome de carbone, sont les chiffres 0 ou 1 ou
q et r, si Y¹ est l'atome d'azote, sont les chiffres 1 ou 2,
R⁴ est l'atome d'azote, un groupe amino, alkyle en C₁ à C₄-amino, di-(alkyle en C₁ à C₄)-alkylamino, alkyle en C₁ à C₆, un groupe cyclo-alkyle en C₃ à C₇, ou cyclo-alcényle en C₃ à C₇ éventuellement substitué par un groupe hydroxycarbonyle, alkoxycarbonyle en C₁ à C₆, hydroxycarbonyle-alkyle en C₁ à C₃ ou alcoxycarbonyle en C₁à C₆-alkyle en C₁ à C₃, un groupe amino-alkyle en C₂ à C₇, alkyle en C₁ à C₄-amino-alkyle en C₂ à C₇, di-(alkyle en C₁ à C₄-amino)-alkyle en C₂ à C₇, alkyle en C₁ à C₄-aminocarbonyle, di-(alkyle en C₁ à C₄)-aminocarbonyle, aminocarbonyl-alkyle en C₁à C₃, alkyle en C₁ à C₄-aminocarbonyl-alkyle en C₁ à C₃, di-(alkyle en C₁ à C₄)-aminocarbonyl-alkyle en C₁ à C₃, aminocarbonylamino-alkyle en C₁ à C₃, alkoxycarbonyle en C₁ à C₆, alkoxycarbonyle en C₁ à C₆-alkyle en C₁ à C₃ ou hydroxycarbonyle-alkyle en C₁ à C₃,
un groupe phényle, pyridinyle ou diazinyle qui peuvent être substitués respectivement par un atome d'halogène, par un groupe alkyle en C₁ à C₃, alkoxy en C₁ à C₃, hydroxy, amino, alkyle en C₁ à C₄-amino, di-(alkyle en C₁ à C₄)-amino, amino-alkyle en C₁ à C₃, alkyle en C₁ à C₄-amino-alkyle en C₁ à C₃, di-(alkyle en C₁ à C₄)-amino-alkyle en C₁ à C₃,
un hétérocycle choisi parmi les groupes azacycloalkyle de 4 à 7 chaînons, un groupe oxaza, S,S-Dioxothiaza et diazacycloalkyle de 6 à 7 chaînons et un groupe azabicycloalkyle de 7 à 9 chaînons,
les hétérocycles mono- et bicycliques cités précédemment étant liés par un atome d'azote ou de carbone à Y¹ dans la formule (II),
dans les hétérocycles mono- et bicycliques précédemment cités, un groupe méthylène non relié directement à un atome d'azote, d'oxygène ou de soufre pouvant être substitué par un ou deux atomes de fluor, et
les hétérocycles mono- et bicycliques précédemment cités pouvant être mono- ou disubstitués par un groupe hydroxy-, alkyle en C₁ à C₃, ou hydroxy-alkyle en C₁ à C₃, ou monosubstitués par un groupe benzyle, cyclo-alkyle en C₃ à C₆, hydroxycyclo-alkyle en C₃ à C₆, cyclo-alkyle en C₃ à C₆-alkyle en C₁ à C₃, alkylcarbonyle en C₁ à C₄, alkylcarbonyle en C₁ à C₄-alkyle en C₁ à C₃, hydroxy, alcoxy en C₁ à C₄, amino, alkylamino en C₁ à C₄, di-(alkyle en C₁ à C₄)-amino, alcoxycarbonyle en C₁ à C₃, hydroxycarbonyl-carbonyle, alkoxycarbonyle en C₁ à C₃-carbonyle, hydroxycarbonyl-alkyle en C₁ à C₃, alkoxycarbonyle en C₁ à C₃-alkyle en C₁ à C₃, hydroxycarbonyl-alkylcarbonyle en C₁ à C₃, alcoxycarbonyle en C₁ à C₃-alkylcarbonyle en C₁ à C₃, aminosulfonyle, alkylaminosulfonyle en C₁ à C₄, di-(alkyle en C₁ à C₄)-aminosulfonyle, alkylsulfonyle en C₁ à C₃, cyclo-alkylsulfonyle en C₃ à C₇, aminocarbonyl-alkyle en C₁ à C₃, alkylaminocarbonyle en C₁ à C₄-alkyle en C₁ à C₃, Di-(alkyle en C₁ à C₄)-aminocarbonyl-alkyle en C₁ à C₃, hydroxyaminocarbonyl-alkyle en C₁ à C₃, alcoxyaminocarbonyle en C₁ à C₃-alkyle en C₁ à C₃ ou hydroxy-(alkyle en C₁ à C₃)-aminocarbonyl-alkyle en C₁ à C₃,
ou également, si Y¹ est l'atome de carbone, le groupe hydroxycarbonyle, aminométhyle, alkyle en C₁ à C₄-aminométhyle ou di-(alkyle en C₁ à C₄)-aminométhyle,
R⁵ est un atome d'hydrogène, un radical alkyle en C₁ à C₃ ou, si Y¹ est un atome d'azote, représente également une paire d'électrons,
R⁶ et R⁷ qui peuvent être identiques ou différents, sont respectivement un atome d'hydrogène, un groupe alkyle en C₁ à C₃ ou également, si Y¹ est un atome de carbone, un groupe amino, alkylamino en C₁ à C₃ ou di-(alkyle en C₁ à C₃)-amino, les deux groupes alkyle en C₁ à C₃ pouvant être liés l'un à l'autre en formant un cycle, et
R⁸ et R⁹, qui peuvent être identiques ou différents, représentent respectivement un atome d'hydrogène ou un groupe alkyle en C₁ à C₃,
où sauf indication contraire, tous les groupes alkyle, alcényle et alcinyle mentionnés ou contenus dans les radicaux définis précédemment peuvent être linéaires ou ramifiés, chaque groupe méthine contenu dans les radicaux définis précédemment pouvant être substitué par un atome de fluor, chaque groupe méthylène pouvant être substitué par jusqu'à 2 atomes de fluor et chaque groupe méthyle pouvant être substitué par jusqu'à 3 atomes de fluor et deux groupes alkyle et alcényle liés à un atome d'azote pouvant être liés l'un à l'autre en formant un cycle hétérocyclique saturé ou insaturé de 4 à 7 chaînons,
tous les radicaux aromatiques ou hétéroaromatiques mentionnés ou contenus dans les radicaux définis précédemment pouvant être mono-, di ou trisubstitués en outre par un atome d'halogène, un groupe cyano ou hydroxy et les substituants pouvant être identiques ou différents,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

8. Antagonistes CGRP de formule générale (I) selon la revendication 1, dans lesquels A, X, D, E, G, M, Q et R¹ sont tels que définis dans l'une des revendications 1 à 6 et R² est l'atome d'hydrogène ou
un groupe phénylméthyle ou un groupe alkyle en C₂ à C₇ qui peut être substitué en position ω par un groupe cyclo-alkyle en C₃ à C₇, cyclo-alcényle en C₃ à C₇, phényle, pyridinyle, hydroxy, amino, alkylamino, en C₁ à C₆, di-(alkyle en C₁ à C₆)-amino, hydroxycarbonyle, alkoxycarbonyle en C₁ à C₆, aminocarbonyle, aminocarbonylamino, alkylamino en C₁ à C₆, 4-morpholinyle,
les radicaux phényle et pyridinyle contenus dans les groupes définis précédemment pour R² ou comme substituants, pouvant être en outre mono- di ou trisubstitués dans le squelette de carbone par un atome d'halogène, par un groupe alkyle en C₁ à C₃, alcoxy en C₁ à C₃, hydroxy, amino, alkylamino, en C₁ à C₃, di-(alkyle en C₁ à C₃)-amino, amino-alkyle en C₁ à C₃, alkyle en C₁ à C₃-aminoalkyle en C₁ à C₃, di-(alkyle en C₁ à C₃)-amino-alkyle en C₁ à C₃, alkylcarbonylamino en C₁ à C₃, alkylcarbonyle en C₁ à C₃-alkylamino en C₁ à C₃, aminocarbonyle, alkylaminocarbonyle en C₁ à C₃, ou di-(alkyle en C₁ à C₃)-aminocarbonyle et les substituants pouvant être identiques ou différents,
R³ est un atome d'hydrogène ou un groupe alkyle en C₁ à C₃, ou
R² et R³ forment conjointement avec l'atome d'azote inclus, un radical de formule générale 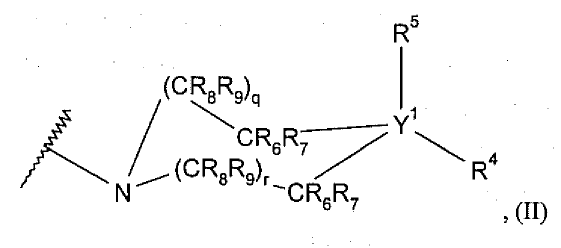 dans laquelle
Y¹ est l'atome de carbone ou, si R⁵ représente une paire d'électrons libres, est également l'atome d'azote,
q et r, si Y¹ est l'atome de carbone, sont les chiffres 0 ou 1 ou
q et r, si Y¹ est l'atome d'azote, sont les chiffres 1 ou 2,
R⁴ est l'atome d'hydrogène, un groupe amino, alkyle en C₁ à C₄-amino, di-(alkyle en C₁ à C₄)-alkylamino, alkyle en C₁ à C₆, cyclo-alkyle en C₃ à C₇, cyclo-alcényle en C₃ à C₇, amino-alkyle en C₂ à C₇, alkyle en C₁ à C₄-amino-alkyle en C₂ à C₇, di-(alkyle en C₁ à C₄-amino)-alkyle en C₂ à C₇, alkyle en C₁ à C4-aminocarbonyle, di-(alkyle en C₁ à C₄)-aminocarbonyle, aminocarbonyl-alkyle en C₁ à C₃, alkyle en C₁à C₄-aminocarbonyl-alkyle en C₁ à C₃, di-(alkyle en C₁ à C₄)-aminocarbonyl-alkyle en C₁ à C₃, aminocarbonylamino-alkyle en C₁ à C₃, alkoxycarbonyle en C₁ à C₆, alkoxycarbonyle en C₁ à C₆-alkyle en C₁ à C₃ ou hydroxycarbonyle-alkyle en C₁ à C₃,
un groupe phényle, pyridinyle ou diazinyle qui peuvent être substitués respectivement par un atome d'halogène, par un groupe alkyle en C₁ à C₃, alcoxy en C₁ à à C₃, hydroxy, amino, alkyle en C₁ à C₄-amino, di-(alkyle en C₁ à C₄)-amino, amino-alkyle en C₁ à C₃, alkyle en C₁ à C₄-amino-alkyle en C₁ à C₃, di-(alkyle en C₁ à C₄)-amino-alkyle en C₁ à C₃,
un hétérocycle choisi parmi les groupes azacycloalkyle de 4 à 7 chaînons, un groupe oxaza et diazacycloalkyle de 6 à 7 chaînons et un groupe azabicycloalkyle de 7 à 9 chaînons,
les hétérocycles mono- et bicycliques cités précédemment étant liés par un atome d'azote ou de carbone à Y¹ dans la formule (II),
dans les hétérocycles mono- et bicycliques précédemment cités, un groupe méthylène non relié directement à un atome d'azote, d'oxygène ou de soufre pouvant être substitué par un ou deux atomes de fluor, et
les hétérocycles mono- et bicycliques précédemment cités pouvant être substitués une ou plusieurs fois, par exemple, mono- à trisubstitués par un groupe alkyle en C₁ à C₃, ou monosubstitués par un groupe benzyle, cyclo-alkyle en C₃ à C₆, cyclo-alkyle en C₃ à C₆-alkyle en C₁ à C₃, alkylcarbonyle en C₁ à C₄, hydroxy, alcoxy en C₁ à C₄, amino, alkylamino en C₁ à C₄, di-(alkyle en C₁ à C₄)-amino, hydroxycarbonyle, alcoxycarbonyle en C₁à C₃, hydroxycarbonyl-carbonyle, alcoxycarbonyle en C₁ à C₃-carbonyle, hydraxycarbanyl-aIkyle en C₁ à C₃, alcoxycarbonyle en C₁ à C₃-alkyl en C₁ à C₃ ou alkylsulfonyle en C₁ à C₃,
ou également, si Y¹ est l'atome de carbone, le groupe hydroxycarbonyle, aminométhyle, alkyle en C₁ à C₄-aminométhyle ou di-(alkyle en C₁ à C₄)-aminométhyle,
R⁵ est un atome d'hydrogène, un radical alkyle en C₁ à C₃ ou, si Y¹ est un atome d'azote, représente également une paire d'électrons,
R⁶ et R⁷, qui peuvent être identiques ou différents, sont respectivement un atome d'hydrogène, un groupe alkyle en C₁ à C₃ ou également, si Y¹ est un atome de carbone, un groupe alkylamino en C₁ à C₃ ou di-(alkyle en C₁ à C₃)-amino, les deux groupes alkyle en C₁ à C₃ pouvant être liés l'un à l'autre en formant un cycle, et
R⁸ et R⁹, qui peuvent être identiques ou différents, représentent respectivement un atome d'hydrogène ou un groupe alkyle en C₁ à C₃,
où sauf indication contraire, tous les groupes alkyle, alcényle et alcinyle mentionnés ou contenus dans les radicaux définis précédemment peuvent être linéaires ou ramifiés, chaque groupe méthine contenu dans les radicaux définis précédemment pouvant être substitué par un atome de fluor, chaque groupe méthylène pouvant être substitué par jusqu'à 2 atomes de fluor et chaque groupe méthyle pouvant être substitué par jusqu'à 3 atomes de fluor et deux groupes alkyle et alcényle liés à un atome d'azote pouvant être liés l'un à l'autre en formant un cycle hétérocyclique saturé ou insaturé de 4 à 7 chaînons,
tous les radicaux aromatiques ou hétéroaromatiques mentionnés ou contenus dans les radicaux définis précédemment pouvant être mono-, di ou trisubstitués en outre par un atome d'halogène, un groupe cyano ou hydroxy et les substituants pouvant être identiques ou différents,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

9. Antagonistes CGRP de formule générale (1) selon la revendication 1, dans lesquels A, X, D, E, G, M, Q et R¹ sont tels que définis dans l'une des revendications 1 à 6 et R² est l'atome d'hydrogène ou
un groupe phénylméthyle ou un groupe alkyle en C₂ à C₇ qui peut être substitué en position ω par un groupe phényle, amino, alkylamino en C₁ à C₆, di-(alkyle en C₁ à C₆)-amino,
les groupes phényle et phénylméthyle mentionnés précédemment pouvant être en outre mono- ou disubstitués au niveau d'un atome de carbone aromatique, par un atome d'halogène, un groupe alkyle en C₁ à C₃, alcoxy en C₁ à C₃, amino-alkyle en Ci à C₃, alkylamino en C₁ à C₃-alkyle en C₁ à C₃, ou di-(alkyle en C₁ à C₃)-amino-alkyle en C₁ à C₃, et les substituants pouvant être identiques ou différents,
R³ est un atome d'hydrogène ou un groupe alkyle en C₁ à C₃, ou
R² et R³ forment conjointement avec l'atome d'azote inclus, un radical de formule générale 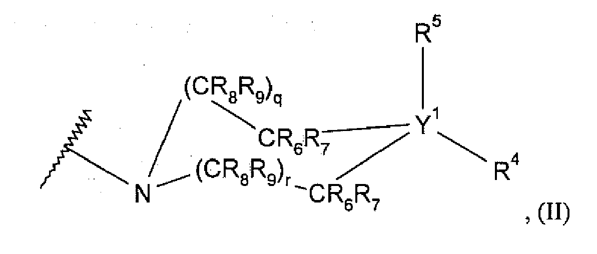 dans laquelle
Y¹ est l'atome de carbone ou, si R⁵ représente une paire d'électrons libres, est également l'atome d'azote,
q et r, si Y¹ est l'atome de carbone, sont les chiffres 0 ou 1 ou
q et r, si Y¹ est l'atome d'azote, sont les chiffres 1 ou 2,
R⁴ est l'atome d'hydrogène, un groupe amino, alkyle en C₁ à C₄-amino, di-(alkyle en C₁ à C₄)-alkylamino, alkyle en C₁ à C₆, un groupe cyclo-alkyle en C₃ à C₇, ou cyclo-alcényle en C₃ à C₇ éventuellement substitué par un groupe hydroxycarbonyle, alkoxycarbonyle en C₁ à C₆, hydroxycarbonyle-alkyle en C₁ à C₃ ou alcoxycarbonyle en C₁ à C₆-alkyle en C₁ à C₃, un groupe amino-alkyle en C₂ à C₇, alkyle en C₁ à C₄-amino-alkyle en C₂ à C₇, di-(alkyle en C₁ à C₄-amino)-alkyle en C₂ à C₇, alcoxycarbonyle en C₁ à C₆, alcoxycarbonyle en C₁ à C₆-alkyle en C₁ à C₃ ou hydroxycarbonyle-alkyle en C₁ à C₃,
un groupe phényle ou pyridyle qui peut être substitué respectivement par un atome d'halogène, par un groupe alkyle en C₁ à C₃, alkoxy en C₁ à C₃, amino, alkyle en C₁ à C₄-amino, di-(alkyle en C₁ à C₄)-amino,
un hétérocycle choisi parmi un groupe azacycloalkyle de 6 à 7 chaînons, un groupe S,S-dioxothiaza et diazacycloalkyle de 6 à 7 chaînons et un groupe azabicycloalkyle de 7 à 9 chaînons,
les hétérocycles mono- et bicycliques cités précédemment étant liés par un atome d'azote ou de carbone à Y¹ dans la formule (II),
dans les hétérocycles mono- et bicycliques précédemment cités, un groupe méthylène non relié directement à un atome d'azote, d'oxygène ou de soufre pouvant être substitué par un ou deux atomes de fluor, et
les hétérocycles mono- et bicycliques précédemment cités pouvant être mono- ou disubstitués par un groupe hydroxy-, alkyle en C₁ à C₃, ou hydroxy-alkyle en C₁ à C₃, par un groupe benzyle, cyclo-alkyle en C₃ à C₆, hydroxycyclo-alkyle en C₃ à C₆, cyclo-alkyle en C₃ à C₆-alkyle en C₁ à C₃, alkylcarbonyle en C₁ à C₃-alkyle en C₁ à C₃, amino, alkylamino en C₁ à C₄ ou di-(alkyle en C₁ à C₄)-amino, hydroxycarbonyl-carbonyle, alkoxycarbonyle en C₁ à C₆-carbonyle, hydroxycarbonyl-alkyle carbonyle en C₁ à C₃, alkoxycarbonyle en C₁ à C₃-alkylcarbonyle en C₁ à C₃, aminosulfonyle, alkylaminosulfonyle en C₁ à C₄, di-(alkyle en C₁ à C₄)-aminosulfonyle, cyclo-alkylsulfonyle en C₃ à C₇, aminocarbonyl-alkyle en C₁ à C₃, alkylaminocarbonyle en C₁ à C₄-alkyle en C₁ à C₃, di-(alkyle en C₁ à C₄)-aminocarbonyl-alkyle en C₁ à C₃, hydroxyaminocarbonyl-alkyle en C₁ à C₃, alcoxyaminocarbonyle en C₁ à C₃-alkyle en C₁ à C₃ ou hydroxy-(alkyle en C₁ à C₃)-aminocarbonyl-alkyle en C₁ à C₃,
ou également, si Y¹ est l'atome de carbone, le groupe hydroxycarbonyle, aminométhyle, alkyle en C₁ à C₄-aminométhyle ou di-(alkyle en C₁ à C₄)-aminométhyle,
R⁵ est un atome d'hydrogène ou, si Y¹ est un atome d'azote, représente également une paire d'électrons libres,
R⁶ et R⁷, qui peuvent être identiques ou différents, sont respectivement un atome d'hydrogène, un groupe alkyle en C₁ à C₃ ou également, si Y¹ est un atome de carbone, un groupe alkylamino en C₁ à C₃ ou di-(alkyle en C₁ à C₃)-amino, les deux groupes alkyle en C₁ à C₃ pouvant être liés l'un à l'autre en formant un cycle, et
R⁸ et R⁹, qui peuvent être identiques ou différents, représentent respectivement un atome d'hydrogène ou un groupe alkyle en C₁ à C₃,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

10. Antagonistes CGRP de formule générale (I) selon la revendication 1, dans lesquels
A, X, D, E, G, M, Q et R¹ sont tels que définis dans l'une des revendications 1 à 6 et R² est un groupe phénylméthyle ou un groupe alkyle en C₂ à C₇ qui peut être substitué en position ω par un groupe phényle, amino, alkylamino en C₁ à C₆, di-(alkyle en C₁ à C₆)-amino,
les groupes phényle et phénylméthyle mentionnés précédemment pouvant être substitués au niveau d'un atome de carbone aromatique, par un groupe amino-alkyle en C₁ à C₃, alkylamino en C₁ à C₃-alkyle en C₁ à C₃, ou di-(alkyle en C₁ à C₃)-amino-alkyle en C₁ à C₃,
R³ est un atome d'hydrogène ou un groupe alkyle en C₁ à C₃, ou
R² et R³ forment conjointement avec l'atome d'azote inclus, un radical de formule générale 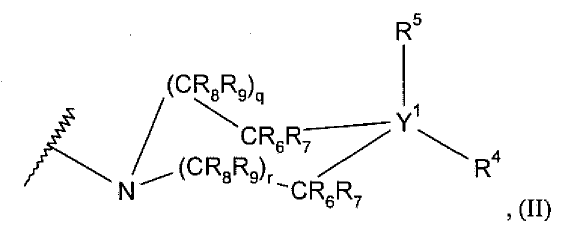 dans laquelle
R⁶ et R⁷ sont respectivement, un atome d'hydrogène ou un groupe diméthylamino,
R⁸ et R⁹ sont respectivement un atome d'hydrogène et
(a) Y¹ est l'atome de carbone,
q et r sont les chiffres 0 ou 1
R⁴ est l'atome d'hydrogène,
un groupe phényle, pyridinyle ou pyrimidinyle qui peut être substitué respectivement par un atome d'halogène, par un groupe amino, méthylamino, diméthylamino, méthyle ou méthoxy,
un groupe hydroxy, 2-diéthylamino-éthyle, amino, méthylamino, diméthylamino, diéthylamino, pyrrolidin-1-yle, 3-hydroxy-pyrrolidin-1-yle, 2-hydroxycarbonyl-pyrrolidin-1-yle, 2-méthoxycarbonyl-pyrrolidin-1-yle, pipéridin-1-yle, 4,4-diméthylpipéridin-1-yle, 4-amino-4-méthyl-pipéridin-1-yle, 2-hydroxycarbonyl-pipéridin-1-yle, 2-méthoxycarbonyl-pipéridin-1-yle, 4-hydroxyméthyl-pipéridin-1-yle, 4-(1-hydroxycyclopropyl)-pipéridin-1-yle, 4-amino-pipéridin-1-yle, 4-méthylamino-piperidin-1-yle, 4-diméthylamino-pipéridin-1-yle, 4-hydroxy-4-méthyl-pipéridin-1-yle, 4-hydroxy-4-éthyl-pipéridin-1-yle, 4-hydroxy-4-trifluorméthyl-pipéridin-1-yle, 4-hydroxy-4-hydroxyméthyl-pipéridin-1-yle, 3-amino-pipéridin-1-yle, 3-méthylamino-pipéridin-1-yle, 3-diméthylamino-pipéridin-1-yle, 3-hydroxy-pipéridin-1-yle, 4-hydroxy-pipéridin-1-yle, 4-hydroxycarbonylméthyl-pipéridin-1-yle, 4-éthoxycarbonylméthyl-pipéridin-1-yle, perhydro-azépin-1-yle, perhydro-1,4-diazépin-1-yle, 4-méthyl-perhydro-1,4-diazépin-1-yle, 1-méthyl-pipéridin-4-yle, pipéridin-4-yle, 1-éthylpipéridin-4-yle, 1-(2-hydroxyéthyl)-pipéridin-4-yle, 1-cyclopropyl-pipéridin-4-yle, 1-cyclopropyl-méthyl-pipéridin-4-yle, 1-méthylsulfonyl-pipéridin-4-yle, 1-éthylsulfonyl-pipéridin-4-yle, 1-isopropylsulfonyl-pipéridin-4-yle, 1-cyclopropylsulfonyl-pipéridin-4-yle, 4-hydroxy-1-méthylsulfonyl-pipéridin-4-yle, 1-aminosulfonyl-pipéridin-4-yle, 1-(méthylaminosulfonyl)-pipéridin-4-yle, 1-(diméthylamino-sulfonyl)-pipéridin-4-yle, 1-hydroxycarbonylméthyl-pipéridin-4-yle, 1-éthoxycarbonylméthyl-pipéridin-4-yle, 1-(2-hydroxycarbonyléthyl)-pipéridin-4-yle, 1-(2-éthoxycarbonyléthyl)-pipéridin-4-yle, 1-(3-hydroxycarbonyl-propionyl)-pipéridin-4-yle, 1-(3-éthoxycarbonyl-propionyl)-pipéridin-4-yle, 1-(hydroxycarbamoyl-méthyl)-pipéridin-4-yle, 1-(hydroxy-méthyl-carbamoyl-méthyl)-pipéridin-4-yle, 1-(méthoxycarbamoyl-méthyl)-pipéridin-4-yle, 1-oxalyl-pipéridin-4-yle, 1-éthoxyoxalyl-pipéridin-4-yle, pipérazin-1-yle, 4-méthyl-pipérazin-1-yle, 4-cyclopropylméthyl-pipérazin-1-yle, 4-éthyl-pipérazin-1-yle, 4-(2-hydroxyéthyl)-pipérazin-1-yle, 4-cyclopropyl-pipérazin-1-yle, 4-méthyl-sulfonyl-pipérazin-1-yle, 4-aminosulfonyl-pipérazin-1-yle, 4-(méthylaminosulfonyl)-pipérazin-1-yle, 4-(dimethylaminosulfonyl)-pipérazin-1-yle, 4-hydroxycarbonylméthyl-pipérazin-1-yle, 4-éthoxycarbonylméthyl-piperazin-1-yle, 4-(2-hydroxycarbonyléthyl)-pipérazin-1-yle, 4-(2-éthoxycarbonyléthyl)-pipérazin-1-yle, 4-(3-hydroxycarbonyl-propionyl)-pipérazin-1-yle, 4-(3-éthoxycarbonyl-propionyl)-pipérazin-1-yle, 4-(hydroxycarbamoyl)-méthyl-pipérazin-1-yle, 4-(hydroxy-méthyl-carbamoyl)-méthyl-pipérazin-1-yle, 4-(méthoxycarbamoyl)-méthyl-pipérazin-1-yle, 1,2-diméthyl-pipérazin-1-yle, 3-méthyl-pipérazin-1-yle, 3,4,5-triméthyl-pipérazin-1-yle, 3,5-diméthyl-pipérazin-1-yle, 3,3,4-triméthyl-pipérazin-1-yle, 3,3-diméthyl-pipérazin-1-yle, 3,3,4,5,5-pentaméthyl-pipérazin-1-yle, 3,3,5,5-tétraméthyl-piperazin-1-yl, 3,3,3-trifluor-2-oxo-propyl)-pipérazin-1-yle, morpholin-4-yle, 1,1-dioxo-1λ⁶-thiomorpholin-4-yle, tétrahydropyran-4-yle, 4,4-difluor-pipéridin-1-yle, 8-méthyl-8-aza-bicyclo[3.2.1]oct-3-yle, 8-aza-bicyclo[3.2.1]oct-3-yle, azétidin-1-yle, 1-(méthoxycarbonylméthyl)-pipéridin-4-yle, 1-(éthoxycarbonylméthyl)-pipéridin-4-yle, 4-(éthoxycarbonyl-méthyl)-pipérazin-1-yle, 1-hydroxycarbonylméthyl-pîpéridin-4-yle ou 4-hydroxycarbonylméthyl-pipérazin-1-yle et , R⁵ est un atome d'hydrogène, ou
(b) Y¹ est un atome d'azote,
q et r sont les chiffres 1 ou 2,
R⁴ est l'atome d'hydrogène,
un groupe phényle, pyridinyle ou pyrimidinyle qui peut être substitué respectivement par un atome d'halogène, un groupe amino, méthylamino, diméthylamino, méthyle ou méthoxy,
un groupe méthyle, éthyle, isopropyle, cyclopropyle, cyclopentyle, cyclohexyle, cycloheptyle, 4-hydroxycarbonylméthyl-cylohexyle, 4-éthoxycarbonylméthyl-cyclohexyle, cyclopropylméthyle, 2-diéthylamino-propyle, 1-quinuclidin-3-yle, tétrahydropyran-4-yle, 1-pipéridin-4-yle, 1-méthyl-pipéridin-4-yle, 1-éthyl-pipéridin-4-yle, 1-(2-hydroxyéthyl)-pipéridin-4-yle, 1-méthylsulfonyl-pipéridin-4-yle, 1-aminosulfonyl-pipéridin-4-yle, 1-(méthylaminosulfonyl)-pipéridin-4-yle, 1-(diméthylaminosulfonyl)-pipéridin-4-yle, 1-hydroxycarbonylméthyl-pipéridin-4-yle, 1-éthoxycarbonylméthyl-pipéridin-4-yle, 1-(2-hydroxycarbonyléthyl)-pipéridin-4-yle, 1-(2-éthoxycarbonyléthyl)-pipéridin-4-yle, 1-(3-hydroxycarbonyl-propionyl)-pipéridin-4-yle, 1-(3-éthoxycarbonyl-propionyl)-pipéridin-4-yle, 1-(hydroxycarbamoyl-méthyl)-pipéridin-4-yle, 1-(hydroxy-méthyl-carbamoyl-méthyl)-pipéridin-4-yle, 1-(méthoxycarbamoyl-méthyl)-pipéridin-4-yle, 1 -cyclopropyl-pipéridin-4-yle, 1-cyclopropylméthyl-pipéridin-4-yle, 1-hydroxycarbonylméthyl-pipéridin-4-yle ou 1-éthoxycarbonylméthyl-pipéridin-4-yle et
R⁵ représente une paire d'électrons libres,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

11. Antagonistes CGRP de formule générale (I) selon la revendication 1, dans lesquels
A, X, D, E, G, M, Q et R¹ sont tels que définis dans l'une des revendications 1 à 6 et R² est un groupe phénylméthyle ou un groupe alkyle en C₂ à C₇ qui peut être substitué en position ω par un groupe phényle, amino, alkylamino en C₁ à C₆, di-(alkyle en C₁ à C₆)-amino,
les groupes phényle et phénylméthyle mentionnés précédemment pouvant être substitués au niveau d'un atome de carbone aromatique, par un groupe amino-alkyle en C₁ à C₃, alkylamino en C₁ à C₃-alkyle en C₁ à C₃, ou di-(alkyle en C₁ à C₃)-amino-alkyle en C₁ à C₃,
R³ est un atome d'hydrogène ou un groupe alkyle en C₁ à C₃, ou
R² et R³ forment conjointement avec l'atome d'azote inclus, un radical de formule générale 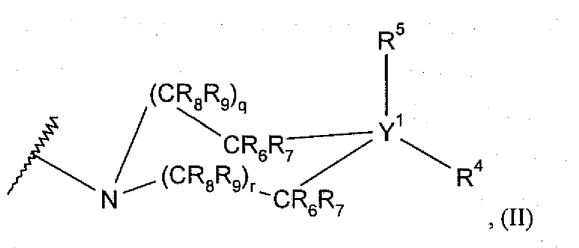 dans laquelle
R⁶ et R⁷ sont respectivement, un atome d'hydrogène ou un groupe diméthylamino,
R⁸ et R⁹ sont respectivement, un atome d'hydrogène et
(a) Y¹ est l'atome de carbone,
q et r sont les chiffres 0 ou 1
R⁴ est l'atome d'hydrogène,
un groupe phényle, pyridinyle ou pyrimidinyle qui peut être substitué respectivement par un atome d'halogène, par un groupe amino, methylamino, diméthylamino, méthyle ou méthoxy,
un groupe hydroxy, 2-diéthylamino-éthyle, amino, méthylamino, diméthylamino, diéthylamino, pyrrolidin-1-yle, pipéridin-1-yle, 4-amino-pipéridin-1-yle, 4-méthylamino-pipéridin-1-yle, 4-diméthylamino-pipéridin-1-yle, 3-amino-pipéridin-1-yle, 3-méthylamino-pipéridin-1-yle, 3-diméthylamino-pipéridin-1-yle, perhydro-azépin-1-yle, perhydro-1,4-diazépin-1-yle, 4-méthyl-perhydro-1,4-diazépin-1-yle, 1-méthyl-pipéridin-4-yle, pipéridin-4-yle, 1-éthylpipéridin-4-yle, 1-cyclopropyl-pipéridin-4-yle, 1-cyclopropylméthyl-pipéridin-4-yle, pipérazin-1-yle, 4-méthyl-pipérazin-1-yle, 4-cyclopropylméthyl-pipérazin-1-yle, 4-éthyl-pipérazin-1-yle, 4-cyclopropyl-pipérazin-1-yle, 1,2-diméthyl-pipérazin-1-yle, 3-méthyl-pipérazin-1-yle, 3,4,5-triméthyl-pipérazin-1-yle, 3,5-diméthyl-pipérazin-1-yle, 3,3,4-triméthyl-pipérazin-1-yle, 3,3-diméthyl-pipérazin-1-yle, 3,3,4,5,5-pentaméthyl-pipérazin-1-yle, 8-méthyl-8-aza-bicyclo[3.2.1]oct-3-yle, 8-aza-bicyclo[3.2.1]oct-3-yle, azétidin-1-yle, 1-(méthoxycarbonylméthyl)-pipéridin-4-yle, 1-(éthoxycarbonylméthyl)-pipéridin-4-yle, 4-(éthoxycarbonylméthyl)-pipérazin-1-yle, 1-hydroxycarbonylméthyl-pipéridin-4-yle ou 4-hydroxycarbonylméthyl-pipérazin-1-yle, et R⁵ est un atome d'hydrogène, ou
(b) Y¹ est un atome d'azote,
q et r sont les chiffres 1 ou 2,
R⁴ est l'atome d'hydrogène,
un groupe phényle, pyridinyle ou pyrimidinyle qui peut être substitué respectivement par un atome d'halogène, un groupe amino, méthylamino, diméthylamino, méthyle ou méthoxy,
un groupe méthyle, éthyle, isopropyle, cyclopropyle, cyclopropylméthyle, 2-diéthylamino-propyle, 1-quinuclidin-3-yle, 1-pipéridin-4-yle, 1-méthyl-pipéridin-4-yle, 1-éthyl-pipéridin-4-yle, 1-cyclopropyl-pipéridin-4-yle, 1-cyclopropylméthyl-pipéridin-4-yle, 1-hydroxycarbonylméthyl-pipéridin-4-yle ou 1-éthoxycarbonylméthyl-pipéridin-4-yle et
R⁵ représente une paire d'électrons libres,
leurs tautomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

12. Antagonistes CGRP de formule générale (I) selon la revendication 1, dans lesquels
D, E, G, M, Q, R¹, R² et R³ sont tels que définis dans l'une des revendications 1 à 10 et
A et X sont respectivement un atome d'oxygène,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

13. Antagonistes CGRP de formule générale (I) selon la revendication 1, dans lesquels
A et X sont respectivement un atome d'oxygène,
R¹ est un groupe 1,3,4,5-tétrahydro-1,3-benzodiazépin-2-on-3-yle, 3,4-dihydro-1*H-*quinazolin-2-on-3-yle, 5-phényl-2,4-dihydro-1,2,4-triazol-3-on-2-yle, 1,3-dihydro-imidazo[4,5-*c*]quinolin-2-on-3-yle, 1,3-dihydro-napht[1,2-*d*]imidazol-2-on-3-yle, 1,3-dihydro-benzimidazol-2-on-3-yle, 4-phényl-1,3-dihydro-imidazol-2-on-1-yle, 3,4-dihydro-1*H*-thiéno[3,2-*d*]pyrimidin-2-on-3-yle ou 3,4-dihydro-1*H*-thiéno[3,4-*d*]pyrimidin-2-on-3-yle,
et R² et R³ sont tels que définis dans la revendication 1 ou 2,
les hétérocycles mentionnés précédemment pour R¹ pouvant être substitués en outre dans le squelette de carbone par un groupe méthoxy,
et tous les radicaux et parties de molécules aromatiques et hétéroaromatiques mentionnés ou contenus dans les radicaux définis précédemment pour R¹, pouvant en outre être mono-, di ou tri-substitués par des atomes d'halogène, des groupes cyano ou hydroxy et les substituants pouvant être identiques ou différents,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

14. Antagonistes CGRP de formule générale (I) selon la revendication 1, dans lesquels
A et X sont respectivement un atome d'oxygène,
R¹ est tel que défini dans la revendication 1,
D et E sont respectivement un groupe méthine,
G est un groupe méthine substitué par le groupe R^{a},
M est un groupe méthine substitué par le groupe R^{b},
Q est un groupe méthine substitué par le groupe R^{c} et
R^{a}, R^{b} et R^{c} sont respectivement, indépendamment les uns des autres, un atome d'hydrogène ou d'halogène, un groupe méthyle, difluorméthyle, trifluorméthyle, éthyle, vinyle, éthinyle, cyano, hydroxy, méthoxy, difluorméthoxy, trifluorméthoxy, amino, méthylamino ou dimethylamino,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

15. Antagonistes CGRP de formule générale (I) selon la revendication 1, dans lesquels
A et X sont respectivement un atome d'oxygène,
D et E sont respectivement un groupe méthine,
G est un groupe méthine substitué par le groupe R^{a},
M est un groupe méthine substitué par le groupe R^{b},
Q est un groupe méthine substitué par le groupe R^{c} et
R^{a}, R^{b} et R^{c} sont respectivement, indépendamment les uns des autres, un atome d'hydrogène ou d'halogène, un groupe alkyle en C₁ à C₃, trifluorméthyle, cyano, hydroxy, méthoxy, trifluorméthoxy, amino, méthylamino ou diméthylamino,
R¹ est un groupe 1,3,4,5-tétrahydro-1,3-benzodiazépin-2-on-3-yle, 3,4-dihydro-1*H-*quinazolin-2-on-3-yle, 5-phényl-2,4-dihydro-1,2,4-triazol-3-on-2-yle, 1,3-dihydro-imidazo[4,5-*c*]quinolin-2-on-3-yle, 1,3-dihydro-napht[1,2-*d*]imidazol-2-on-3-yle, 1,3-dihydro-benzimidazol-2-on-3-yle, 4-phényl-1,3-dihydro-imidazol-2-on-1-yle, 3,4-dihydro-1*H*-thiéno[3,2-*d*]pyrimidin-2-on-3-yle ou 3,4-dihydro-1*H*-thiéno[3,4-*d*]pyrimidin-2-on-3-yle qui sont mono-, di- ou trisubstitués au niveau d'un atome de carbone insaturé de la partie aromatique ou hétéroaromatique par un atome d'halogène, par un groupe cyano ou hydroxy et les substituants peuvent être identiques ou différents, de préférence toutefois, ils ne sont pas substitués, R² et R³ conjointement avec l'atome d'azote inclus forment un radical de formule générale 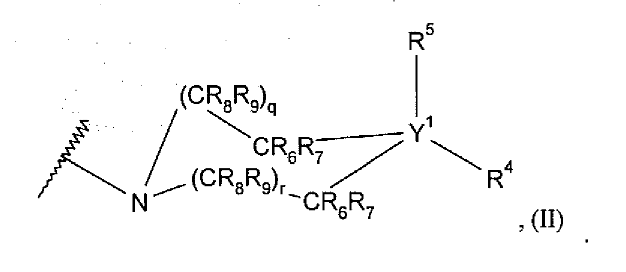 dans laquelle
Y¹ est l'atome de carbone ou, si R⁵ est une paire d'électrons libres, également l'atome d'azote,
q et r, si Y¹ est l'atome de carbone, sont les chiffres 0, 1 ou 2, le total de q et r étant de 1, 2 ou 3,
q et r, si Y¹ est l'atome d'azote, sont les chiffres 1 ou 2, le total de q et r étant de 2 ou 3,
R⁴ étant l'atome d'hydrogène, un groupe amino, alkyle en C₁ à C₄-amino, di-(alkyle en C₁ à C₄)-alkylamino, alkyle en C₁ à C₆, un groupe cyclo-alkyle en C₃ à C₇, amino-alkyle en C₂ à C₇, alkyle en C₁ à C₄-amino-alkyle en C₂ à C₇, di-(alkyle en C₁ à C₄ - amino)-alkyle en C₂ à C₇, alkoxycarbonyle en C₁ à C₆, alcoxycarbonyle en C₁ à C₆-alkyle en C₁ à C₃, ou hydroxycarbonyle-alkyle en C₁ à C₃, éventuellement substitués par un groupe hydroxycarbonyle, alcoxycarbonyle en C₁ à C₆, hydroxycarbonyle-alkyle en C₁ à C₃ ou alcoxycarbonyle en C₁ à C₆-alkyle en C₁ à C₃ un groupe phényle ou pyridyle qui peut être substitué respectivement par un atome d'halogène, par un groupe alkyle en C₁ à C₃, alcoxy en C₁ à C₃, amino, alkyle en C₁ à C₄-amino, ou di-(alkyle en C₁ à C₄)-amino,
un hétérocycle choisi dans un groupe azacycloalkyle ou S,S-dioxothiaza de 5 à 7 chaînons, et un groupe diazacycloalkyle de 6 à 7 chaînons,
les hétérocycles précédemment cités étant liés par un atome d'azote ou un atome de carbone à Y¹ dans la formule (II),
pouvant être substitués par un ou deux groupes hydroxy, alkyle en C₁ à C₃, ou hydroxy-alkyle en C₁ à C₃, ou par un groupe cyclo-alkyl en C₃ à C₆, hydroxy-cycloalkyle en C₃ à C₆, cyclo-alkyle en C₃ à C₆-alkyle en C₁ à C₃, alkylcarbonyle en C₁ à C₃-alkyle en C₁ à C₃, amino, alkylamino en C₁ à C₄, di-(alkyle en C₁ à C₄)-amino, hydroxycarbonyl-carbonyle, alcoxycarbonyle en C₁ à C₃-carbonyle, hydroxycarbonyl-alkylcarbonyle en C₁ à C₃, alcoxycarbonyle en C₁ à C₃-alkylcarbonyle en C₁ à C₃, aminosulfonyle, alkylaminosulfonyle en C₁ à C₄, di-(alkyle en C₁ à C₄)-aminosulfonyle, cyclo-alkylsulfonyle en C₃ à C₇, aminocarbonyl-alkyle en C₁ à C₃, alkylaminocarbonyle en C₁ à C₄-alkyle en C₁ à C₃, di-(alkyle en C₁ à C₄)-aminocarbonyl-alkyle en C₁ à C₃, hydroxyaminocarbonyl-alkyle en C₁ à C₃, alcoxyaminocarbonyle en C₁ à C₃, alkyle en C₁ à C₃, ou hydroxy-(alkyle en C₁ à C₃)-aminocarbonyl-alkyle en C₁ à C₃,
R⁵ est un atome d'hydrogène ou si Y¹ est un atome d'azote, une paire d'électrons libres et
R⁶, R⁷, R⁸ et R⁹ qui peuvent être identiques ou différents, sont respectivement un atome d'hydrogène ou un groupe alkyle en C₁ à C₃,
leurs tautomères, leurs isomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

16. Antagonistes CGRP de formule générale (1) selon la revendication 1, dans lesquels
A et X sont respectivement un atome d'oxygène,
D et E sont respectivement un groupe méthine,
G est un groupe méthine substitué par le groupe R^{a},
M est un groupe méthine substitué par le groupe R^{b},
Q est un groupe méthine substitué par le groupe R^{c} et
R^{a}, R^{b} et R^{c} sont respectivement, indépendamment les uns des autres, un atome d'hydrogène ou d'halogène, un groupe alkyle en C₁ à C₃, trifluorméthyle, cyano, hydroxy, méthoxy, trifluorméthoxy, amino, méthylamino ou diméthylamino,
R¹ est un groupe 1,3,4,5-tétrahydro-1,3-benzodiazépin-2-on-3-yle, 3,4-dihydro-1*H-*quinazolin-2-on-3-yle, 5-phényl-2,4-dihydro-1,2,4-triazol-3-on-2-yle, 1,3-dihydro-imidazo[4,5-*c*]quinolin-2-on-3-yle, 1,3-dihydro-napht[1,2-*d*]imidazol-2-on-3-yle, 1,3-dihydro-benzimidazol-2-on-3-yle, 4-phényl-1,3-dihydro-imidazol-2-on-1-yle, 3,4-dihydro-1*H*-thieno[3,2-*d*]pyrimidin-2-on-3-yle ou 3,4-dihydro-1*H*-thiéno[3,4-*d*]pyrimidin-2-on-3-yle qui sont mono-, di- ou trisubstitués au niveau d'un atome de carbone insaturé de la partie aromatique ou hétéroaromatique par un atome d'halogène, par un groupe cyano ou hydroxy et les substituants peuvent être identiques ou différents, de préférence toutefois, ils ne sont pas substitués,
R² et R³ conjointement avec l'atome d'azote inclus forment un radical de formule générale 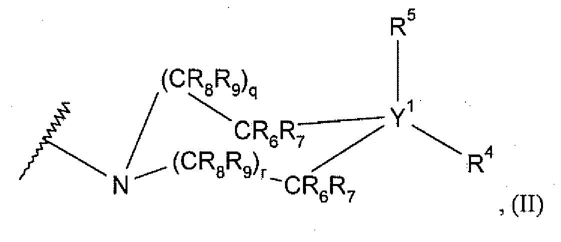 Y¹ est l'atome de carbone ou, si R⁵ est une paire d'électrons libres, également l'atome d'azote,
q et r, si Y¹ est l'atome de carbone, sont les chiffres 0, 1 ou 2, le total de q et r étant de 1, 2 ou 3,
q et r, si Y¹ est l'atome d'azote, sont les chiffres 1 ou 2, le total de q et r étant de 2 ou 3,
R⁴ étant l'atome d'hydrogène, un groupe amino, alkyle en C₁ à C₄-amino, di-(alkyle en C₁ à C₄)-alkylamino, alkyle en C₁ à C₆, cyclo-alkyle en C₃ à C₇, amino-alkyle en C₂ à C₇, alkyle en C₁ à C₄-amino-alkyle en C₂ à C₇, di-(alkyle en C₁ à C₄ -amino)-alkyle en C₂ à C₇, alcoxycarbonyle en C₁ à C₆, alcoxycarbonyle en C₁ à C₆-alkyle en C₁ à C₃; ou hydroxycarbonyle-alkyle en C₁ à C₃,
un groupe phényle ou pyridyle qui peut être substitué respectivement par un atome d'halogène, par un groupe alkyle en C₁ à C₃, alcoxy en C₁ à C₃, amino, alkyle en C₁ à C₄-amino, ou di-(alkyleen C₁ à C₄)-amino,
un hétérocycle choisi dans un groupe azacycloalkyle de 5 à 7 chaînons, et un groupe diazacycloalkyle de 6 à 7 chaînons,
les hétérocycles précédemment cités étant liés par un atome d'azote ou un atome de carbone à Y¹ dans la formule (II),
pouvant être substitués par un groupe alkyle en C₁ à C₃ cyclo-alkyl en C₃ à C₆, cyclo-alkyle en C₃ à C₆-alkyle en C₁ à C₃, amino, alkylamino en C₁ à C₄ ou di-(alkyle en C₁ à C₄)-amino,
R⁵ est un atome d'hydrogène ou si Y¹ est un atome d'azote, une paire d'électrons libres et
R⁶, R⁷, R⁸ et R⁹ qui peuvent être identiques ou différents, sont respectivement un atome d'hydrogène ou un groupe alkyle en C₁ à C₃,
leurs tautomères, leurs diastéréomères, leurs énantiomères, leurs hydrates, leurs mélanges et leurs sels et les hydrates des sels, en particulier leurs sels physiologiquement acceptables avec des acides ou bases anorganiques ou organiques.

17. Composés suivants de formule générale 1 selon la revendication 1 :
| | Structure |
|---|---|
| (1) | 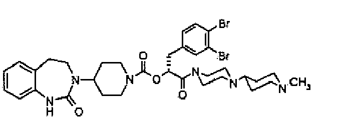 |
| (2) | 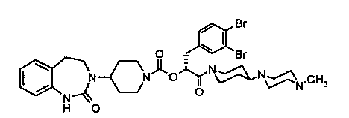 |
| (3) | 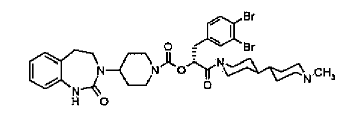 |
| (4) | 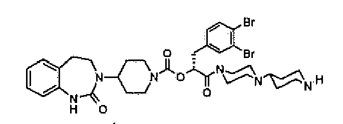 |
| (5) | 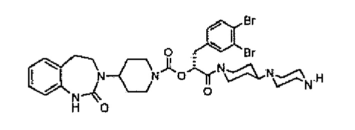 |
| (6) | 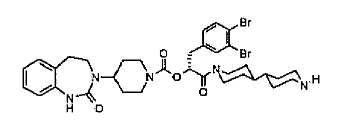 |
| (7) | 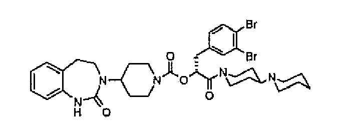 |
| (8) | 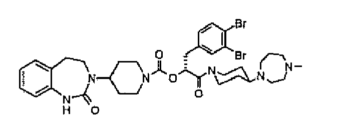 |
| (9) | 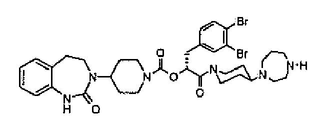 |
| (10) | 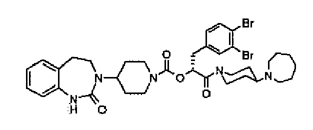 |
| (11) | 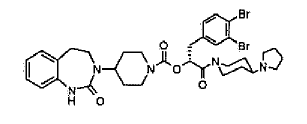 |
| (12) | 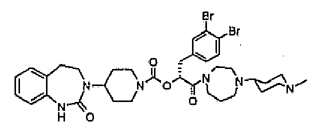 |
| (13) | 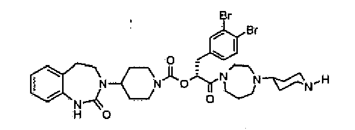 |
| (14) | 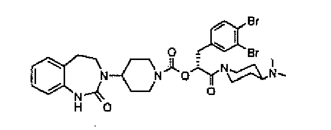 |
| (15) | 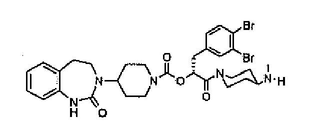 |
| (16) | 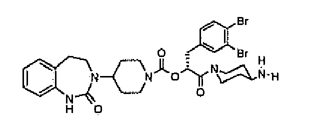 |
| (17) | 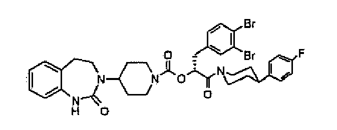 |
| (18) | 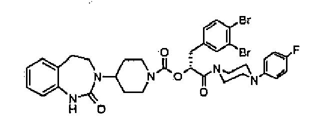 |
| (19) | 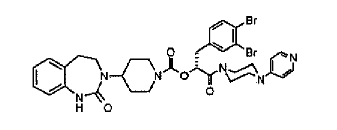 |
| (20) | 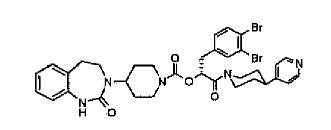 |
| (21) | 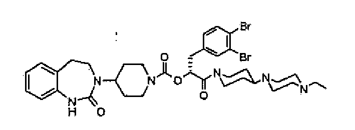 |
| (22) | 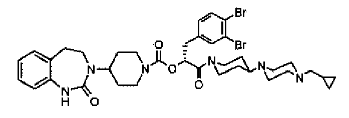 |
| (23) | 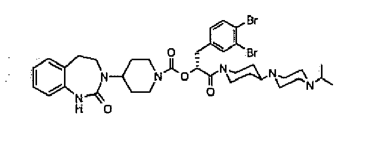 |
| (24) | 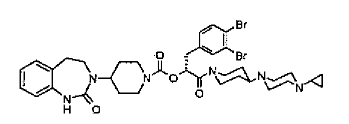 |
| (25) | 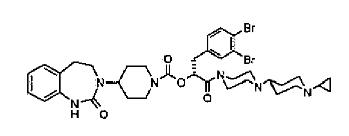 |
| (26) | 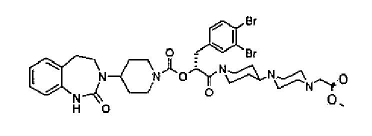 |
| (27) | 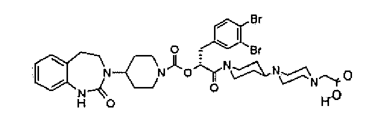 |
| (28) | 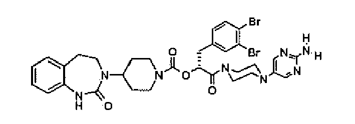 |
| (29) | 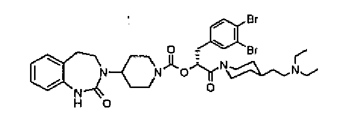 |
| (30) | 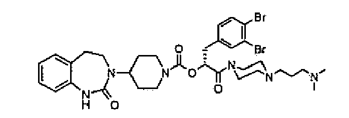 |
| (31) | 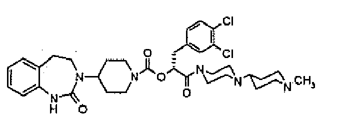 |
| (32) | 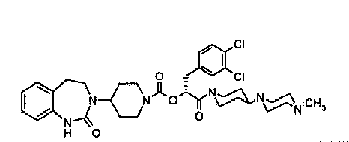 |
| (33) | 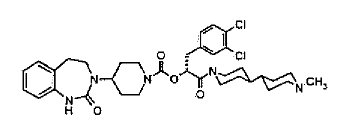 |
| (34) | 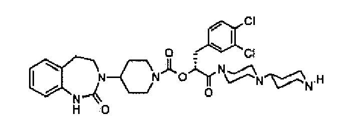 |
| (35) | 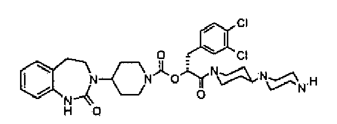 |
| (36) | 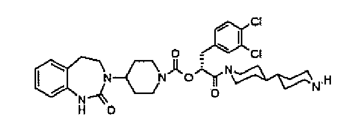 |
| (37) | 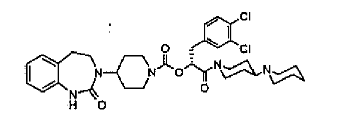 |
| (38) | 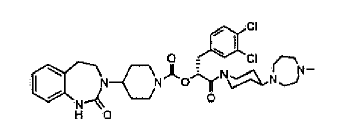 |
| (39) | 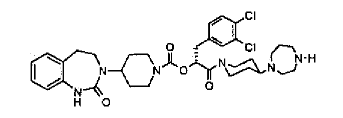 |
| (40) | 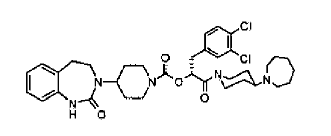 |
| (41) | 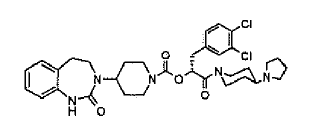 |
| (42) | 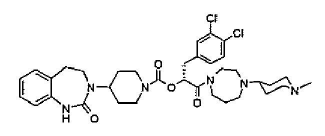 |
| (43) | 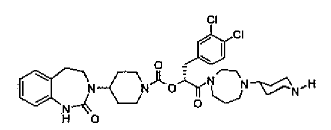 |
| (44) | 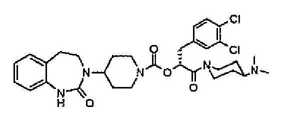 |
| (45) | 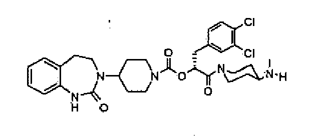 |
| (46) | 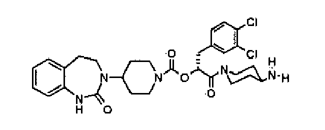 |
| (47) | 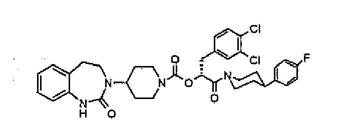 |
| (48) | 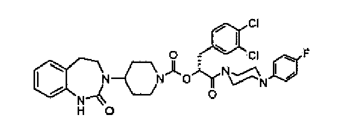 |
| (49) | 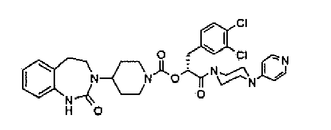 |
| (50) | 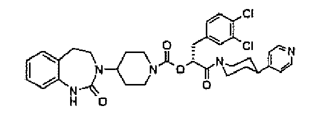 |
| (51) | 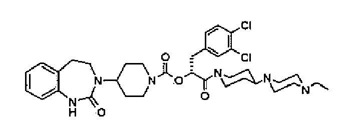 |
| (52) | 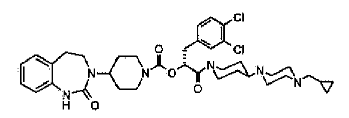 |
| (53) | 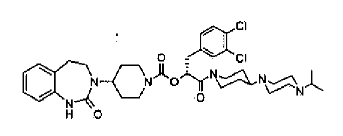 |
| (54) | 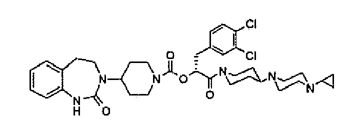 |
| (55) | 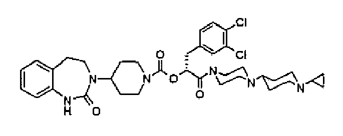 |
| (56) | 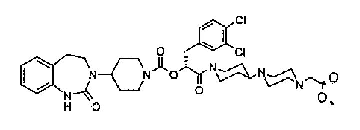 |
| (57) | 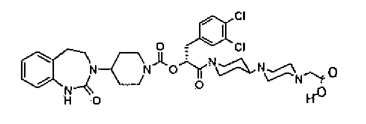 |
| (58) | 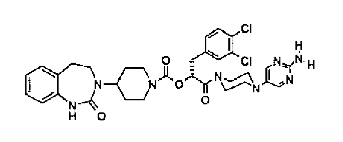 |
| (59) | 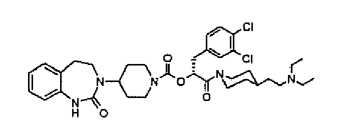 |
| (60) | 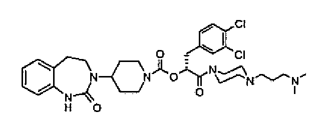 |
| (61) | 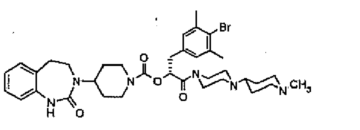 |
| (62) | 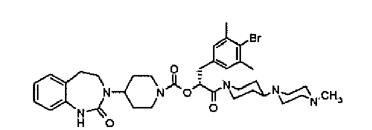 |
| (63) | 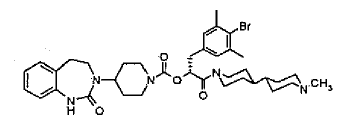 |
| (64) | 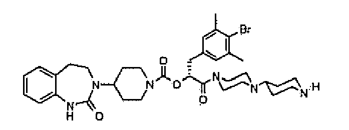 |
| (65) | 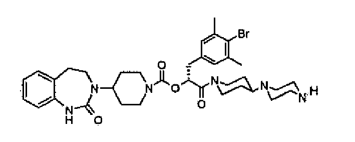 |
| (66) | 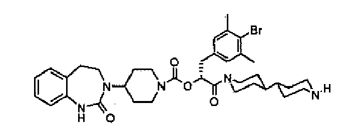 |
| (67) | 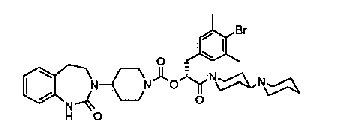 |
| (68) | 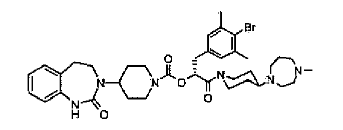 |
| (69) | 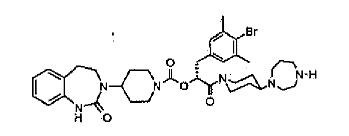 |
| (70) | 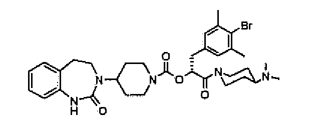 |
| (71) | 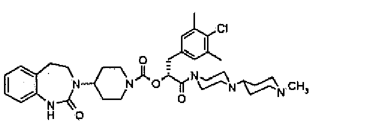 |
| (72) | 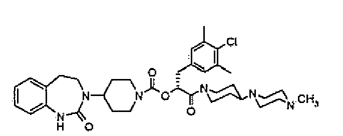 |
| (73) | 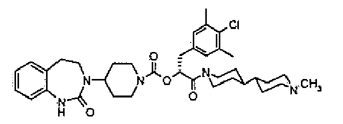 |
| (74) | 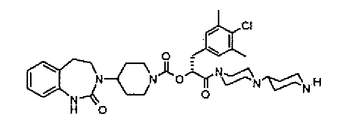 |
| (75) | 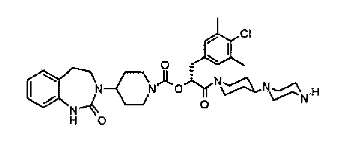 |
| (76) | 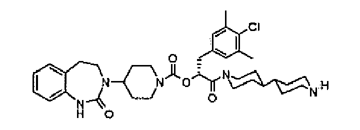 |
| (77) | 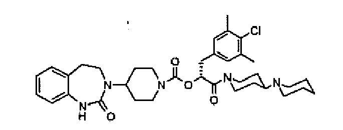 |
| (78) | 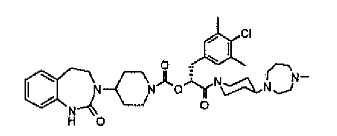 |
| (79) | 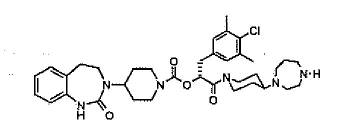 |
| (80) | 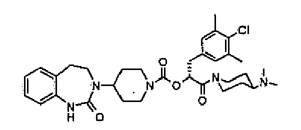 |
| (81) | 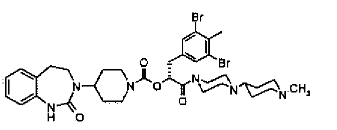 |
| (82) | 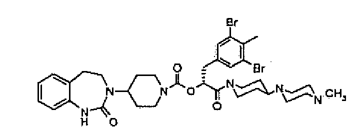 |
| (83) | 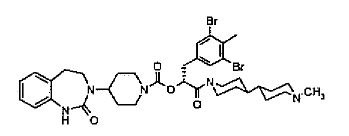 |
| (84) | 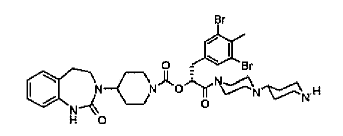 |
| (85) | 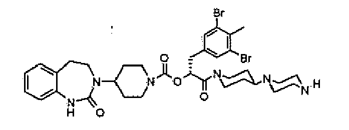 |
| (86) | 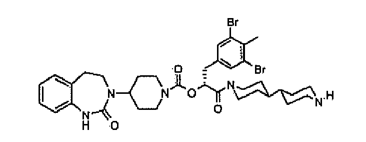 |
| (87) | 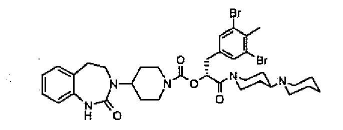 |
| (88) | 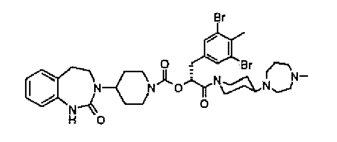 |
| (89) | 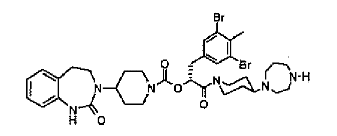 |
| (90) | 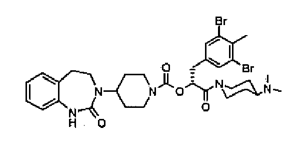 |
| (91) | 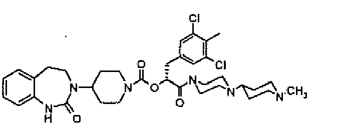 |
| (92) | 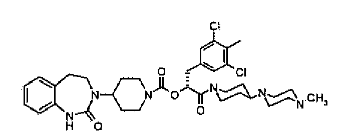 |
| (93) | 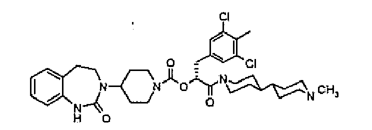 |
| (94) | 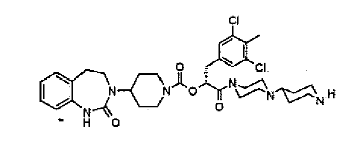 |
| (95) | 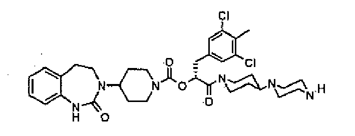 |
| (96) | 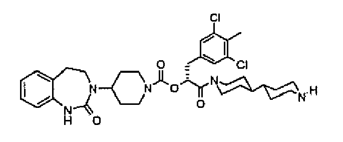 |
| (97) | 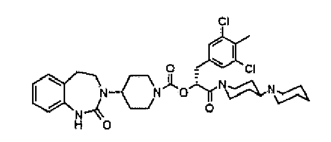 |
| (98) | 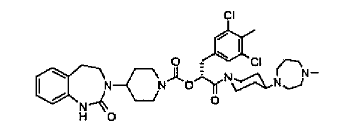 |
| (99) | 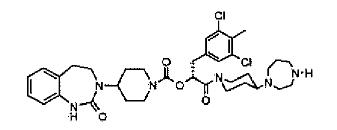 |
| (100) | 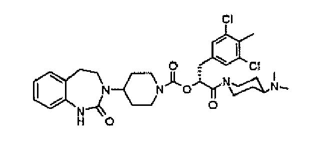 |
| (101) | 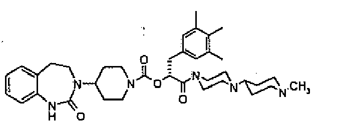 |
| (102) | 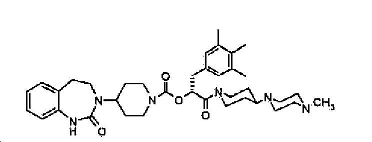 |
| (103) | 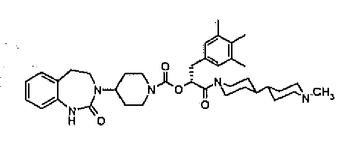 |
| (104) | 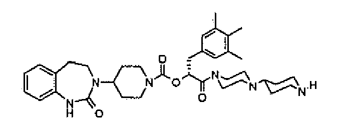 |
| (105) | 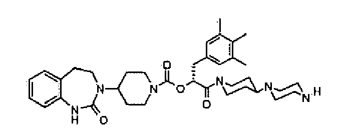 |
| (106) | 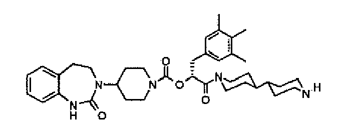 |
| (107) | 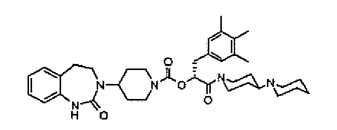 |
| (108) | 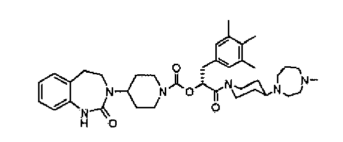 |
| (109) | 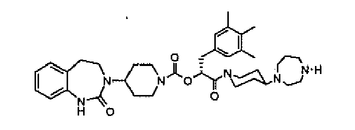 |
| (110) | 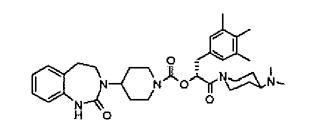 |
| (111) | 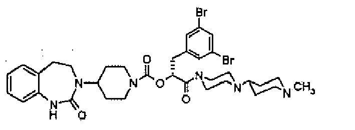 |
| (112) | 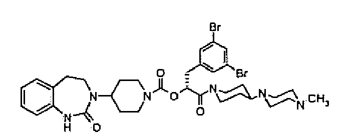 |
| (113) | 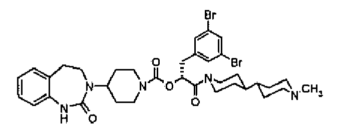 |
| (114) | 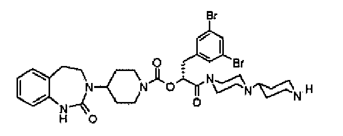 |
| (115) | 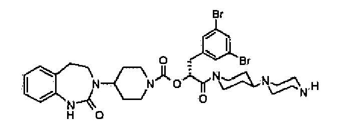 |
| (116) | 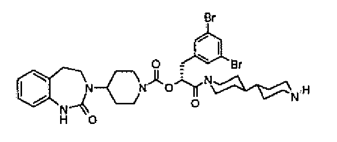 |
| (117) | 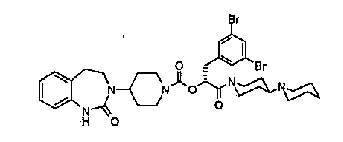 |
| (118) | 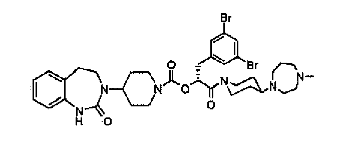 |
| (119) | 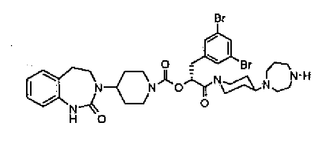 |
| (120) | 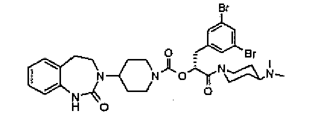 |
| (121) | 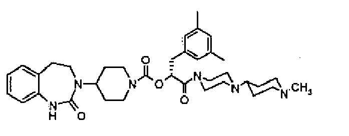 |
| (122) | 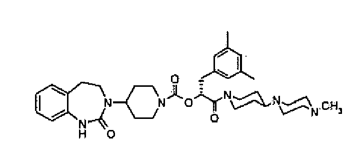 |
| (123) | 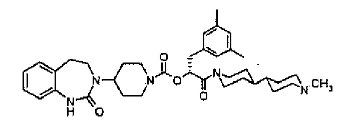 |
| (124) | 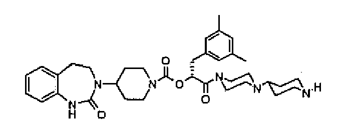 |
| (125) | 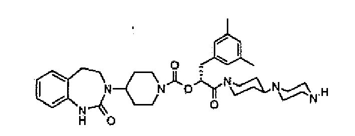 |
| (126) | 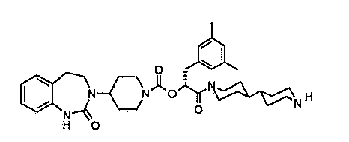 |
| (127) | 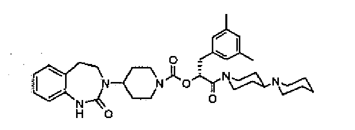 |
| (128) | 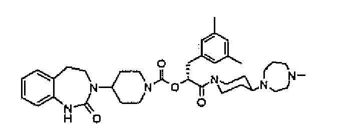 |
| (129) | 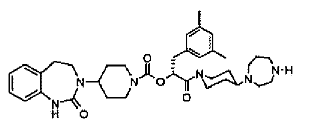 |
| (130) | 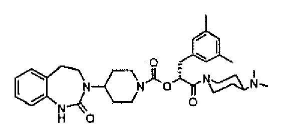 |
| (131) | 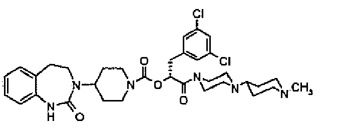 |
| (132) | 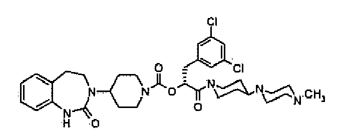 |
| (133) | 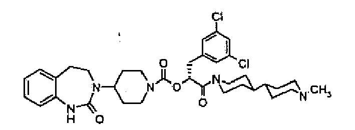 |
| (134) | 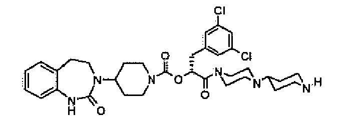 |
| (135) | 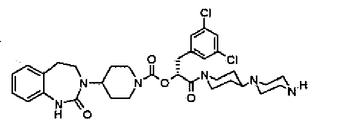 |
| (136) | 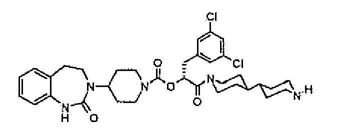 |
| (137) | 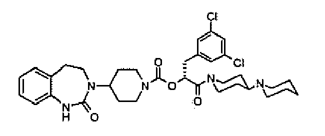 |
| (138) | 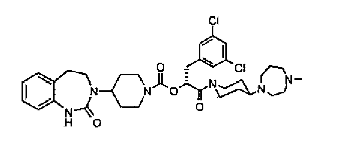 |
| (139) | 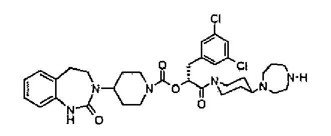 |
| (140) | 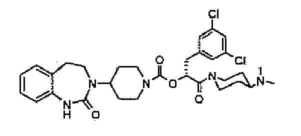 |
| (141) | 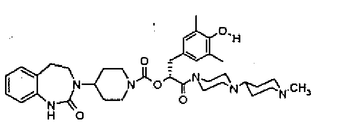 |
| (142) | 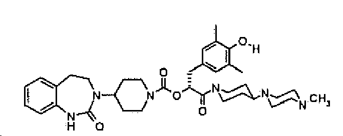 |
| (143) | 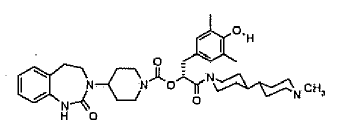 |
| (144) | 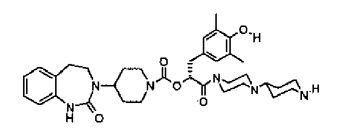 |
| (145) | 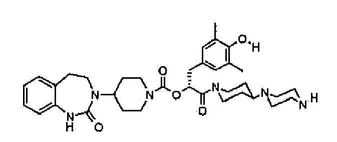 |
| (146) | 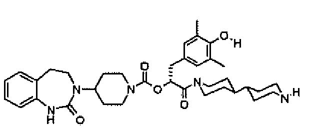 |
| (147) | 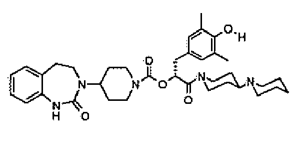 |
| (148) | 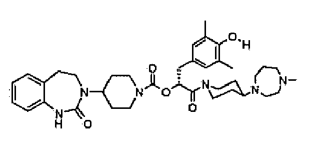 |
| (149) | 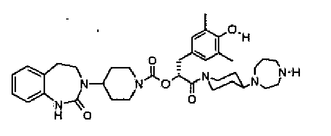 |
| (150) | 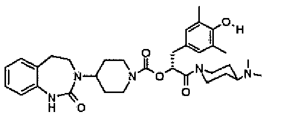 |
| (151) | 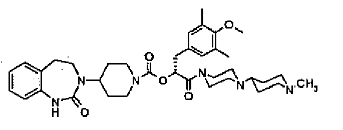 |
| (152) | 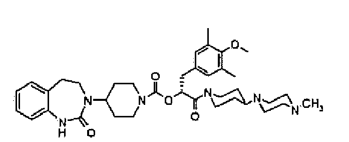 |
| (153) | 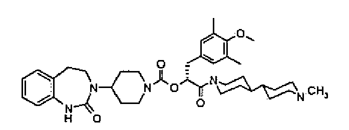 |
| (154) | 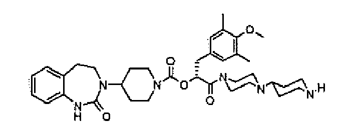 |
| (155) | 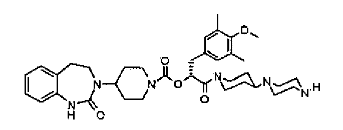 |
| (156) | 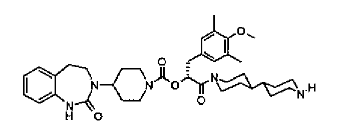 |
| (157) | 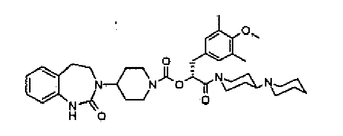 |
| (158) | 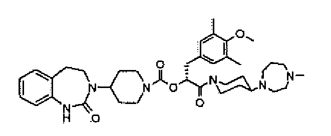 |
| (159) | 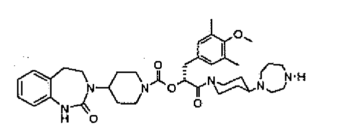 |
| (160) | 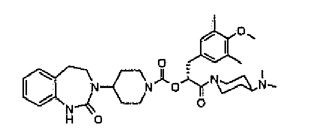 |
| (161) | 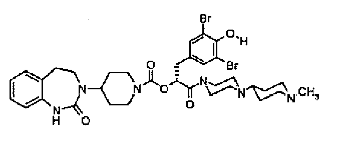 |
| (162) | 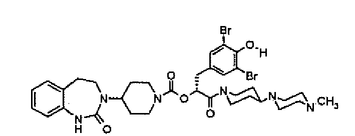 |
| (163) | 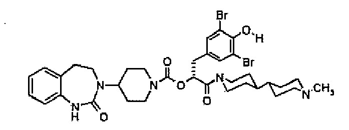 |
| (164) | 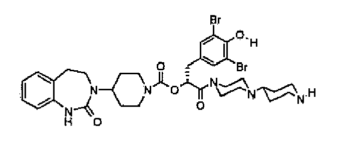 |
| (165) | 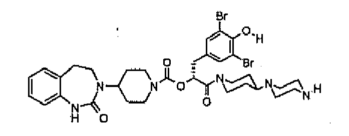 |
| (166) | 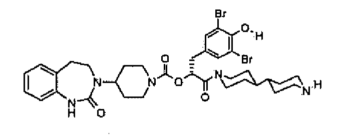 |
| (167) | 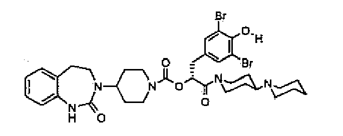 |
| (168) | 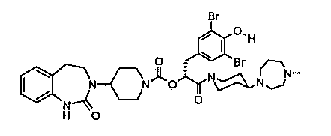 |
| (169) | 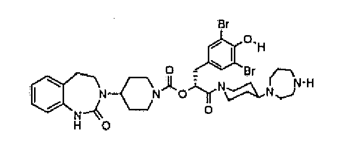 |
| (170) | 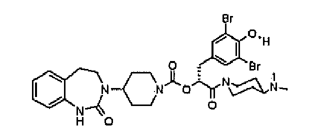 |
| (171) | 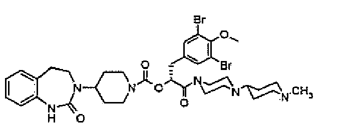 |
| (172) | 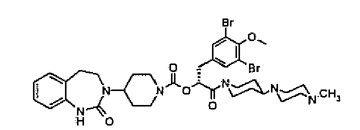 |
| (173) | 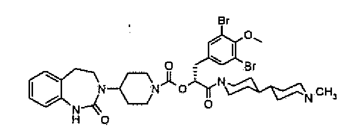 |
| (174) | 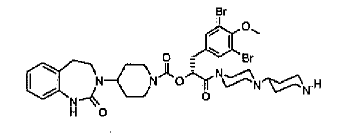 |
| (175) | 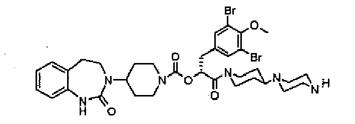 |
| (176) | 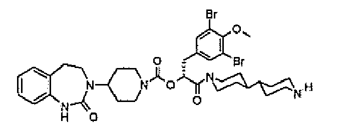 |
| (177) | 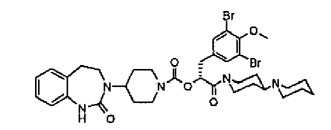 |
| (178) | 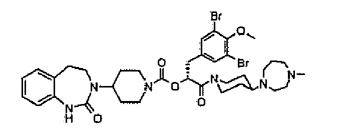 |
| (179) | 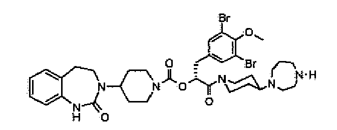 |
| (180) | 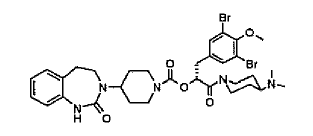 |
| (181) | 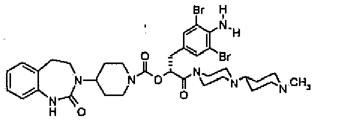 |
| (182) | 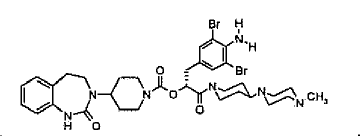 |
| (183) | 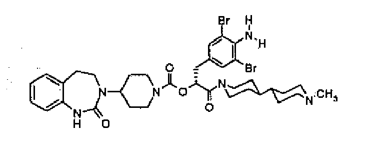 |
| (184) | 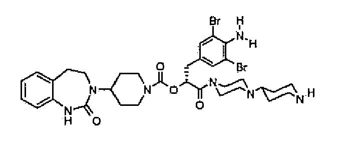 |
| (185) | 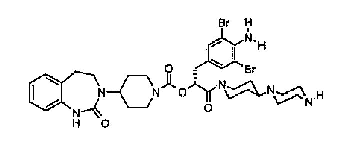 |
| (186) | 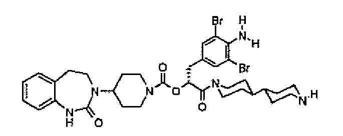 |
| (187) | 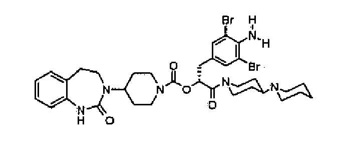 |
| (188) | 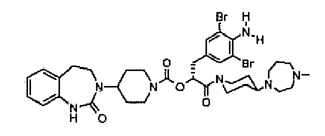 |
| (189) | 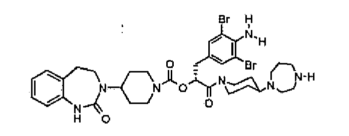 |
| (190) | 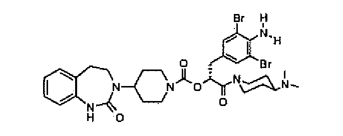 |
| (191) | 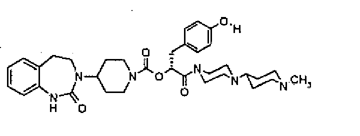 |
| (192) | 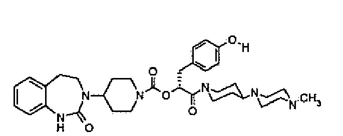 |
| (193) | 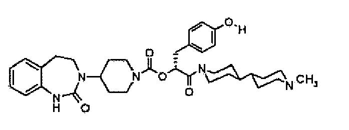 |
| (194) | 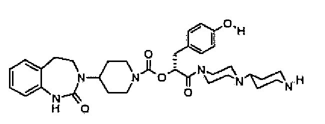 |
| (195) | 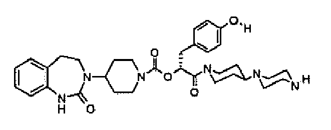 |
| (196) | 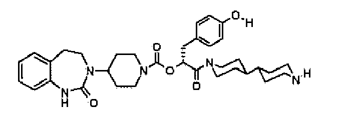 |
| (197) | 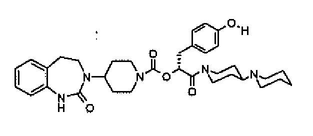 |
| (198) | 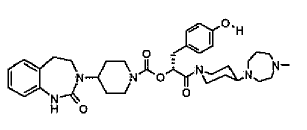 |
| (199) | 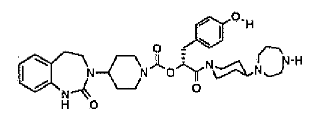 |
| (200) | 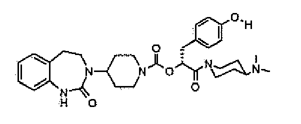 |
| (201) | 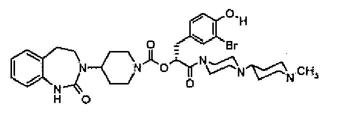 |
| (202) | 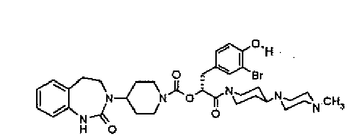 |
| (203) | 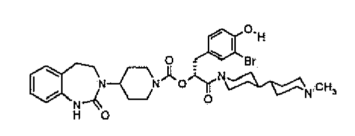 |
| (204) | 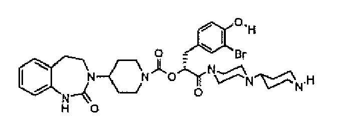 |
| (205) | 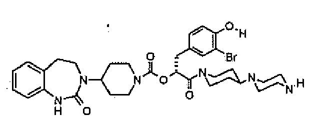 |
| (206) | 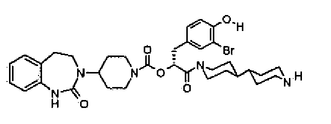 |
| (207) | 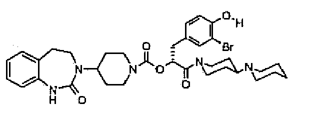 |
| (208) | 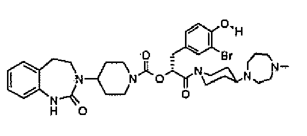 |
| (209) | 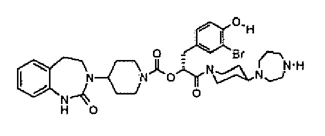 |
| (210) | 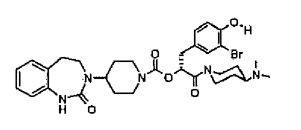 |
| (211) | 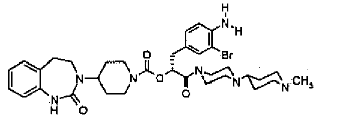 |
| (212) | 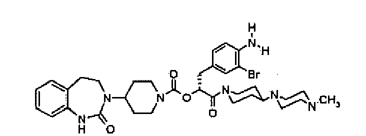 |
| (213) | 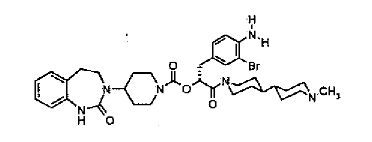 |
| (214) | 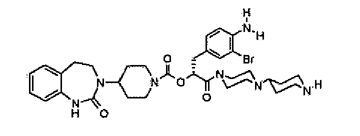 |
| (215) | 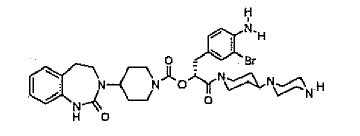 |
| (216) | 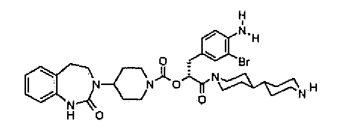 |
| (217) | 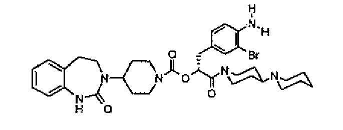 |
| (218) | 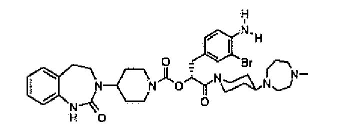 |
| (219) | 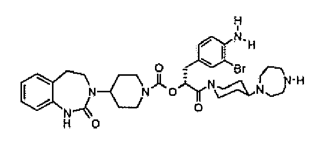 |
| (220) | 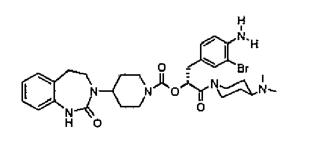 |
| (221) | 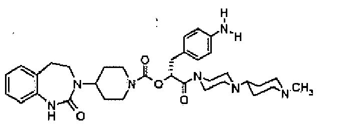 |
| (222) | 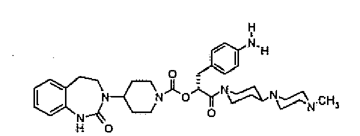 |
| (223) | 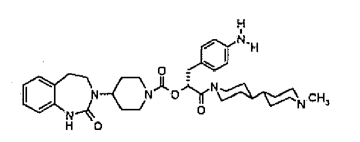 |
| (224) | 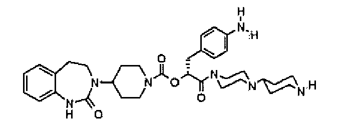 |
| (225) | 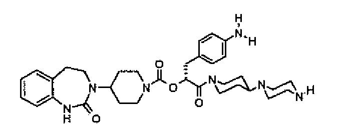 |
| (226) | 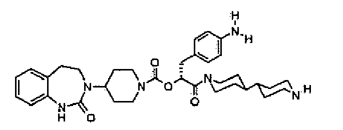 |
| (227) | 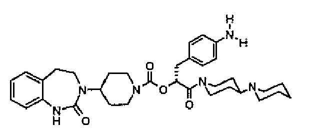 |
| (228) | 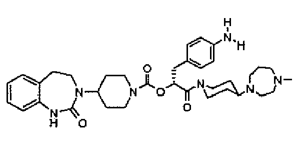 |
| (229) | 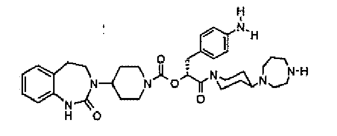 |
| (230) | 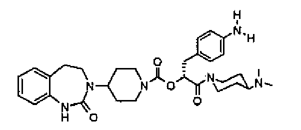 |
| (231) | 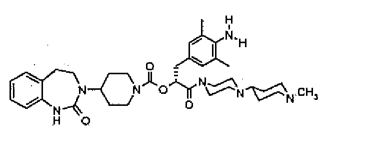 |
| (232) | 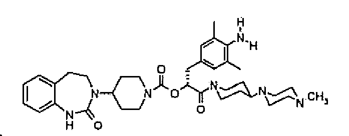 |
| (233) | 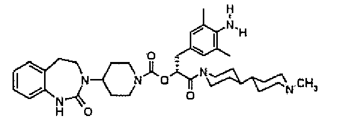 |
| (234) | 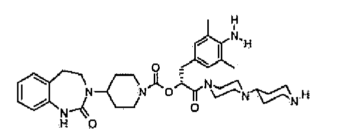 |
| (235) | 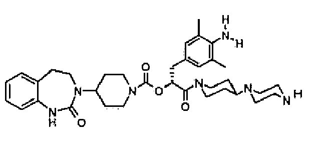 |
| (236) | 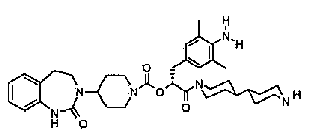 |
| (237) | 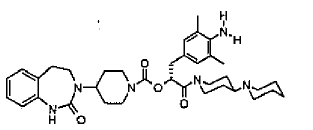 |
| (238) | 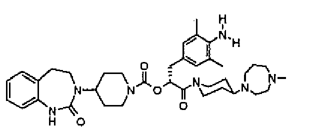 |
| (239) | 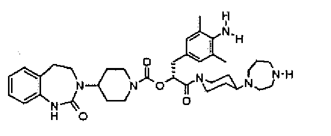 |
| (240) | 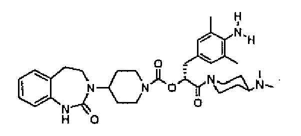 |
| (241) | 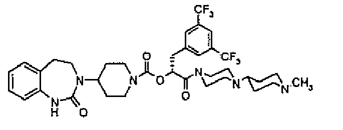 |
| (242) | 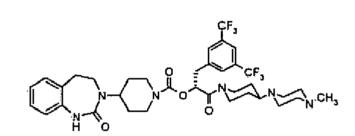 |
| (243) | 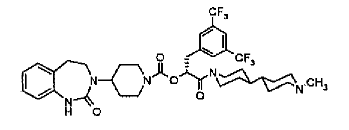 |
| (244) | 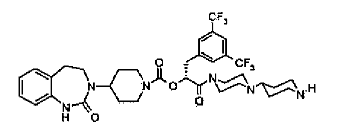 |
| (245) | 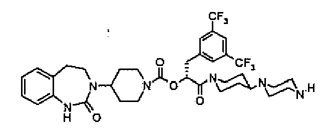 |
| (246) | 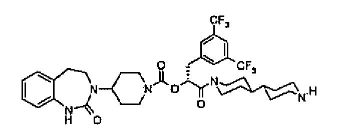 |
| (247) | 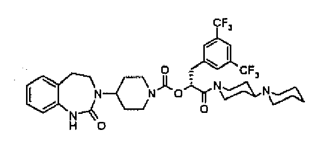 |
| (248) | 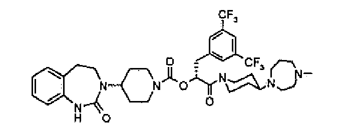 |
| (249) | 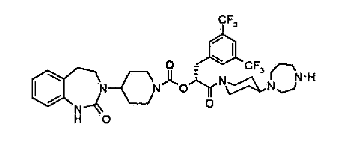 |
| (250) | 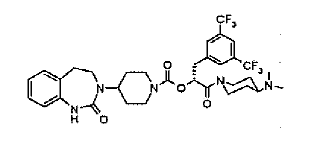 |
| (251) | 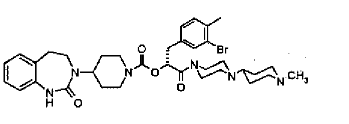 |
| (252) | 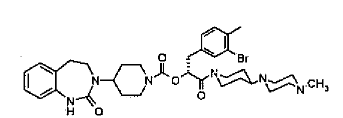 |
| (253) | 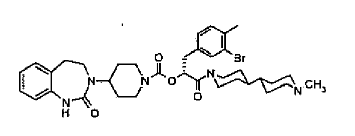 |
| (254) | 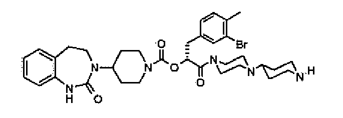 |
| (255) | 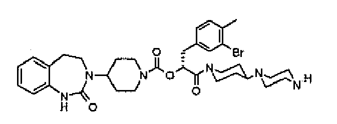 |
| (256) | 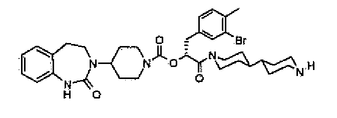 |
| (257) | 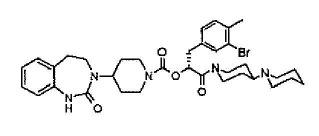 |
| (258) | 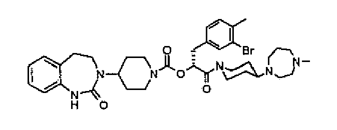 |
| (259) | 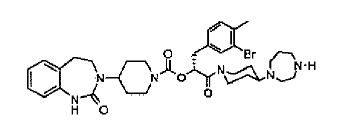 |
| (260) | 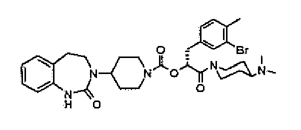 |
| (261) | 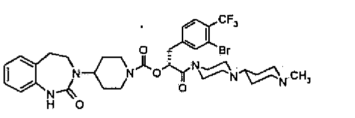 |
| (262) | 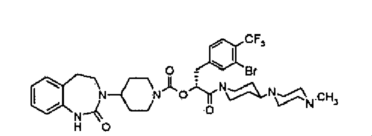 |
| (263) | 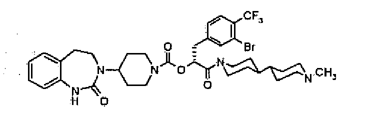 |
| (264) | 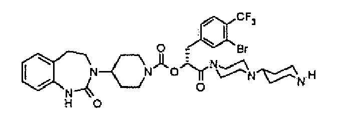 |
| (265) | 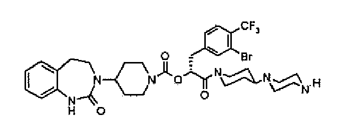 |
| (266) | 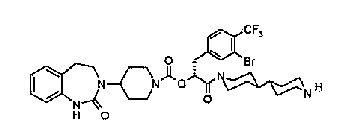 |
| (267) | 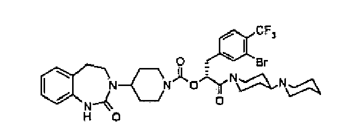 |
| (268) | 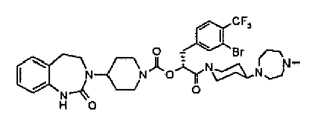 |
| (269) | 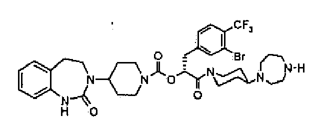 |
| (270) | 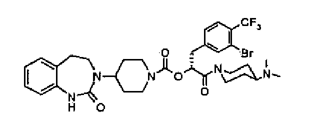 |
| (271) | 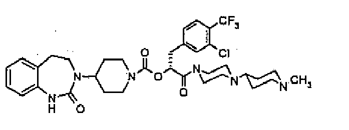 |
| (272) | 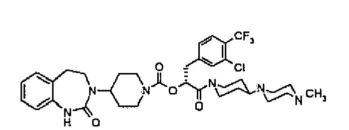 |
| (273) | 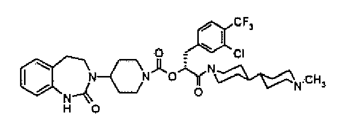 |
| (274) | 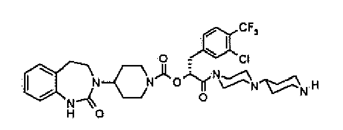 |
| (275) | 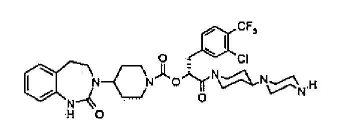 |
| (276) | 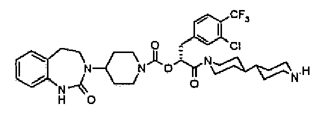 |
| (277) | 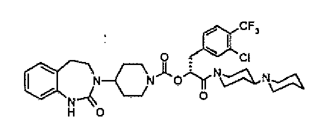 |
| (278) | 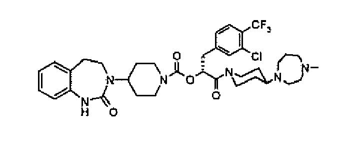 |
| (279) | 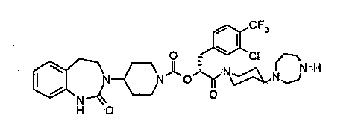 |
| (280) | 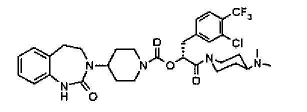 |
| (281) | 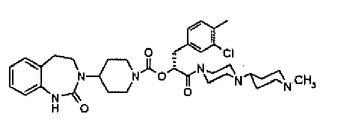 |
| (282) | 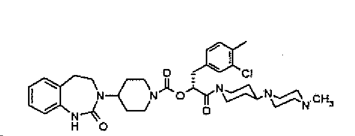 |
| (283) | 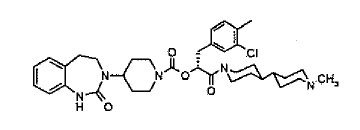 |
| (284) | 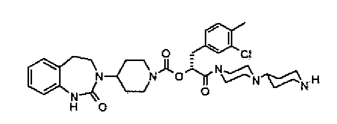 |
| (285) | 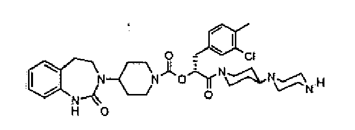 |
| (286) | 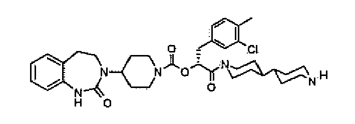 |
| (287) | 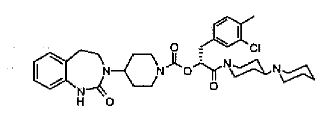 |
| (288) | 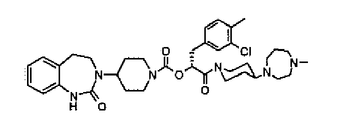 |
| (289) | 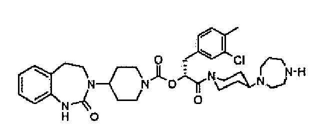 |
| (290) | 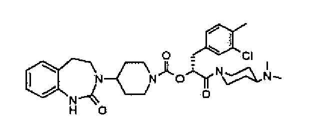 |
| (291) | 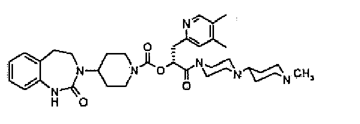 |
| (292) | 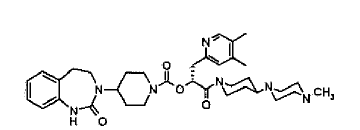 |
| (293) | 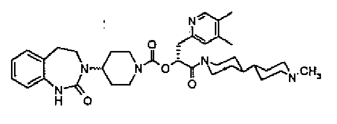 |
| (294) | 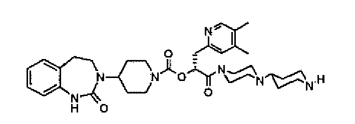 |
| (295) | 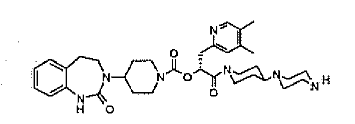 |
| (296) | 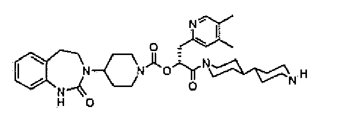 |
| (297) | 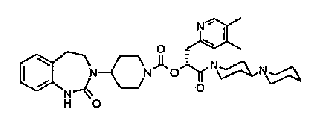 |
| (298) | 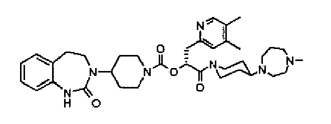 |
| (299) | 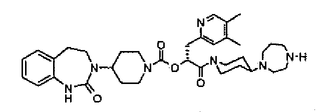 |
| (300) | 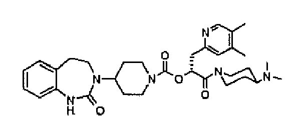 |
| (301) | 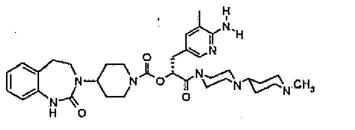 |
| (302) | 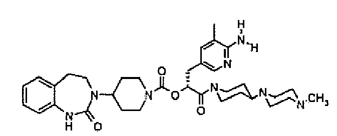 |
| (303) | 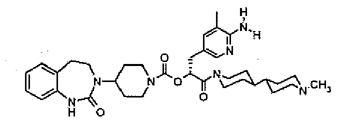 |
| (304) | 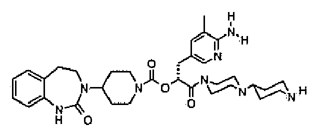 |
| (305) | 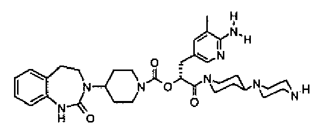 |
| (306) | 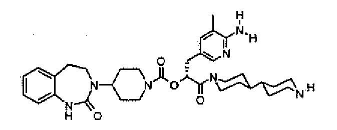 |
| (307) | 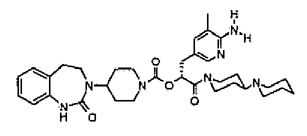 |
| (308) | 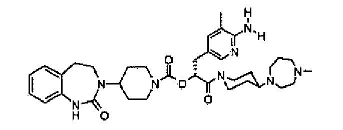 |
| (309) | 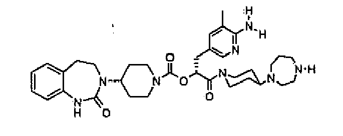 |
| (310) | 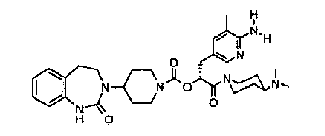 |
| (311) | 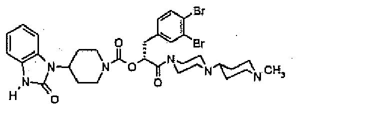 |
| (312) | 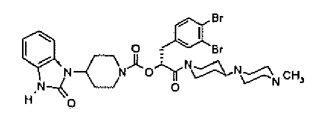 |
| (313) | 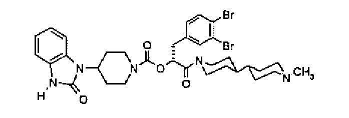 |
| (314) | 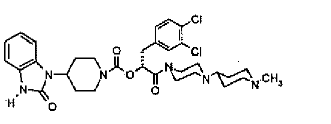 |
| (315) | 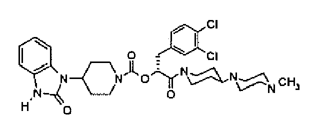 |
| (316) | 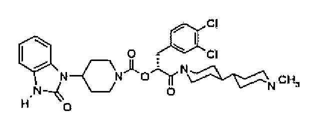 |
| (317) | 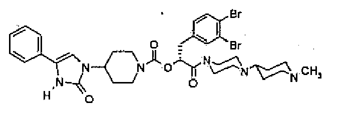 |
| (318) | 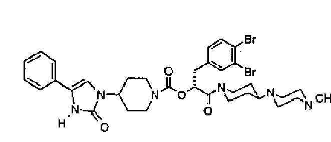 |
| (319) | 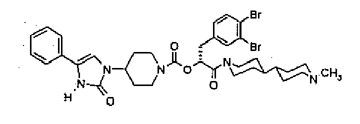 |
| (320) | 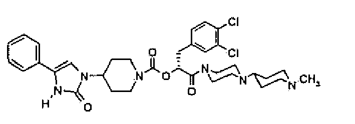 |
| (321) | 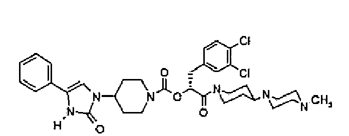 |
| (322) | 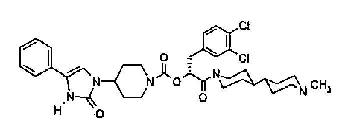 |
| (323) | 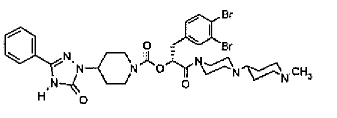 |
| (324) | 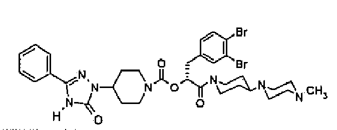 |
| (325) | 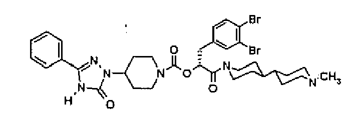 |
| (326) | 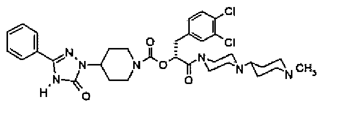 |
| (327) | 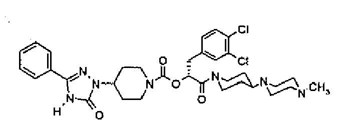 |
| (328) | 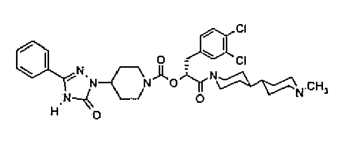 |
| (329) | 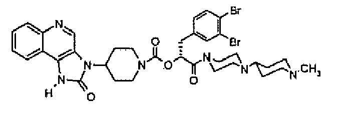 |
| (330) | 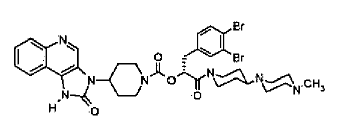 |
| (331) | 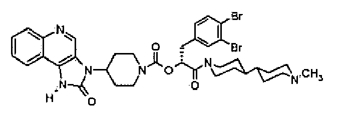 |
| (332) | 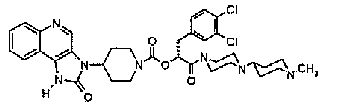 |
| (333) | 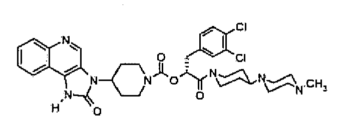 |
| (334) | 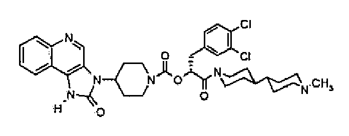 |
| (335) | 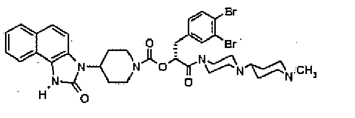 |
| (336) | 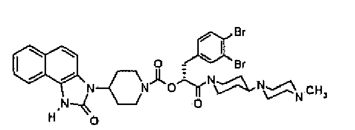 |
| (337) | 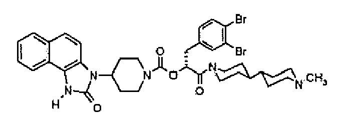 |
| (338) | 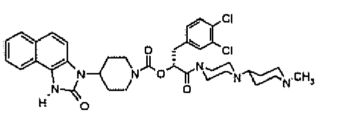 |
| (339) | 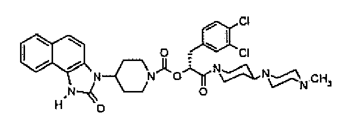 |
| (340) | 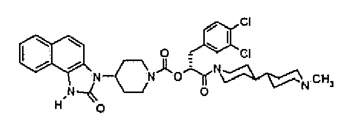 |
| (341) | 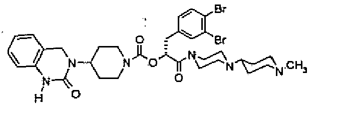 |
| (342) | 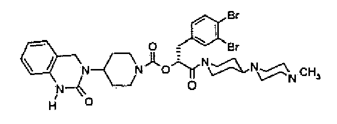 |
| (343) | 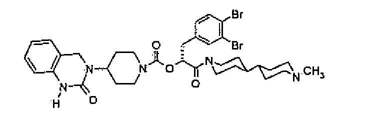 |
| (344) | 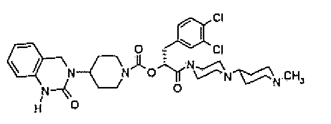 |
| (345) | 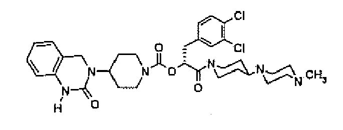 |
| (346) | 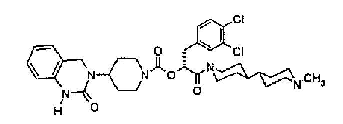 |
| (347) | 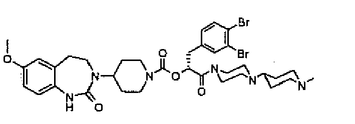 |
| (348) | 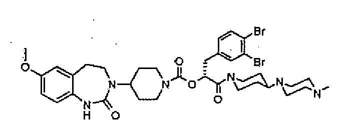 |
| (349) | 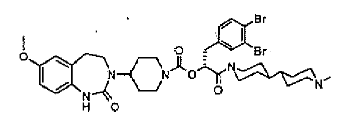 |
| (350) | 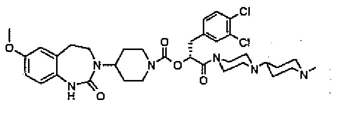 |
| (351) | 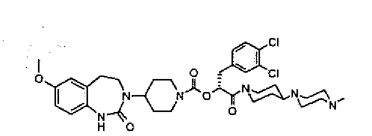 |
| (352) | 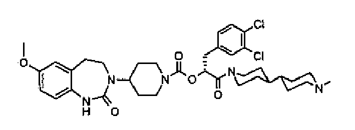 |
| (353) | 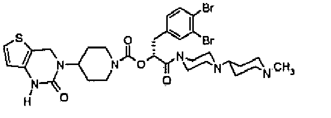 |
| (354) | 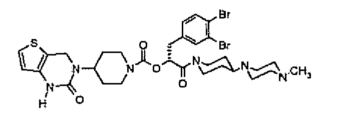 |
| (355) | 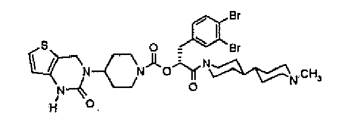 |
| (356) | 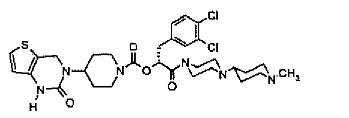 |
| (357) | 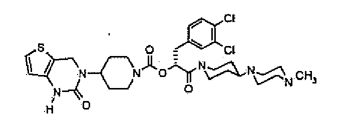 |
| (358) | 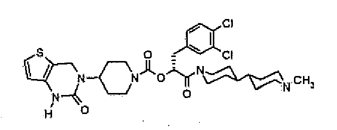 |
| (359) | 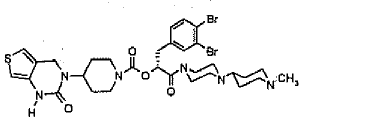 |
| (360) | 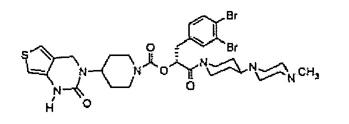 |
| (361) | 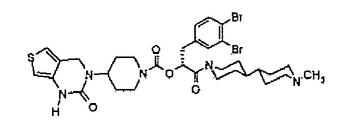 |
| (362) | 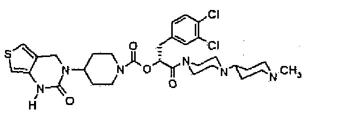 |
| (363) | 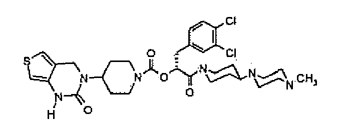 |
| (364) | 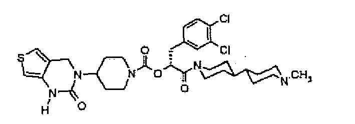 |
leurs énantiomères, leurs diastéréomères et leurs sels.

18. Sels physiologiquement acceptables des composés selon l'une des revendications 1 à 17, avec des acides ou bases anorganiques ou organiques.

19. Médicament, contenant un composé selon l'une des revendications 1 à 17 ou un sel physiologiquement acceptable selon la revendication 18, éventuellement avec un ou plusieurs véhicules et/ou diluants inertes.

20. Utilisation d'un composé selon l'une des revendications 1 à 18, pour la production d'un médicament pour le traitement aigu et prophylactique des céphalées, en particulier des migraines ou des algies vasculaires de la face, pour le traitement du diabète sucré non insulinodépendant ("NIDDM"), du syndrome de douleur régionale complexe (CRPS1), des maladies cardiovasculaires, de la tolérance à la morphine, des diarrhées dues à la toxine *Clostritium,* des maladies de peau, en particulier, des lésions cutanées thermiques et provoquées par un rayonnement comprenant les coups de soleil, des maladies inflammatoires, par exemple, les maladies articulaires inflammatoires (arthrite), les inflammations neurogènes des muqueuses buccales, des maladies pulmonaires inflammatoires, de la rhinite allergique, de l'asthme, des maladies qui s'accompagnent d'un élargissement vasculaire excessif et de ce fait, d'une réduction de l'irrigation des tissus, par exemple, le choc septique et la septicémie, pour l'atténuation des manifestations douloureuses ou la prévention ou le traitement de façon aiguë ou thérapeutique des bouffées congestives dues à un élargissement vasculaire et une augmentation du flux sanguin chez les femmes en période de ménopause présentant une déficience en oestrogènes et les patients souffrant d'un carcinome prostatique soumis à un traitement hormonal.

21. Procédé de production d'un médicament selon la revendication 19; **caractérisé en ce qu'**une liaison est créée de façon non chimique selon l'une des revendications 1 à 18, dans un ou plusieurs véhicules et/ou solvants inertes.

22. Procédé de production des composés de formule générale (I) selon l'une des revendications 1 à 18, **caractérisé en ce que**
(a) une pipéridine de formule générale 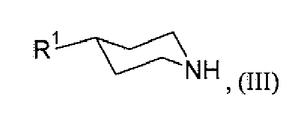 dans laquelle R¹ est tel que défini dans la revendication 1,
est convertie avec un dérivé d'acide carboxylique de formule générale 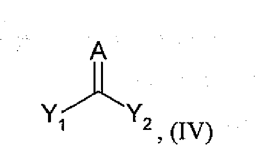 dans laquelle A est tel que défini dans la revendication 1 et Y₁ et Y₂ sont des groupes nucléofuges,
et est convertie avec un composé de formule générale 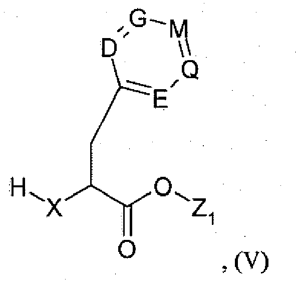 dans laquelle X, D, E, G, M et Q sont tels que définis dans la revendication 1 et Z₁ est un groupe protecteur d'un groupe carboxy, sachant que, avant la réalisation de la réaction dans le radical R¹ d'un composé de formule (III) et/ou dans un composé de formule (V), les fonctions d'acides carboxyliques éventuellement présentes, les fonctions amino primaires ou secondaires ou les fonctions hydroxy sont protégées si nécessaire par un radical protecteur et les radicaux protecteurs éventuellement utilisés sont à nouveau séparés après réalisation de la réaction, ou
(b) un acide carboxylique de formule générale 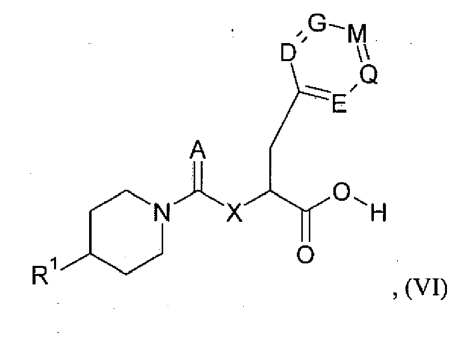 dans laquelle tous les radicaux sont tels que définis dans la revendication 1, sont couplés à une amine de formule générale HNR²R³, dans laquelle R² et R³ sont tels que définis dans la revendication 1, sachant que, avant la réaction dans un composé de formule (VI) et/ou dans les radicaux R² et R³ de l'amine de formule HNR²R³, les fonctions d'acide carboxylique éventuellement présentes, les fonctions amino primaires ou secondaires ou les fonctions hydroxy sont protégées si nécessaire par des radicaux protecteurs et les radicaux protecteurs éventuellement utilisés sont à nouveau séparés après réalisation de la réaction, ou
(c) un composé de formule générale 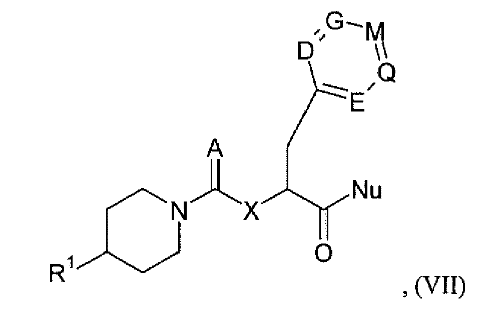 est couplé avec une amine de formule générale HNR²R³, dans laquelle tous les radicaux sont tels que définis dans la revendication 1 et Nu désigne un groupe partant, sachant que, avant la réalisation de la réaction dans un composé de formule (VII) et/ou dans les radicaux R² et R³ de l'amine de formule HNR²R³, les fonctions d'acide carboxylique éventuellement présentes, les fonctions amino primaires ou secondaires ou les fonctions hydroxy sont protégées si nécessaire par des radicaux protecteurs et les radicaux protecteurs éventuellement utilisés sont à nouveau séparés après réalisation de la réaction,
si on le souhaite, un composé ainsi obtenu de formule générale (I) est séparé en ses stéréo-isomères et/ou
un composé ainsi obtenu de formule générale (I) est converti en ses sels, en particulier pour l'utilisation pharmaceutique, en ses sels physiologiquement acceptables.
